# EUROPÄISCHE PATENTSCHRIFT

(11) **EP 2 004 846 B1**
(45) Veröffentlichungstag und Bekanntmachung des Hinweises auf die Patenterteilung: **13.02.2013**
(21) Anmeldenummer: 06791378.0
(22) Anmeldetag: 14.09.2006
(51) Int. Cl.: C12Q 1/68

(54) **EXPRESSIONSPROFILE ZUR VORHERSAGE SEPTISCHER BEDINGUNGEN**
EXPRESSION PROFILES FOR PREDICTING SEPTIC CONDITIONS
PROFILS D'EXPRESSION DESTINES A LA PREVISION D'ETATS SEPTIQUES

(30) Priorität: 21.10.2005 DE 102005050933
(43) Veröffentlichungstag der Anmeldung: 24.12.2008
(73) Patentinhaber: Justus-Liebig-Universität Gießen, 35390 Giessen (DE)
(72) Erfinder: HOSSAIN, Hamid, 35415 Pohlheim (DE); CHAKRABORTY, Trinad, 35394 Giessen (DE); LITTLE, Simon, 35392 Giessen (DE); BEIN, Gregor, 35435 Wettenberg (DE); MENHES, Thilo, 35440 Linden (DE); TCHATALBACHEV, Svetlin, 35392 Giessen (DE)
(74) Vertreter: Meyer-Dulheuer, Karl-Hermann
(86) Internationale Anmeldenummer: PCT/DE2006/001609
(87) Internationale Veröffentlichungsnummer: WO 2007/045197

(56) Entgegenhaltungen:
- WO-A-03/084388
- WO-A-2004/043236
- WO-A-2004/087949
- PRUCHA M ET AL: "EXPRESSION PROFILING: TOWARD AN APPLICATION IN SEPSIS DIAGNOSTICS" SHOCK, Bd. 22, Nr. 1, Juli 2004 (2004-07), Seiten 29-33, XP008036997
- HAHN E L ET AL: "Burn injury with infection alters prostaglandin E2 synthesis and metabolism." THE JOURNAL OF TRAUMA DEC 1999, Bd. 47, Nr. 6, Dezember 1999 (1999-12), Seiten 1052-1057 ; di, XP009082427 ISSN: 0022-5282
- PATHAN N ET AL: "The Complexity Of The Inflammatory Response To Meningococcal Sepsis Revealed By Gene Expression Profiling Using cDNA Microarrays" CRITICAL CARE MEDICINE, Bd. 31, Nr. 12, SUPPL, Februar 2003 (2003-02), Seite A47, XP008037050 ISSN: 0090-3493
- BERNER REINHARD ET AL: "Elevated gene expression of interleukin-8 in cord blood is a sensitive marker for neonatal infection" EUROPEAN JOURNAL OF PEDIATRICS, SPRINGER VERLAG, Bd. 159, Nr. 3, März 2000 (2000-03), Seiten 205-210, XP002419700 ISSN: 0340-6199
- TAKALA A ET AL: "MARKERS OF INFLAMMATION IN SEPSIS" ANNALS OF MEDICINE, FINNISH MEDICAL SOCIETY DUODECIM, HELSINKI, FI, Bd. 34, Nr. 7-8, 1. Oktober 2002 (2002-10-01), Seiten 614-623, XP009055727 ISSN: 0785-3890
- HOLMES C L ET AL: "Genetic polymorphisms in sepsis and septic shock: role in prognosis and potential for therapy" CHEST, THE COLLEGE, CHICAGO, IL, US, Bd. 124, Nr. 3, September 2003 (2003-09), Seiten 1103-1115, XP009019267 ISSN: 0012-3692
- HARBARTH S ET AL: "Diagnostic value of procalcitonin, interleukin-6, and interleukin-8 in critically ill patients admitted with suspected sepsis" AMERICAN JOURNAL OF RESPIRATORY AND CRITICAL CARE MEDICINE, AMERICAN LUNG ASSOCIATION, NEW YORK, NY, US, Bd. 164, Nr. 3, 1. August 2001 (2001-08-01), Seiten 396-402, XP002350238 ISSN: 1073-449X

## Beschreibung

Die vorliegende Erfindung betrifft ein Verfahren zur Erstellung und Auswertung von Expressionsprofilen einer bestimmten Gruppe von Genen, um damit in der Blutprobe eines Patienten die Wahrscheinlichkeit für Sepsis und septische Zustände zu bestimmen.

### Einleitung des allgemeinen Gebietes der Erfindung

Die schwere Sepsis und der septische Schock sind die führenden Todesursachen auf nicht-kardiologischen Intensivstationen weltweit. In Deutschland wird jährlich bei ca. 1 % aller Krankenhauspatienten ein septischer Schock diagnostiziert. Die Mortalitätsrate der schweren Sepsis stagniert seit Jahrzehnten bei ca. 40 %, auch bei bekanntem Erreger, zielgerichteter antibiotischer Behandlung und adäquater supportiver Intensivtherapie.

Ältere Vorstellungen stellten direkte toxische Effekte mikrobieller Komponenten auf Wirtszellen in den Mittelpunkt pathophysiologischer Konzepte der Sepsis. Im letzten Jahrzehnt änderte sich der Blickwinkel dahingehend, dass die in Richtung Hyperinflammation dysregulierte Wirtsantwort selbst als organschädigend angesehen wird: die unkoordinierte Aktivierung verschiedener proinflammatorischer Kaskaden provoziert Störungen der Gefäßpermeabilität und Mikrozirkulation, die wiederum zur kardiovaskulären Dysfunktion und zum Organversagen führen.

Der Begriff SIRS ('Systemic Inflammatory Response Syndrome') beschreibt diese dysregulierte systemische Entzündungskaskade, die zu tief greifenden Störungen der Makro- und Mikrozirkulation mit konsekutiver Multiorganschädigung führt. Das wichtigste Startereignis für ein SIRS sind schwere Infektionen, bei denen Bakterien und/oder ihre Strukturkomponenten wie Endotoxine und diverse Exotoxine in den Intravasal- und/oder Lymphraum gelangen. Über Reaktionen mit Schlüsselmolekülen des Abwehrsystems wird dann eine generalisierte Entzündungsreaktion ausgelöst mit systemischer Ausschüttung biologischer Mediatoren wie Zytokine (insbesondere Interleukin-1 und TNFα), aktivierte Komplement-Komponenten, Gerinnungsfaktoren und Lidpidmediatoren. Grundsätzlich kann jede Infektion in eine Sepsis ausarten.

Weitere Auslöser eines SIRS sind schwere Polytraumen, Verbrennungen und ausgedehnte Gewebsnekrosen. Es ist jedoch bislang ungeklärt, ob diese primär nicht-infektiösen Auslöser in der Tat ein 'steriles' SIRS darstellen oder ob auch hier eine sekundäre Erregerinvasion und/oder der Eintritt bakterieller Toxine in die systemische Zirkulation durch Verlust von Schrankenfunktion und Wirtsabwehrpotential zentral beteiligt sind. Denn bereits wenige Stunden nach einem Trauma, einer großflächigen Verbrennung oder fortschreitenden Pankreatitis können Störungen der Granulozytenfunktion festgestellt werden. Veränderungen in der Chemotaxis, der Adhärenz, der Phagozytoseleistung und des sogenannten "Oxidativen Burst" sind Ausdruck dieses Prozesses einer beeinträchtigten zellulären Immunfunktion.

Aus klinischen Beobachtungen und experimentellen Infektionsmodellen ist bekannt, dass die individuelle Resistenz oder Suszeptibilität gegenüber Krankheitserregern u. a. von genetischen Faktoren gesteuert wird. Im humanem Genom findet sich alle 1000 Basen ein Basenaustausch (single nucleotide polymorphism, SNP), der in einer gegebenen Population mit einer Häufigkeit über 1 % auftritt. Damit ergeben sich bei der Größe des Humangenoms etwa drei Millionen Varianten. Einige dieser Varianten haben funktionelle oder krankhafte Bedeutung, da sie in Bereichen der Gene liegen, die Einfluss auf die Expression eines Gens ausüben oder einen Aminosäureaustausch zur Folge haben. Diese Varianten können beispielsweise in der individuellen Immunantwort gegen Krankheitserreger zu einem resistenten oder suszeptiblen Phänotyp führen. In der Regel wirken bei der Ausprägung eines Phänotyps im Rahmen der Immunantwort zahlreiche verschiedene Gene auf komplexe Weise zusammen.

Einige klinisch wichtige Genvarianten an Genen der Immunantwort gegen Krankheitserreger wurden in der Vergangenheit bereits identifiziert. Ein interessantes Beispiel sind Polymorphismen an Immunantwortgenen, die mit unterschiedlichen Verlaufsformen nach bakteriellen Infektionen assoziiert wurden. Beispielsweise wurden Varianten des Plasminogen-Aktivator-Inhibitor-Typ-1-Gens (PAI-1) mit dem Risiko eines Schocks im Rahmen einer Meningokokken-Erkrankung sowie der Letalität nach Polytrauma assoziiert. Ein SNP in der Promotor-Region des Tumor-Nekrose-Faktor-alpha-Gens wurde mit der Mortalität im Rahmen des septischen Schocks assoziiert.

Bei der Beurteilung von Polymorphismen unterliegt die prognostische Wertigkeit vielen Einzeleinflüssen, die zum jetzigen Zeitpunkt nur teilweise und keinesfalls umfassend bei Betrachtung eines einzelnen Genpolymorphismus erfasst werden können.

Die Diagnose der Sepsis erfolgt anhand des Vorhandenseins verschiedener Entzündungsreaktionen auch als Systemic inflammatoryresponse syndrome (SIRS) bekannt, die den gesamten Organismus betreffen. Charakteristisch ist dabei das Auftreten von mindestens zwei typischen Symptomen, wie:
- Körpertemperatur über 38°C oder unter 36°C
- Pulsfrequenz über 90 Schläge pro Minute
- Atemfrequenz über 20 Züge pro Minute
- Anstieg der Zahl weißer Blutkörperchen (Leukozyten >12.000/mm³ oder <4.000/mm³, oder 10% unreife)

Allerdings sind diese Symptome wenig spezifisch, weil sie auch bei einer Vielzahl von Patienten mit anderen Erkrankungen auftreten.

Eine schwere Sepsis liegt bei verminderter Organdurchblutung, Blutdruckabfall und Organfunktionsstörung vor. Kommt es infolge der Sepsis zu einer Störung der Funktion mehrerer Organe, spricht man vom Multi-Organversagen ("Multiple organ dysfunction syndrome" - MODS).

Es besteht der dringende Bedarf an einer frühen, differenzierten und zuverlässigen Sepsisdiagnose. Ein idealer Sepsisindikator sollte eine frühzeitige Diagnose ermöglichen und bei der Unterscheidung von infektiösen und nicht-infektiösen systemischen Entzündungen helfen. Außerdem sollte es möglich sein, Informationen über den Krankheitsverlauf, den Schweregrad und die Aussichtschancen zu erhalten. Keiner der derzeit bekannten potentiellen Sepsismarker (z.B. C-reaktives Protein, Procalcitonin) erfüllt bislang die Erwartungen der Mediziner vollständig. Erst mit Vorhandensein einer frühen, differenzierten und zuverlässigen Sepsisdiagnose ist es möglich die richtige Therapie einzuleiten. Die Überlebenschance ist umso größer, je früher dies erfolgt, weil das Risiko von Organfunktionsstörungen reduziert wird.

Sepsis-Patienten, insbesondere Patienten mit einem septischen Schock, benötigen kostenintensive stationäre intensivmedizinischer Betreuung. Dies beinhaltet u.a. die Verabreichung von Antibiotika, blutdrucksteigernden Medikamenten und die apparative Unterstützung von Organdysfunktionen.

Der Stand der Technik kennt zur Klärung der Infektionsursache nur die mikrobiologische Analyse von Patientenproben. Diese Verfahren sind zeitaufwendig und leider gelingt der Nachweis der Infektionserreger nur bei ca. 15-20% der Sepsis-patienten. Ca. 25-40% der Patienten versterben trotz einer Antibiotikatherapie und Intensivbehandlung, häufig, weil die Sepsis zu spät erkannt wurde.

Die Entwicklung der Mikroarray-Technologie macht es möglich 10.000 Gene und deren Expressionsprodukte gleichzeitig zu analysieren und zu vergleichen. Die so erstellten Genexpressionsprofile finden z.B. bei der Krebsdiagnostik Einsatz.

In der DE 10315031 wird vorgeschlagen, Genexpressionsprofile für die Diagnostik der Sepsis einzusetzen. Die Erfassung der Genexpression allein ist aber nur ein Baustein des Systems, welches für die Entstehung von Krankheitszuständen verantwortlich ist und erlaubt daher nur einen limitierten Einblick in die relevanten Pathomechanismen. Erst der systembiologische Ansatz mit Einbeziehung aller vorhandenen Systemdaten, wie z.B. des Genoms (Genotypisierung), Transkriptoms (Genexpression), Proteoms (Protein-/Metabolitbestimmung) und Phänotyps (Labor-/Vitalparameter) erlaubt einen ganzheitlichen Einblick in die Vorgänge, die zu einem Krankheitsprozess beitragen und führt damit zu einer verbesserten Diagnostik. WO 03/084388 offenbart ein Verfahren zurr Detektion einer feihen Sepsis duch Analyse einer Vielzahlvon Biomarken über einen Zeitverlauf aus einer Blutprobe.

### Aufgabe

Aufgabe der vorliegenden Erfindung ist es ein zuverlässiges und dififerenziertes Verfahren zur Diagnose von Sepsis und/oder septischen Zuständen im Blut von Patienten bereitzustellen, das klinische und genomische Daten aus dem Blut dieser Patienten berücksichtigt.

### Lösung der Aufgabe

Diese Aufgabe wird erfindungsgemäß gelöst durch die Verfahren gemäß Ansprüche 1 bis 4 und den mit diesen Verfahren erhaltenen Expressionsprofiles die die Korrelation mit klinischen und genomischen Daten aufweisen.

Das erfindungsgemäße Verfahren zur Diagnose von Sepsis und/oder septischen Zuständen im Blut von Patienten mit Infektionen ermöglicht eine wesentlich frühere Erkennung des Krankheitsbildes, als es nach klinischen Kriterien möglich ist. So erhält man etwa 4-7 Tage vor der klinischen Ausprägung des septischen Zustandes einen Hinweis auf die zu erwartende septische Reaktion und kann geeignete medizinische Vorkehrungen treffen.

Das erfindungsgemäße Verfahren beinhaltet die sorgfältige Aufbereitung der biologischen Probe insbesondere die Aufbereitung von Blut, das aus einem Säuger, insbesondere einem Menschen stammt und ermöglicht eine RNA-Extraktion aus Vollblut, die im klinischen Alltag eine rasche und dauerhafte Stabilisierung der RNA sowie ausreichende RNA-Mengen aus möglichst wenig Vollblut erlaubt.

Die Erstellung eines Expressionsprofiles erfolgt mit Genen, die nach Analyse von klinischen, laborchemischen und genomischen Daten als besonders relevant für die Vorhersage von Sepsis erkannt wurden.

### Ausführungsbeispiele

Für die Erstellung von optimalen Genexpressionsprofilen wird eine prospektive Patientenstudie zur Erforschung der Wirtsantwort auf eine schwere Infektion oder ein schweres Trauma erstellt. Hierbei werden schwer erkrankte Patienten rekrutiert und deren klinische, laborchemische und genomische Daten erhoben und miteinander verknüpft um Aussagen über Entwicklung und Verlauf einer Sepsis zu machen. Die Fragen, die gestellt werden sind: 1) Können aufgrund der Expressionsprofile Patientengruppen stratifiziert werden? 2) Gibt es Unterschiede in der Genexpression von Patienten mit und ohne Sepsis? 3) Können im Expressionsmuster frühe von späten Phasen der Sepsis unterschieden werden? 4) Können in der Genexpression organspezifische Muster identifiziert werden? 5) Kann durch das Expressionsmusters eine Vorhersage über die Sepsis gemacht werden? 6) Kann das Expressionsmuster prognostisch genutzt werden? 7) Können Biomarker identifiziert werden, die als Surrogatmarker für den Verlauf oder Ausgang der Erkrankung eingesetzt werden können?

Zunächst werden drei Patientenkohorten untersucht (Erwachsene Patienten mit Polytrauma, Erwachsene mit schwerer Pneumonie, Frühgeborene unter der 32. Schwangerschaftswoche) und dabei klinische und genomische Daten aus dem Blut dieser Patienten ausgewertet, um Aussagen über Krankheitsverlauf und Prognose der Sepsis zu machen. Entscheidende Kriterien sind die Regelung der Patienteneinschleusung (Einschluss/Ausschlusskriterien), die Regelung der Blutentnahme (Zeiten, Blutentnahmesystem), Regelung des Transports und der Lagerung der Proben, Regelung der Eingabe der klinischen und personenbezogenen Daten.

Das biologische Probenmaterial (beispielsweise Blut) wird mit einer besonderen Technik aufbereitet, um zuverlässige und differenzierbare Resultate zu erzielen, die auch für die Routinediagnostik geeignet sind.

Das erfindungsgemäße Verfahren zum in-vitro Nachweis von Sepsis und/oder Sepsis-ähnlichen Zuständen in einer biologischen Probe eines Säugers umfasst folgende Schritte:
i) Isolieren von RNA aus einer biologischen Probe eines Säugers
ii) Markieren der RNA aus Schritt i) mit einem detektierbaren Marker
iii) Hybridisieren der RNA aus Schritt ii) mit einer DNA, die auf einem Microarray angeordnet ist und mindestens ein für Sepsis spezifisches Gen oder Genfragment ist, unter Hybridisierungsbedingungen
iv) Quantitatives Erfassen der Markierungssignale der hybridisierten RNA aus Schritt iii) in einem Expressionsprofil
v) Vergleich des Expressionsprofiles aus Schritt iv) mit einer Kontrollprobe hinsichtlich stärkerer oder schwächerer Expression der für Sepsis spezifischen Gene oder Genfragmente
i) Verknüpfung des Expressionsprofiles aus Schritt v) mit Protein- und Metabolitmustern aus der biologischen Probe

### 1. Probenaufbereitung

Als Probe wird biologisches Material eines Säugers wie Liquor, Urin, Trachealsekret, Seminalflüssigkeit, Ascitesflüssigkeit, Speichel, Punkttat oder Lymphflüssigkeit, bevorzugt Blut verwendet.

Die Blutentnahme der Patienten erfolgt vorzugsweise in kommerziell erhältlichen PaxGene-Röhrchen nach Herstellerangaben (PreAnalytix GmbH, Schweiz). Blut von Frühgeborenen liegt in Form von Nabelschnurblut vor. Es werden zweimal 750-800µl PaxGene-Lösung und zweimal 250 - 300µl Nabelschnurblut in eine separate sterile Küvette z.B. eine Plastik- oder Glasküvette gefüllt. Blut von Erwachsenen liegt in Form von Vollblut vor. Es werden zweimal 2,5 ml Vollblut in ein gebrauchsfertiges mit PaxGene-Lösung befülltes Röhrchen gefüllt. Die Proben werden in den PaxGene-Röhrchen für 2-4 Stunden bei Raumtemperatur gelagert und danach bei -80°C eingefroren. In diesem Zustand können die Proben über Jahre gelagert werden. Der Vorteil des Lösungsgemischs der PaxGene-Röhrchen liegt in der raschen und dauerhaften Stabilisierung der RNA. Dadurch wird gewährleistet, dass auch bei zeitlicher Verzögerung des Transports (klinischer Alltag) die RNA nicht degradiert wird. Alternativ kann auch EDTA-Blut abgenommen werden, hierbei müssen aber der Transport und die RNA-Extraktion sofort erfolgen. Die RNA-Extraktion erfolgt hierbei nach gängigen Verfahren.

### 2. RNA-Prozessierung

Zunächst erfolgt die Isolierung der RNA aus Proben von Schritt 1 nach Standardverfahren z.B. nach Arbeitsanleitung des PAXgene™ Blood RNA Kits (PreAnalytix). Die isolierte RNA wird danach mit einem speziellen Protokoll zur Fällung aufgereinigt, um die Qualität der RNA zu erhöhen:
A. Zugabe von 0.1 Volumeneinheiten 3 M Natrium-Acetat, pH 5.2, und 3 Volumeneinheiten Ethanol (98%) zu dem RNA-Extrakt.
B. Mischen und Inkubation des Gemisches bei -20°C für 4 Stunden oder bei -80°C für mindestens 1 Stunde zur Präzipitation der RNA.
C. Zentrifugation des Gemisches bei 12,000 x g für 30 Minuten bei 4°C.
D. Waschen des RNA-Pellets mit 2 Volumeneinheiten 75% (v/v) Ethanol für 2 Min.
E. Zentrifugation des Gemisches bei 12,000 x g für 5 min bei 4°C. alternativ kann Schritt D wiederholt werden.
F. Trocknen des Pellets mittels Einsatz eines SpeedVac ohne Erhitzen und Aufnahme der RNA in steriles 35 µl H₂O.

Abschließend wird die RNA quantifiziert, beispielsweise durch Messung mit Nanodrop, und ein Qualitätsprofil wird mit dem Agilent Bioanalyzer® erstellt.

### 3. In-vitro-Transkription und Hybridisierung

Vor der weiteren Verarbeitung weisen die RNA-Proben ein regelrechtes Agilent-Profil und eine Wellenlängenratio bei 260/280 zwischen 1,8-2,1 auf. Die Markierung und Umschreibung der RNA in floureszenz-markierter cRNA erfolgt mit 2 µg RNA und wird beispielsweise mit dem "CodeLink Expression Assay Reagent Kit, Manual Prep" (Amersham Biosciences) entsprechend dem "CodeLink Target Labelling and Array Hybridisation" Protokoll durchgeführt. Die Fluoreszenzmarkierung erfolgt mit Streptavidin-Cy5, kann aber auch mit anderen gängigen Floureszenzmarkem durchgeführt werden. Die markierte cRNA wird auf Ihre Qualität und Quantität überprüft. 30 µg cRNA werden fragmentiert und als Triplikate auf die CodeLink Human Set I Microarrays (Fa. Amersham Bioscience) über Nacht bei 37°C im Schüttler hybridisiert. Das Färben und Waschen der Arrays erfolgt in entsprechenden Vorrichtungen nach dem Protokoll des Herstellers (Amersham Biosciences). Abschließend erfolgt das Scannen der Arrays mittels Genepix 4000b Axxon Instr. (Molecular Dynamics).

Alternativ werden als Marker auch Farbmarker oder Radioaktivmarker eingesetzt, dies ist dem Fachmann bekannt und kann mit kommerziell erhältlichem Material durchgeführt werden.

Es ist allgemein bekannt, dass es schwierig ist, Blut insbesondere Blut aus Säuglingen als Lieferant des Transkriptoms diagnostisch zu nutzen. Das erfindungsgemäße Verfahren überwindet diese Schwierigkeit und zeigt, dass die in dieser Weise aufbereiteten Proben sich ideal eignen, um in Expressionsanalysen eingesetzt zu werden und ein zuverlässiges, einfach zu gewinnendes diagnostisches Mittel darstellen.

Insgesamt können mit Microarrays unterschiedlicher Herkunft und Zusammensetzung 10000-50000 Gene gleichzeitig untersucht werden. Beispielsweise werden mit dem CodeLink Human Set I Microarrays (Fa. Amersham Bioscience) 9945 Gene untersucht. Von diesen sind etwa 6710 Gene differentielle reguliert zwischen Patienten mit und ohne Sepsis über einen Zeitverlauf von 14 Tagen, wobei als Kriterium für differentielle Regulation 0.5 < Standardabweichung der Expressionsintensität < 10 definiert wird. Die 6710 Gene sind in Tabelle 1 aufgelistet.

Mittels eines statistischen Verfahrens, wie beispielsweise Rankproducts (RP), Statistical Analysis of Microarrays (SAM), EDGE oder einer anderen geeigneten Methode, können statistisch signifikant regulierte Gene mit einem selbst festlegbaren Grad des Fehlers 1. Art (z.B. (False Discovery Rate (=FDR) oder P-Wert als Maße für die Schätzung von falsch-positiven Genen) ermittelt werden.

Durch Änderung des FDR oder P-Wertes ändert sich auch die Zahl der signifikanten Gene. Prinzipiell reicht die Skala des FDR von sehr restriktiv, wie z.B. 0,02% mit nur 20 signifikanten Genen oder 1% mit 120 Genen bis hin zu sehr umfassend wie z.B. 50% mit etwa 1000 Genen. Je größer die FDR, desto höher die Anzahl der regulierten Gene, aber auch desto höher die Anzahl der möglichen falsch-positiven Gene. Es gilt eine FDR zu wählen, die umfassend genug ist um möglichst viele Gene einzubeziehen, die am Geschehen beteiligt sein könnten, aber gleichzeitig die Zahl der möglichen falsch-positiven Gene in einem akzeptablen Bereich hält, wie z.B. eine FDR von 30%, die alle Gene von 0% bis 30% einbezieht, also auch die hochsignifikanten Gene, bei einer allgemein akzeptablen Zahl an falsch-positiven Genen.

Die Zeitspanne zwischen Trauma und Aufnahme in das Krankenhaus (Schockraum, Intensivstation) ist von Patient zu Patient unterschiedlich. Die Untersuchungen haben gezeigt, dass eine Unterscheidung zwischen Patienten mit und ohne Sepsis mittels Genexpressionsprofilen bereits bei Aufnahme des Patienten möglich ist. Vorzugsweise erfolgt für die Erstellung der Profile in einer Zeitspanne von 6 bis 24 Stunden nach Trauma, da nach dieser Zeitspanne die Unterscheidung Sepsis/Nicht-Sepsis deutlich wird (entspricht etwa dem Zeitpunkt der Aufnahme auf Intensivstation). Alternativ werden die Expressionsprofile zu früheren Zeitpunkten, wie z.B. Aufnahme im Schockraum, durchgeführt.

Die Zahl der signifikant regulierten Gene beispielsweise zum Zeitpunkt der Aufnahme auf Intensivstation zwischen Patienten mit und ohne Sepsis beträgt etwa 860 Gene (FDR=30%) und reflektiert die statistisch/biologisch relevante Genexpression zu diesem frühen Zeitpunkt. Tabelle 2 zeigt diese 860 Gene.

Bereits einen Tag später steigt die Zahl der signifikant regulierten Gene auf etwa 1400 Gene (Tabelle 3) und bleibt konstant bei etwa 1100 Genen am 3. Tag (Tabelle 4. Zwischen dem 4. und 7. Tag fällt die Zahl der signifikant regulierten Gene auf max. etwa 270 Gene ab (Tabelle 5). Das ist auch der Zeitraum in dem die septischen Krankheitszustände ihren Höhepunkt erreichen und klinisch die Sepsis diagnostiziert wird. Am 9. Tag steigt die Zahl der signifikant regulierten Gene wieder auf etwa 1100 Gene (Tabelle 6). Die Zahl der insgesamt im Zeitverlauf signifikant regulierten Gene ergab beispielsweise mit RP etwa 2530 Gene bei einer FDR=30% (Tabelle 7).

Dieses Profil der Zahl der signifikant regulierten Gene im Zeitverlauf mit zunächst steigender Zahl der Gene bis Tag 3 nach Aufnahme auf Intensivstation, dann drastischem Abfall zwischen Tag 4 und 7 und dann erneut Anstieg der Zahl ab Tag 8 auf das Niveau von Tag 3 spiegelt sehr gut den klinischen Verlauf des Patienten wider. Vor allem zwischen Tag 4 und 7 wird die Sepsis diagnostiziert, die häufig in diesem Zeitraum mit einer Immunparalyse einhergeht. Durch die Expressionsprofile werden die Gene und damit die Mechanismen identifiziert, die zu diesem Phänotyp beitragen.

Die so ermittelten Gene im Zeitverlauf, aber vor allem zum Zeitpunkt der Aufnahme auf Intensivstation, sind Kandidatengene für die Bestimmung einer Prädispostion für Sepsis und werden erfindungsgemäß diagnostisch genutzt, um Patienten zu identifizieren, die ein höheres Risiko tragen an einer Sepsis zu versterben. Darüber hinaus werden bereits zu diesen Zeitpunkten Gene identifiziert, die mit ihren messbaren Genprodukten (Proteinen) und mit einzelnen klinischen Symptomen im weiteren Krankheitsverlauf in Zusammenhang stehen. Somit ist es möglich sowohl Frühsymptome einer Sepsis vorherzusagen als auch den zu erwartenden Schweregrad zu prognostizieren.

Die Expressionsmuster zum Zeitpunkt der Aufnahme der Patienten zeigen deutliche Unterschiede zwischen Patienten, die Tage später eine Sepsis entwickeln oder keine Sepsis entwickeln. Anhand des Musters werden Patienten frühzeitig ermittelt, die während des stationären Aufenthaltes eine Sepsis entwickeln können. Diese Gruppen von Genen (Tabelle 7) werden anhand des Expressionsprofiles besonders beobachtet und dienen als diagnostische Marker, um die Ausbildung einer Sepsis vorherzusagen. Die frühzeitige Identifikation dieser Patienten führt zu erhöhten Kontrollmaßnahmen und verändertem Therapieregime.

Des Weiteren dienen Expressionsprofile, die während des stationären Aufenthaltes erstellt werden als Verlaufskontrolle bei Polytrauma/schwerer Infektion verwendet (Tabelle 2 bis 6).

Expressionsmuster, die im Verlauf des stationären Aufenthaltes erstellt werden, zeigen bei Patienten, die eine Sepsis entwickeln, eine zunehmende Ausschaltung der transkriptionellen/zellulären Aktivität einer großen Gruppe von Genen. Diese macht sich in der Herunterregulierung von Genen unterschiedlicher funktioneller Gruppen bemerkbar (z.B. angeborene und erworbene Immunität, Apoptose, Transkriptionsfaktoren, Metabolismus). Diese Deaktivierung der zellulären Aktivität/Transkription zeigt sich nicht bei Patienten, die keine Sepsis entwickeln. Zeitgleich mit der Reduzierung der Transkription oben genannter Gengruppen, werden Gene anderer funktioneller Gruppen, vor allem der angeborenen Immunität, vermehrt eingeschaltet. Diese Aktivierung von evolutionsgenetisch ursprünglichen Genen wie z.B. Defensinen ist als Mobilisierung verbliebener Abwehrsysteme jeder einzelnen Zelle zu deuten.

Dieses Profil der frühzeitigen Herunterregulierung der transkriptionellen/zellulären Aktivität ist diagnostisch als Verschlechterung des Zustandes und als Zeichen der beginnenden Sepsis anzusehen.

Unabhängig von der Ebene des Transkriptoms (Ebene 1) werden routinemäßig Laborparameter wie Blutbild, CRP, PCT, Leber- und Nierenwerte, Elektrolyte, etc. bestimmt, die zur Standarddiagnostik auf Intensivstationen gehören. Diese Laborparameter stellen teilweise die Ebene des Proteoms dar (Ebene 2). Es können aber auch Proteine (Genprodukte), die nicht routinemäßig erfasst werden, abhängig von der Genexpression bestimmt werden. Aber nicht nur Proteine, sondern auch Metabolite, die als Zwischenprodukte in Stoffwechselwegen (wie z.B. TNF-α, Glykolyse oder Gykoneogenese, Fettsäurezyklus) entstehen, werden gemessen. Somit werden Genexpressionsprofile mit Protein- und Metabolitmustem verknüpft. Diese Verknüpfung von Transkriptom und Proteom erhöht die Aussagekraft, da hierbei biologisch aktive Zwischen-und Endprodukte der Genexpression, nämlich Genprodukte (Proteine) oder Metabolite mit erfasst werden und somit das Profil mitbestimmen.

Weiterhin werden Vitalparameter wie Blutdruck, Herzfrequenz, Temperatur, etc. bestimmt. Diese gehören zum Standardrepertoire auf Intensivstationen und bilden die Ebene des Phänotyps (Ebene 3). Somit werden Transkriptom, Proteom und Phänotyp beliebig miteinander verknüpft. Dadurch kann die biologische Relevanz der Aussage weiter erhöht werden.

Durch Mutationsscreening (Polymorphismenuntersuchungen, SNP=Single Nucleotide Polymorphism) bei den Patienten wird die Ebene des Genoms (Ebene 0) untersucht. Die SNP-Untersuchungen werden sowohl abhängig von der Genexpressionsanalyse durch Identifizierung von Hotspots auf den Chromosomen als auch unabhängig von der Genexpression durch Zuhilfenahme von bekannten publizierten Daten durchgeführt. Durch die Untersuchung des Genoms ist der Weg vom Genotyp zum Phänotyp komplett beschrieben und erlaubt durch Verknüpfung der vier Ebenen Assoziationen zwischen einem Krankheitszustand (Phänotyp) und einem Genotyp. Dadurch wird erneut die biologische Relevanz einer Aussage gesteigert.

Durch Genotypisierungsstudien bekannter SNPs des TNF-Gens (TNF -1032A>G (rs1799964), TNF -863C>A (rs1800630), TNF -857C>T (rs1799724), TNF - 308G>A (rs1800629), TNF -238G>A (rs361525), TNF 488C>T (rs1800610), TNF 859A>G (rs3093662)) sowie LTA-252A>G (rs909253) und LTA-80C>A (rs2239704) an 159 Patienten kaukasischer Herkunft mit schwerem Trauma konnte eine Assoziation zwischen bestimmten SNPs und einer erhöhten Inzidenz und Mortalität der Sepsis gefunden werden:
Beispielsweise sind die TNF-308A und LTA-252G Allele signifikant (P<0.0001) assoziiert mit erhöhten TNF-α-Blutspiegel (hier:Plasma) am Tag der Aufnahme und an den folgenden 14 Tagen bei Patienten mit schwerem Trauma.

Die TNF-308A, LTA-252G und LTA-80A Allele sind außerdem signifikant (P<0.0001; P<0.042; P<0.031) assoziiert mit der Inzidenz der Sepsis bei Patienten mit schwerem Trauma.

Weiterhin sind die TNF-308A (rs1800629) und LTA-252G (rs909253) Allele signifikant (P<0.0001; P<0.0002) assoziiert mit letalem Ausgang bei Patienten mit schwerem Trauma.

Die Genotypisierung von Patienten bezüglich TNF-308A (rs1800629), LTA-252G (rs909253) und LTA-80A (rs2239704) und die von Beginn an kontinuierliche Messung der TNF-α-Blutspiegel (hier: Plasma) bei z.B. traumatisierten Patienten oder auch sonst schwer erkrankten Patienten sind somit von großem Wert einerseits für die frühzeitige Identifizierung von Patienten, die eine Sepsis entwickeln könnten, und andererseits für die Prognose der Erkrankung (hier: Tod).

Bei Patienten mit diesen Genotypen können bei Aufnahme und auch im weiteren Verlauf Expressionsprofile erstellt werden, die sich vom Profil derer ohne diese Genotypen unterscheiden. Diese Profile können diagnostisch z. B. als "Death Signatures" (Tabelle 8) genutzt werden, um einerseits Risikopatienten zu identifizieren und andererseits frühzeitig eine Progredienz der Erkrankung zu erkennen. Ausgehend vom Genotyp und Expressionsprofil können nun relevante Proteine und deren Spiegel in Körperflüssigkeiten wie z.B. Blut, Urin, Liquor, Trachealsekret, Seminalflüssigkeit, Ascitesflüssigkeit, Speichel, Punkttat oder Lymphflüssigkeit bestimmt werden, die wiederum zum Phänotyp beitragen. Therapeutische Strategien, die beispielsweise an den Stoffwechselwegen dieser Proteine ansetzen, können das Ergebnis (Outcome) beeinflussen und somit den Phänotyp verändern.

Die Erfindung nutzt Expressionprofile (Ebene 1) und die Verknüpfung mit den anderen Ebenen (Proteinspiegel, Vitalwerte, Genotyp) um
a) Patienten zu stratifizieren unabhängig vom Krankheitszustand (dadurch können Patienten in neue Gruppen eingeteilt werden, an die nicht vorher gedacht wurde)
b) Patienten zu klassifizieren (z.B. Risikopatient/kein Risikopatient)
c) einen septischen Krankheitszustand frühzeitig zu diagnostizieren (z.B. bereits bei Aufnahme des Patienten)
d) Krankheitsverläufe (= Progredienz/Verbesserung, z.B. Nieren- oder Leberversagen) frühzeitig zu erkennen und verfolgen
e) den Ausgang der Erkrankung zu prognostizieren (z.B. Tod/Überleben)
und erlaubt dadurch eine verbesserte Einschätzung des Krankheitszustandes und damit auch eine angepasstere Therapie. Durch die Verlaufsbeobachtung der differentiell regulierten Gene (Tabelle 1) können nicht nur Aussagen über gemeinsam regulierte Gene gemacht werden, sondern auch Erkenntnisse über individuelle Genregulation jedes einzelnen Patienten gewonnen werden. Dadurch kann zu jedem Zeitpunkt die Therapie individuell angepasst werden.

Zudem können über die Gene in diesen Expressionsprofilen, Aussagen über die Zelltypen gemacht werden, die zu diesem Profil beigetragen haben (z.B. Monozyten, Granulozyten). Dadurch können diejenigen Zelltypen identifiziert werden, die maßgeblich an der Entstehung von septischen Krankheitszuständen und an der Progredienz beteiligt waren.

Die quantitative Bestimmung dieser Zelltypen kann dann selbst als Marker für den Krankheitsverlauf dienen.

In Gleicherweise können Genotypen, Expressionsprofile, Proteinmuster, Laborparameter und Vitalparameter (= Bestandteile aller 4 Ebenen) als Biomarker identifiziert werden, die einzeln oder in Kombination als Surrogatmarker für Krankheitsverläufe oder einen Krankheitsausgang (z.B. Tod oder Überleben) eingesetzt werden können. Die Surrogatmarker könnten z.B. in Vielfältigerweise bei der Durchführung von klinischen und pharmakologischen Studien eingesetzt werden, um z.B. frühzeitig Verläufe zu prognostizieren und damit die Dauer und Kosten von Studien deutlich zu reduzieren.

Eine weitere Ausführungsform der Erfindung umfasst einen Sepsis-Array für Erwachsene und Frühgeborene. Dazu wird ein Microarray mit den Genen erstellt, die im Zeitverlauf bei der Ausbildung einer Sepsis differentiell reguliert sind und die an der Entstehung einer Entzündungsantwort und Sepsis bei Erwachsenen und Frühgeborenen verantwortlich sind.

Dieser Chip für Sepsisdiagnose findet eine breitere Anwendung in der Diagnostik und wird auch an großen Patientenkollektiven eingesetzt.

In Anlehnung an den Sepsis-Array mit Genen (Oligonukleotiden) wird ein Sepsis-Protein-Chip erstellt.

Dieser Sepsis-Protein-Chip enthält die Genprodukte (z.B. Proteine) der Gene, die im Zeitverlauf bei der Ausbildung einer Sepsis differentiell reguliert sind, Gene aus Tabelle 1 (Seq.-ID 1 bis Seq.-ID 6705), bevorzugt Gene aus Tabelle 7 und 8. Mittels des Sepsis-Protein-Chips können Körperflüssigkeiten wie z.B. Blut (Plasma, Serum), Urin, Liquor oder Trachealsekret zeitnah auf das Vorhandensein dieser Genprodukte untersucht werden.

Die Gene Seq.-ID 1 bis Seq.-ID 6705 werden zur Belegung von Microarrays benutzt.

Die vorliegende Erfindung gibt die Möglichkeit anhand der Transkripte und deren Muster einen Krankheitsprozess zu charakterisieren und Krankheitsverläufe zu beschreiben und/oder frühzeitig vorherzusagen. Es handelt sich hierbei um eine Methode, die man als Transkriptologie bezeichnen kann.

Die Auswertung der Expressionanalysen erfolgt in enger Anlehnung mit den übrigen Patientendaten in einer Datenbankstruktur, in dem die relevanten Daten strukturell und semantisch vereinheitlicht und in einer dem Datenschutz genügenden pseudonymisierten Form gesammelt werden. Dies umfasst die für die Studien eigens hergestellten Datenbanken GRID (GRID-DB) und PIRO (PIRO-DB). Der Einschluss eines Patienten in die Auswertung der Expressionsanalysen und damit die Definition seiner Daten als relevant erfolgt über den Eintrag der ID in die GRID-DB. In der GRID-DB sind alle patientenbezogenen persönlichen Daten, Einschluss- und Ausschlusskriterien sowie alle Diagnosen abgelegt. In GRID-DB werden auch krankheitsbezogene Scores berechnet. Die Daten werden als Textdateien in die PIRO-Datenbank (PIRO-DB) exportiert.

Die über das PDMS (Patient Data Management System) erfassten Daten sind in einer Oracle^{™}-Datenbank abgelegt, deren Struktur sich am HL7-Modell orientiert (ICU Data). Aus den nur für die Systemadministratoren freigegebenen Rohdaten wird über in der Datenbank implementierte SQL-Anweisungen der studienrelevante Datenausschnitt extrahiert, pseudonymisiert und als Datenbank-View den Domänenspezialisten (DS) präsentiert. In diesem pseudonymisierten Bereich werden Hilfstabellen zur Vereinheitlichung, zeitlichen Einordnung, Aggregation der Daten gepflegt.

Aus ICU Data werden Hilfstabellen über eine in Oracle^{™} integrierte Datensicherungsfunktion generiert, welche dem Entity-Attribute-Value (EAV) Datenmodell entsprechen. Aus diesen Hilfstabellen werden mittels SQL-Skripte Textdateien erzeugt, die in eine relationale Oracle-Forschungsdatenbank (PIRO-DB) transferiert werden. Sie bilden dort den Data Mart der klinischen Daten für die klinische und biologische Forschung. Aus diesen Daten wird ein PIRO-Score (Predisposition, Infection, Response, Organ dysfunction) zu früher Diagnosestellung und Prognose von Sepsis-Patienten generiert.

Mit Hilfe der Kombination dieser Datenbanksysteme ist die Integration von Patientendaten aus der klinischen Routinedokumentation und Daten aus der klinischen und biologischen Forschung möglich, dies bildet die Grundlage zur Identifikation spezifischer krankheitsassoziierter Genexpressionsmuster und zur Entwicklung kombinatorischer Scoring-Systeme zur genaueren und frühzeitigen Diagnosestellung und zur Vorhersage des Krankheitsverlaufs.

Zusätzlich werden neue Software-Programme erstellt und in die Auswertung der Genexpressionsprofile eingebunden.

Das QuBE (Query Building Environment) ist ein benutzerfreundliches Perl- und Java-Skript, welches Einzelabfragen als auch komplexe Abfragen der PIRO-DB hinsichtlich personenbezogener, klinischer, laborchemischer und genomischer Daten zulässt (Quellcode 1).

Der DataManager ist ein Datenintergrationsprogramm, geschrieben in Pascal (Boland-Delphi 6), welches die großen Datenmengen, die bei den Microarrays entstehen, zunächst automatisch nach Patienten in entsprechende Ordner sortiert (Quellcode 2). Weiterhin ermittelt das Programm fehlende Daten in den Arrays und ersetzt diese mit Mittelwerten aus technischen Replikaten entsprechender Microarrays. Es berechnet zudem verschiedene Parameter, die Ausschluss über die Qualität eines Arrays geben (z. B. Zahl der eingeschalteten Gene, Zahl der Spots, deren mittlere Intensität niedriger ist als der mittlere lokale Hintergrund). Vorteile des DataManager sind eine Verbesserung der Datenintegration und - aufbereitung sowie der Entscheidungsfindung, welche Arrays in die Analyse eingesetzt werden sollten. Dies ist besonders wichtig im Hinblick auf den Einsatz der Arrays als diagnostisches Mittel. Eine Verfälschung der Ergebnisse durch minderwertige Arrays wird vermieden. QuBe verknüpft Microarraydaten mit klinischen Daten und zeigt den Zusammenhang zwischen Transkriptom und Phänotyp. Diese Information hilft bei der Zuordnung von Expressionsprofilen zu klinischem Phänotyp.

Weiterhin werden als kommerzielle Software-Programme Imagene, CodeLink Batch Submission und Expression Analysis, Avadis, Dchip, SAM, Genesis verwendet.

Tabelle 1 zeigt differentiell regulierte Gene bei polytraumatisierten Patienten mit Sepsis und ohne Sepsis im Zeitverlauf über 9 Tage ohne Angabe der relativen Änderung; Seq.-ID 1-6706
ACC= Accession-Nummer; Symbol= Gensymbol; LLID= LocusLink-ID bzw. Gene-ID. Keine Angaben der relativen Änderung, da diese Gene sowohl gemeinsam als auch individuell bei Patienten mit Sepsis und ohne Sepsis reguliert werden. Kriterium für differentielle Regulation: 0.5 < Standardabweichung der Expressionsintensität jedes Gens über jeden Patienten < 10
Tabelle 2 zeigt signifikant über- und unterexprimierte Gene bei polytraumatisierten Patienten mit Sepsis im Vergleich zur Kontrollgruppe von polytraumatisierten Patienten ohne Sepsis zum Zeitpunkt der Aufnahme; Seq.-ID 6706-7563
ACC= Accession-Nummer; Symbol= Gensymbol; LLID= LocusLink-ID bzw. Gene-ID; Pat. 1 bis 10 = relative Änderung der Genexpression des einzelnen Patienten mit Sepsis gegenüber der Kontrollgruppe bei Aufnahme; FC= Fold Change bzw. relative Änderung der Genexpression der Gruppe der Patienten mit Sepsis gegenüber der Kontrollgruppe bei Aufnahme
Tabelle 3 zeigt signifikant über- und unterexprimierte Gene bei polytraumatisierten Patienten mit Sepsis im Vergleich zur Kontrollgruppe von polytraumatisierten Patienten ohne Sepsis 24 Stunden nach Aufnahme; Seq.-ID 7563-8948
ACC= Accession-Nummer; Symbol= Gensymbol; LLID= LocusLink-ID bzw. Gene-ID; Pat. 1 bis 10 = relative Änderung der Genexpression des einzelnen Patienten mit Sepsis gegenüber der Kontrollgruppe 24 h nach Aufnahme; FC= Fold Change bzw. relative Änderung der Genexpression der Gruppe der Patienten mit Sepsis gegenüber der Kontrollgruppe 24 h nach Aufnahme
Tabelle 4 zeigt signifikant über- und unterexprimierte Gene bei polytraumatisierten Patienten mit Sepsis im Vergleich zur Kontrollgruppe von polytraumatisierten Patienten ohne Sepsis 3 Tage nach Aufnahme; Seq.-ID 8948-10041
ACC= Accession-Nummer; Symbol= Gensymbol; LLID= LocusLink-ID bzw. Gene-ID; Pat. 1 bis 7 = relative Änderung der Genexpression des einzelnen Patienten mit Sepsis gegenüber der Kontrollgruppe 3 Tage nach Aufnahme; FC= Fold Change bzw. relative Änderung der Genexpression der Gruppe der Patienten mit Sepsis gegenüber der Kontrollgruppe 3 Tage nach Aufnahme
Tabelle 5 zeigt signifikant über- und unterexprimierte Gene bei polytraumatisierten Patienten mit Sepsis im Vergleich zur Kontrollgruppe von polytraumatisierten Patienten ohne Sepsis 5 Tage nach Aufnahme; Seq.-ID 10041-10306
ACC= Accession-Nummer; Symbol= Gensymbol; LLID= LocusLink-ID bzw. Gene-ID; Pat. 1 bis 7 = relative Änderung der Genexpression des einzelnen Patienten mit Sepsis gegenüber der Kontrollgruppe 5 Tage nach Aufnahme; FC= Fold Change bzw. relative Änderung der Genexpression der Gruppe der Patienten mit Sepsis gegenüber der Kontrollgruppe 5 Tage nach Aufnahme
Tabelle 6 zeigt signifikant über- und unterexprimierte Gene bei polytraumatisierten Patienten mit Sepsis im Vergleich zur Kontrollgruppe von polytraumatisierten Patienten ohne Sepsis 9 Tage nach Aufnahme; Seq.-ID 10306-11379
ACC= Accession-Nummer; Symbol= Gensymbol; LLID= LocusLink-ID bzw. Gene-ID; Pat. 1 bis 10 = relative Änderung der Genexpression des einzelnen Patienten mit Sepsis gegenüber der Kontrollgruppe 9 Tage nach Aufnahme; FC= Fold Change bzw. relative Änderung der Genexpression der Gruppe der Patienten mit Sepsis gegenüber der Kontrollgruppe 9 Tage nach Aufnahme
Tabelle 7 zeigt signifikant über- und unterexprimierte Gene bei polytraumatisierten Patienten mit Sepsis im Vergleich zur Kontrollgruppe von polytraumatisierten Patienten ohne Sepsis im Zeitverlauf über 9 Tage; Seq.-ID 11379-13905
ACC= Accession-Nummer; Symbol= Gensymbol; LLID= LocusLink-ID bzw. Gene-ID; FC_1 bis 9 = Fold Change bzw. relative Änderung der Genexpression bei Patienten mit Sepsis gegenüber der Kontrollgruppe bei Aufnahme (FC-0), nach 1d (FC_1), 3d (FC_3), 5d (FC_5) und 9d (FC_9)
Tabelle 8 zeigt signifikant regulierte Gene bei polytraumatisierten Patienten mit Sepsis und letalem Ausgang und gegenüber polytraumatisierten Patienten mit Sepsis aber ohne letalem Ausgang zum Zeitpunkt der Aufnahme ("Death Signature"); Seq.-ID 13906-14075
ACC= Accession-Nummer; Symbol= Gensymbol; LLID= LocusLink-ID bzw. Gene-ID; FC= Fold Change bzw. relative Änderung der Genexpression der Gruppe der Patienten mit Sepsis und letalem Ausgang gegenüber Patienten mit Sepsis ohne letalem Ausgang bei Aufnahme

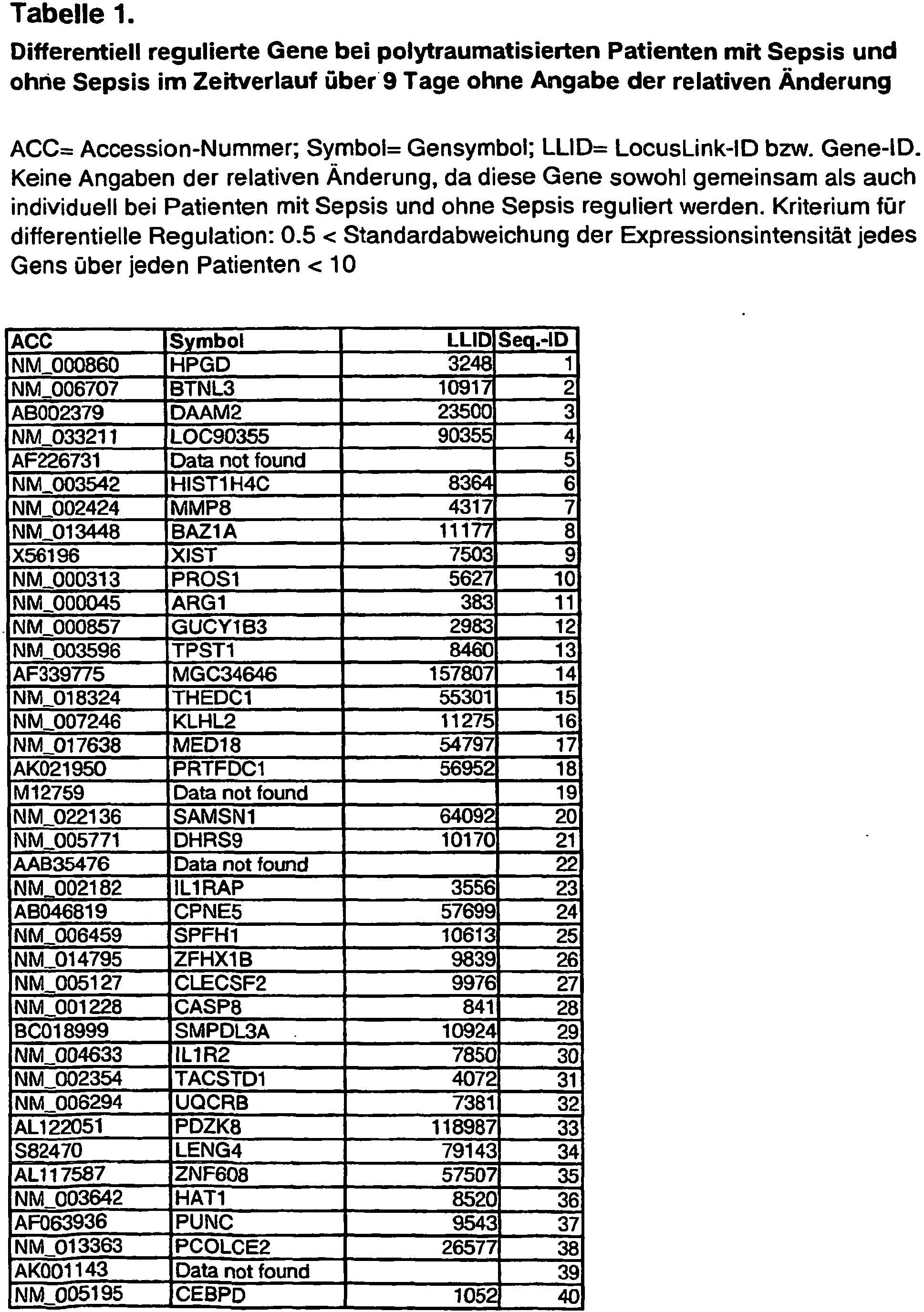

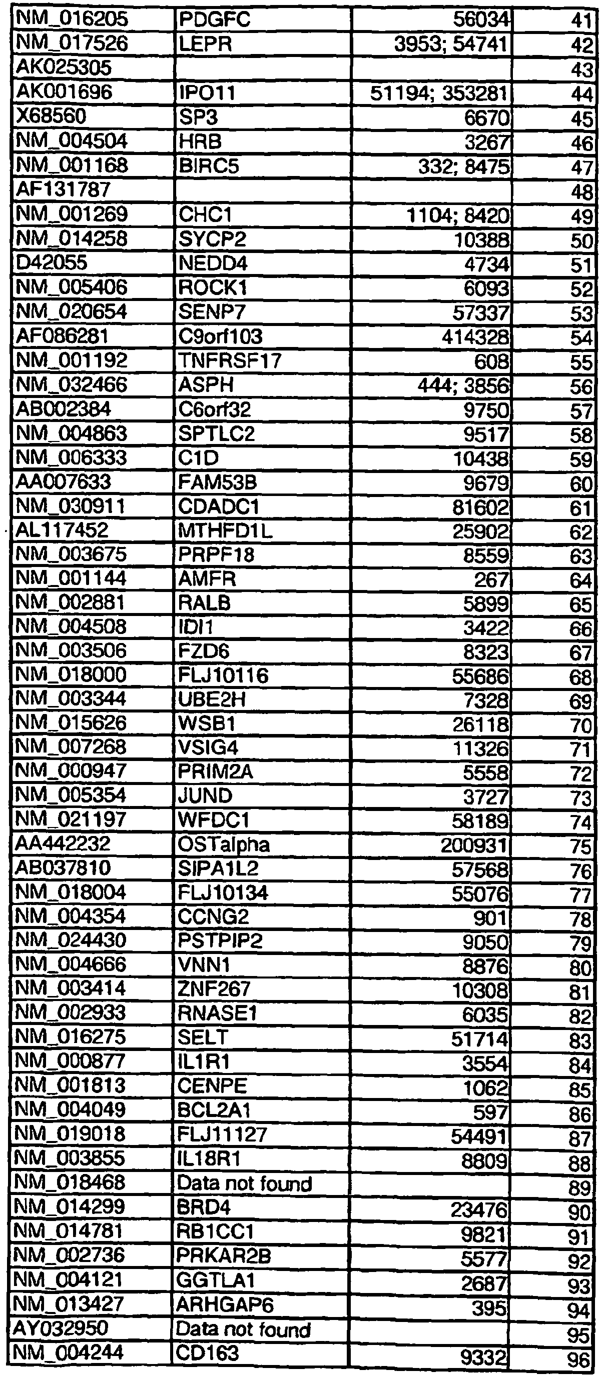

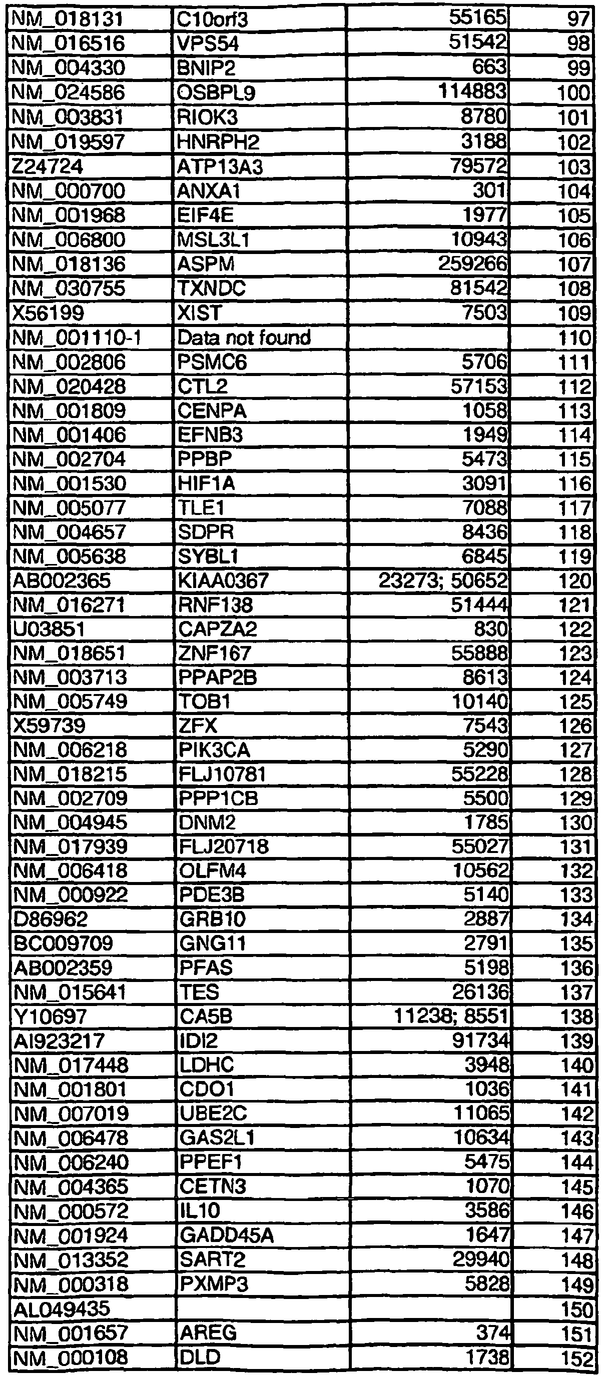

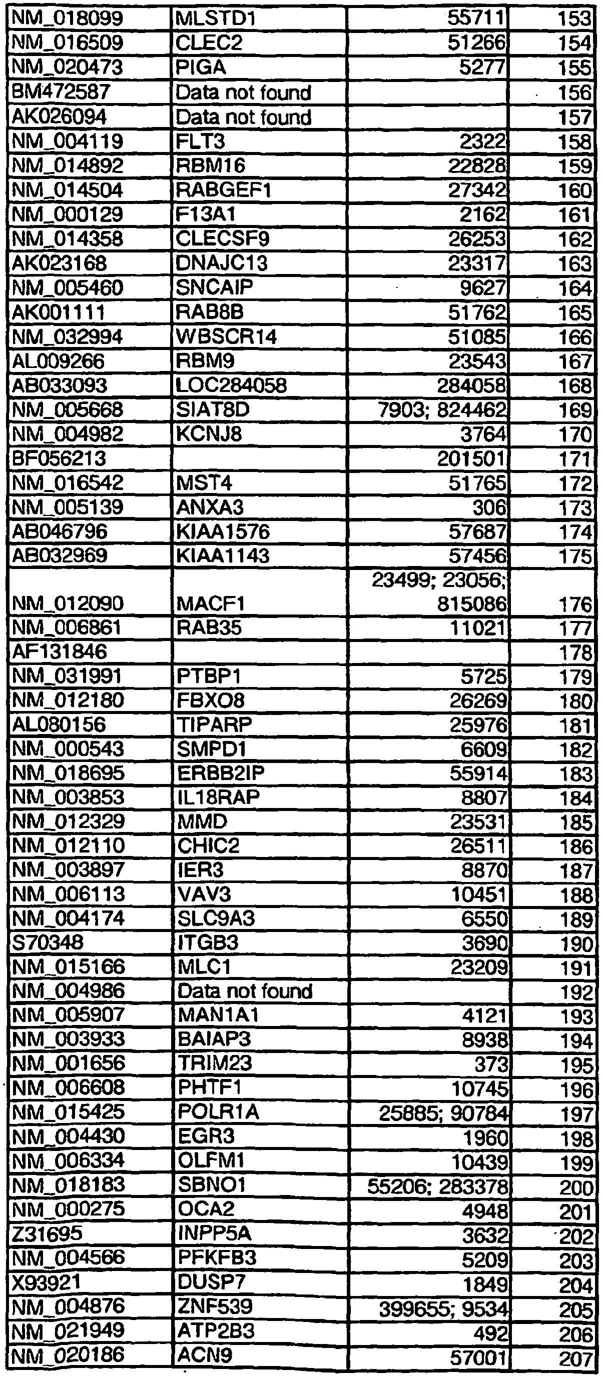

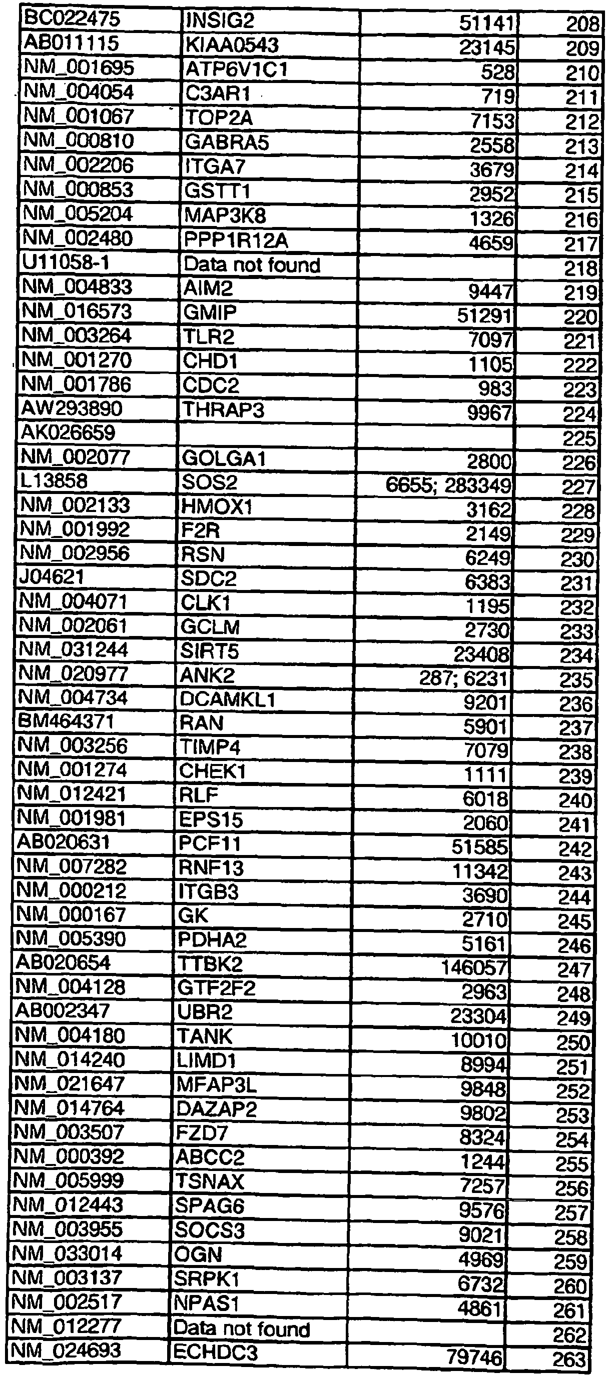

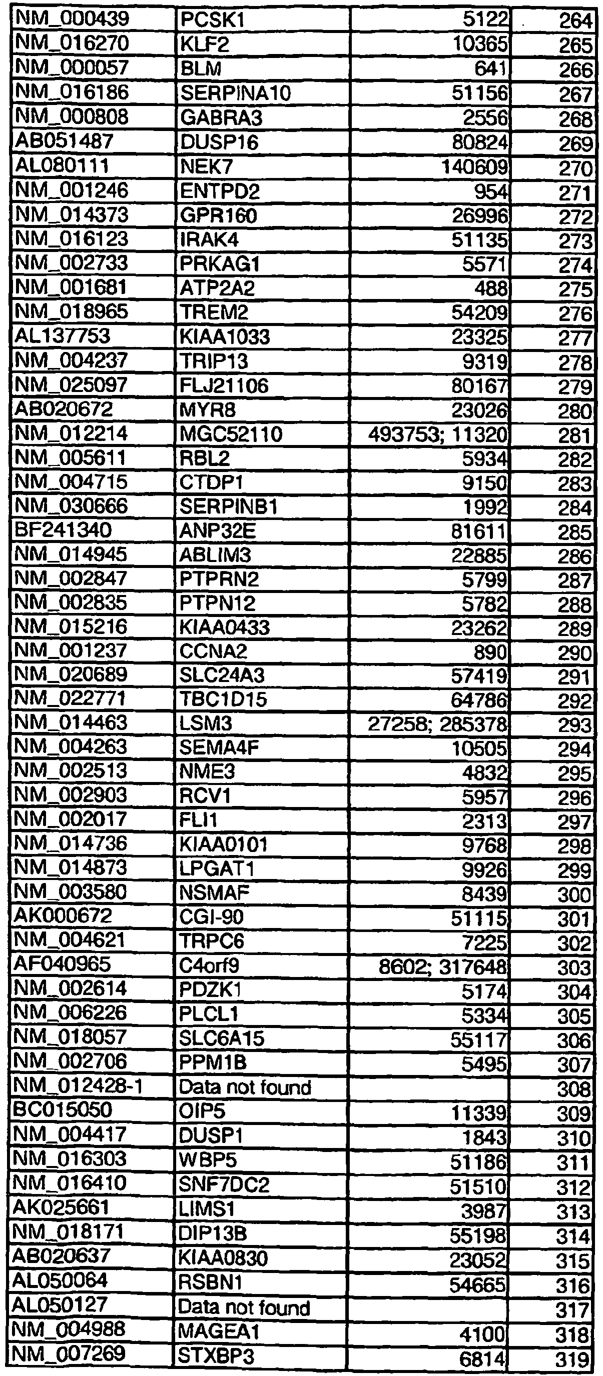

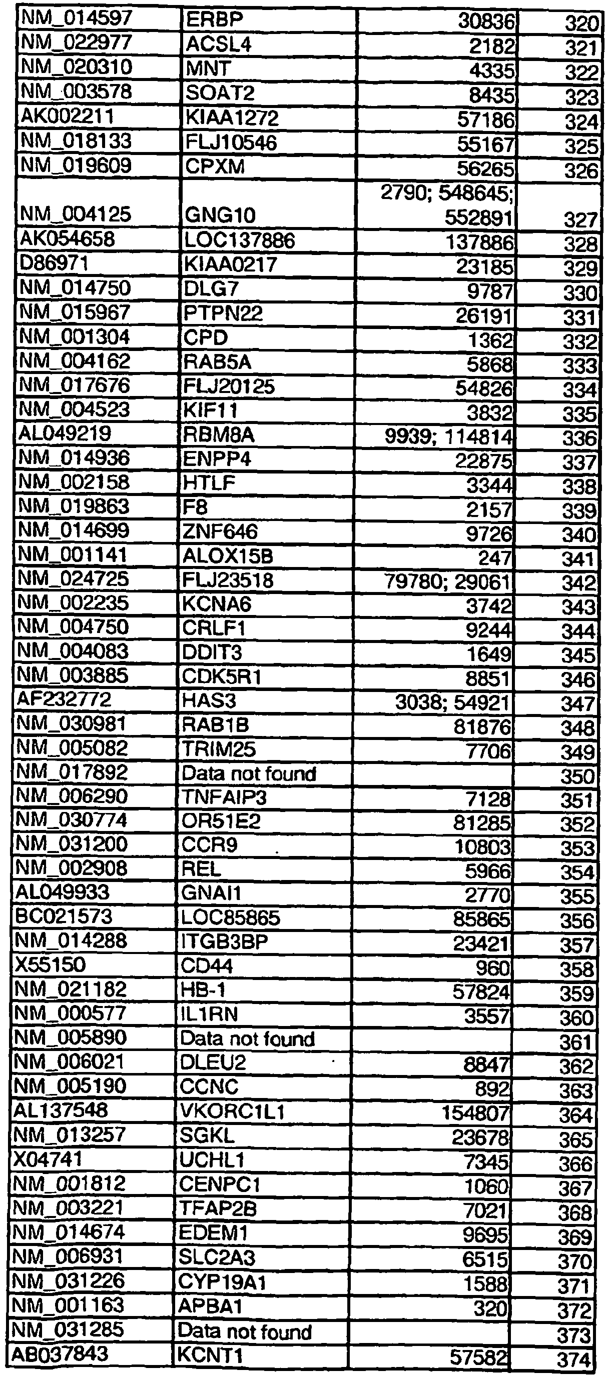

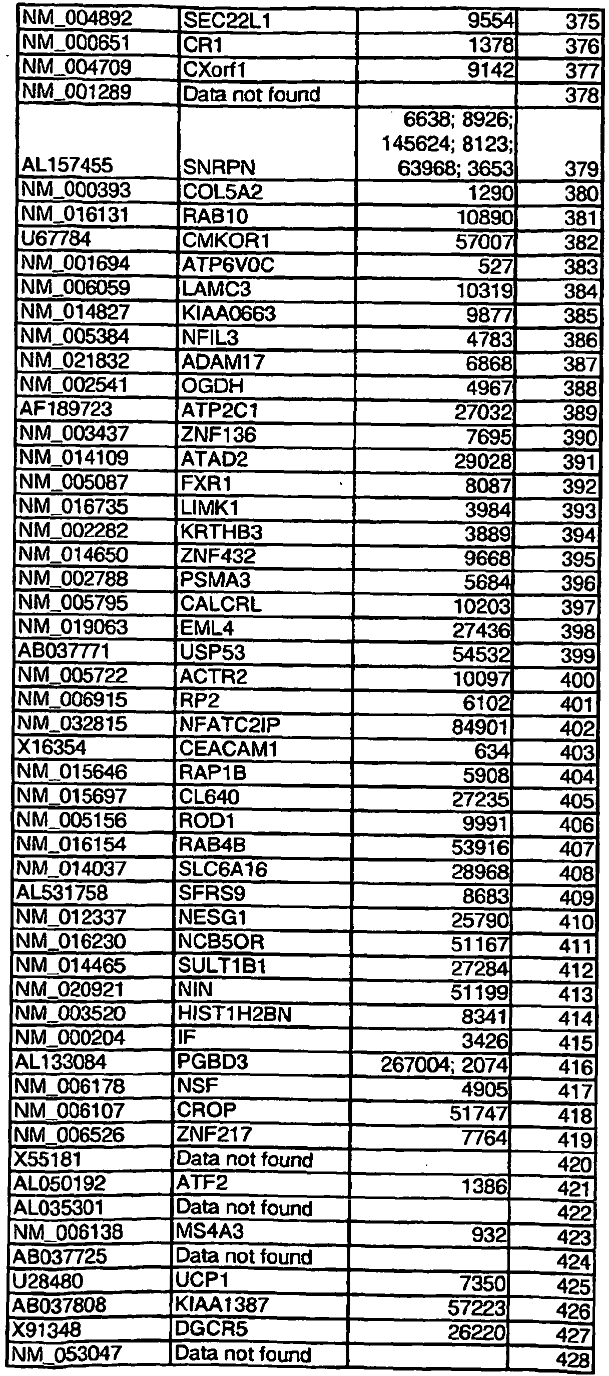

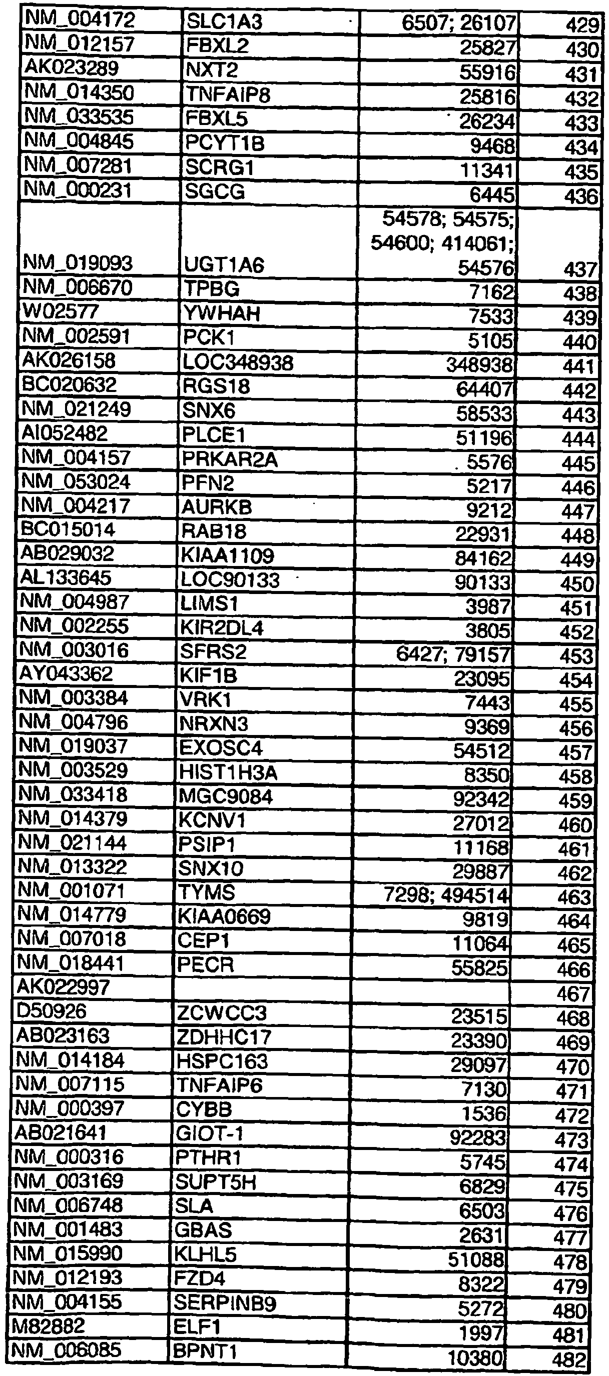

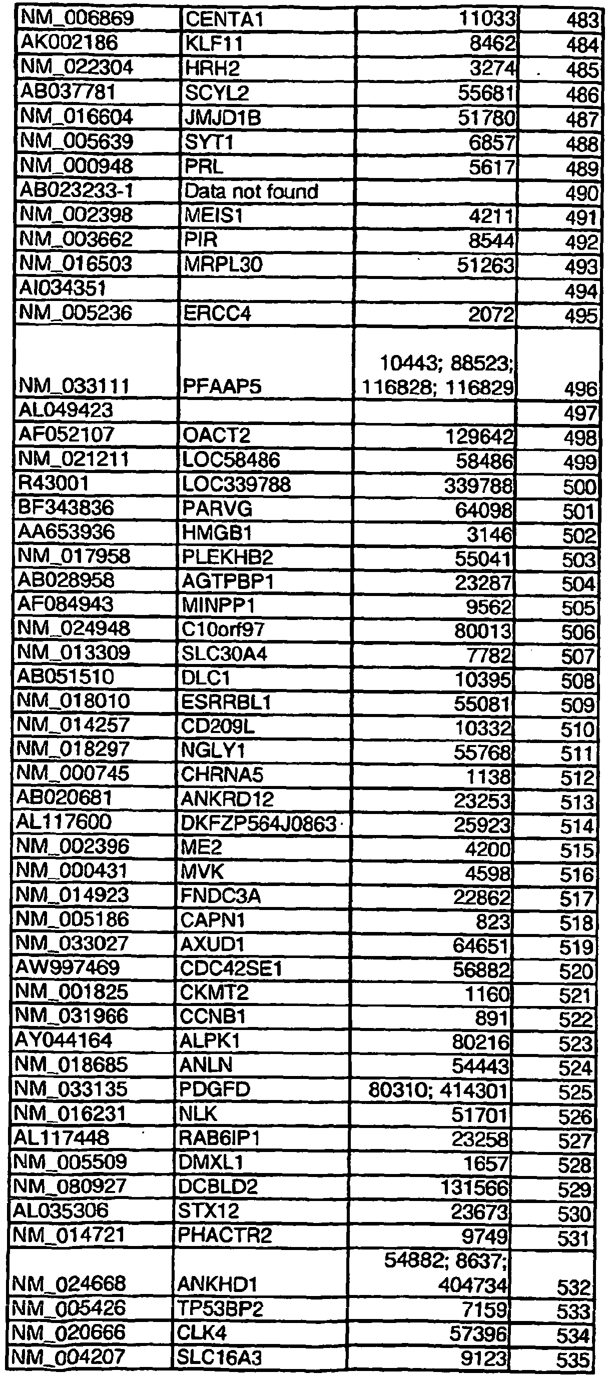

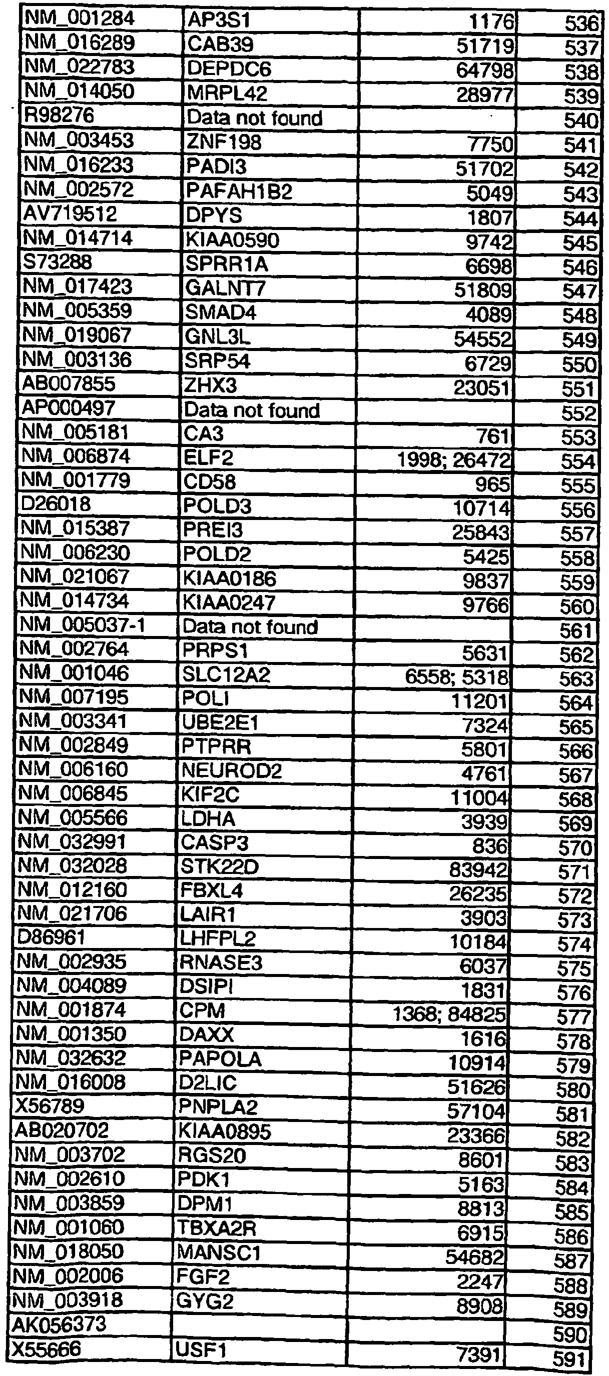

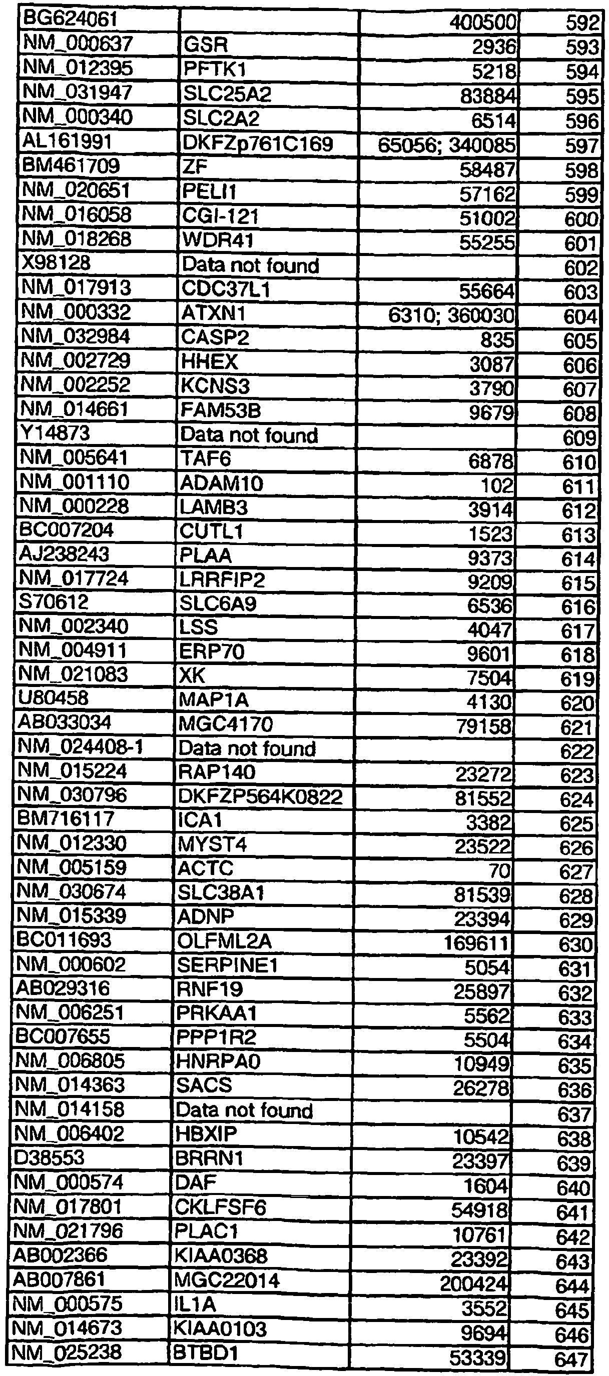

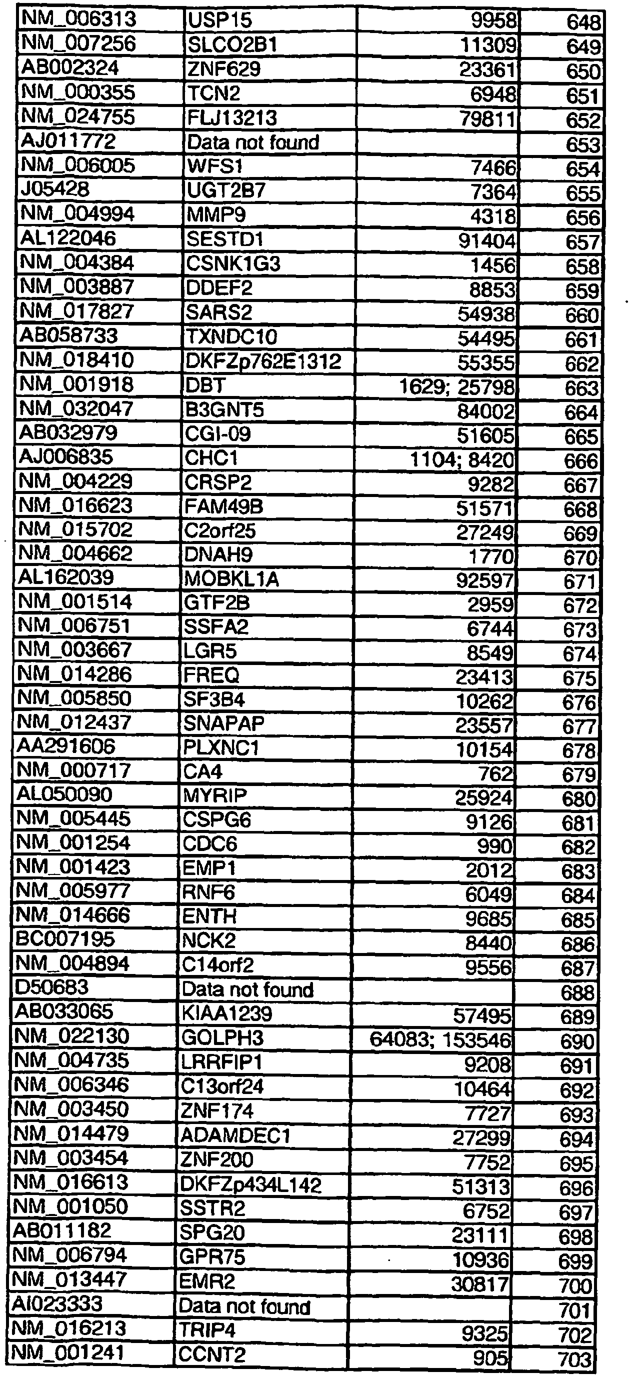

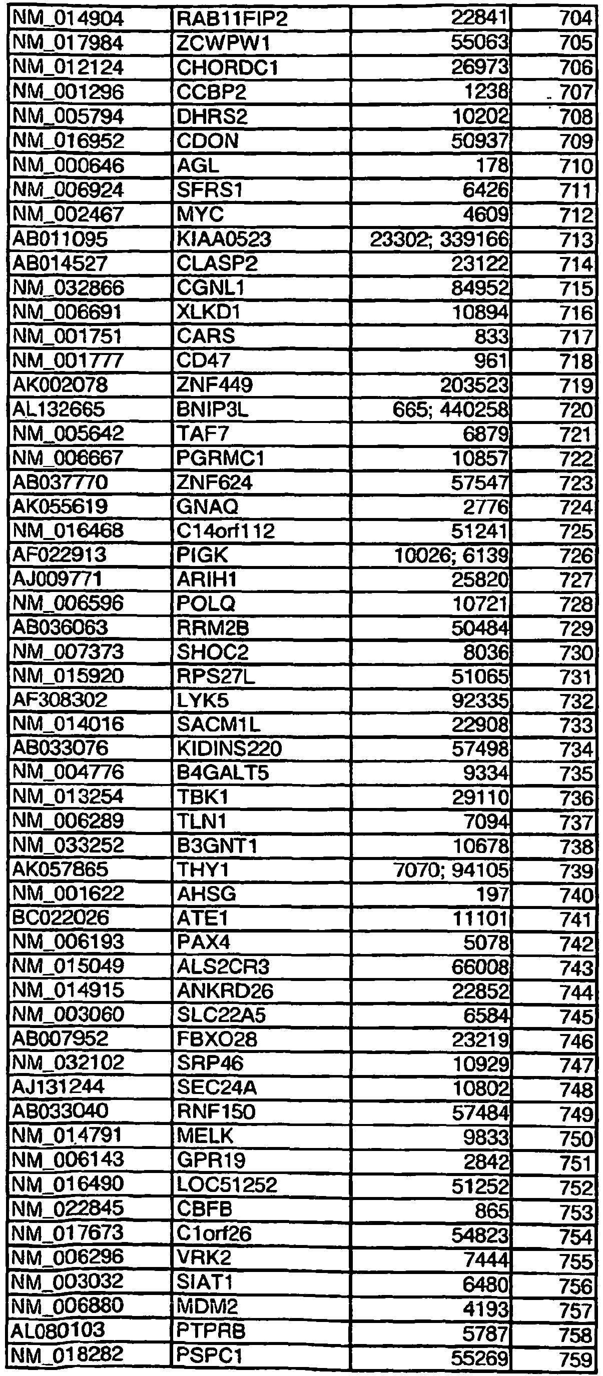

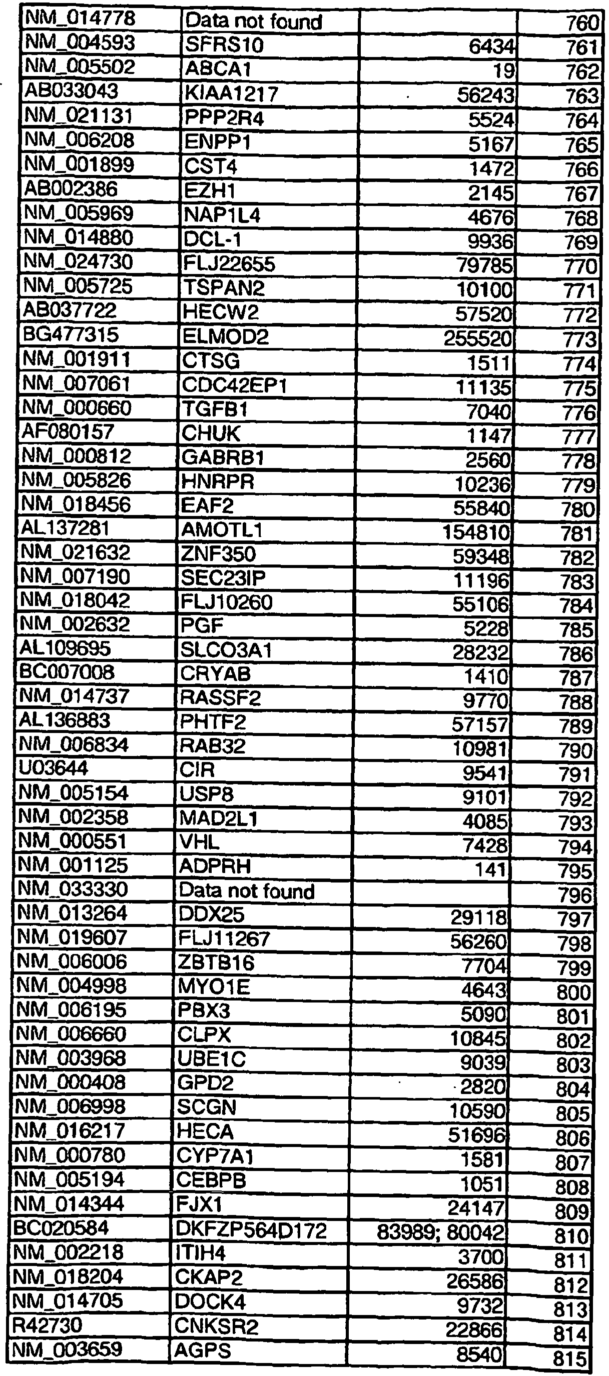

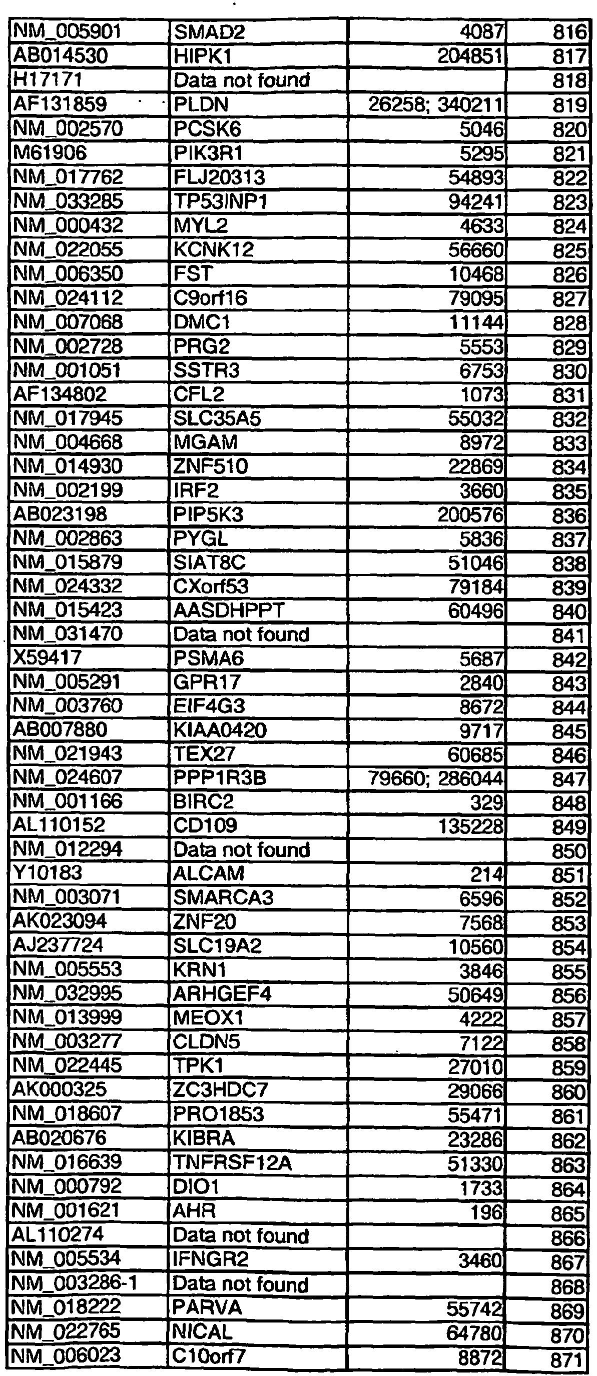

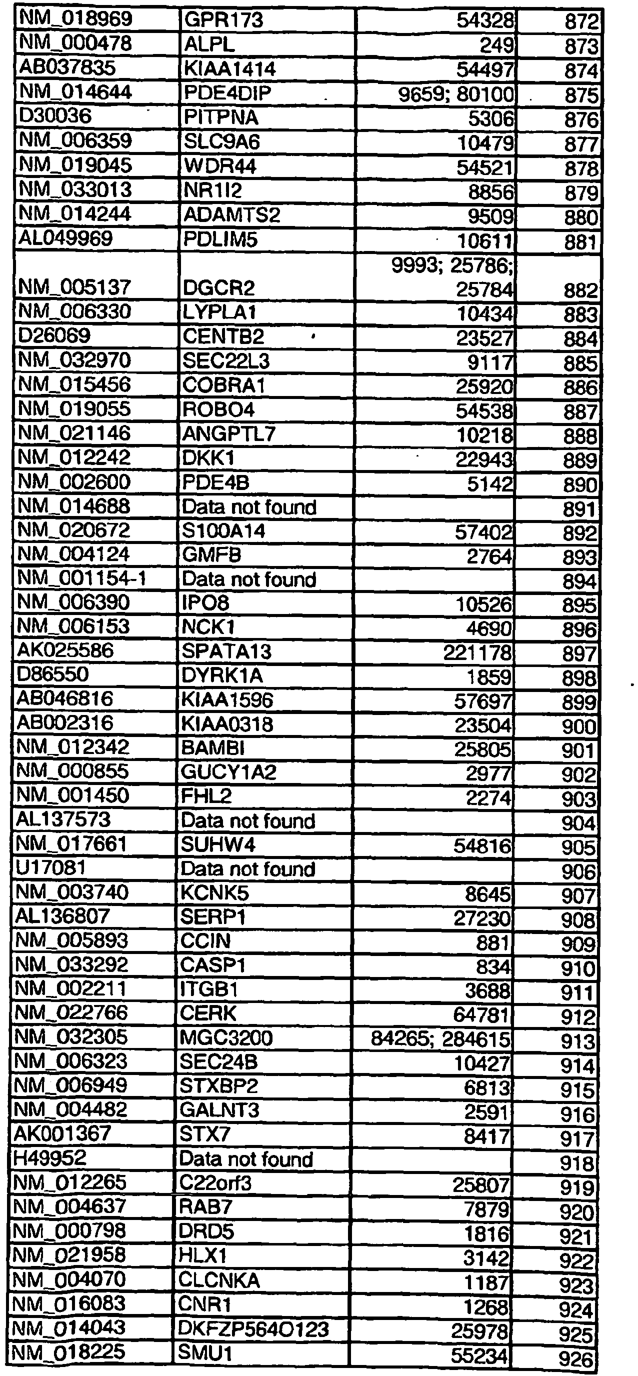

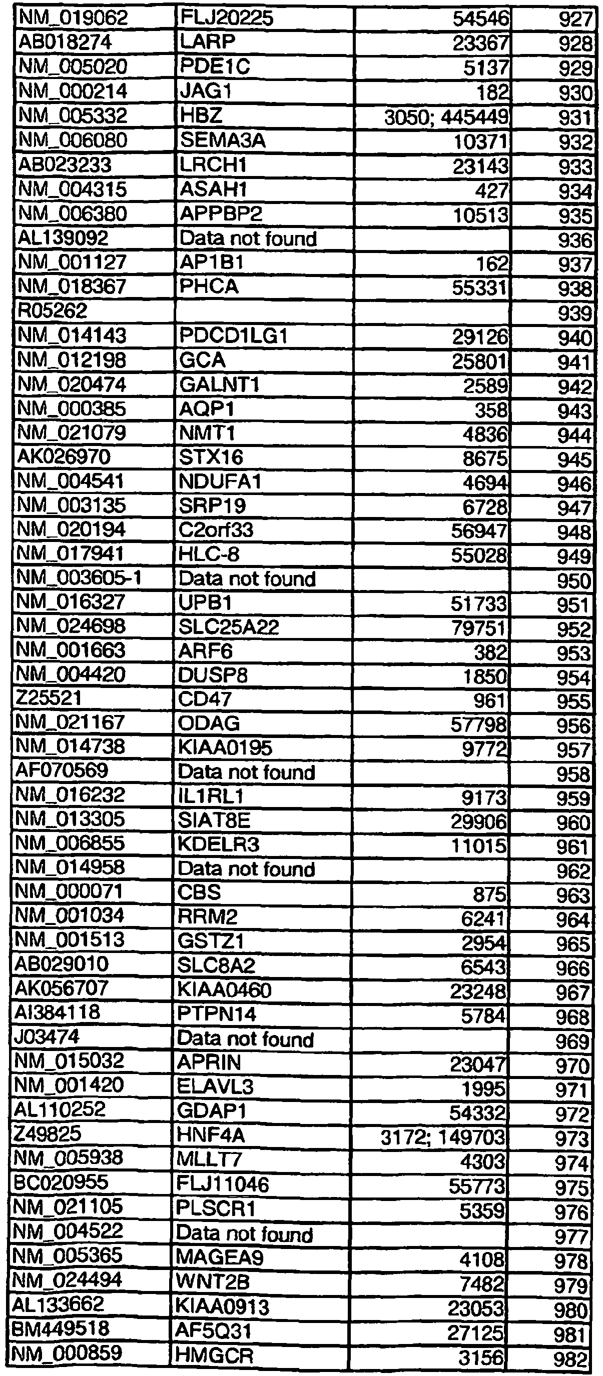

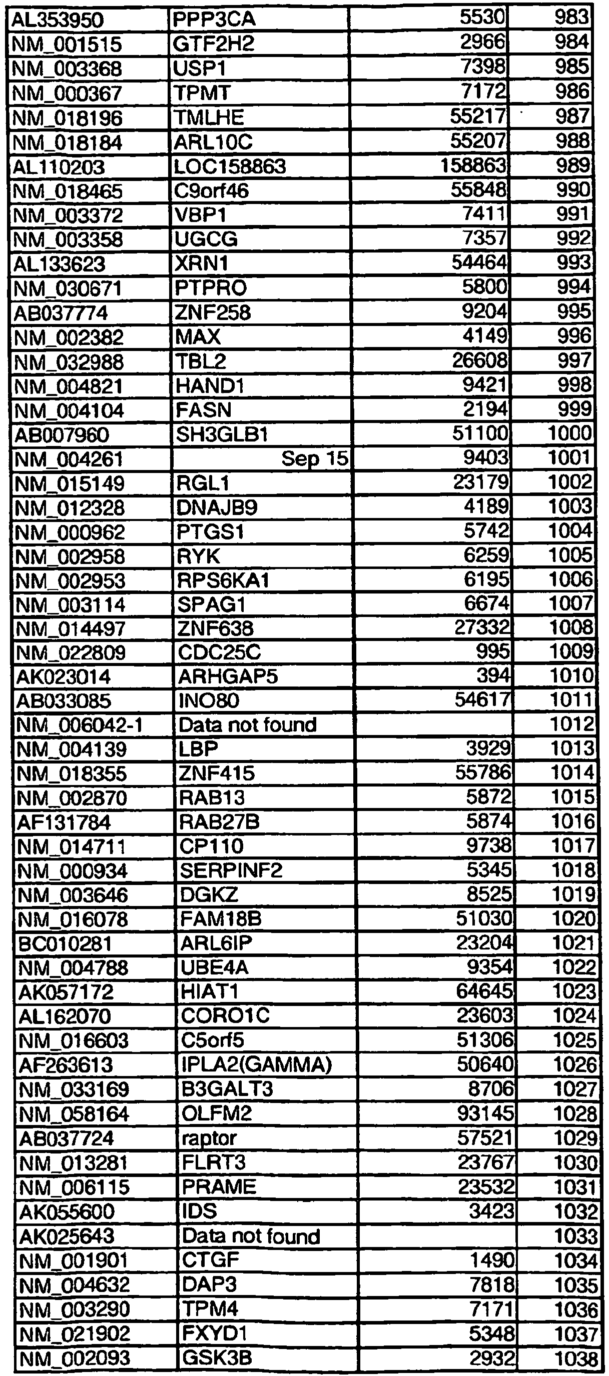

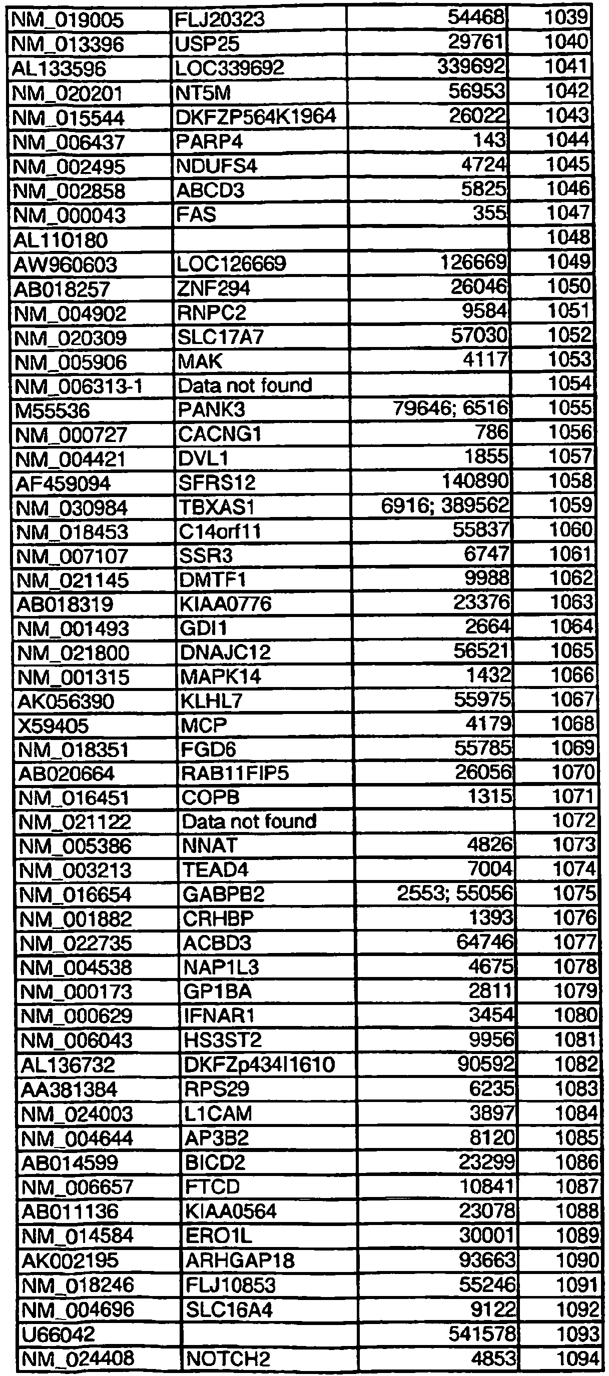

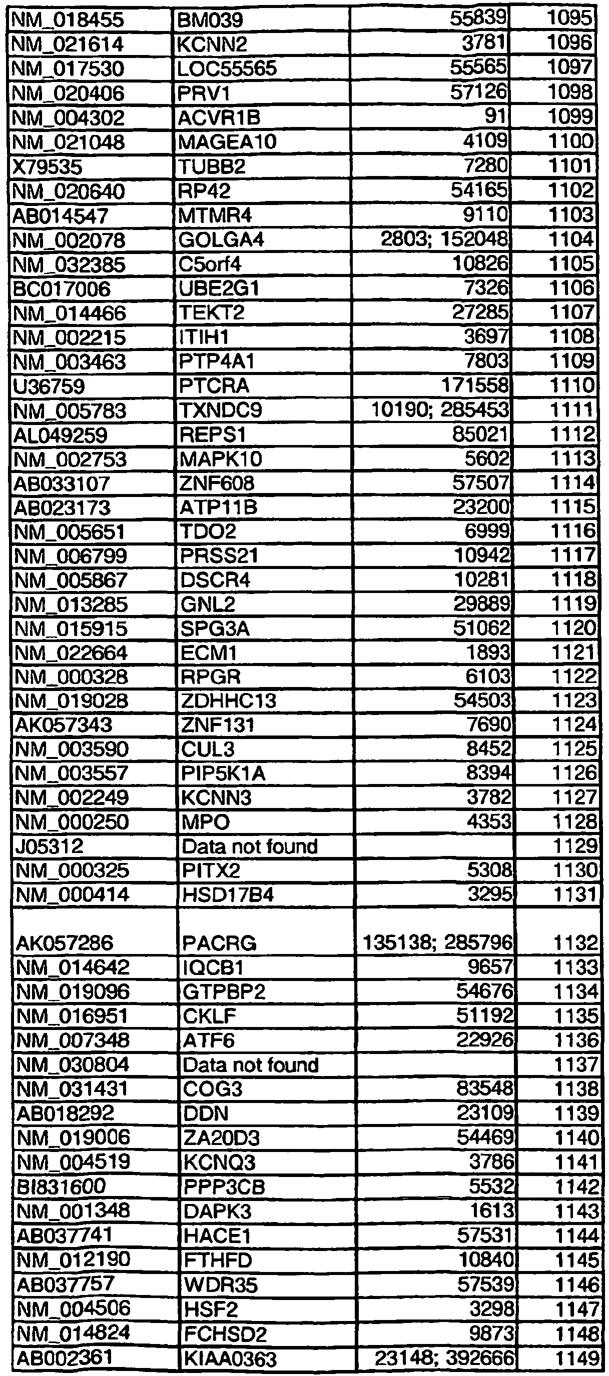

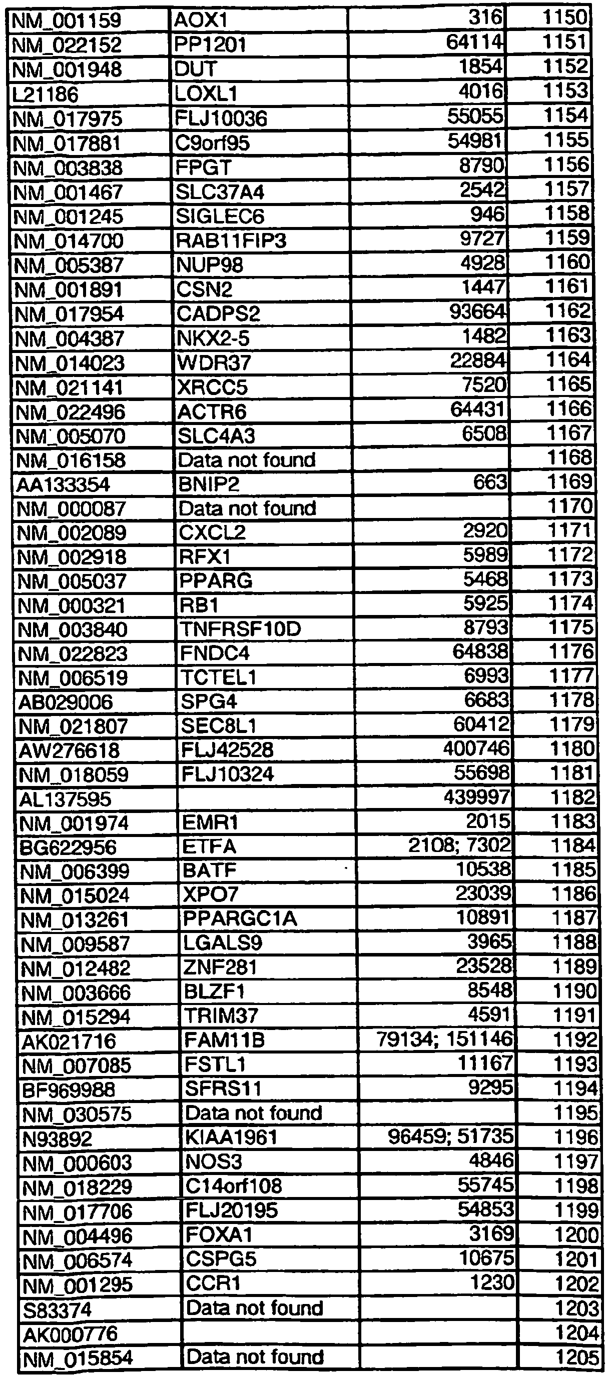

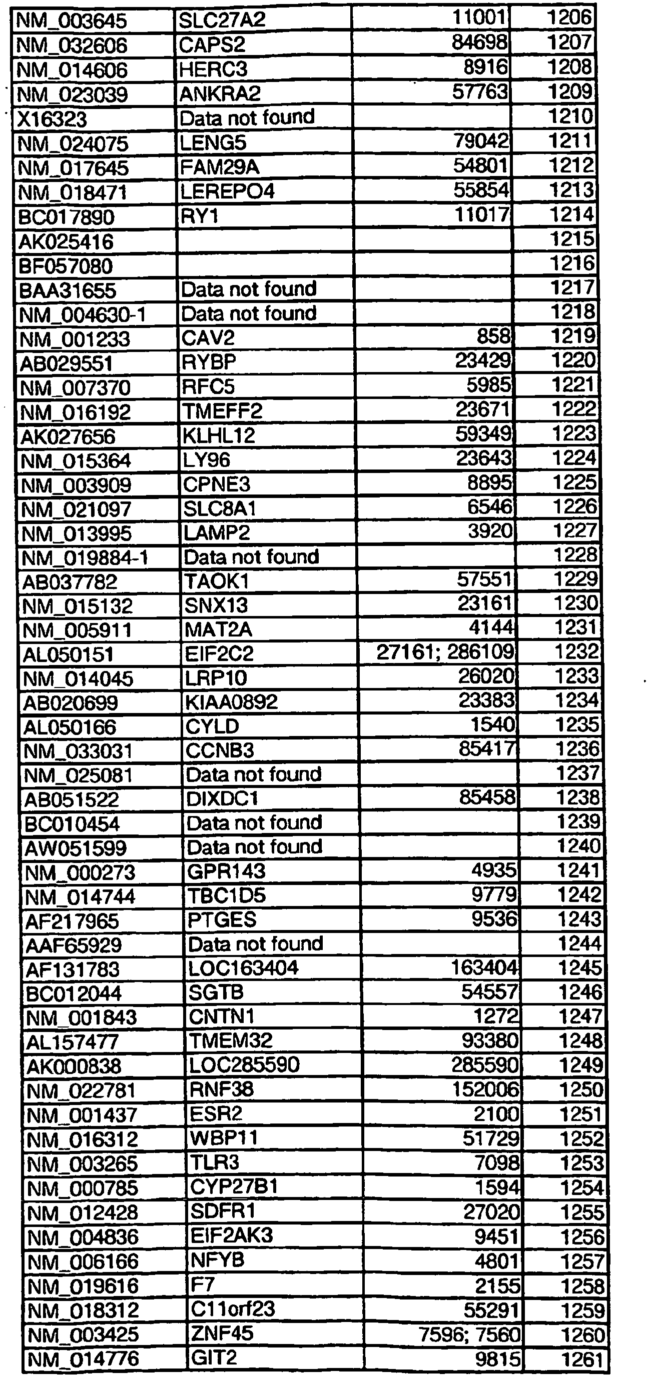

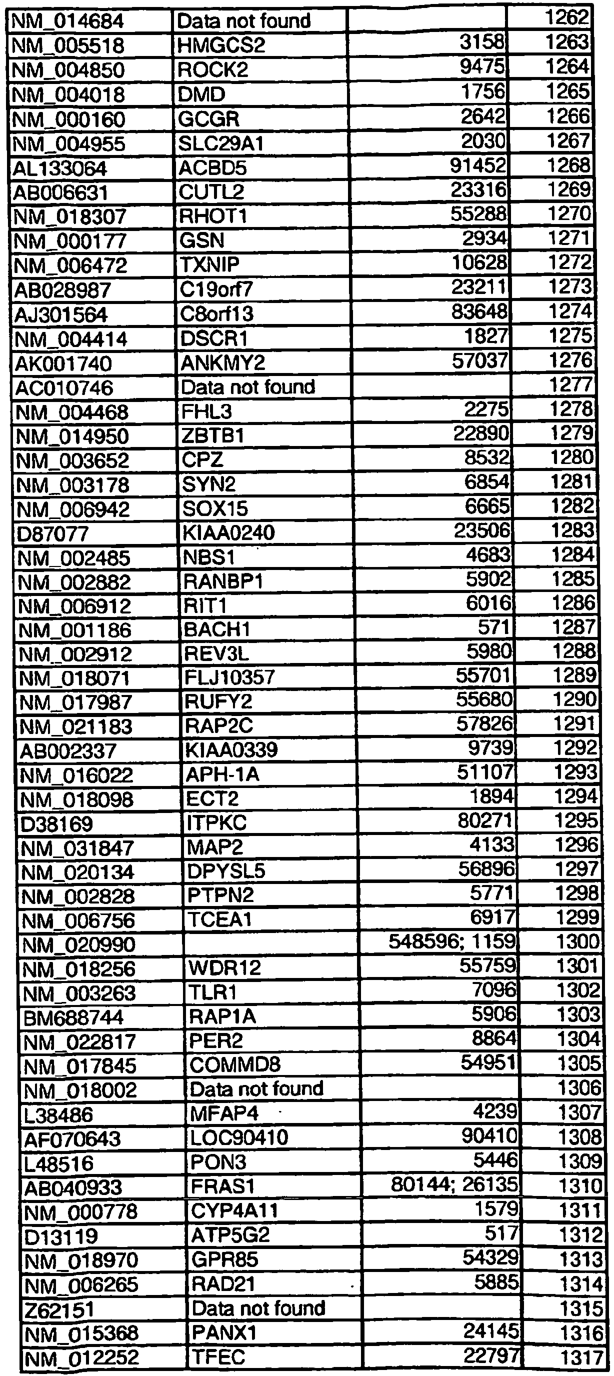

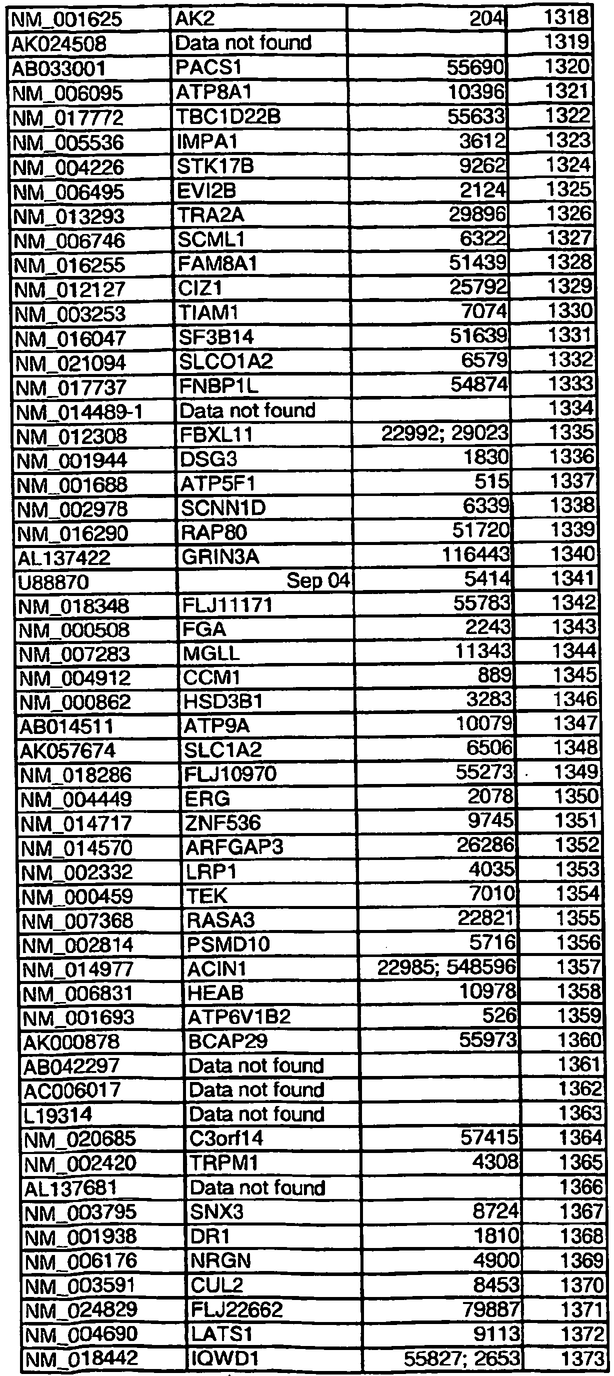

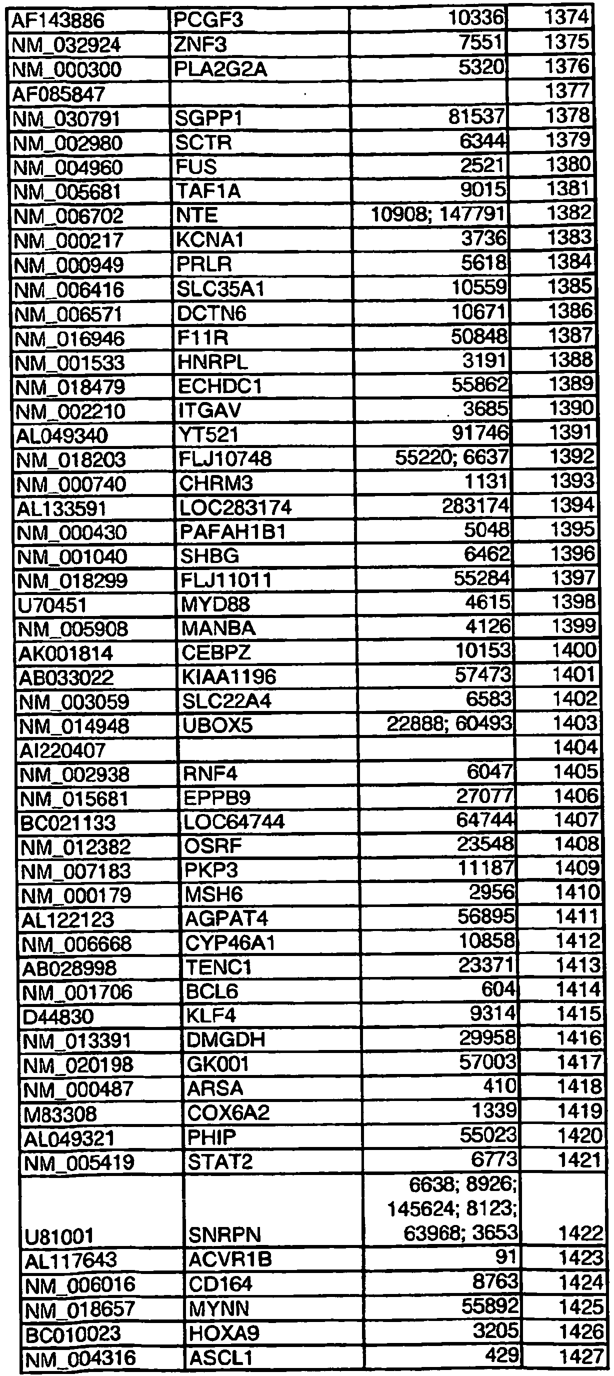

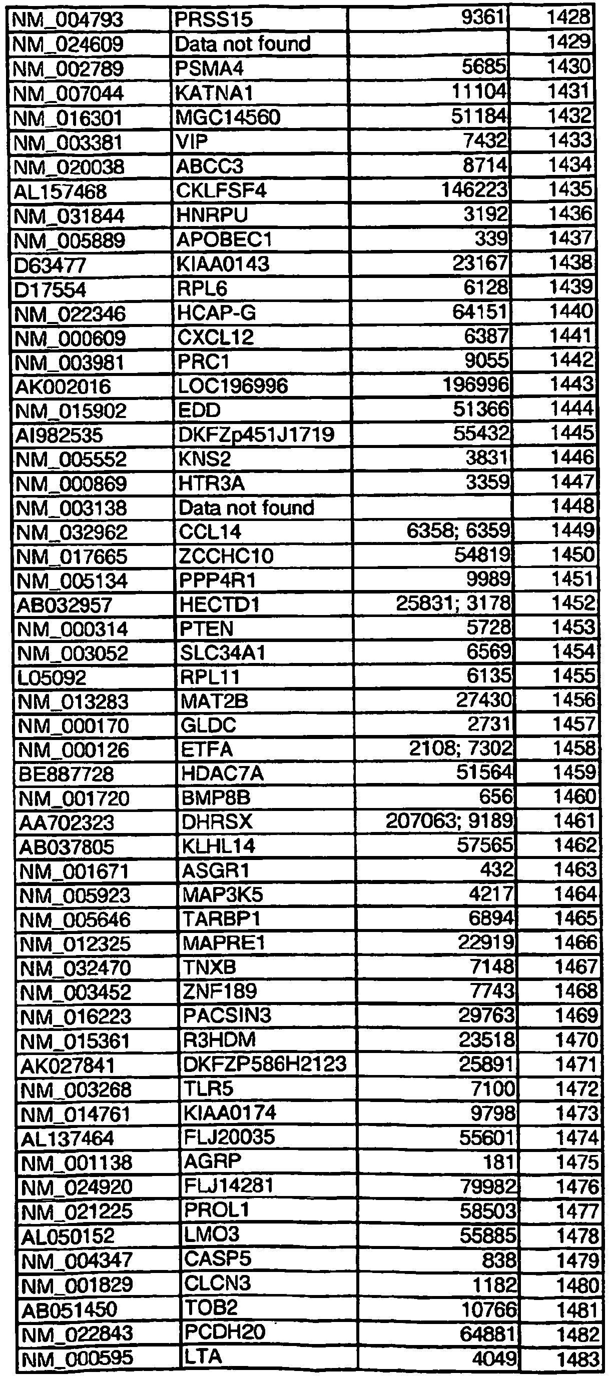

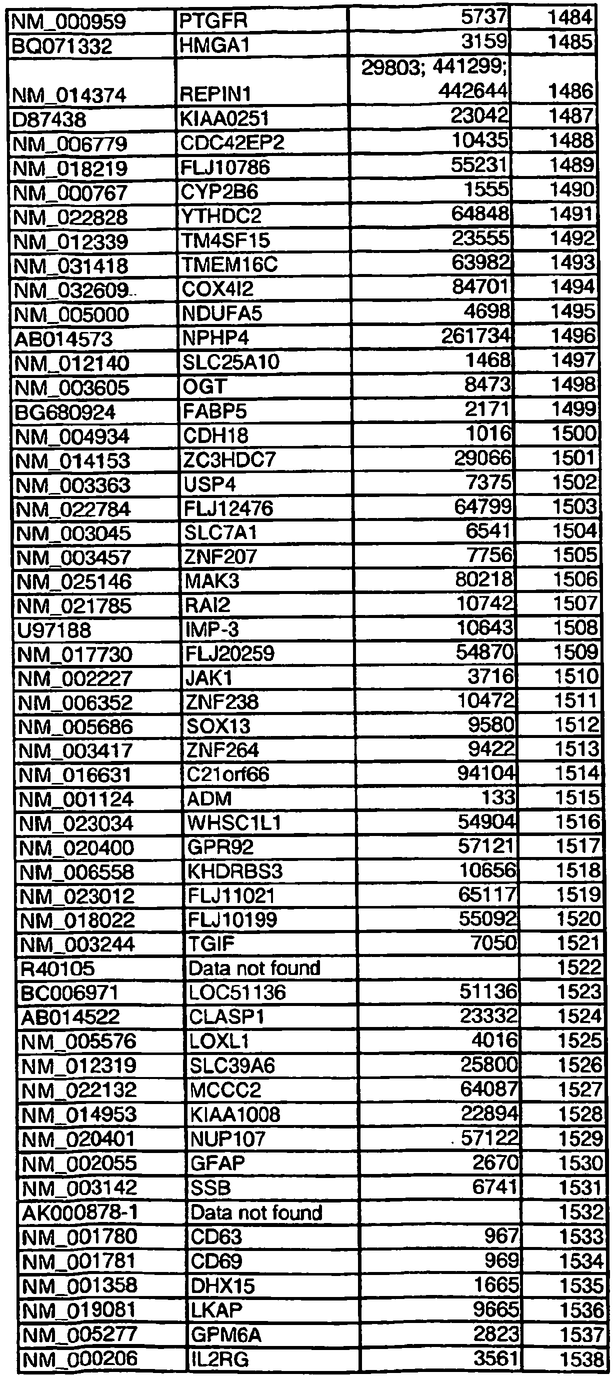

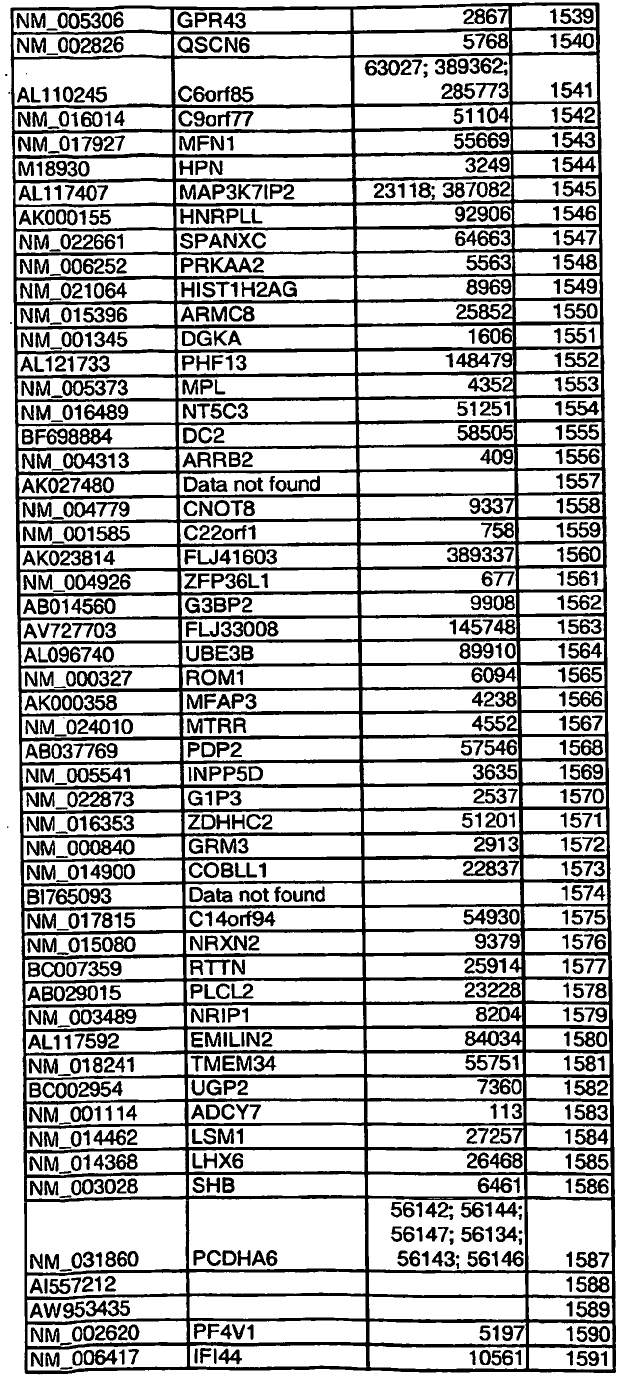

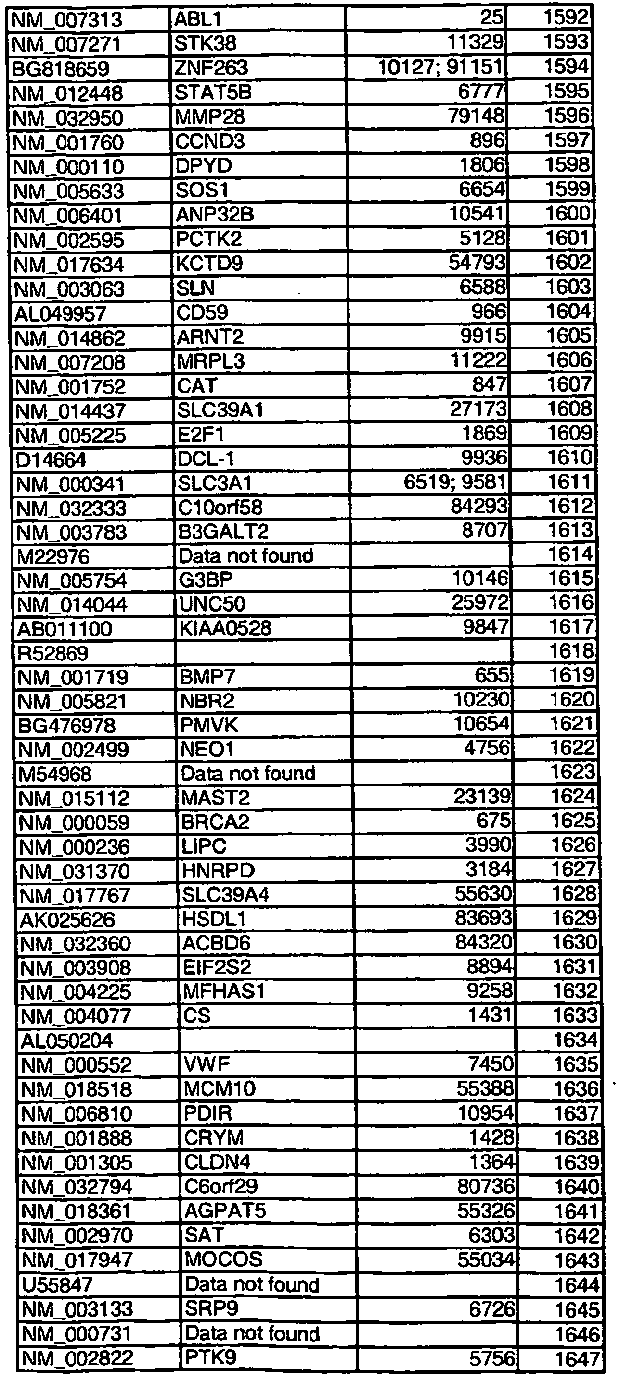

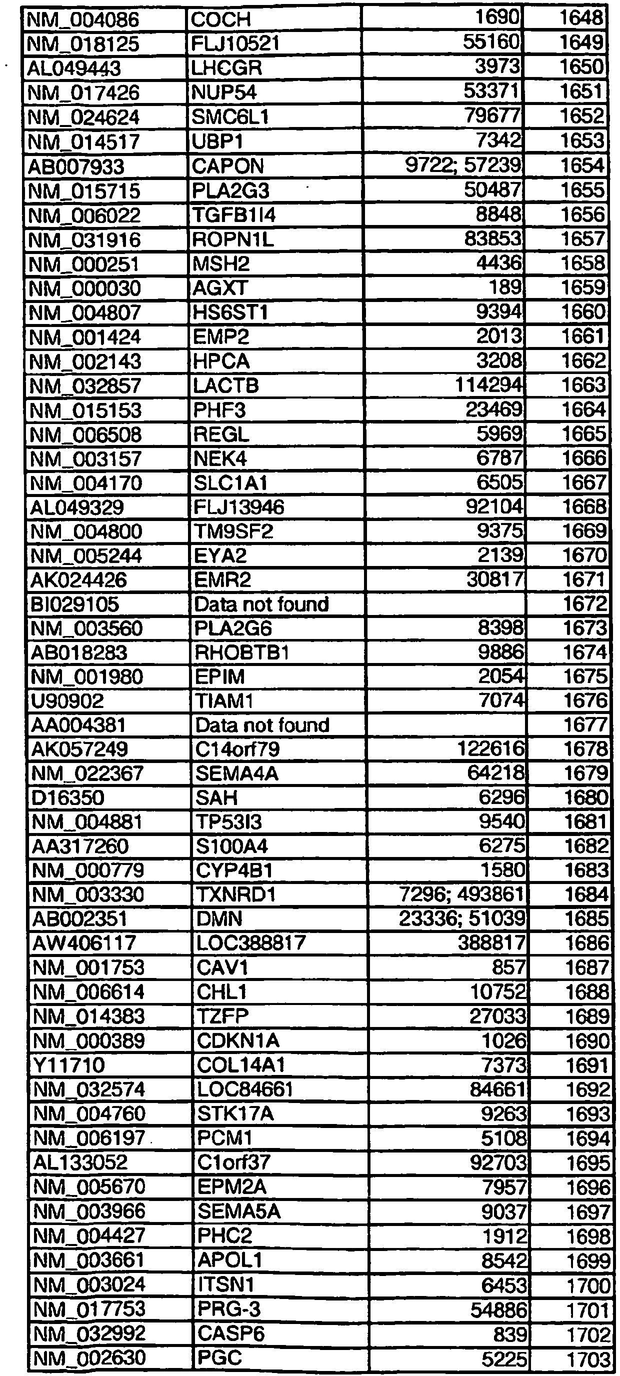

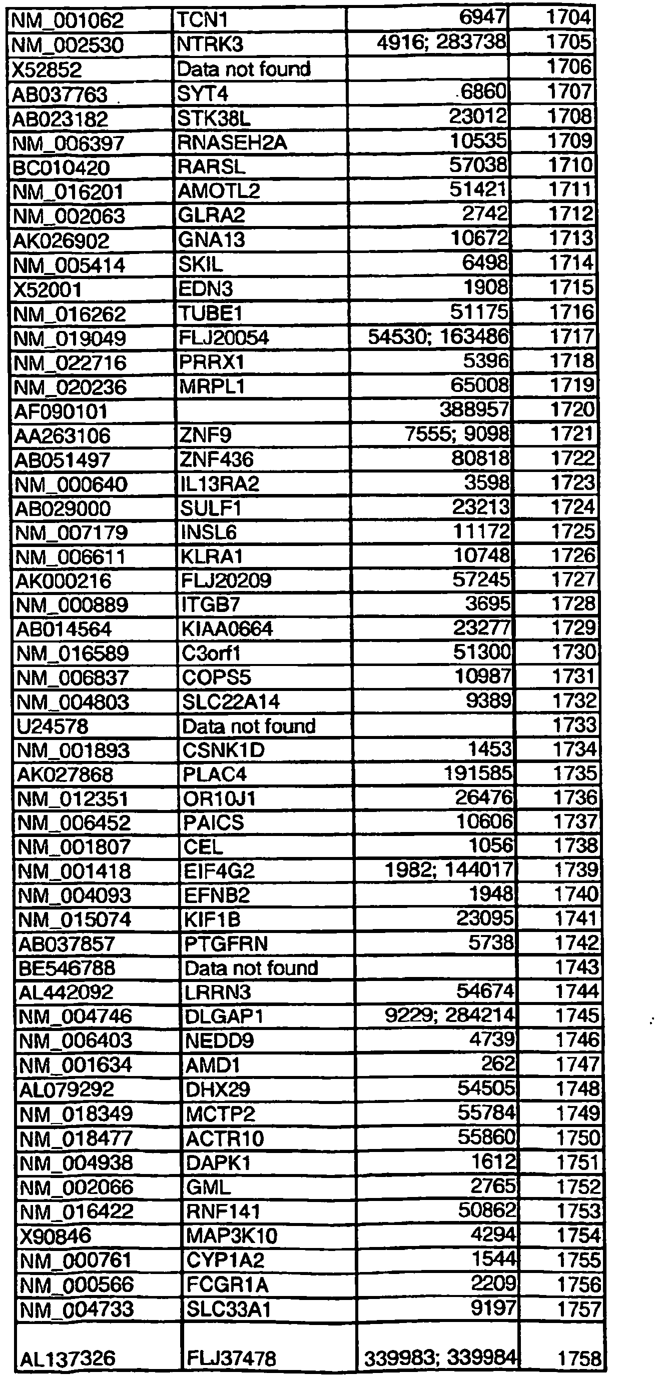

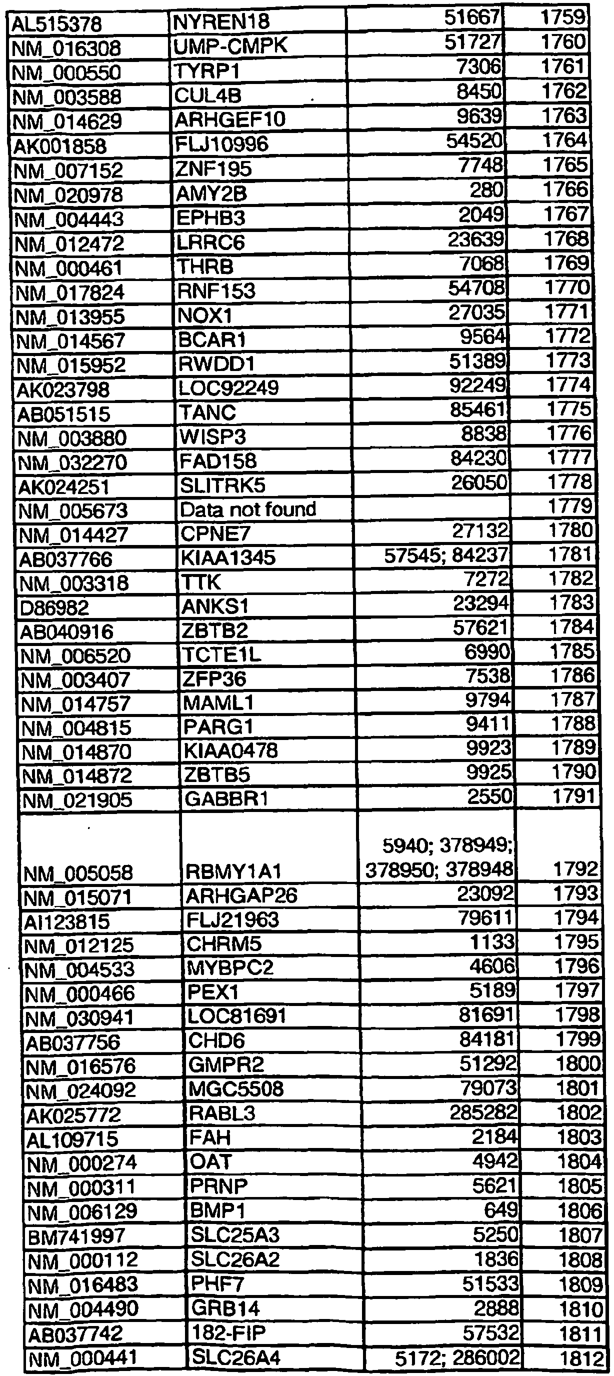

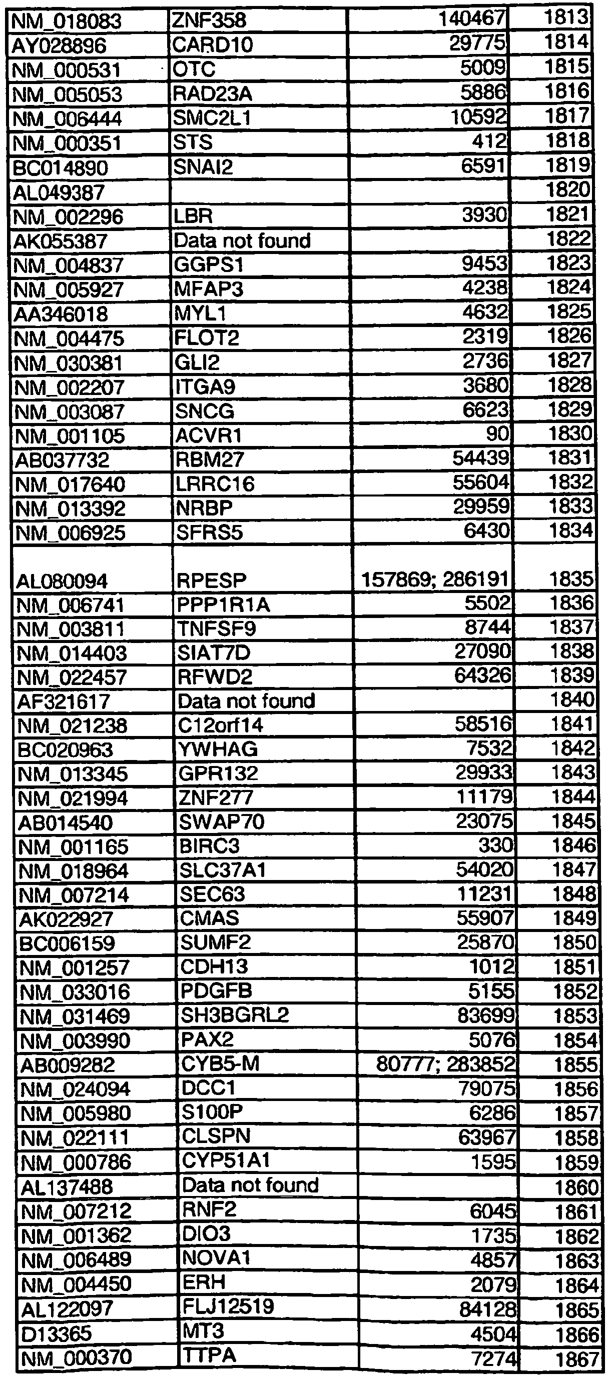

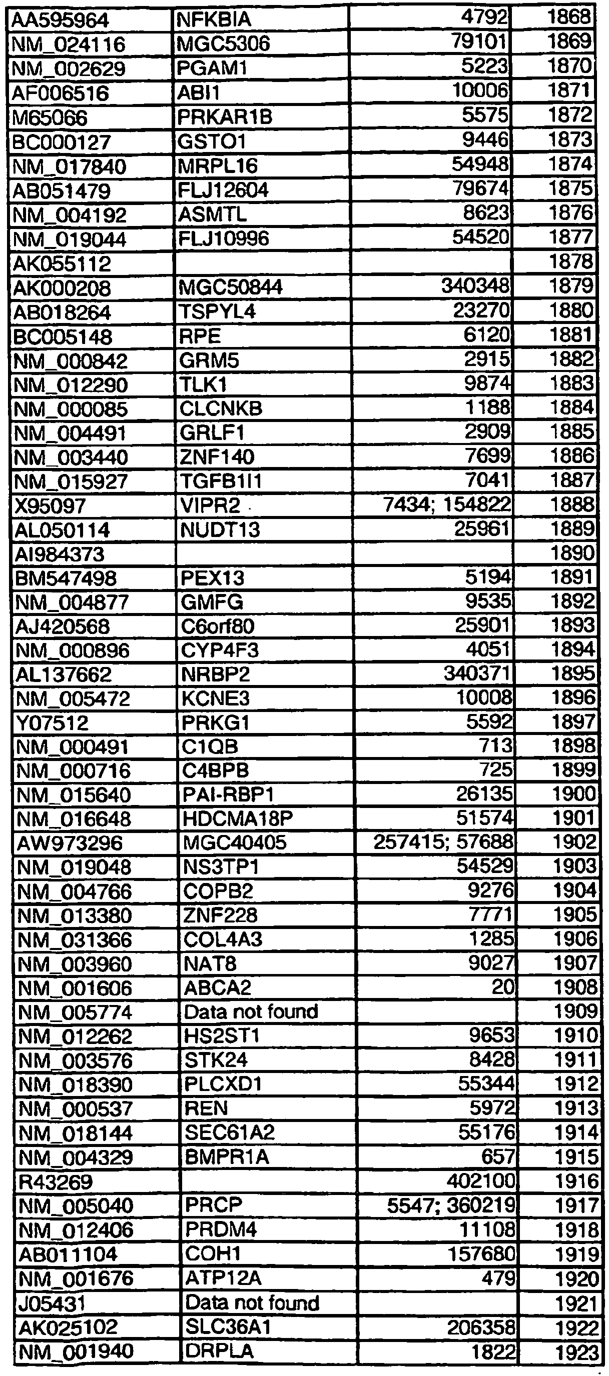

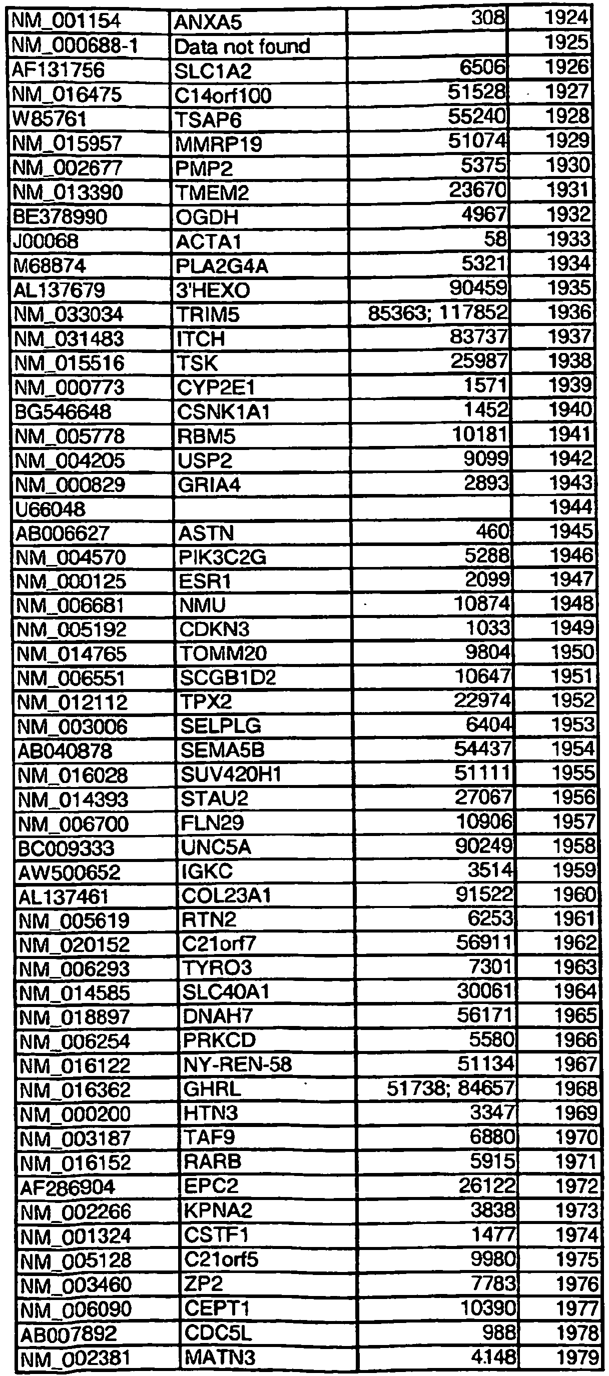

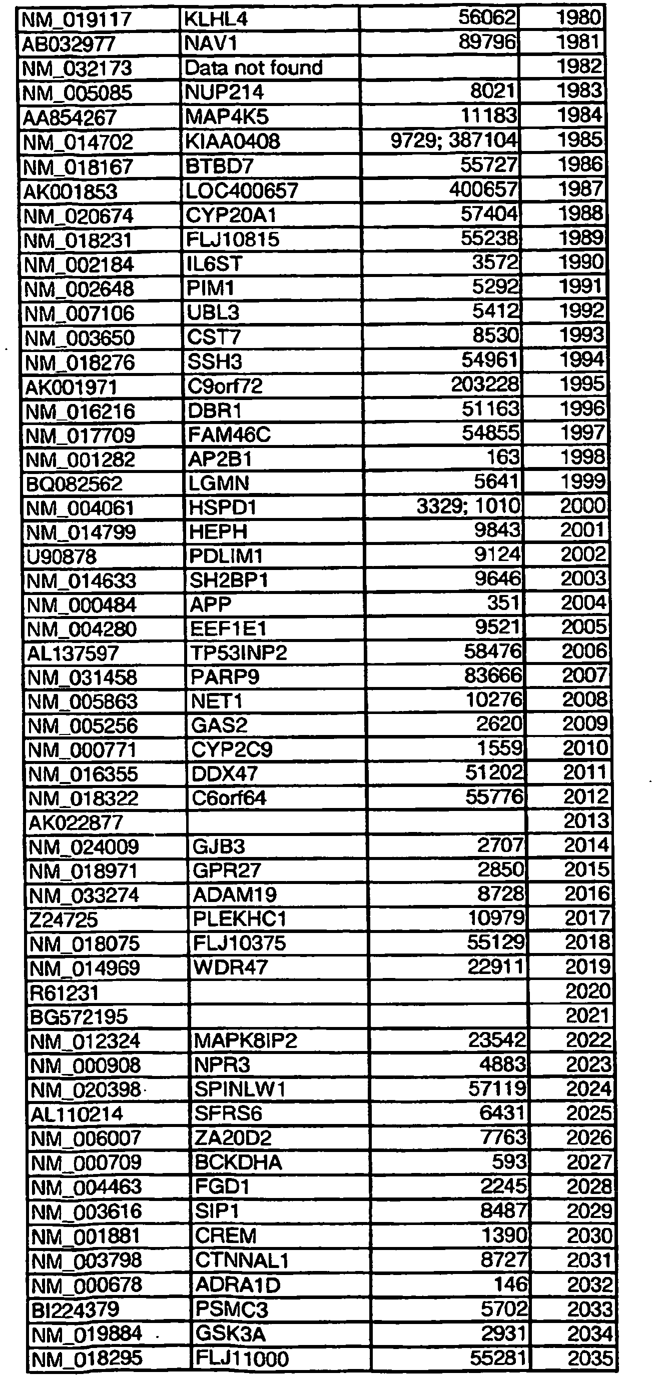

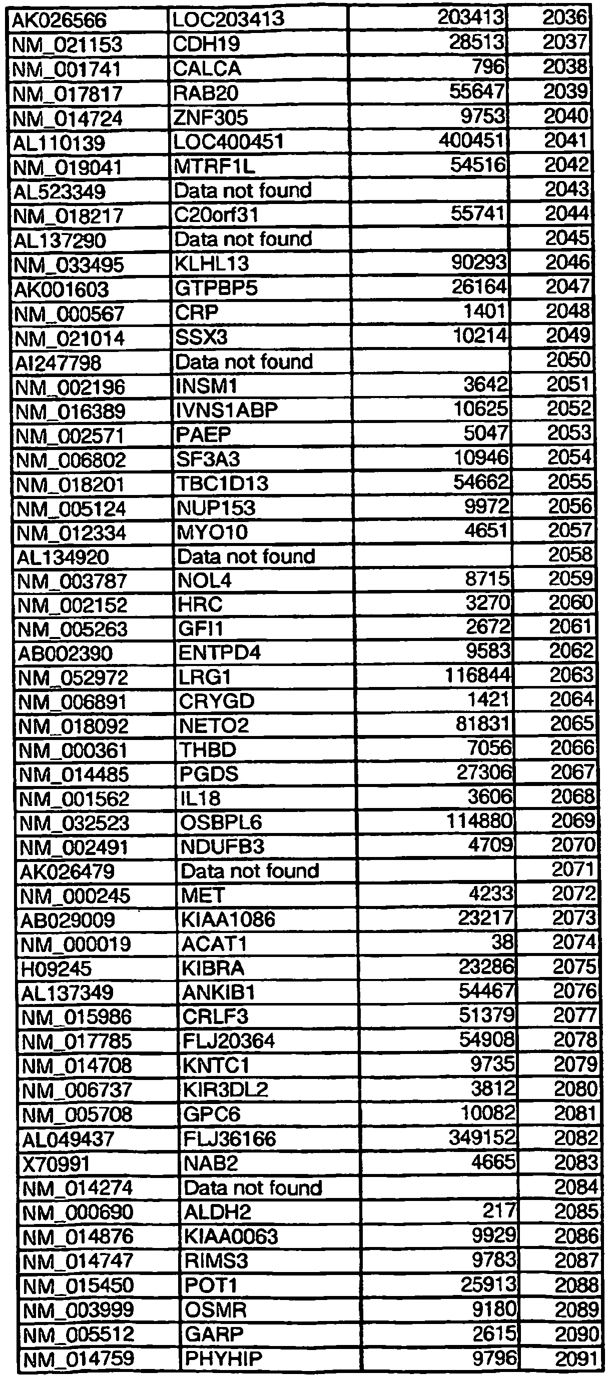

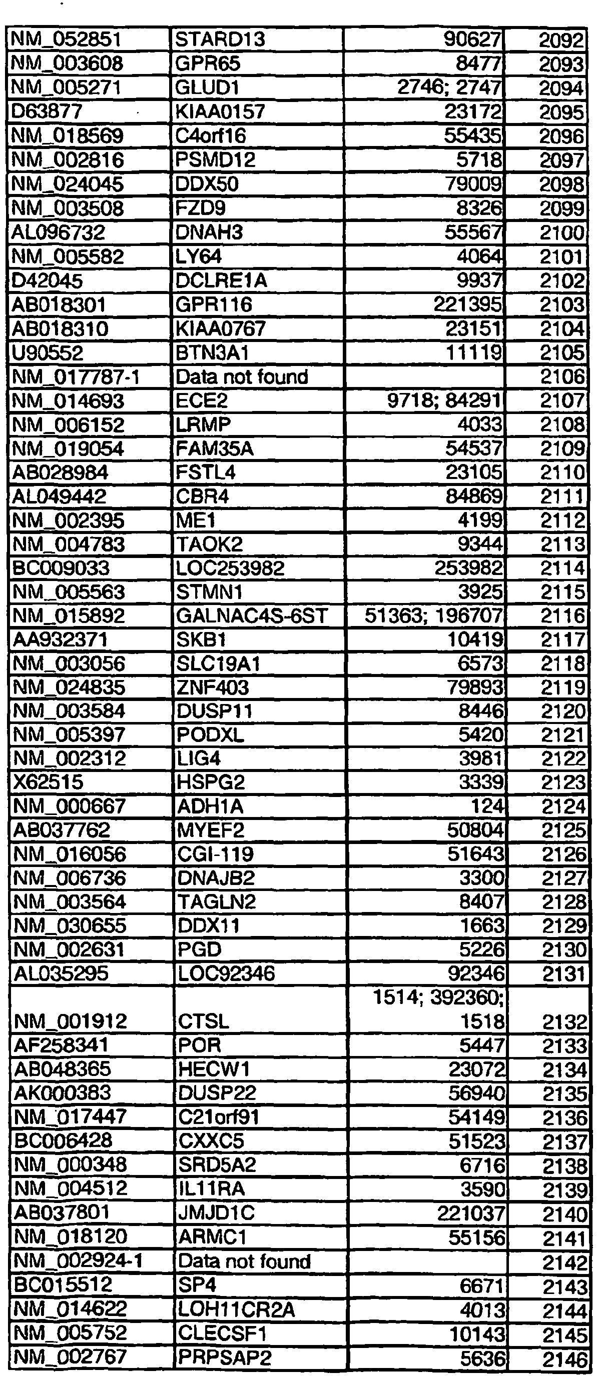

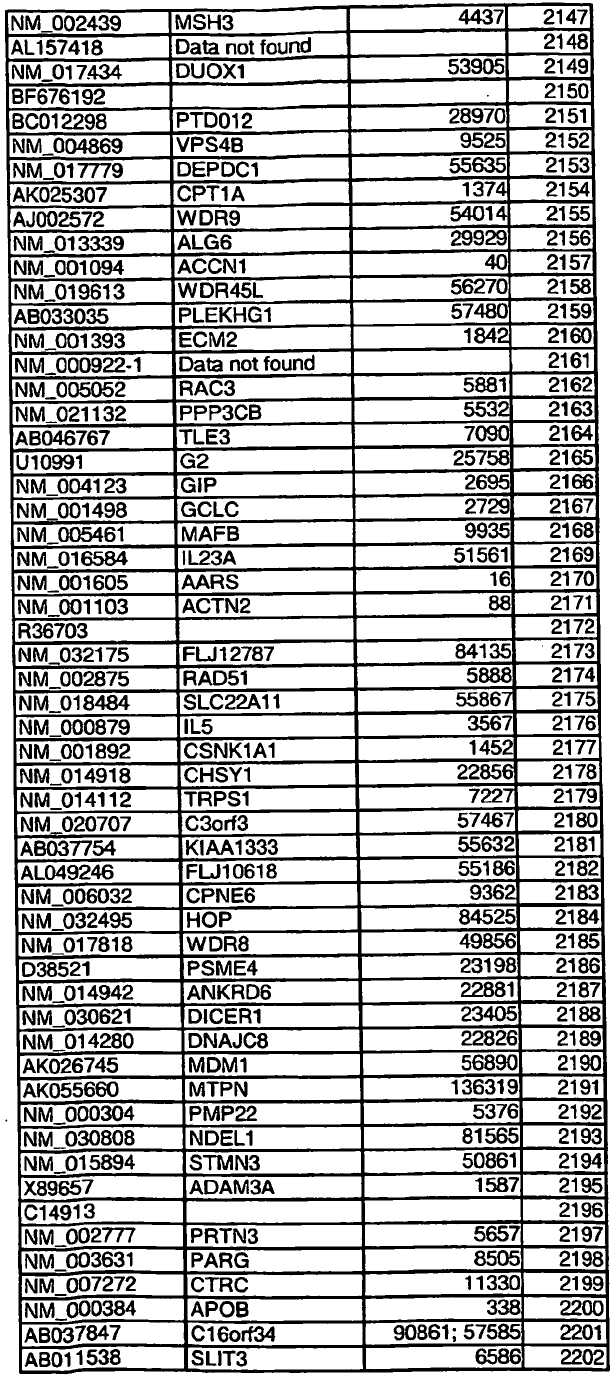

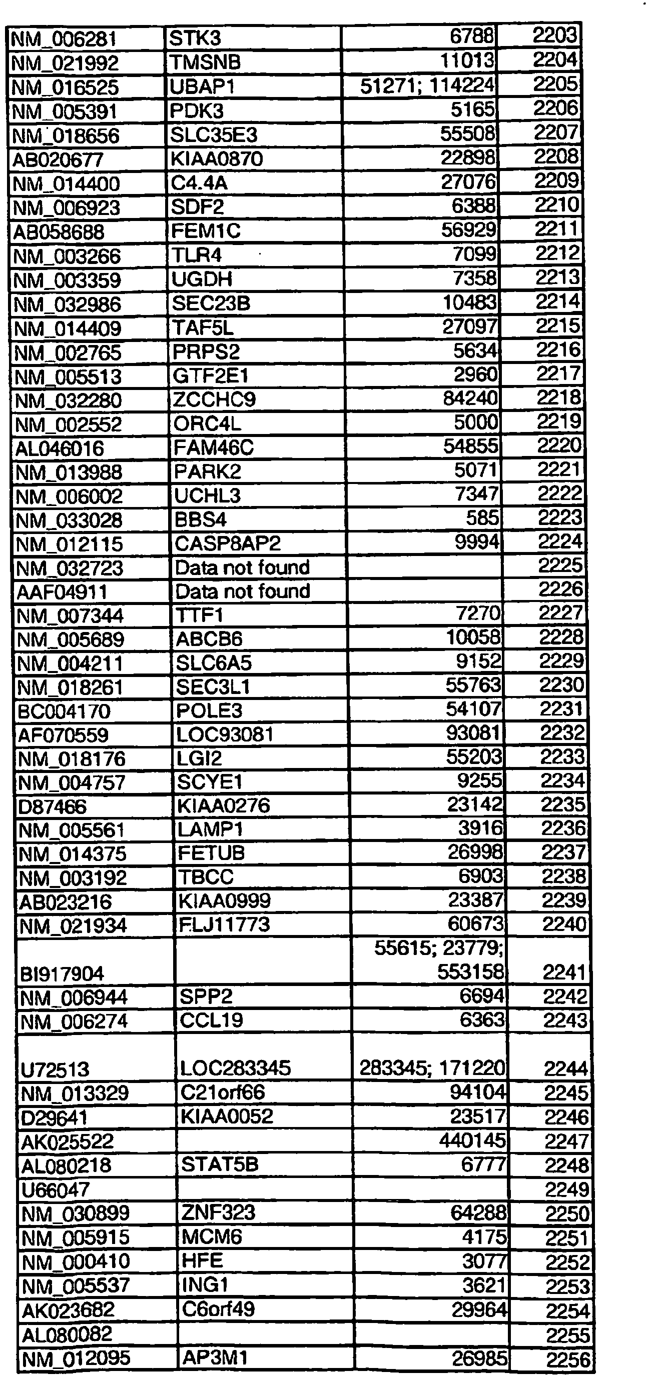

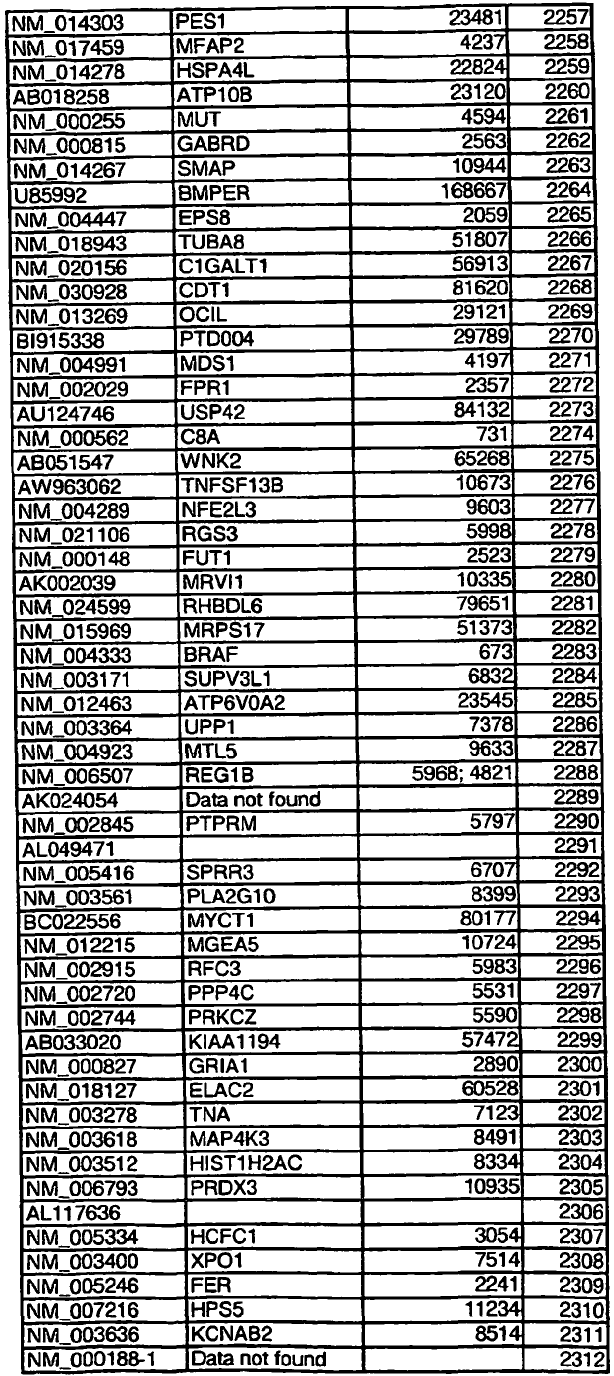

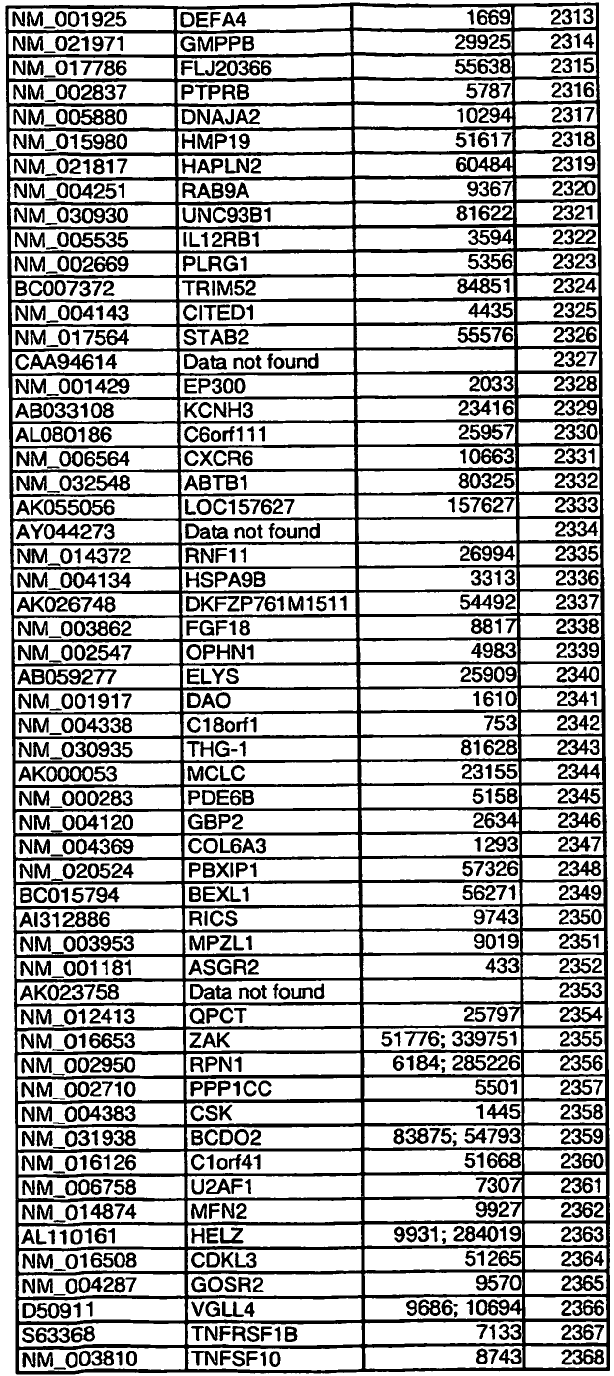

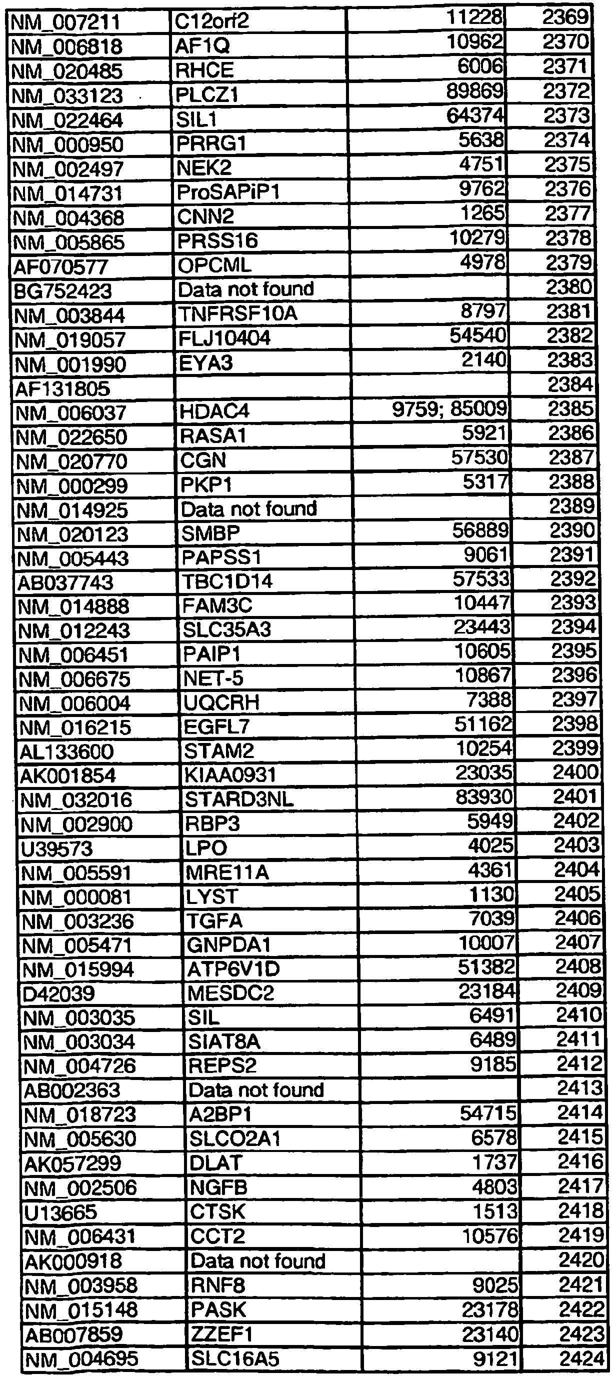

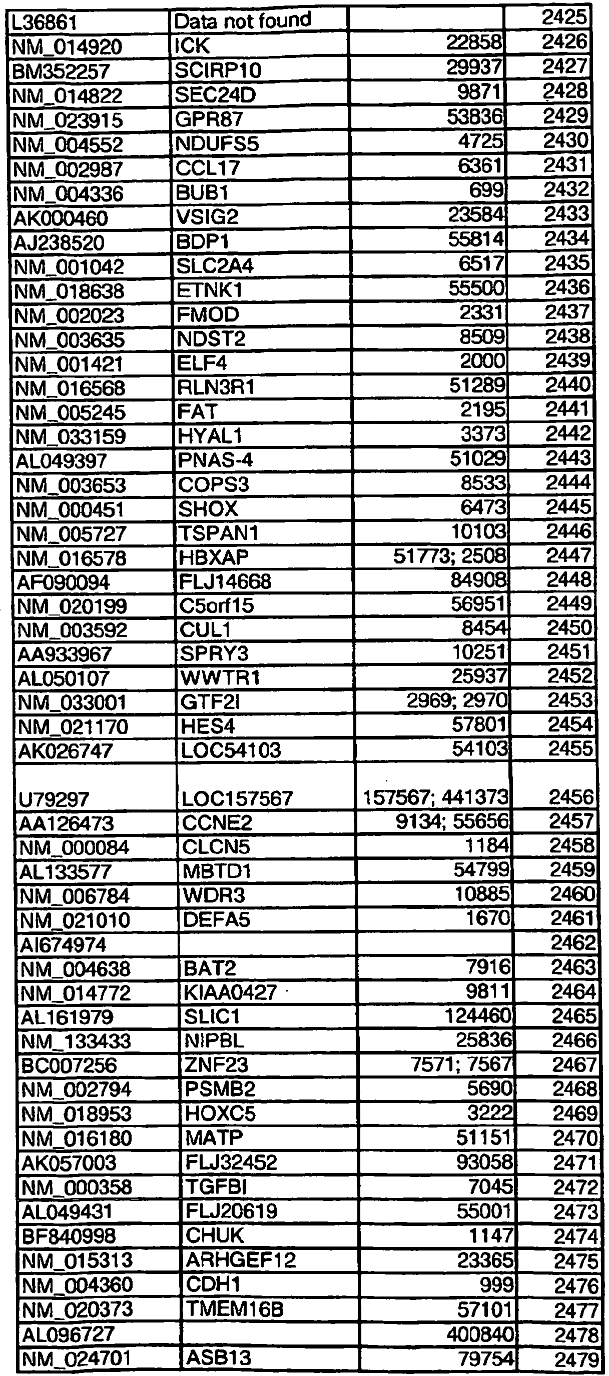

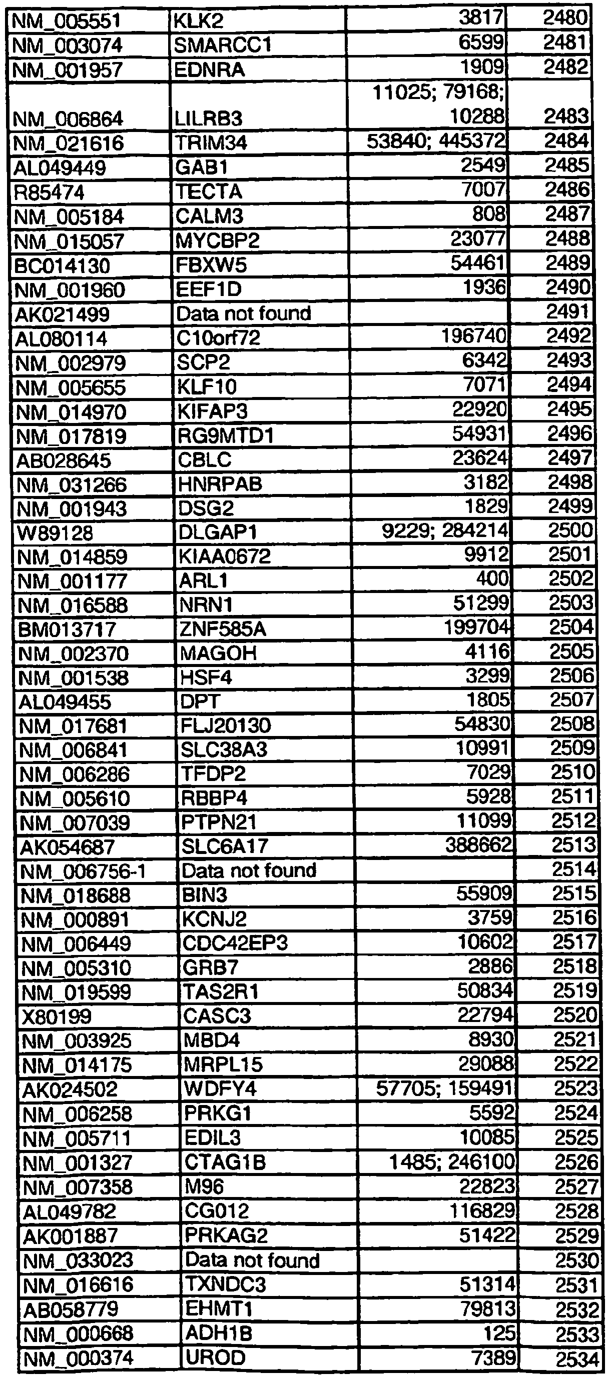

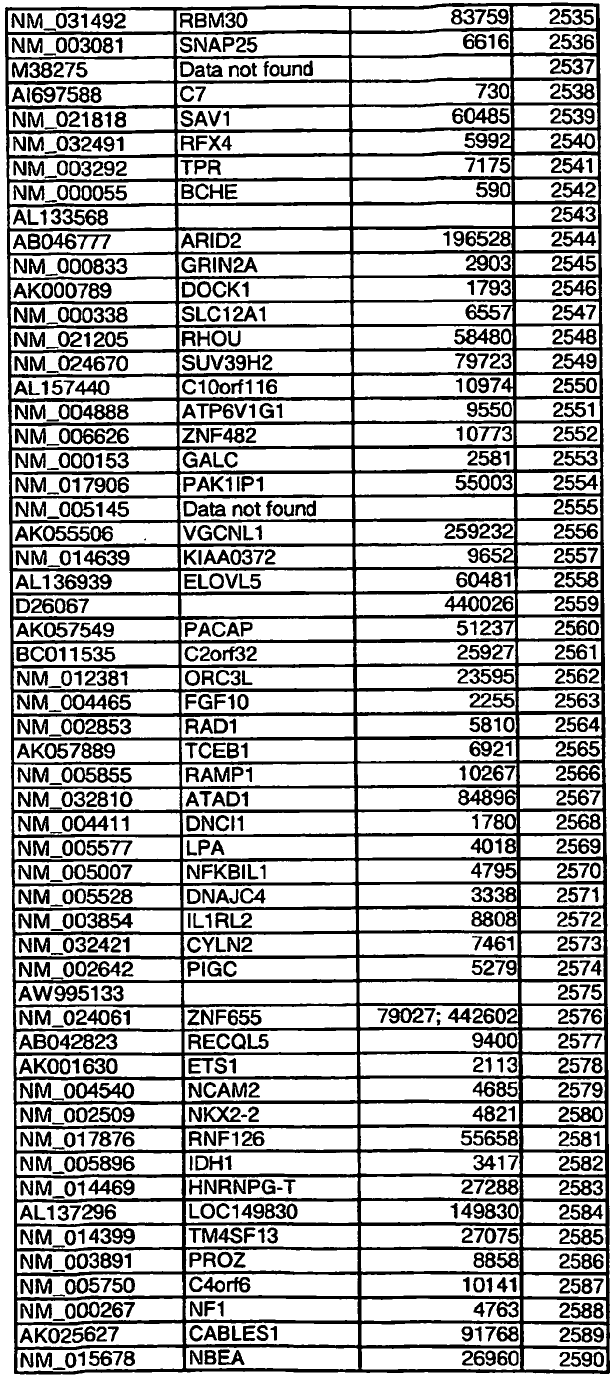

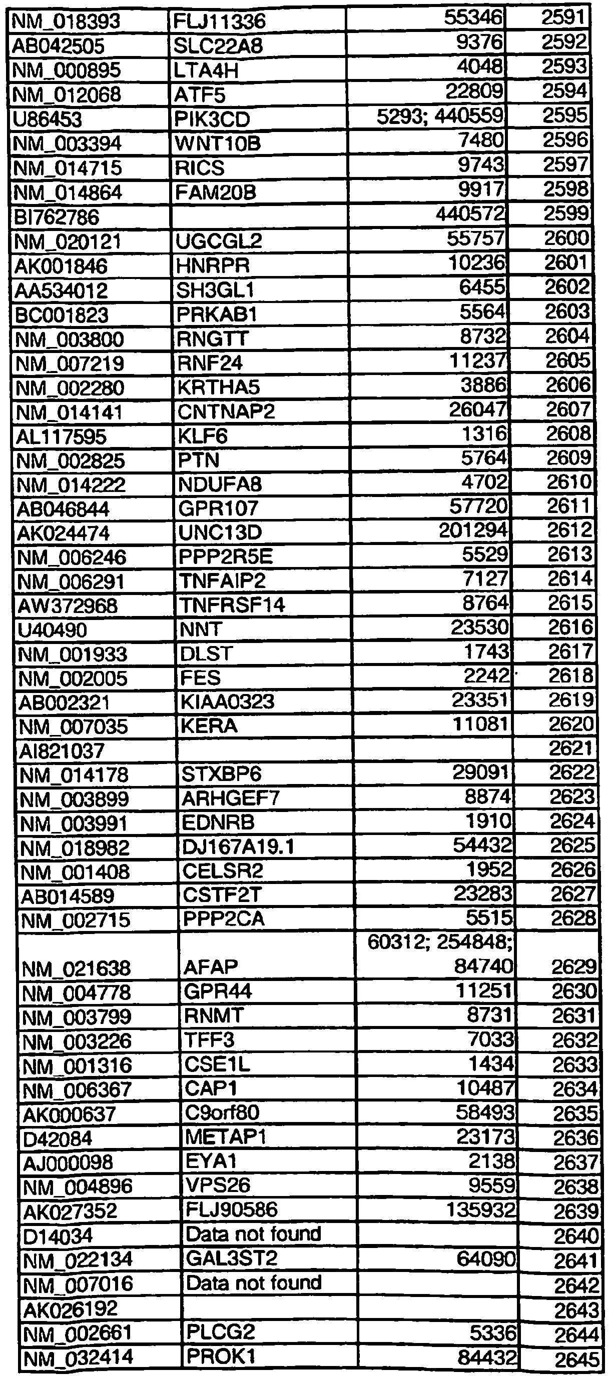

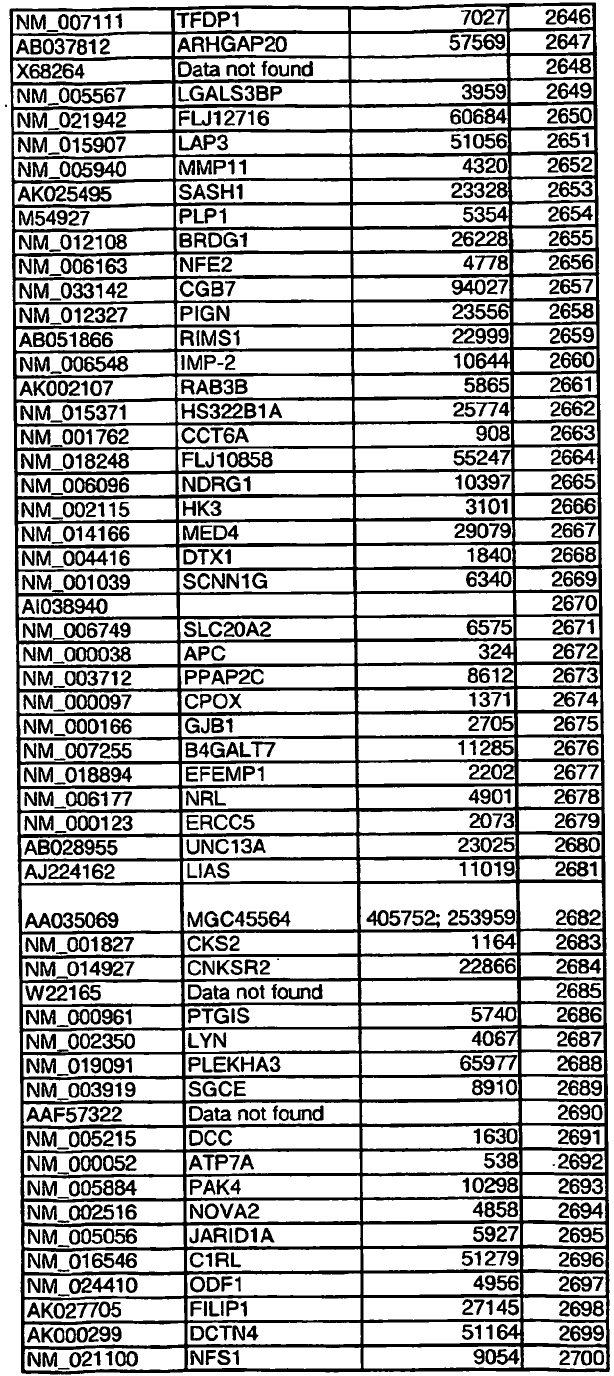

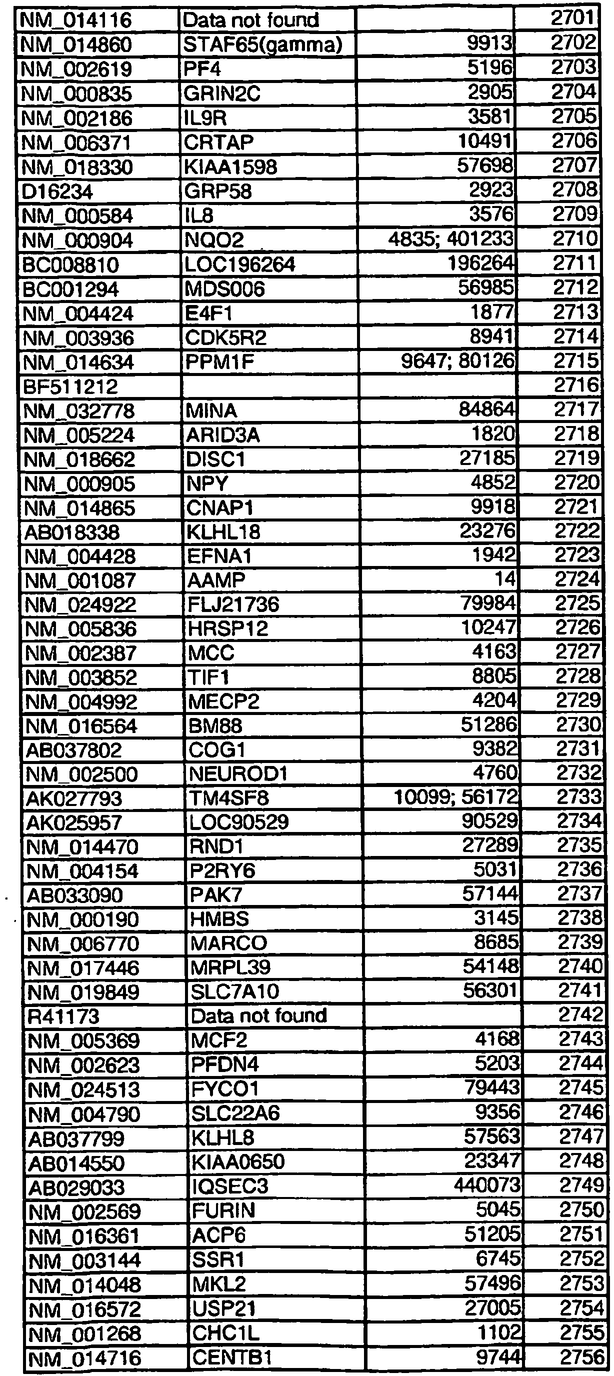

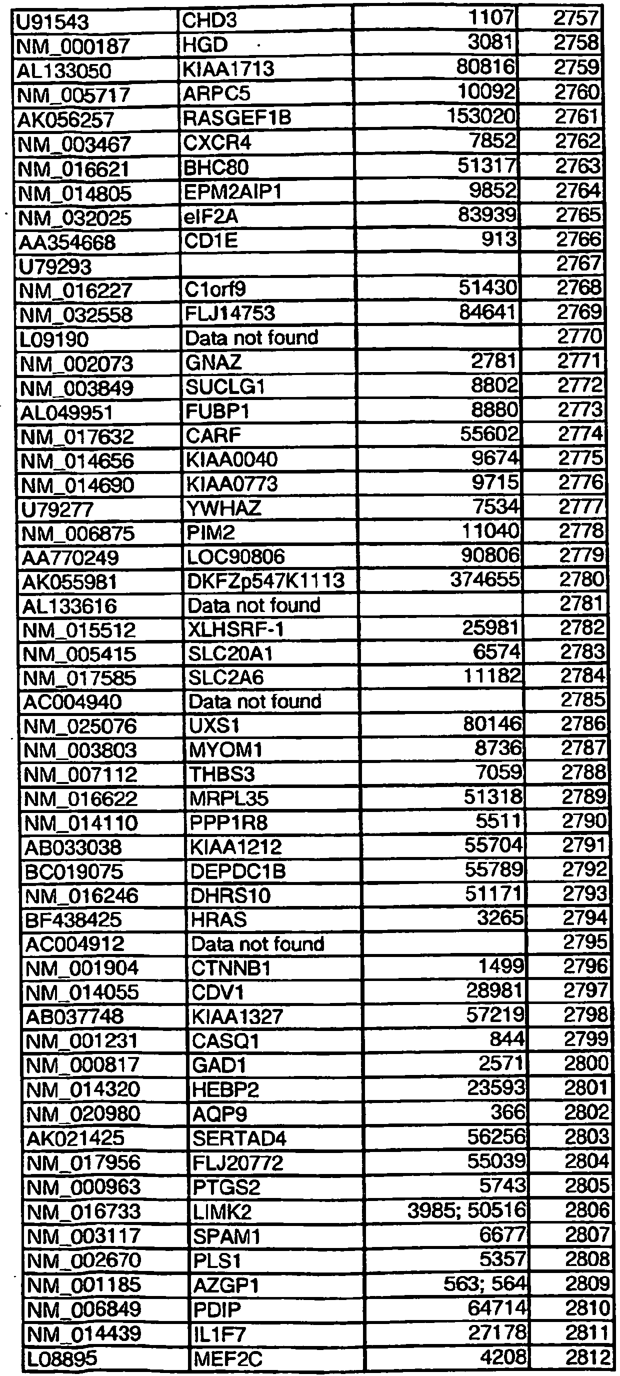

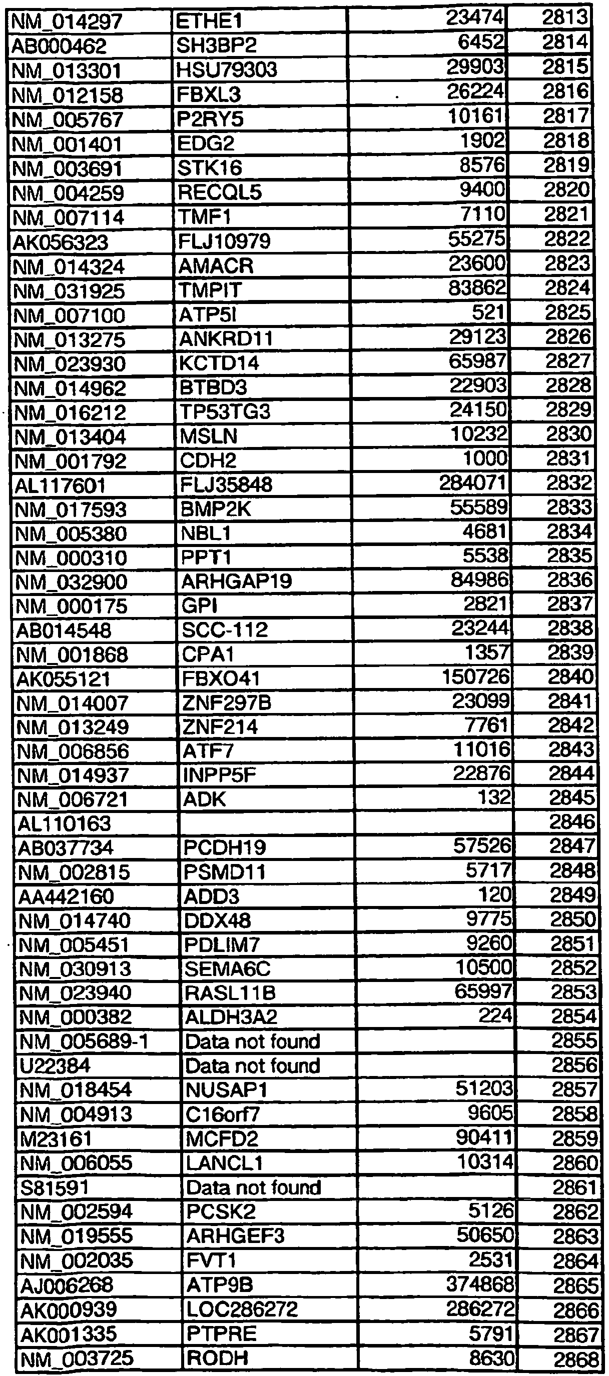

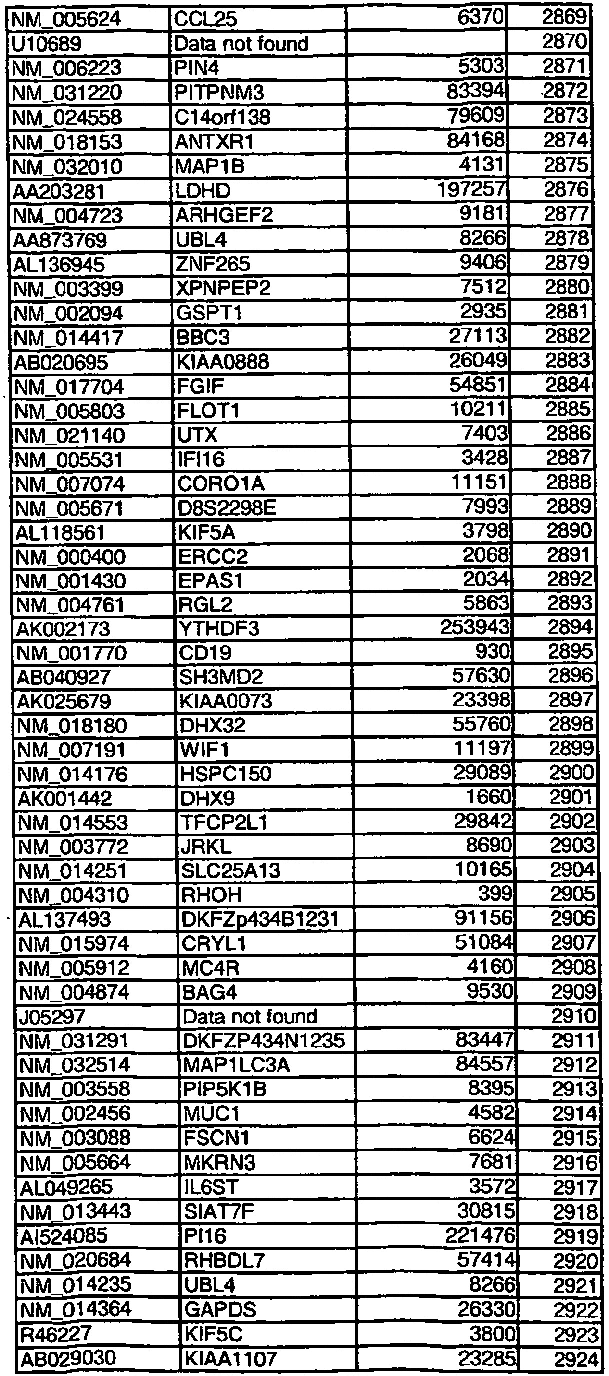

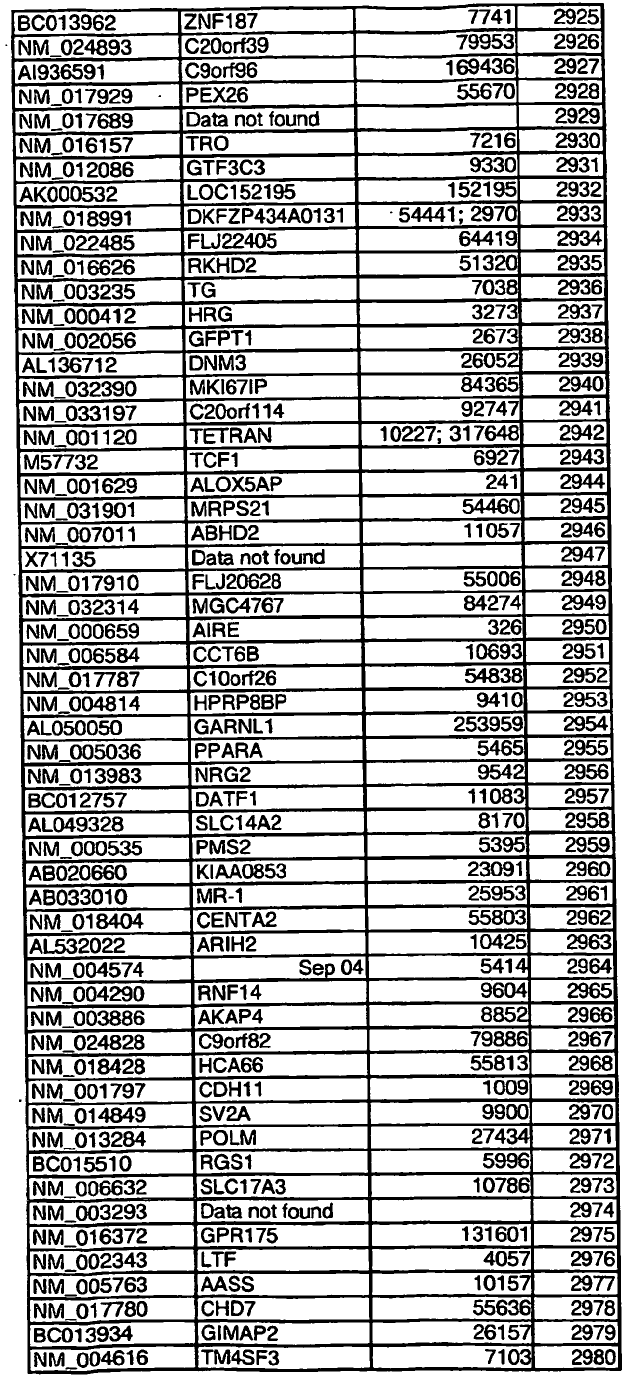

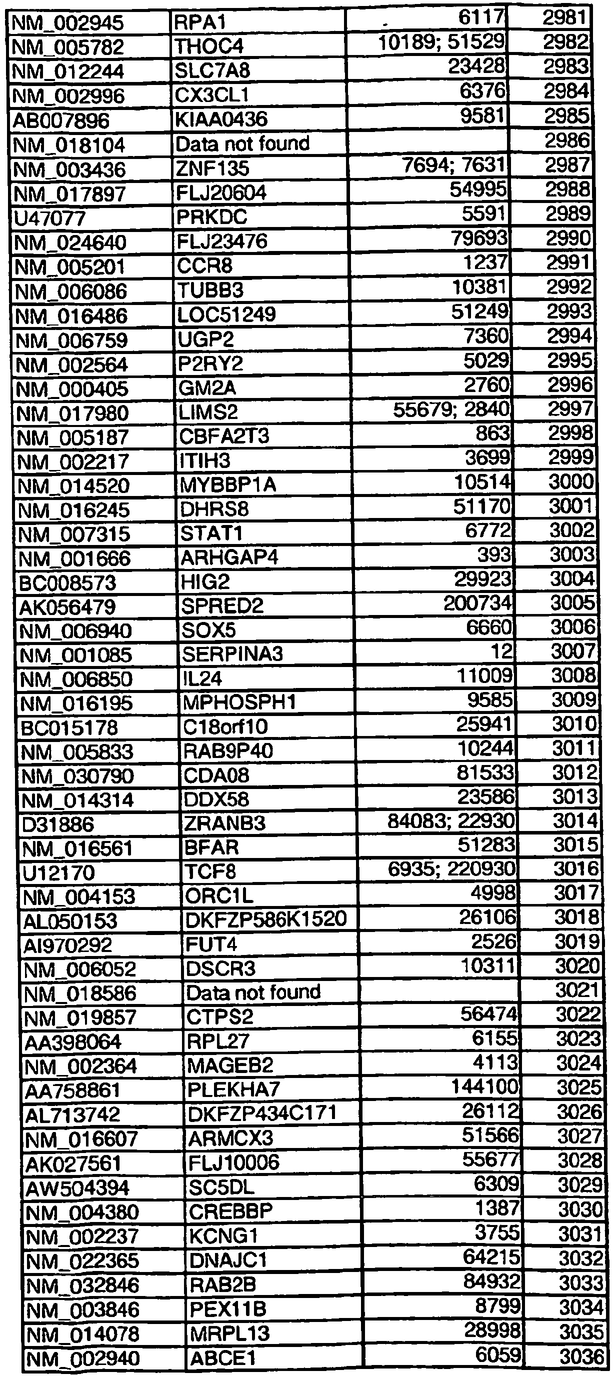

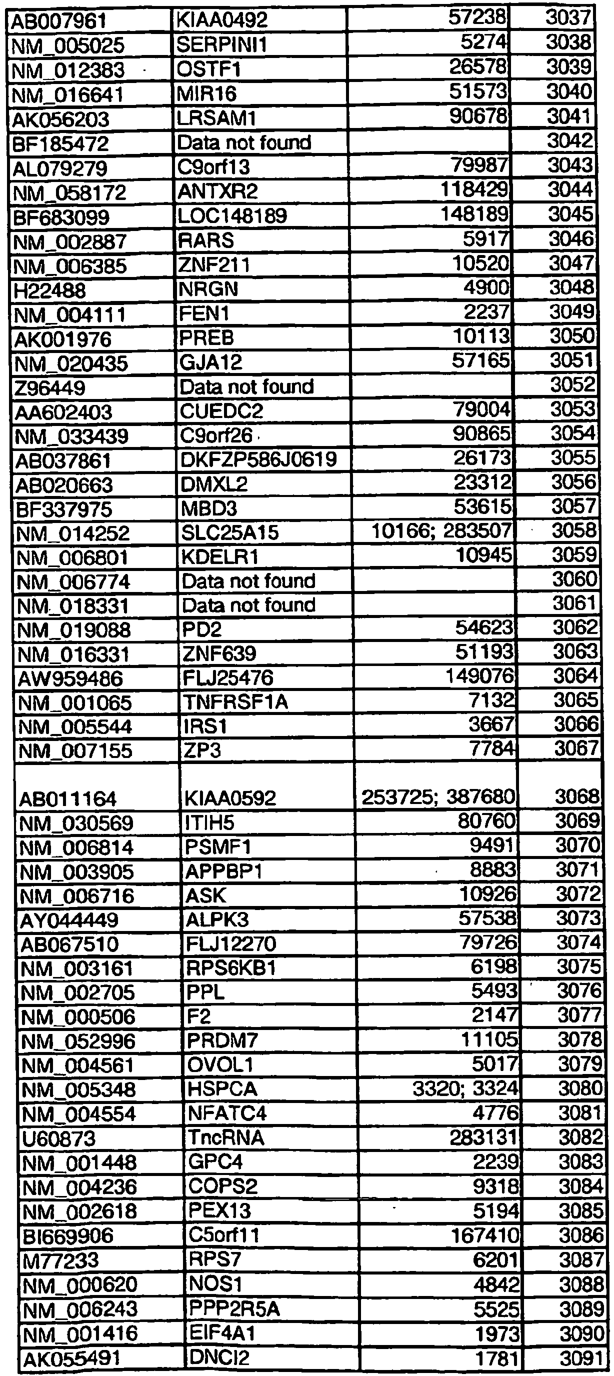

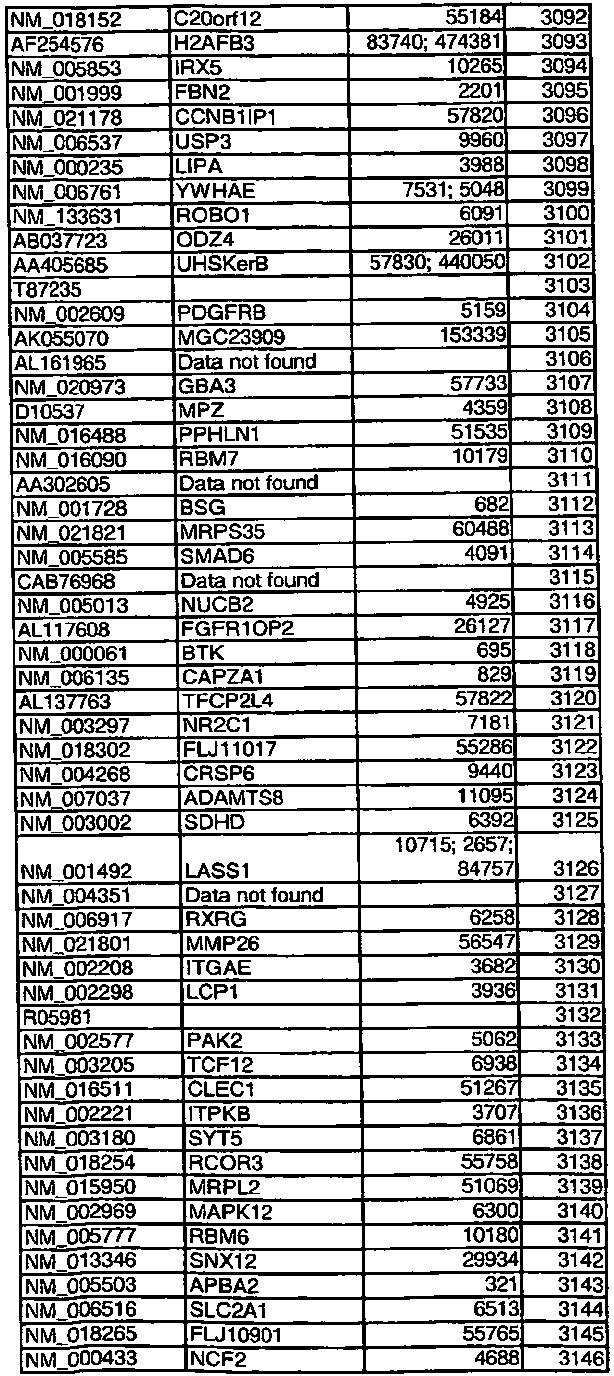

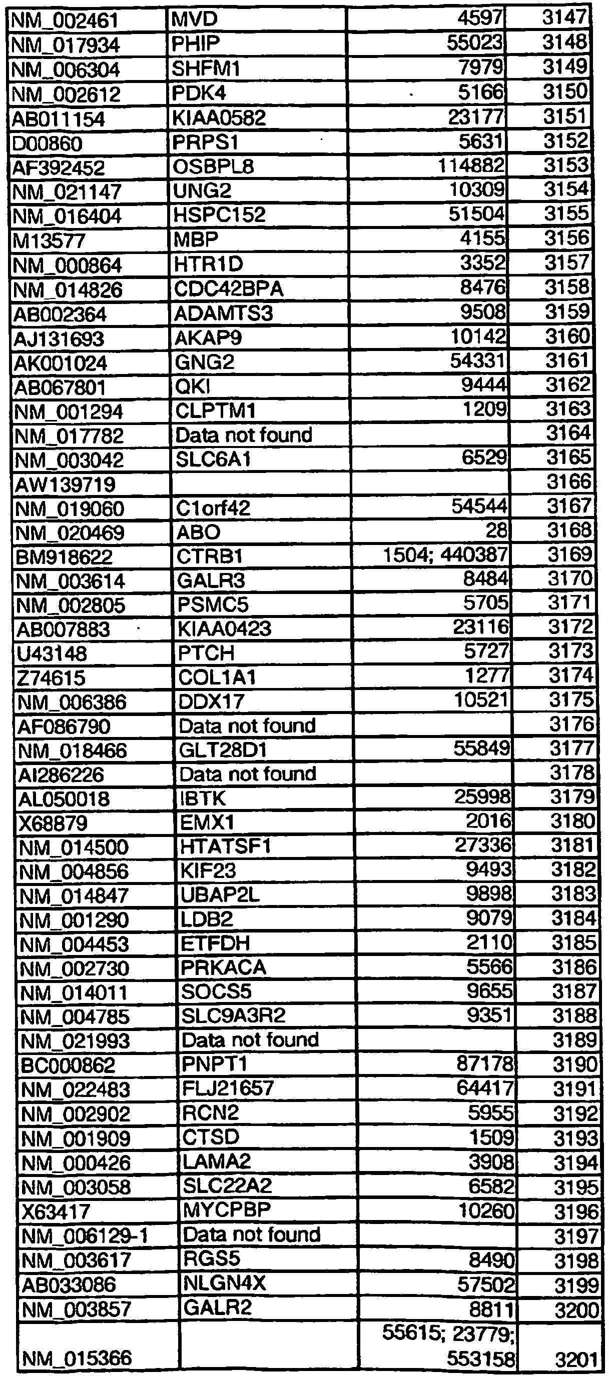

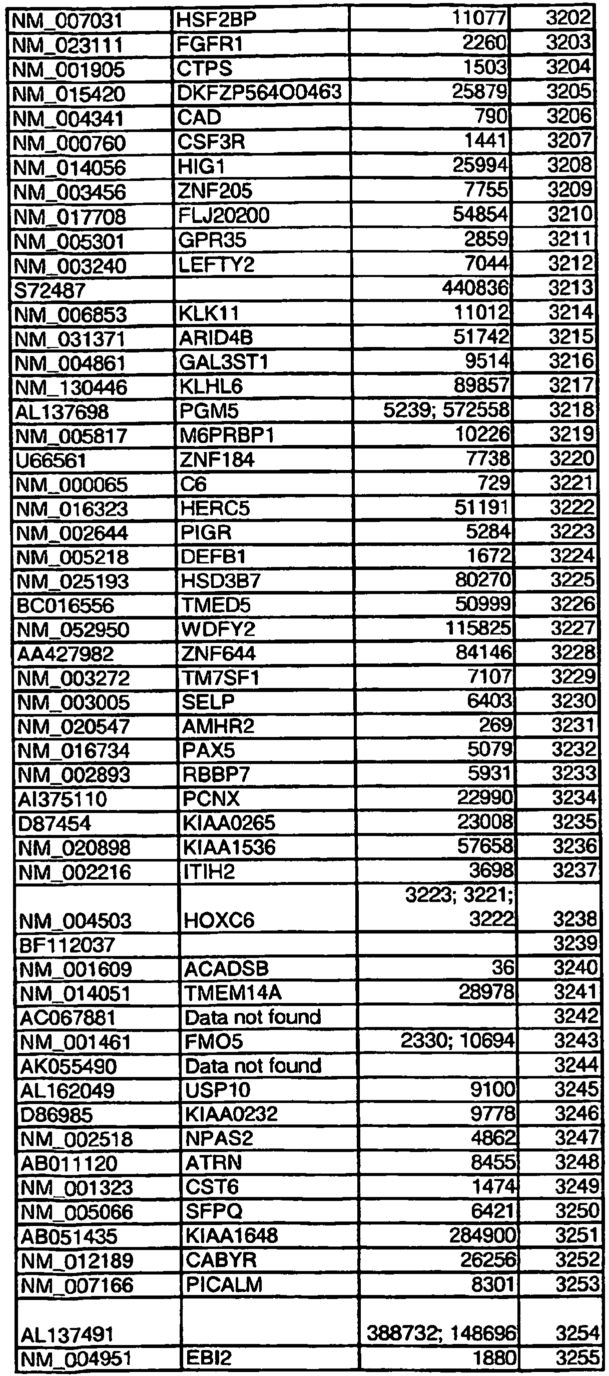

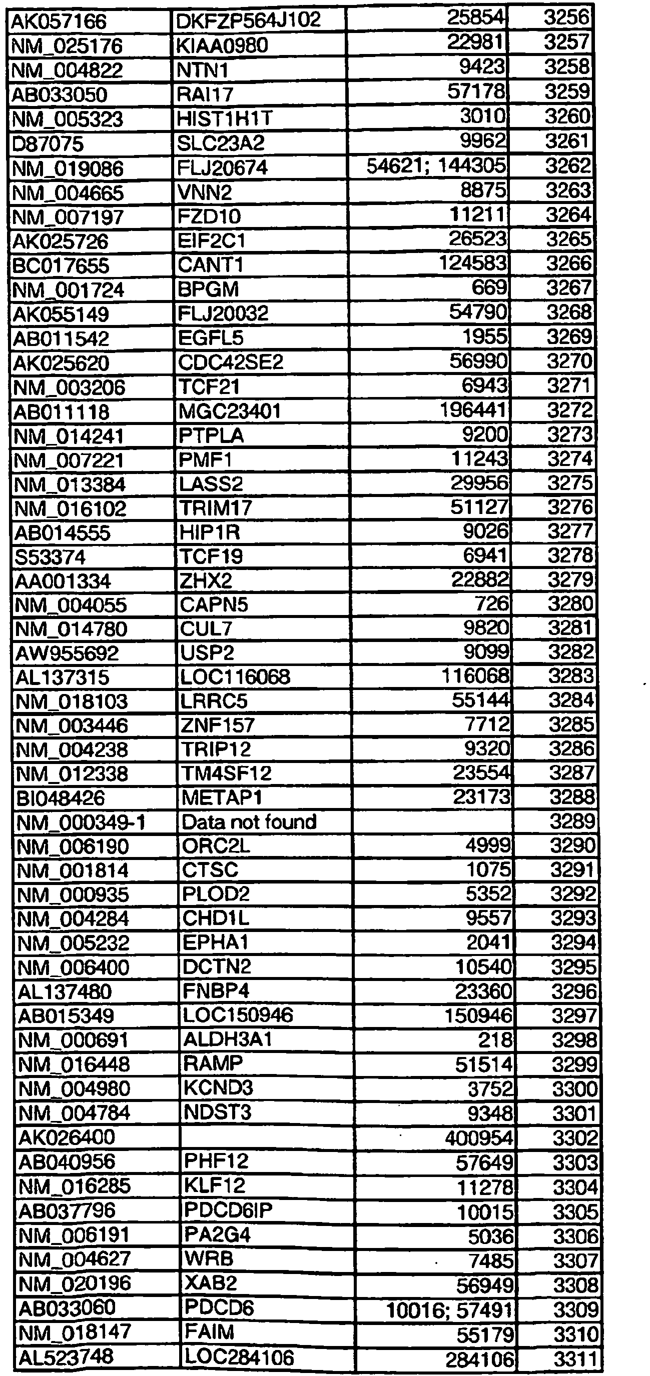

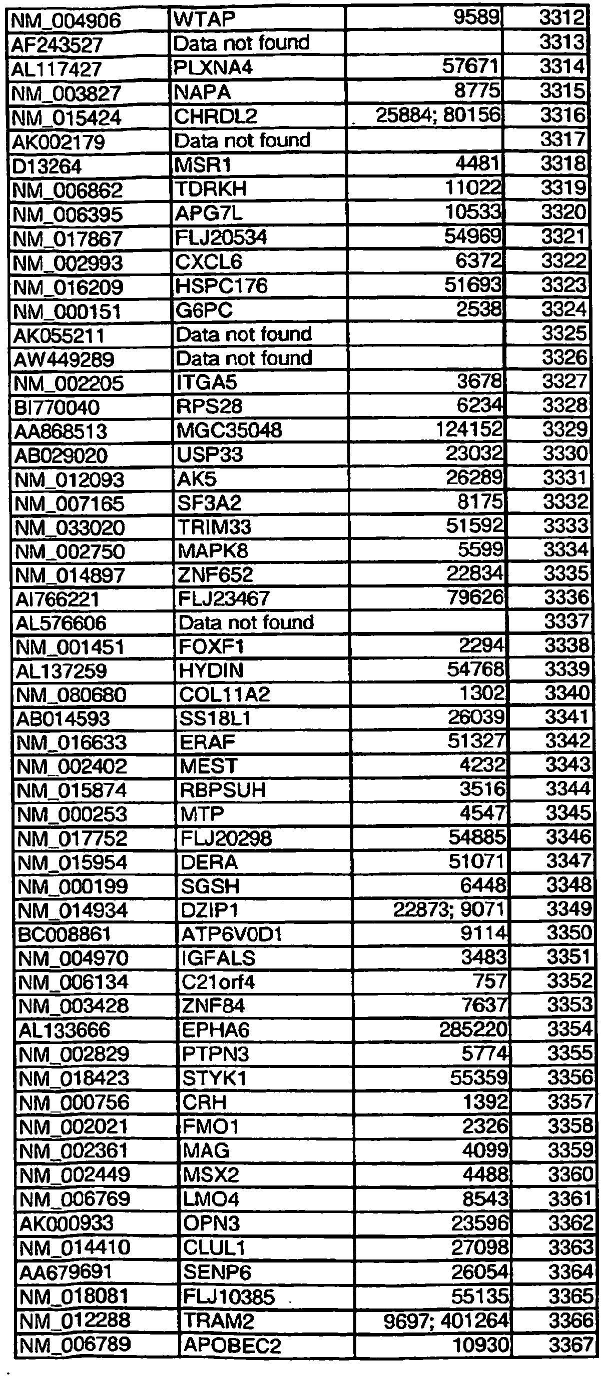

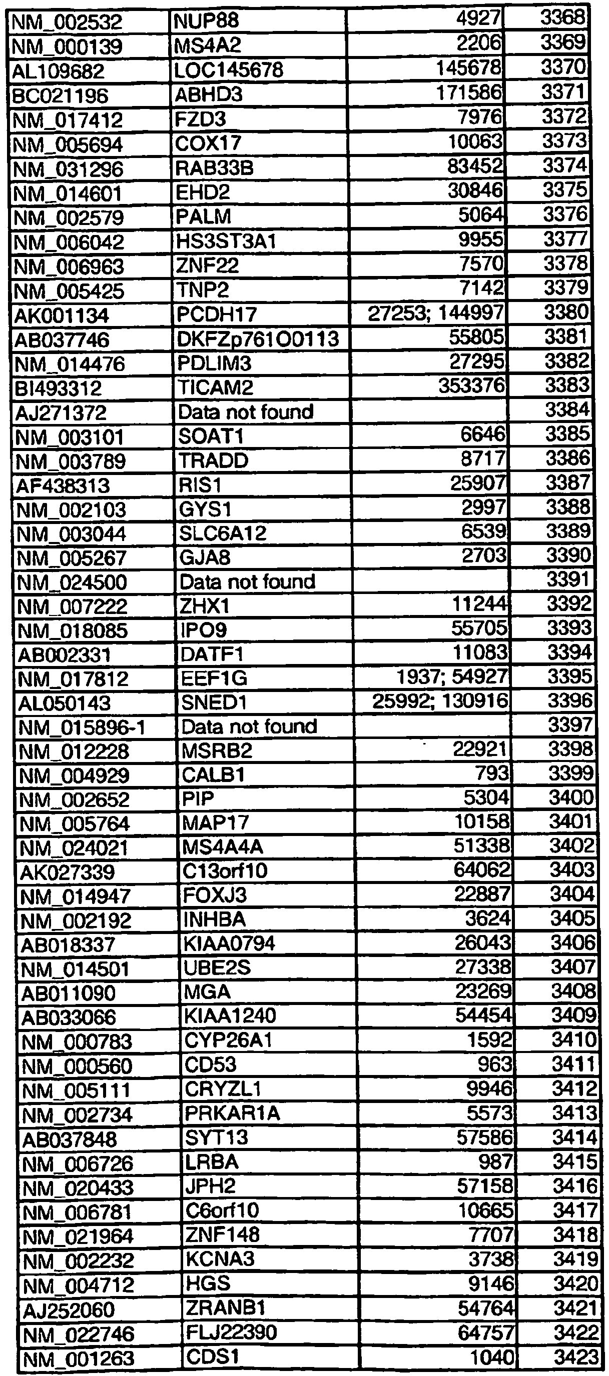

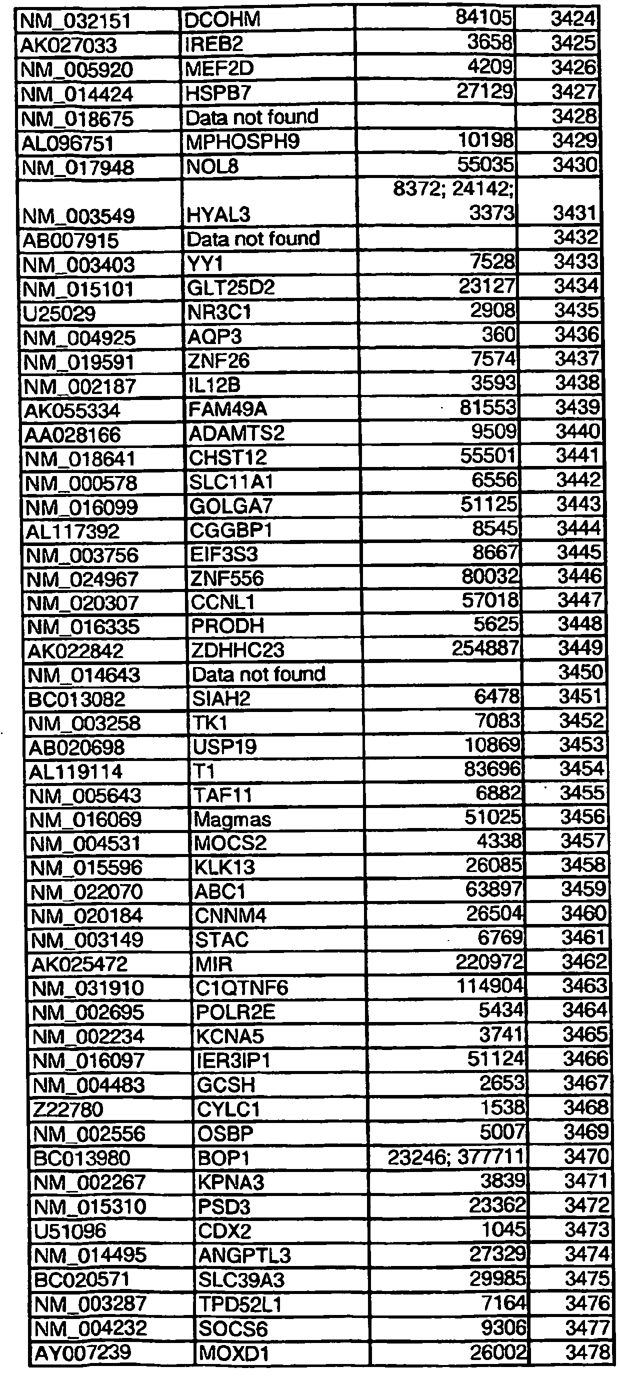

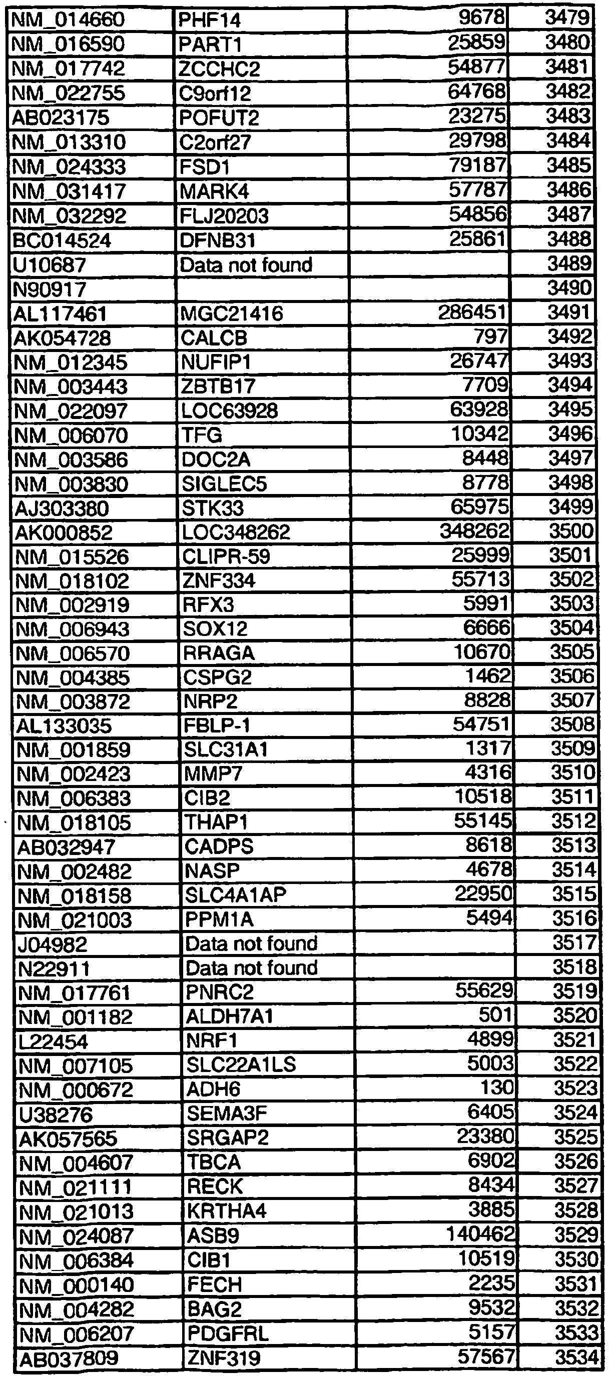

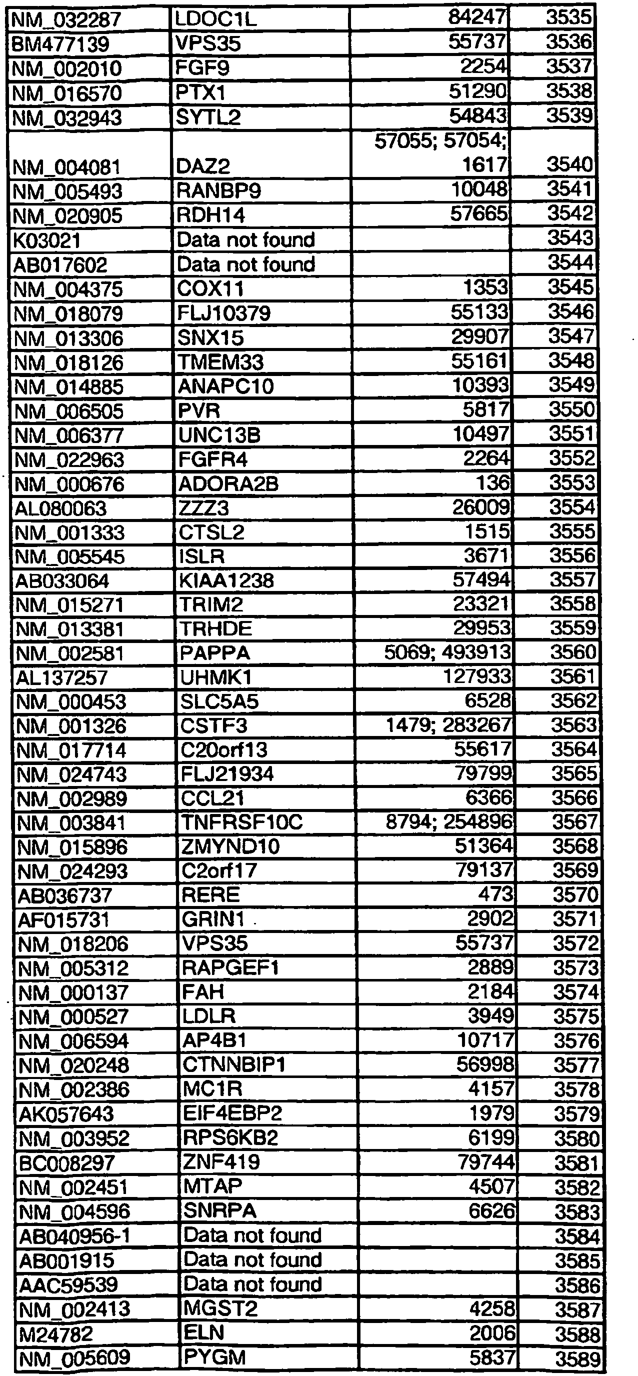

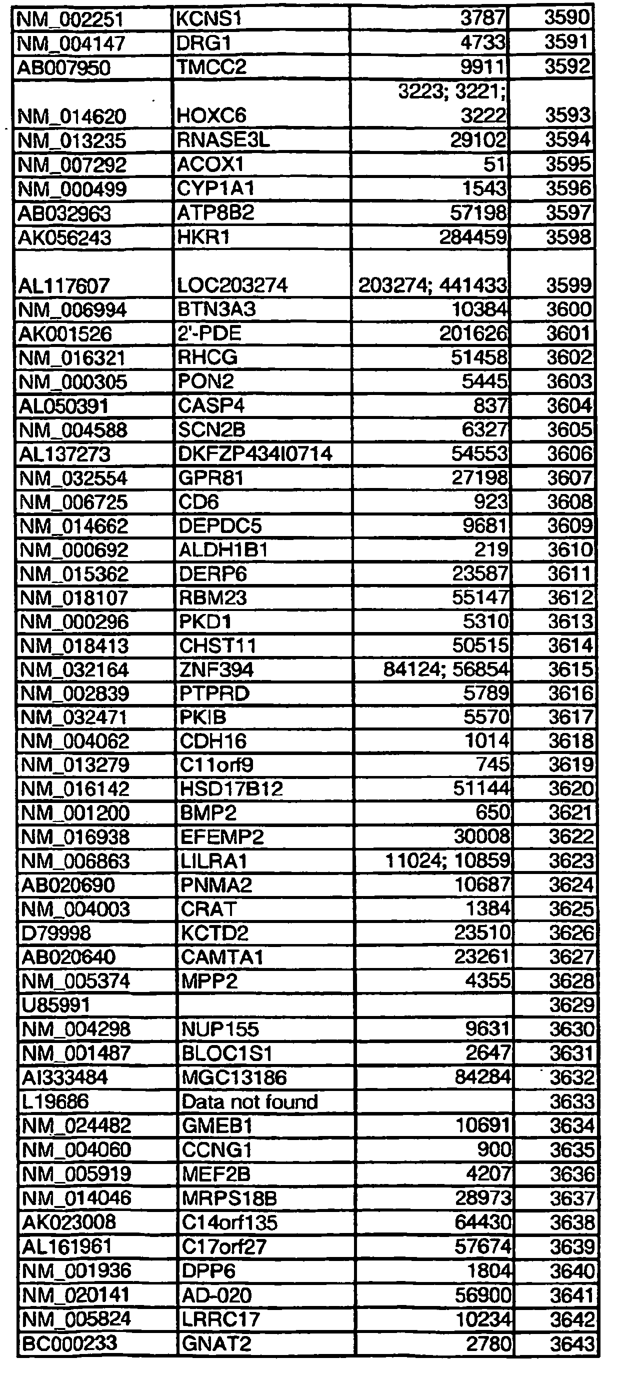

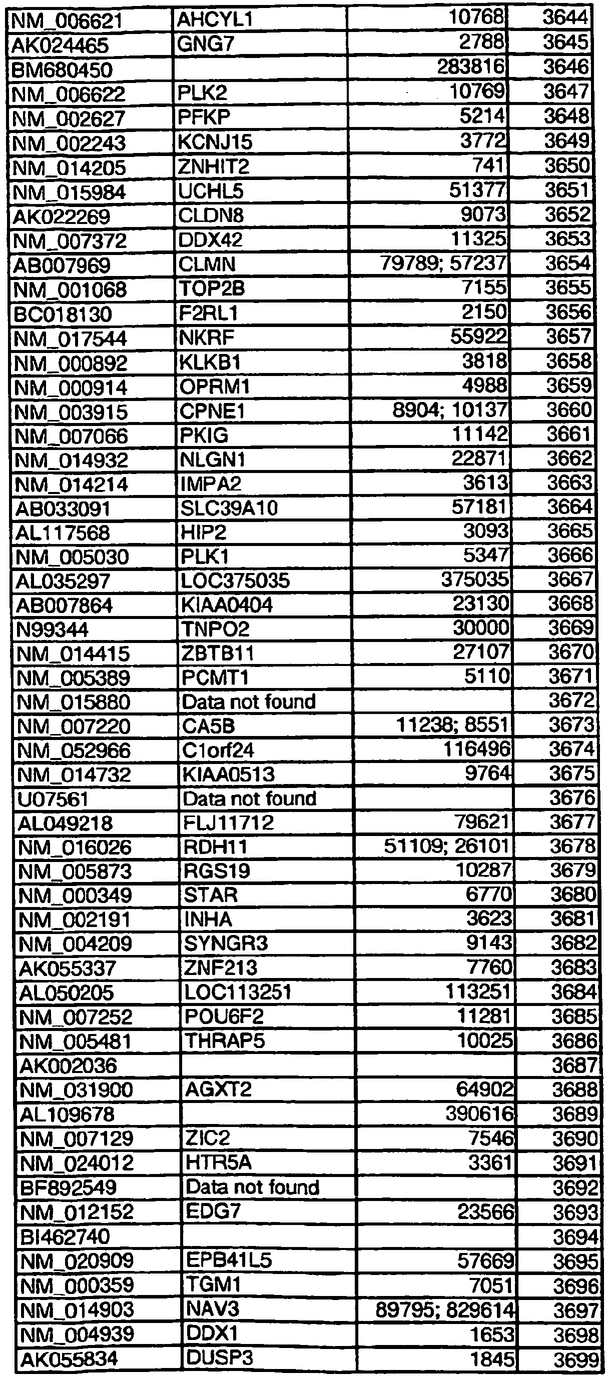

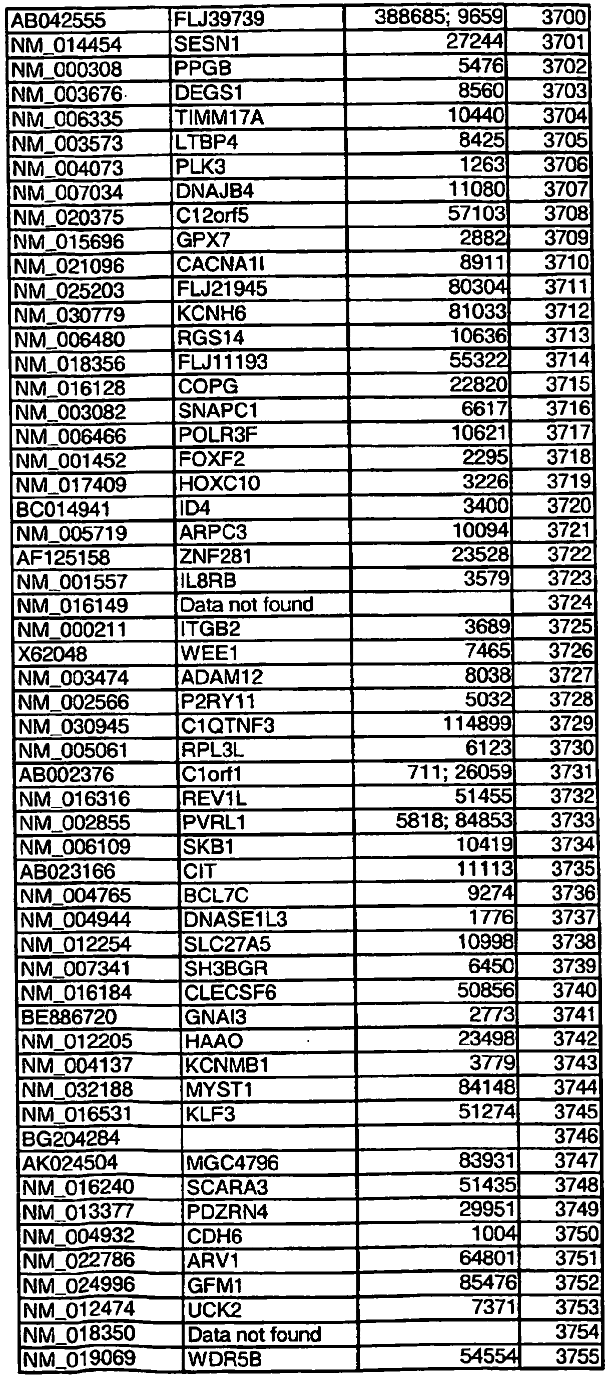

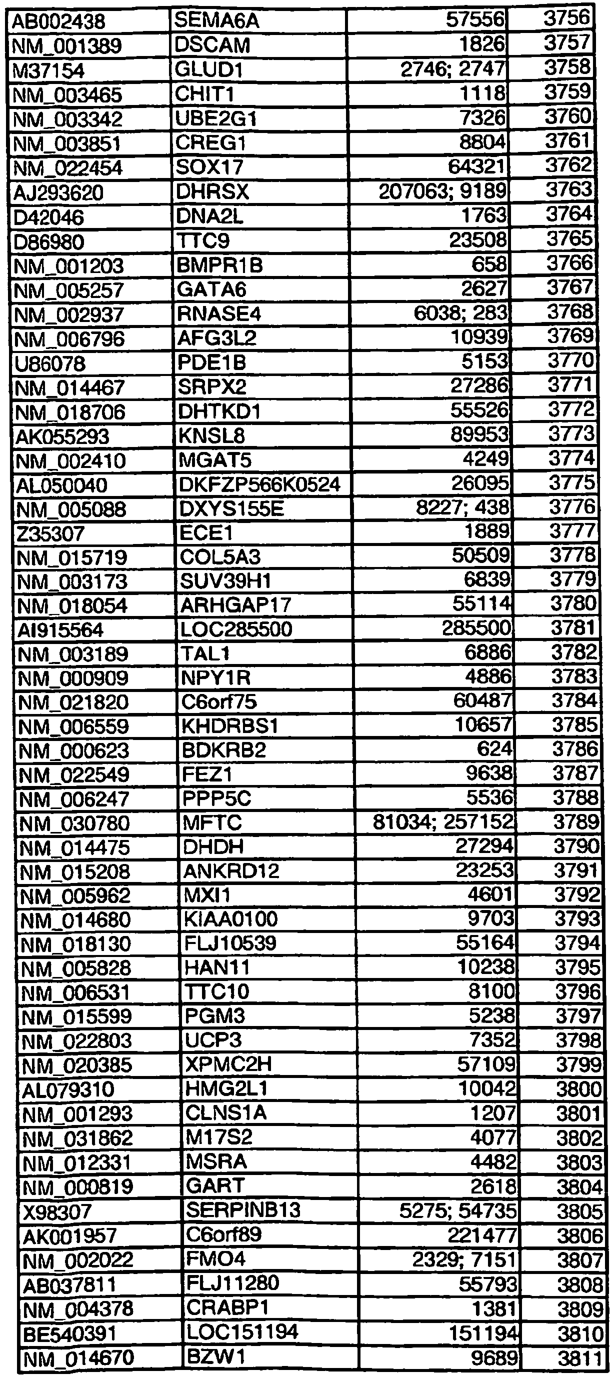

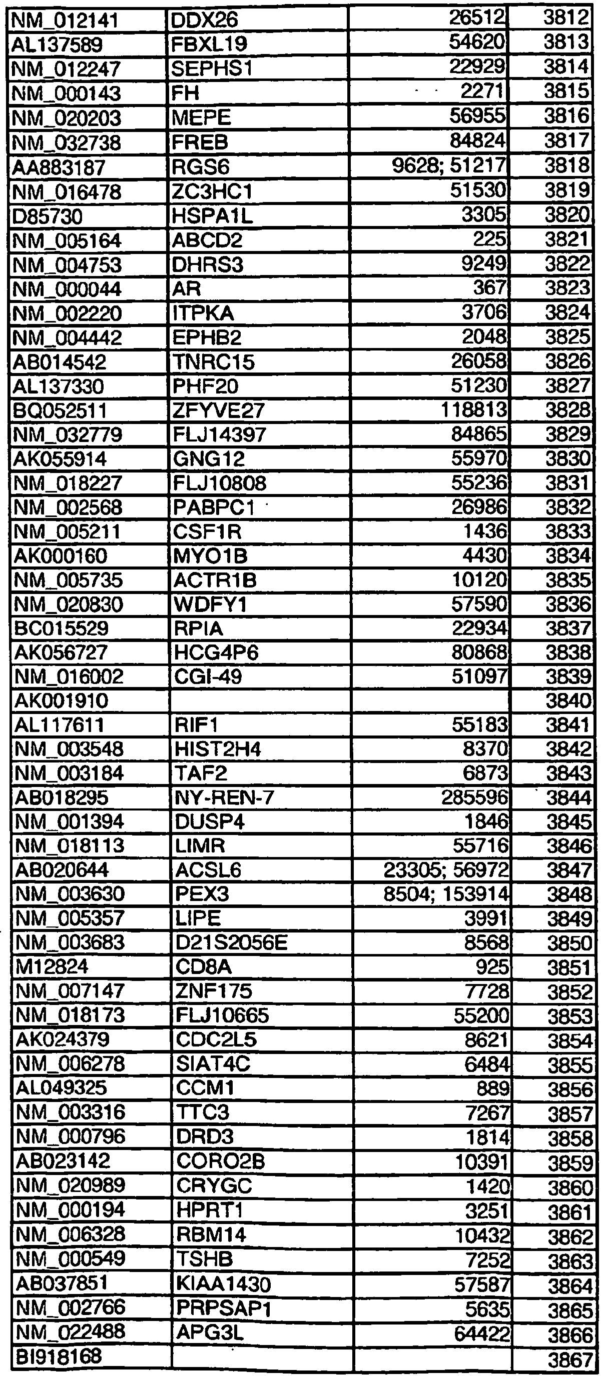

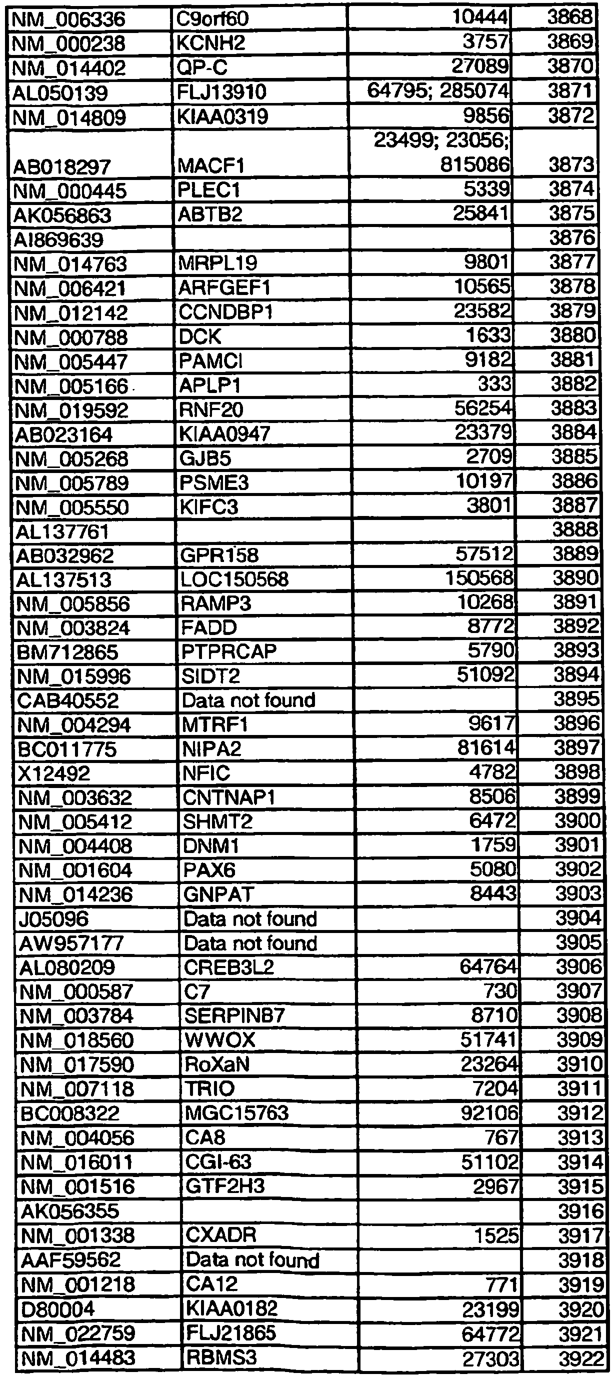

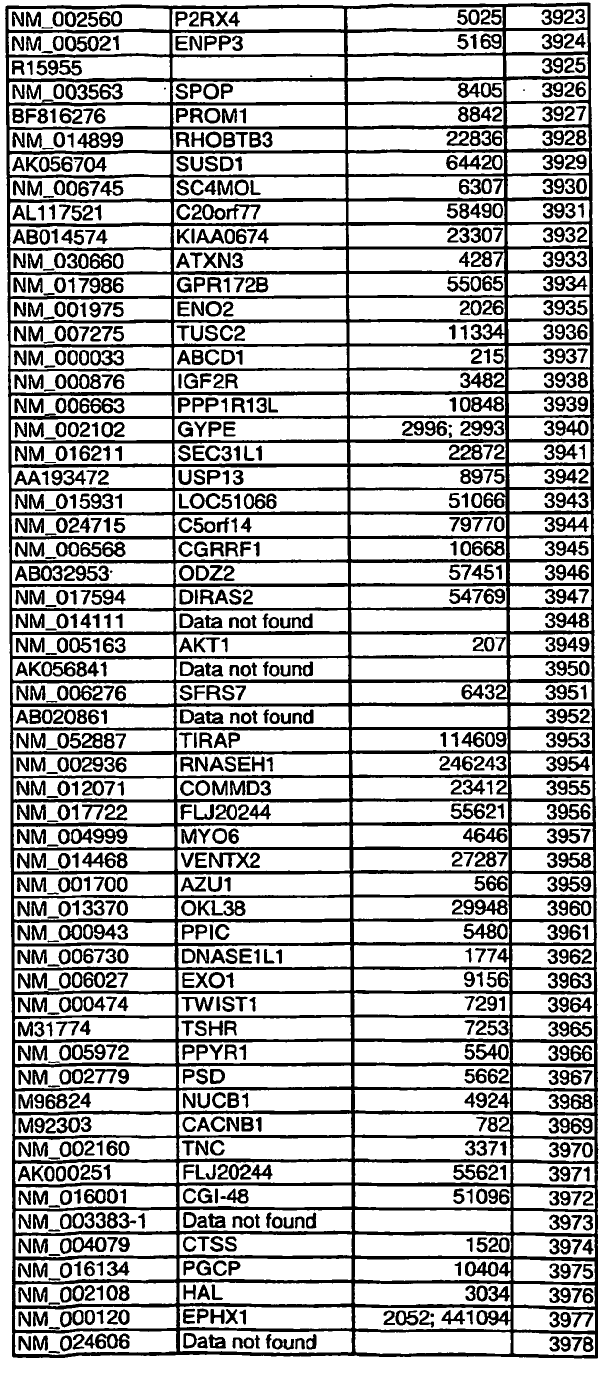

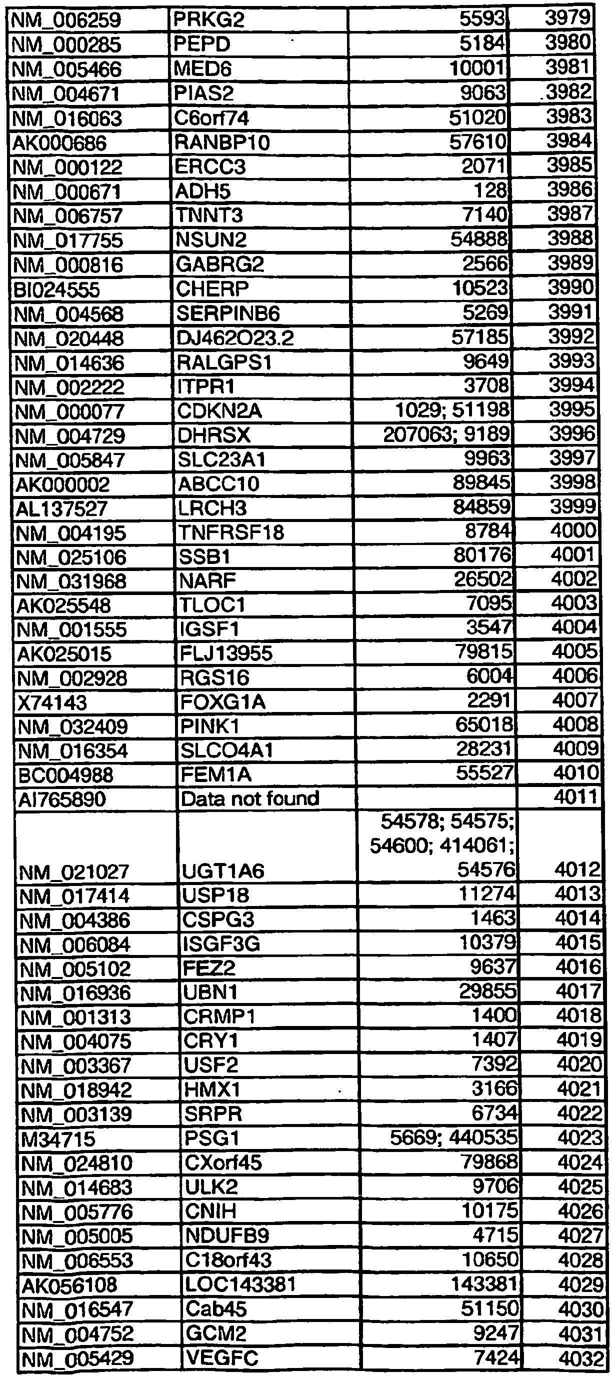

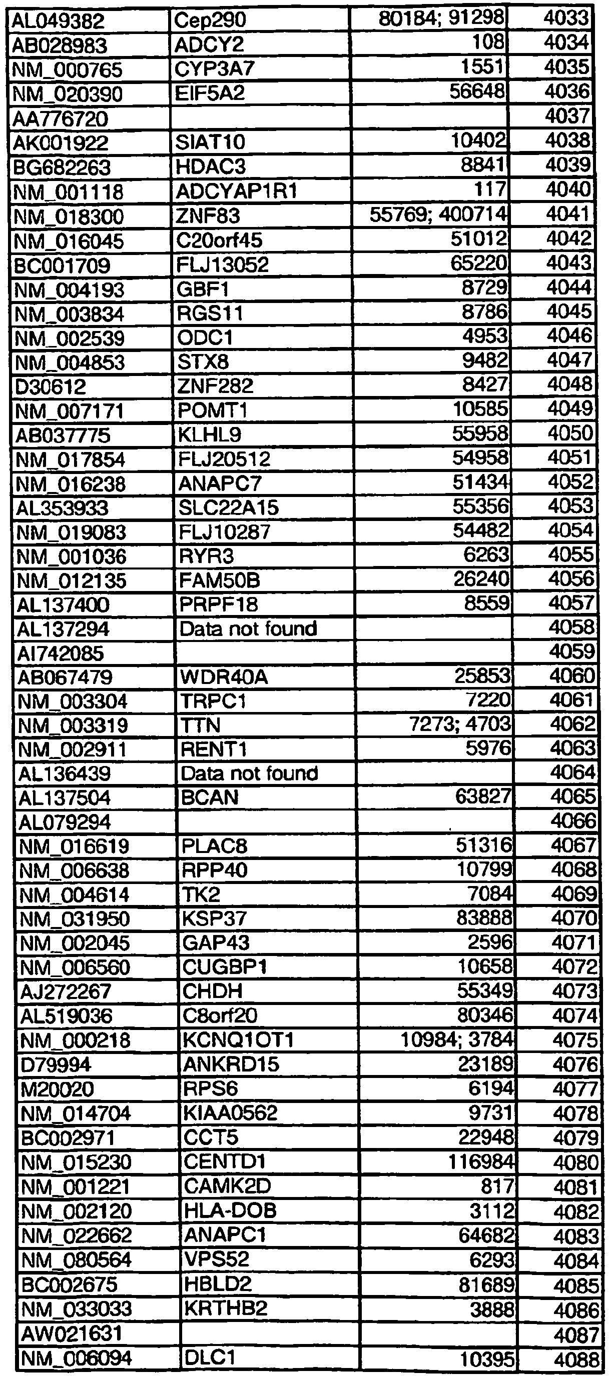

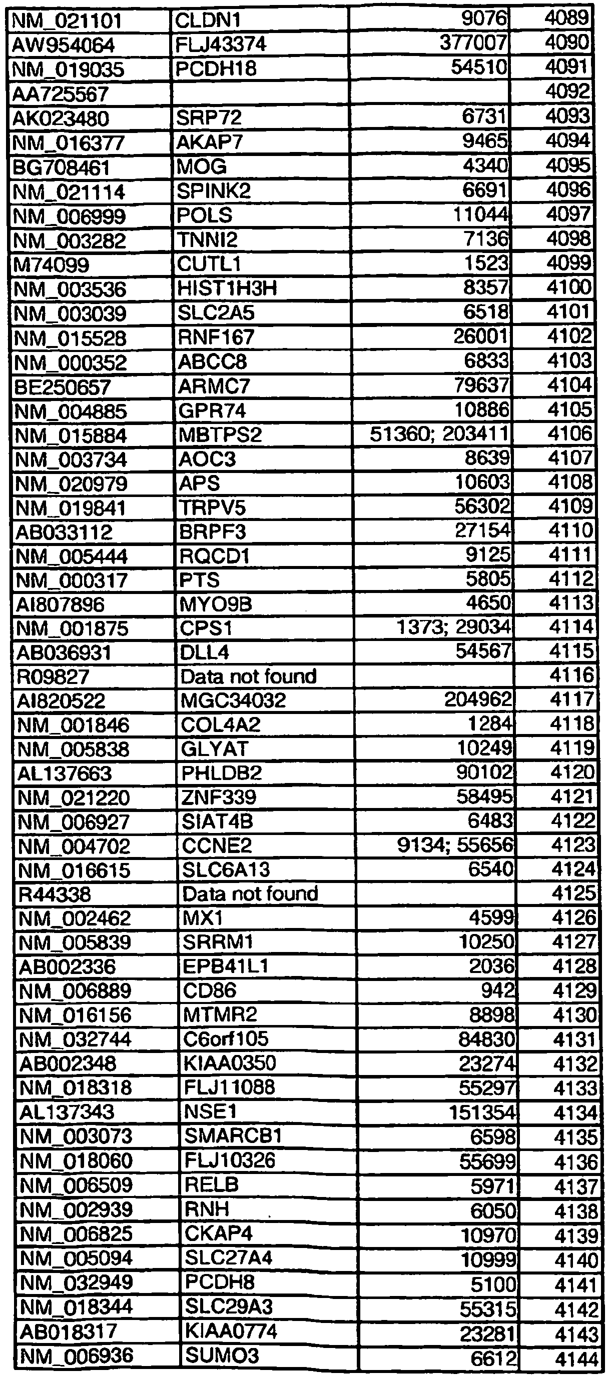

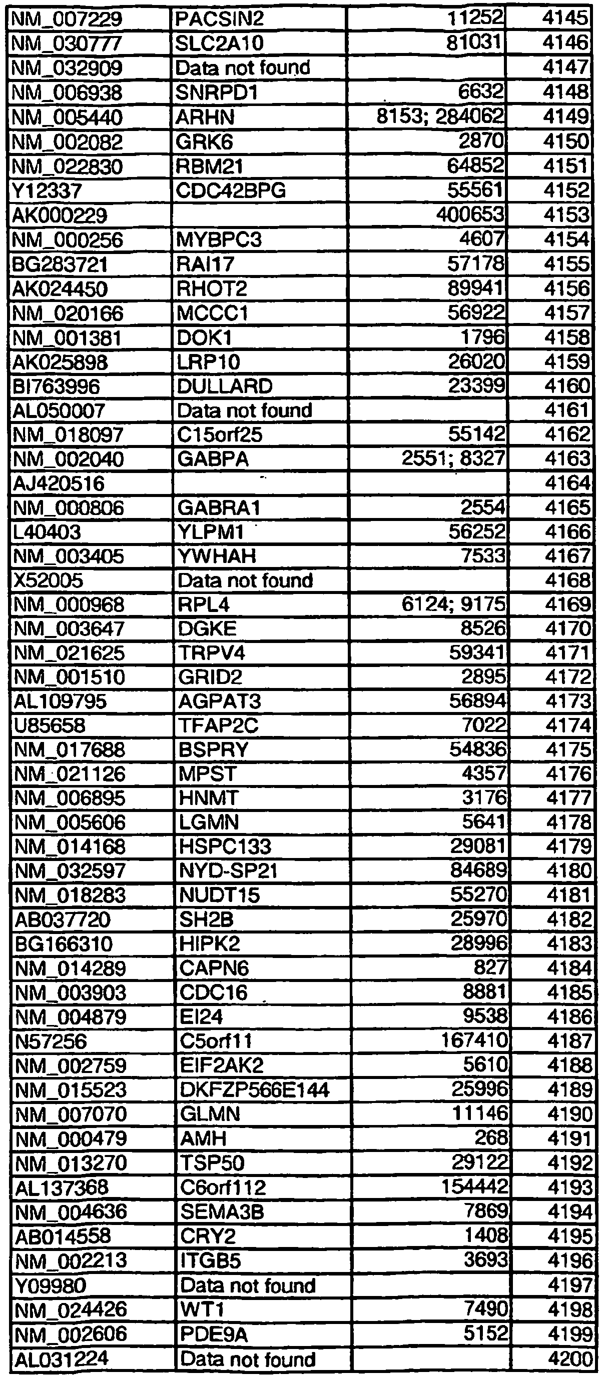

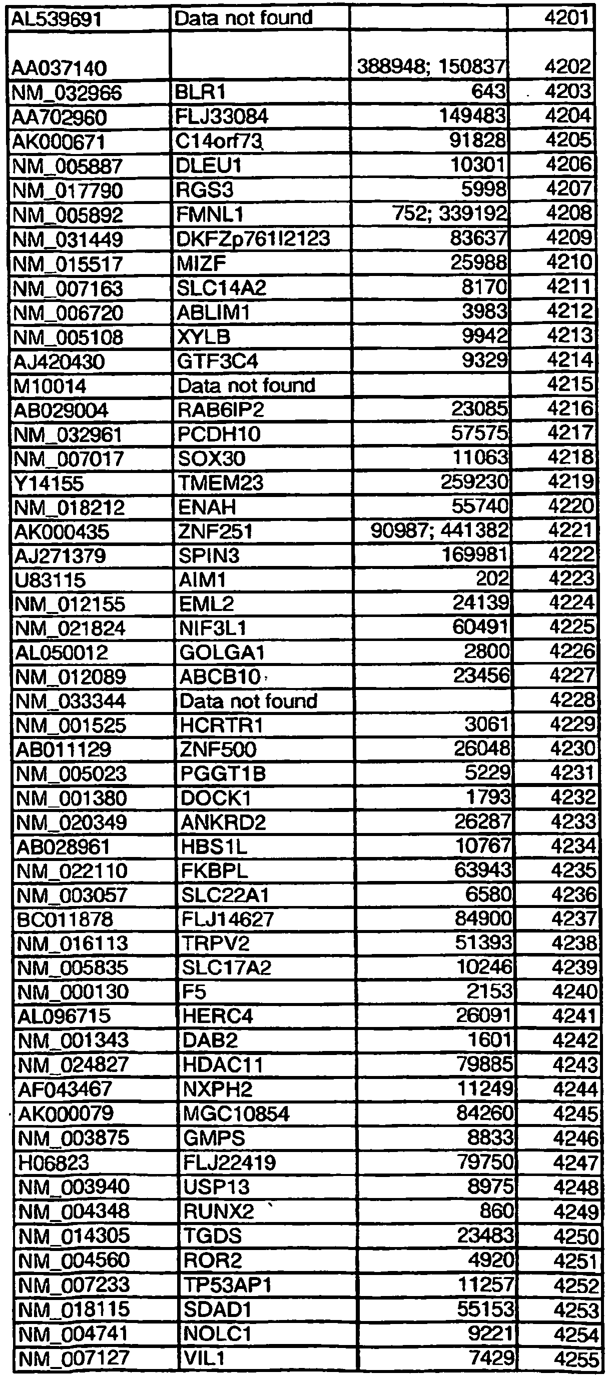

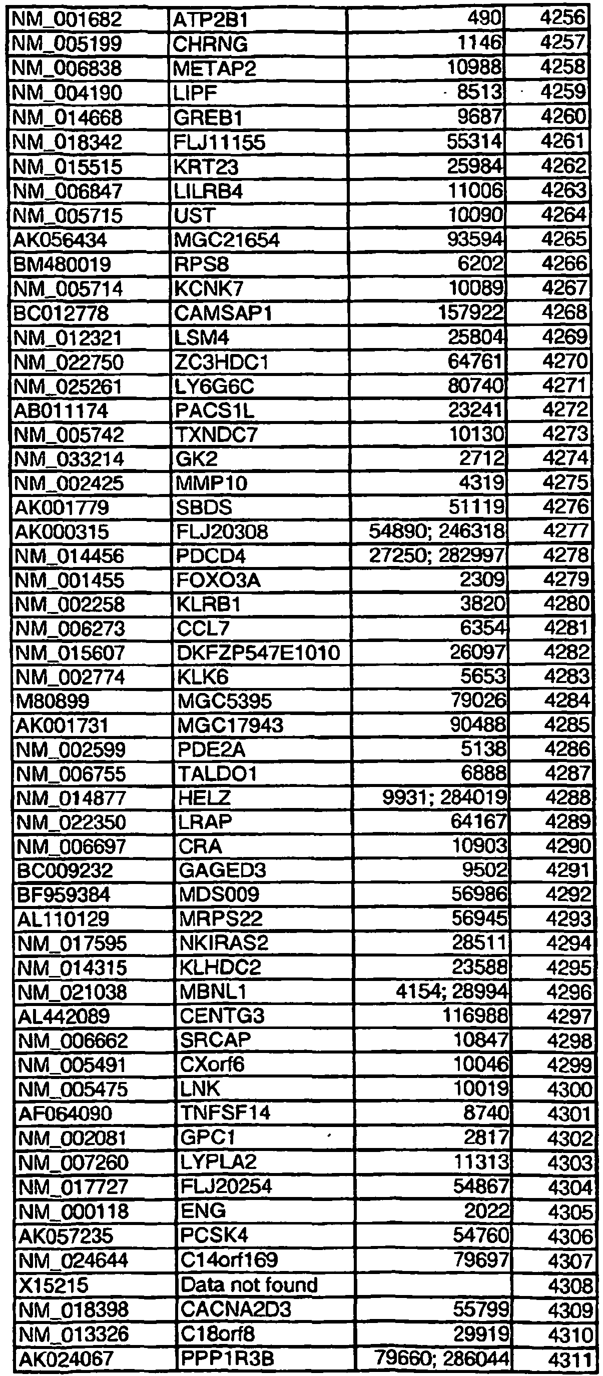

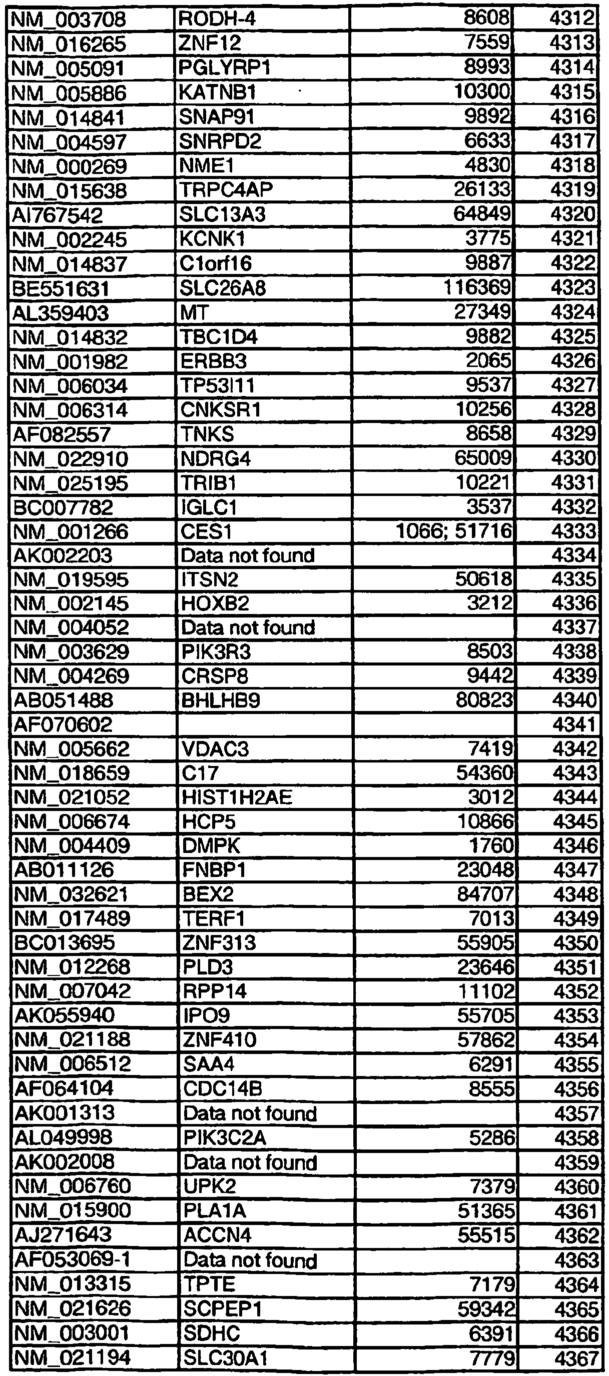

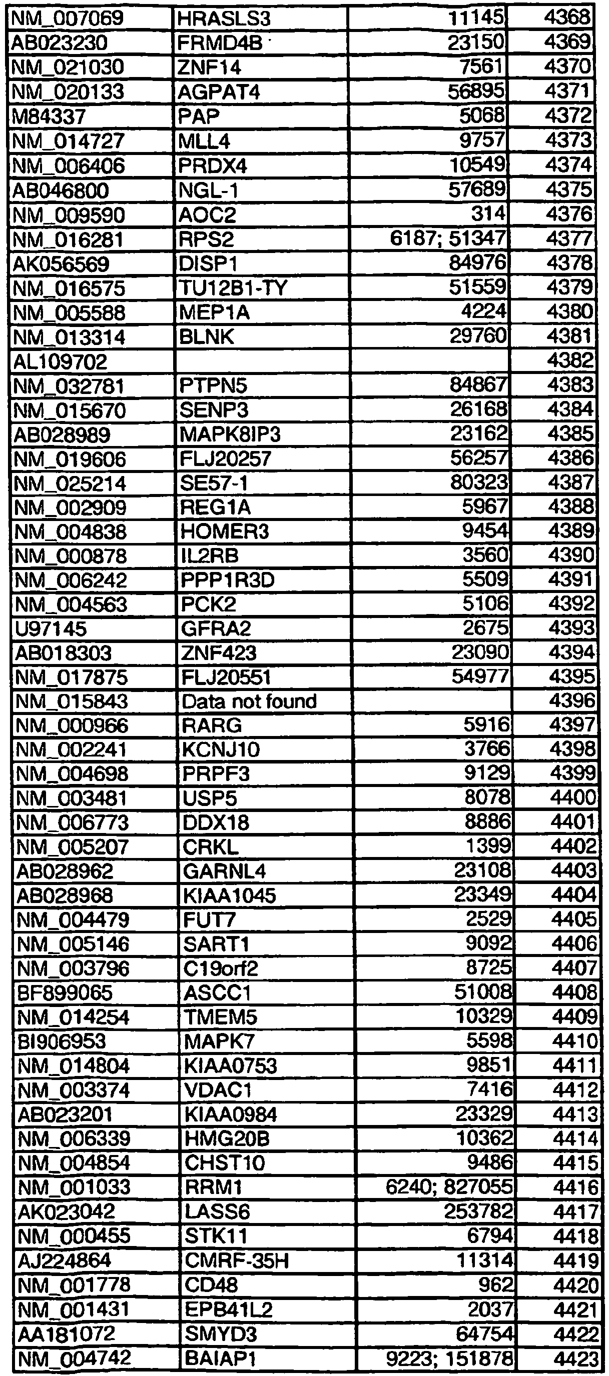

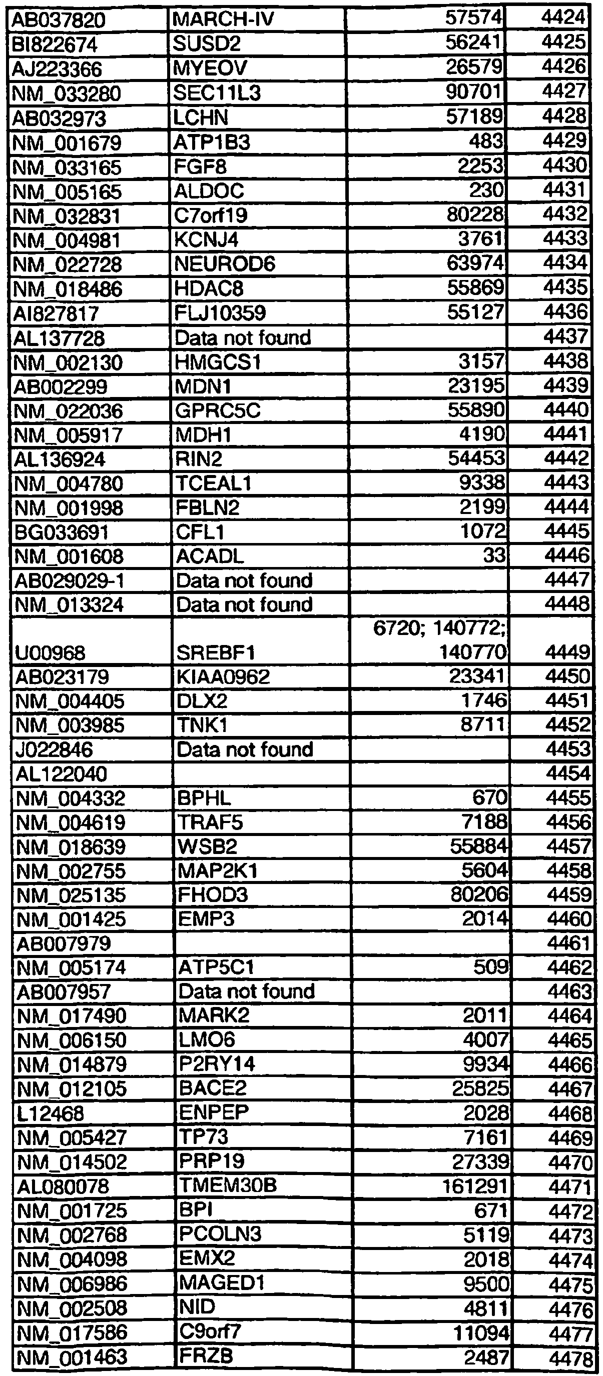

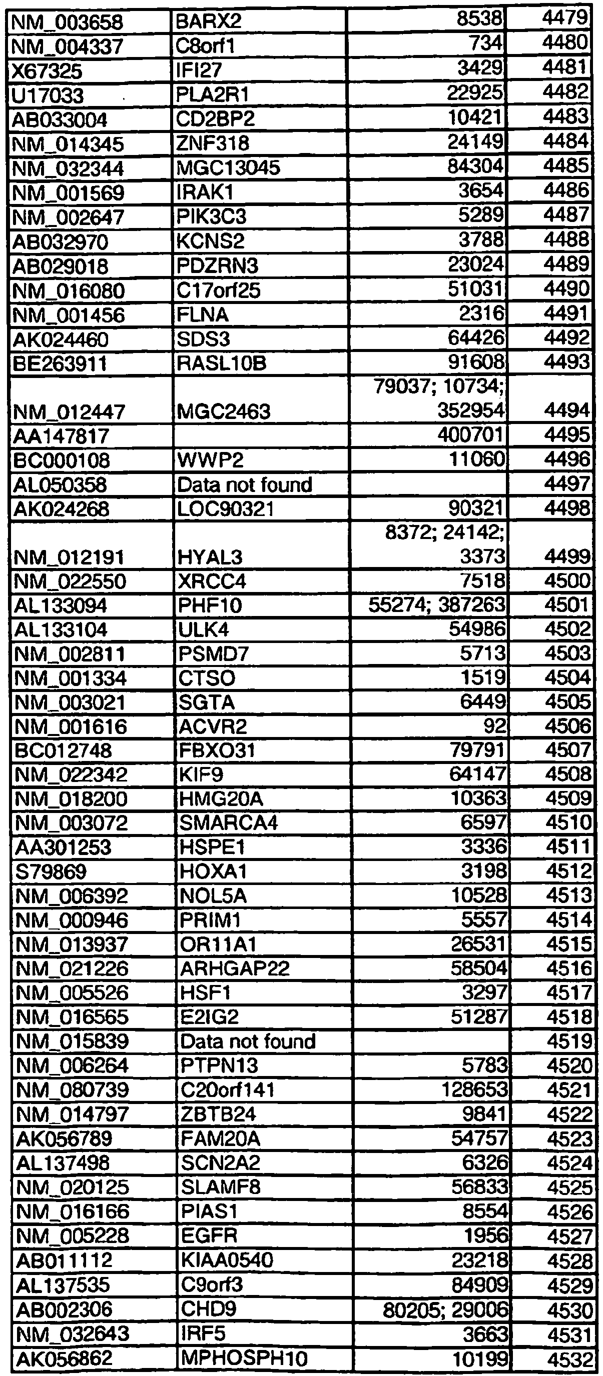

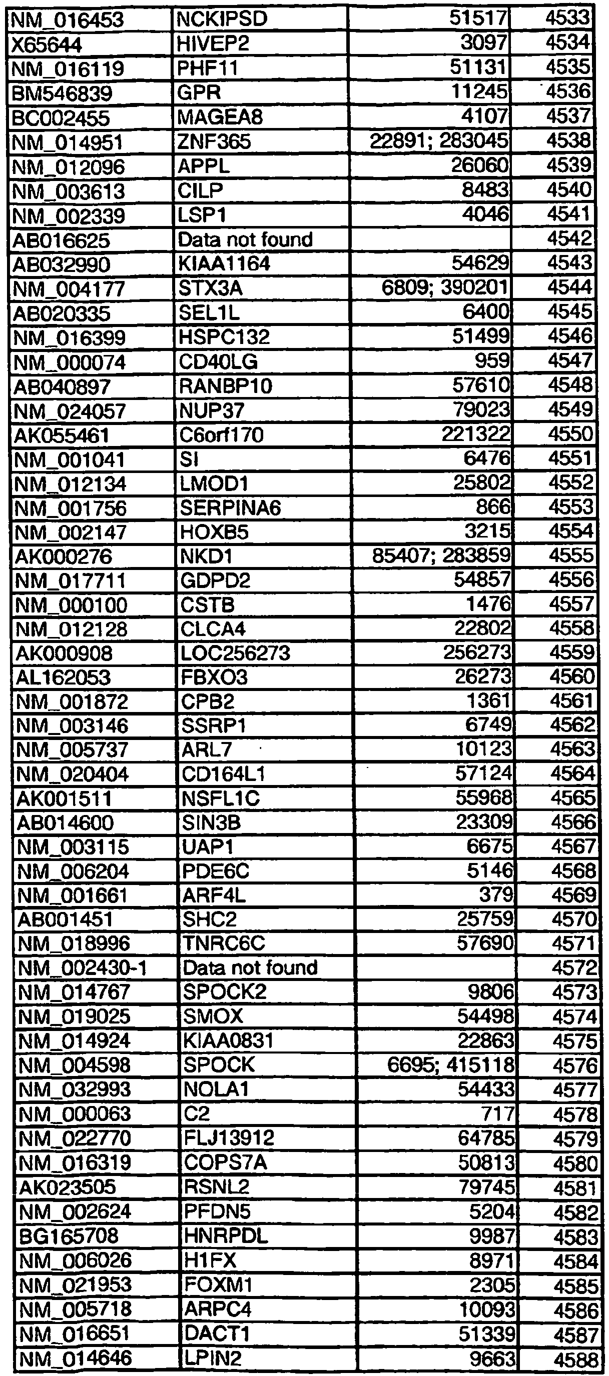

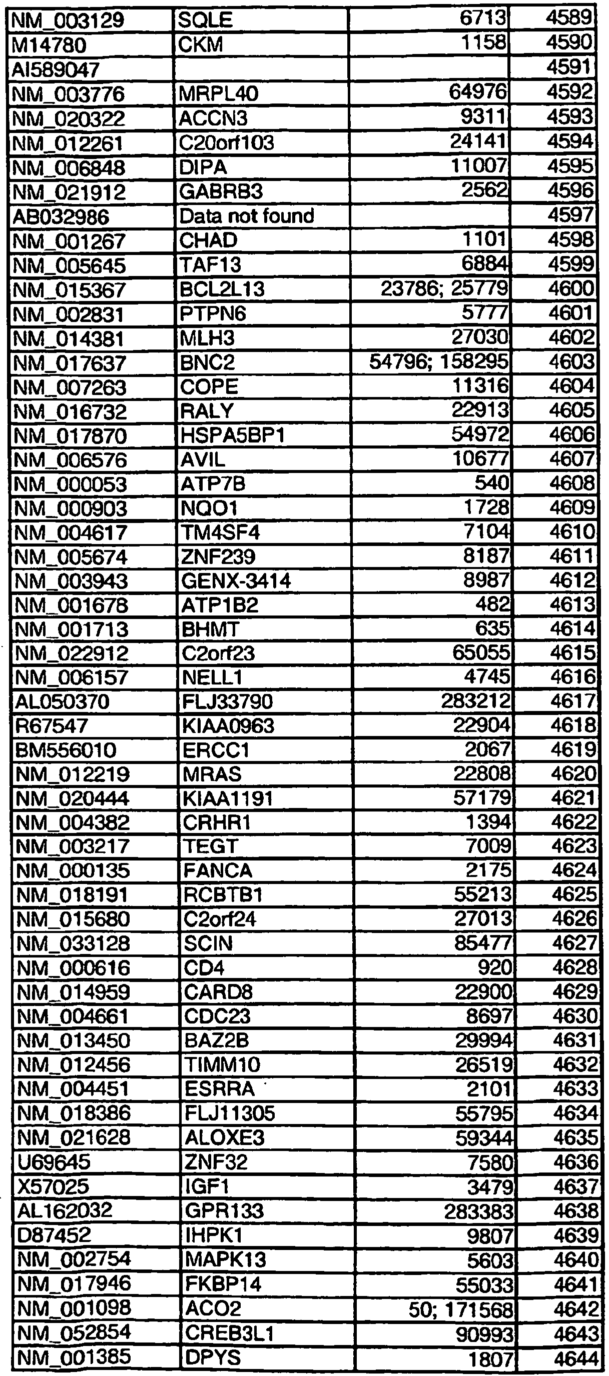

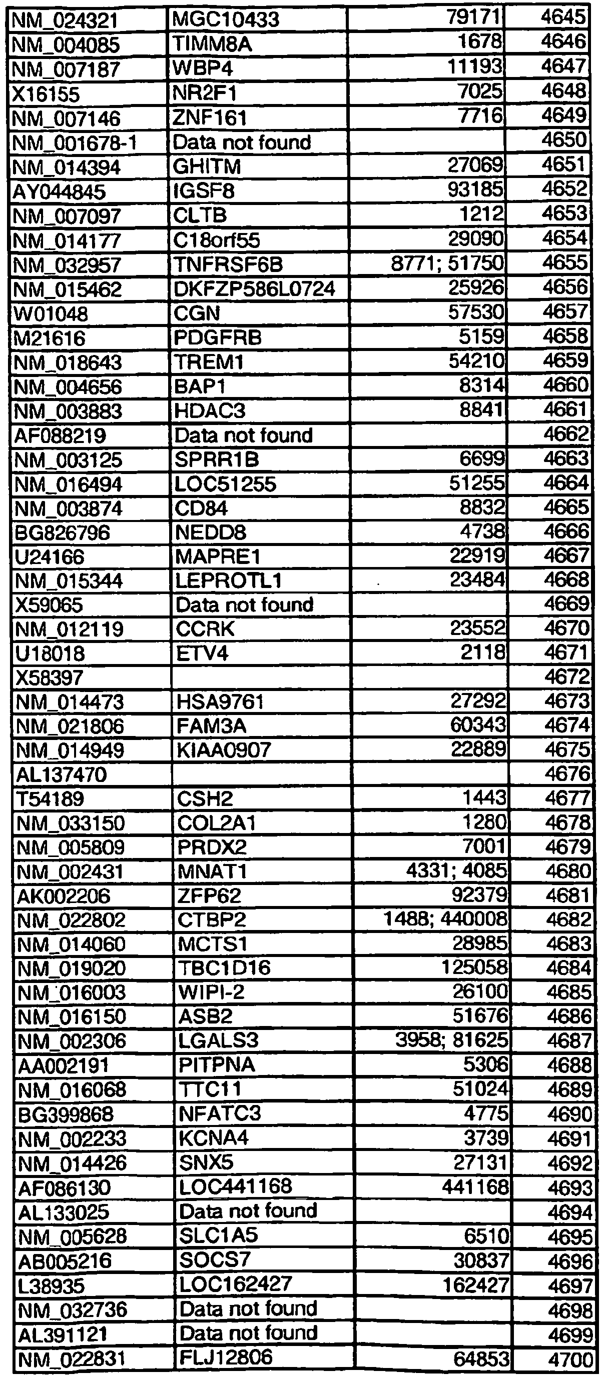

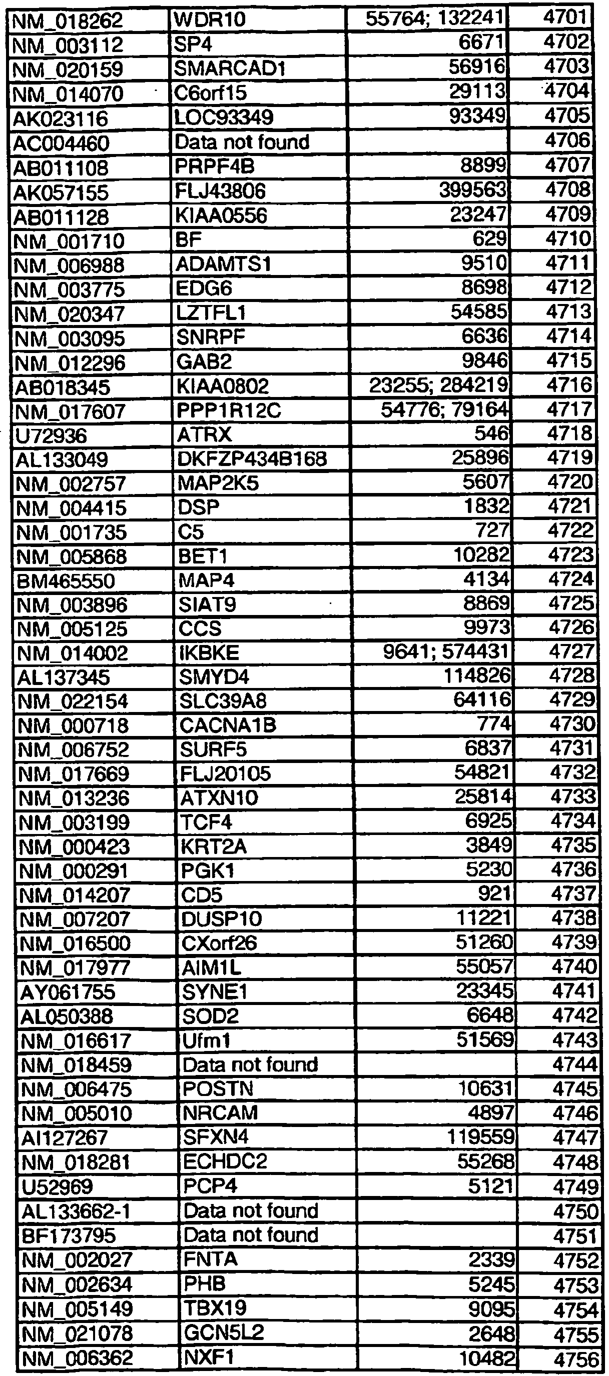

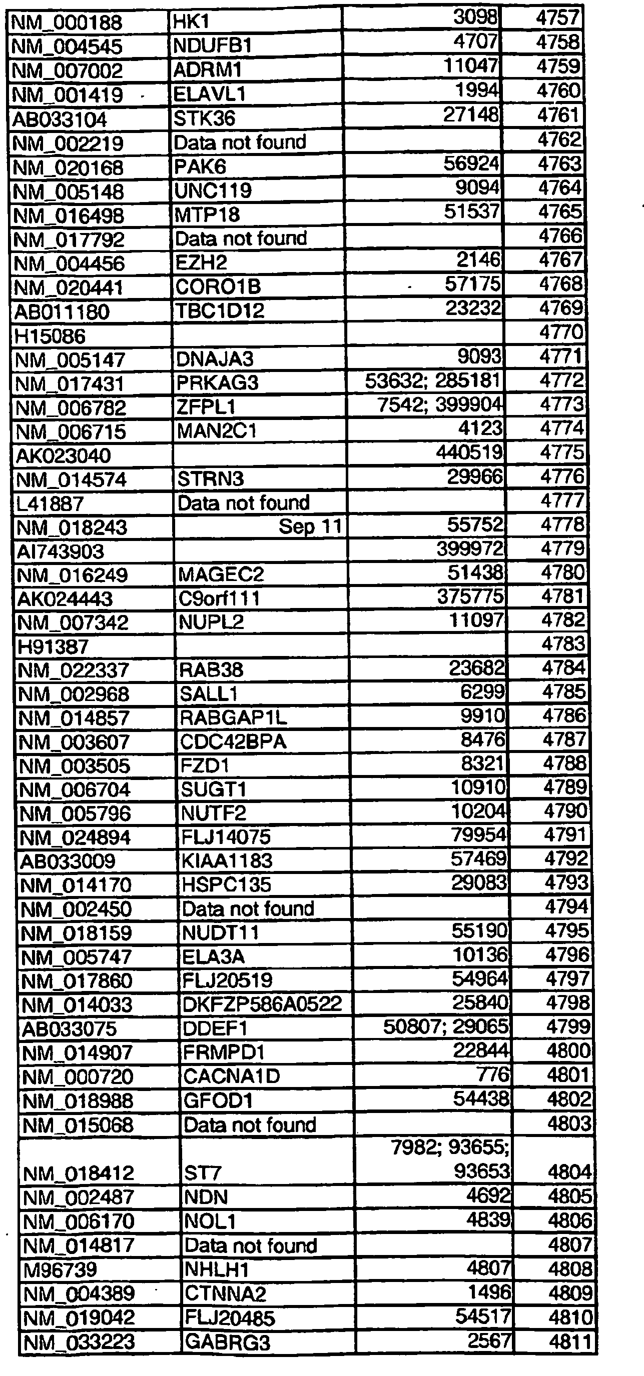

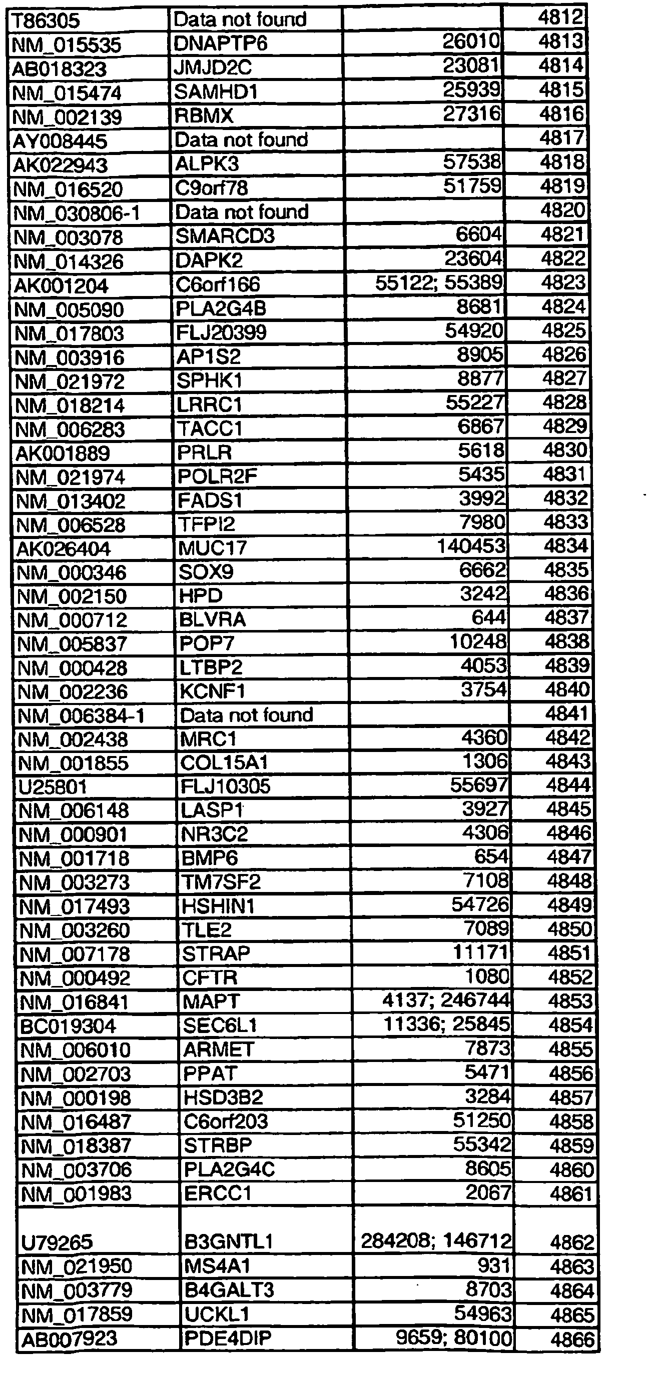

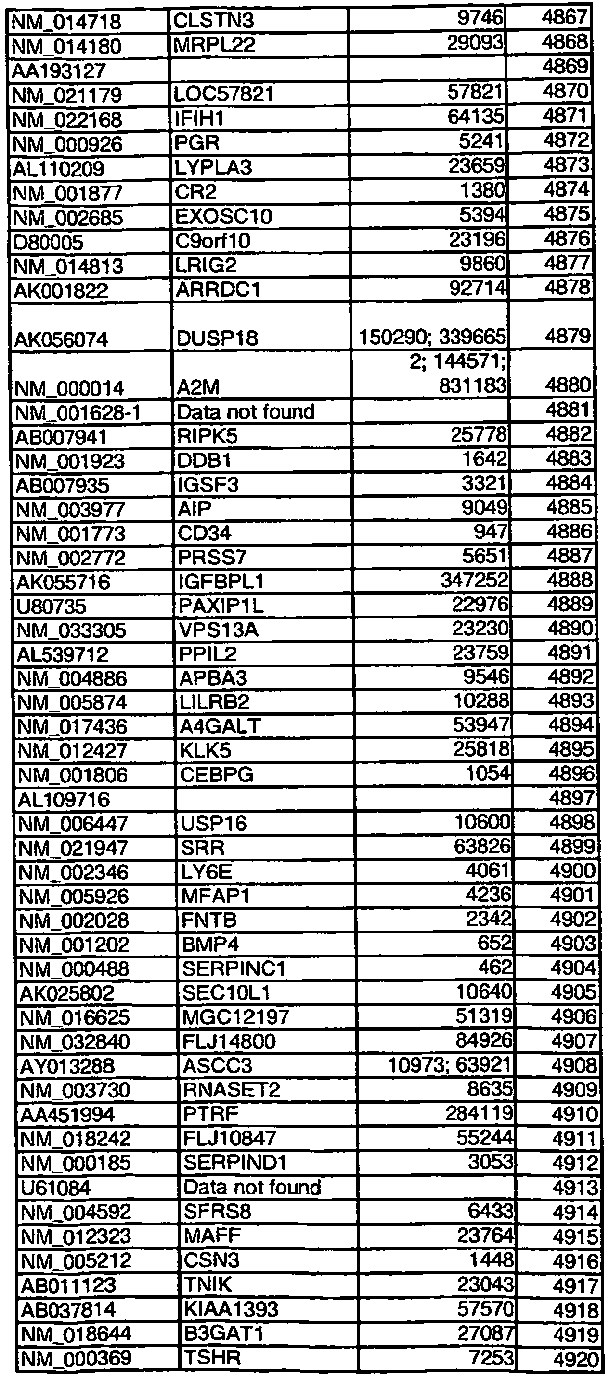

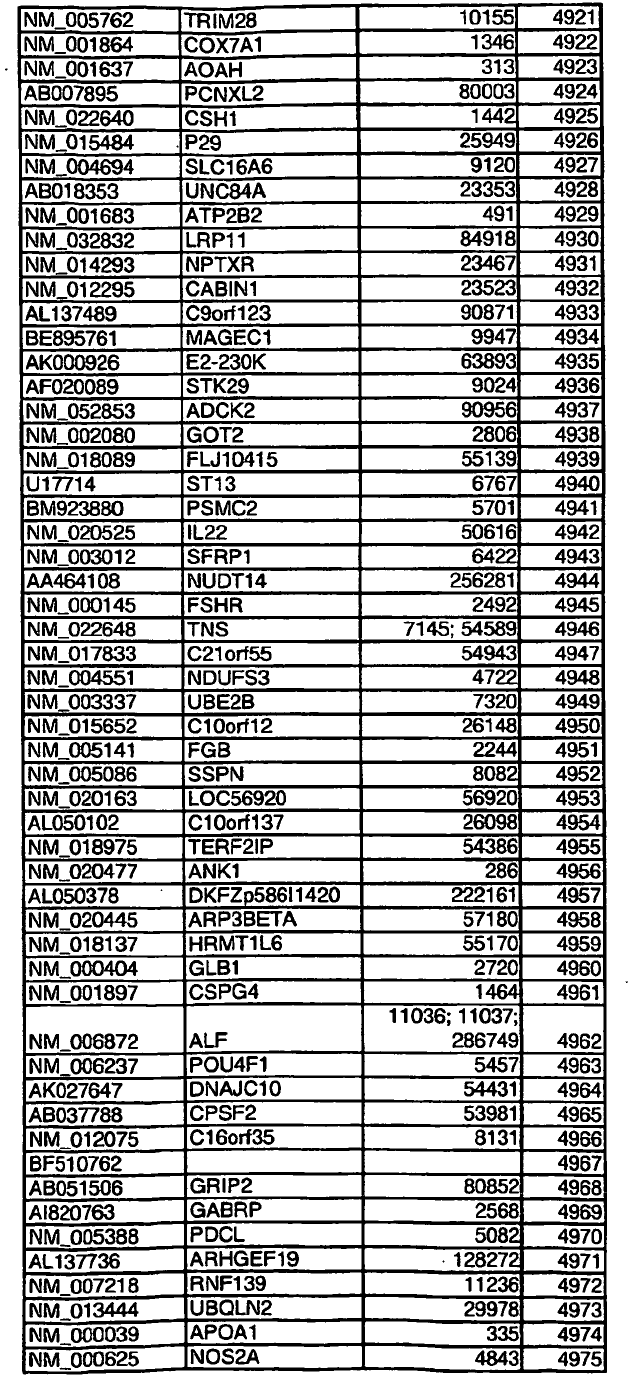

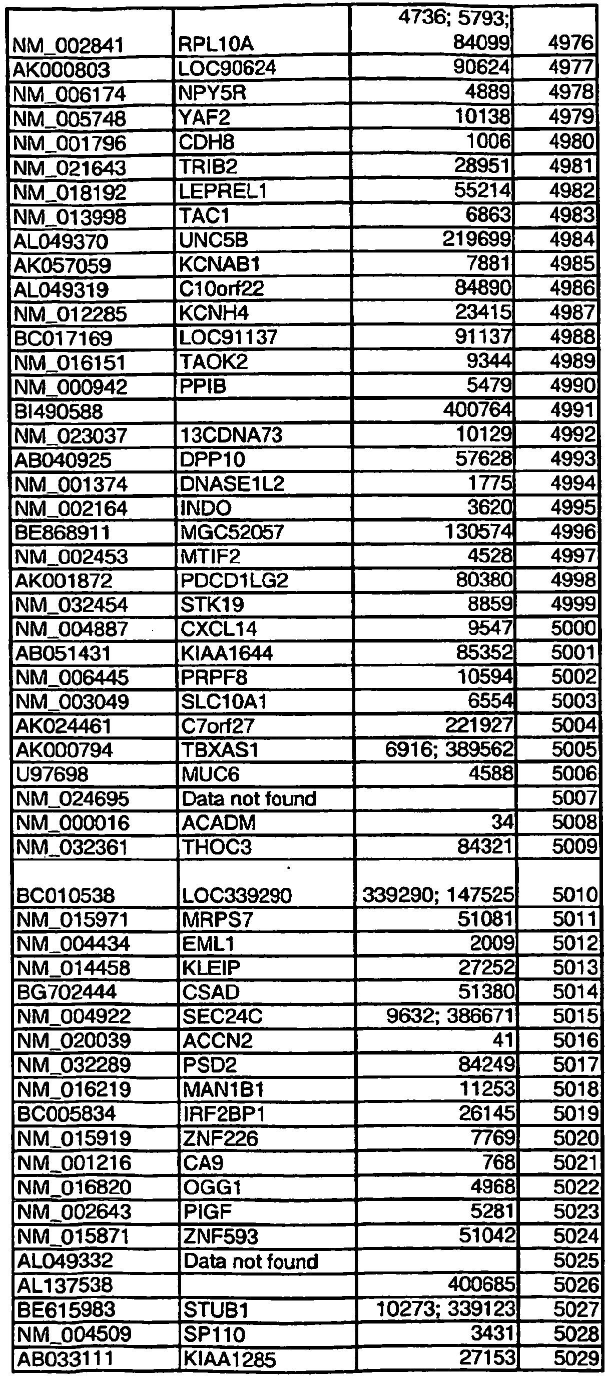

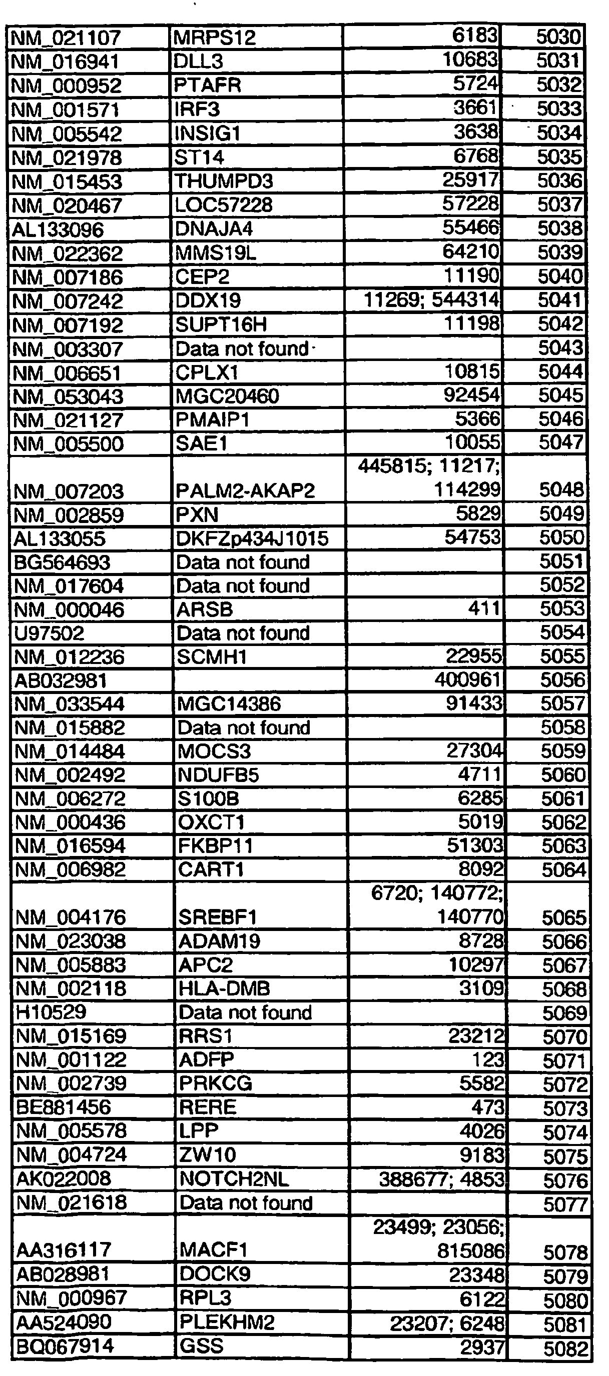

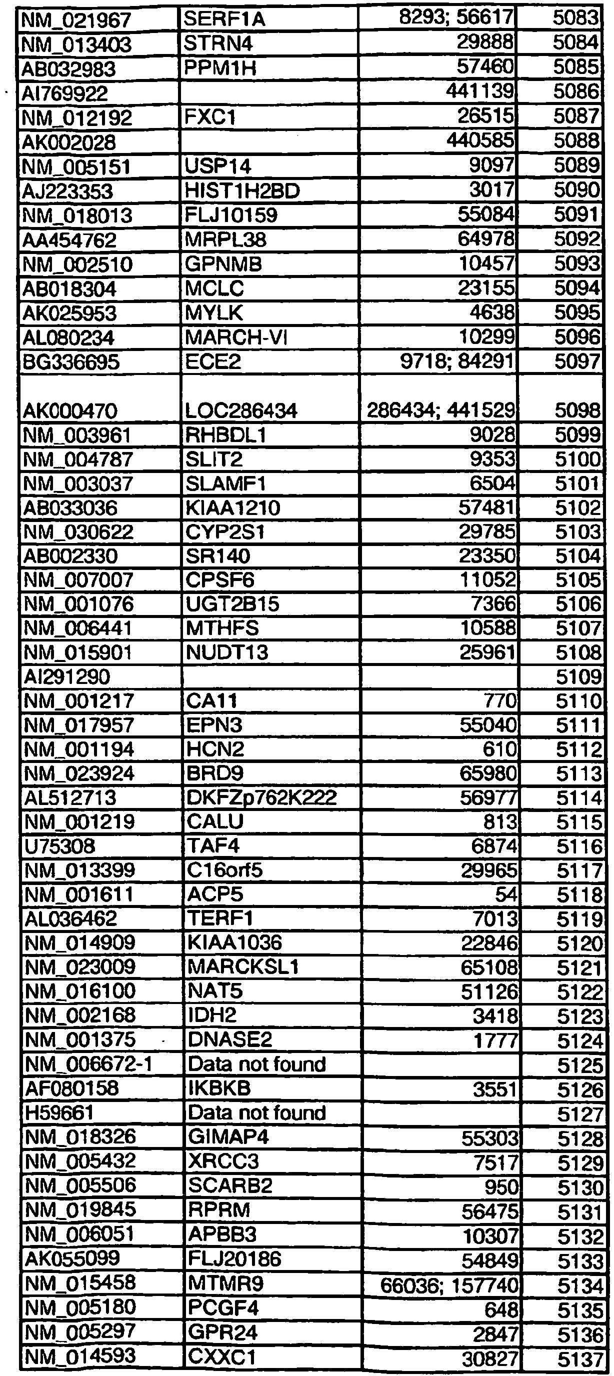

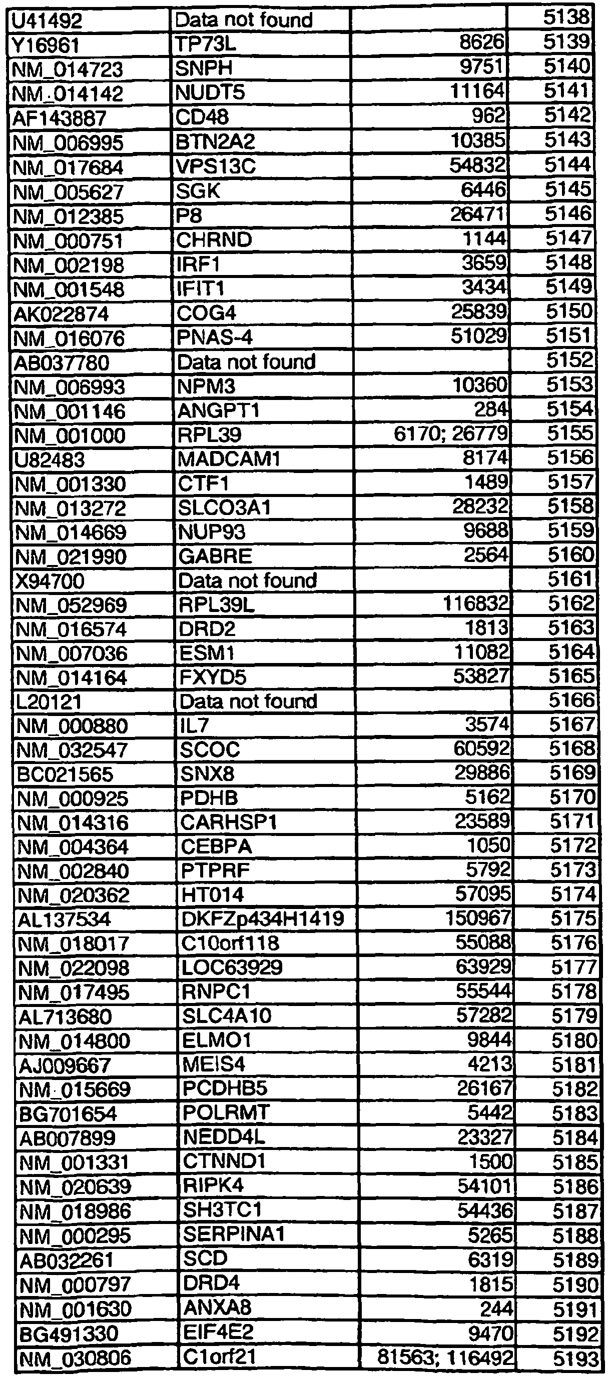

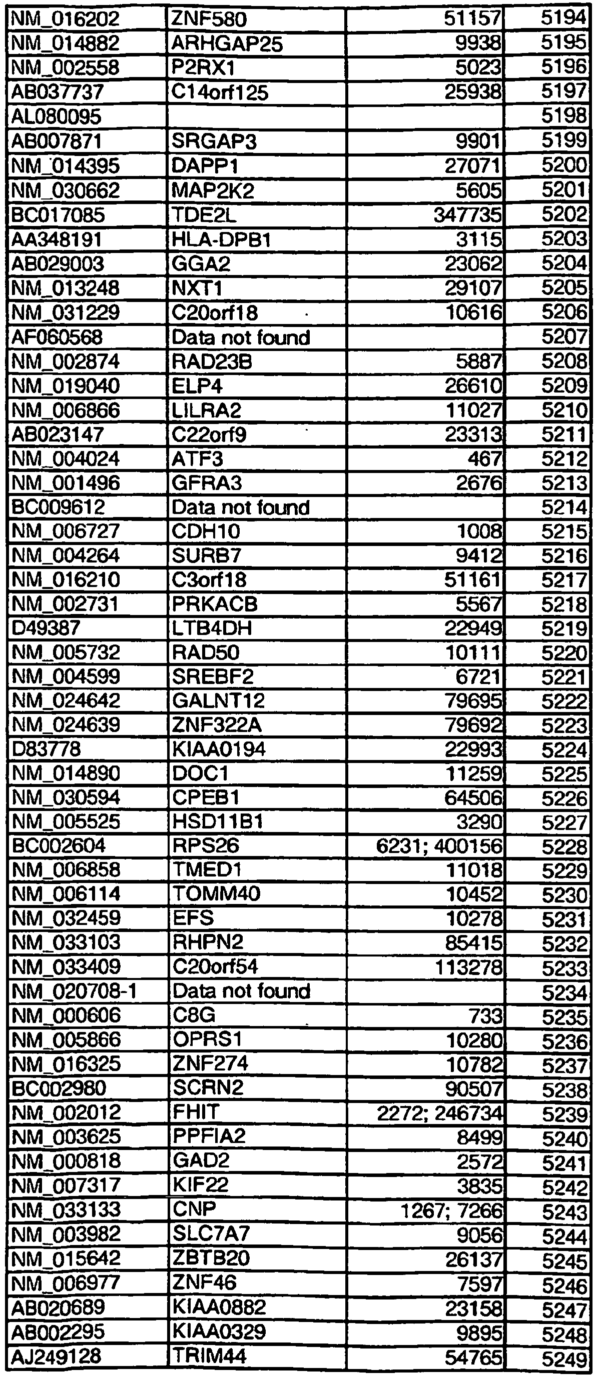

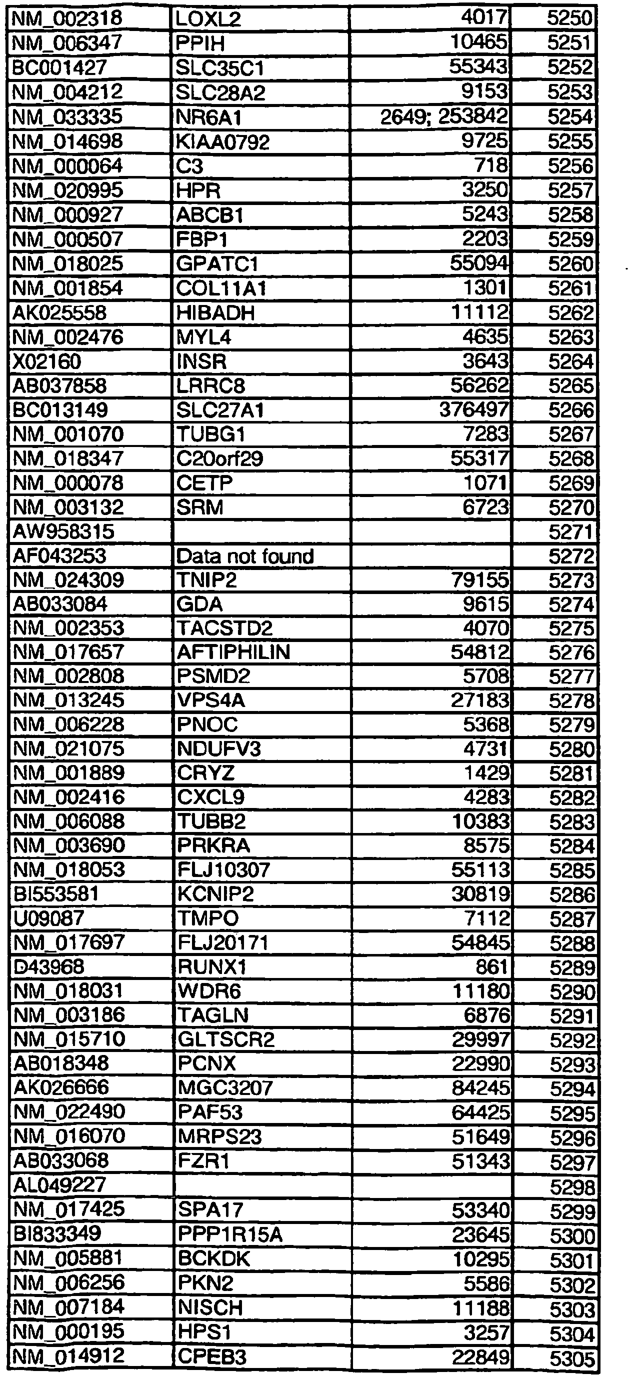

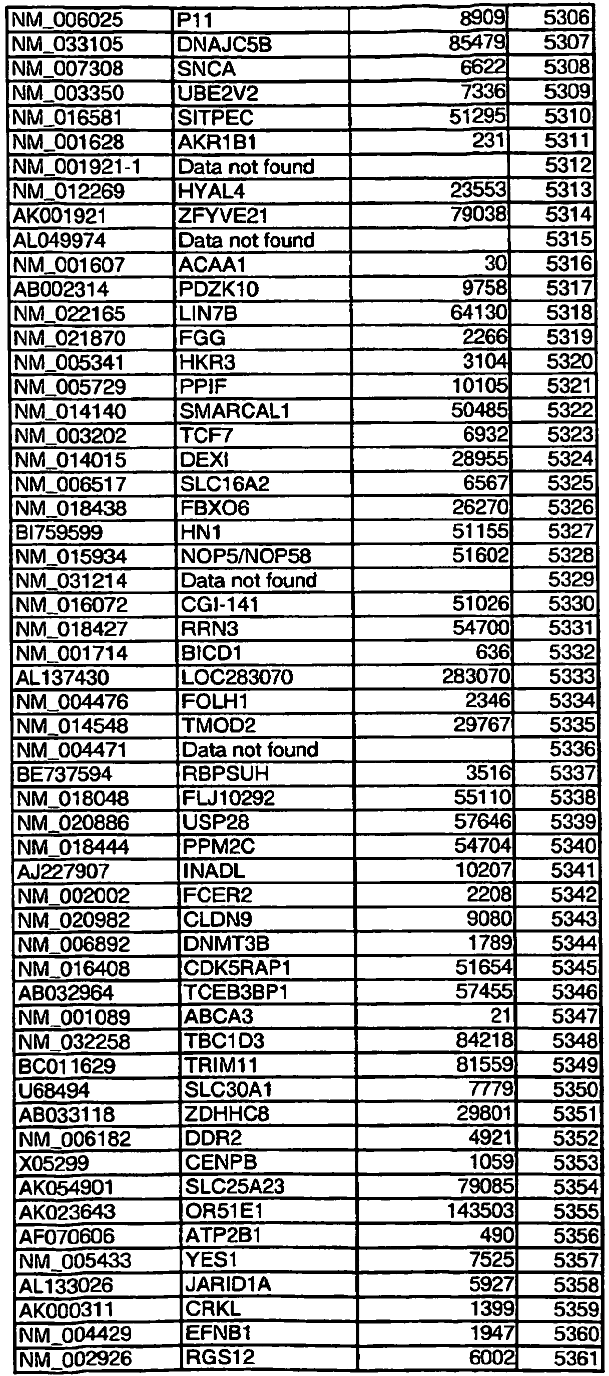

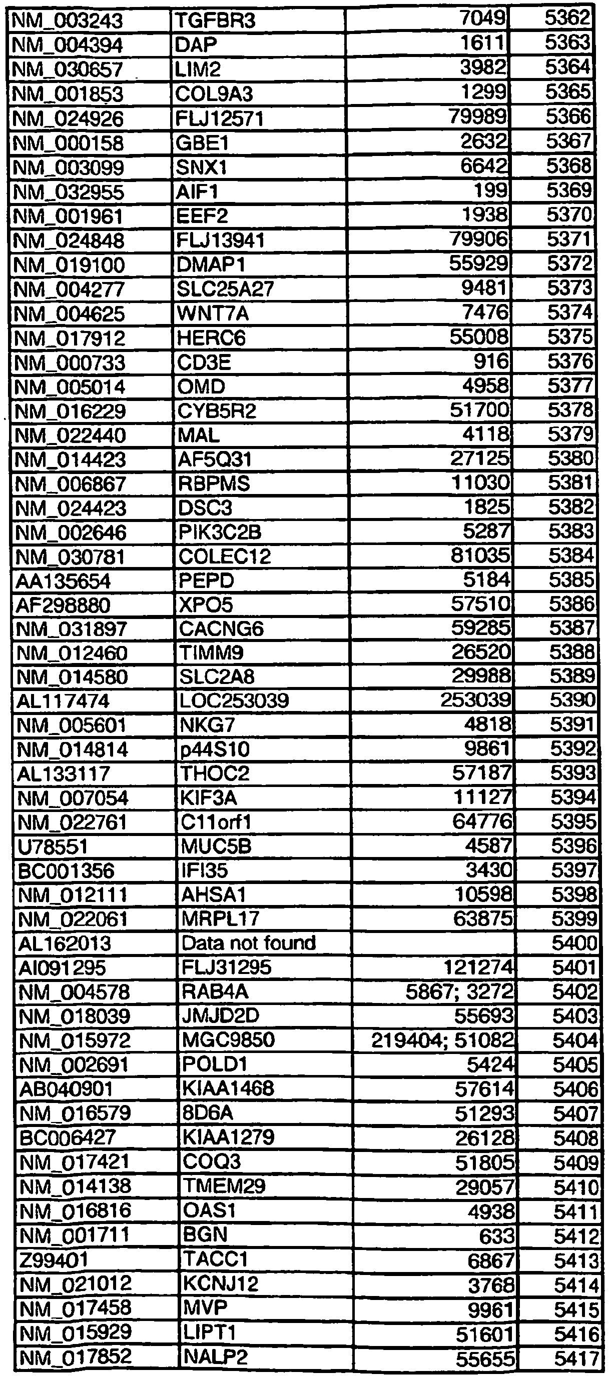

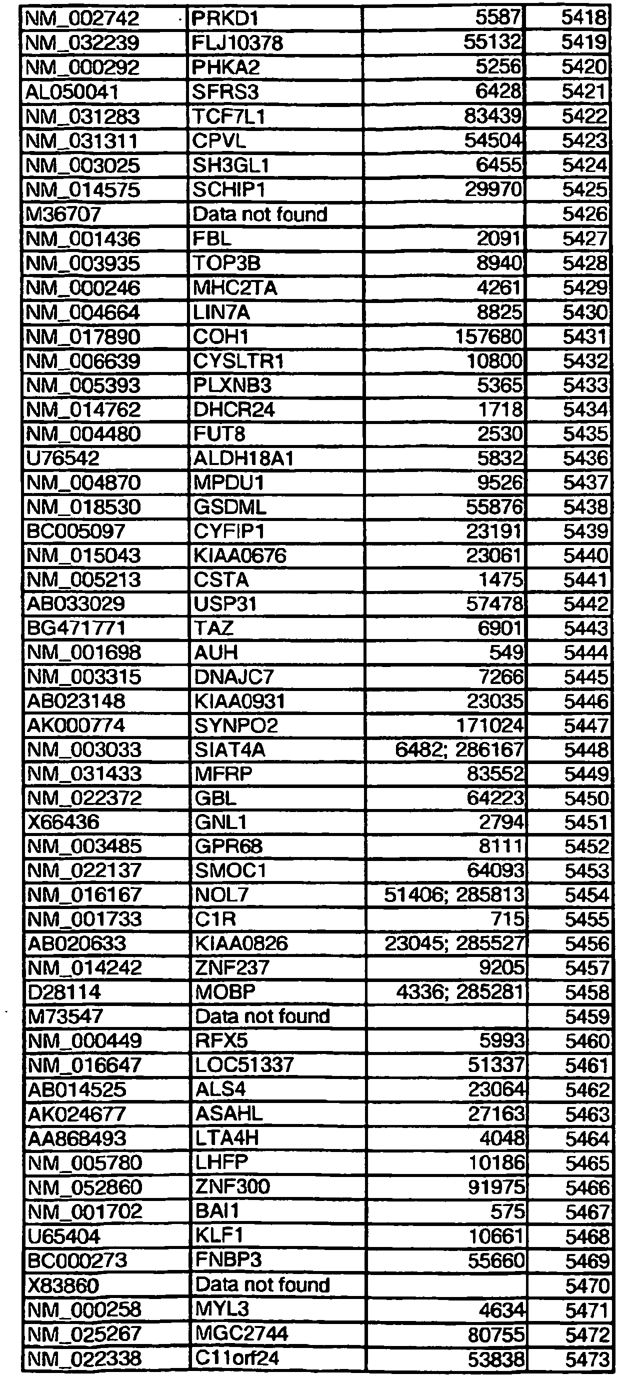

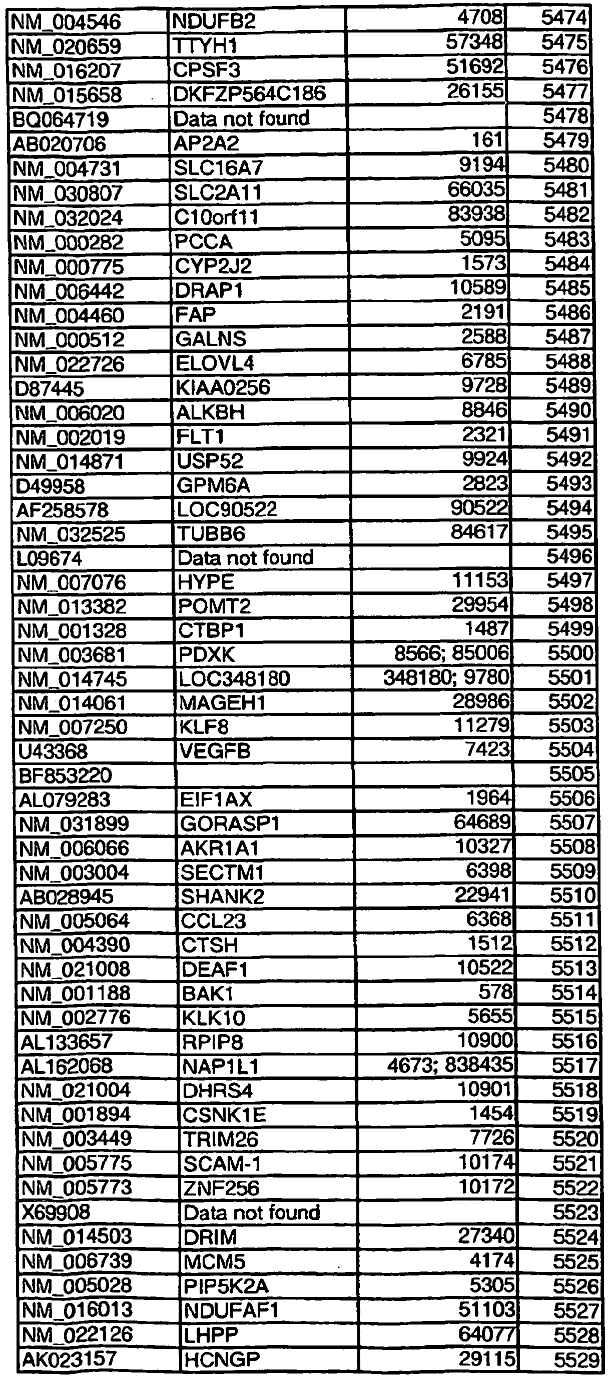

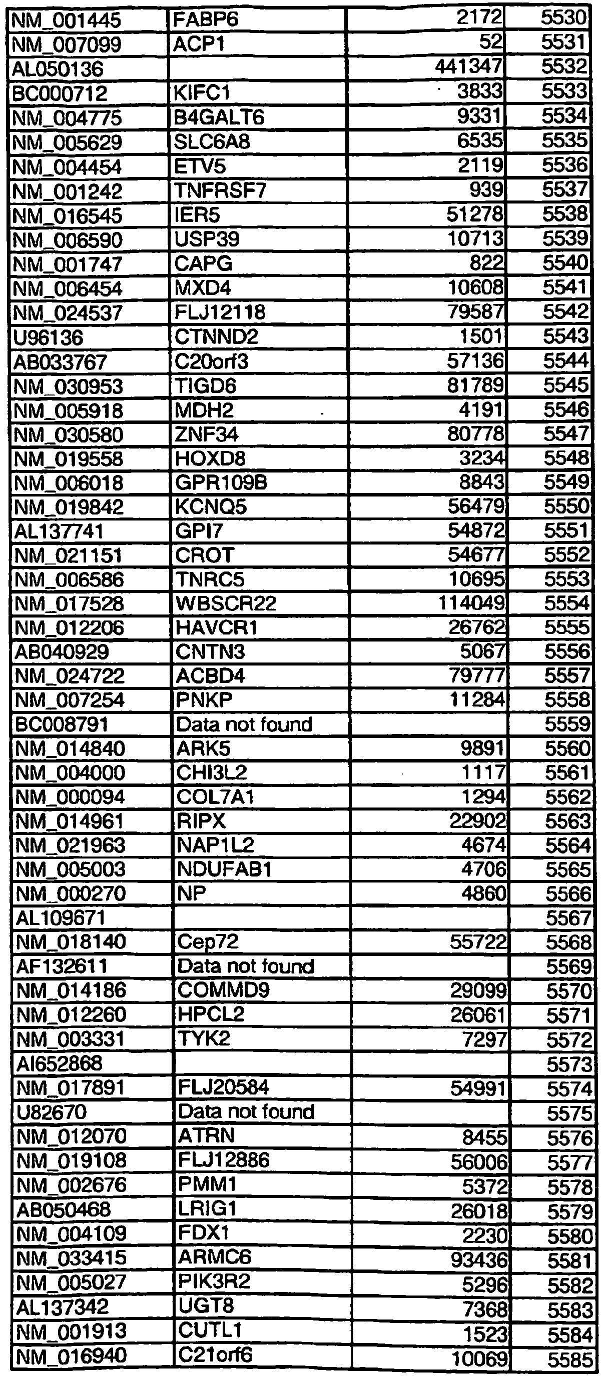

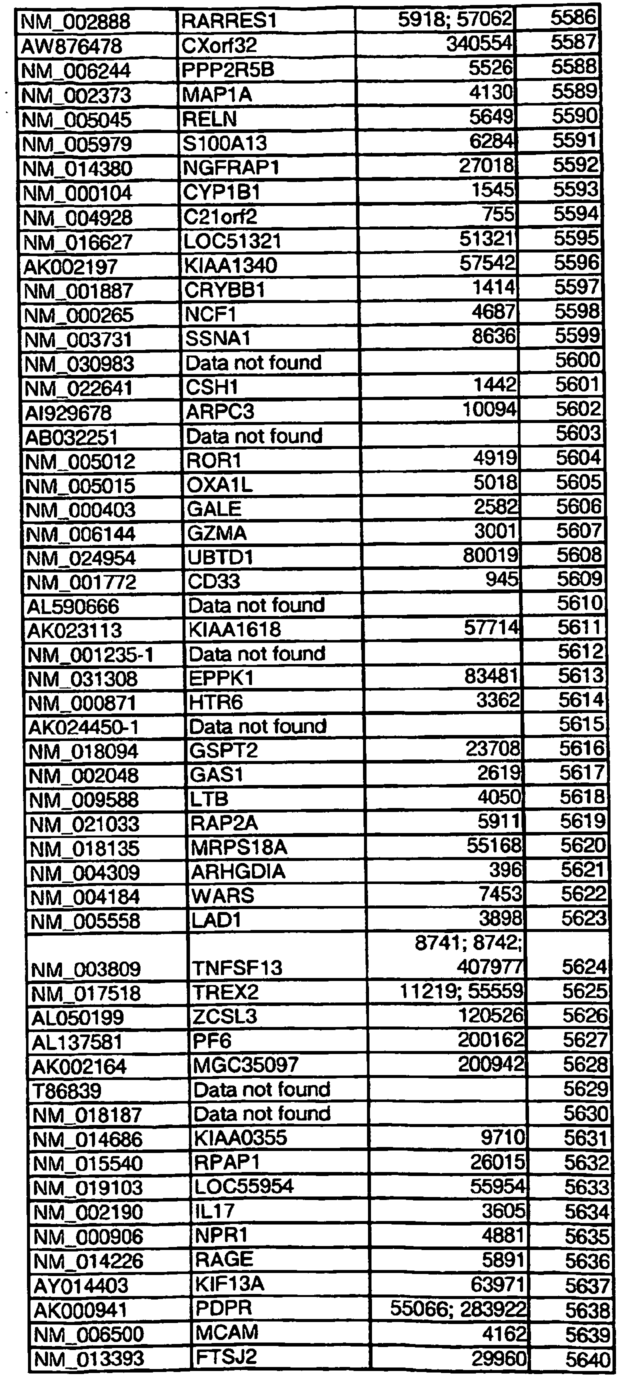

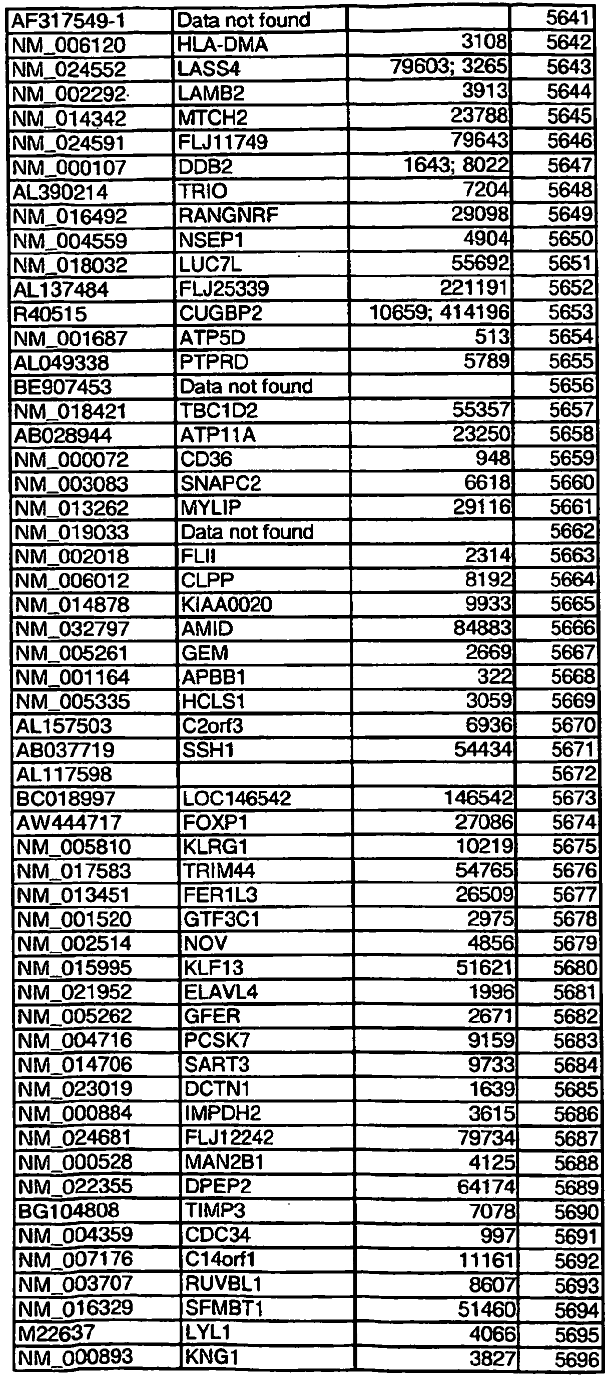

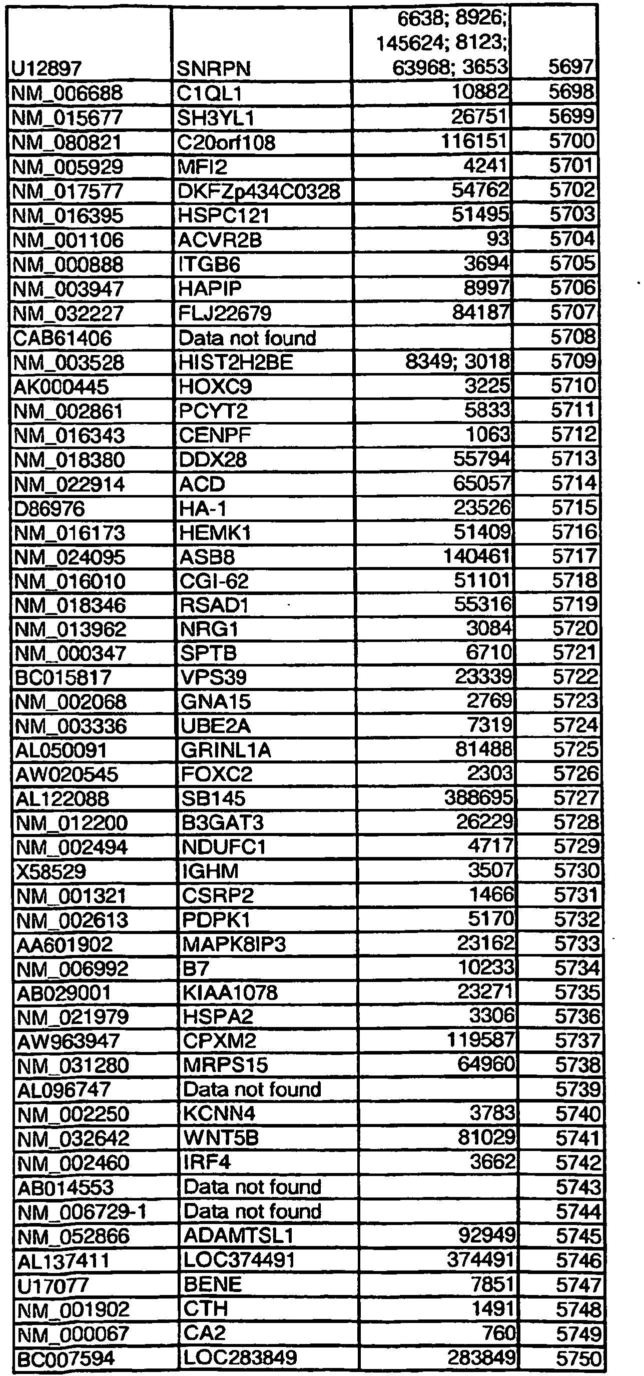

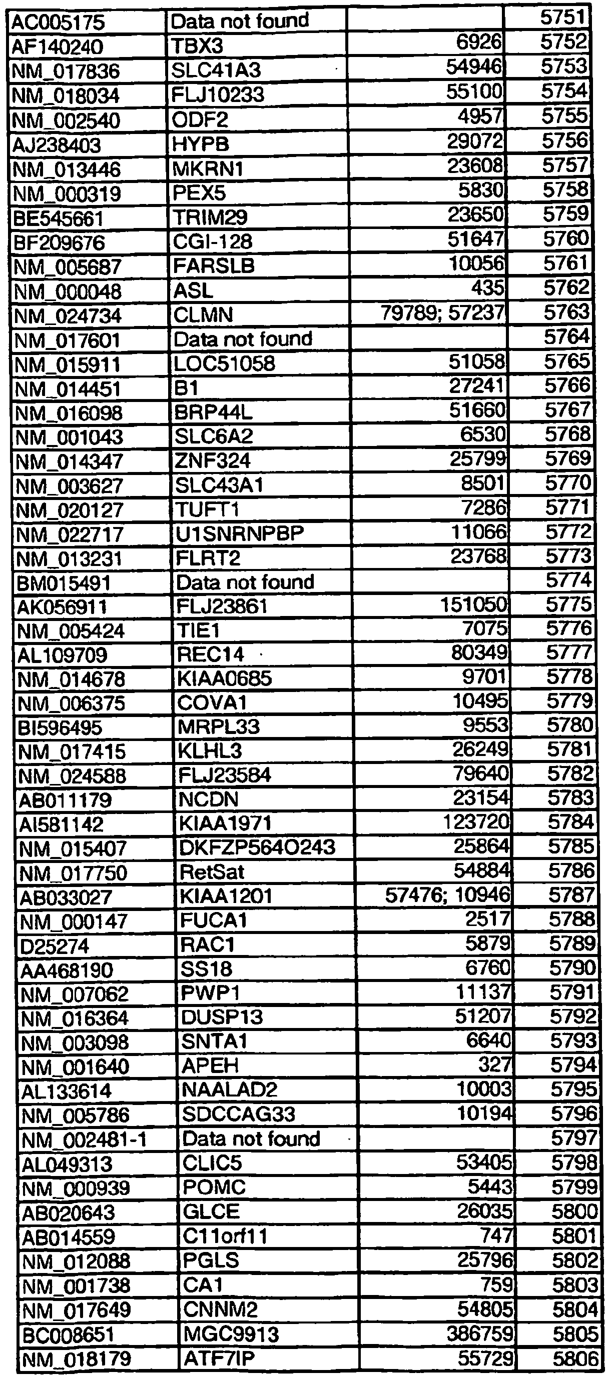

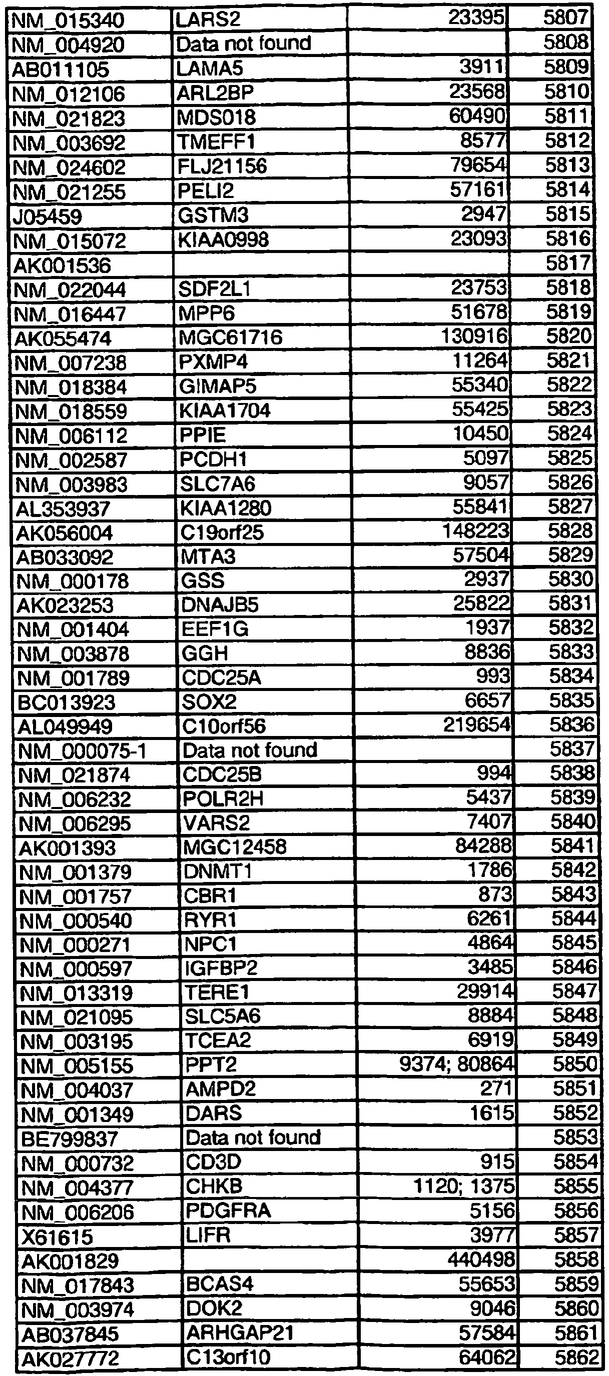

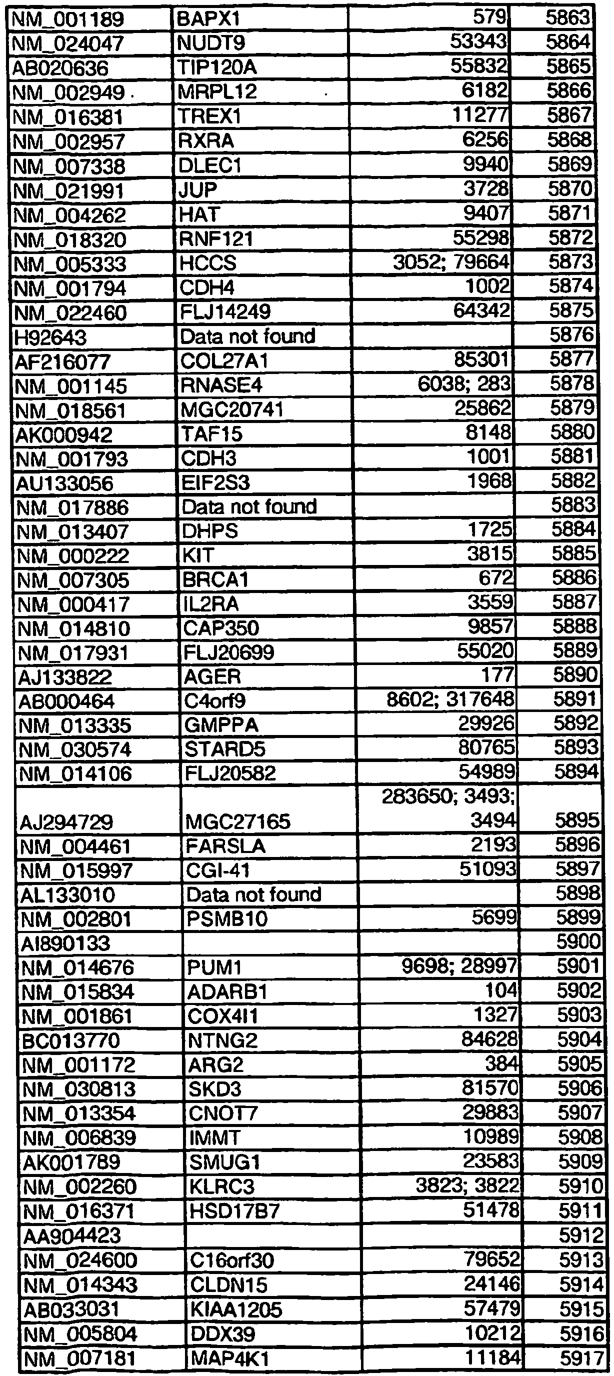

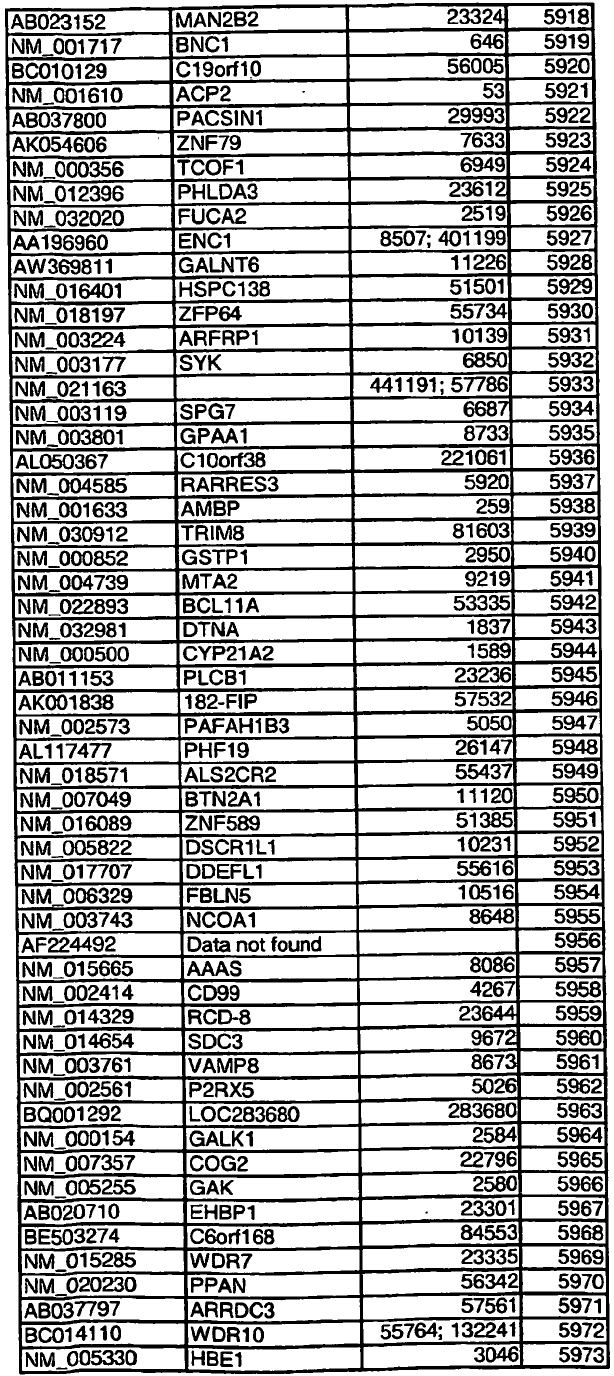

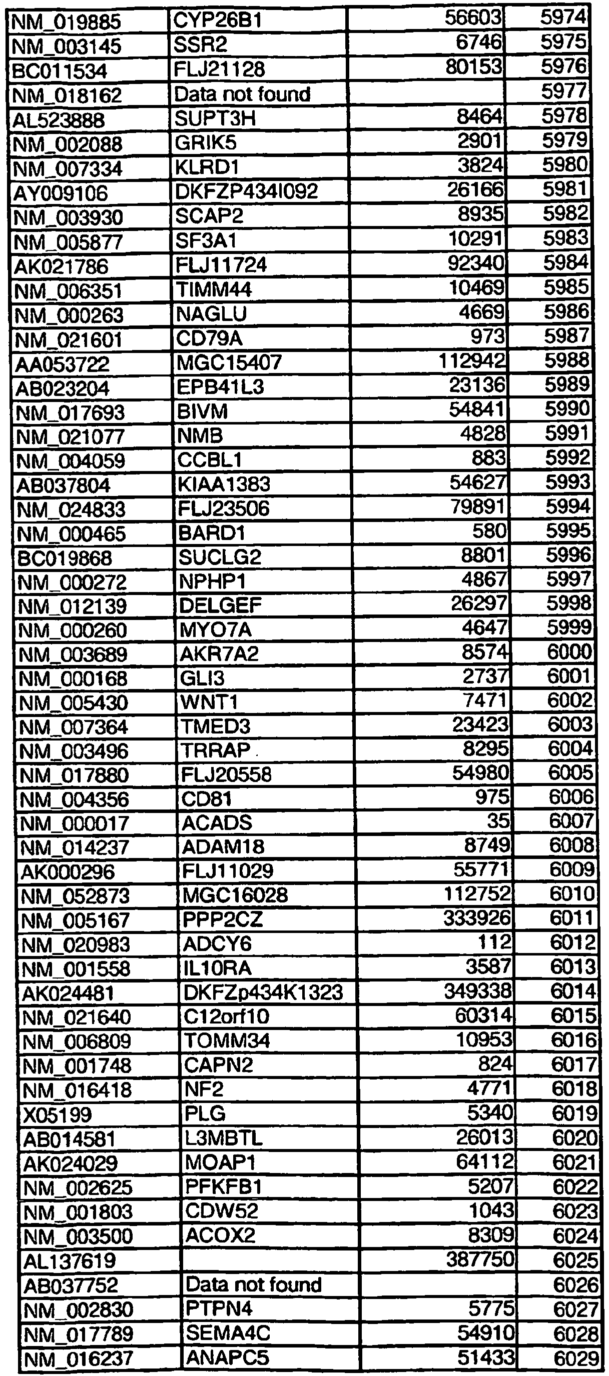

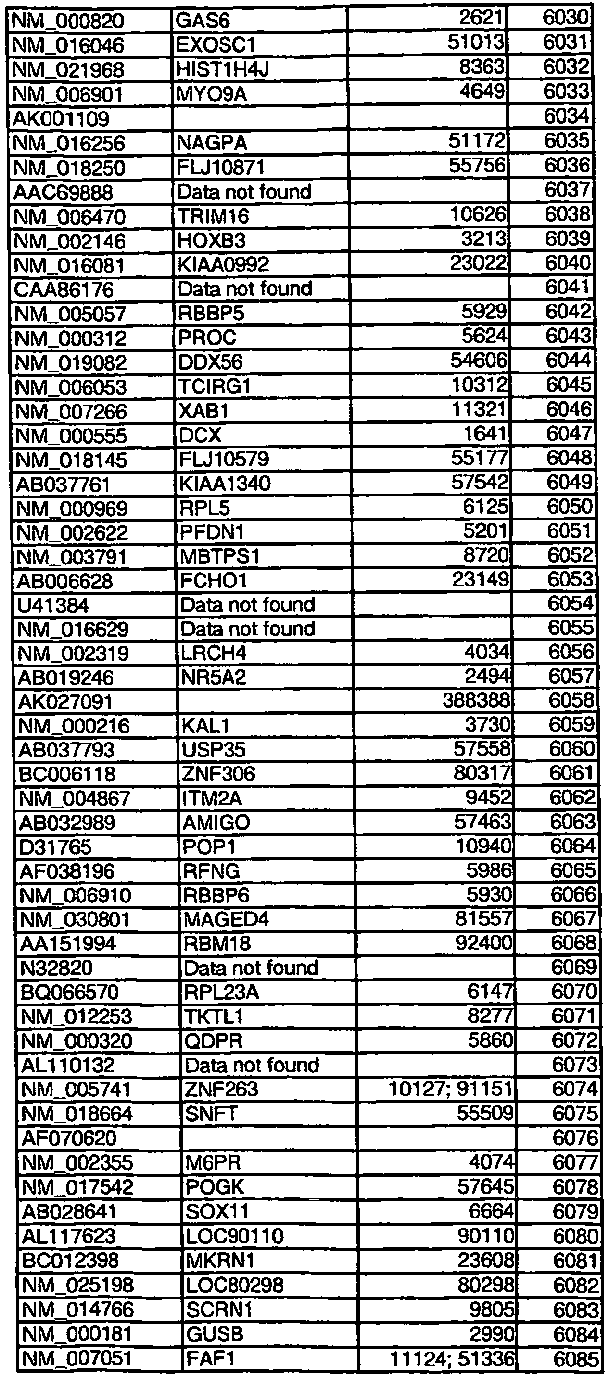

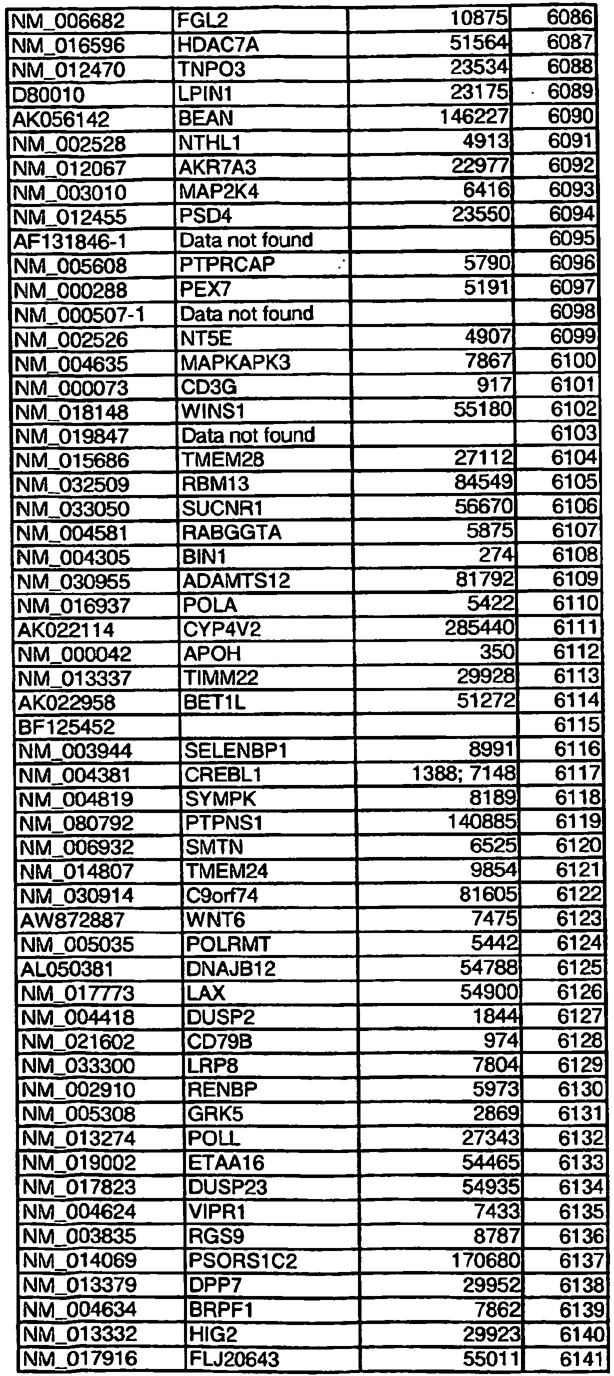

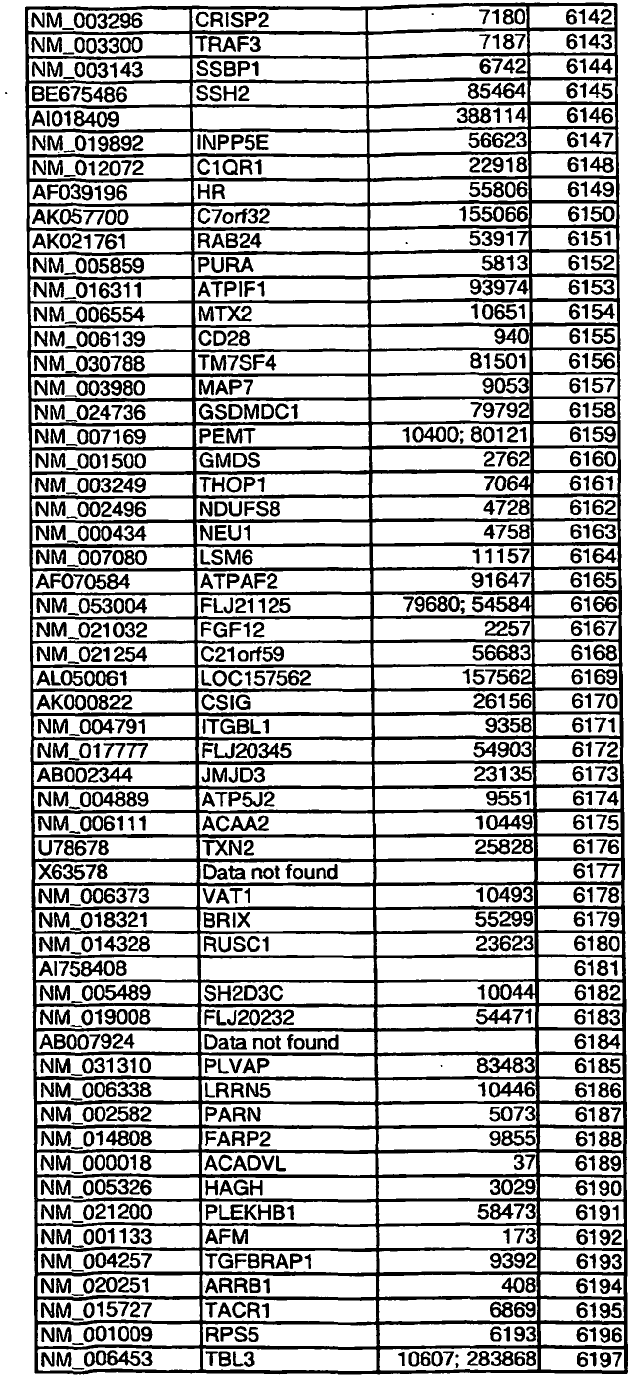

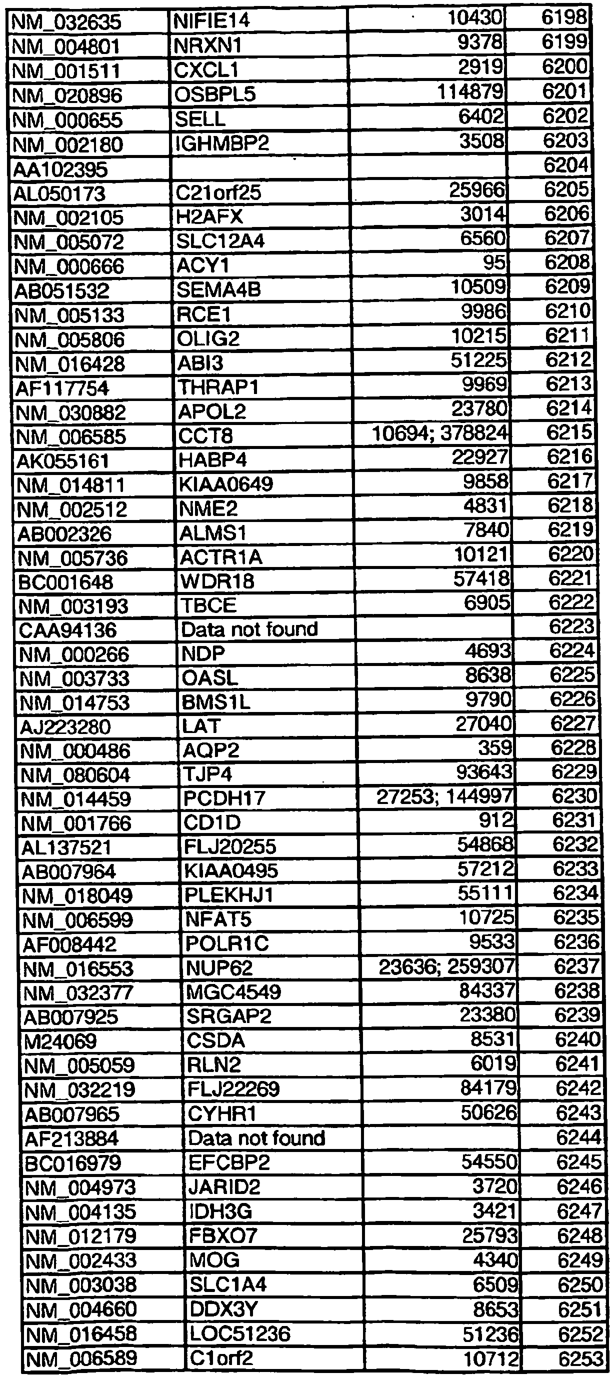

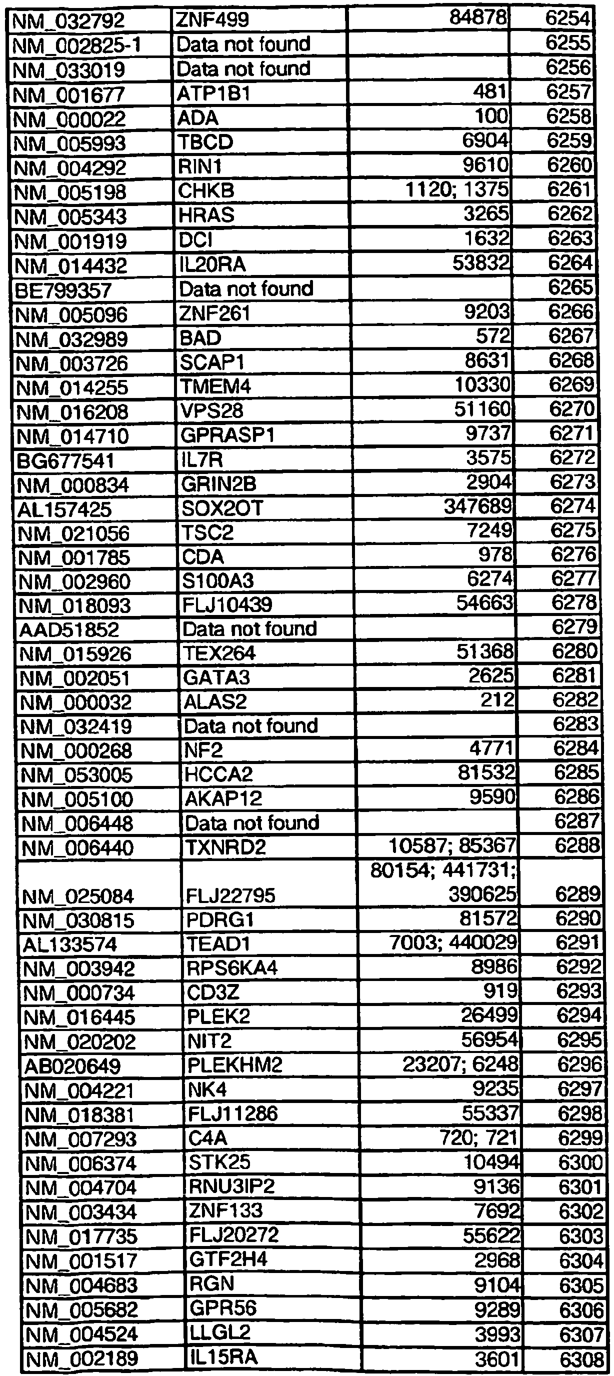

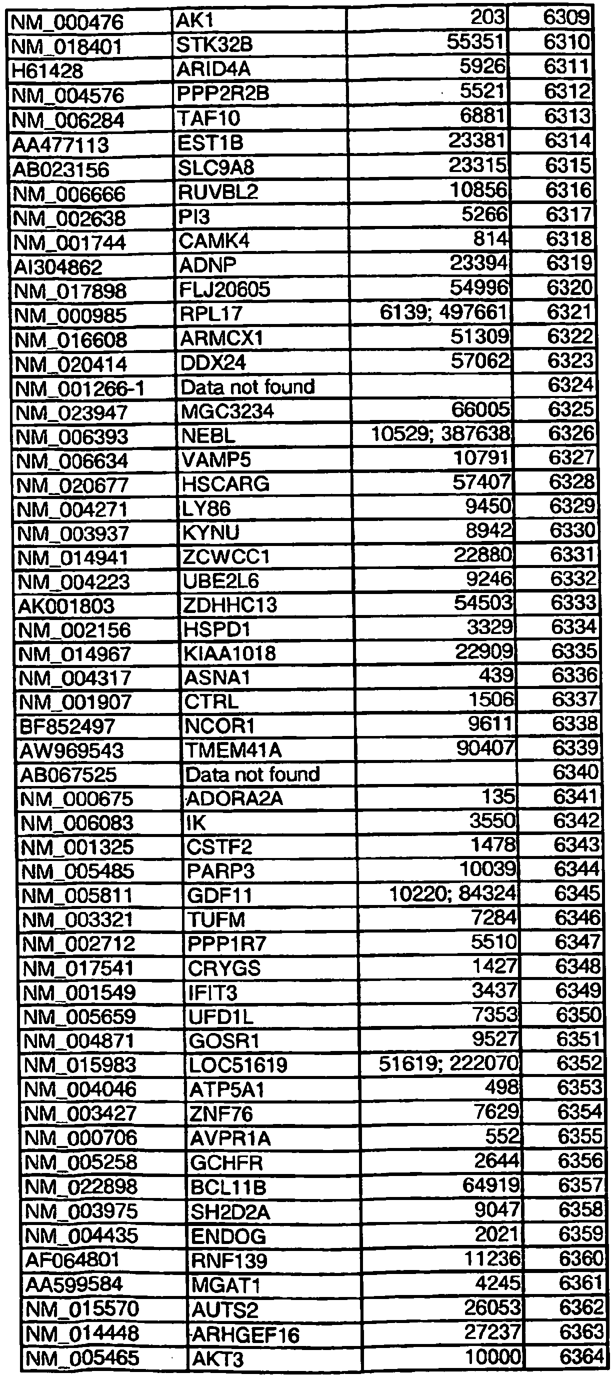

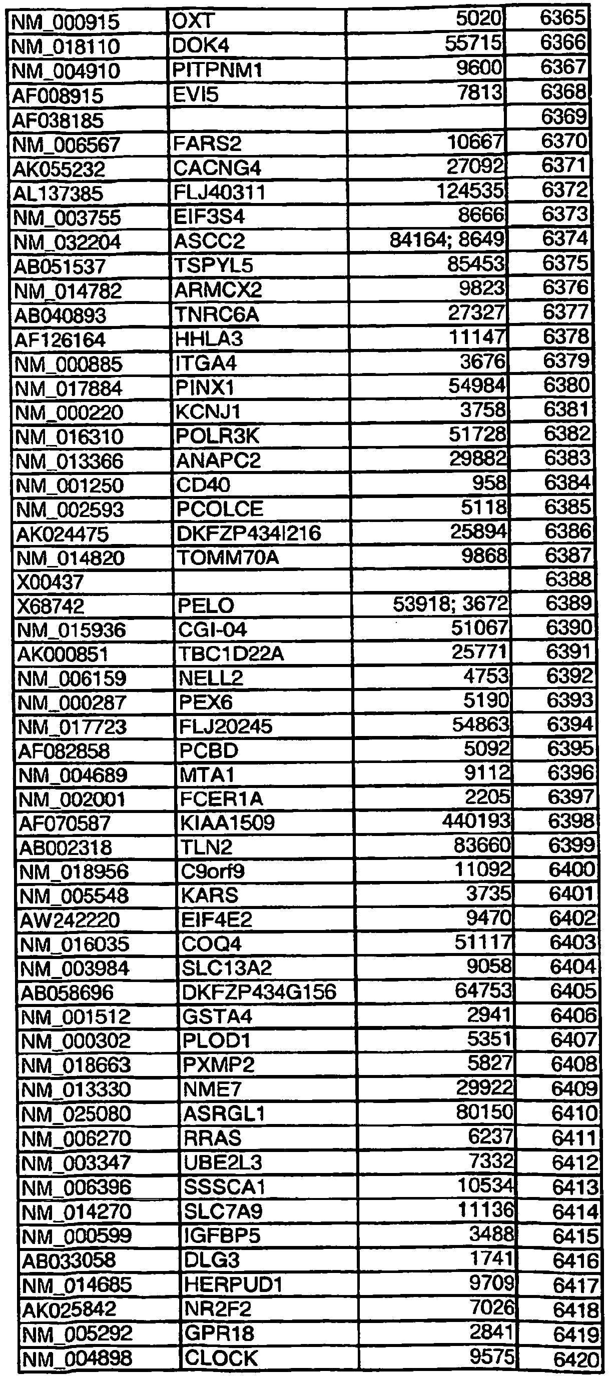

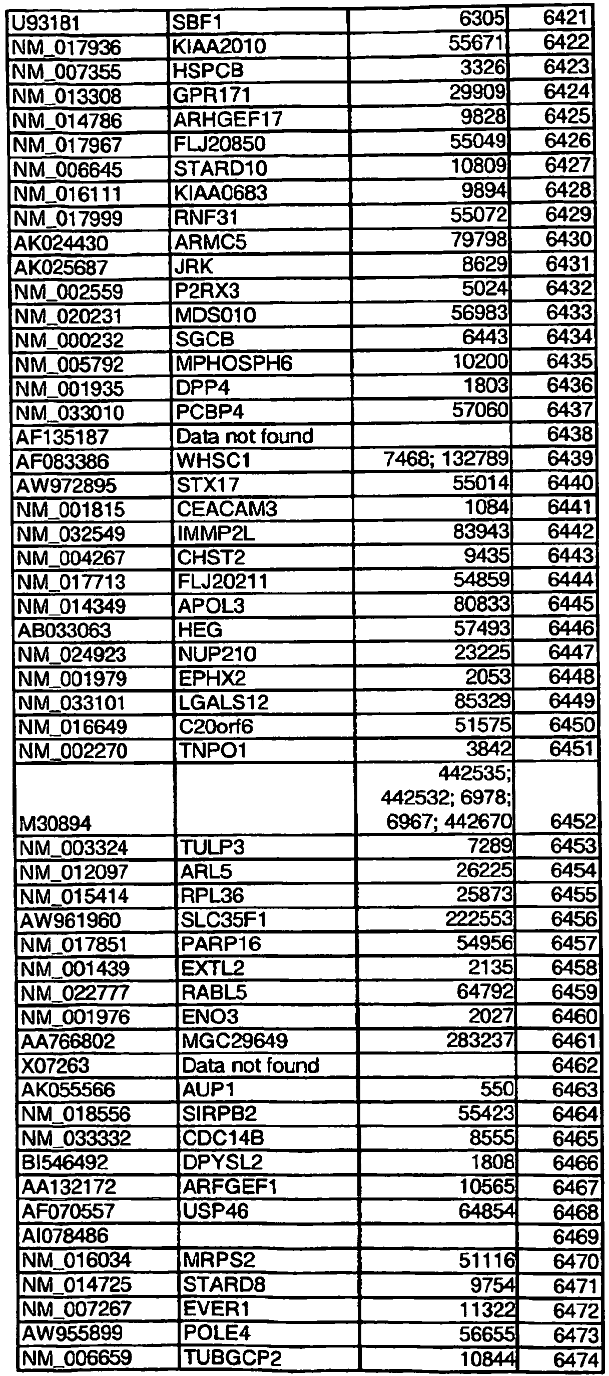

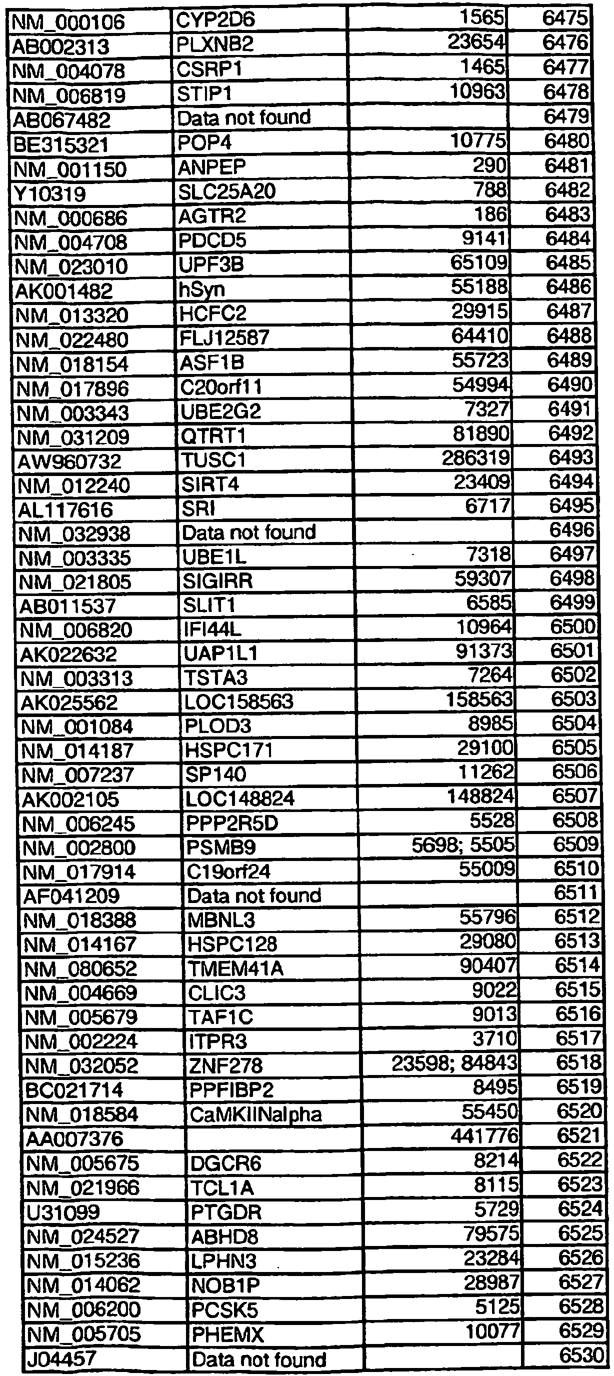

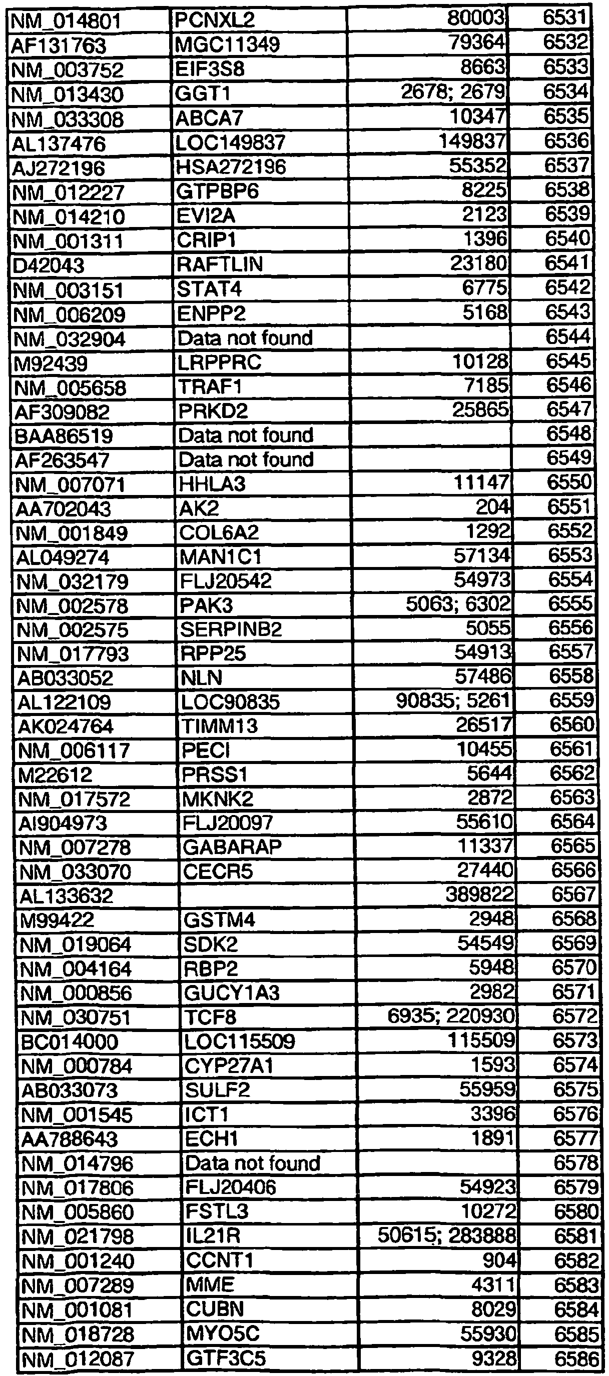

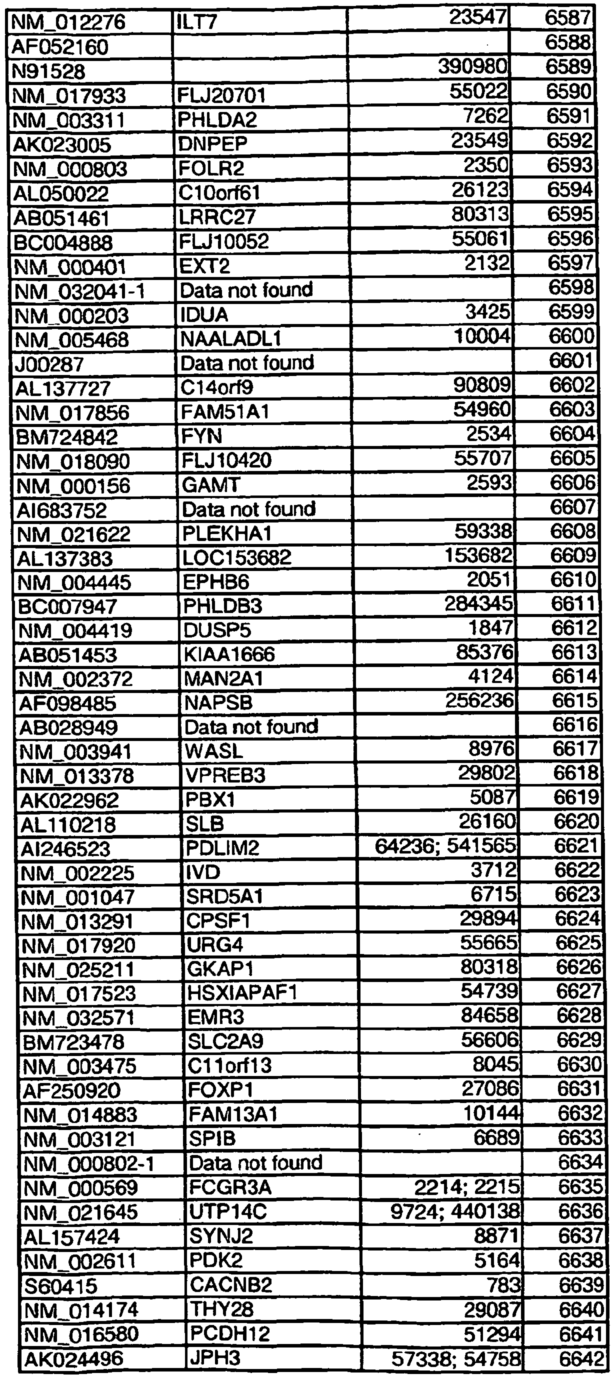

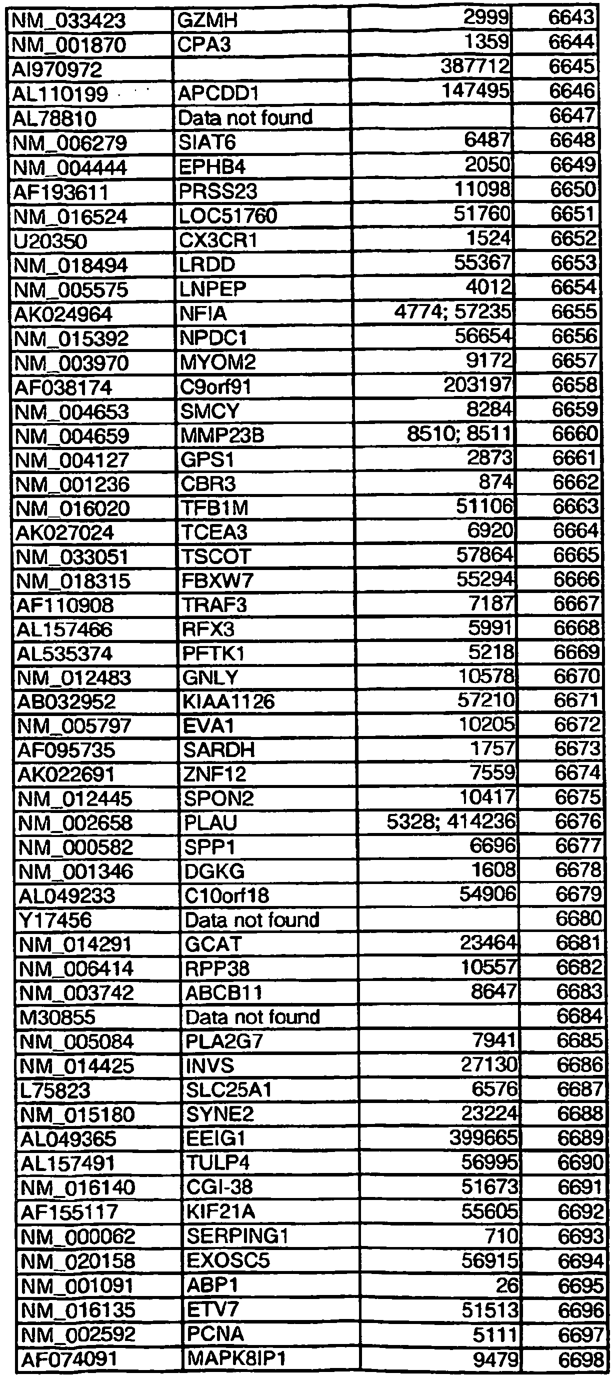

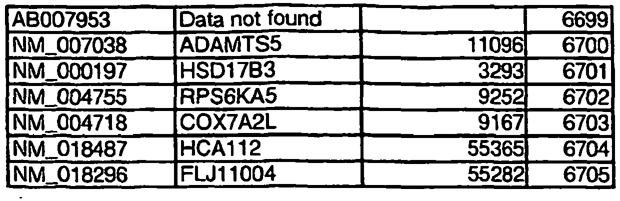

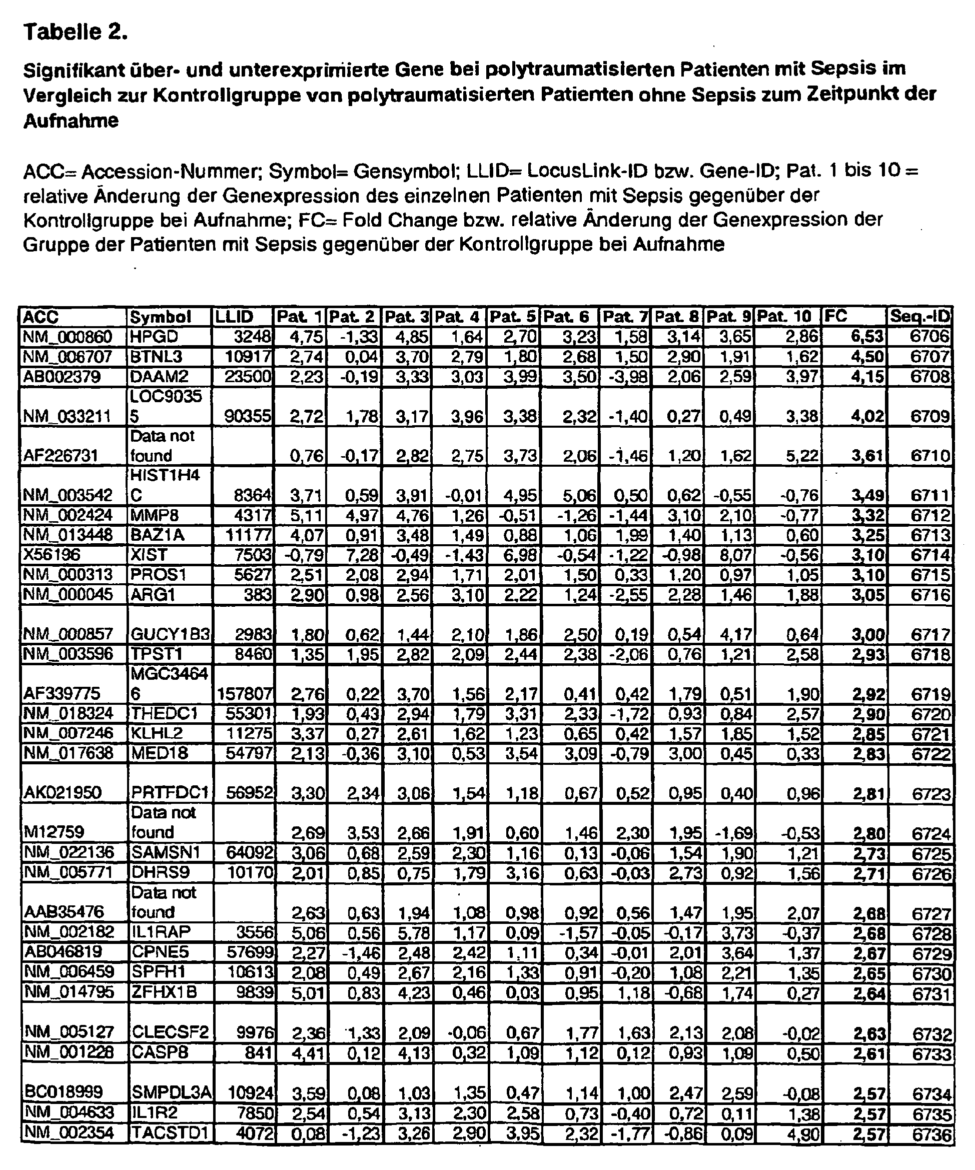

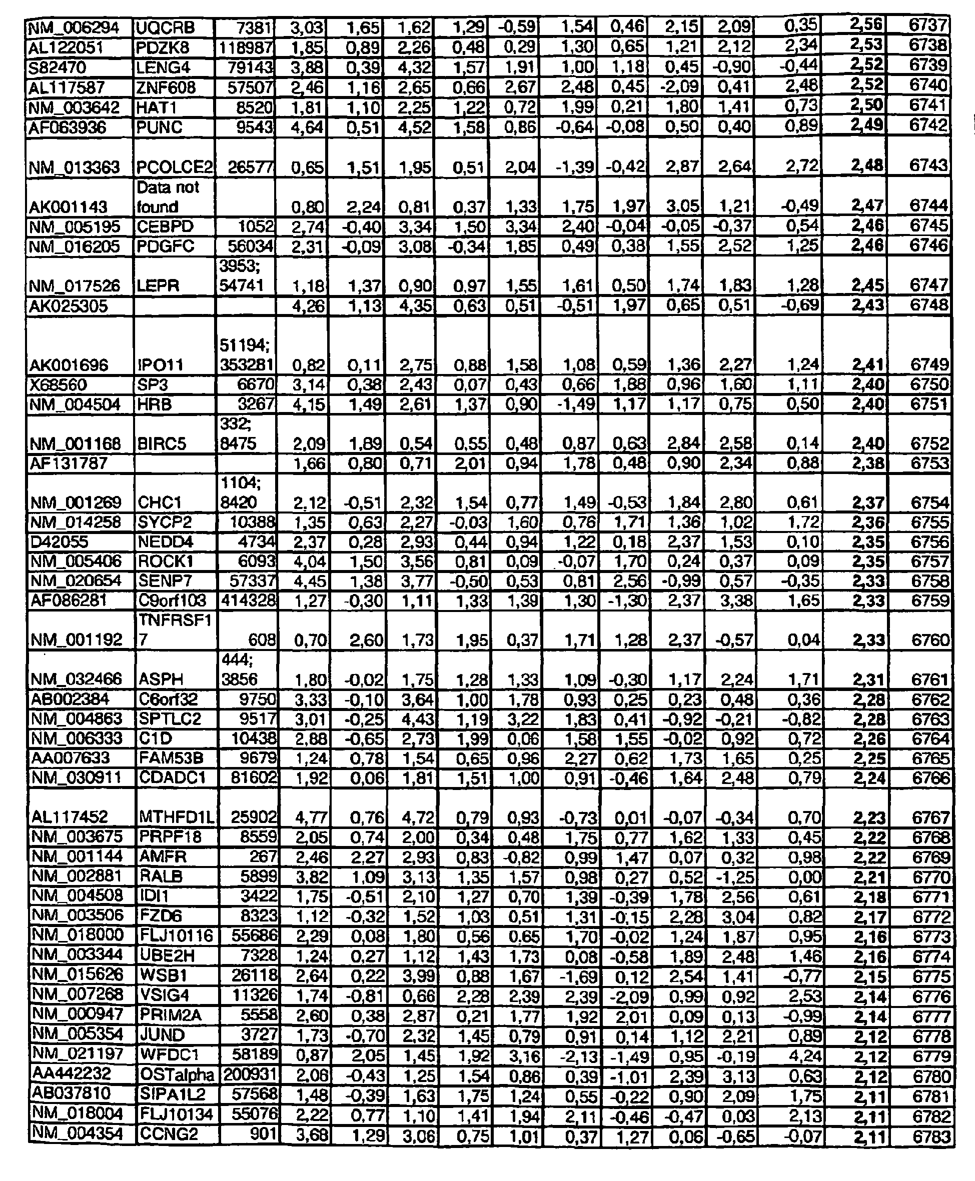

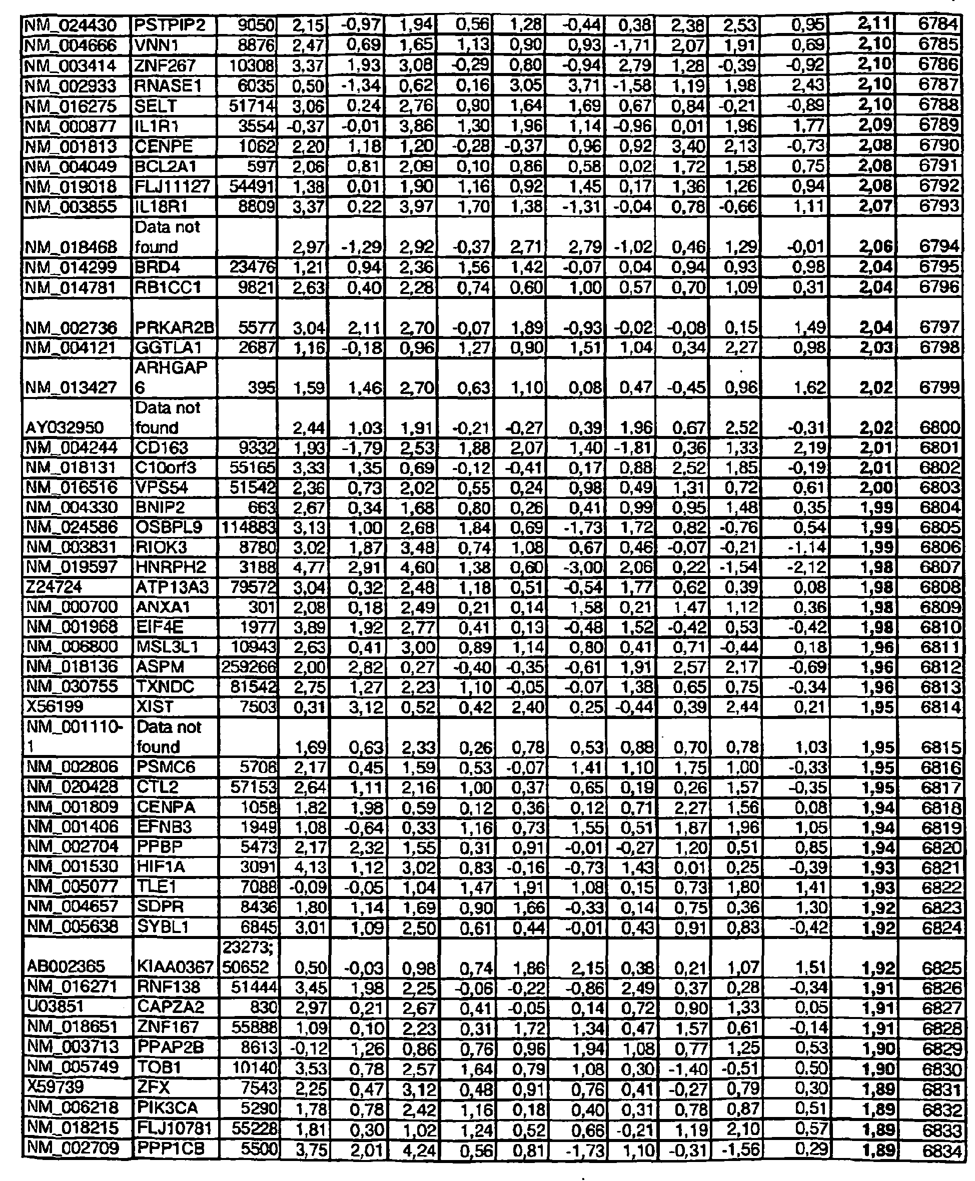

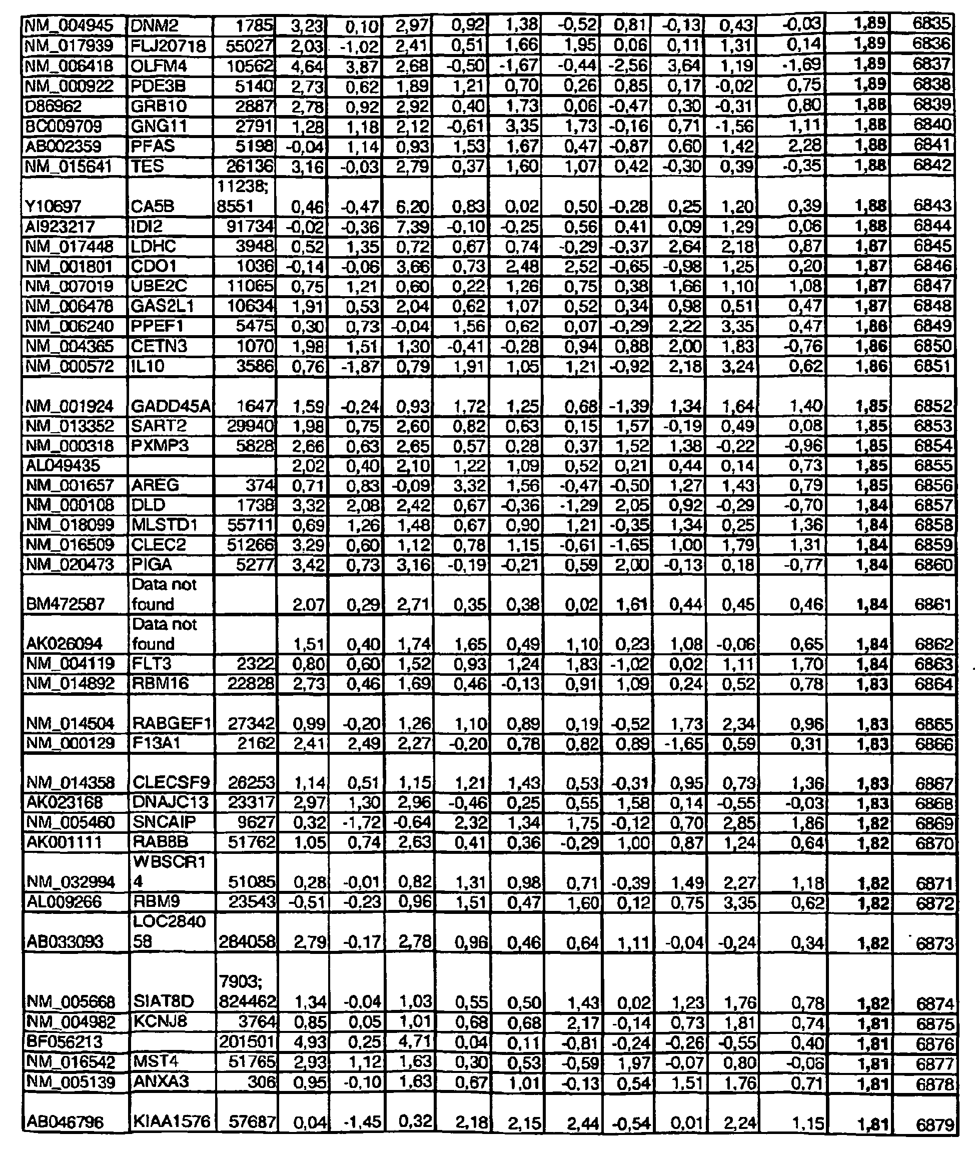

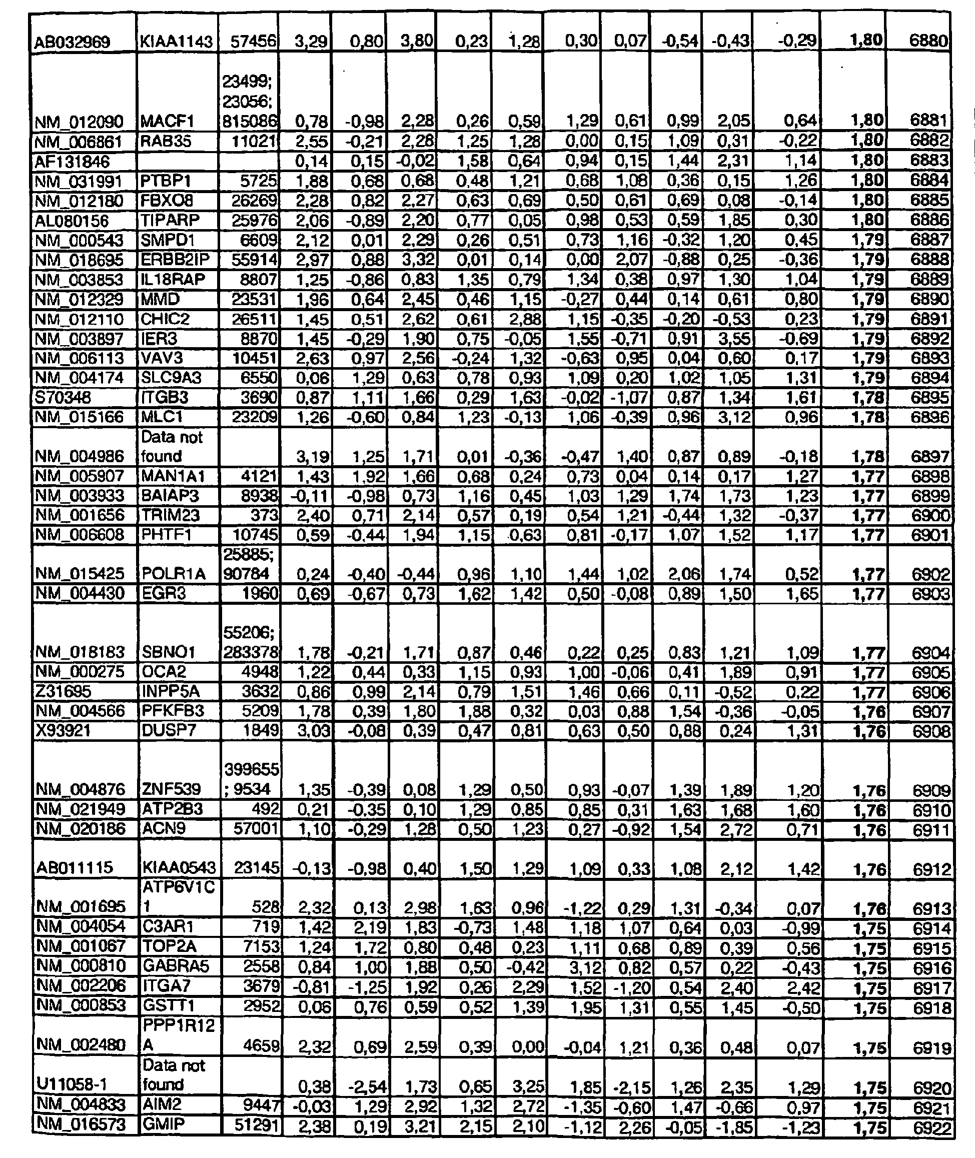

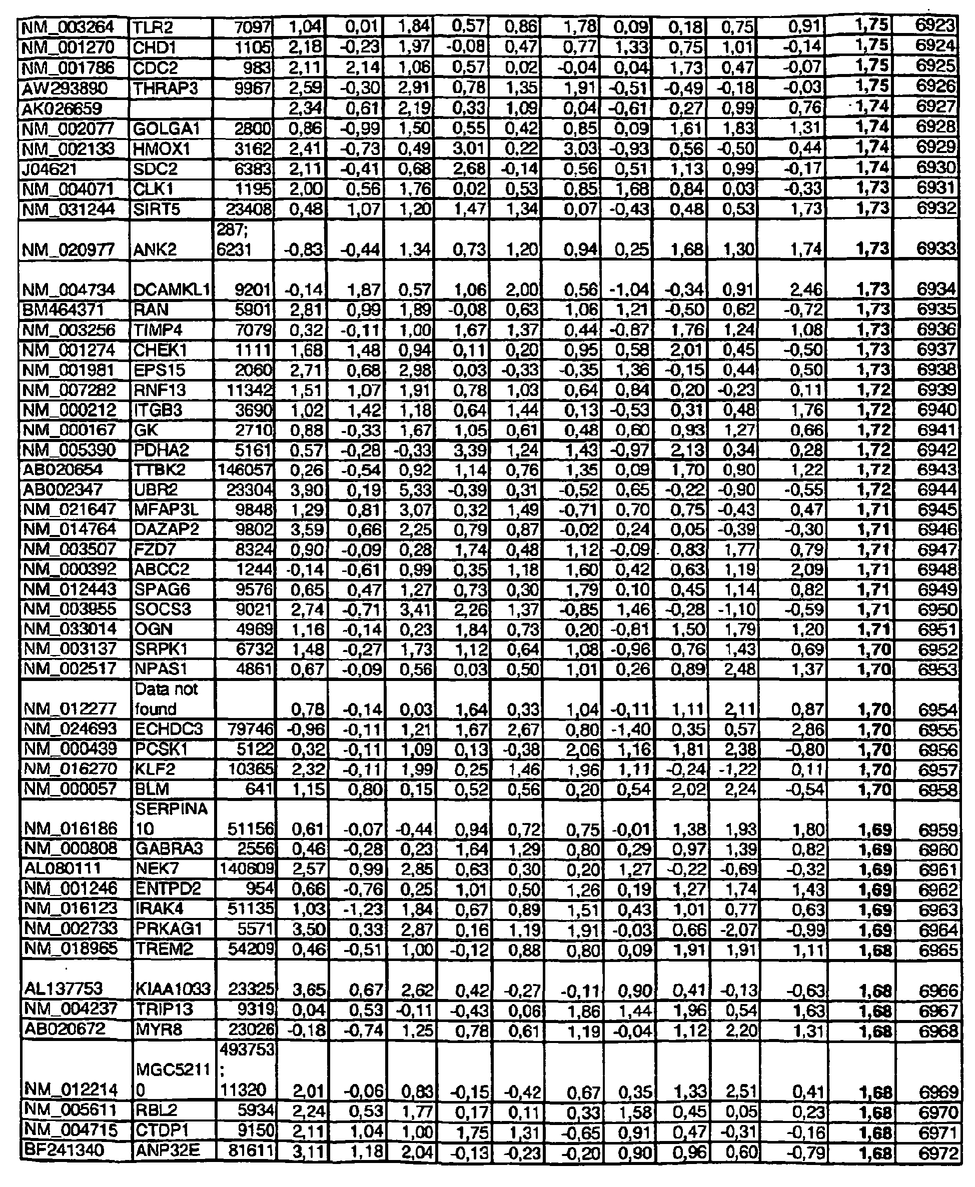

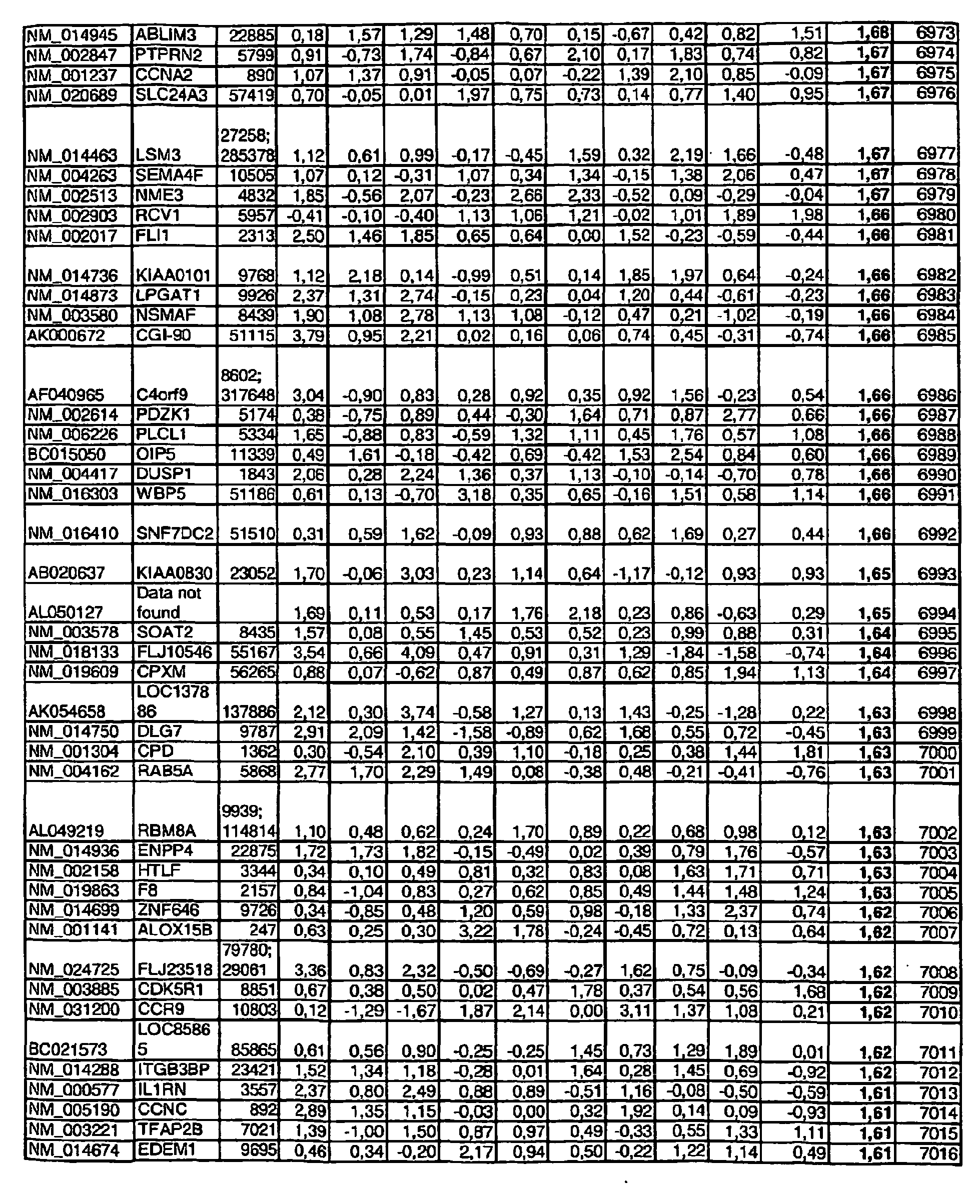

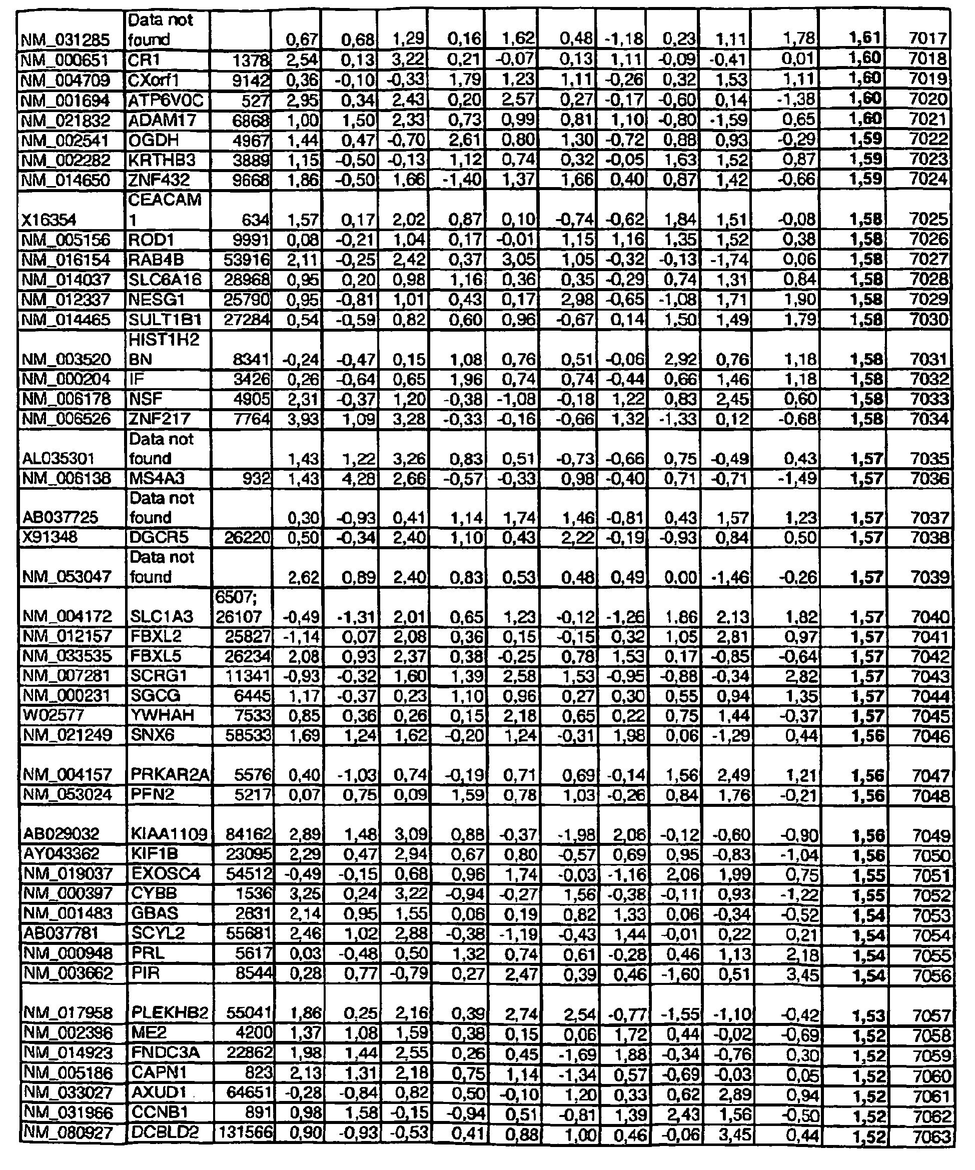

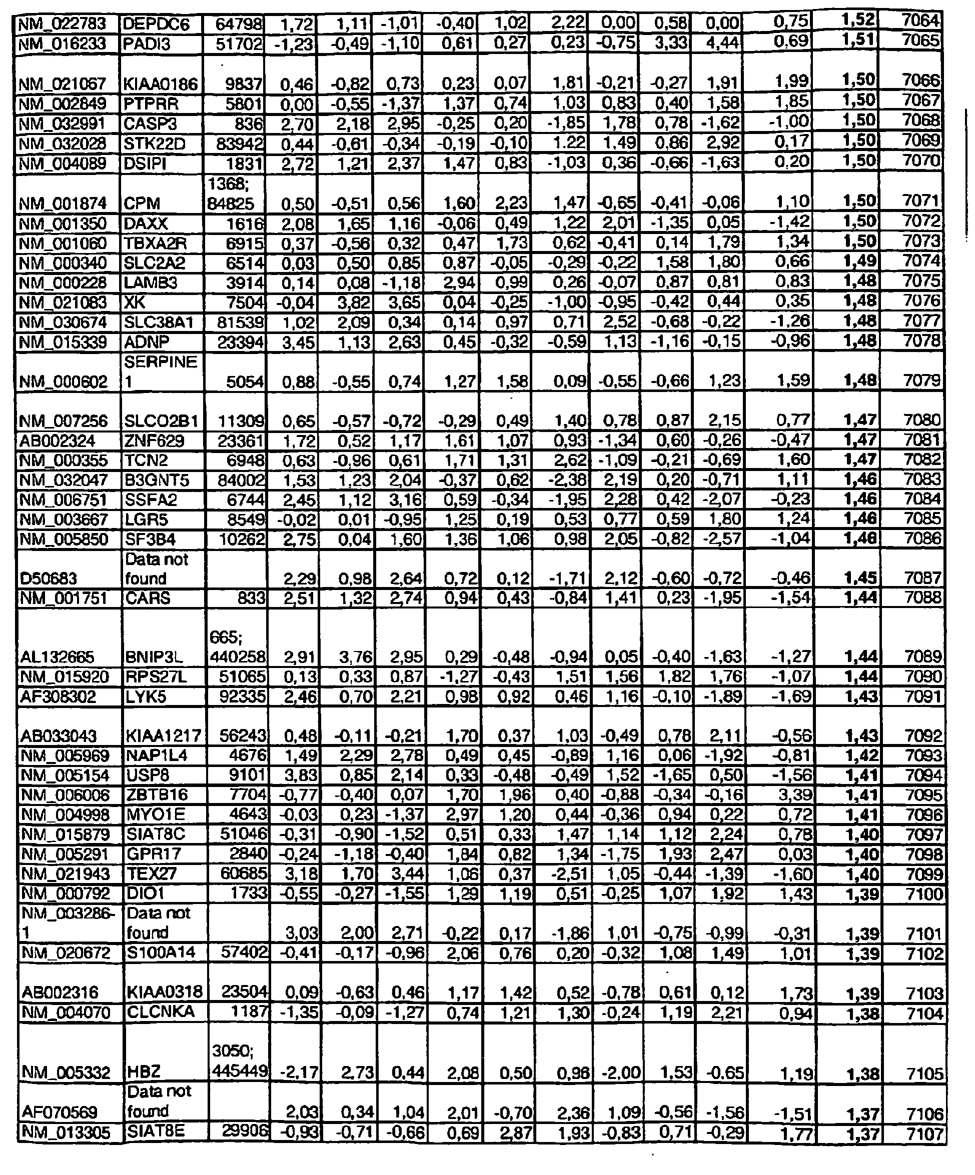

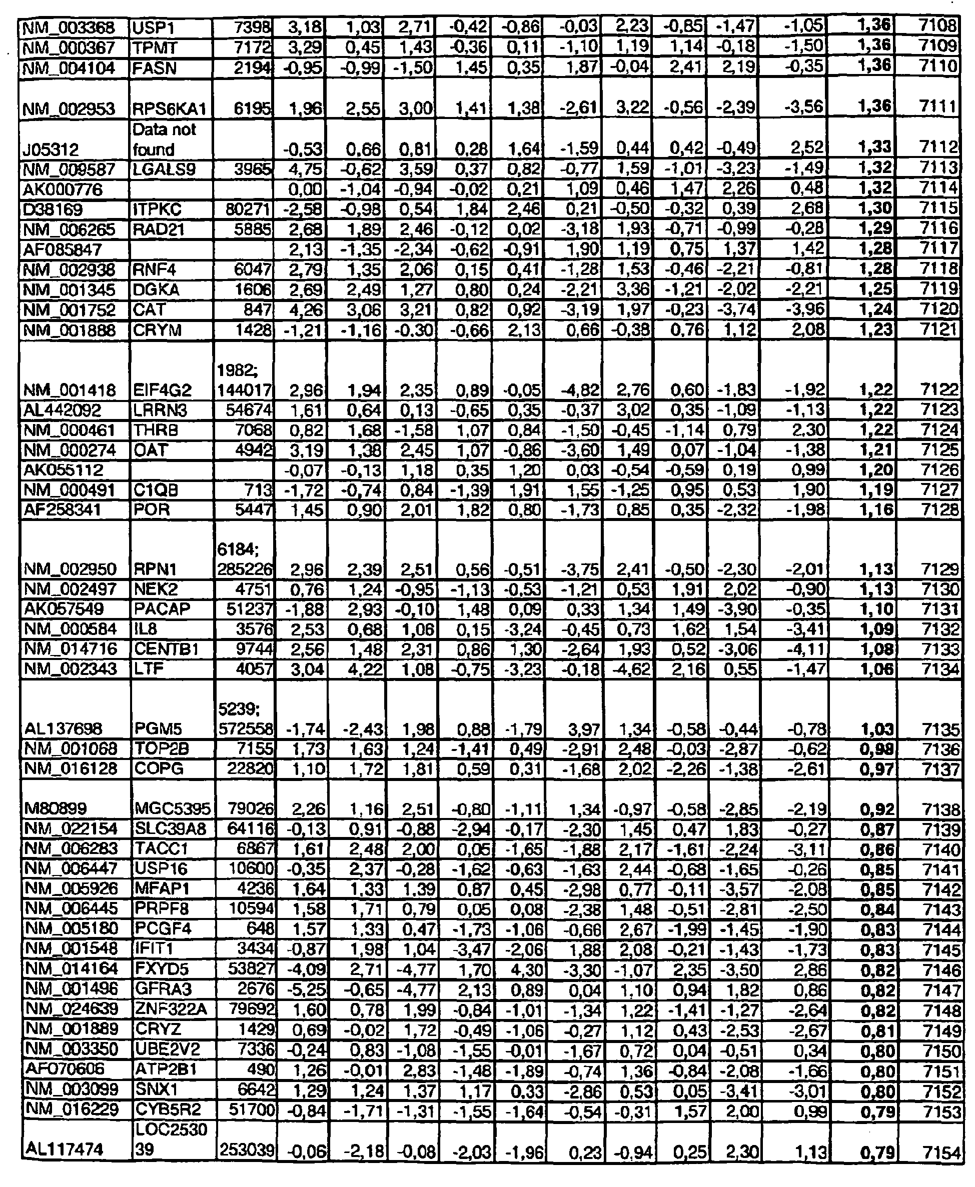

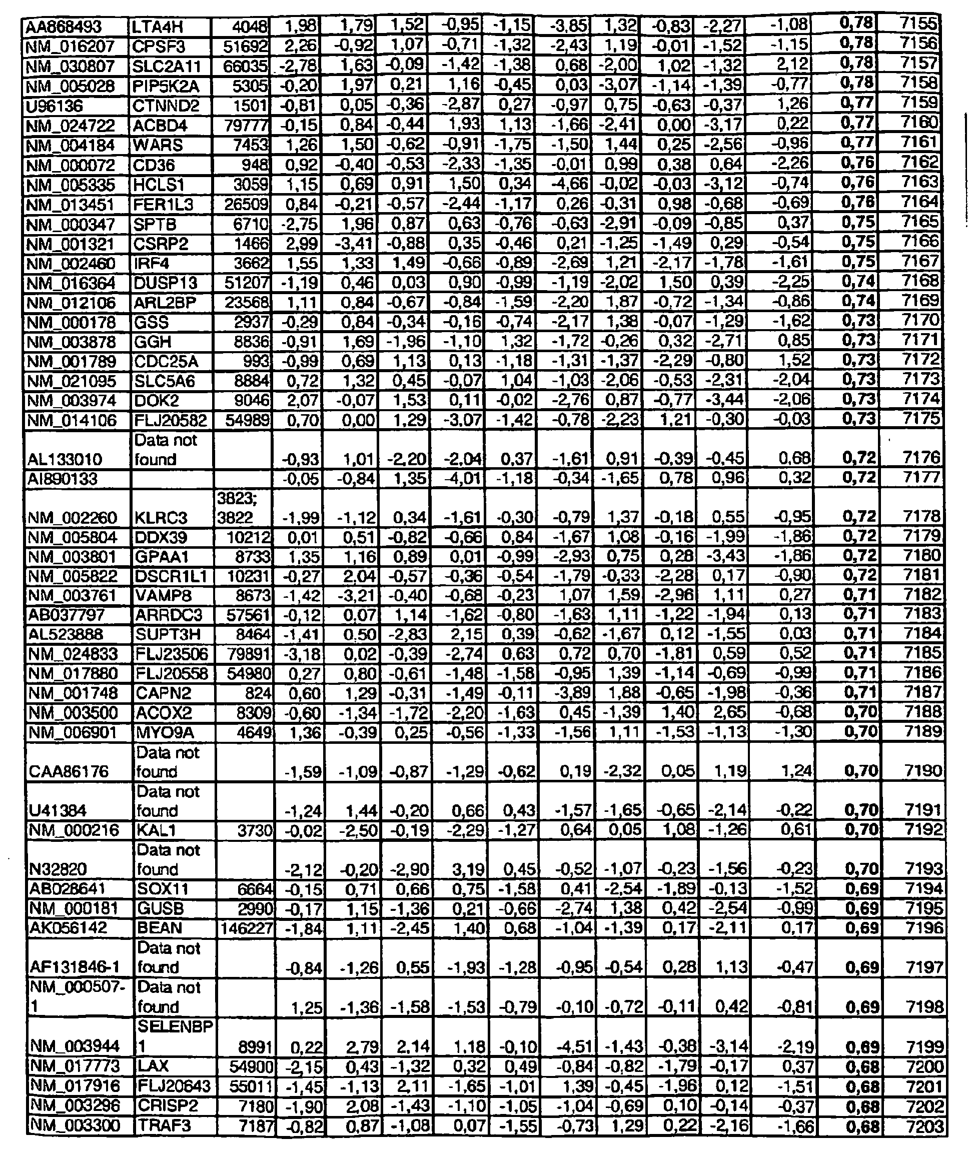

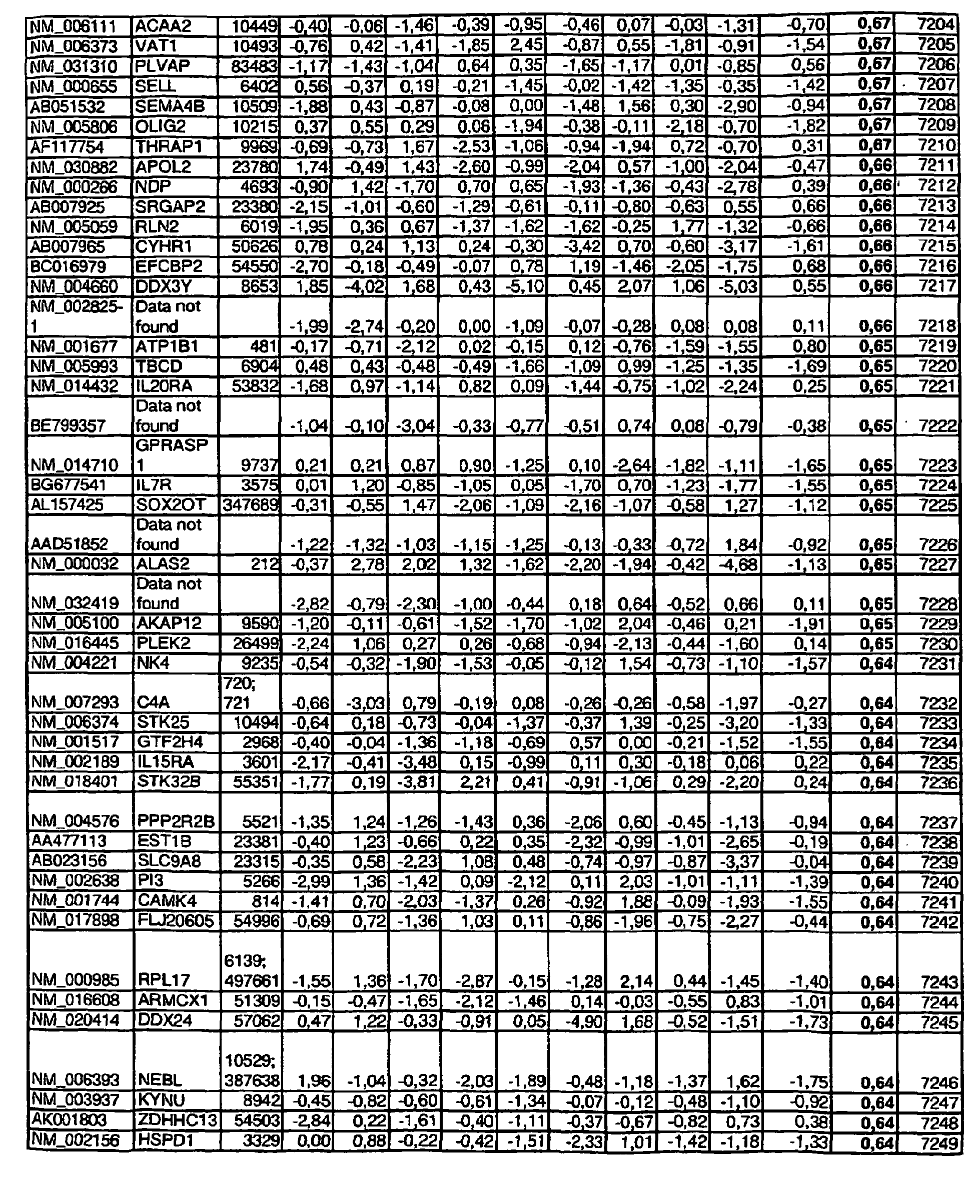

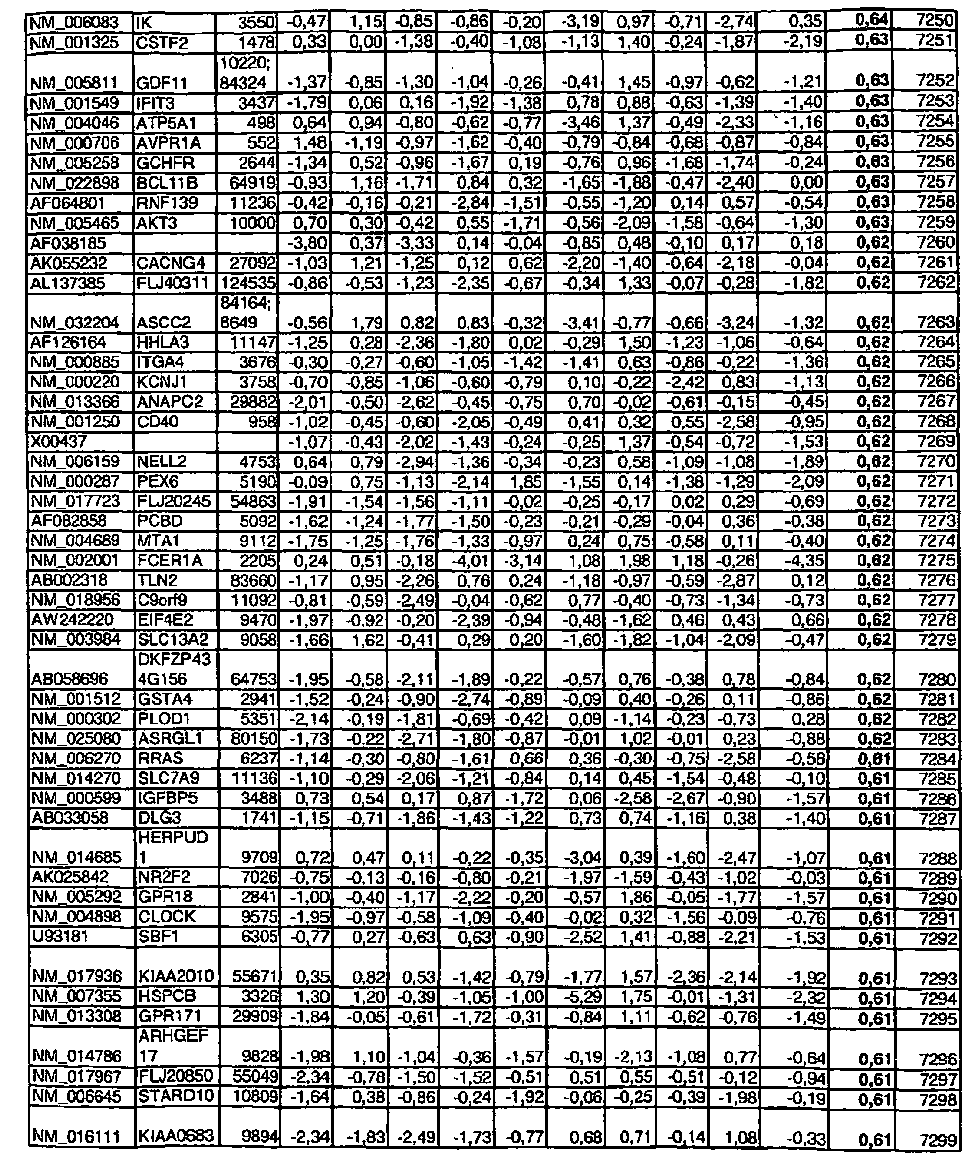

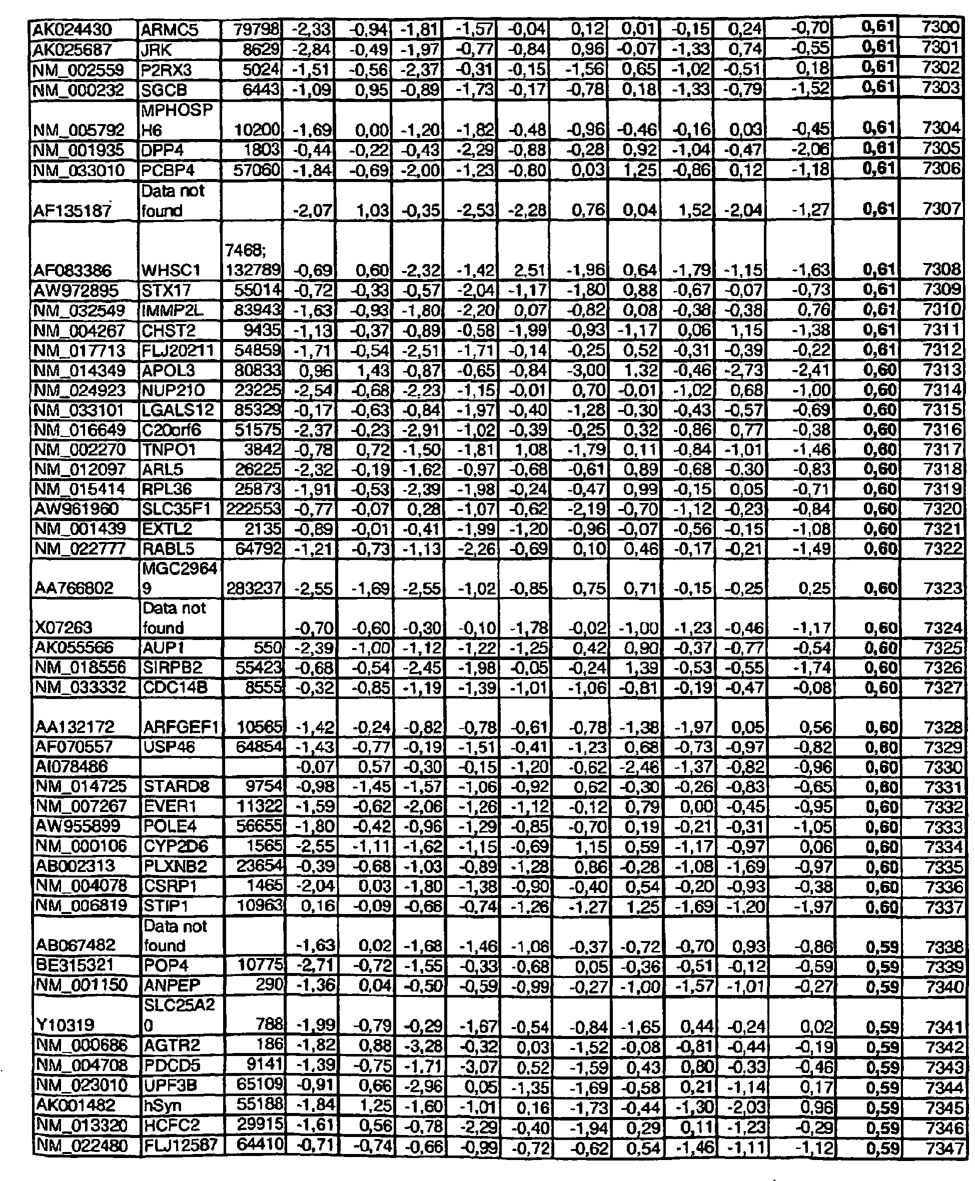

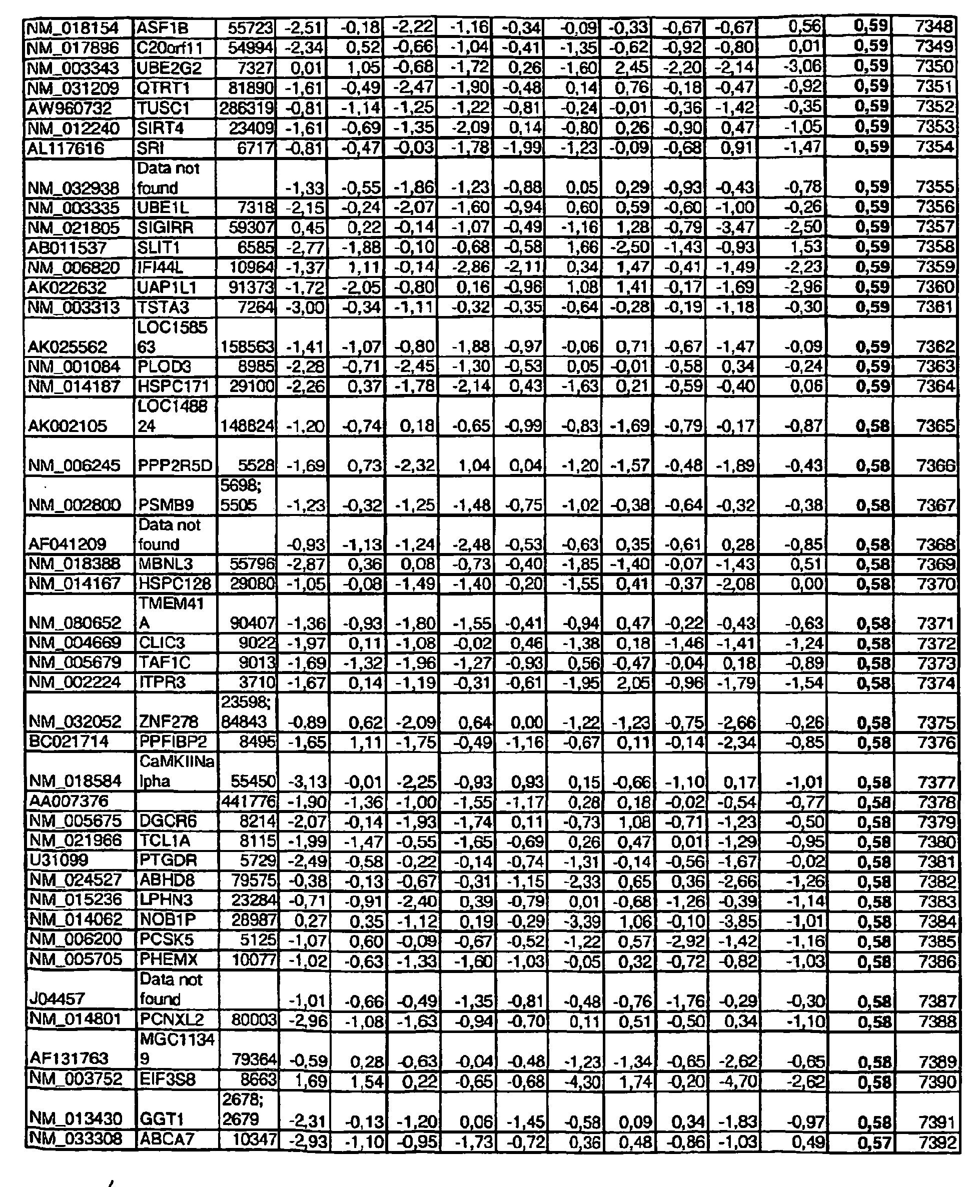

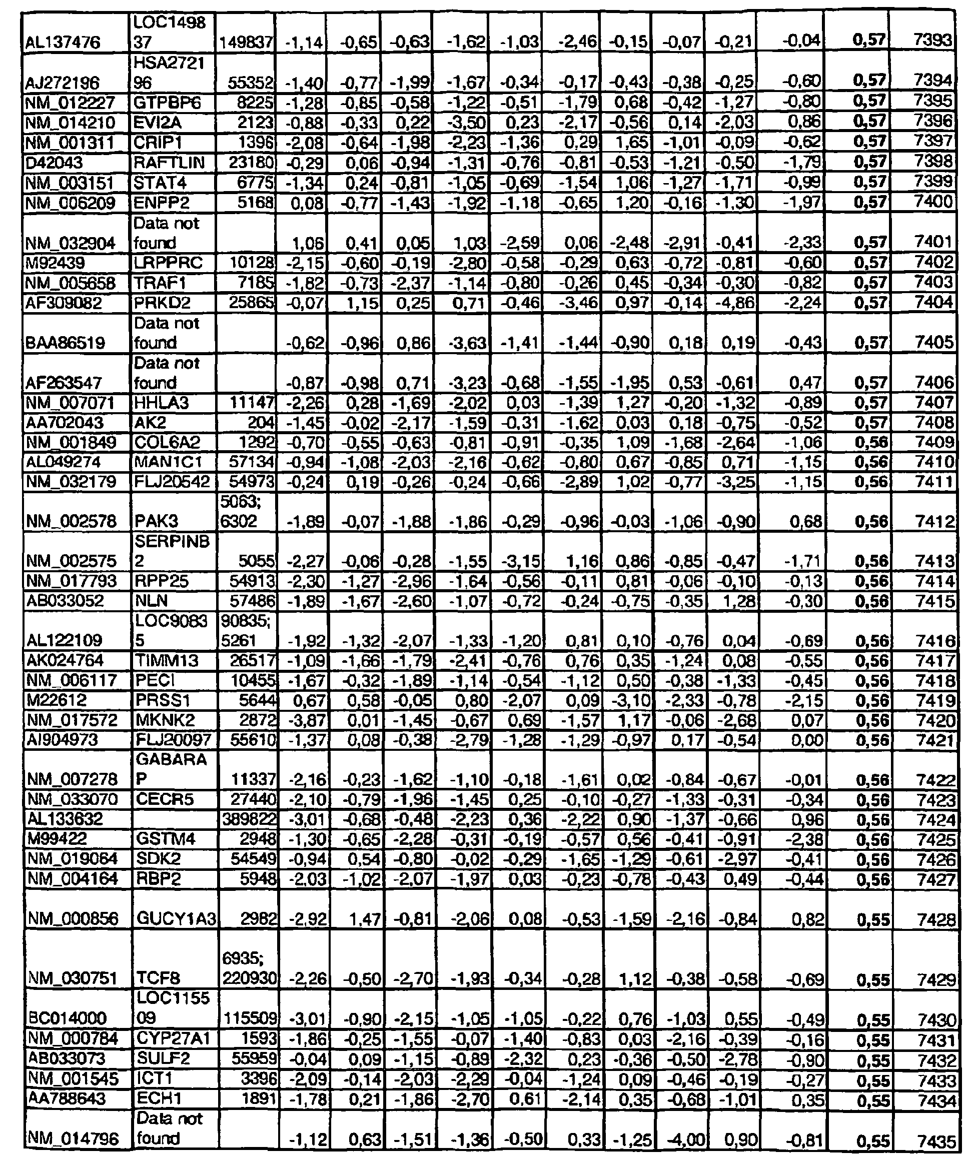

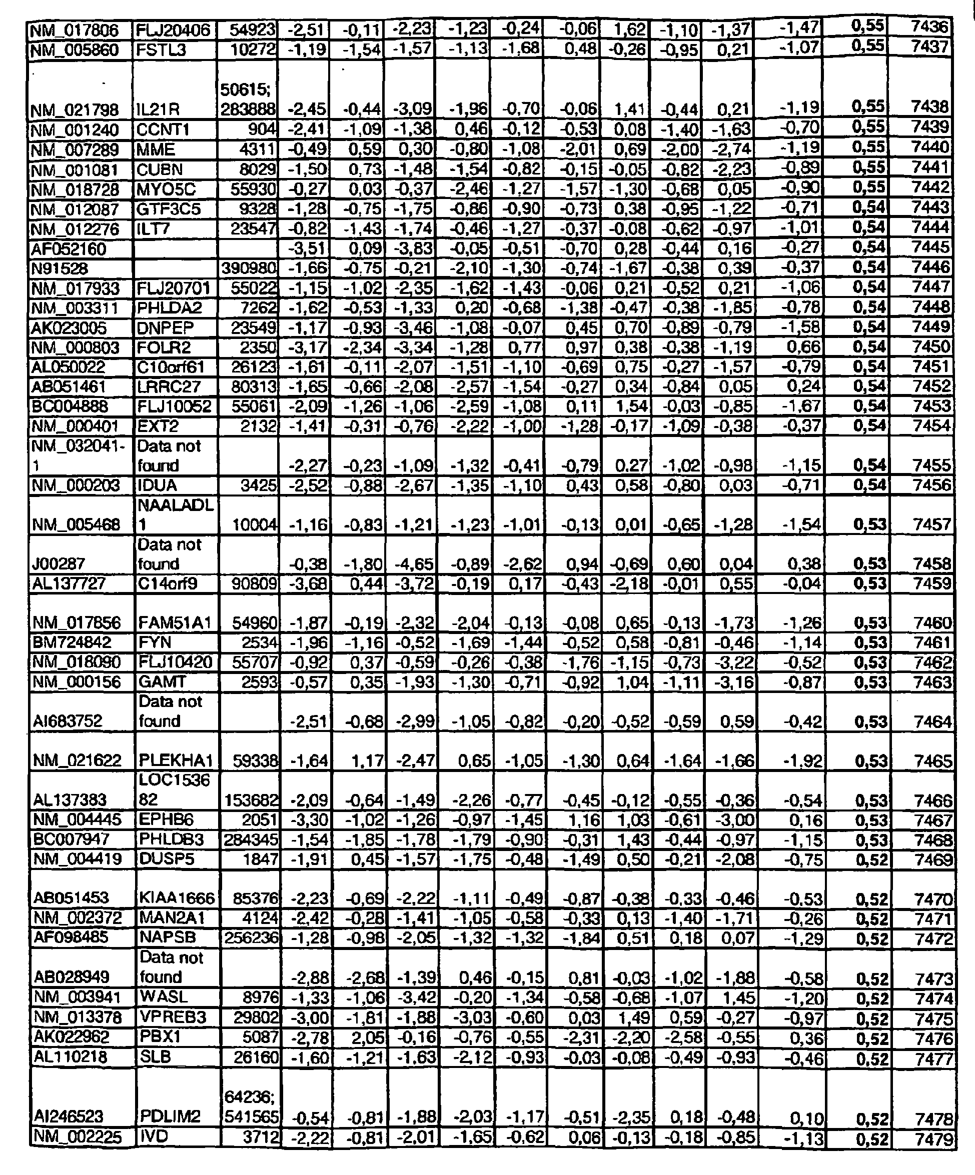

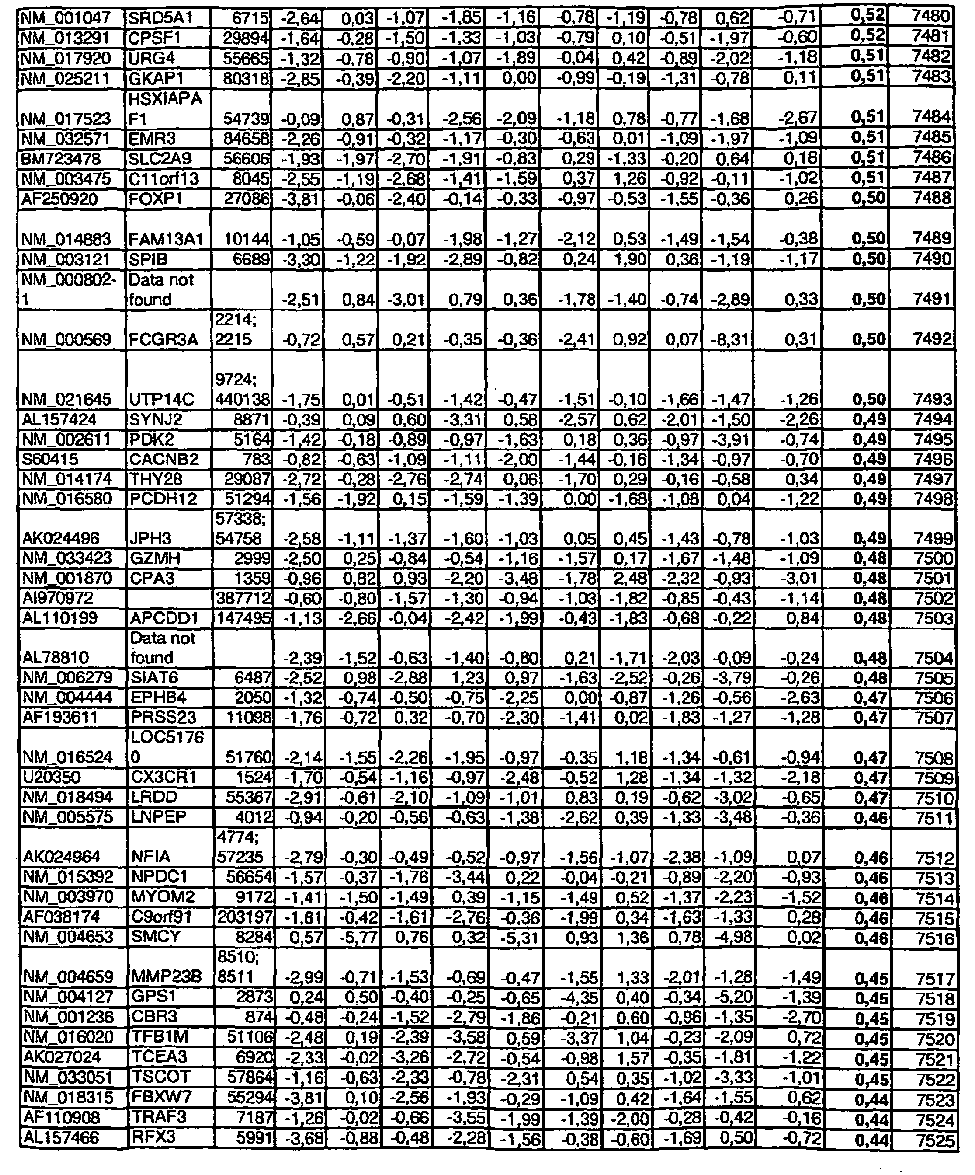

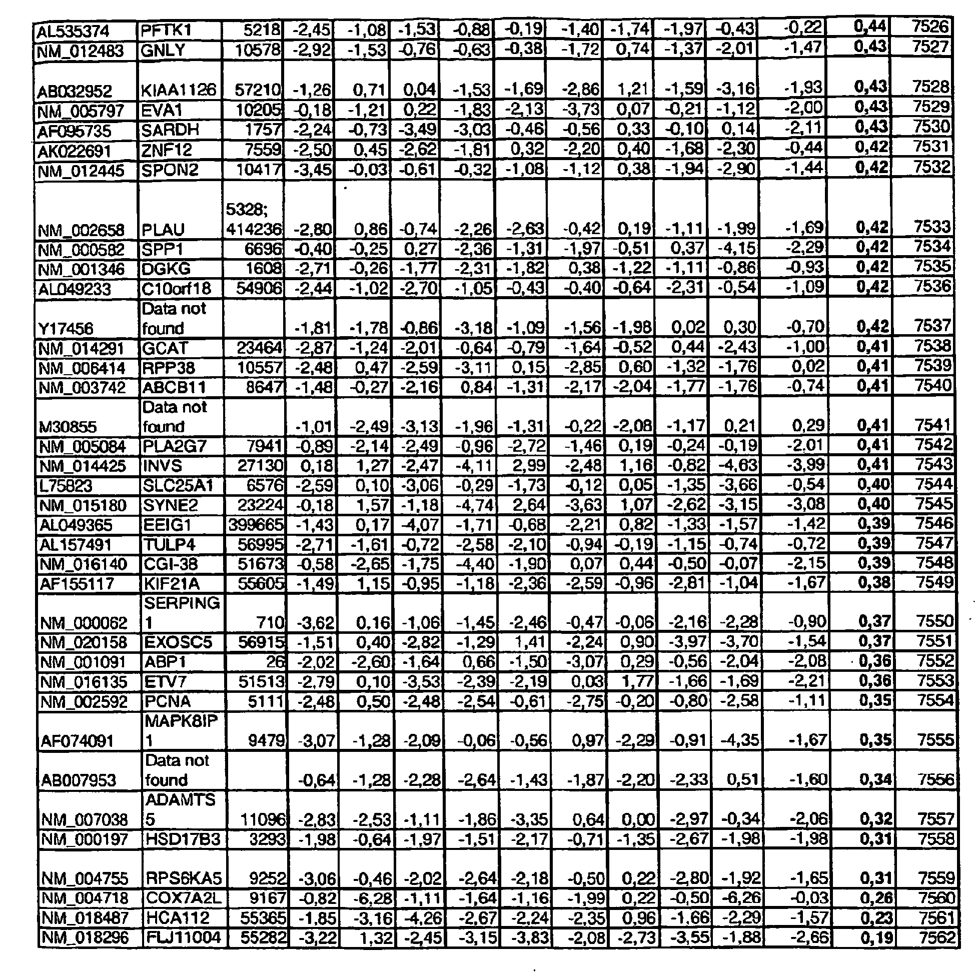

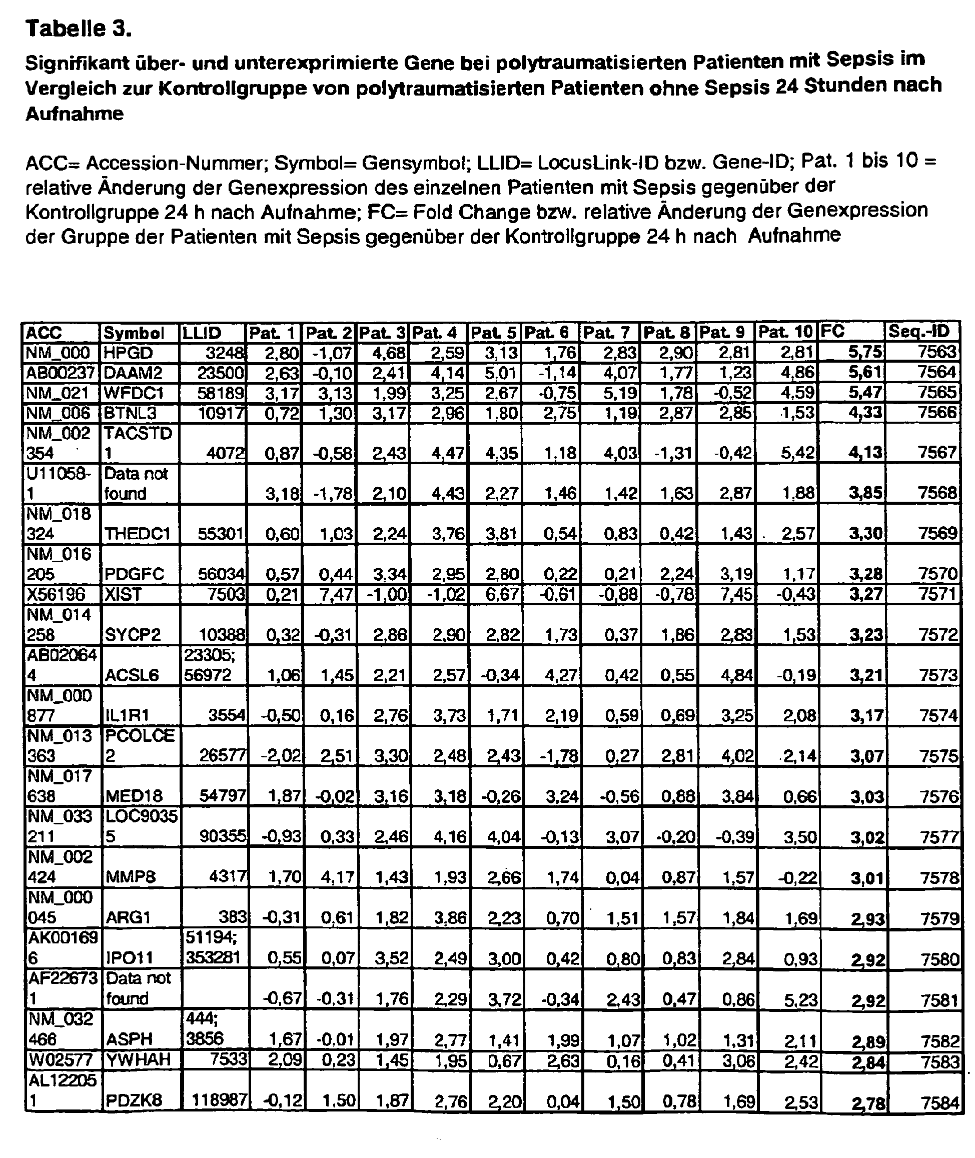

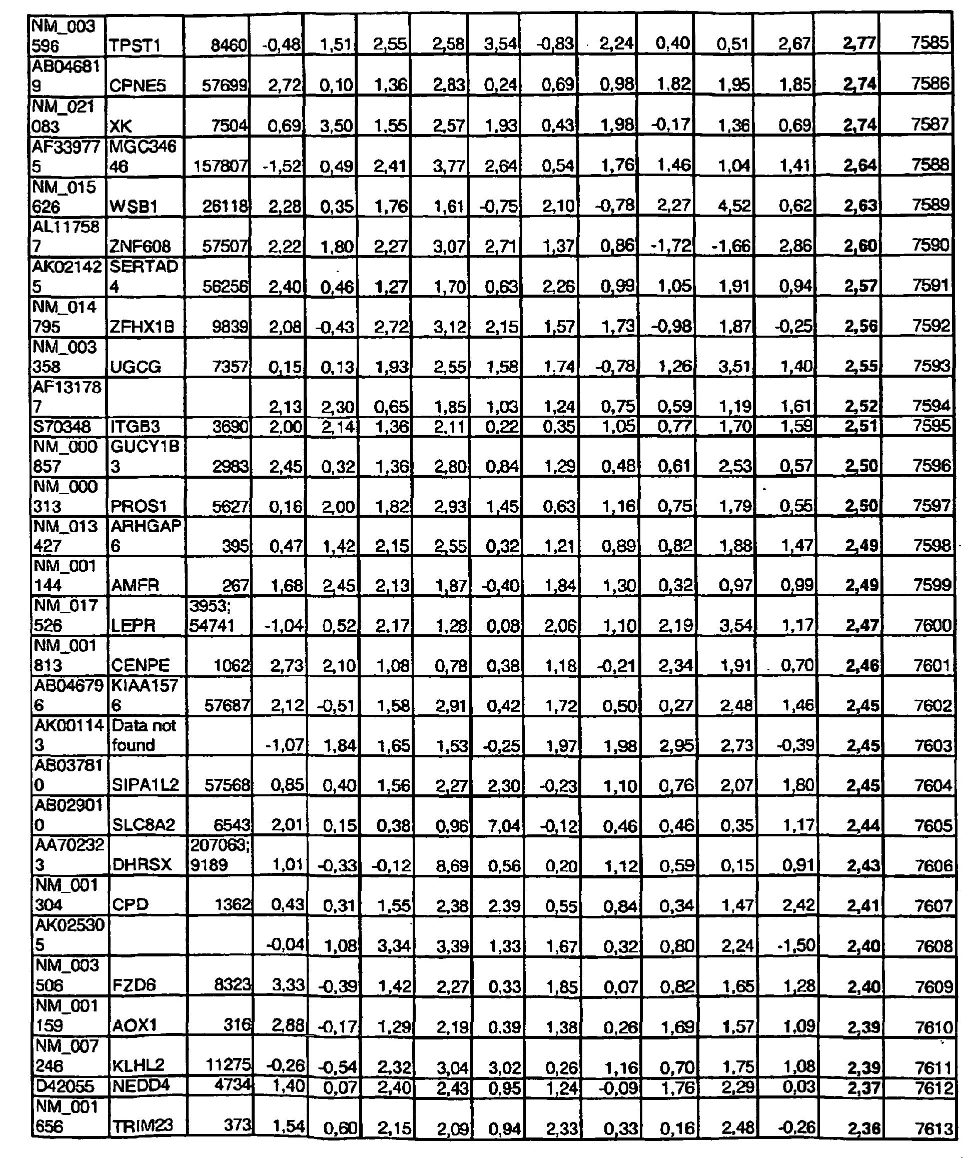

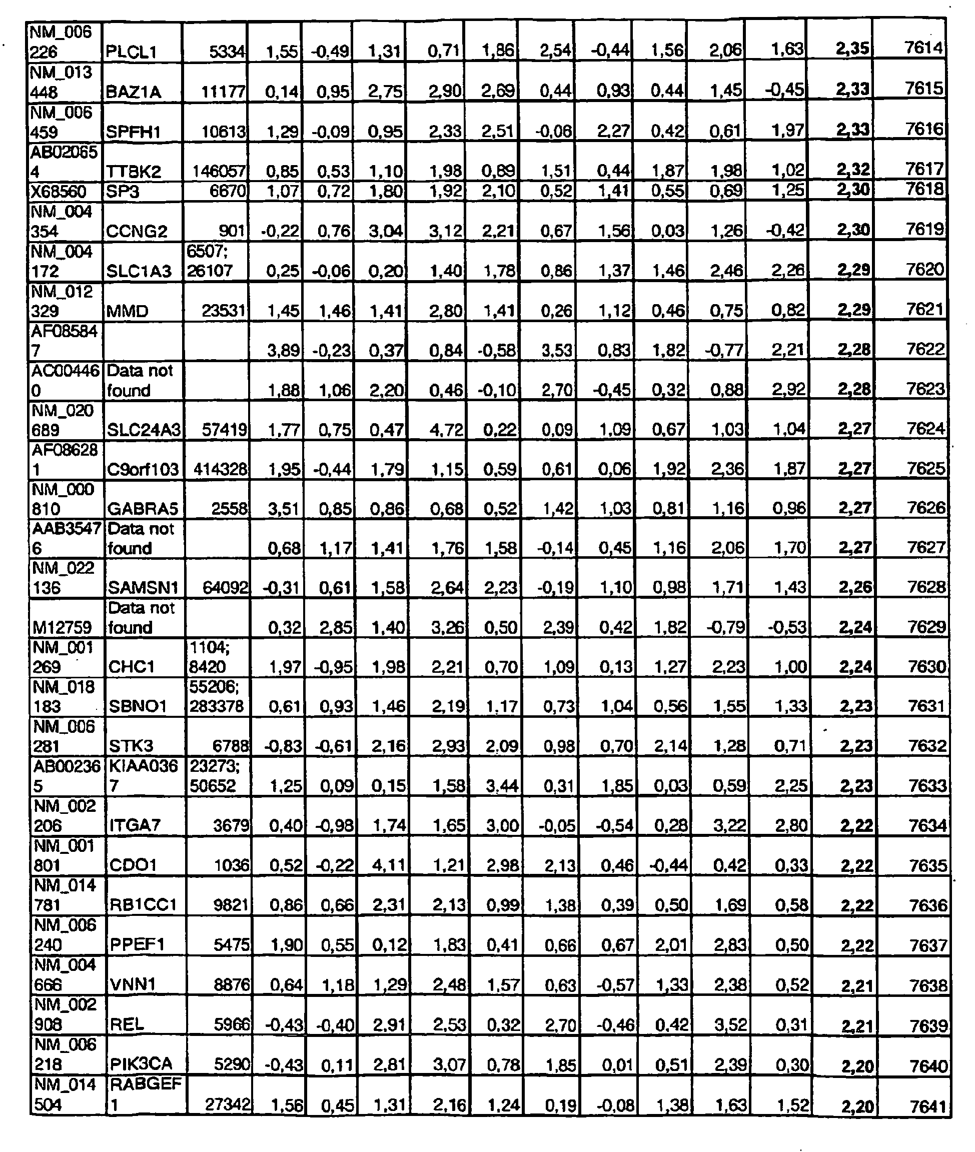

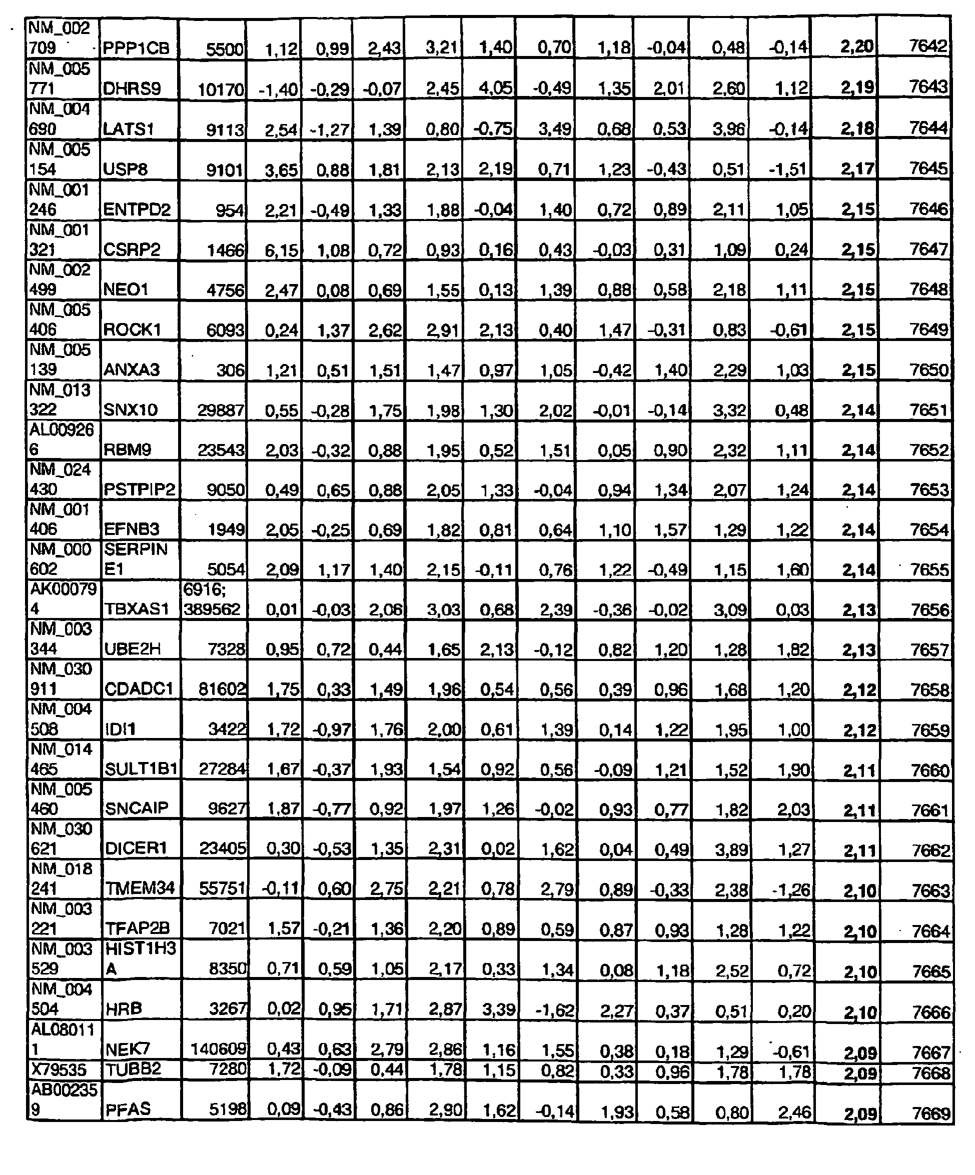

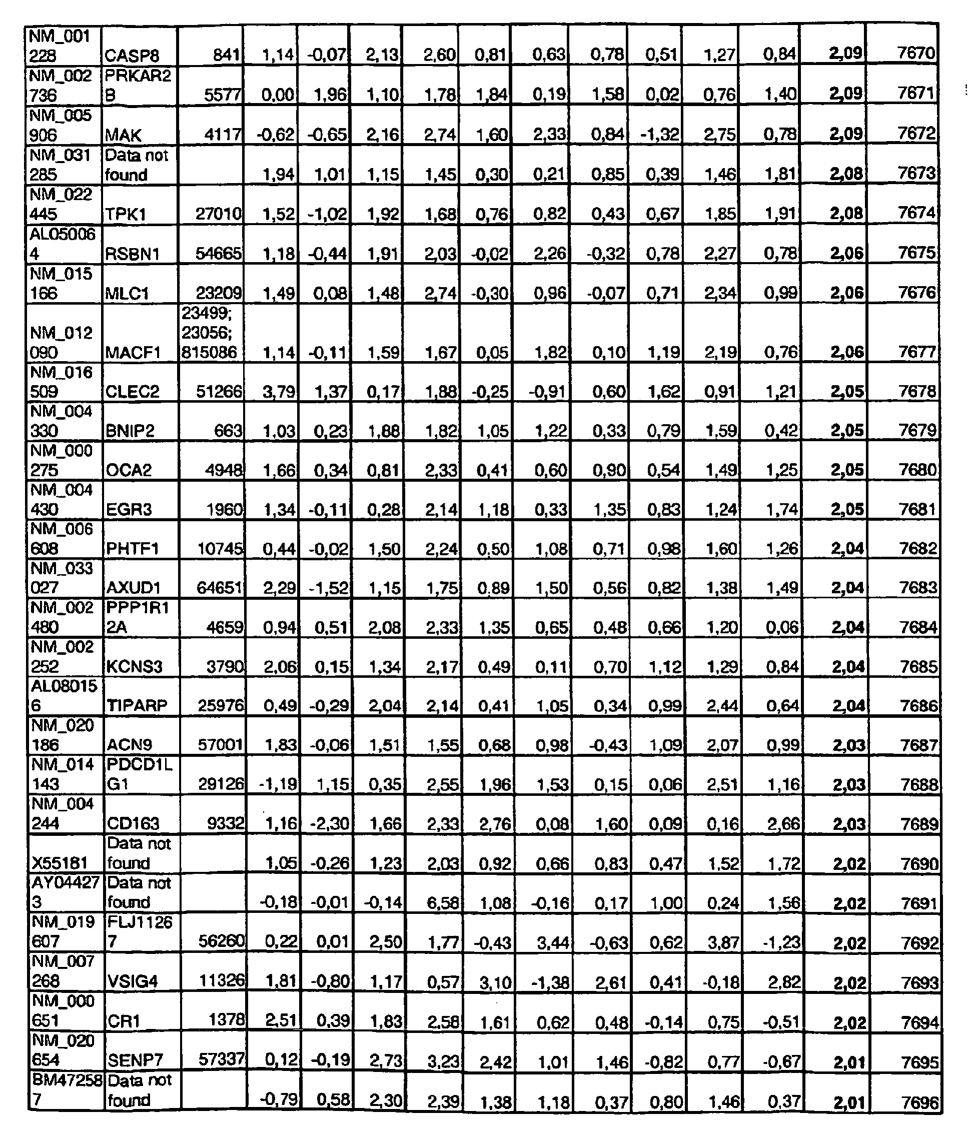

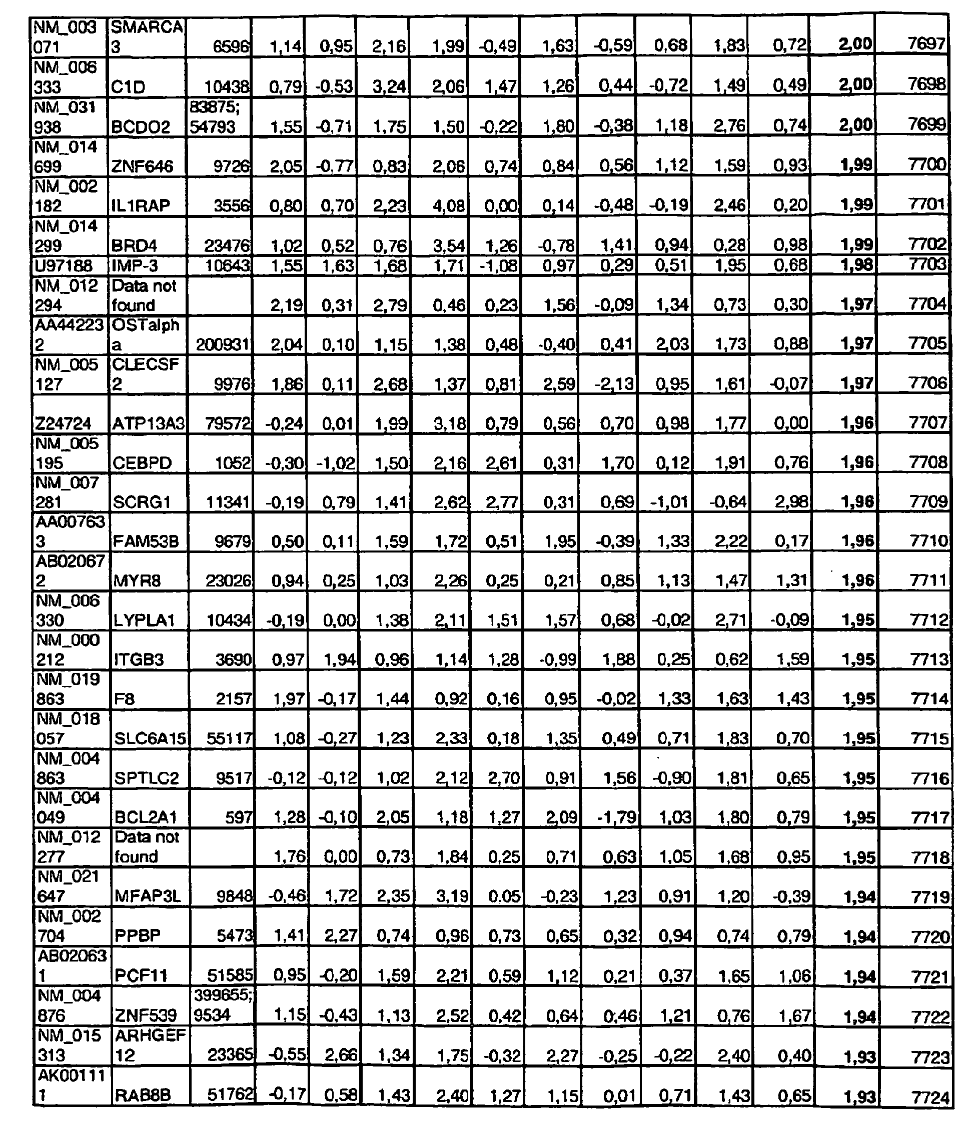

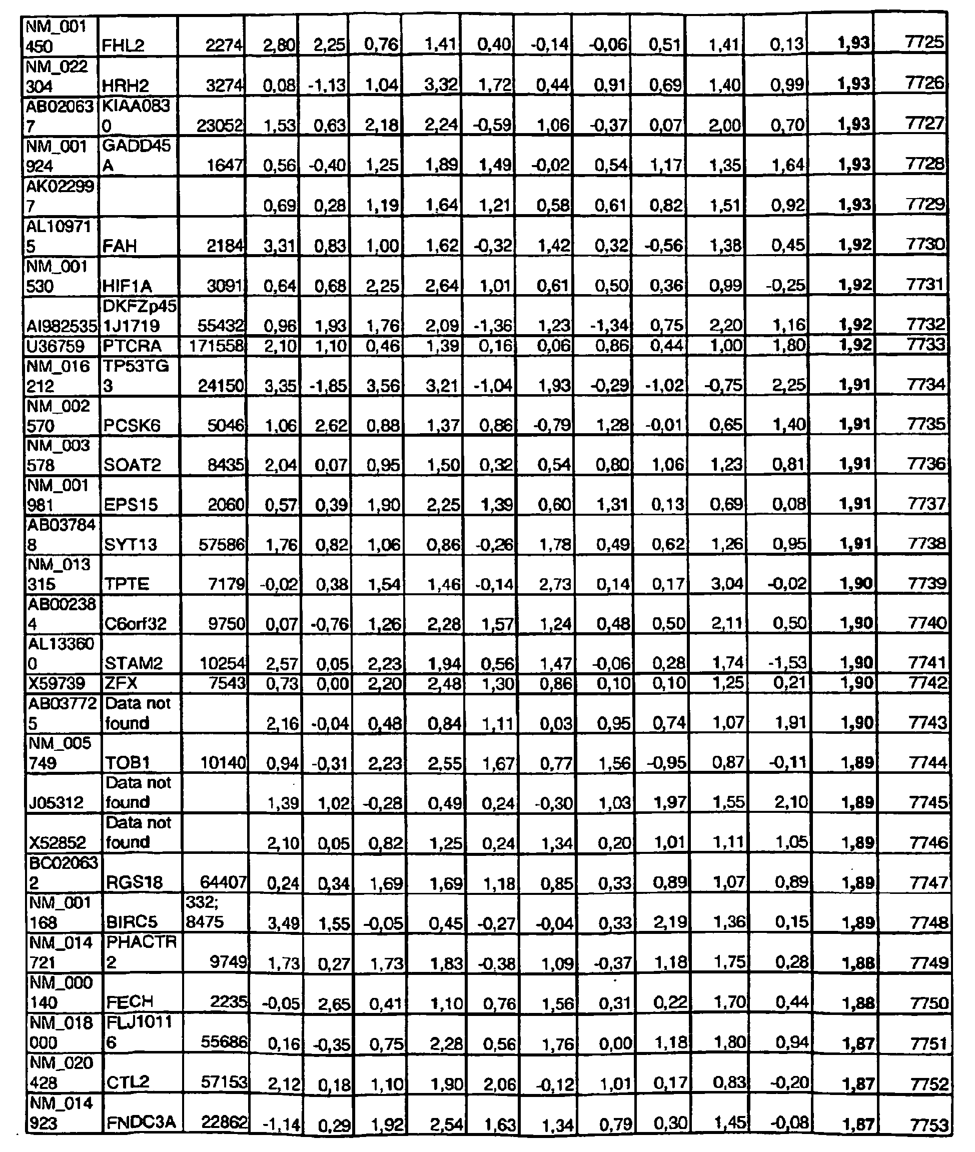

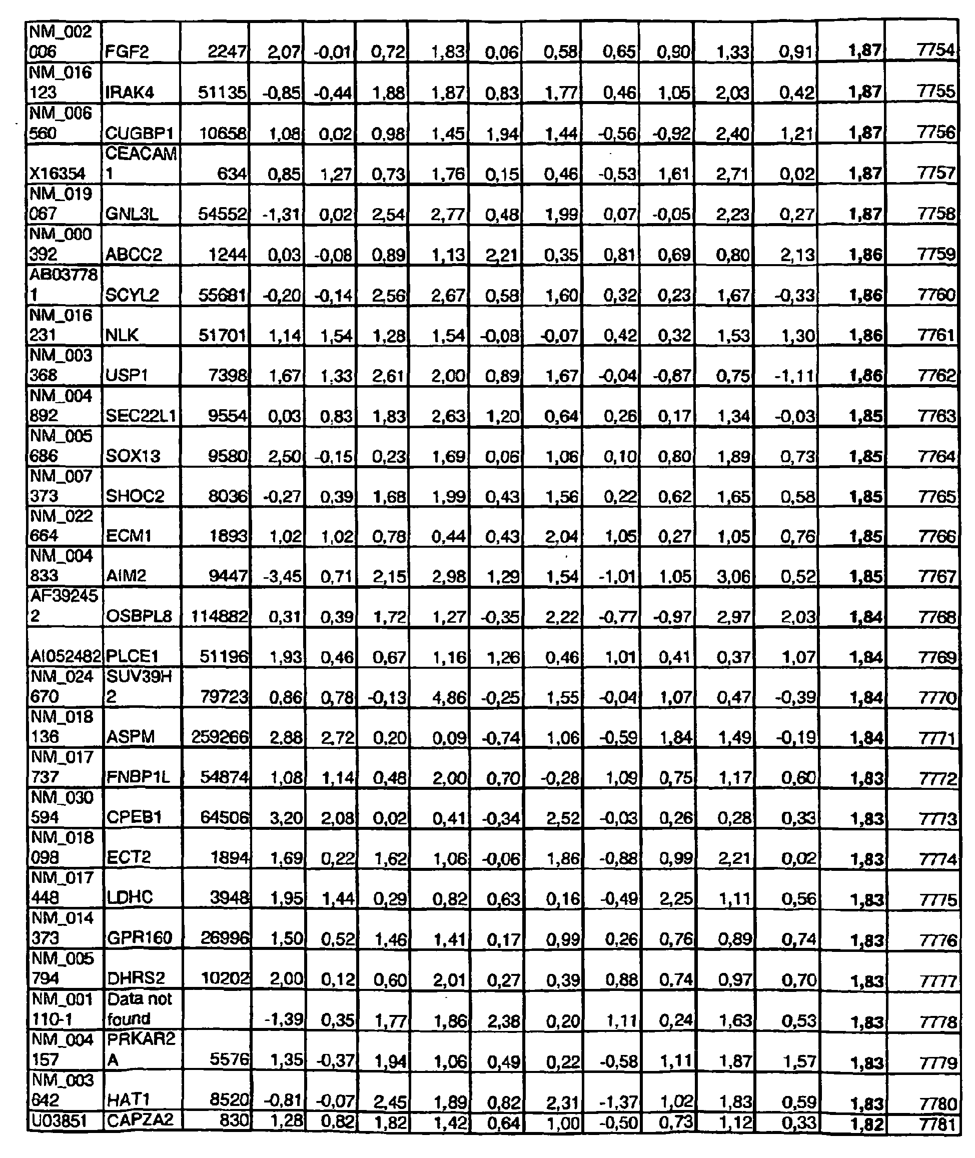

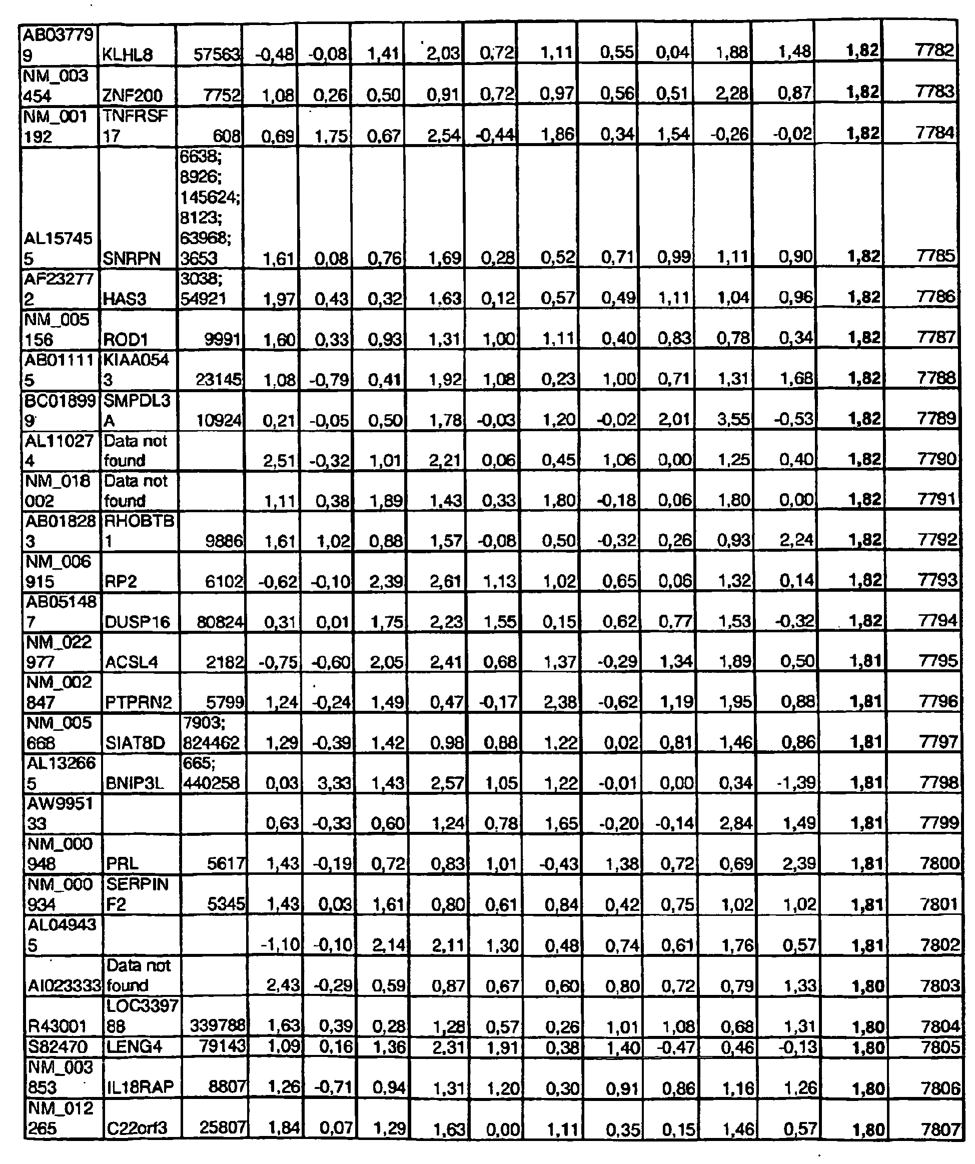

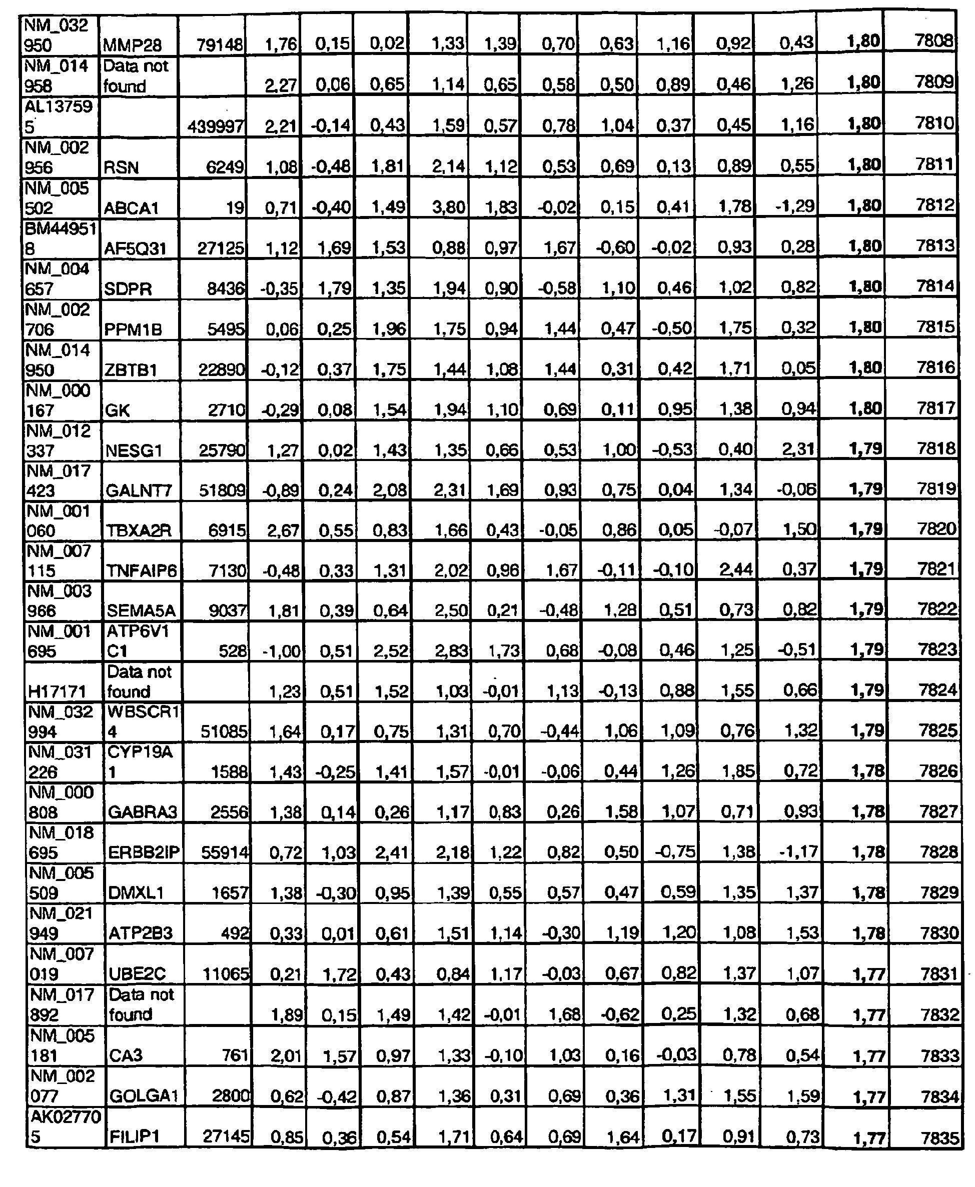

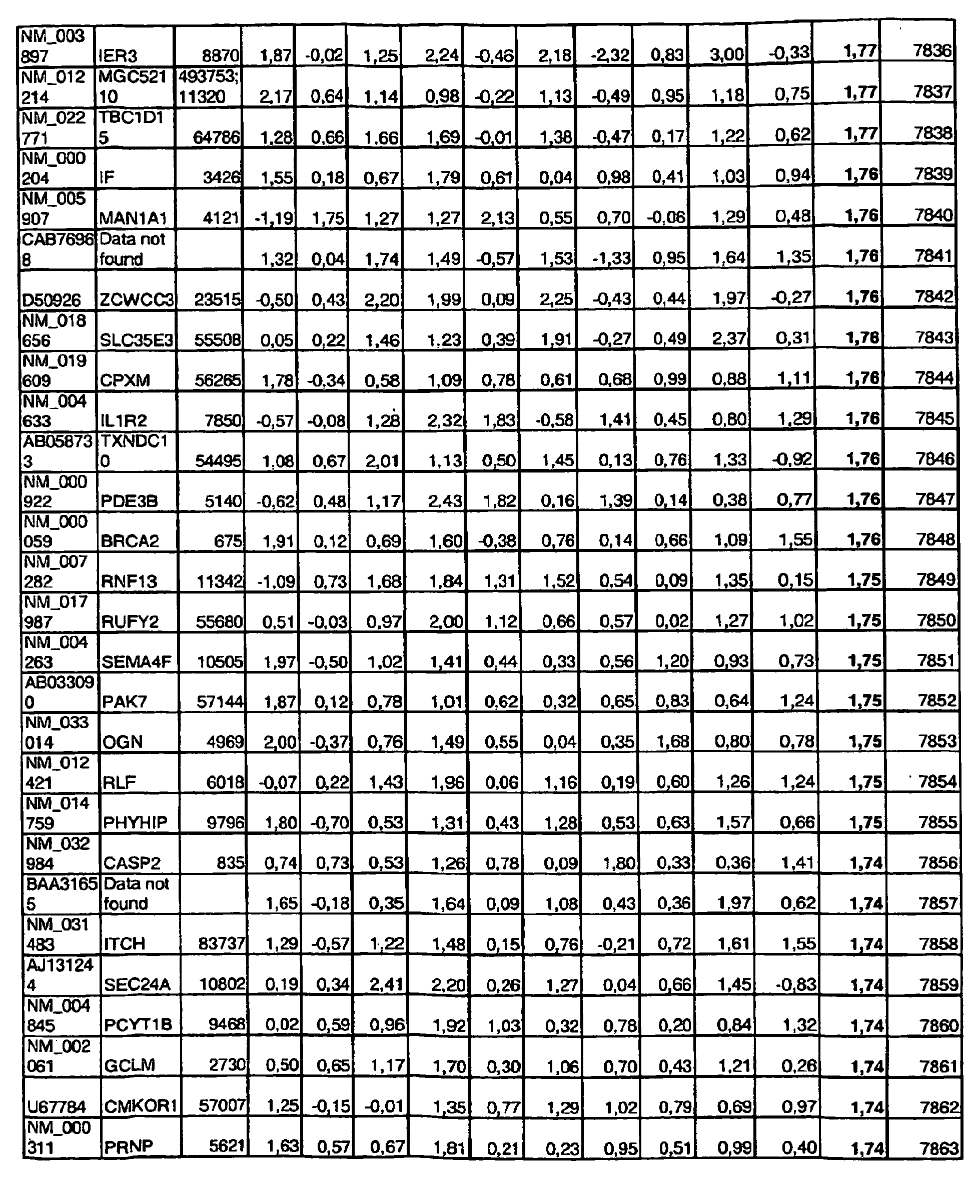

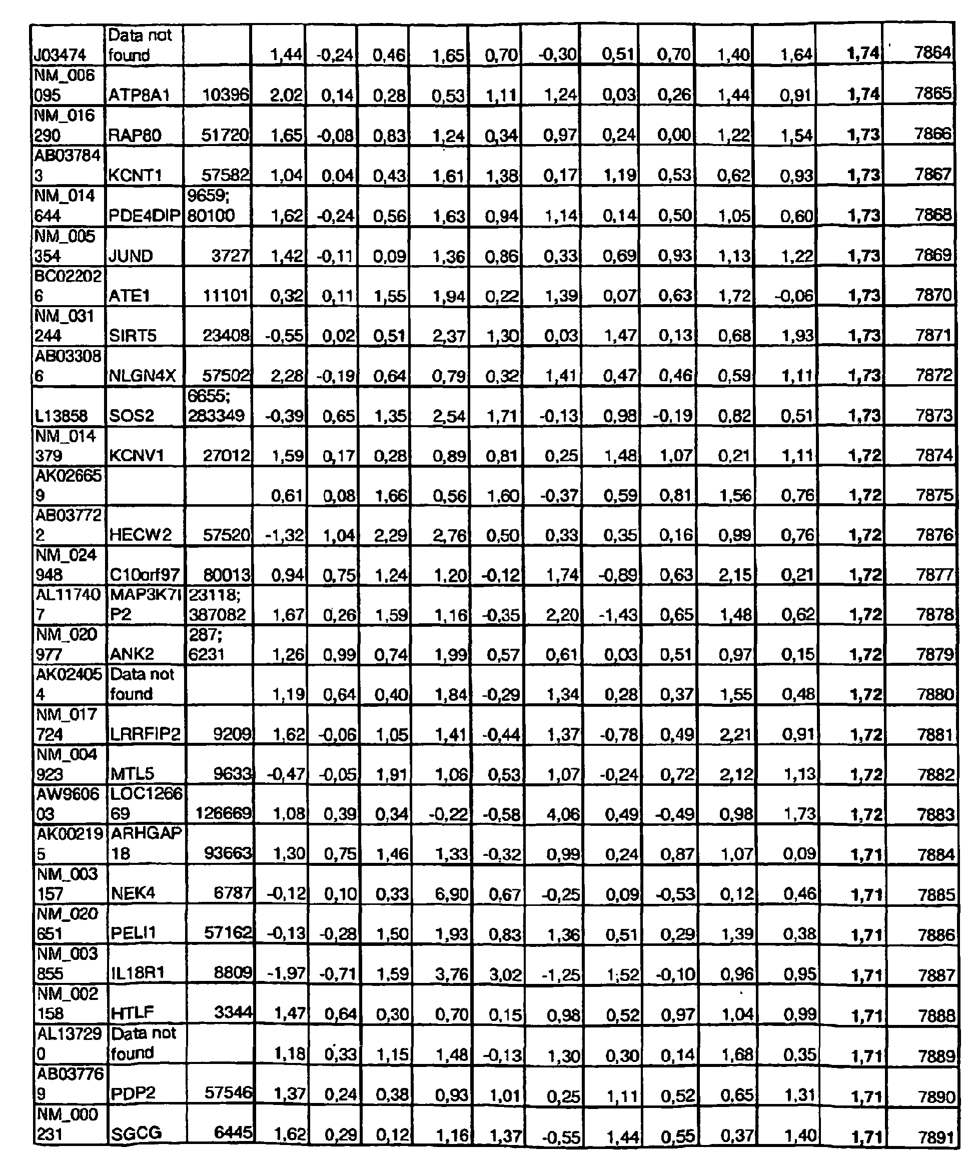

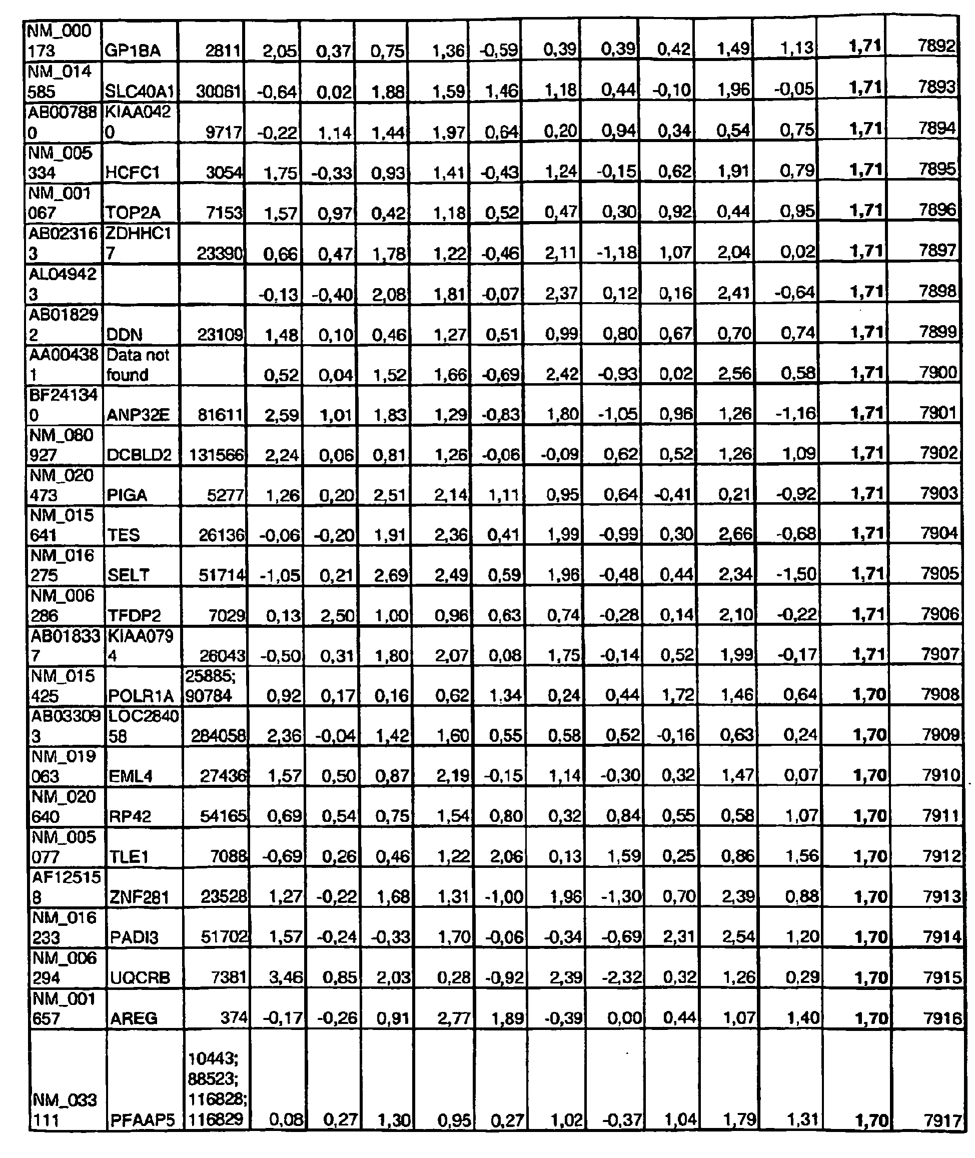

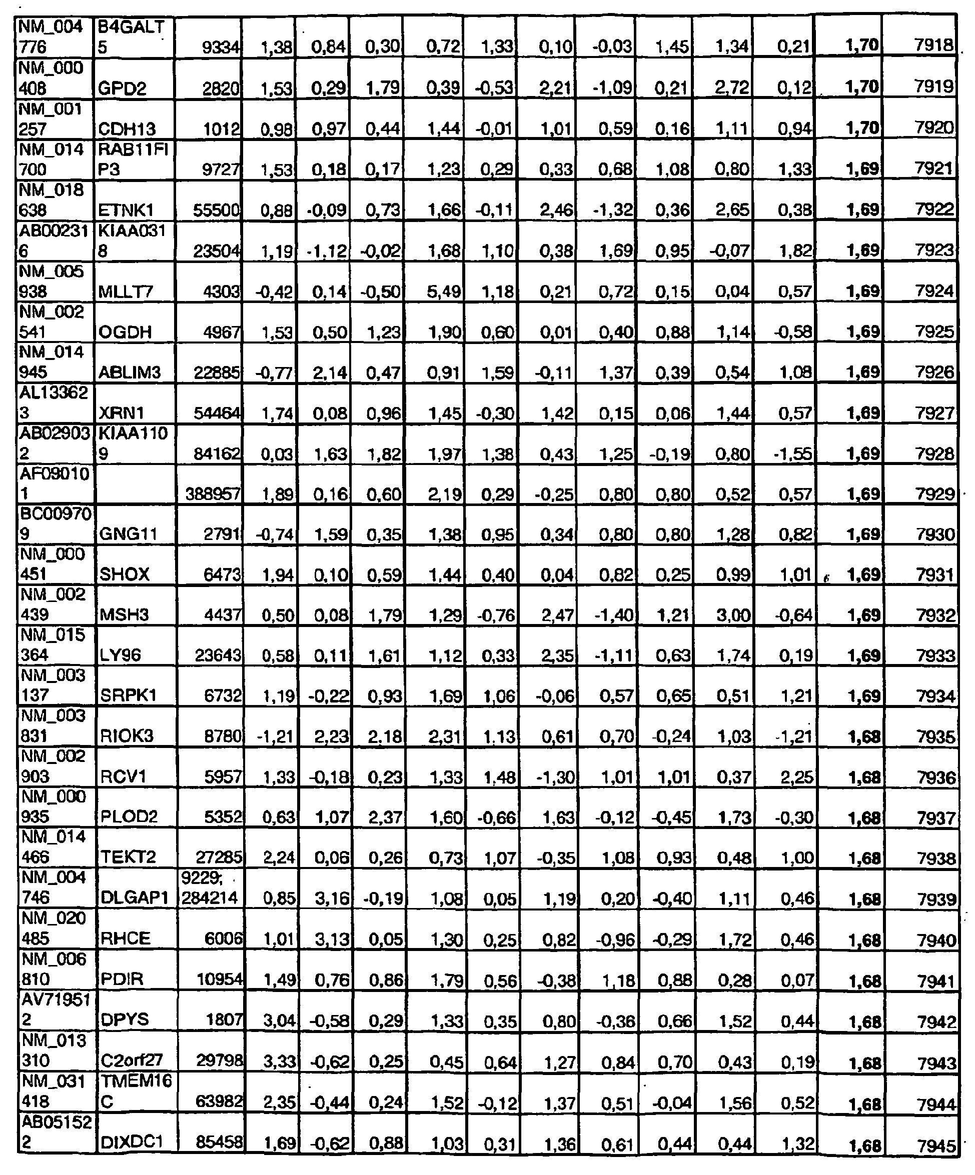

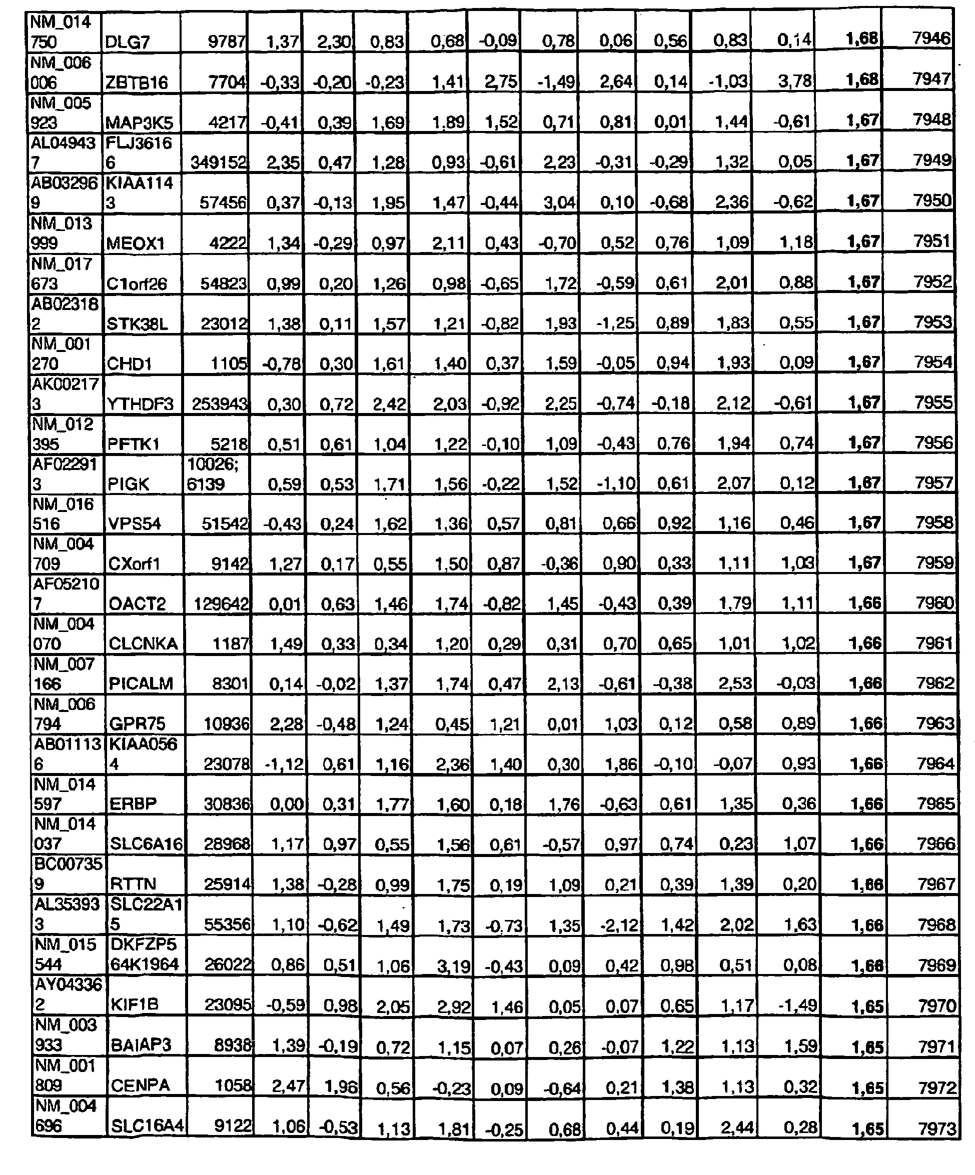

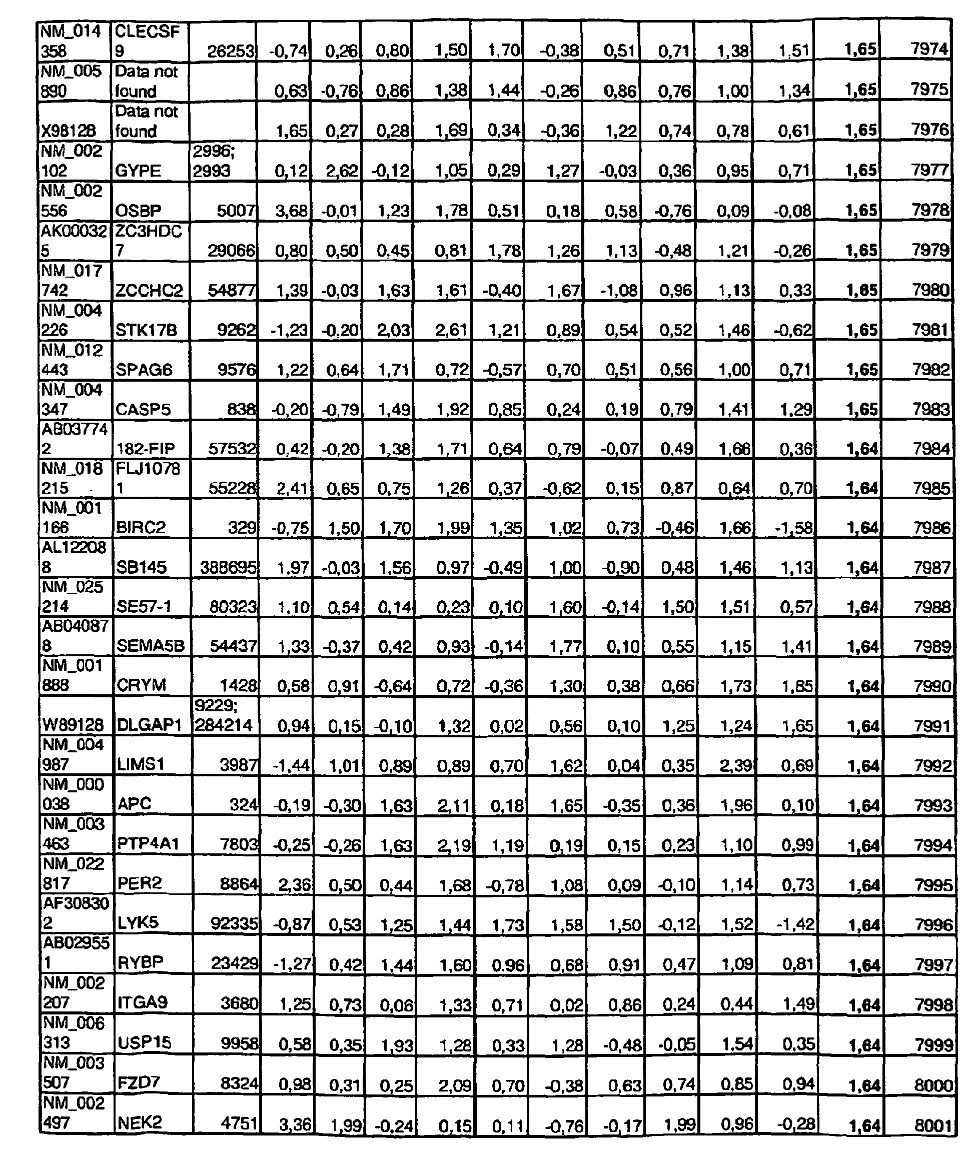

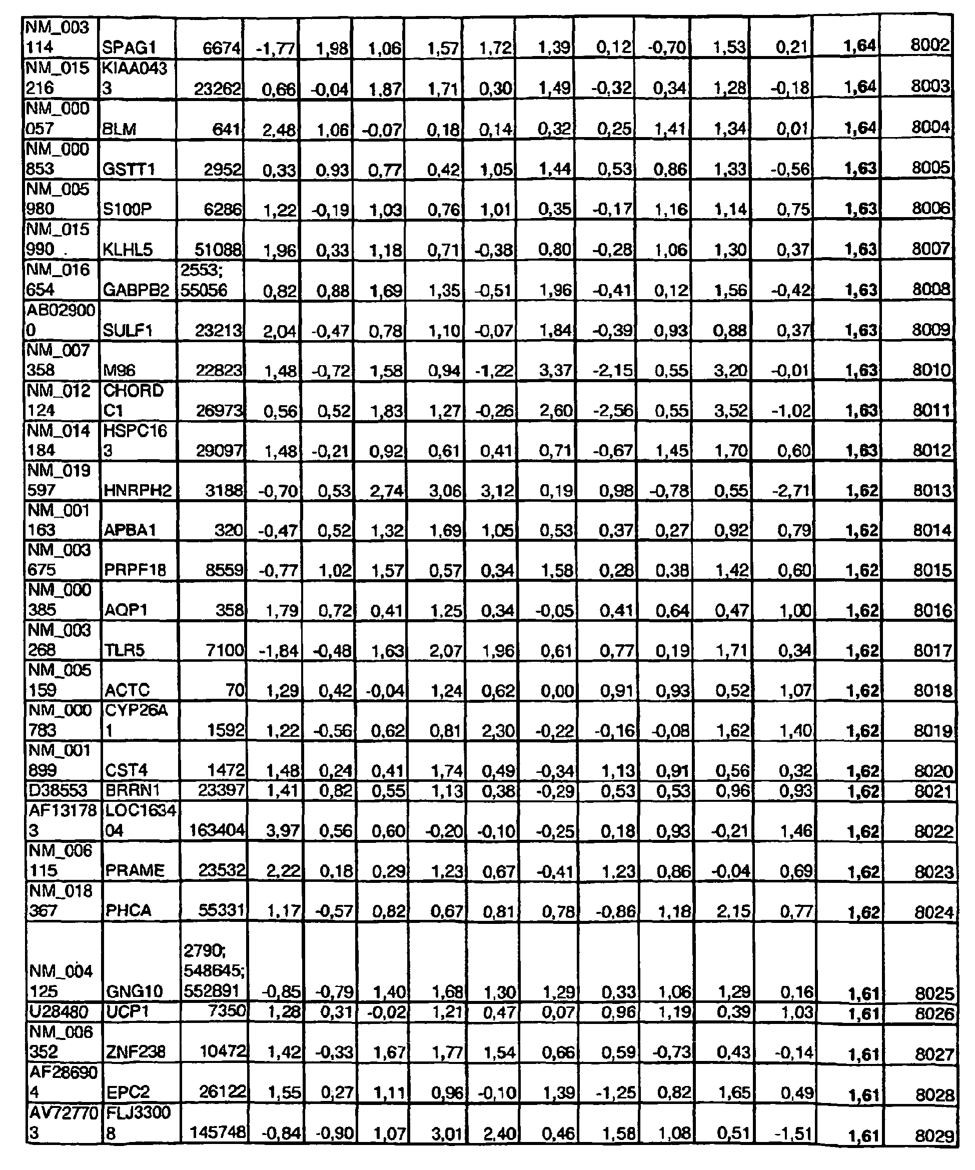

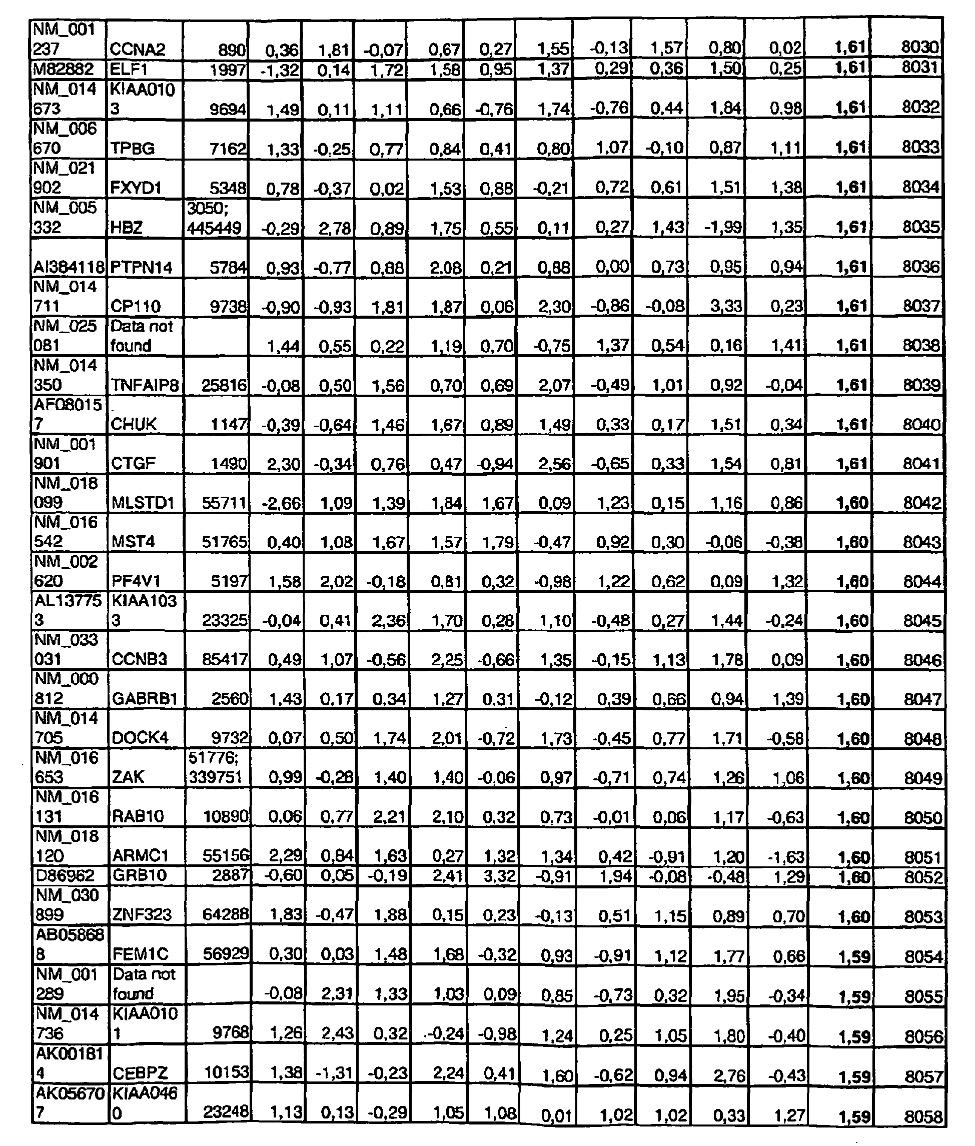

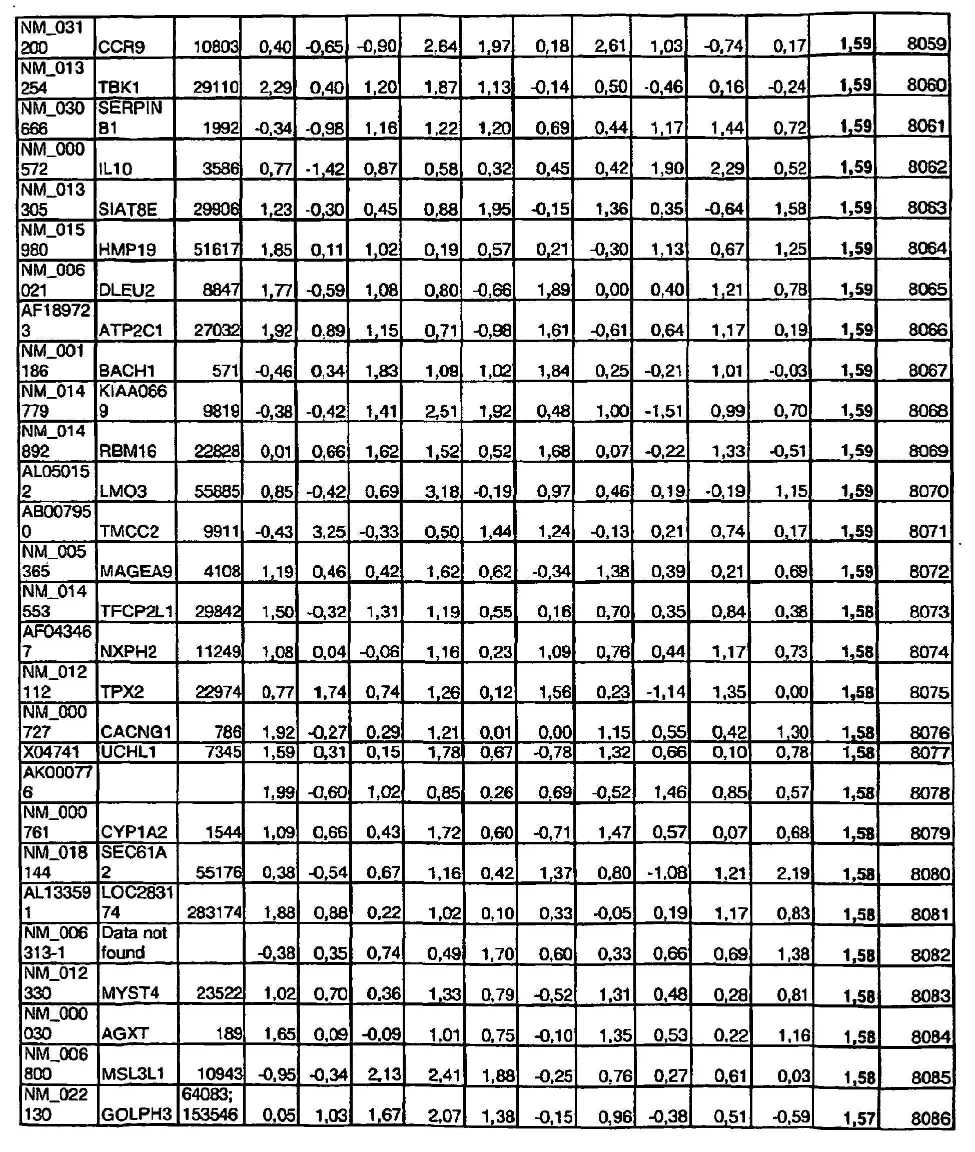

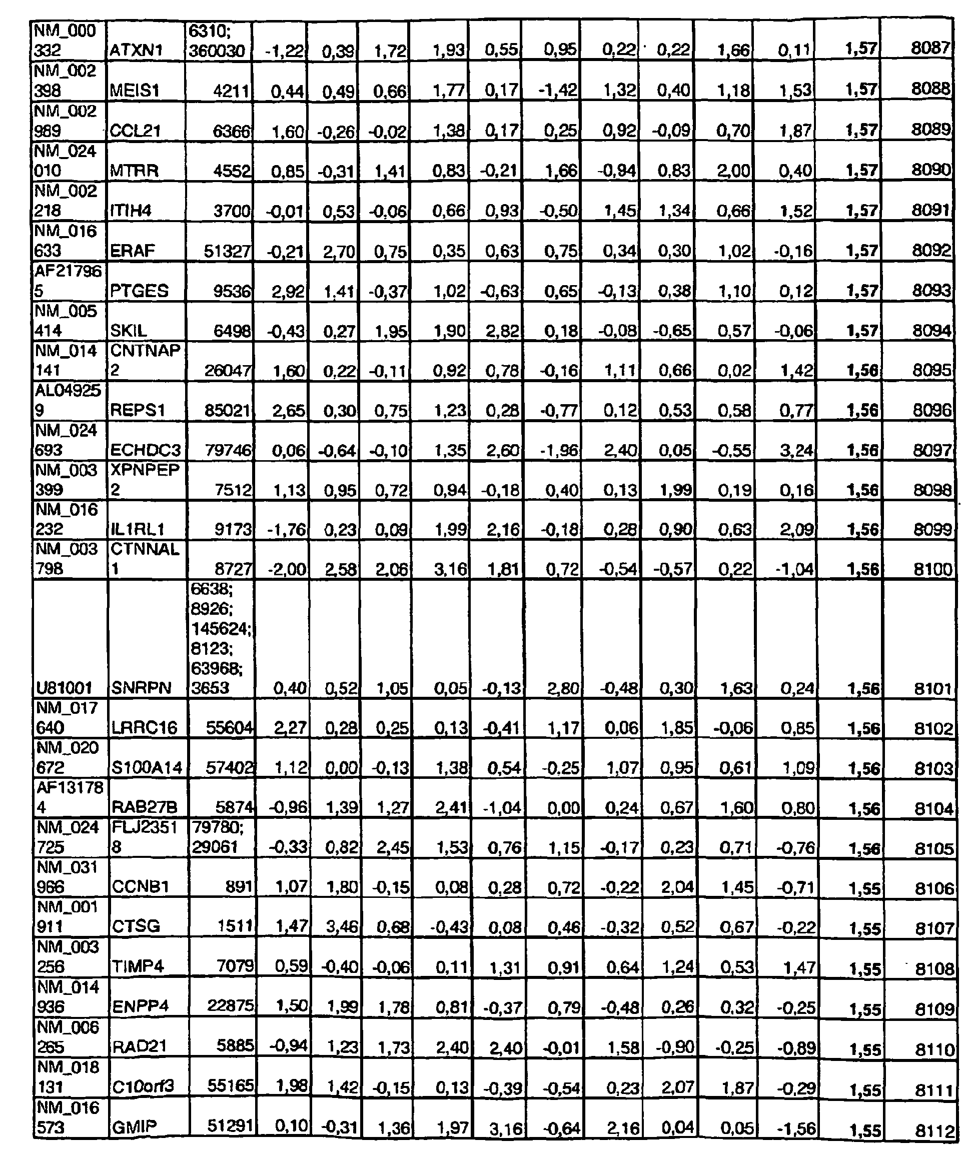

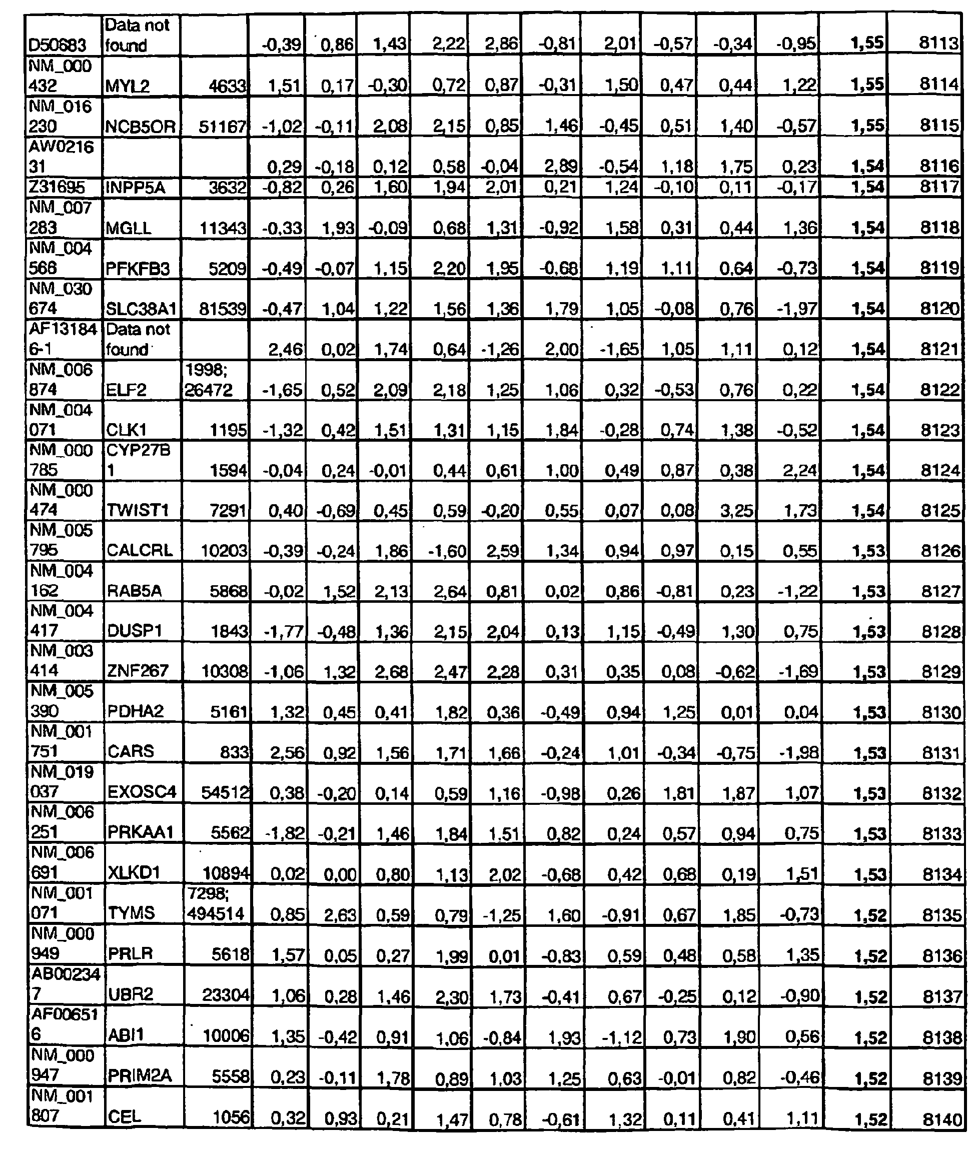

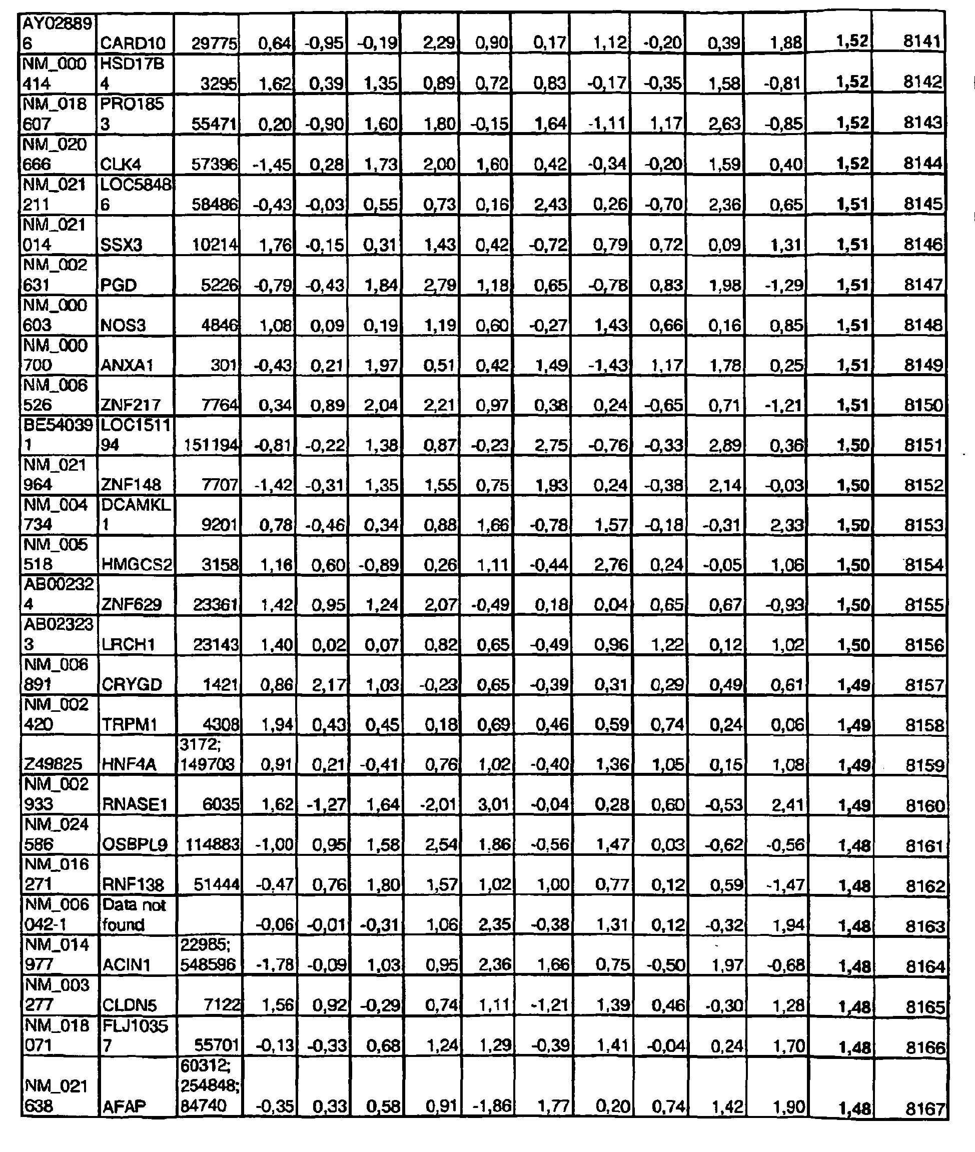

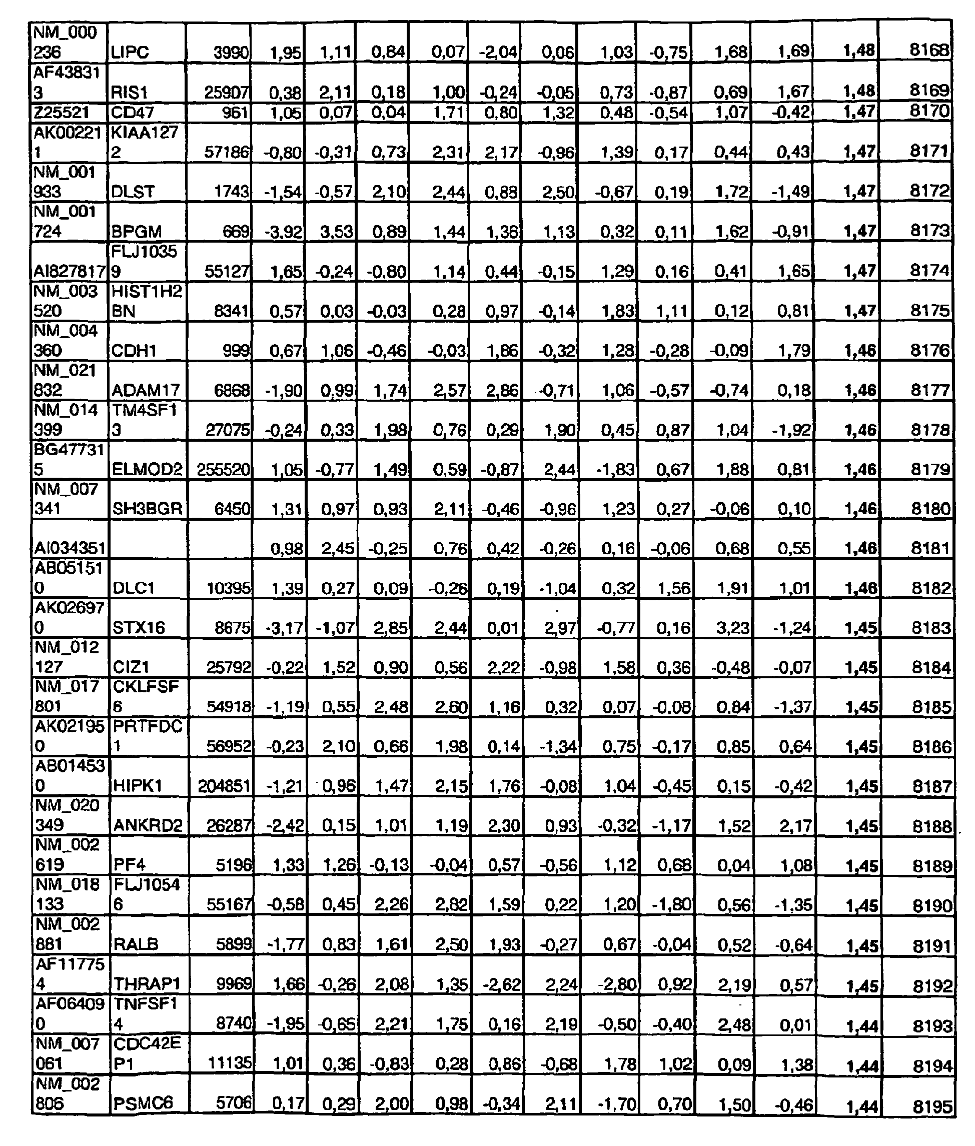

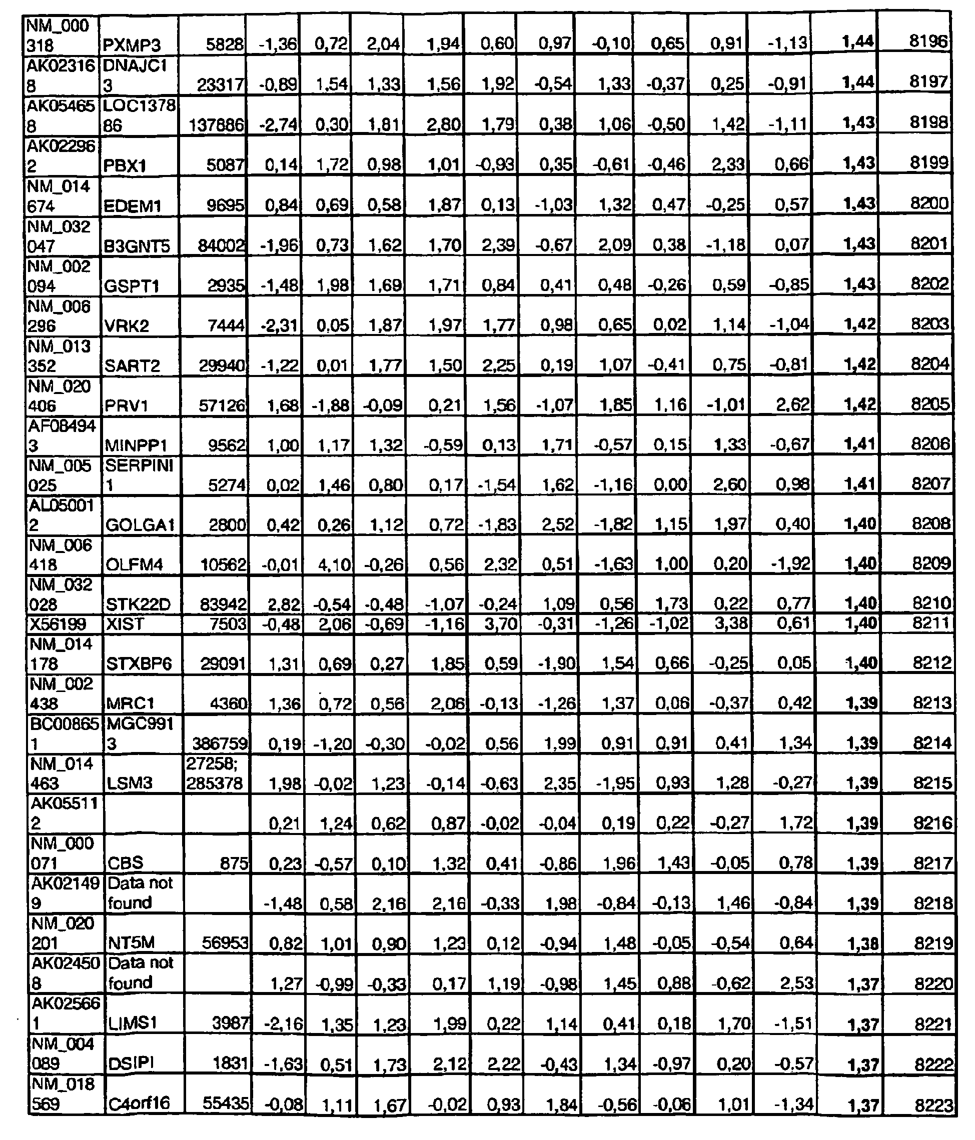

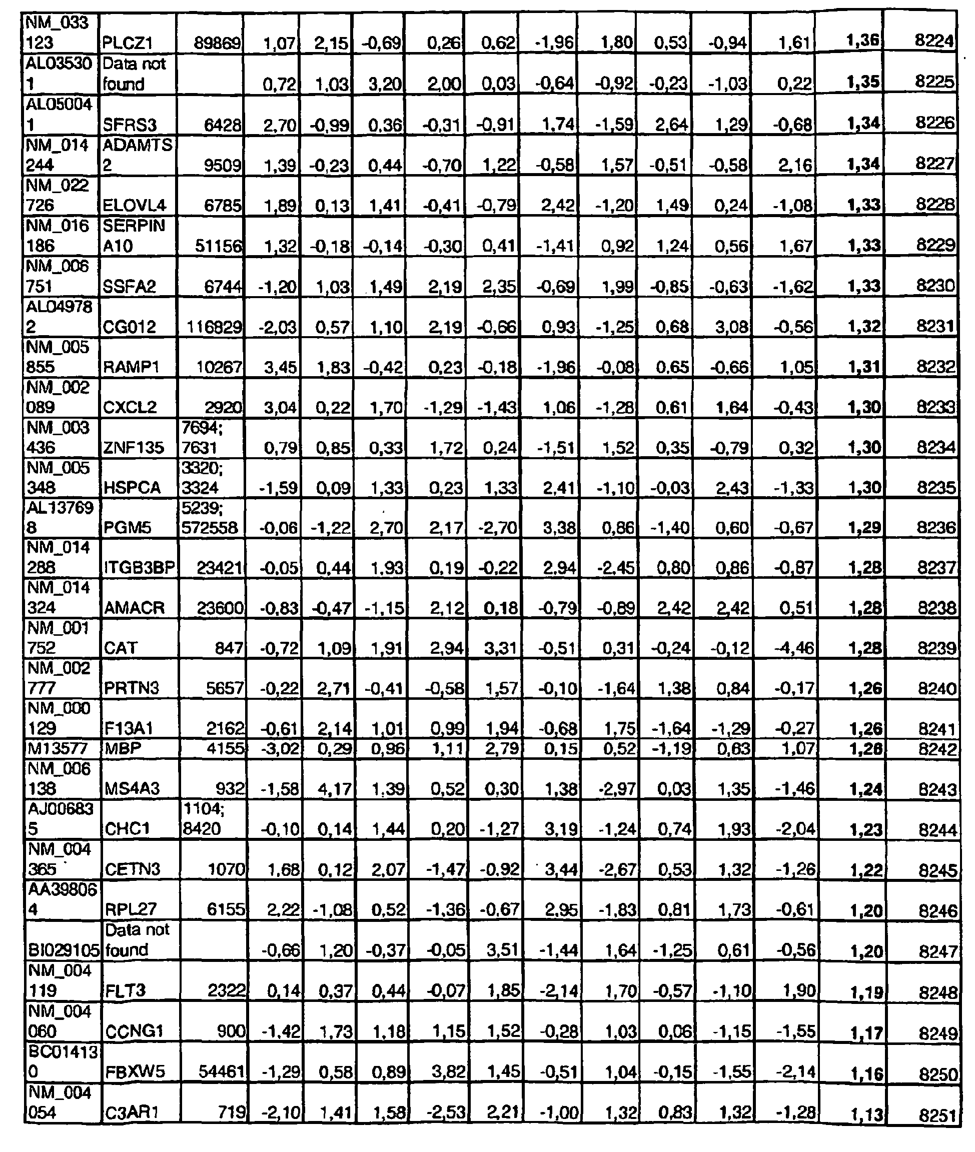

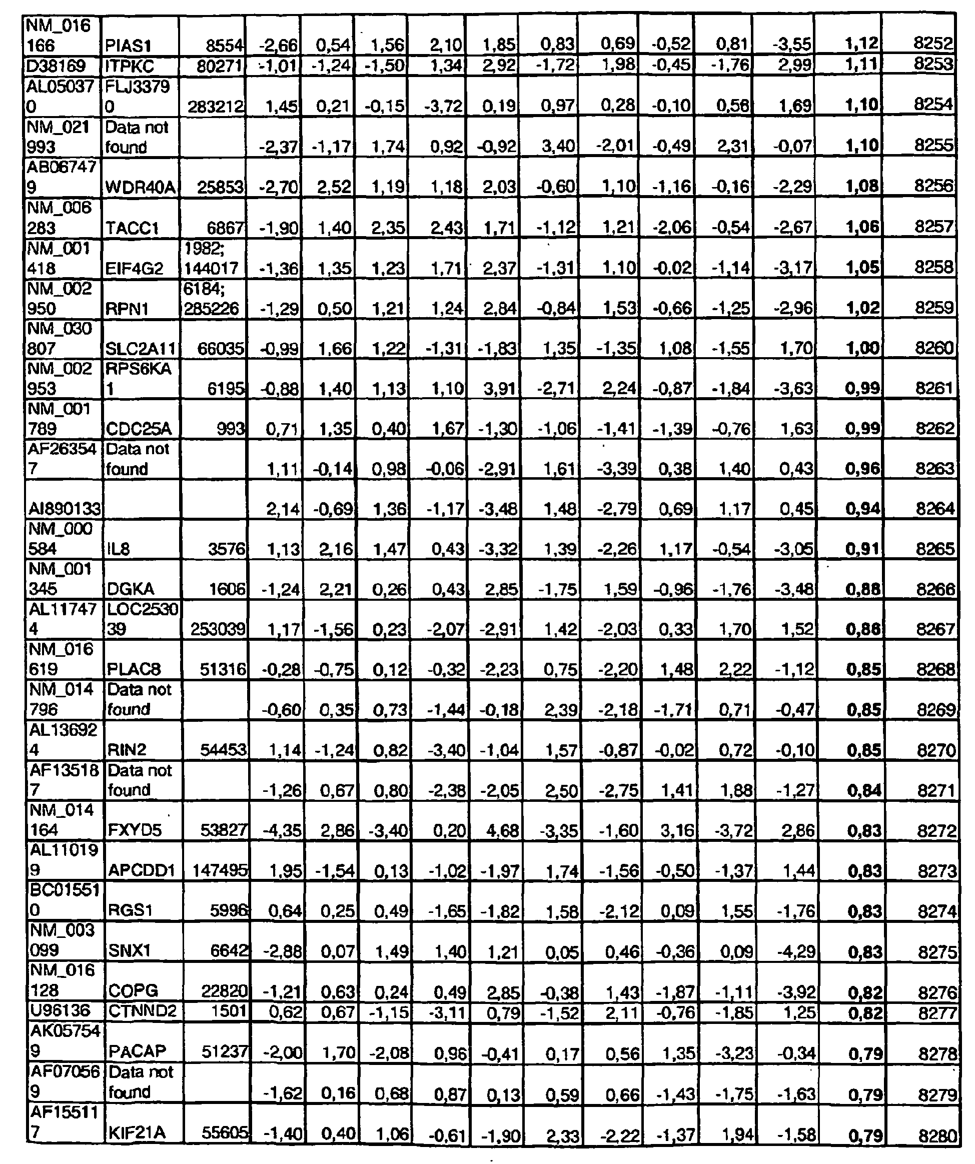

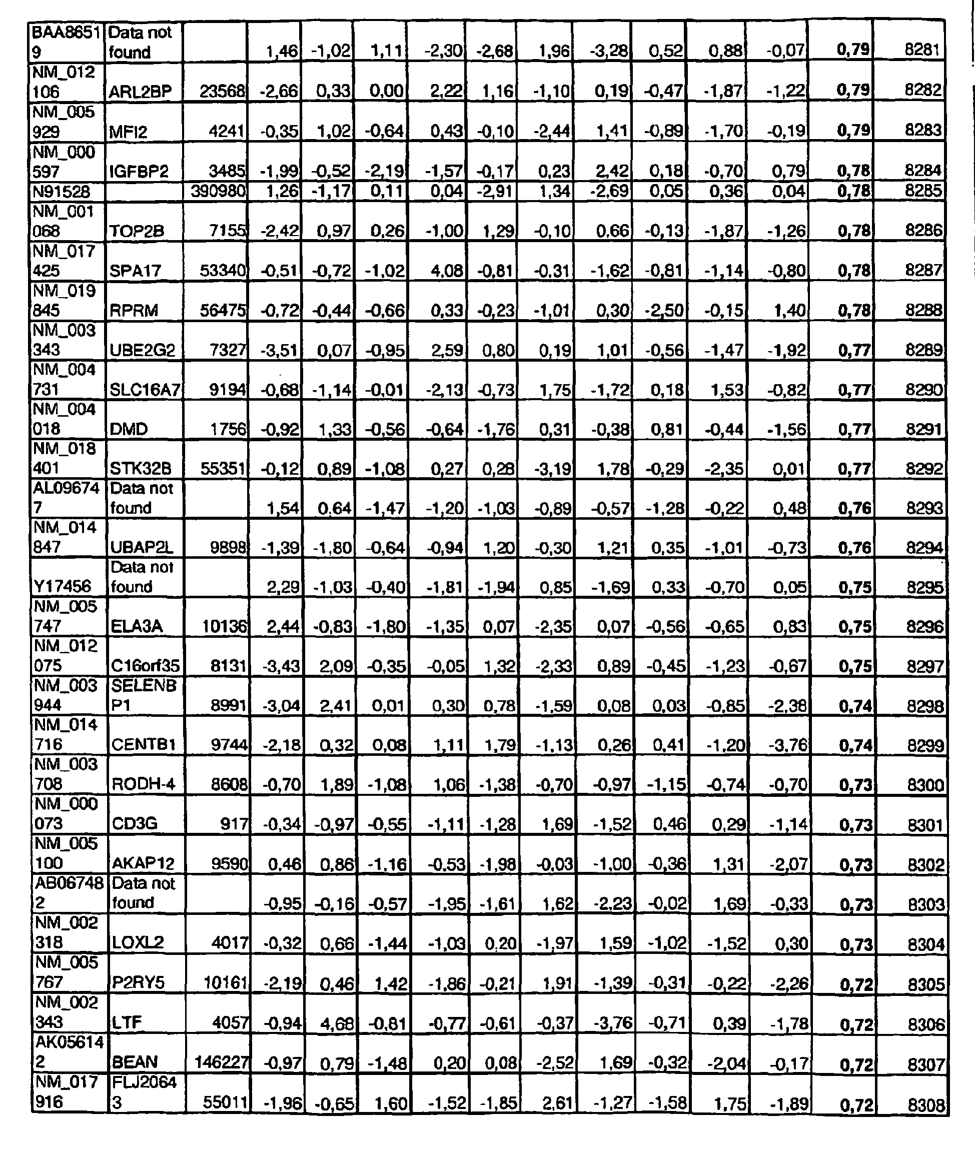

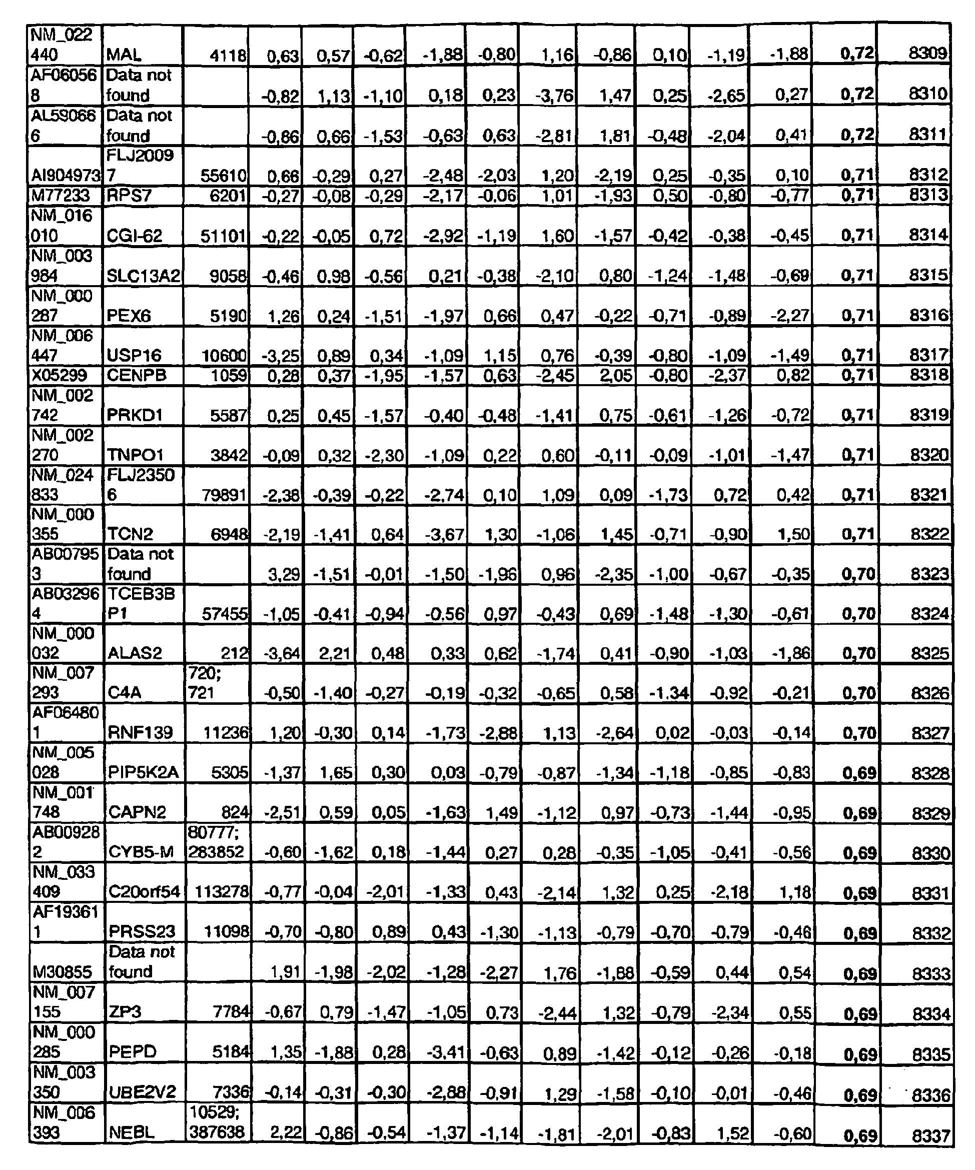

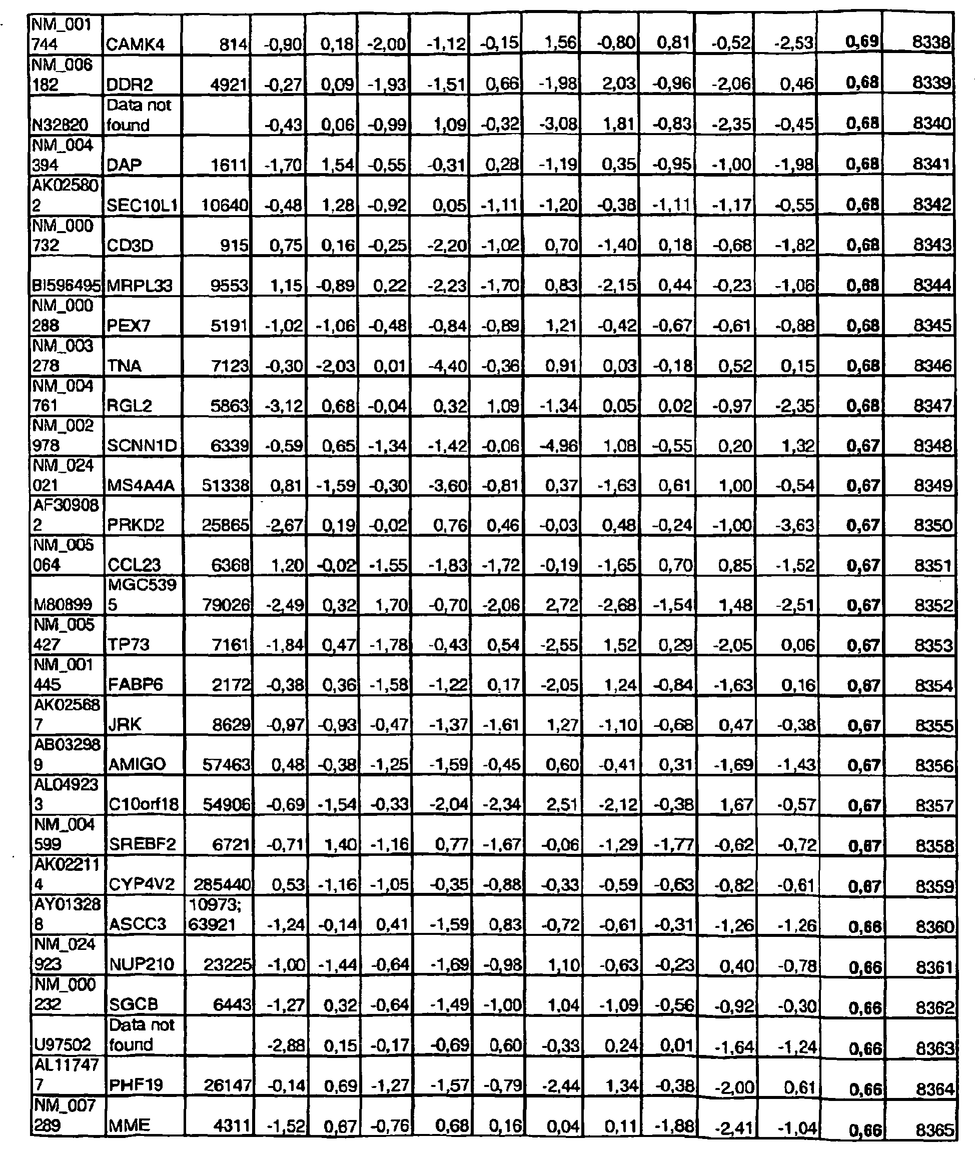

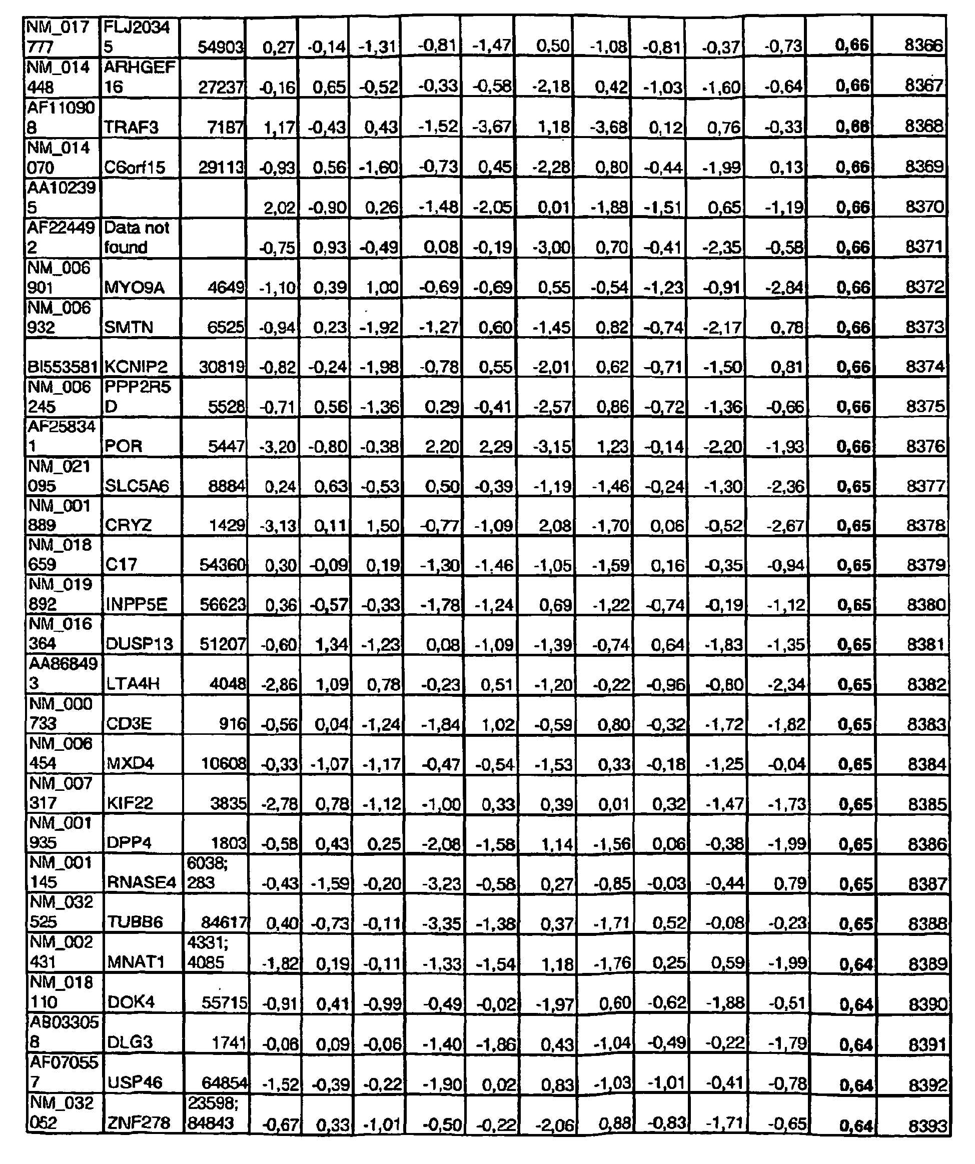

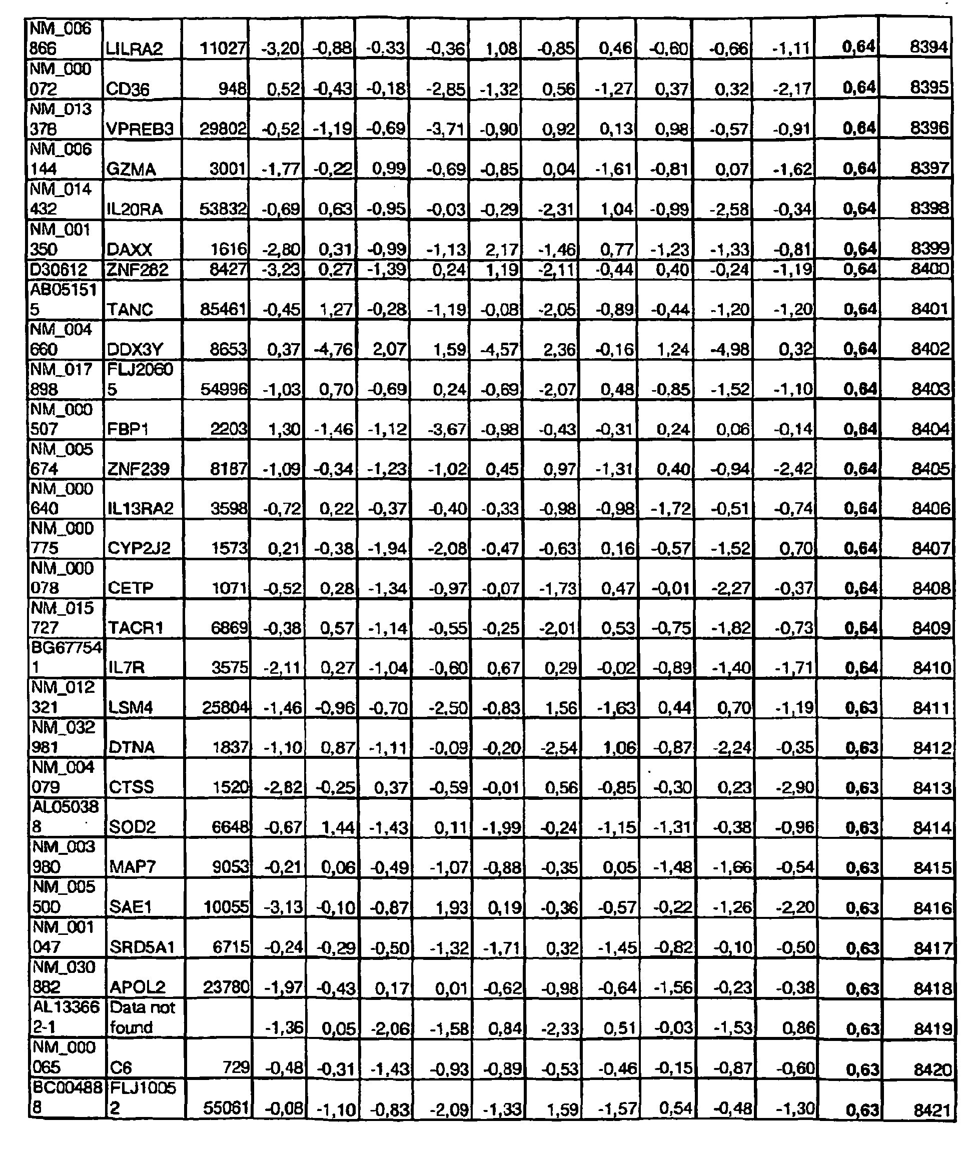

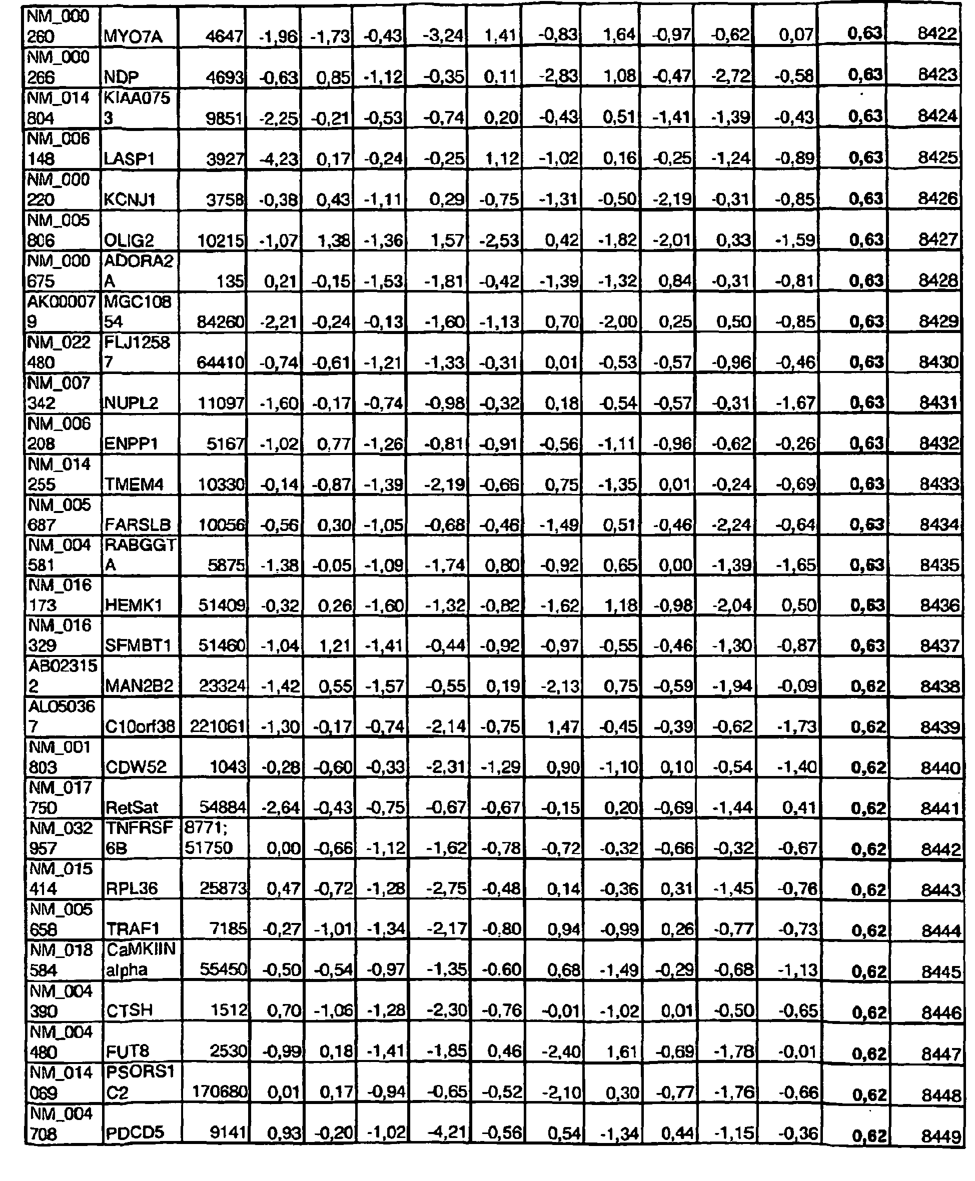

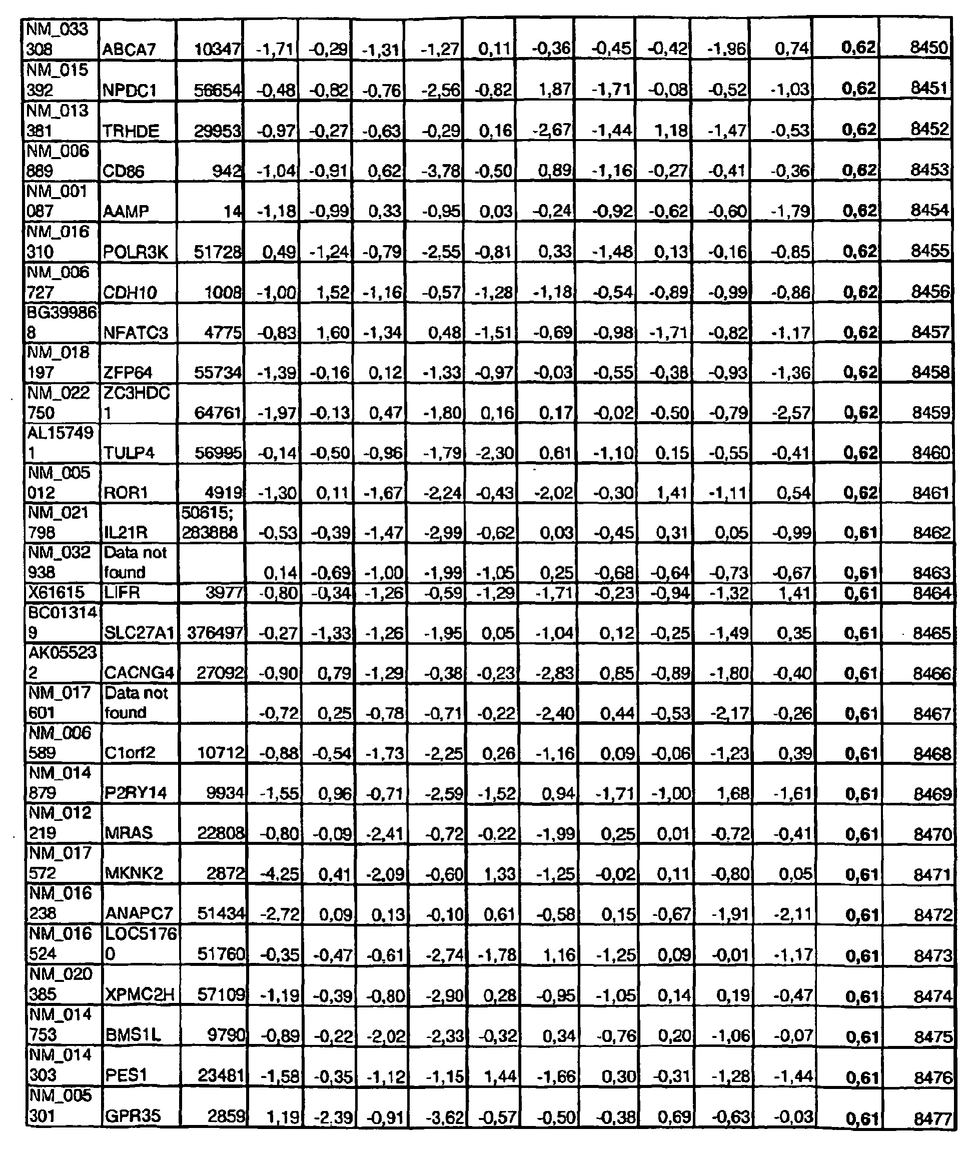

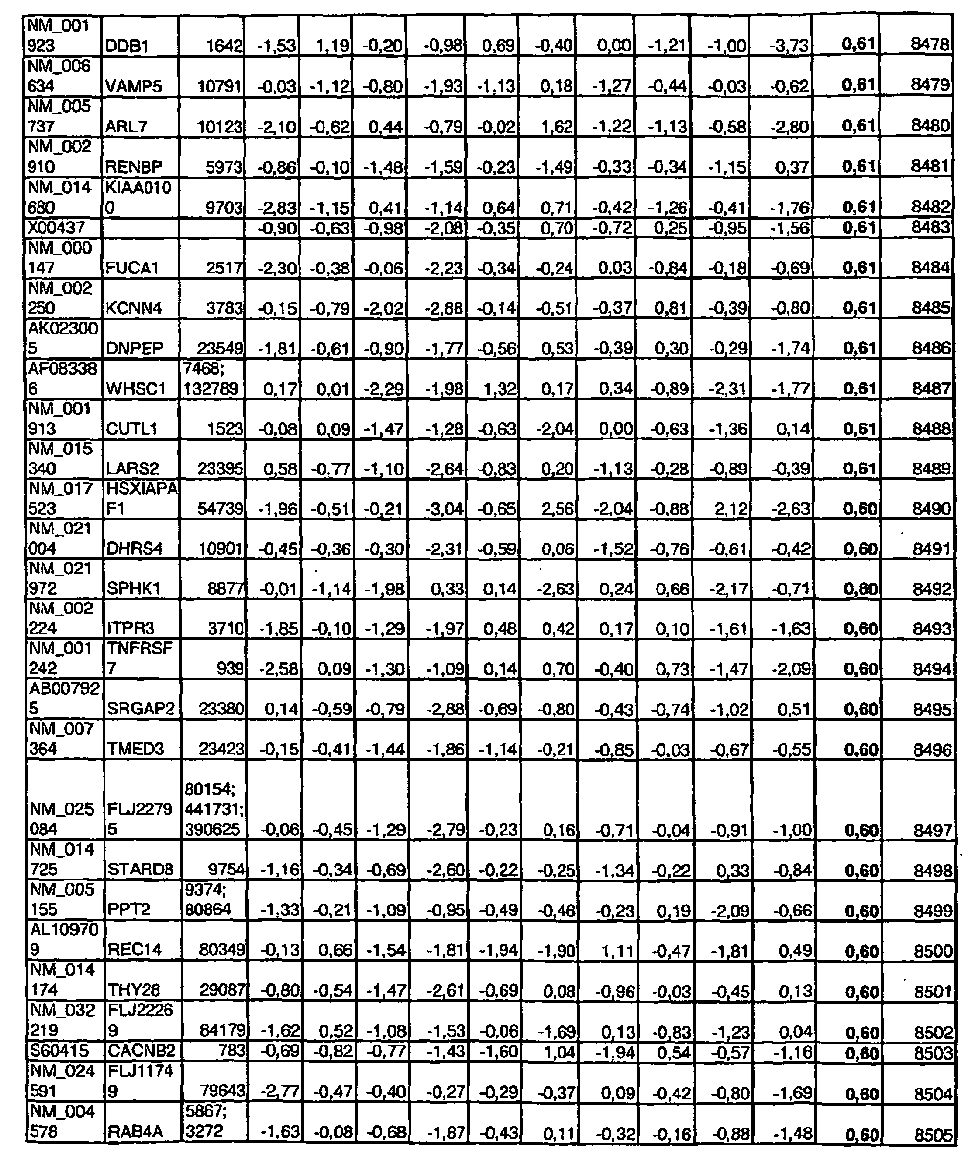

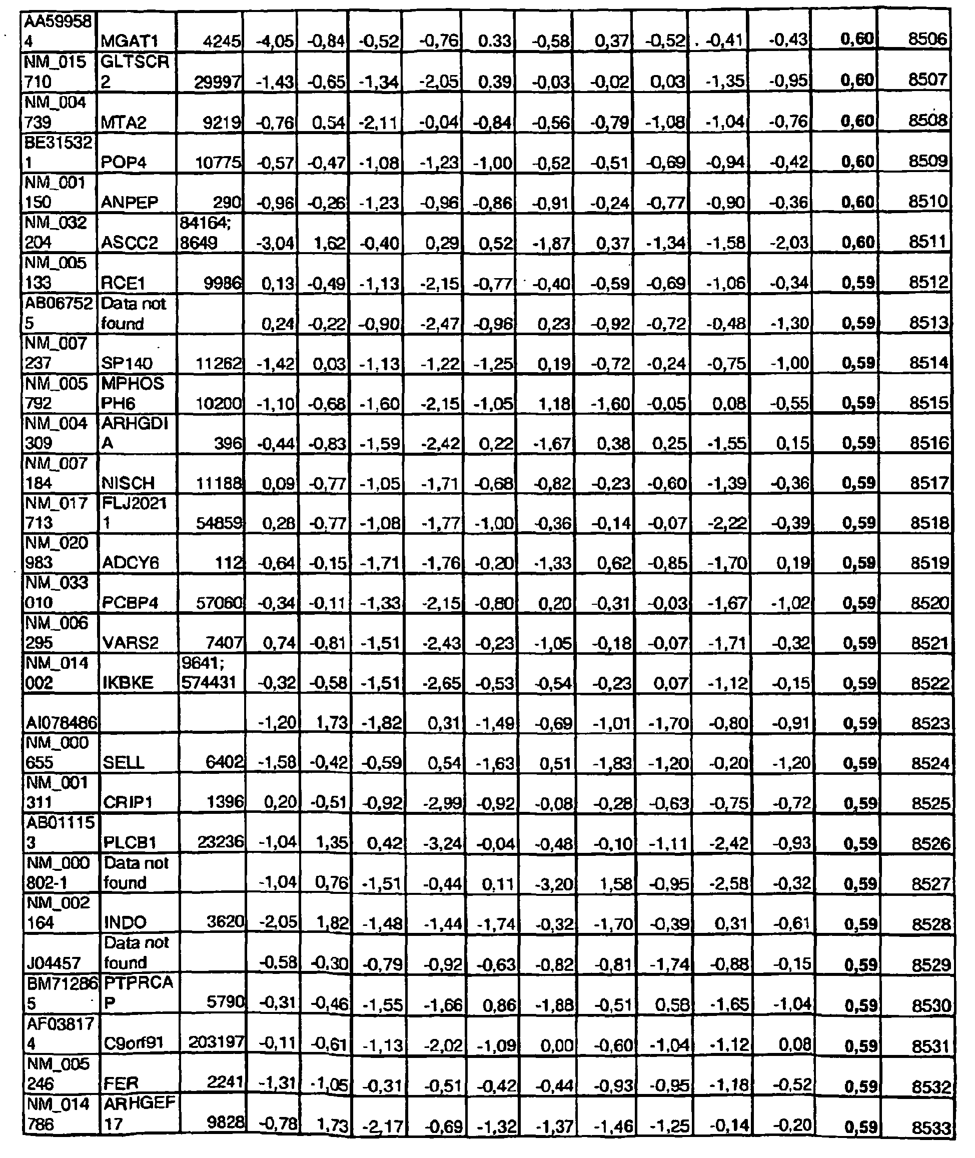

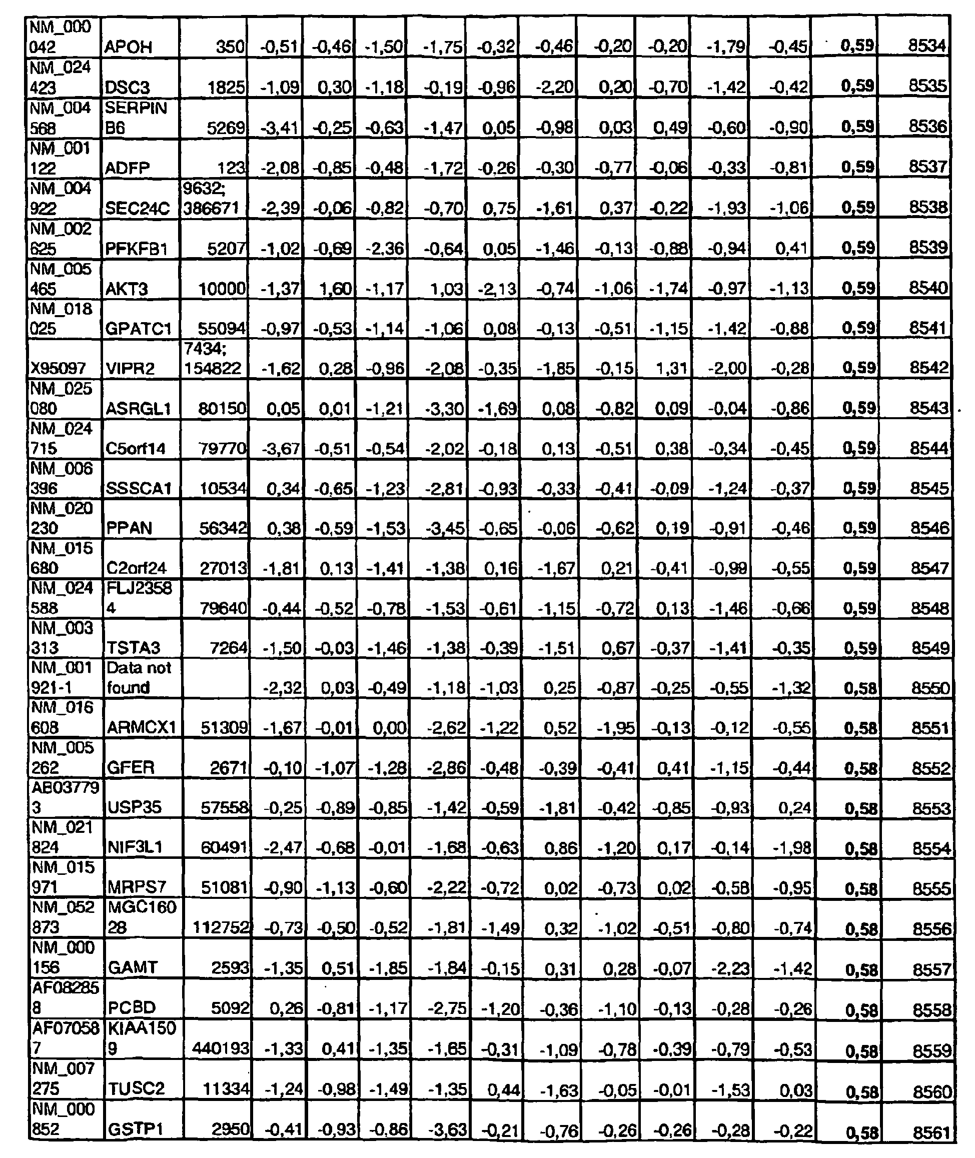

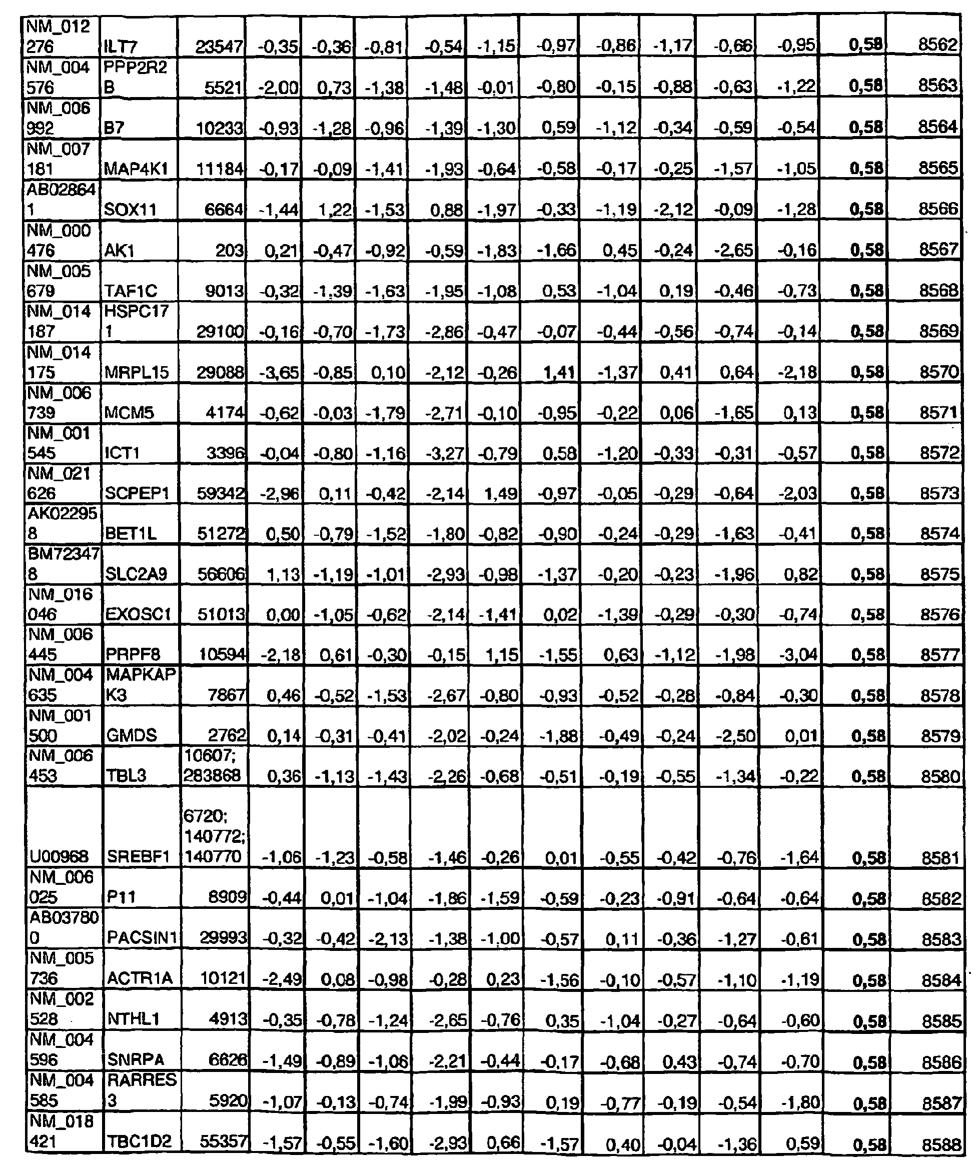

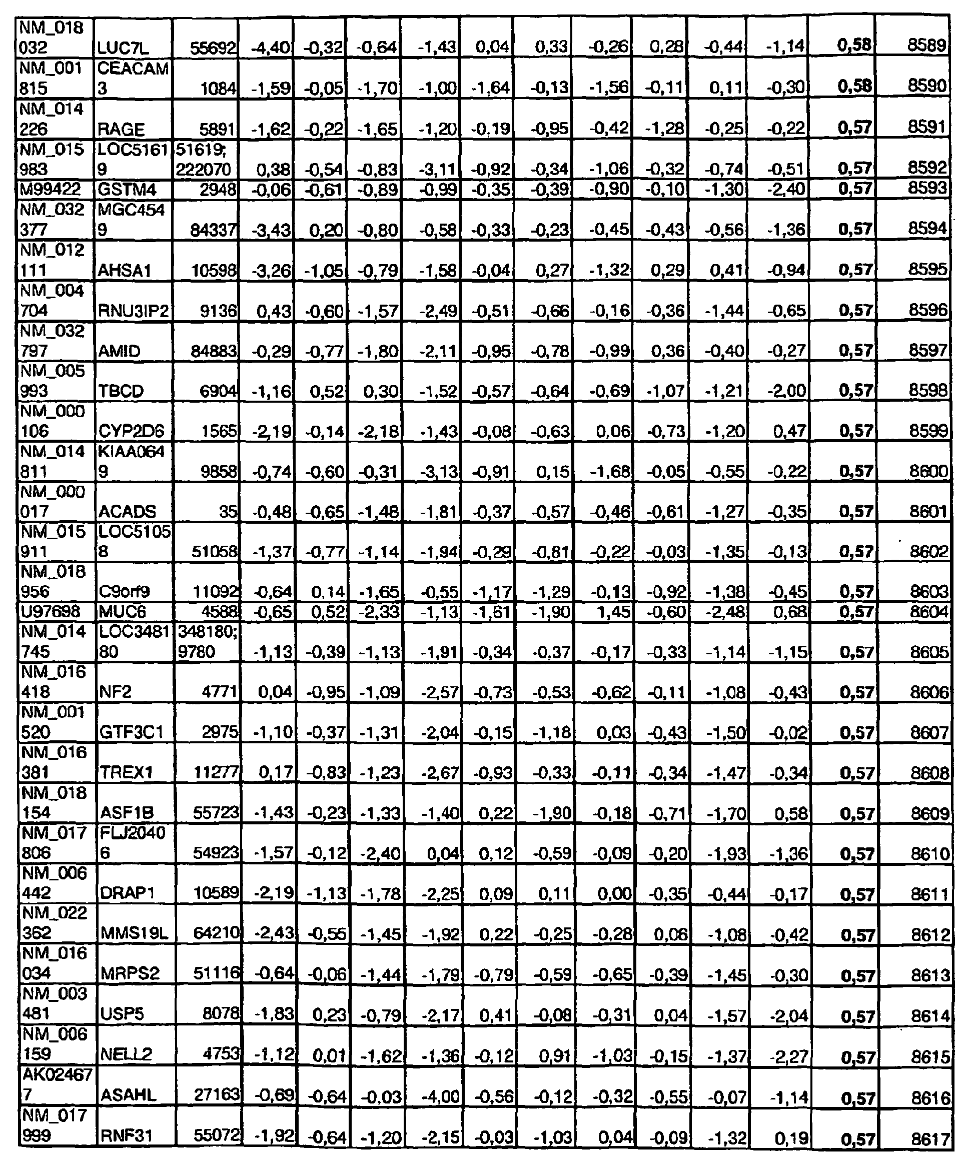

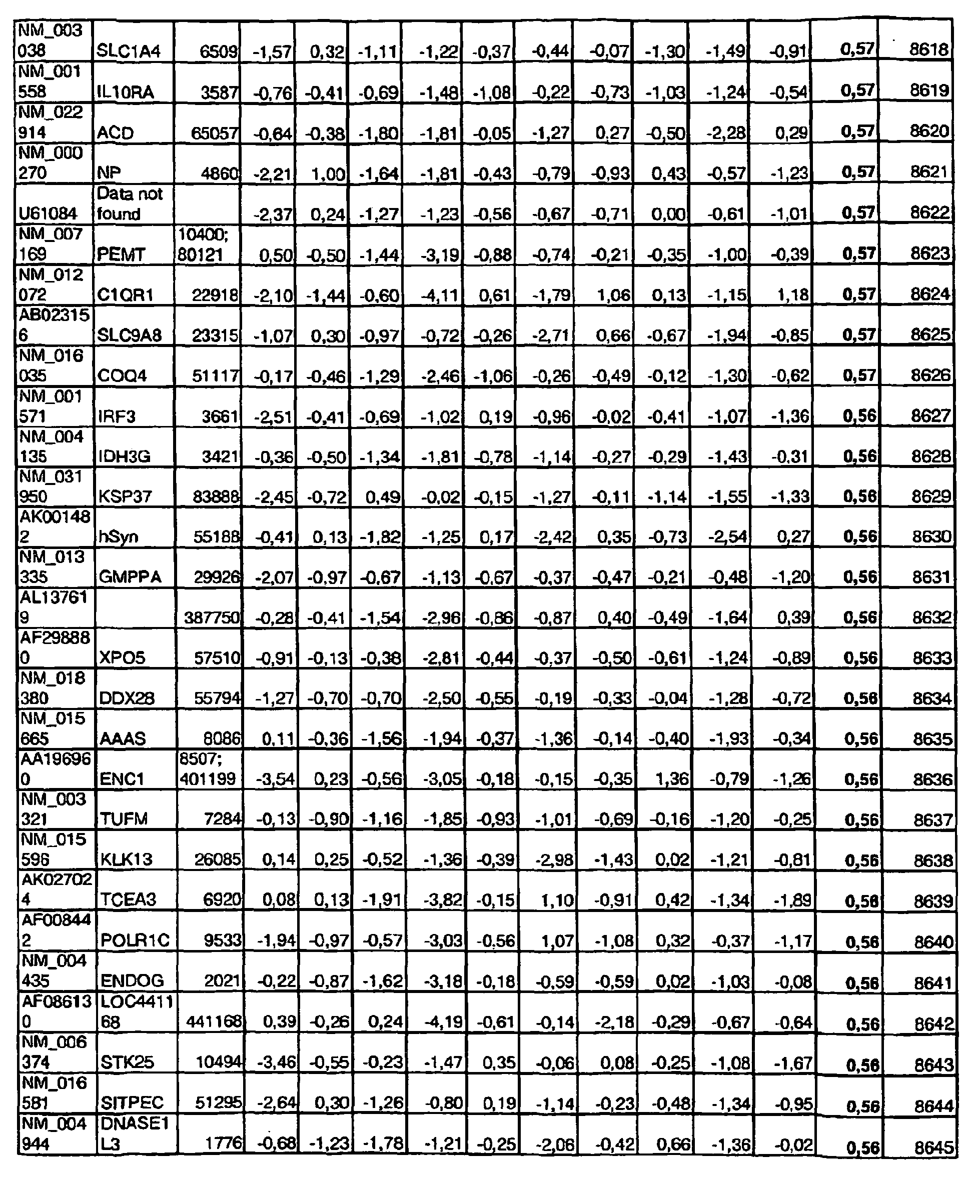

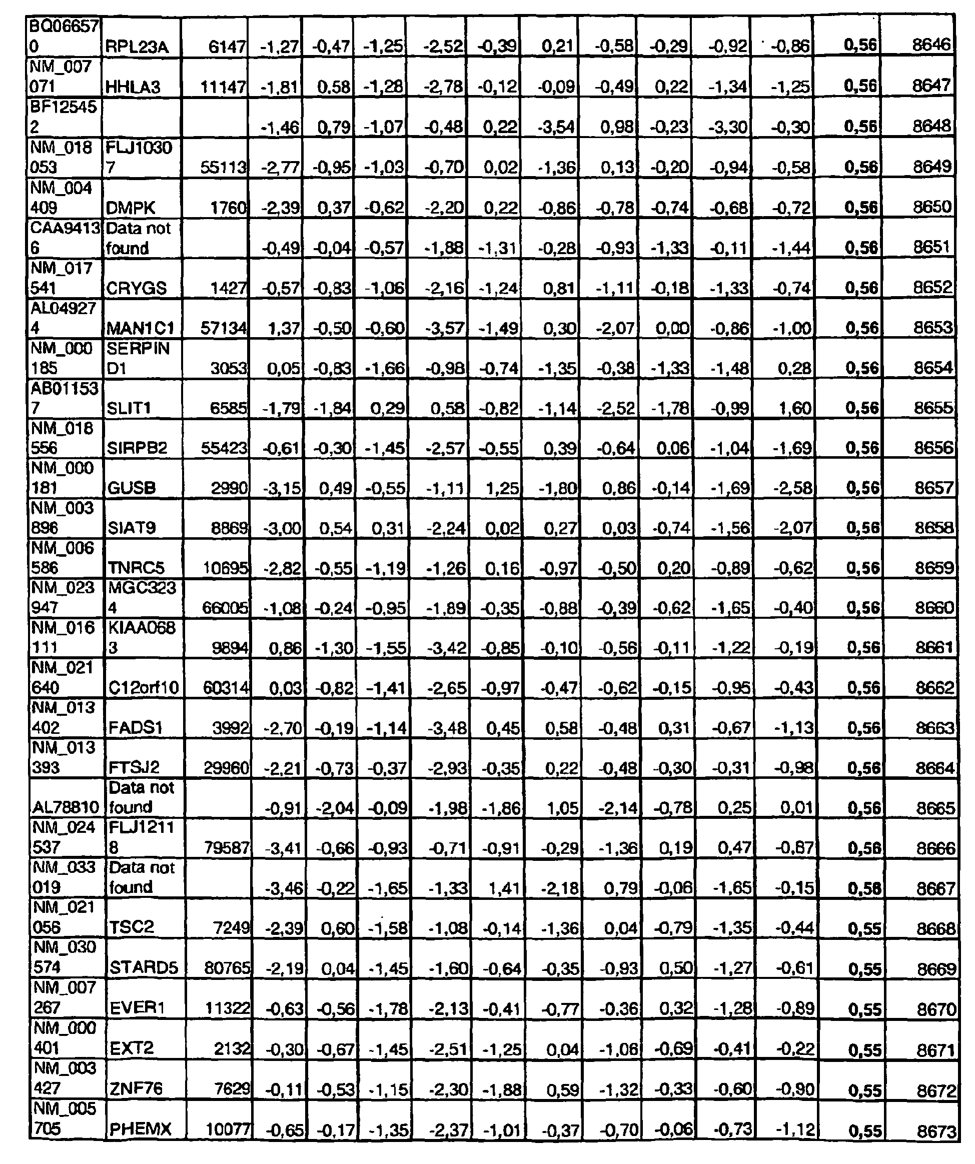

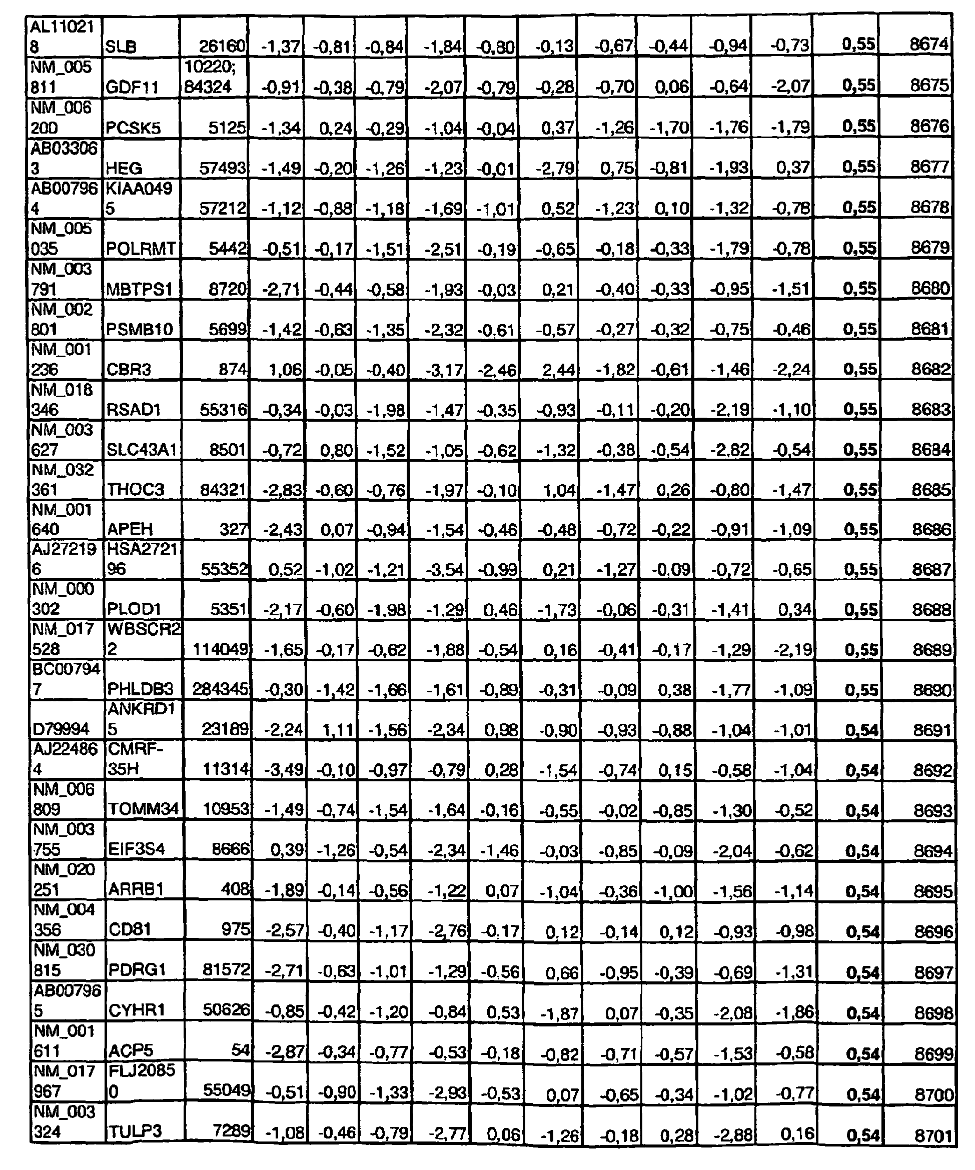

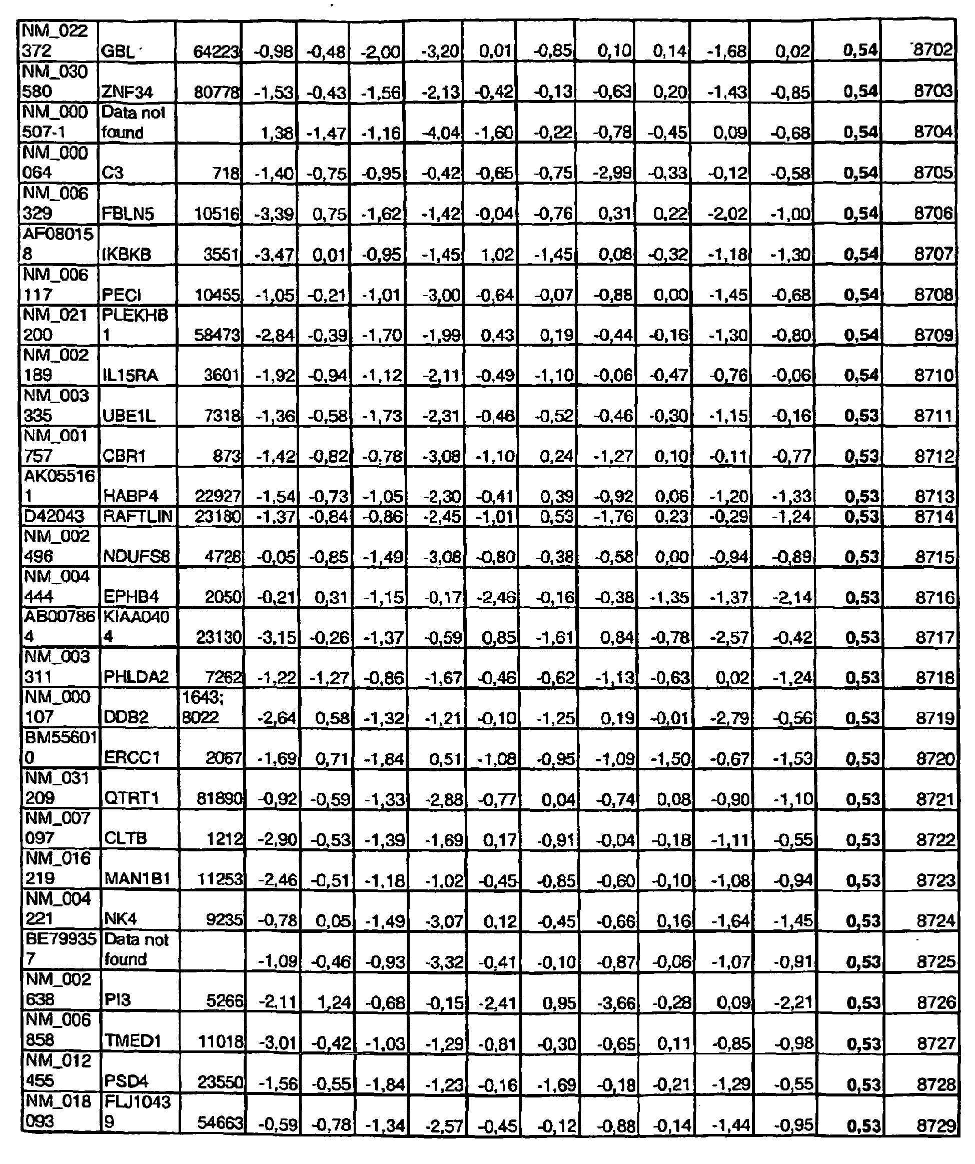

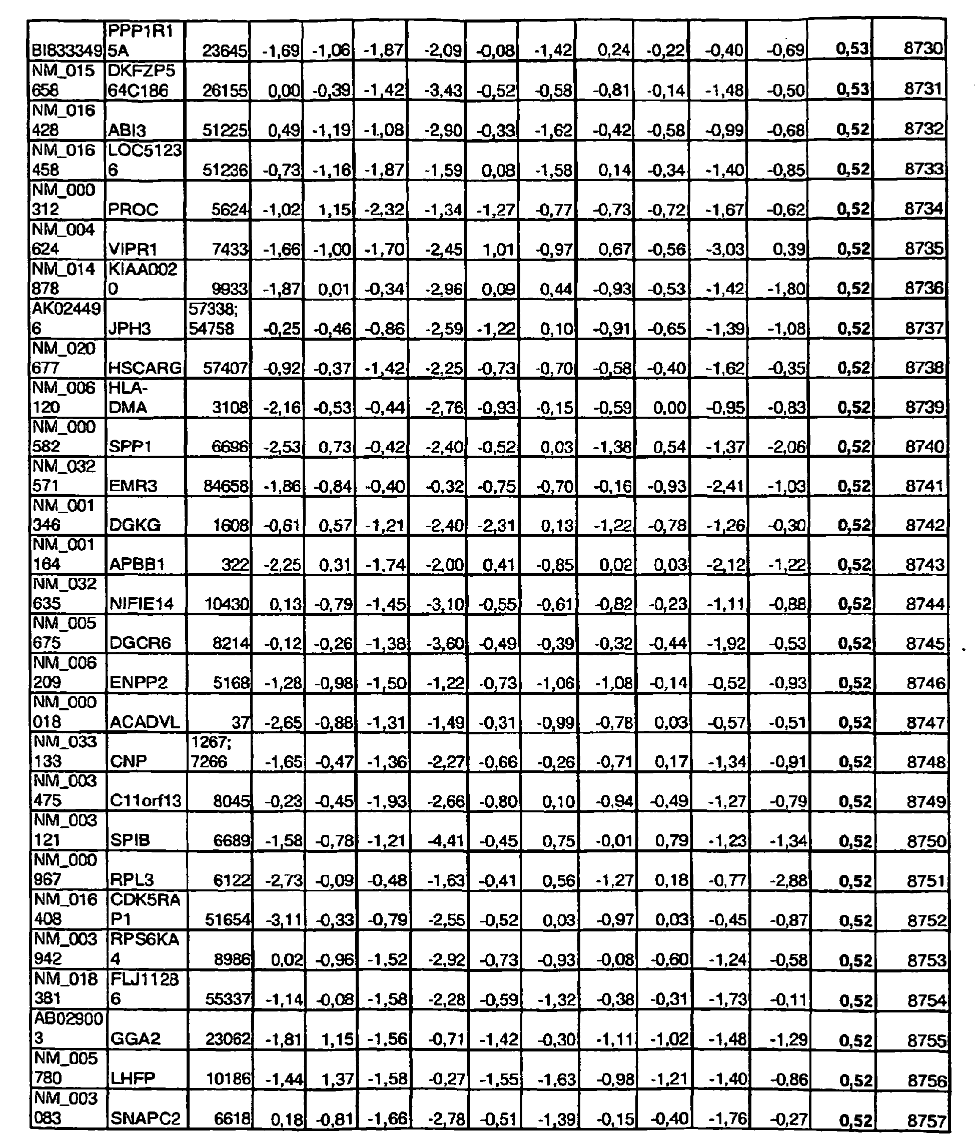

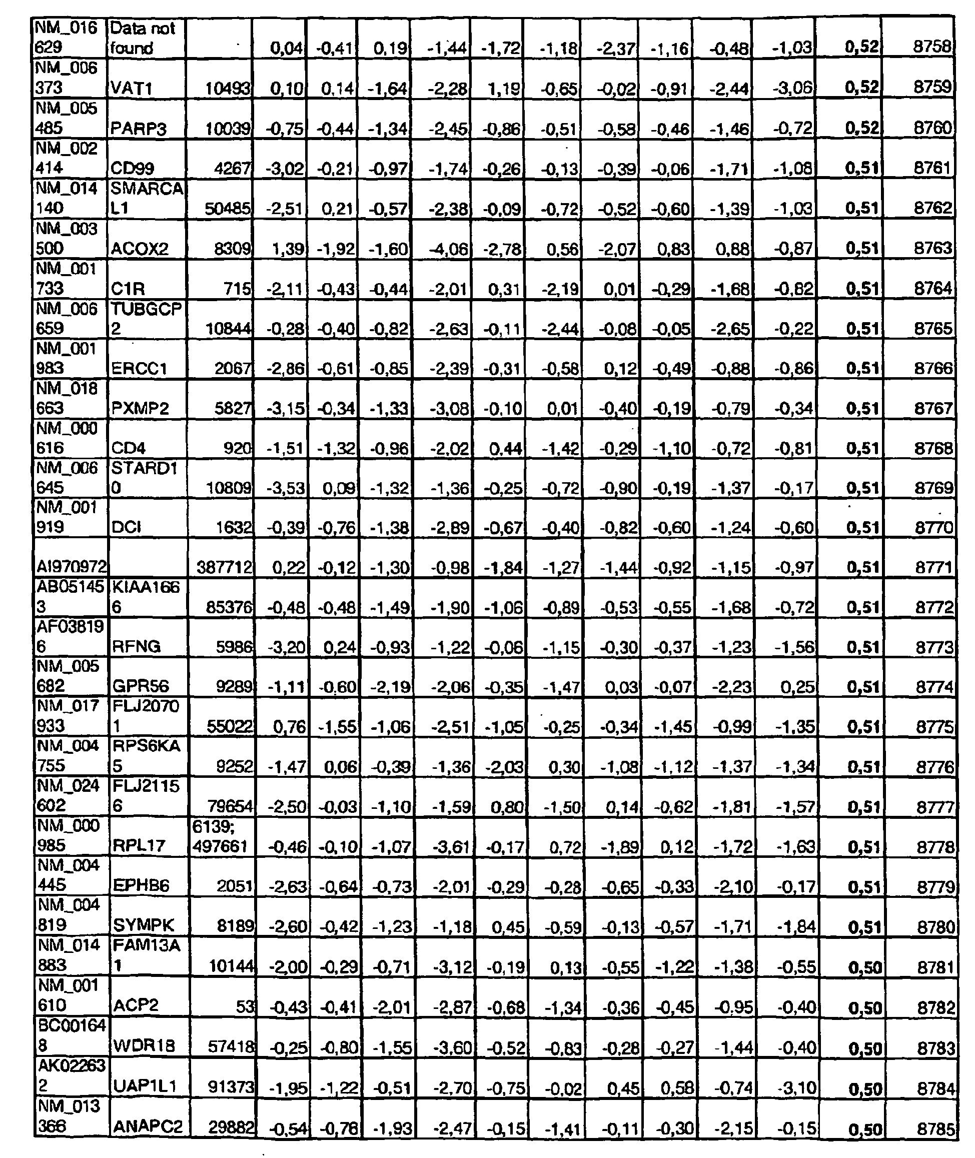

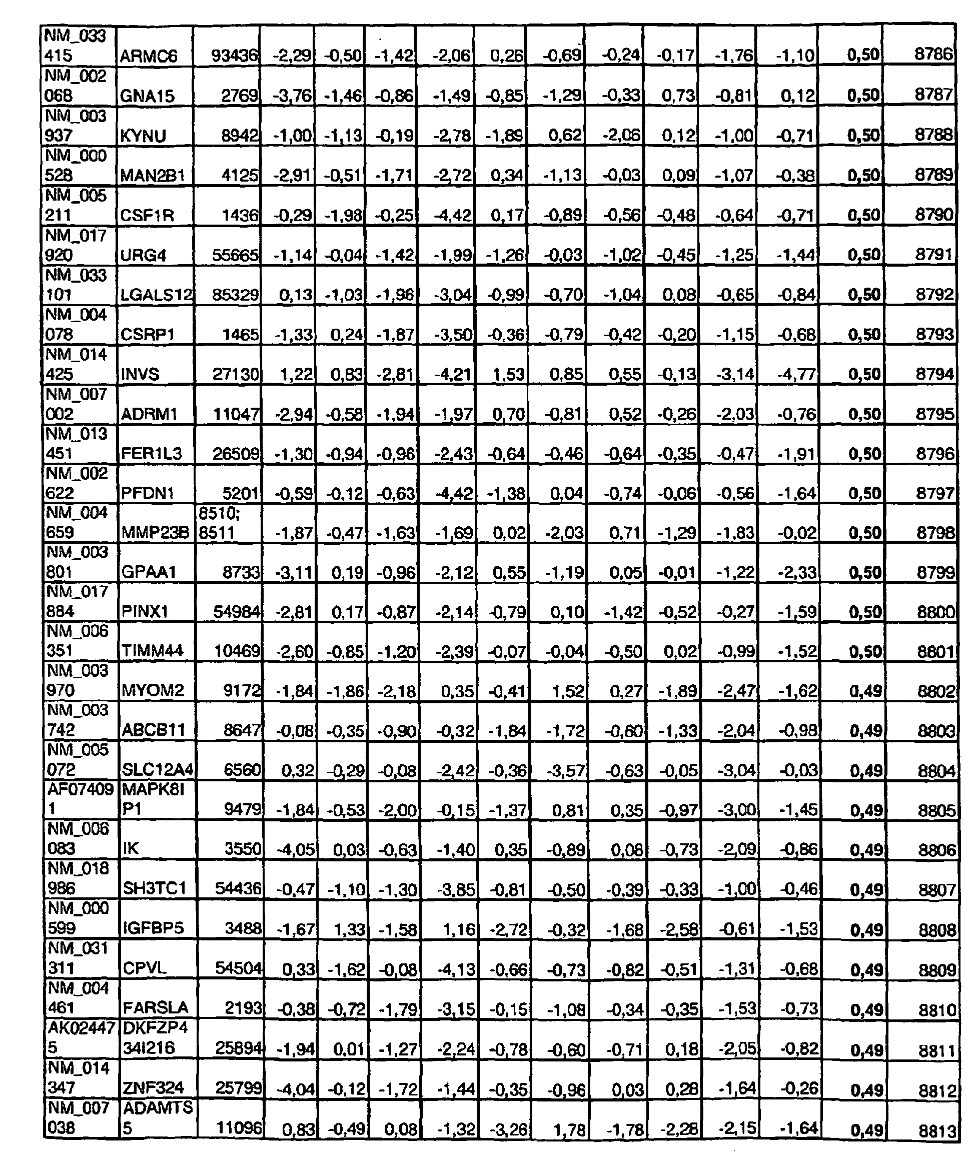

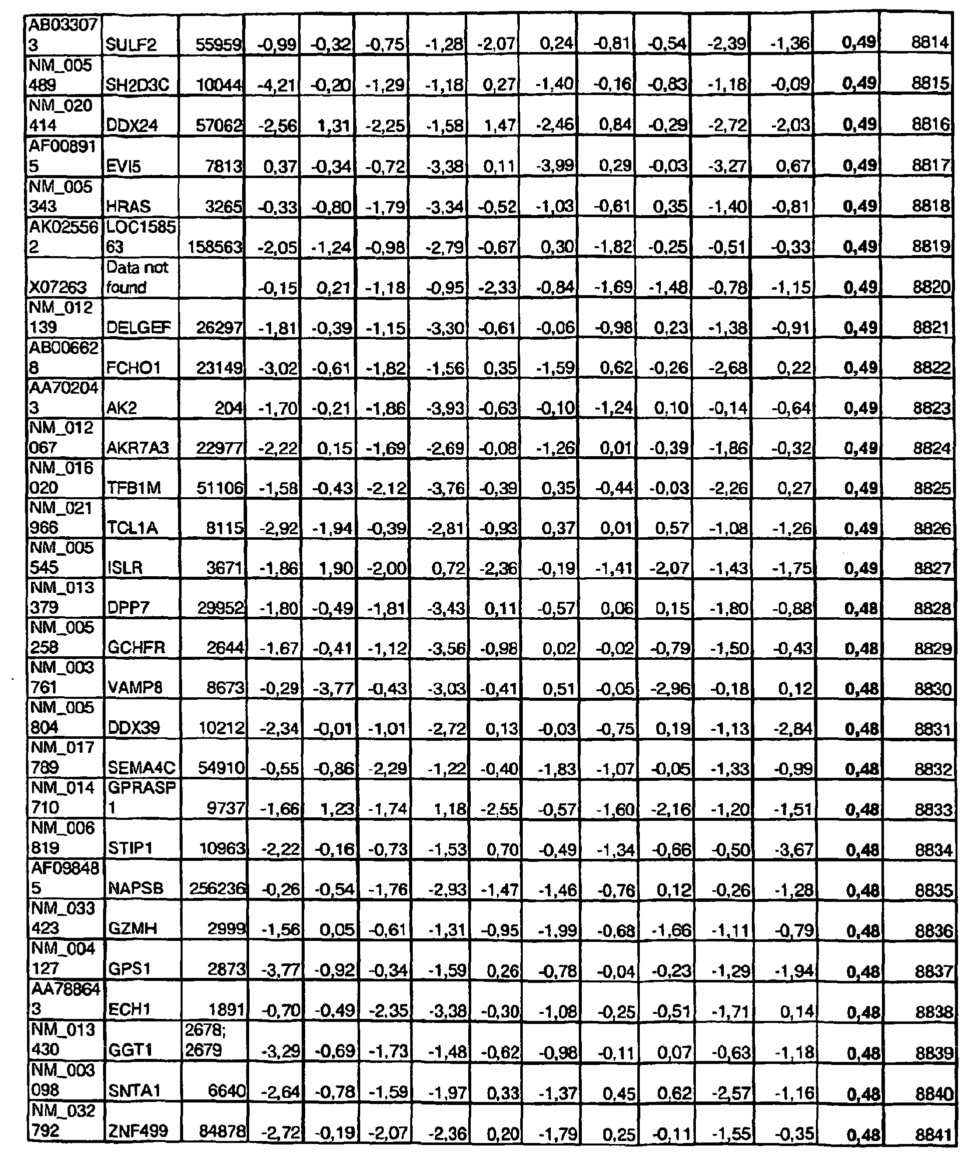

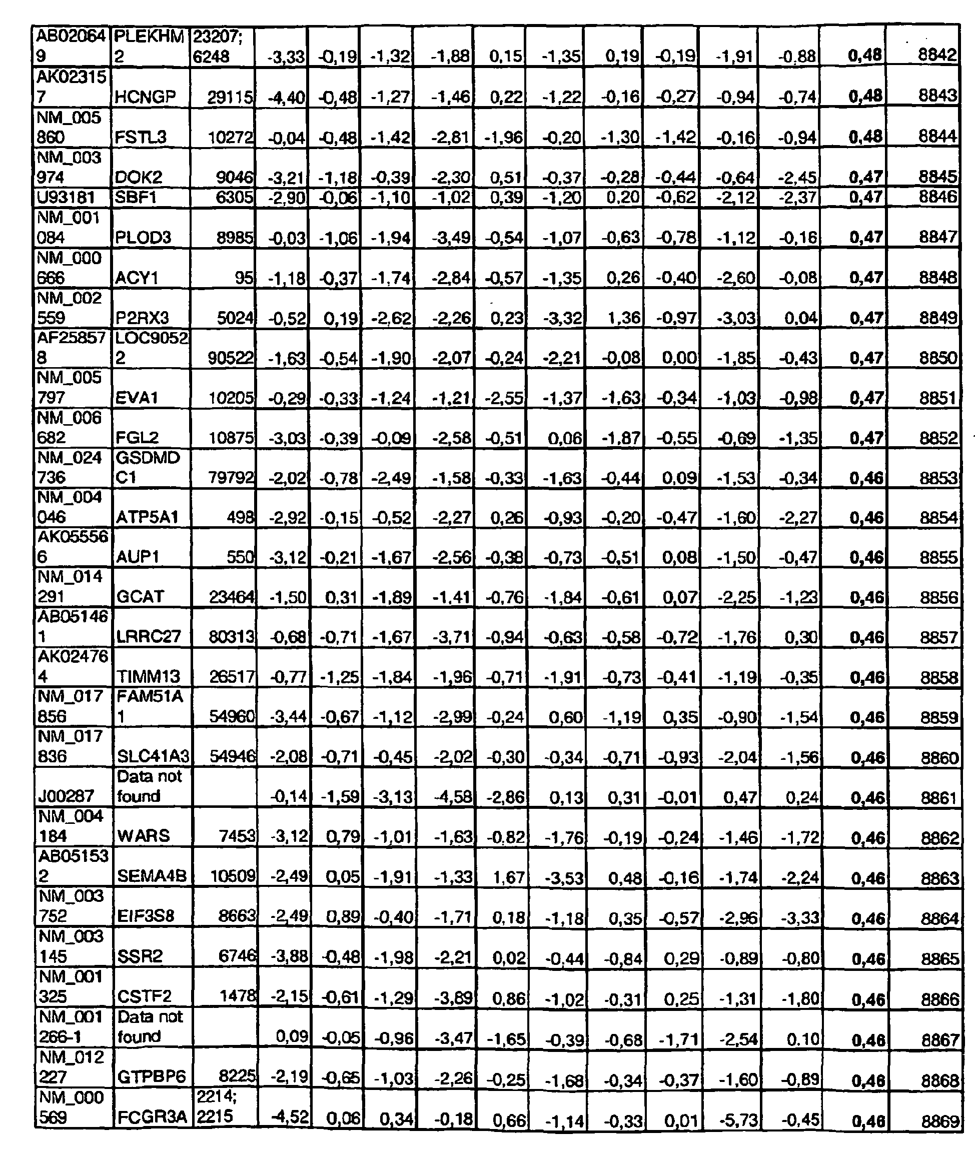

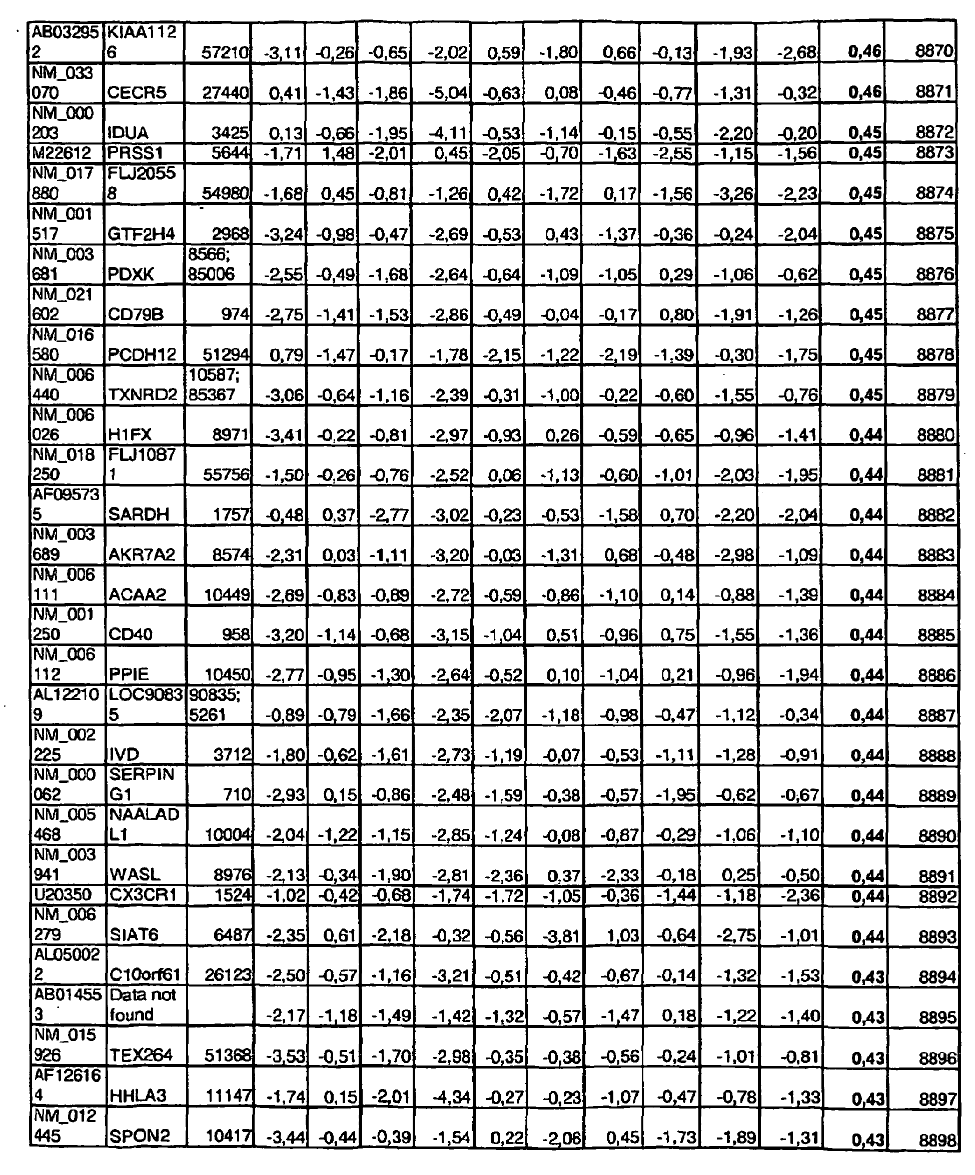

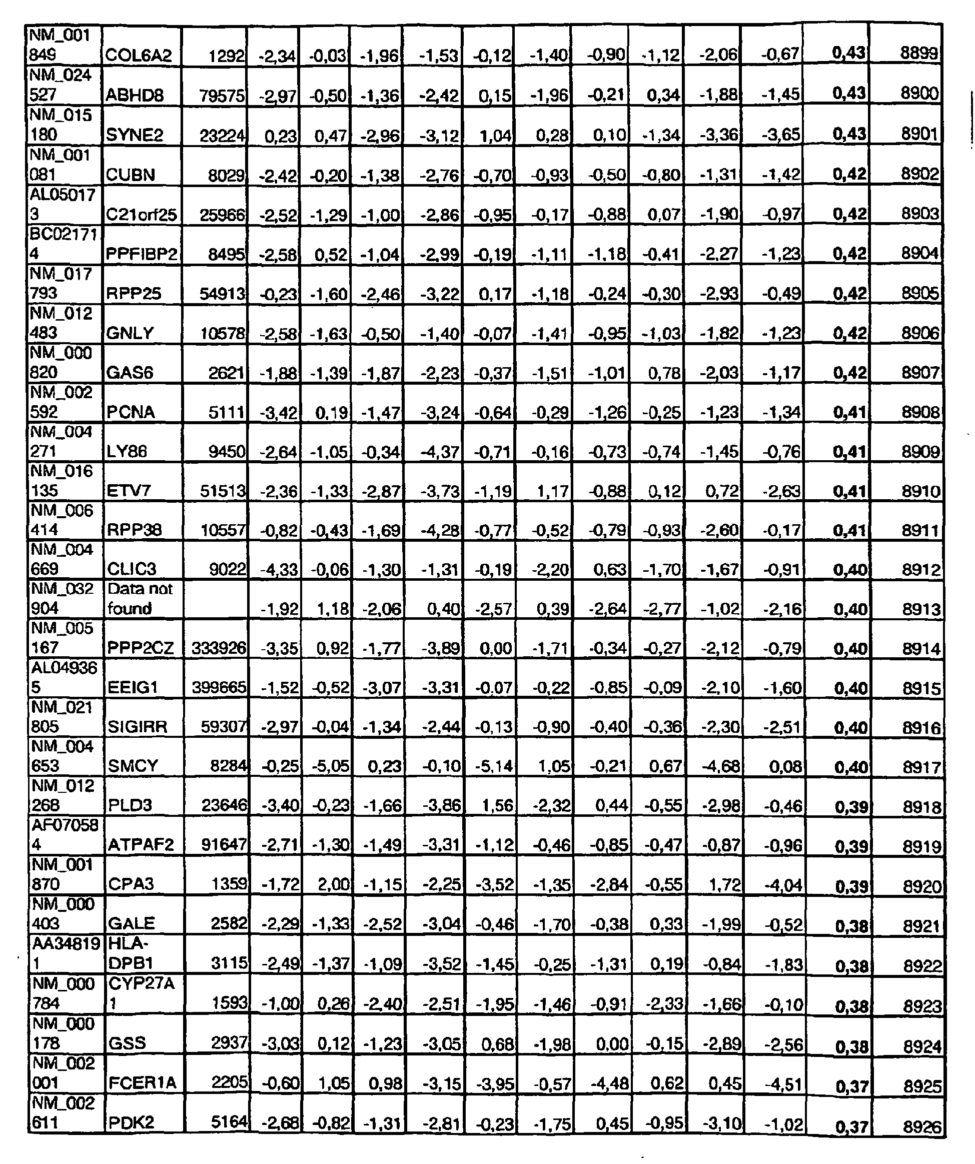

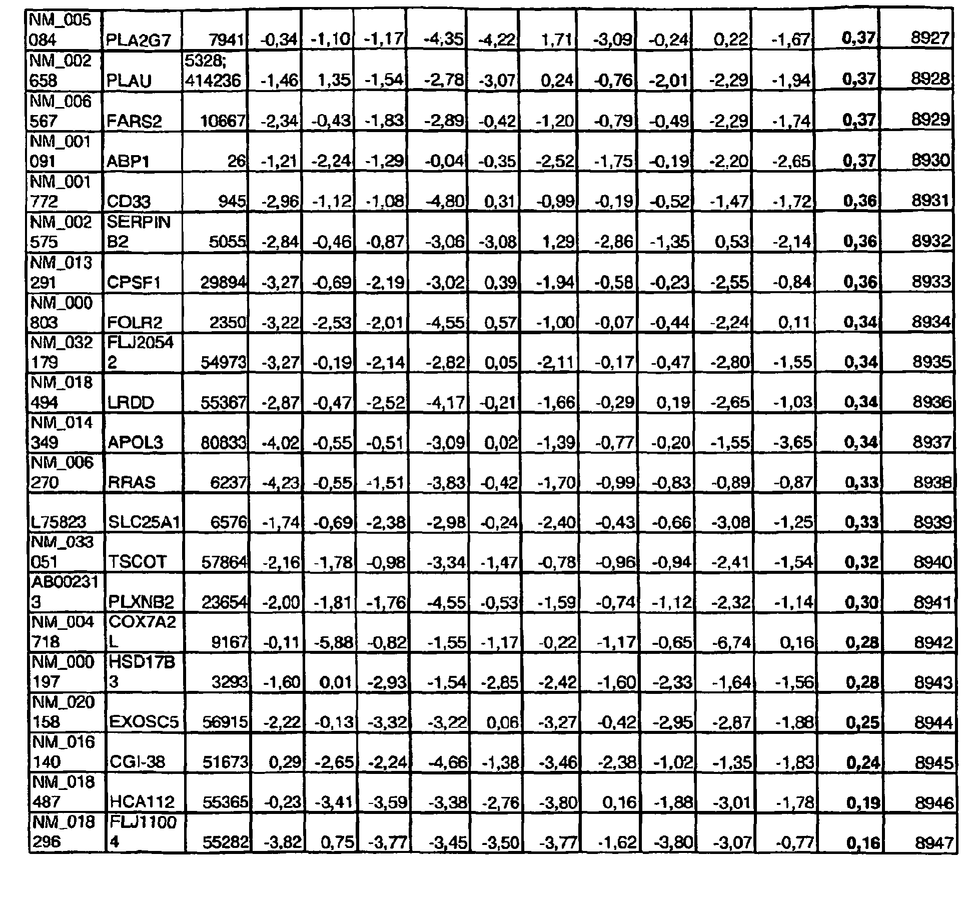

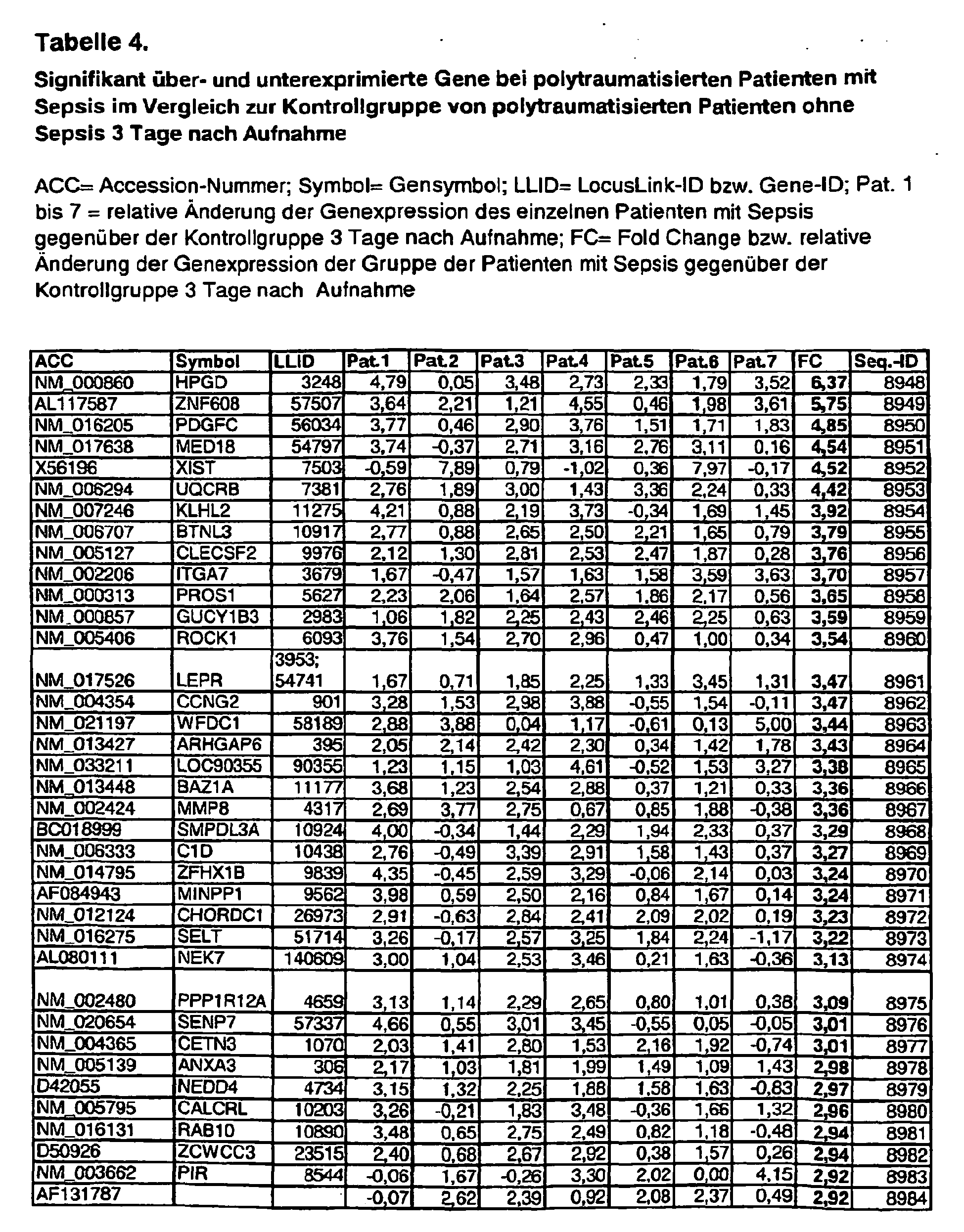

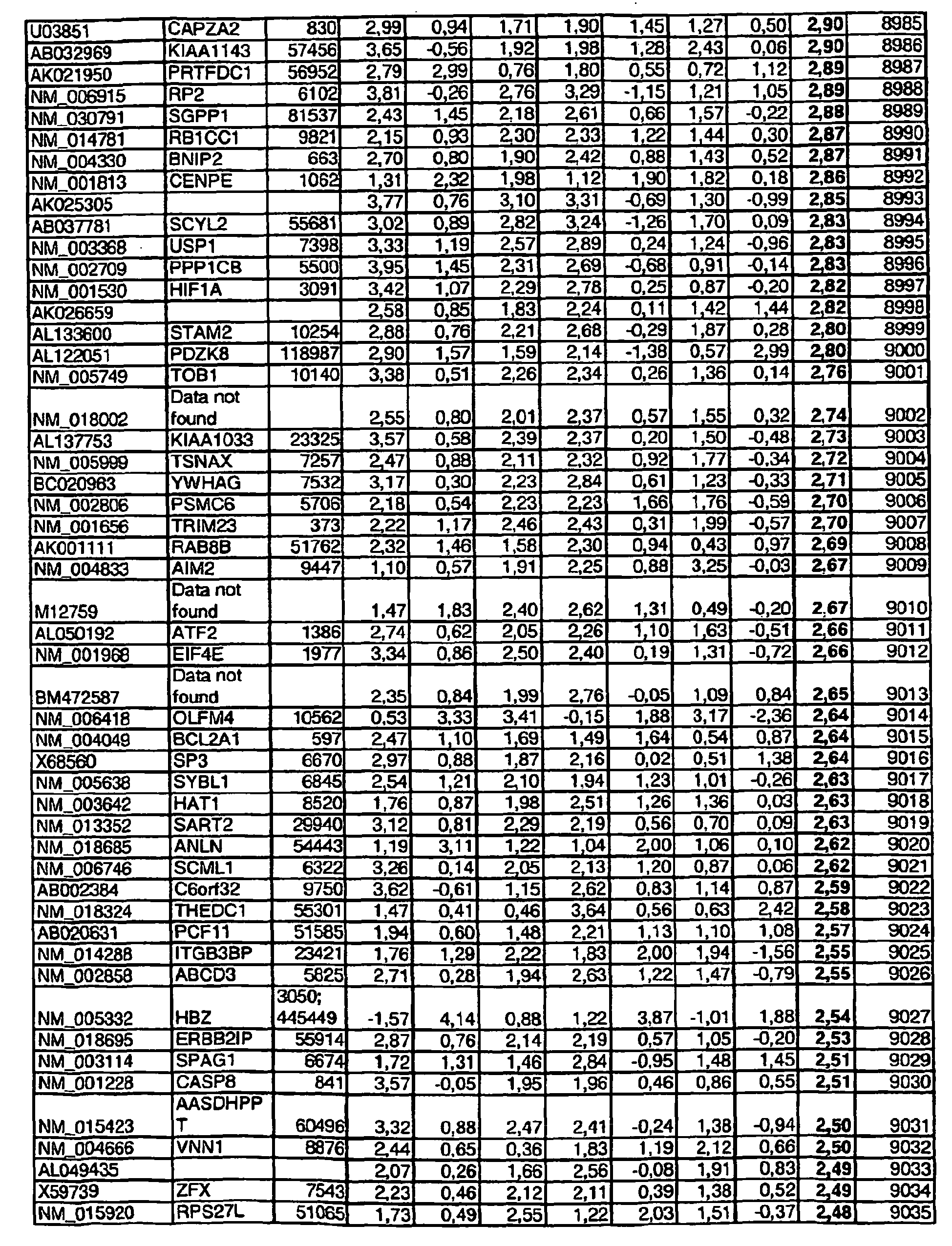

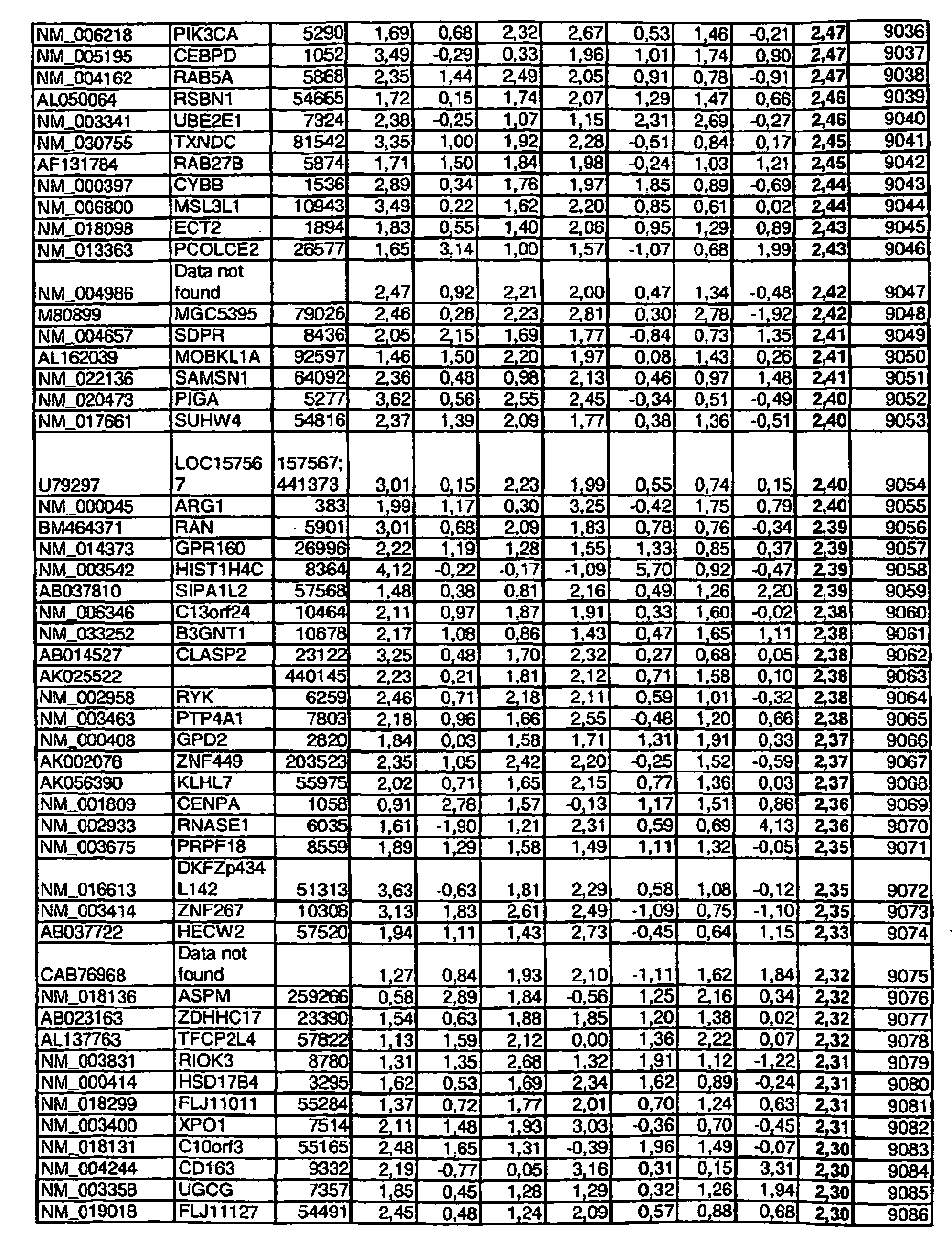

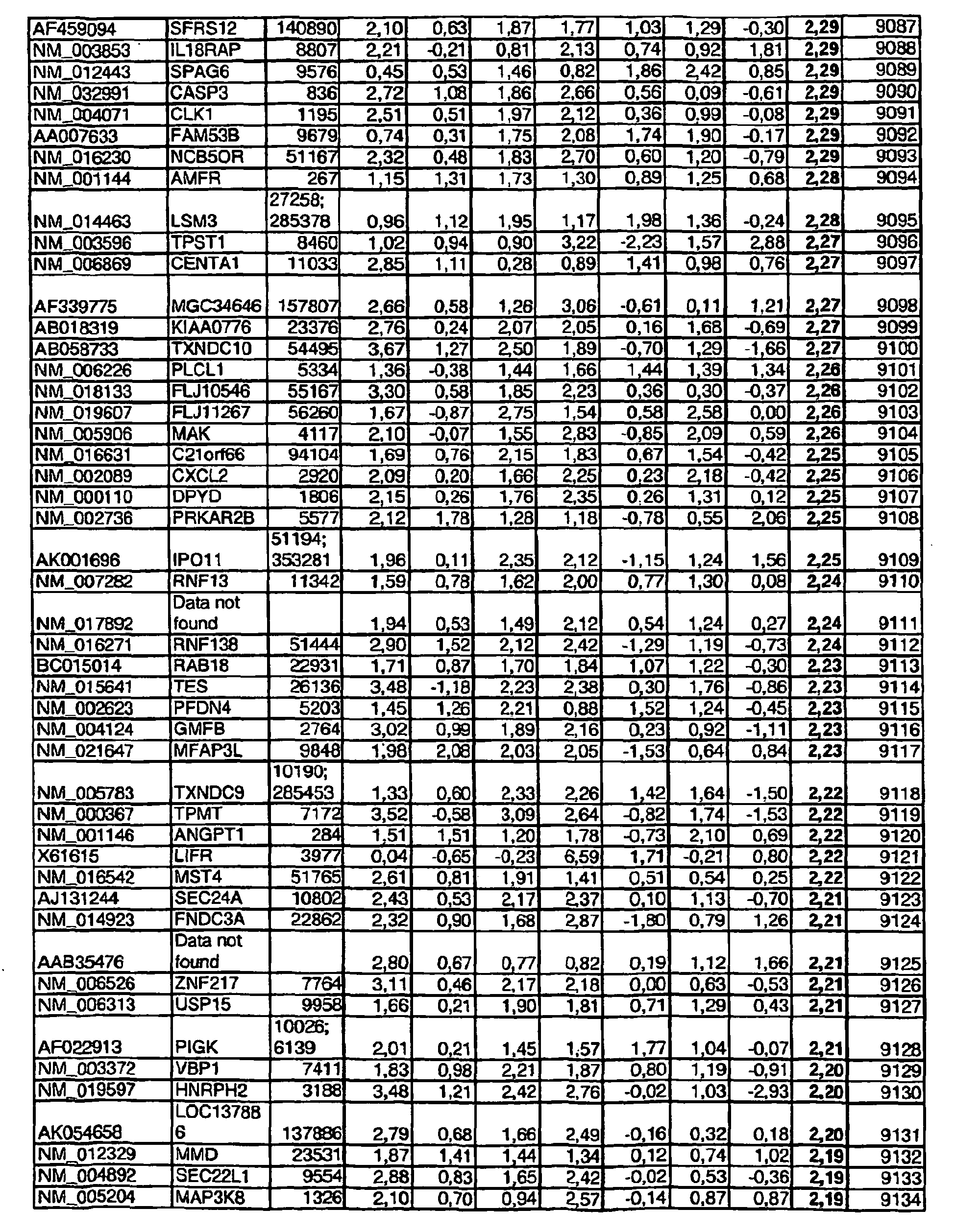

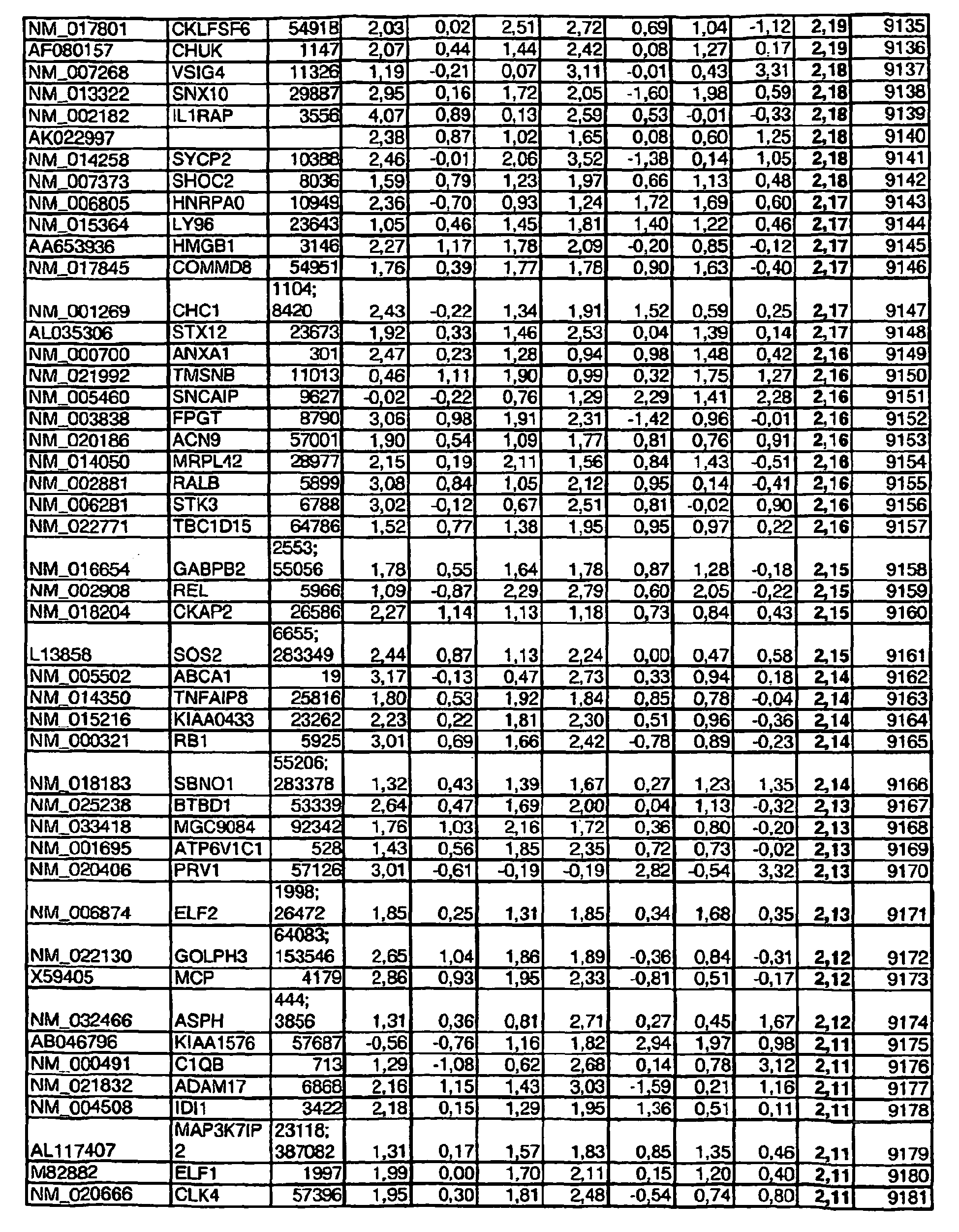

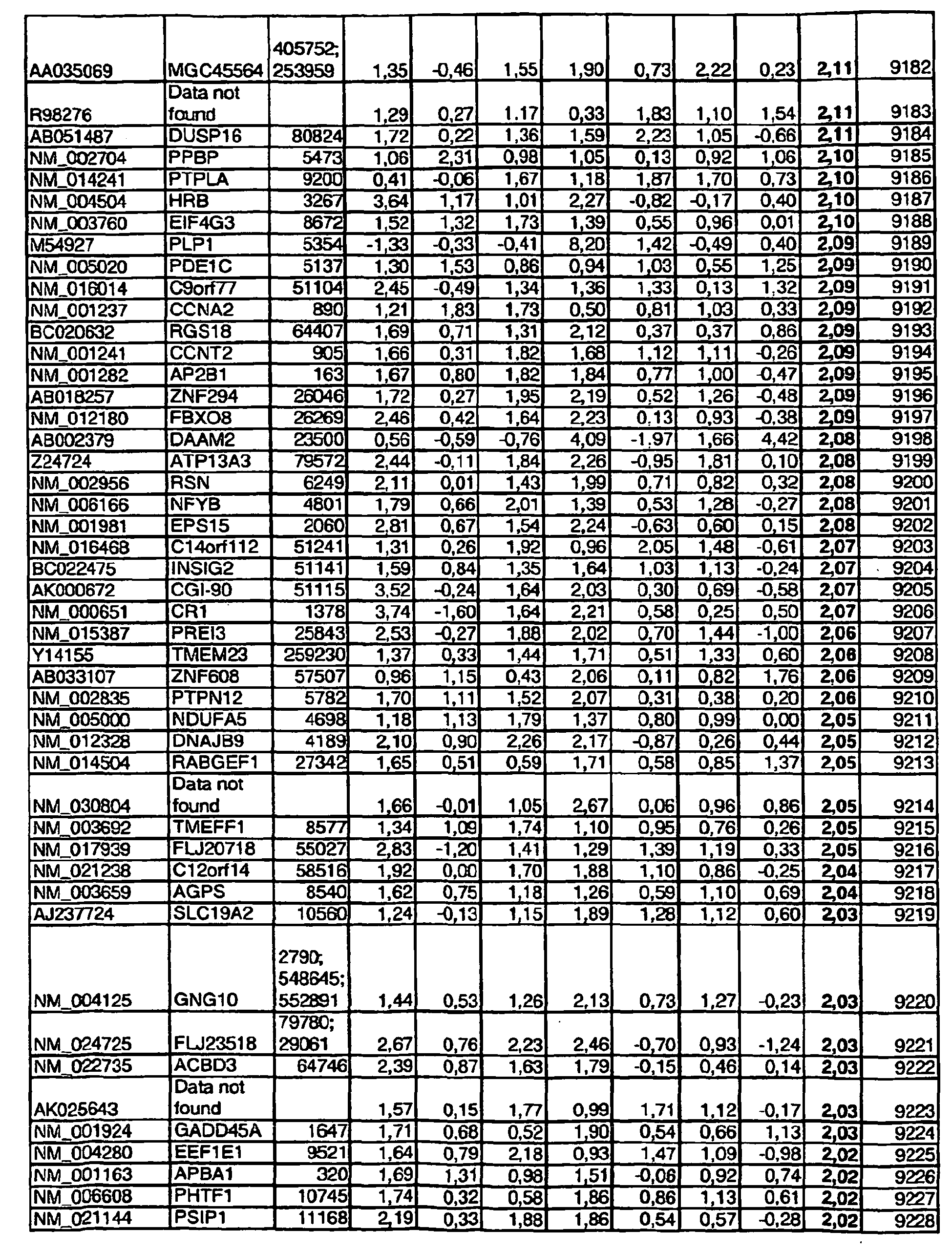

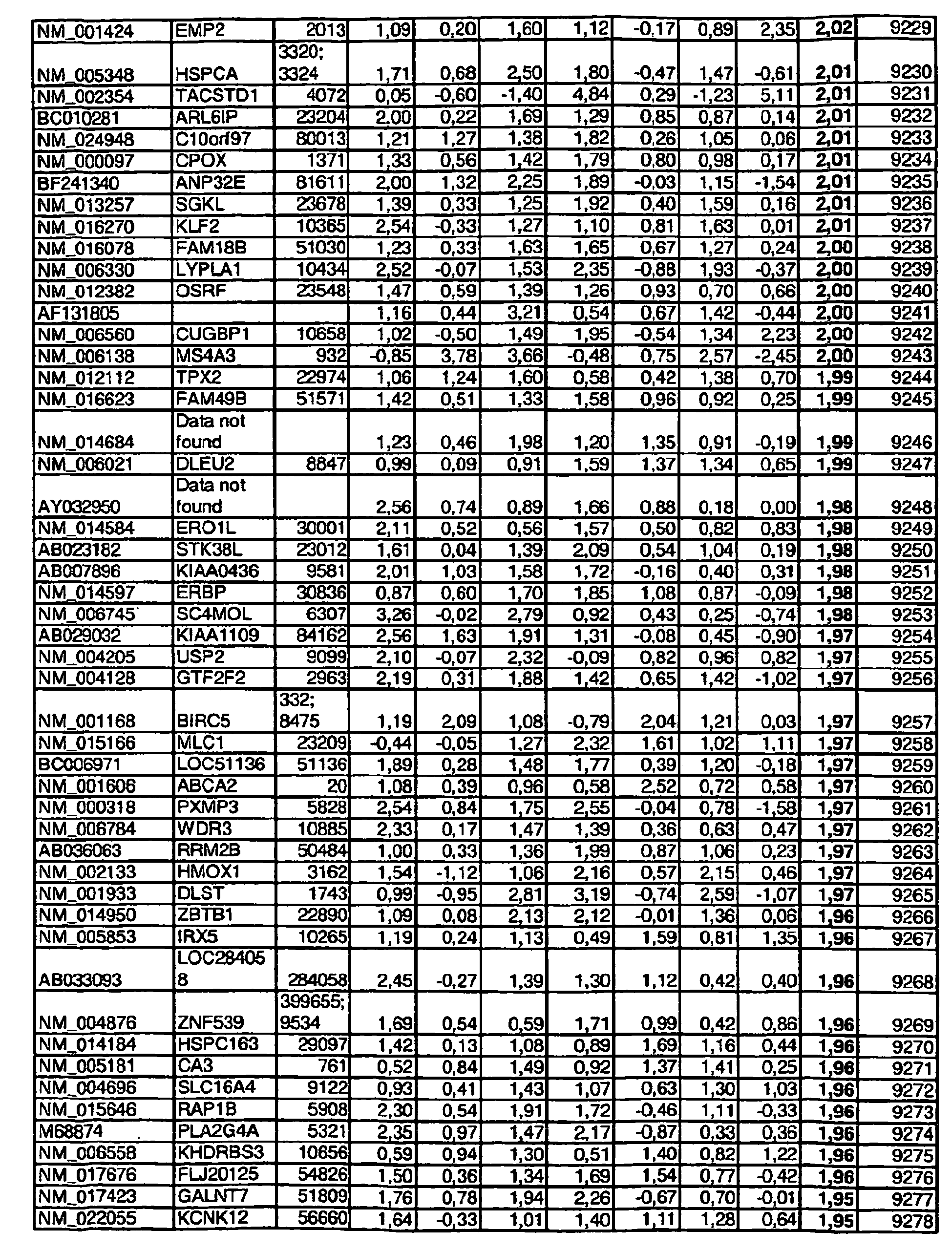

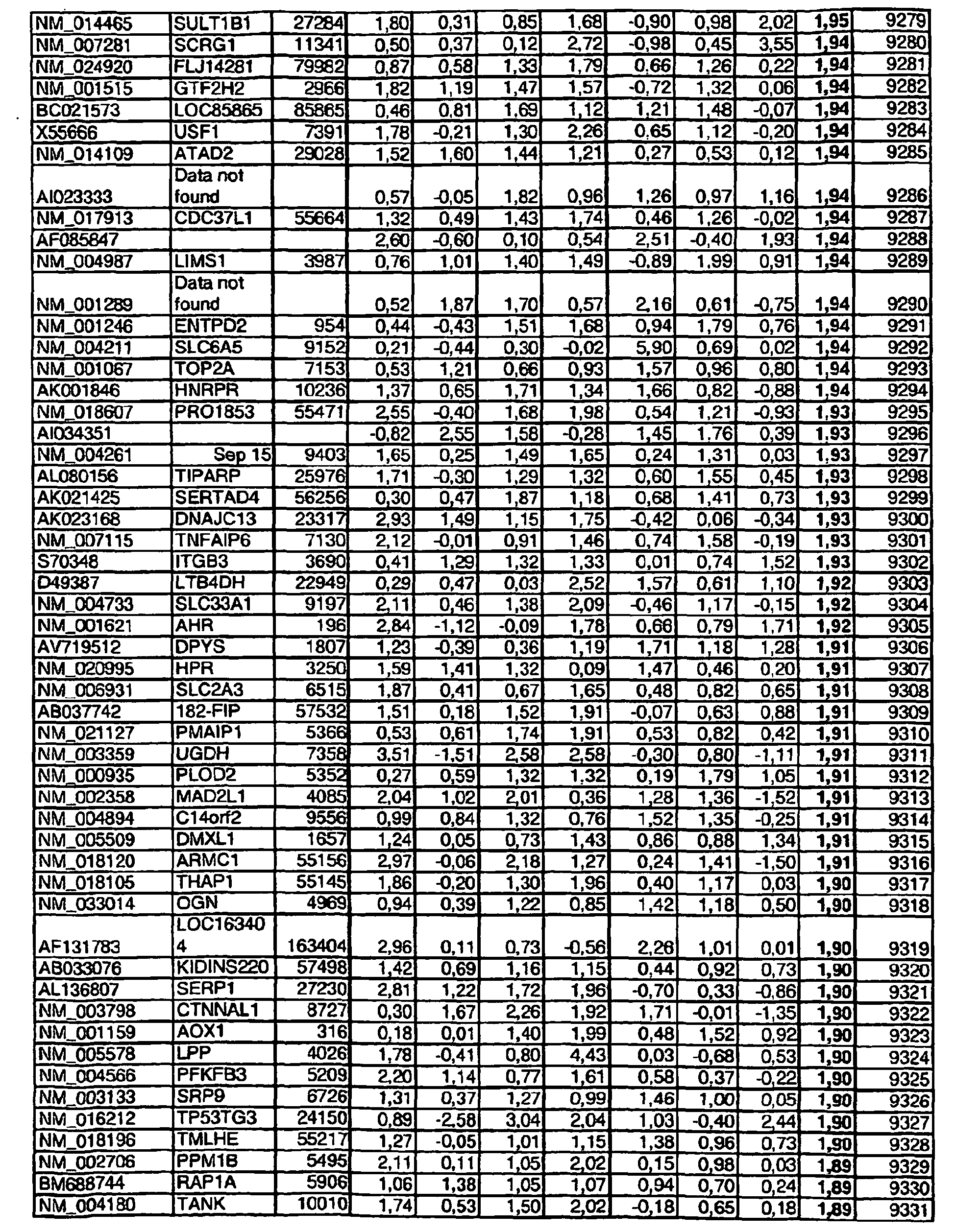

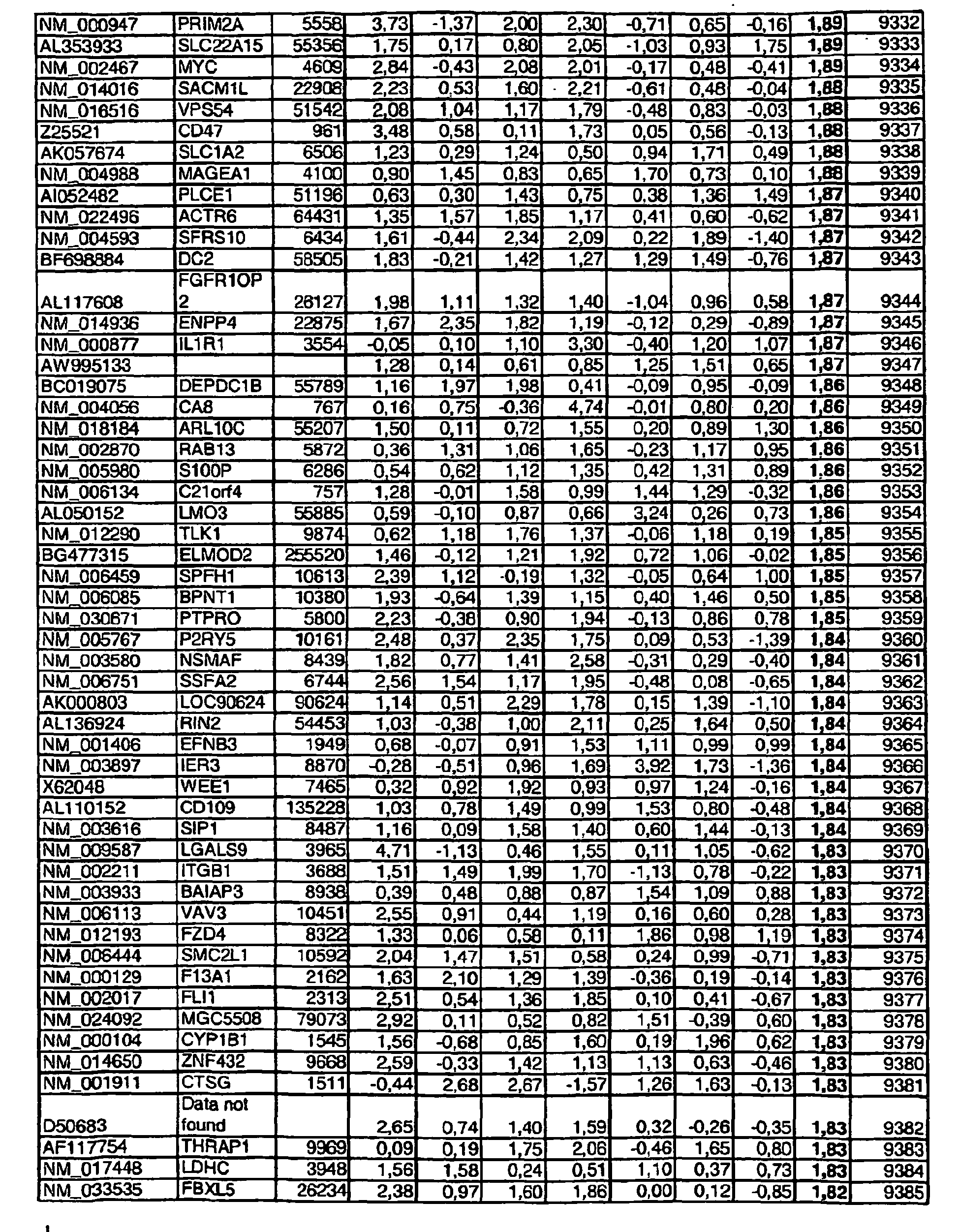

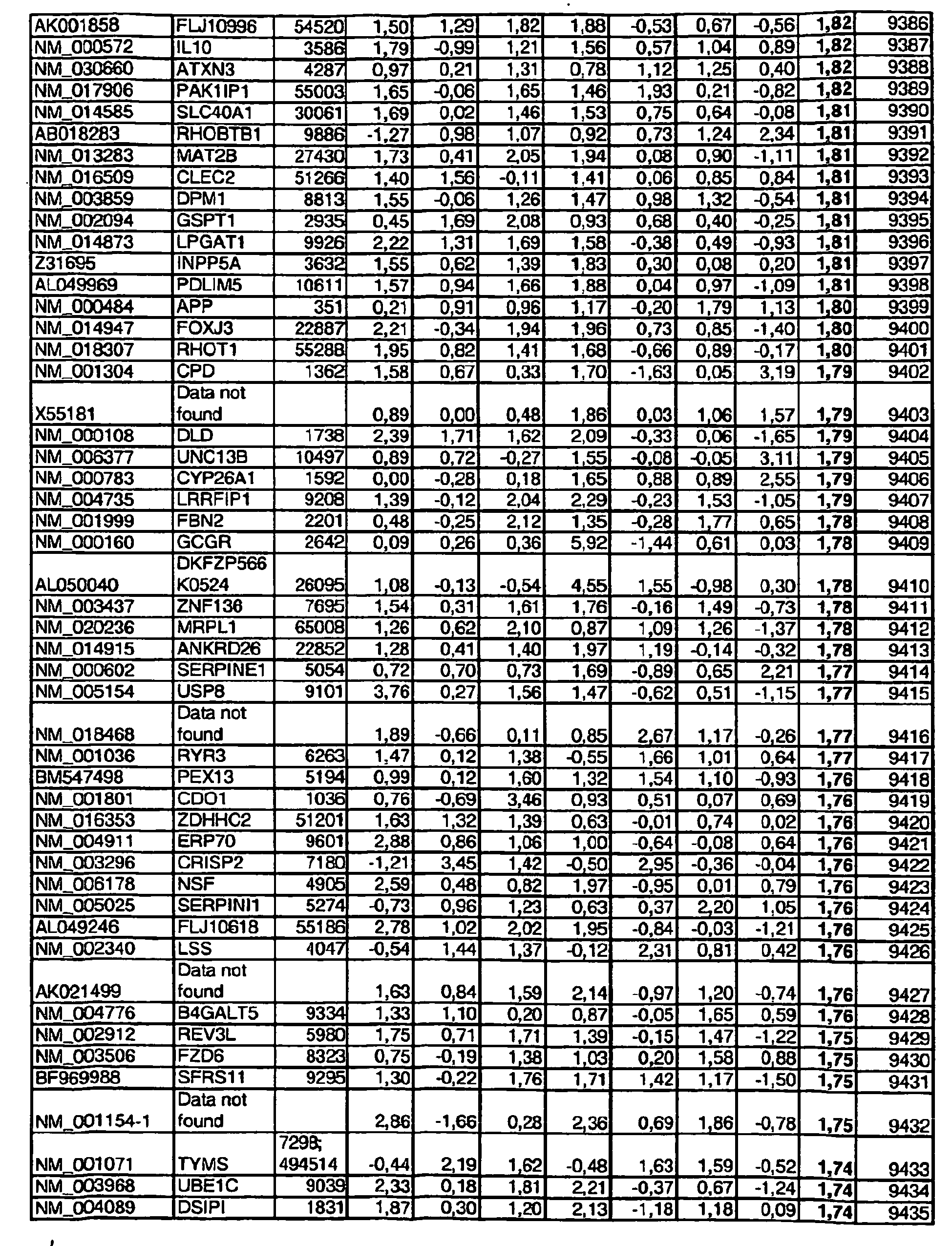

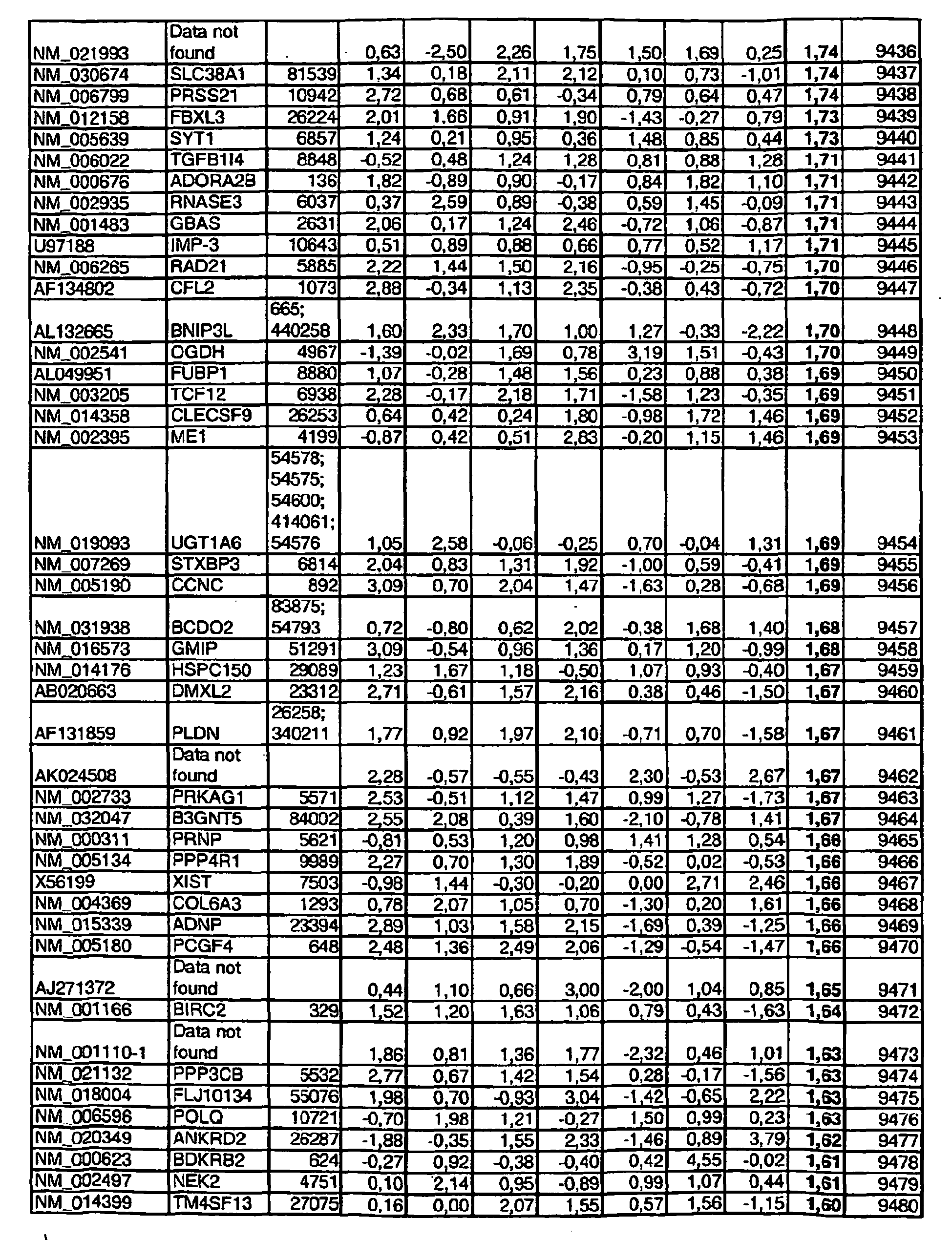

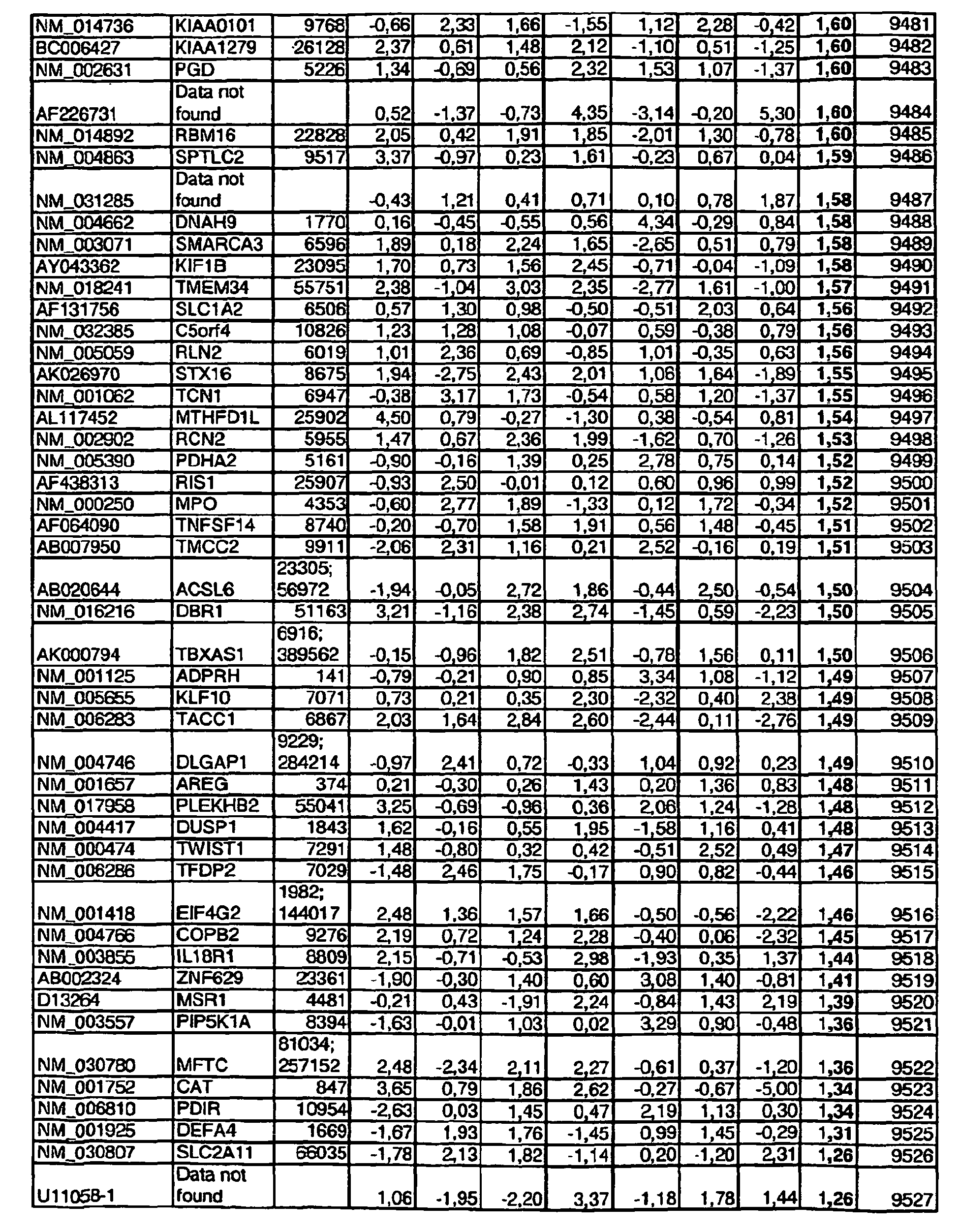

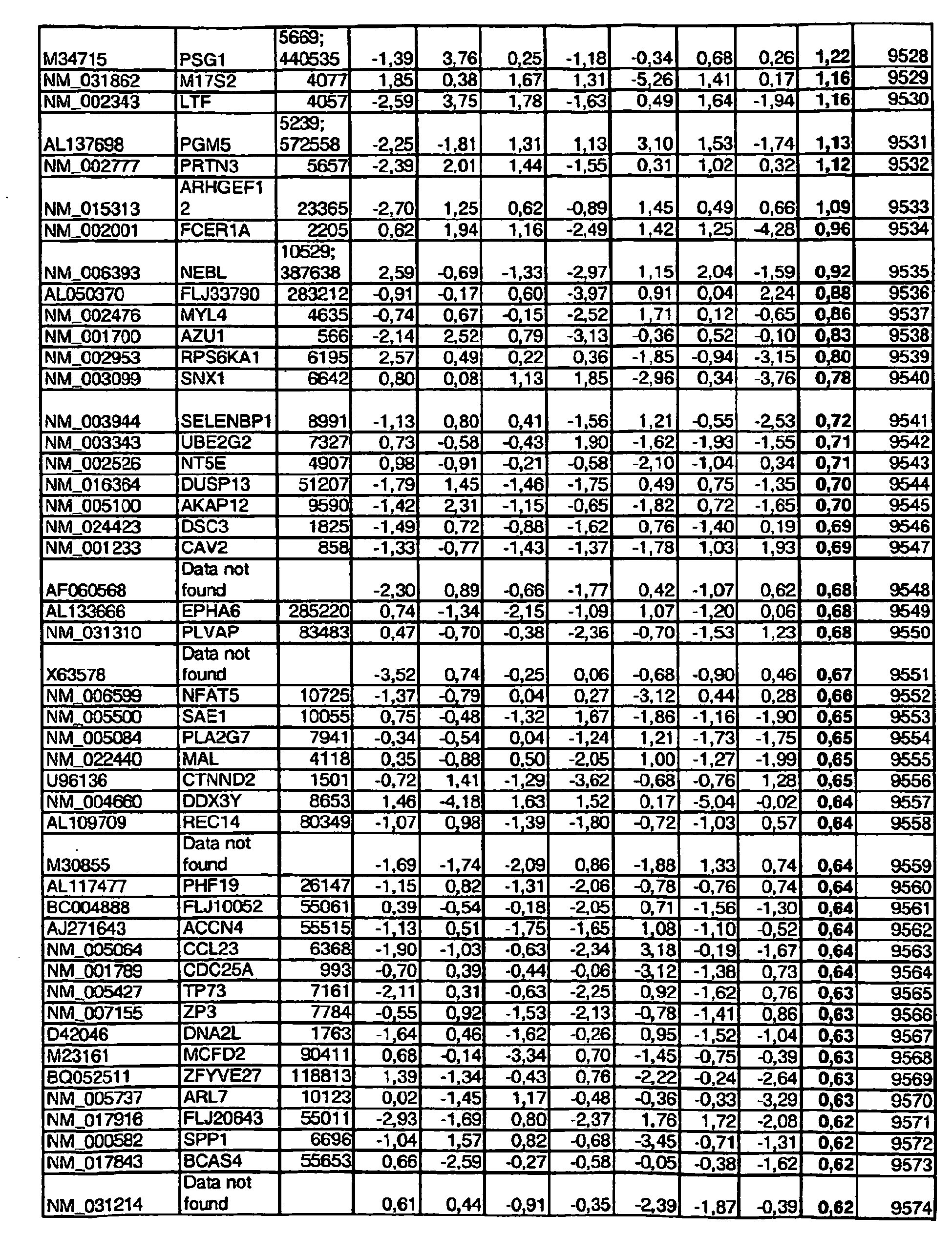

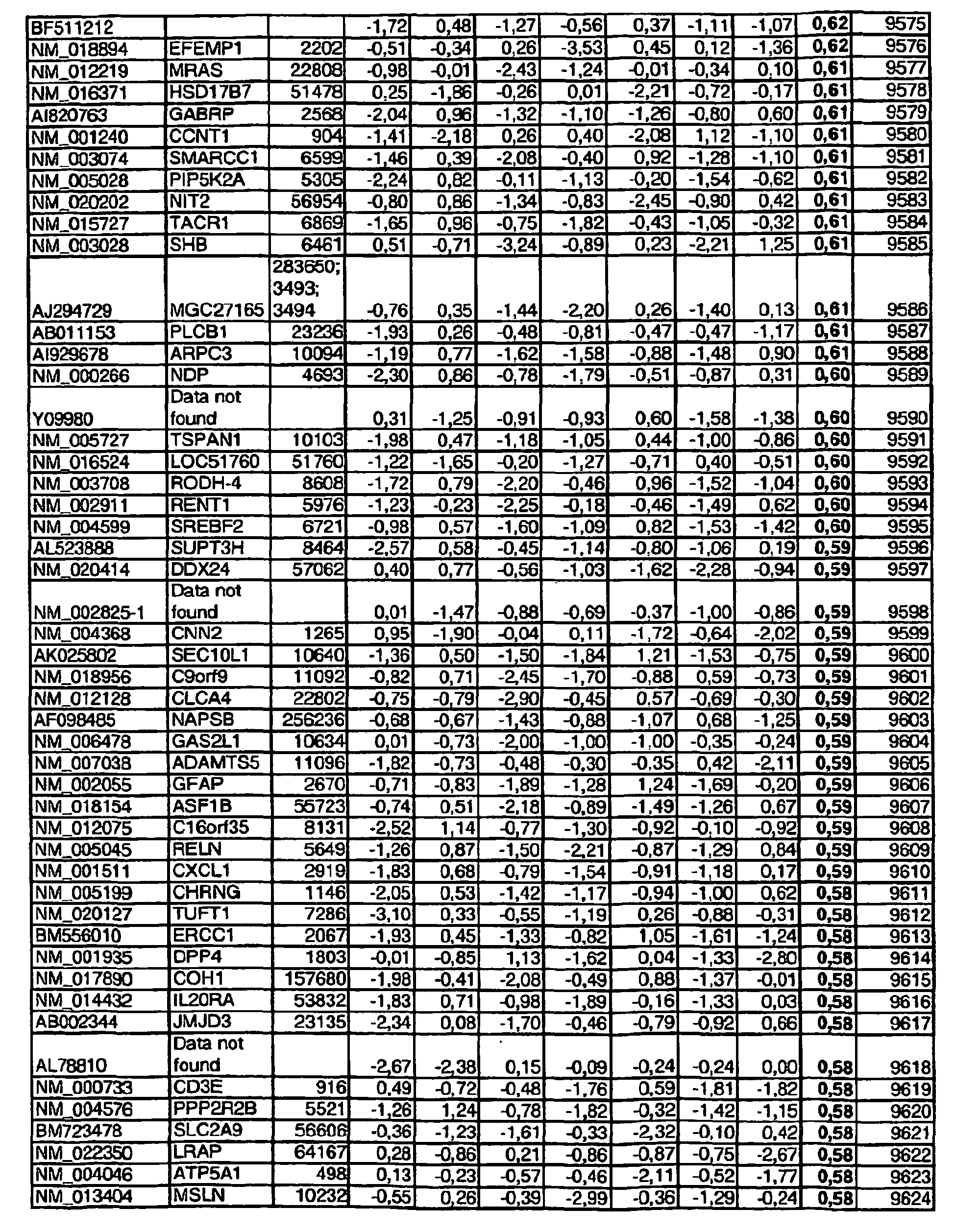

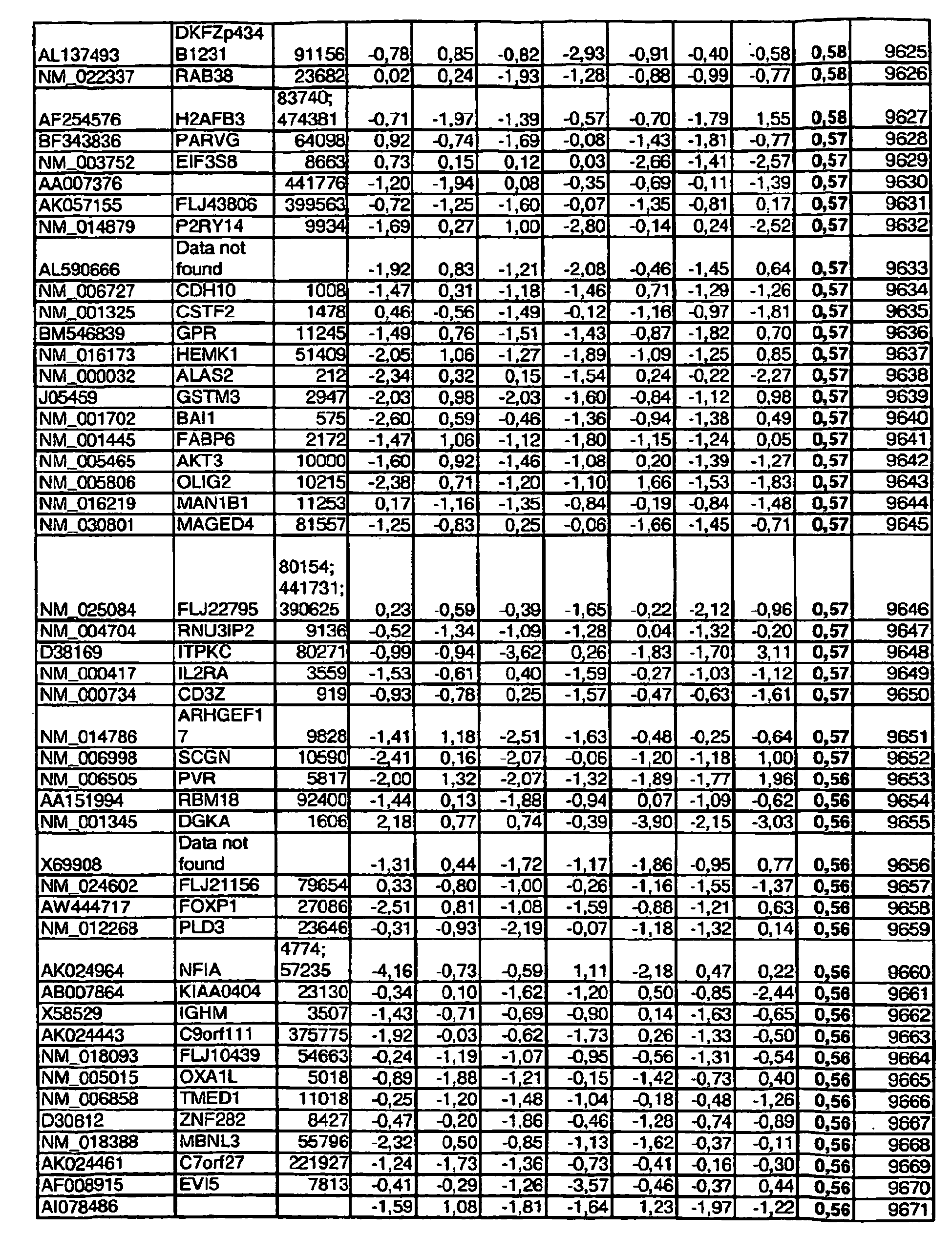

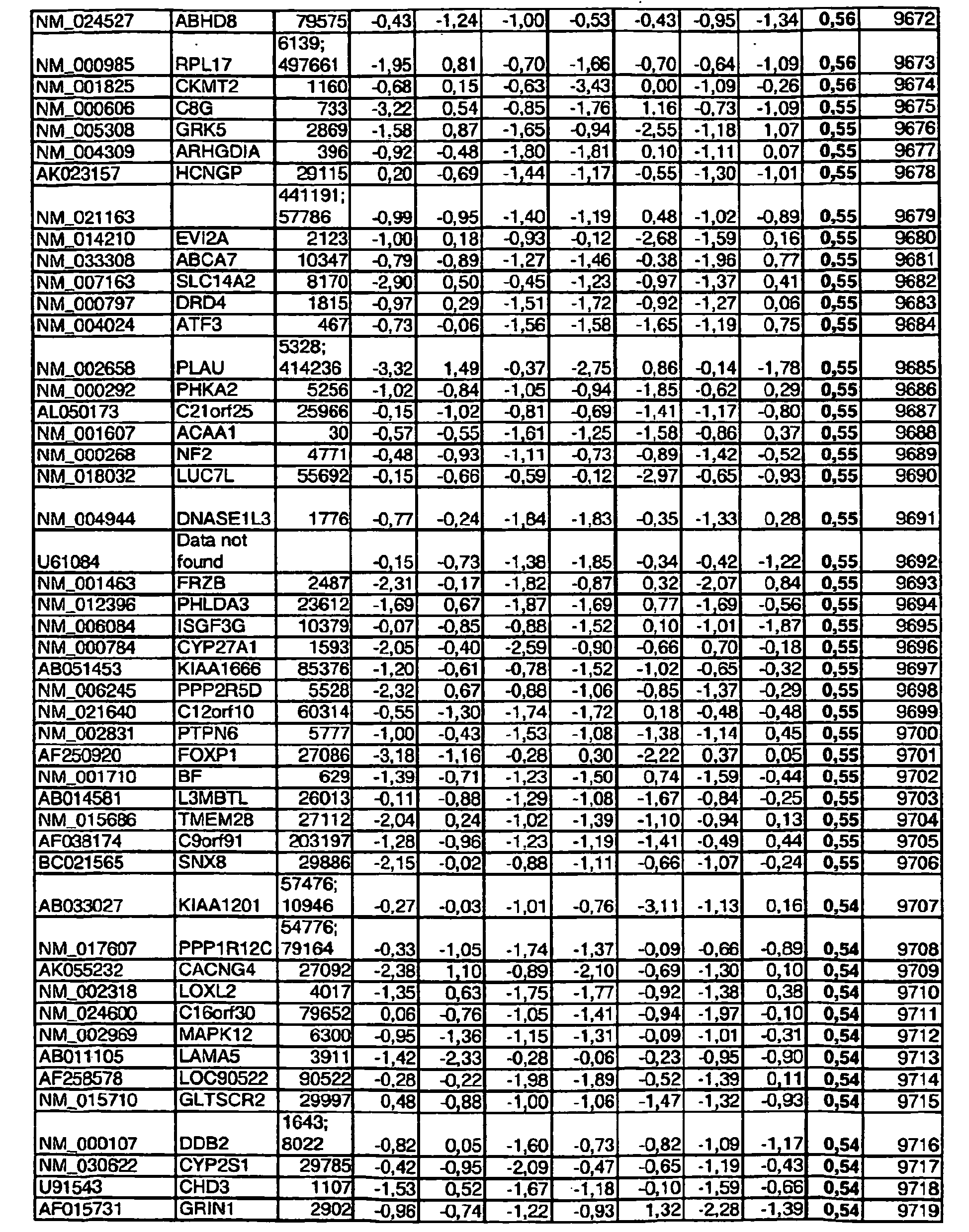

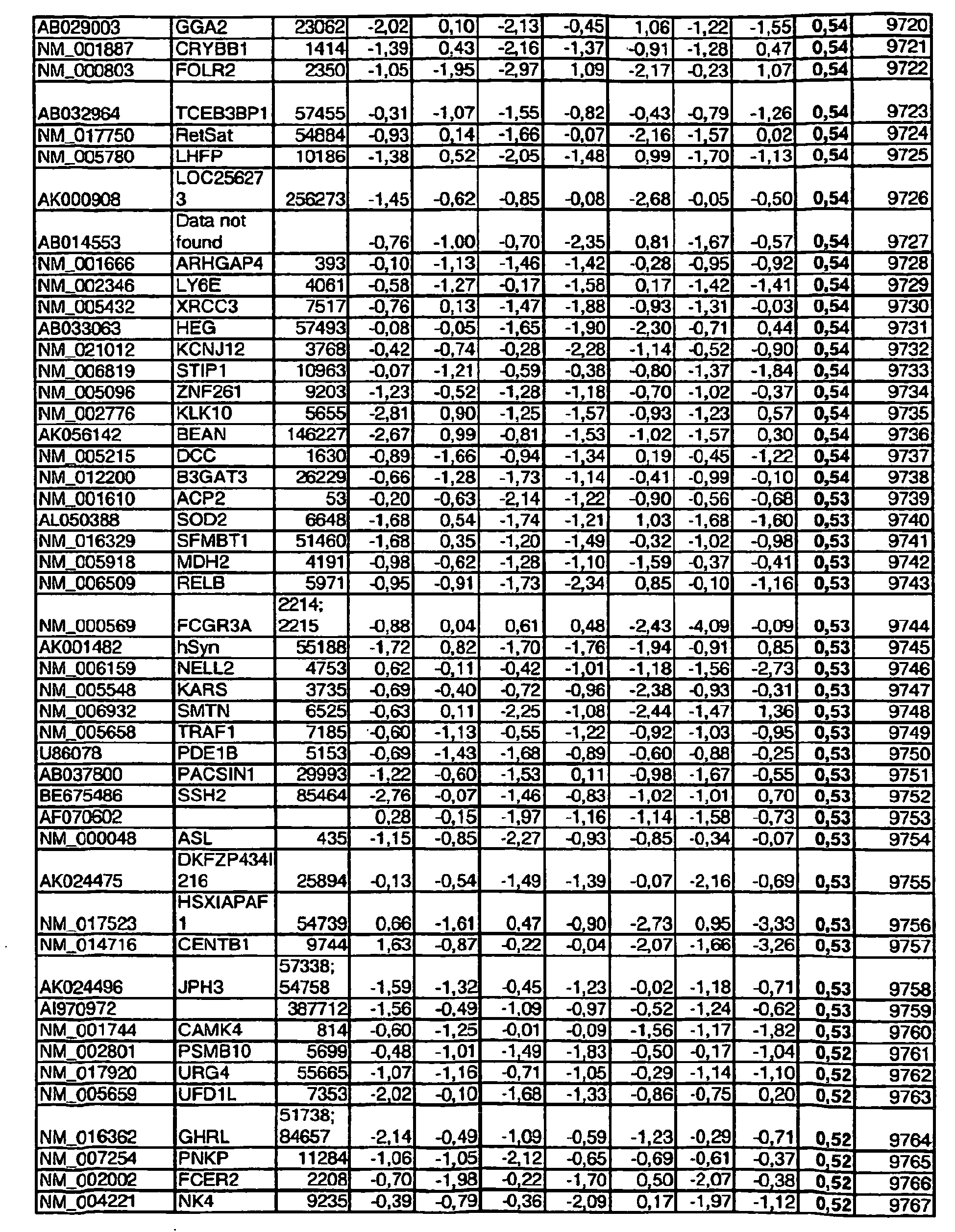

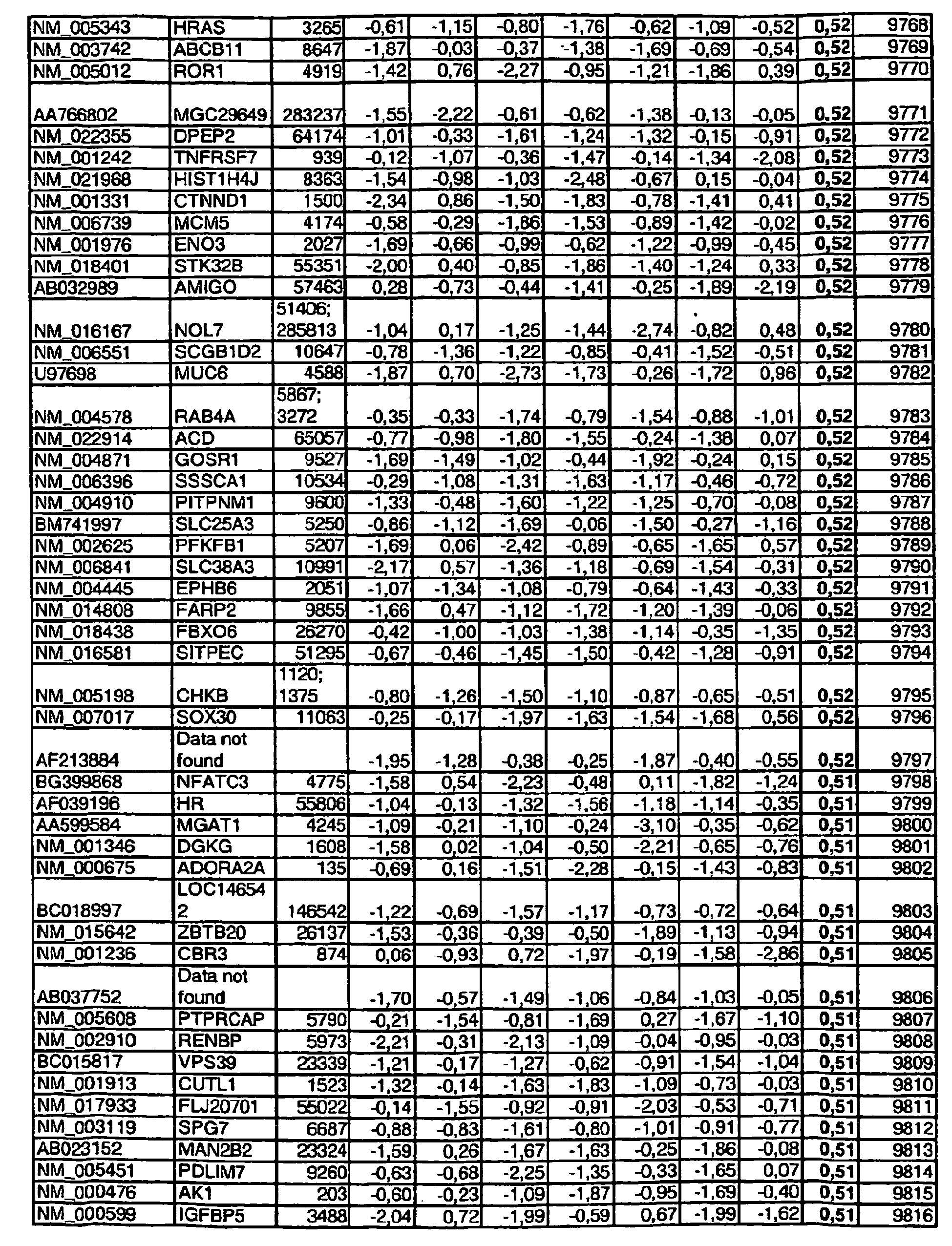

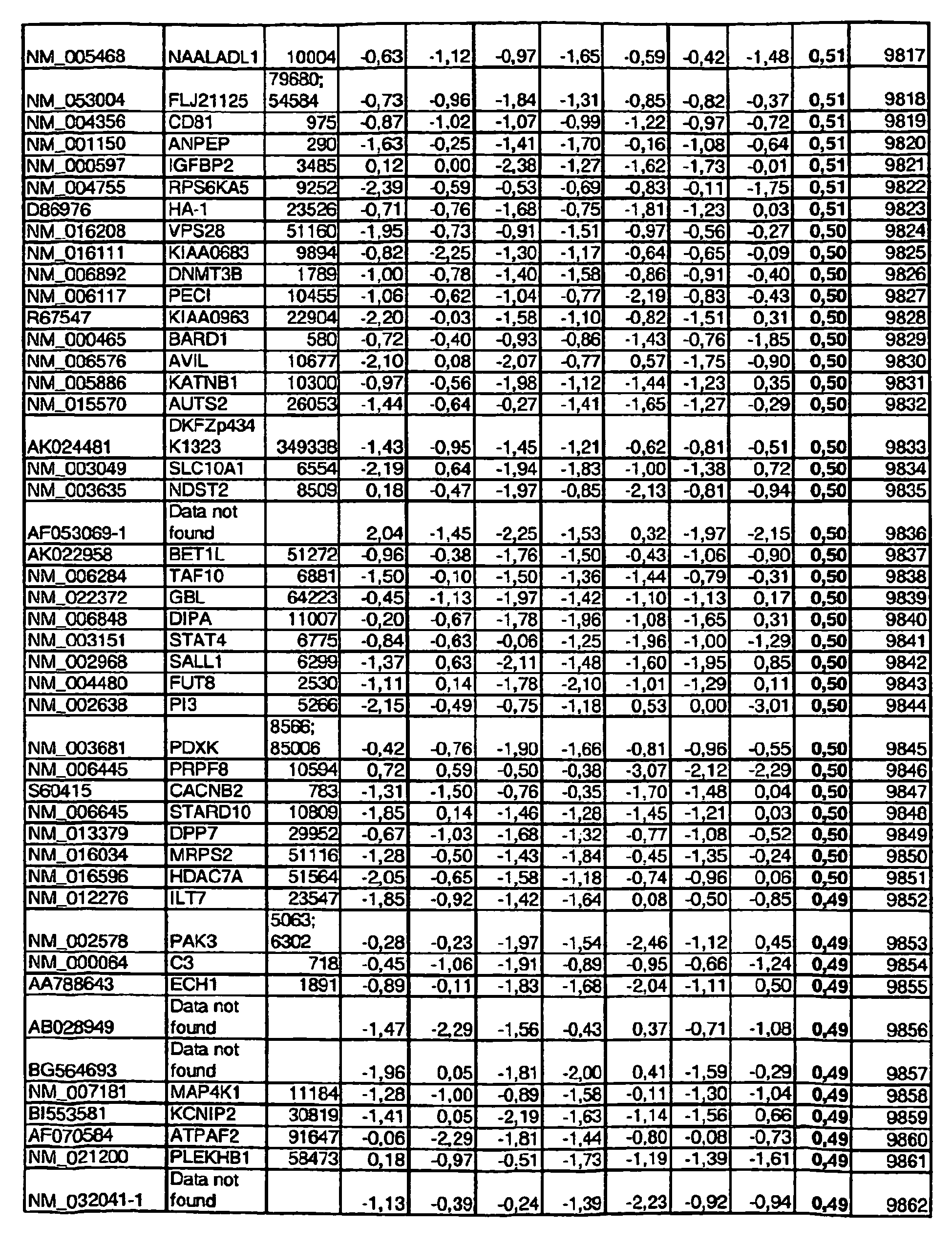

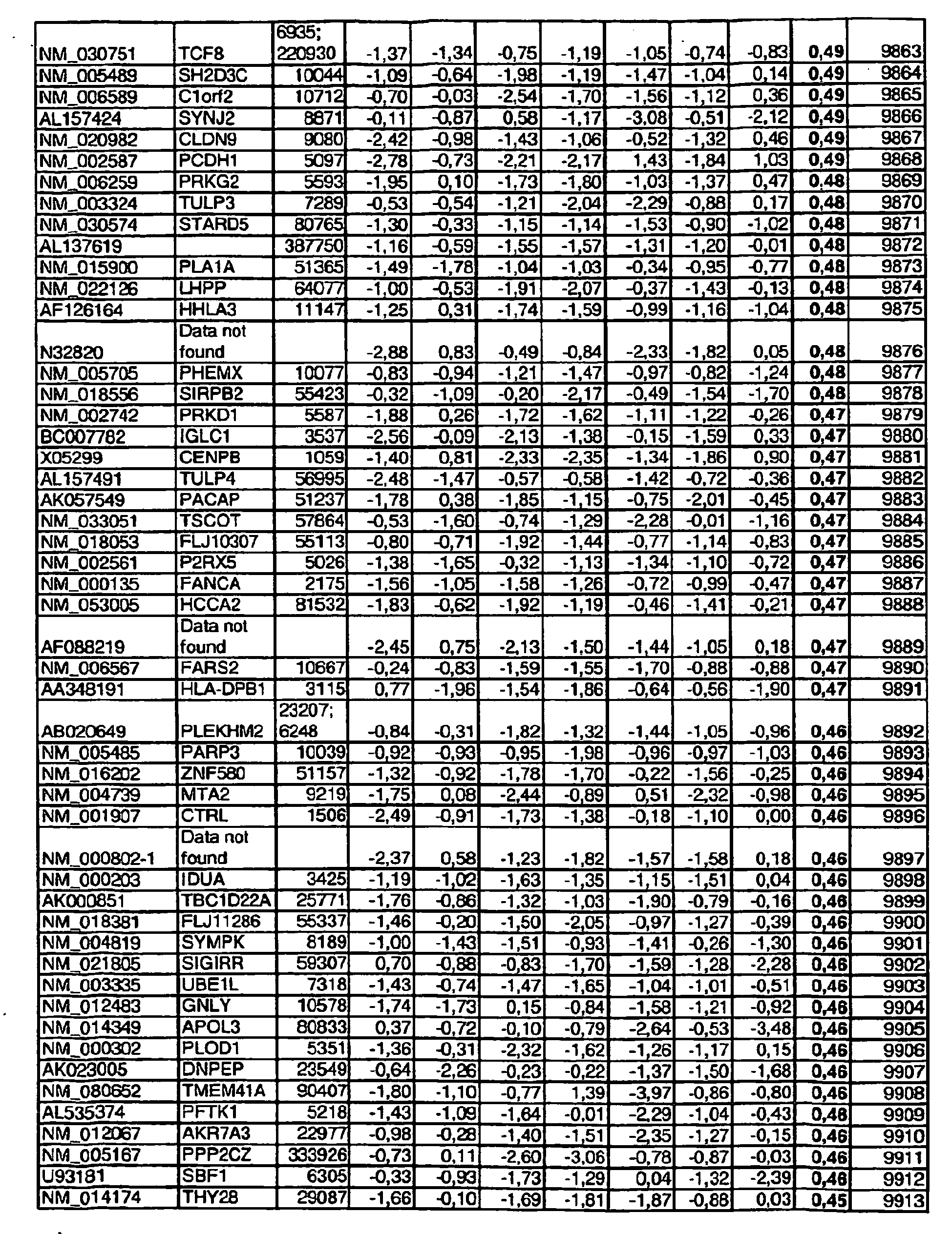

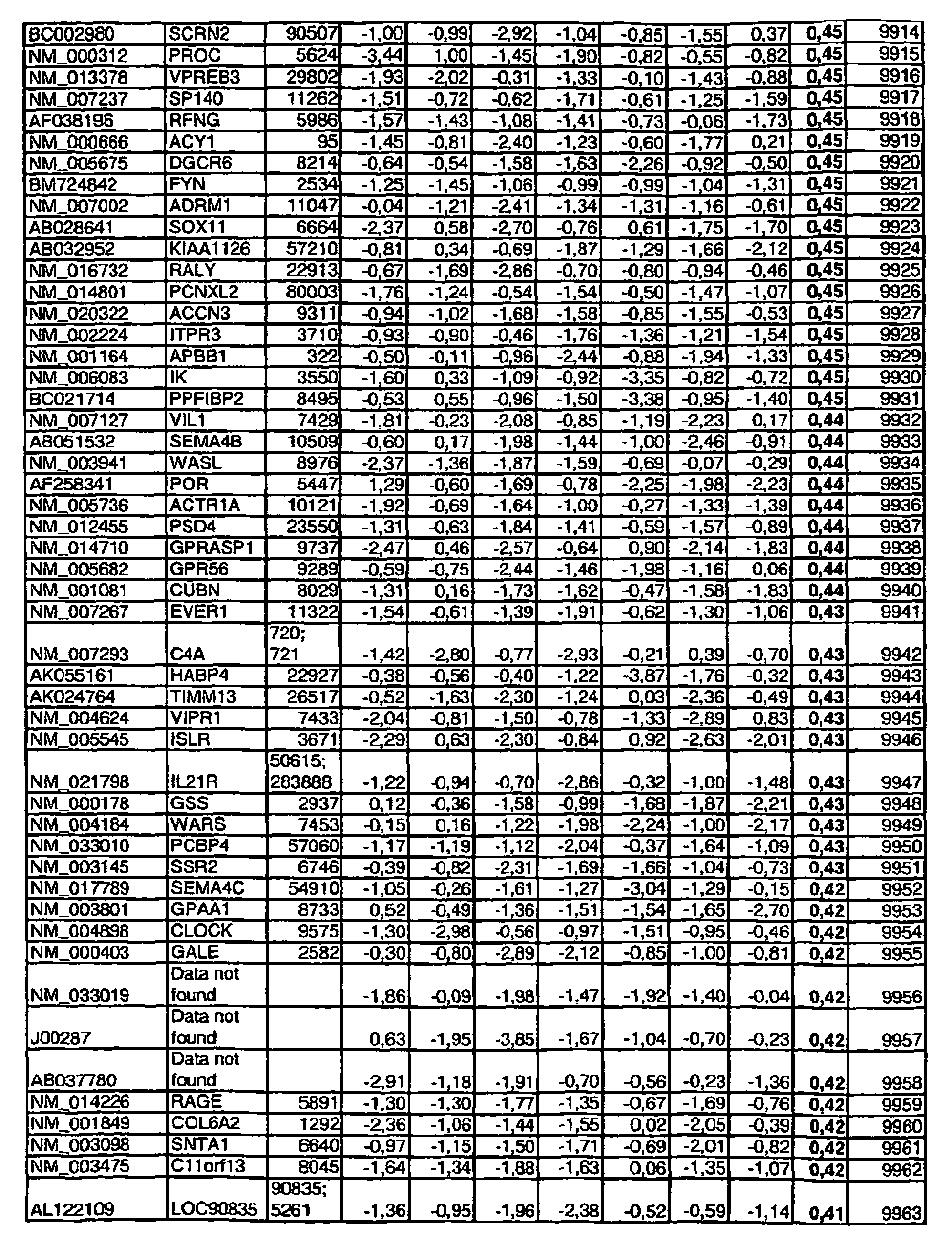

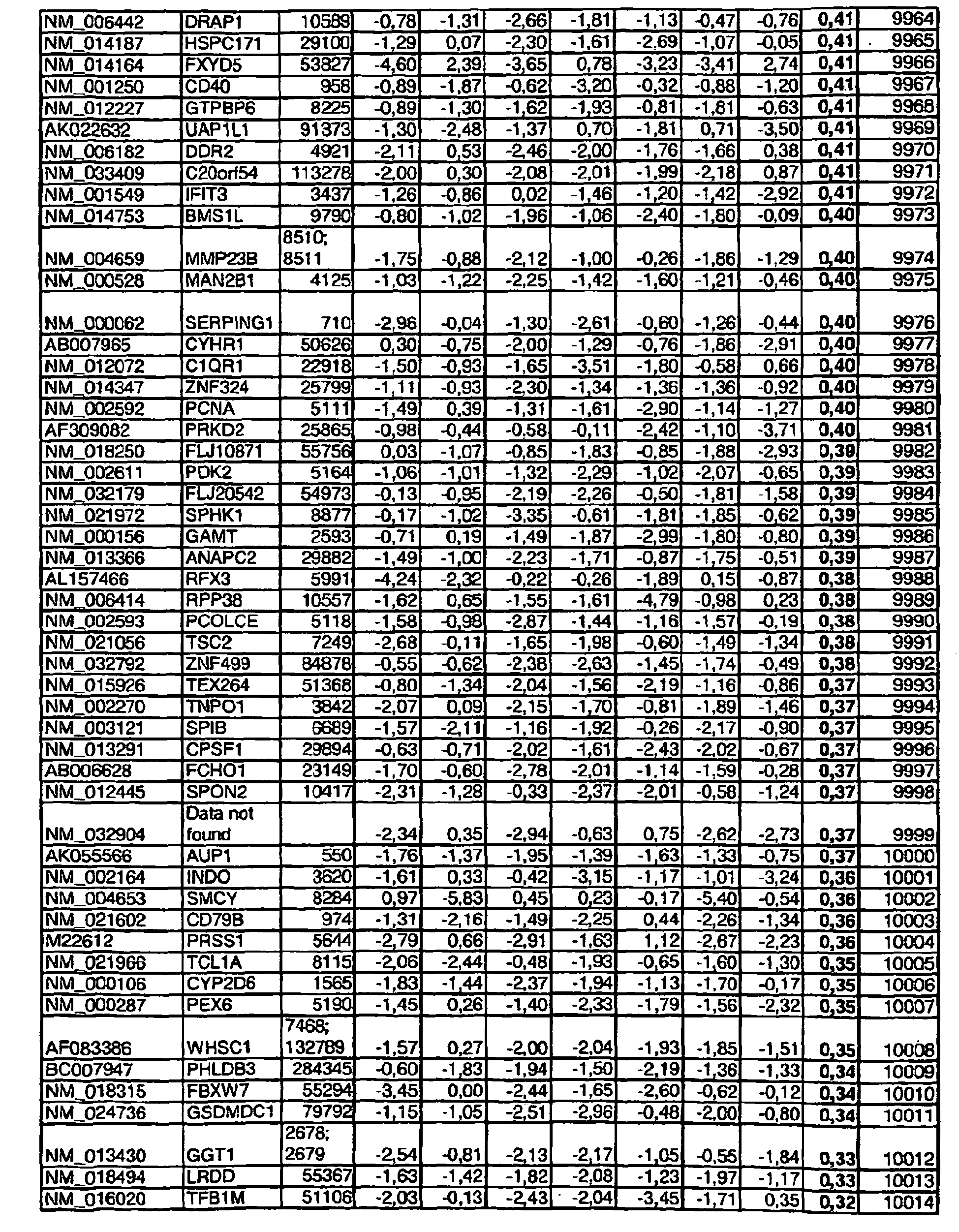

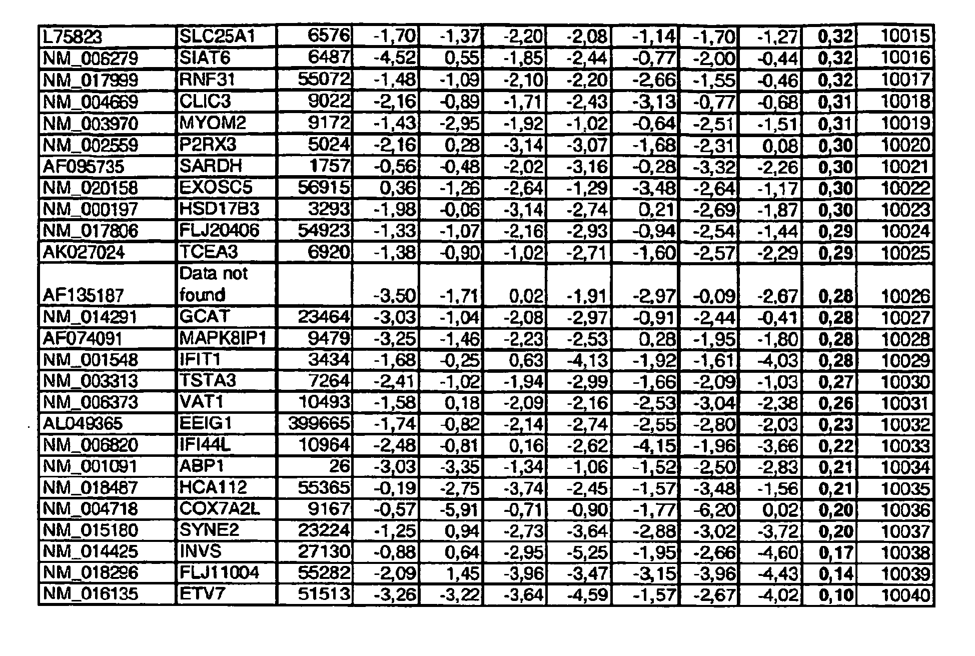

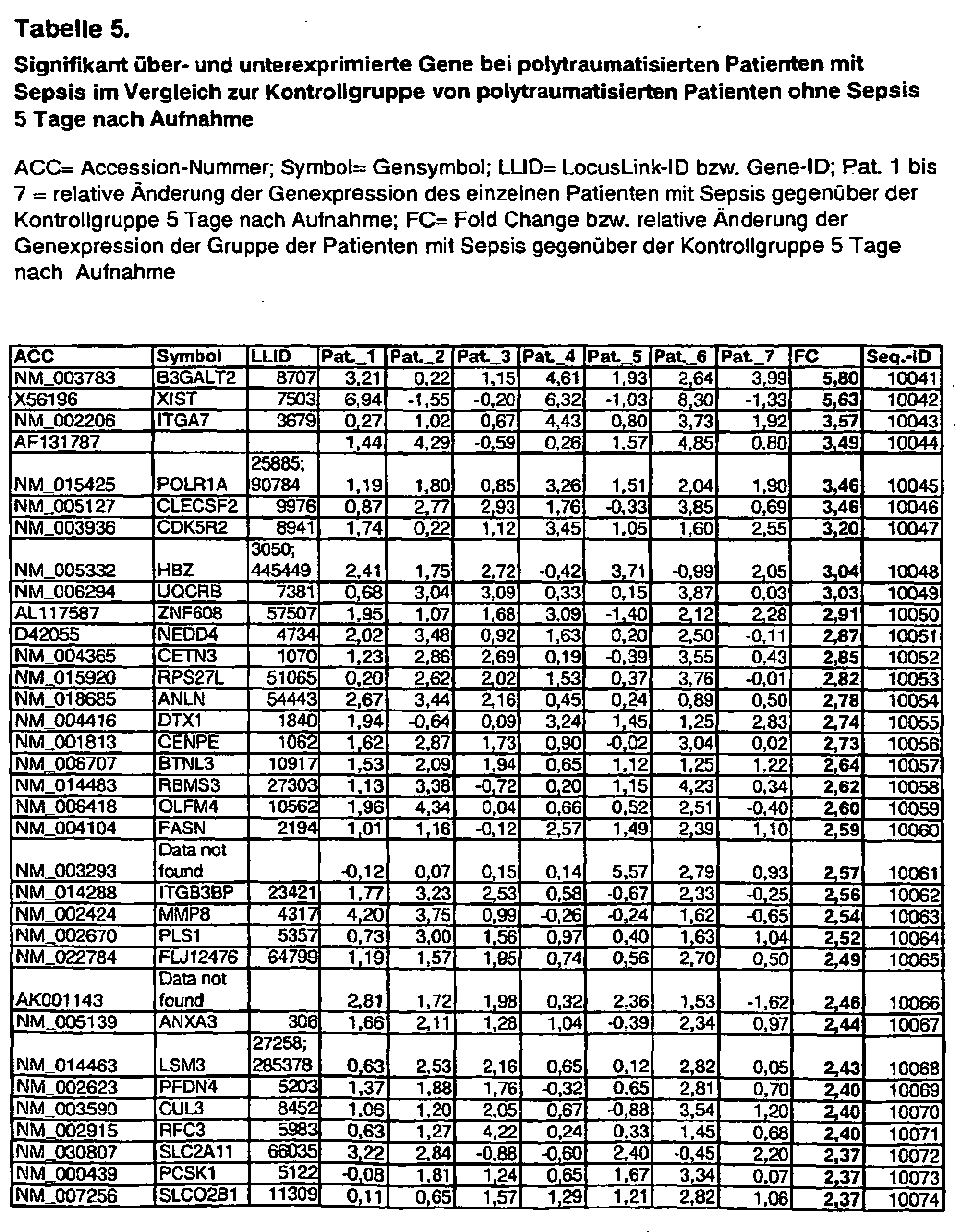

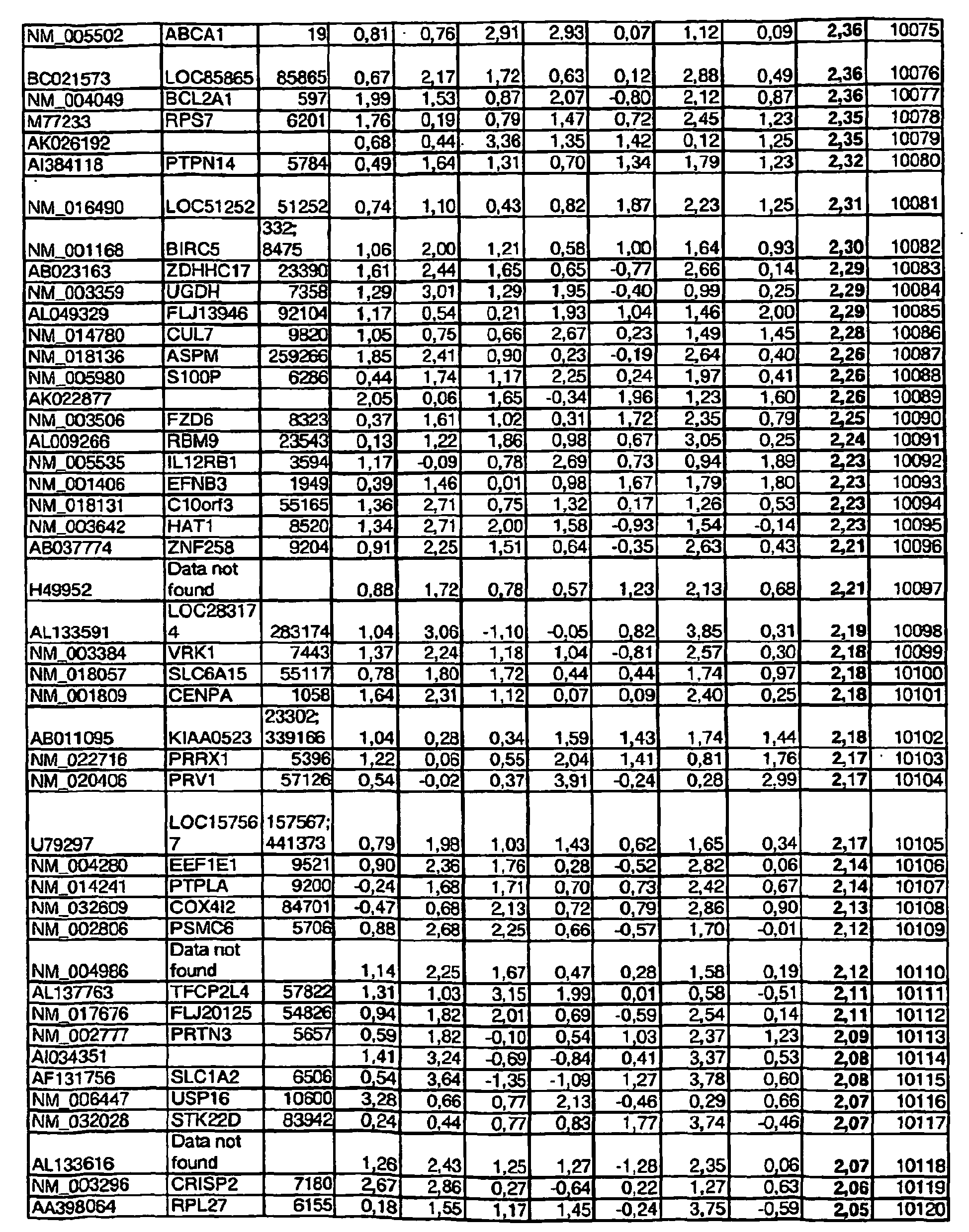

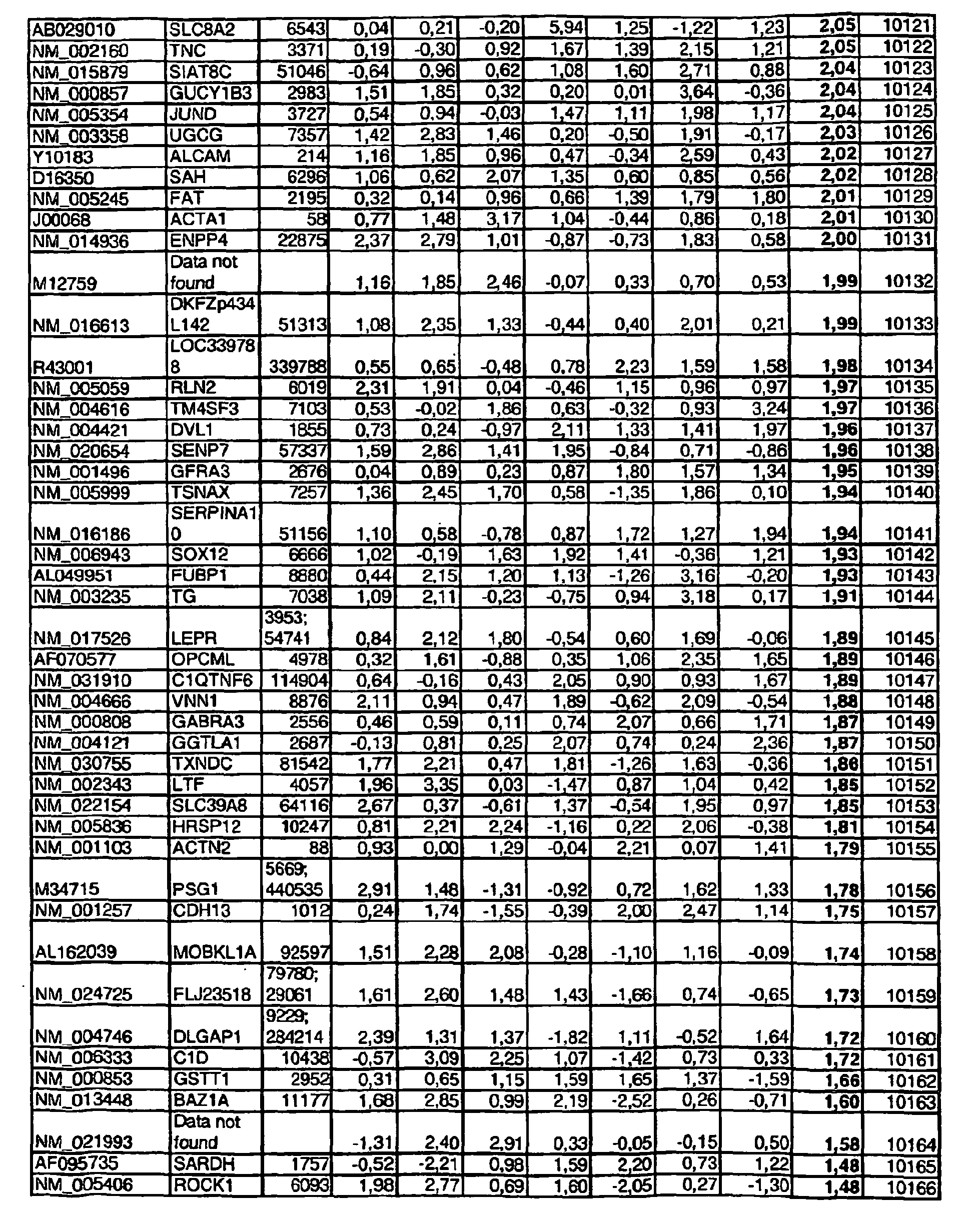

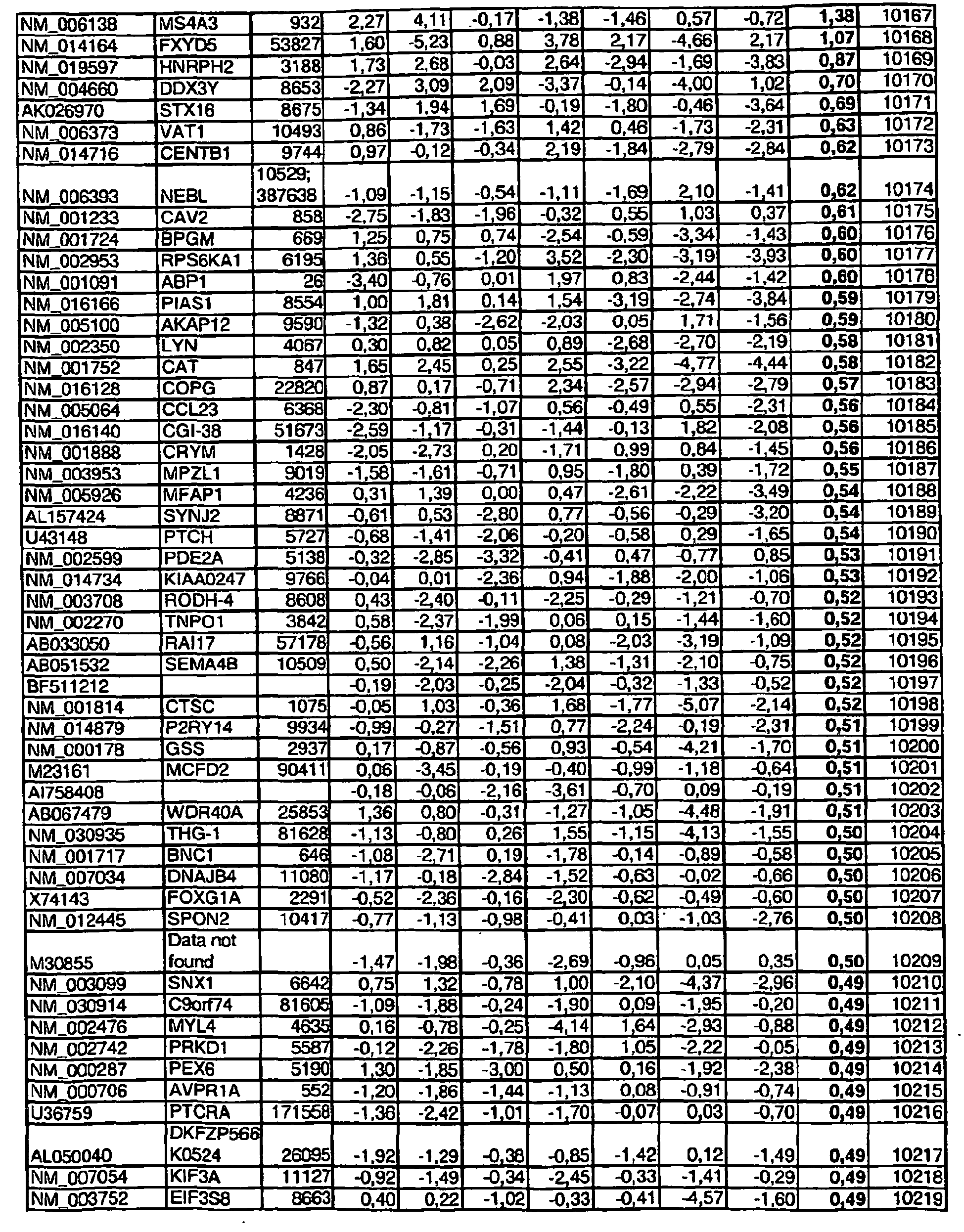

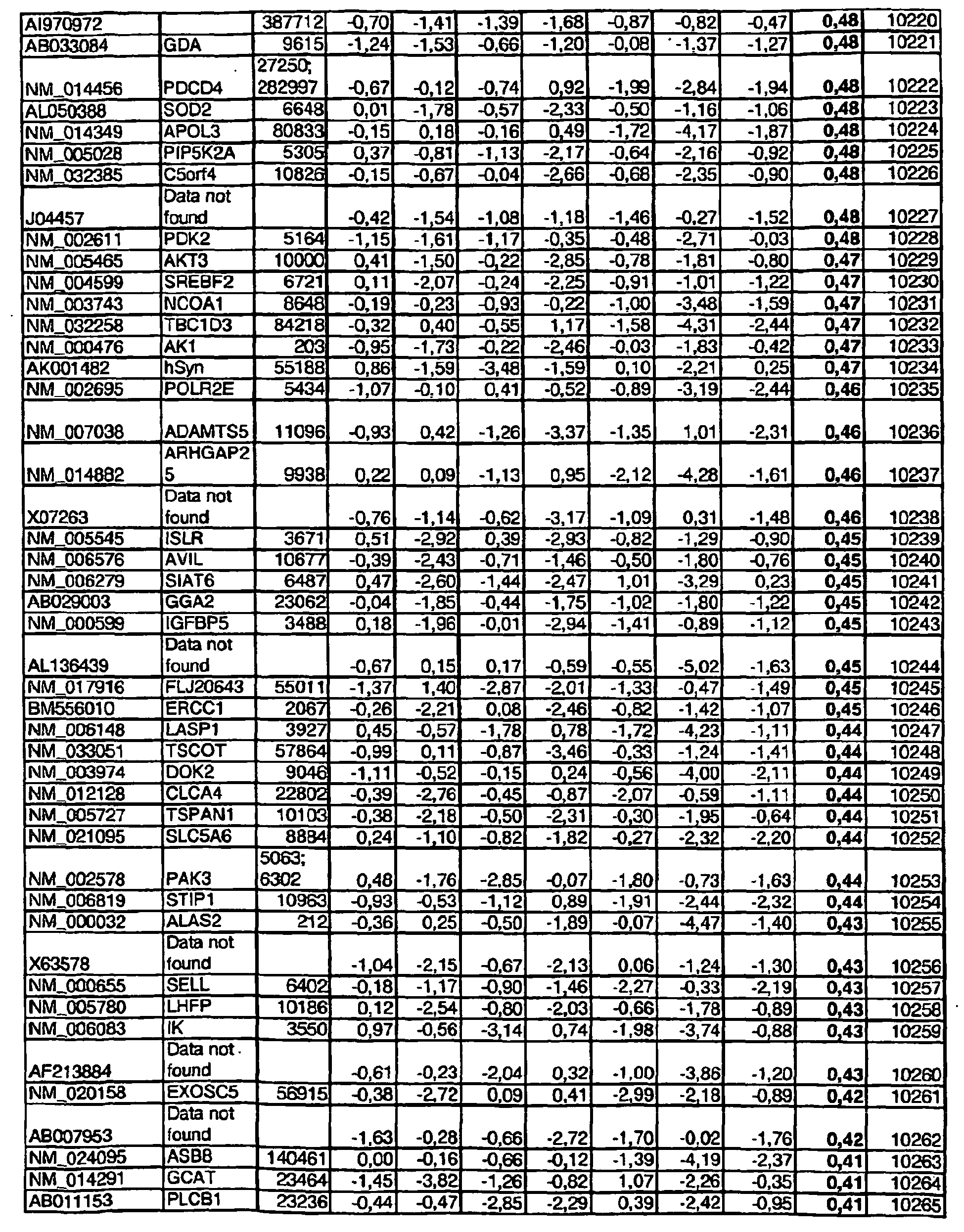

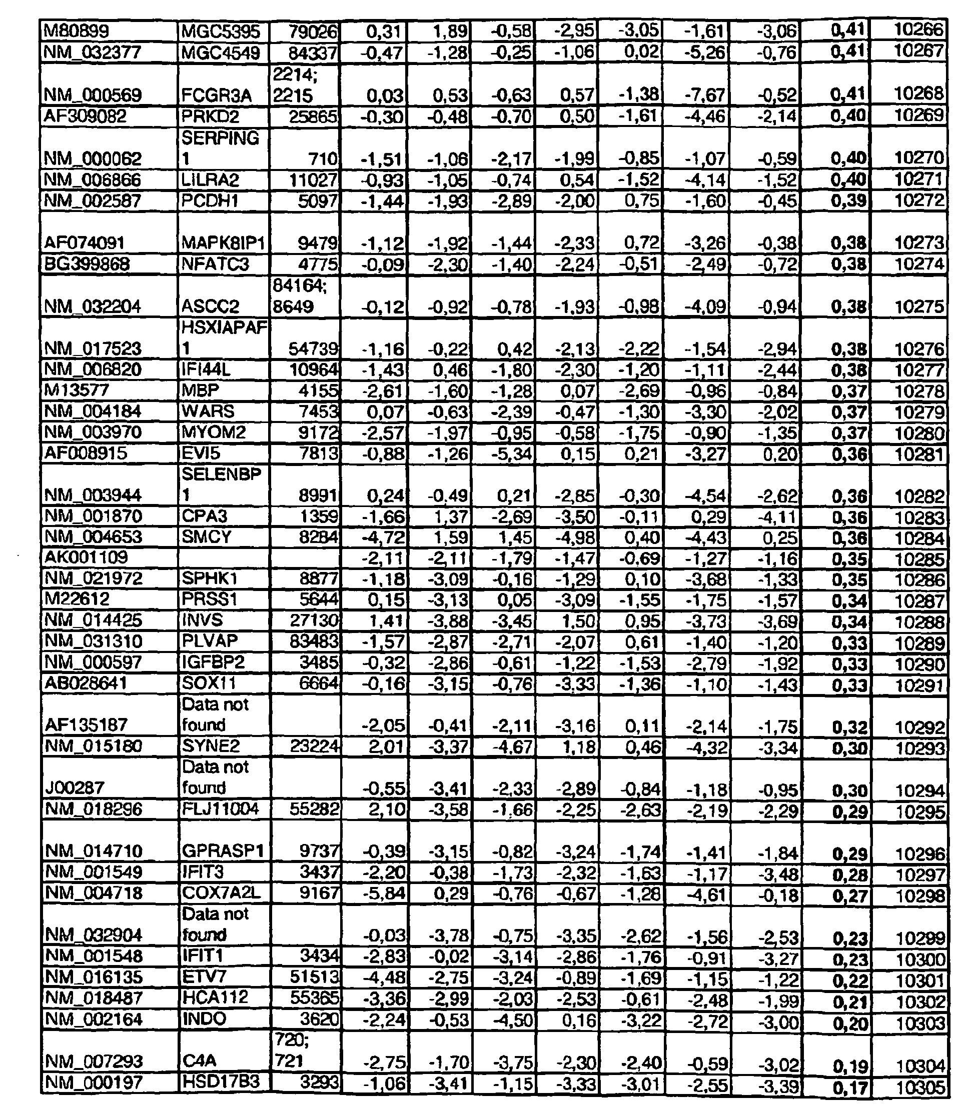

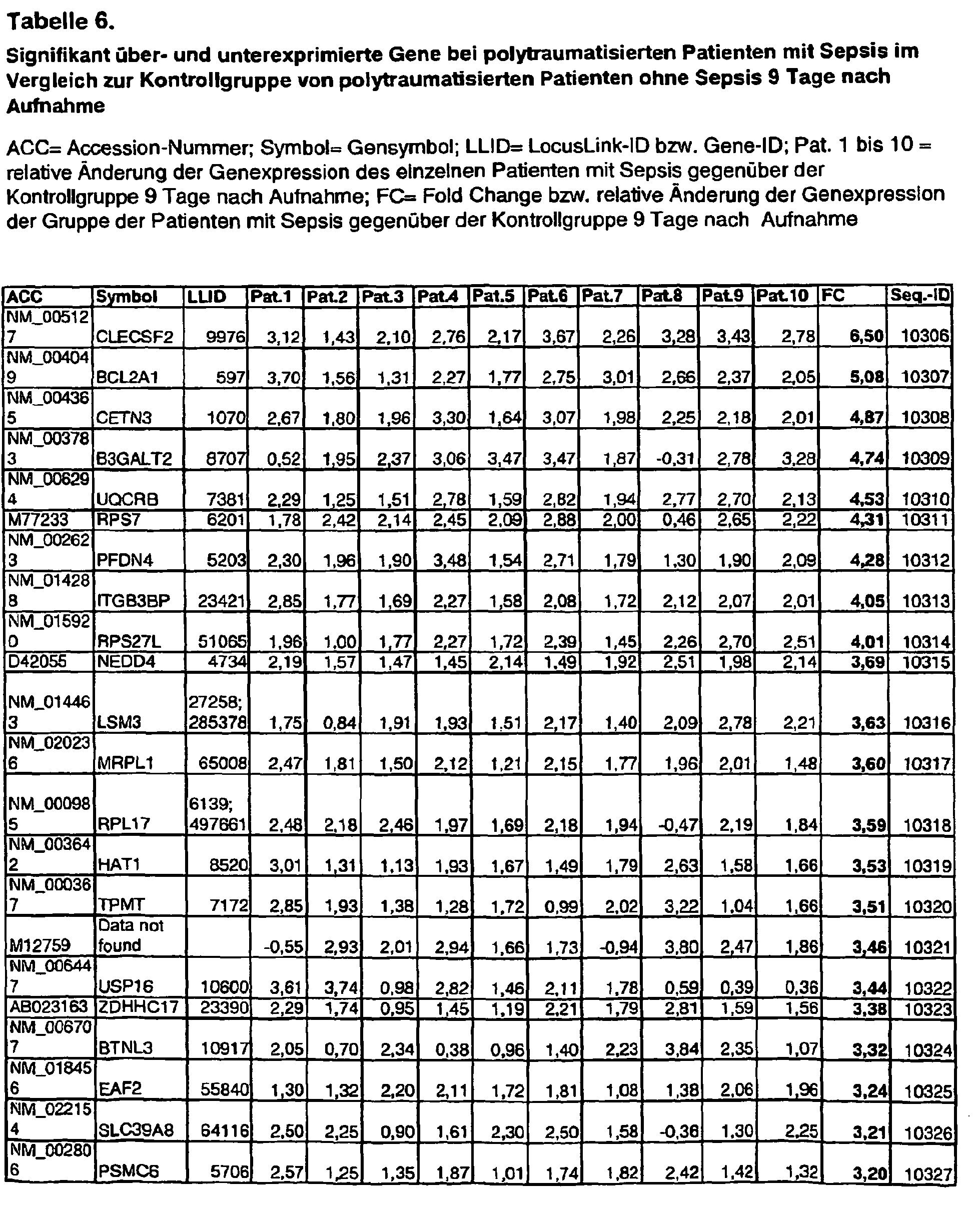

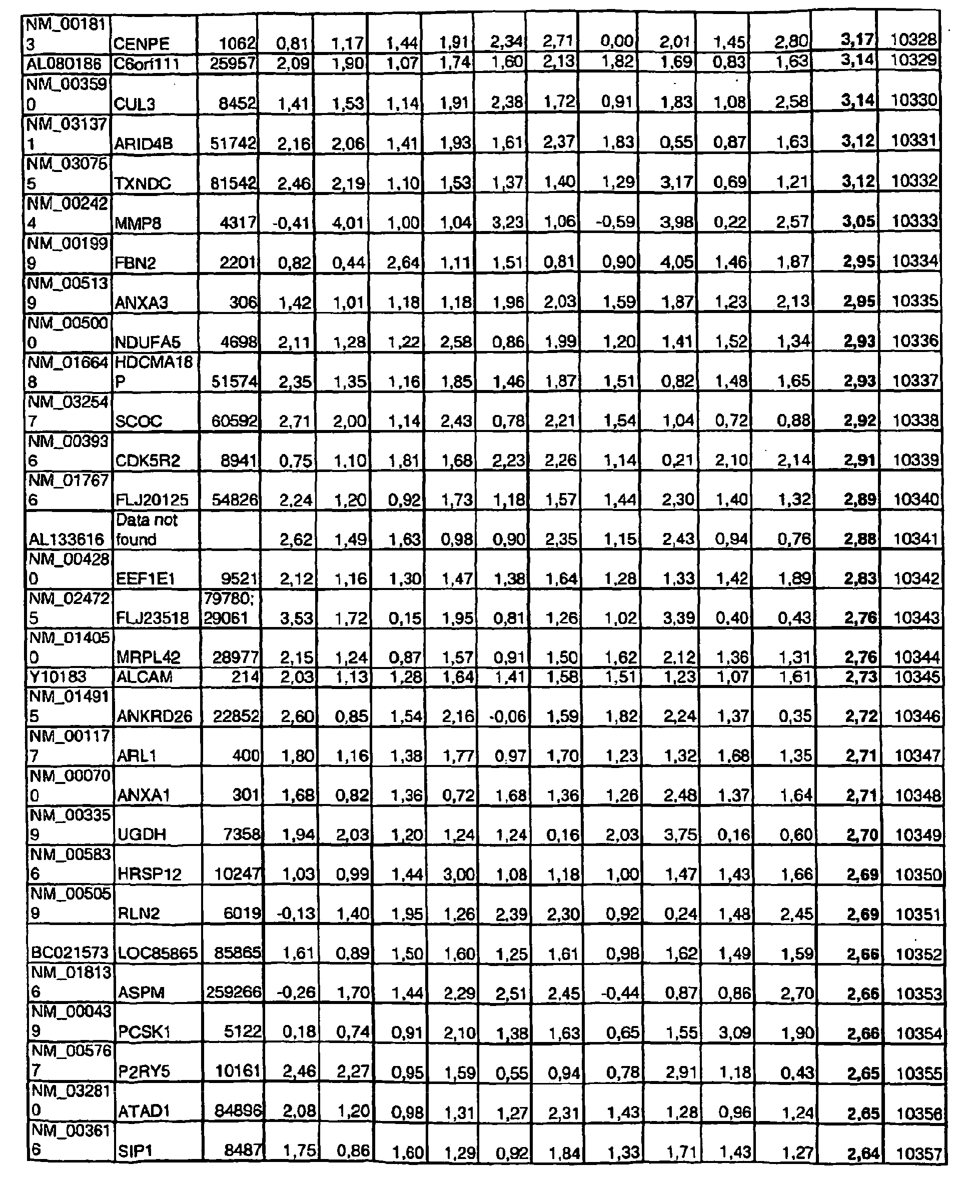

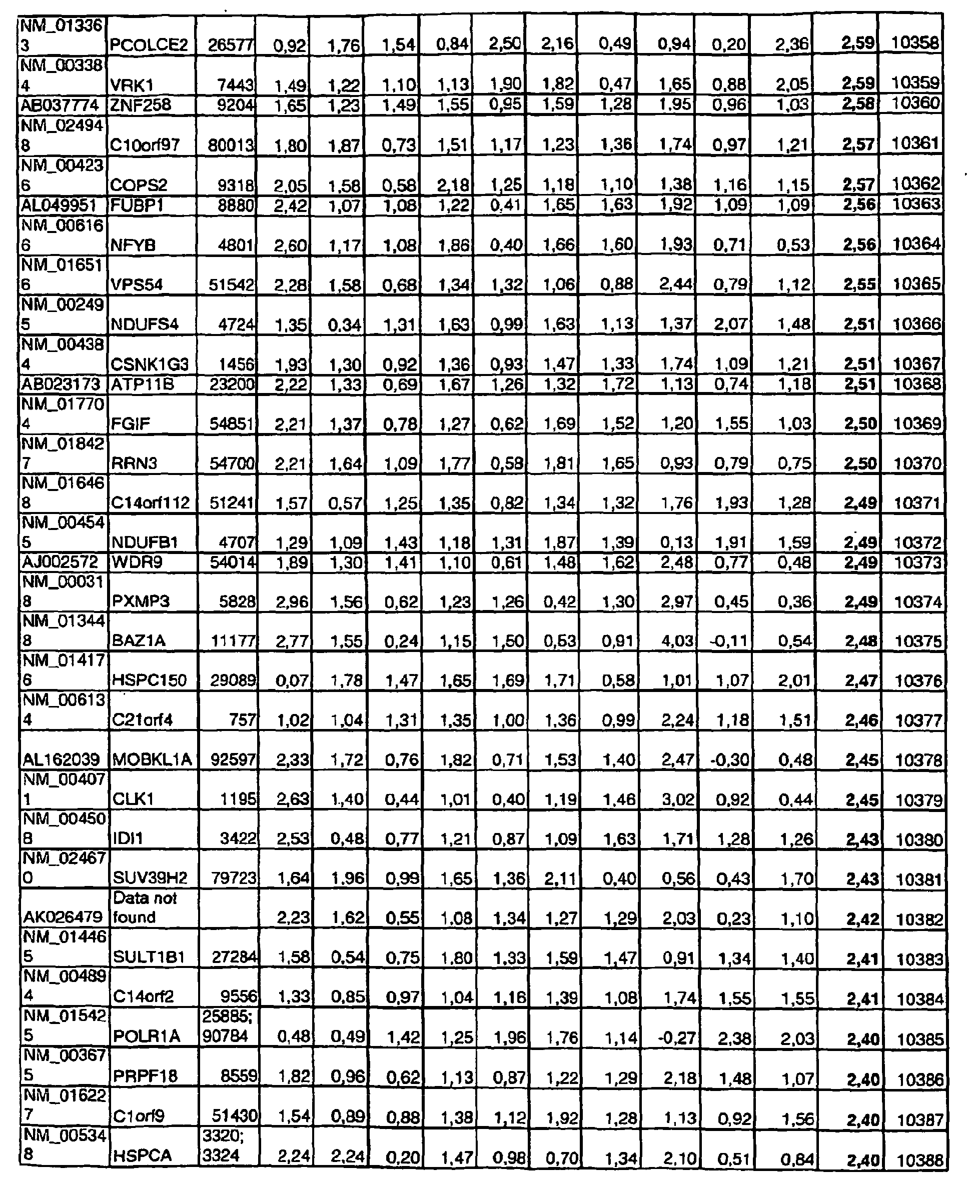

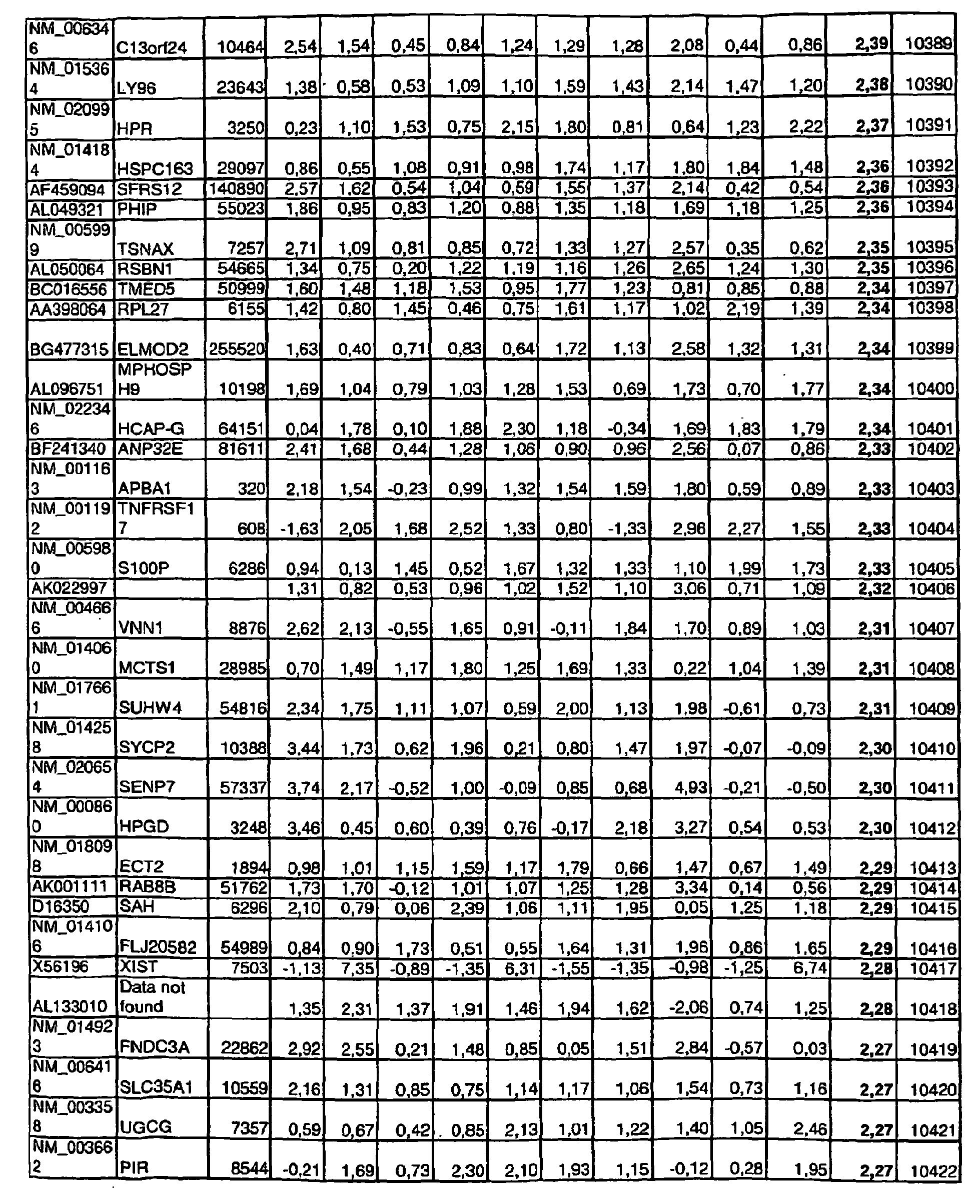

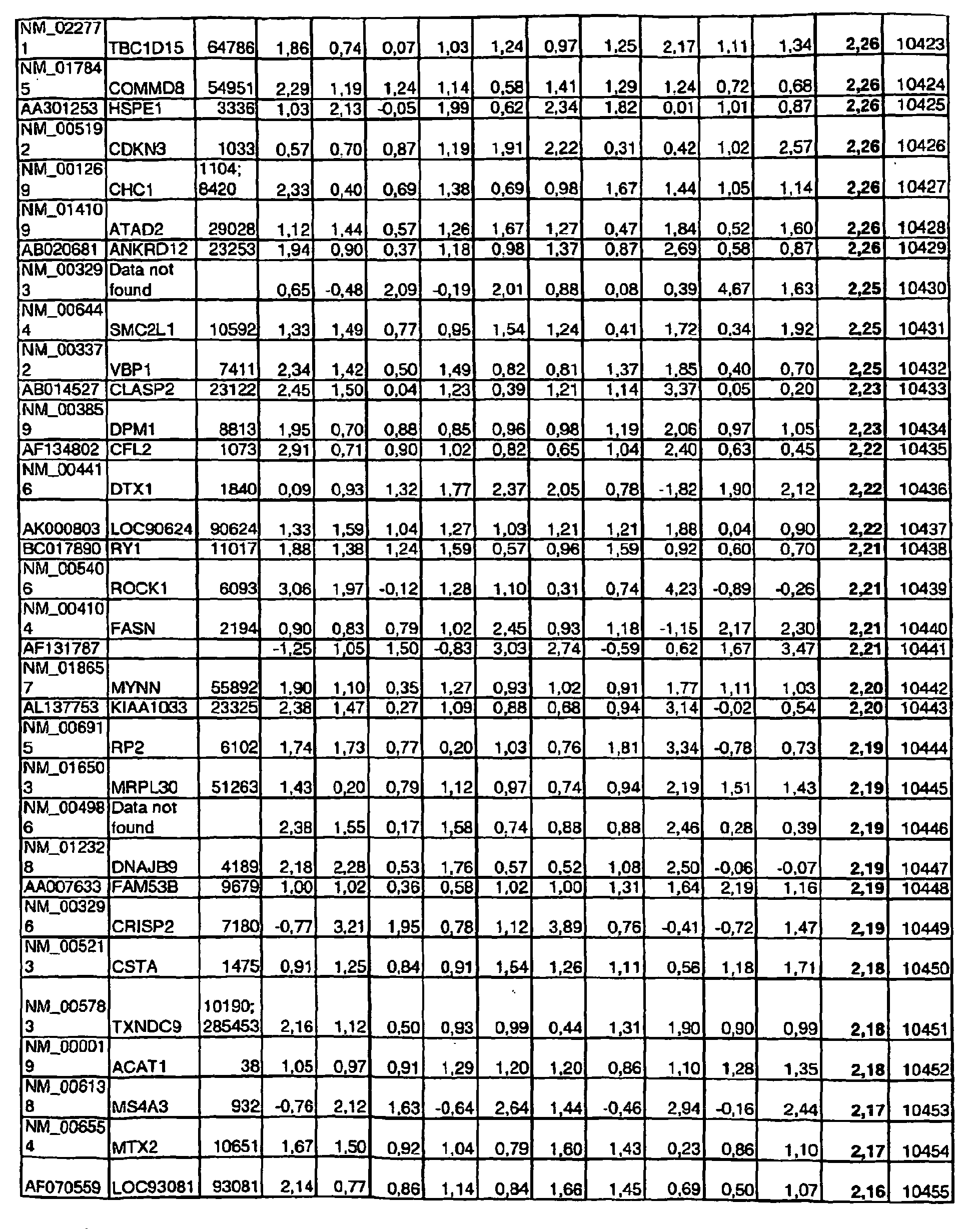

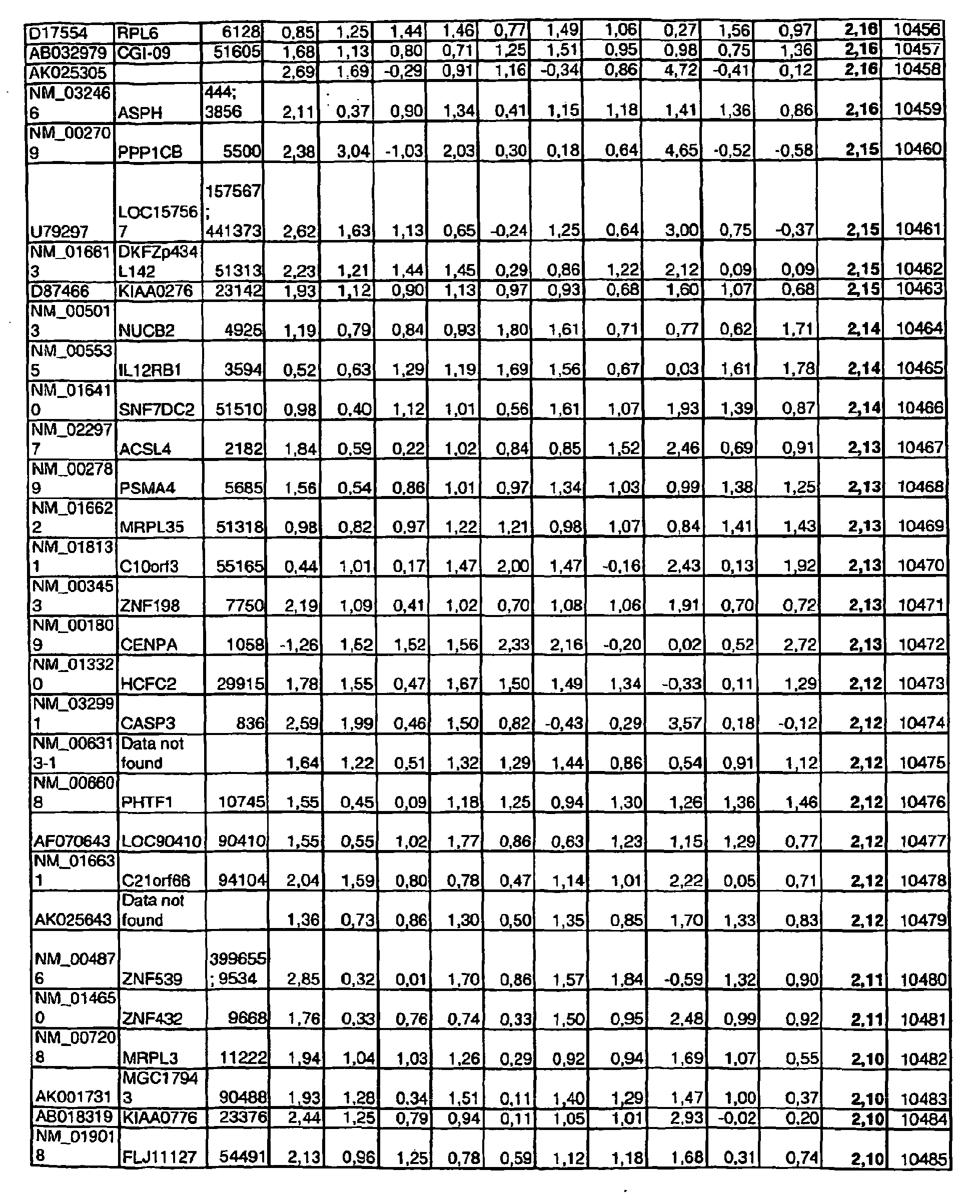

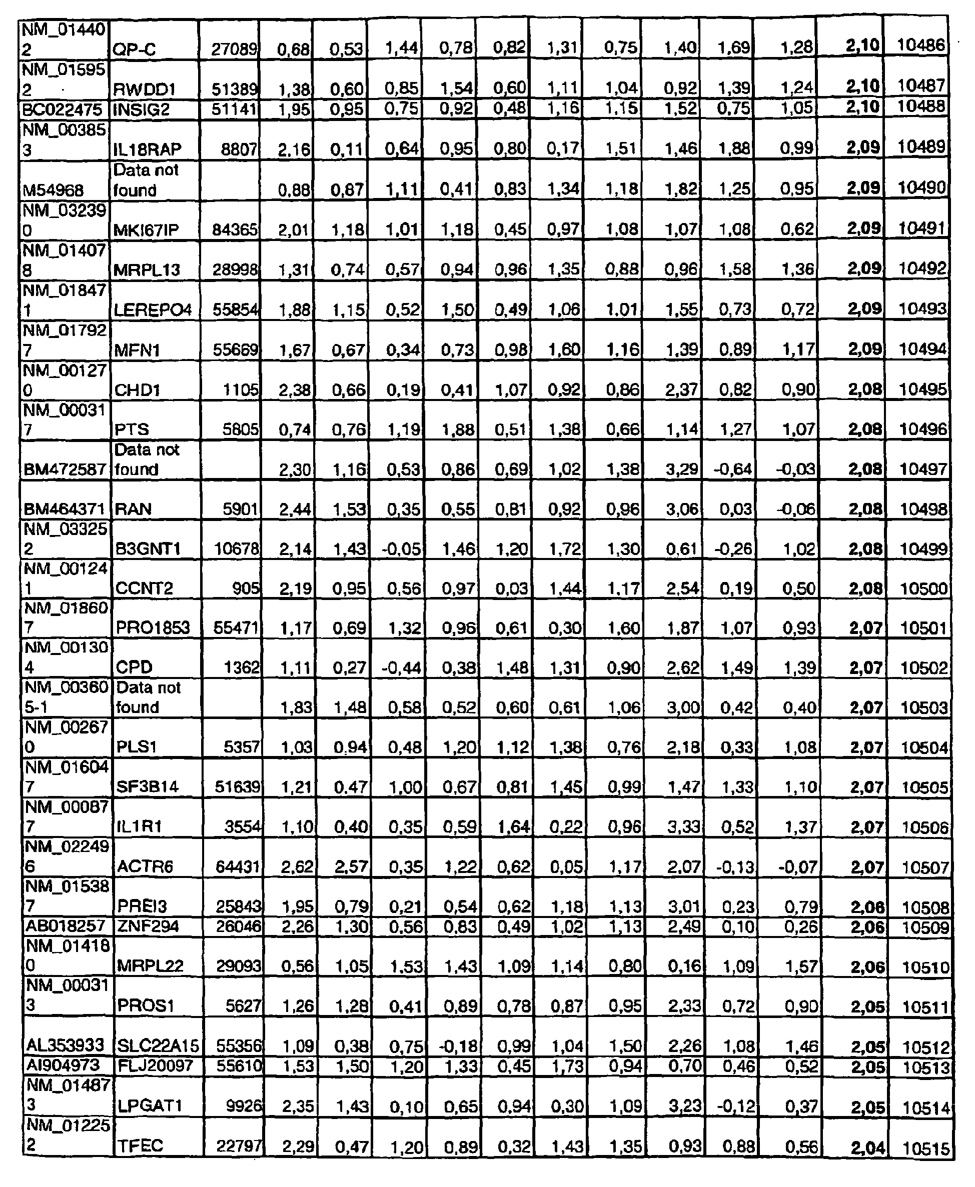

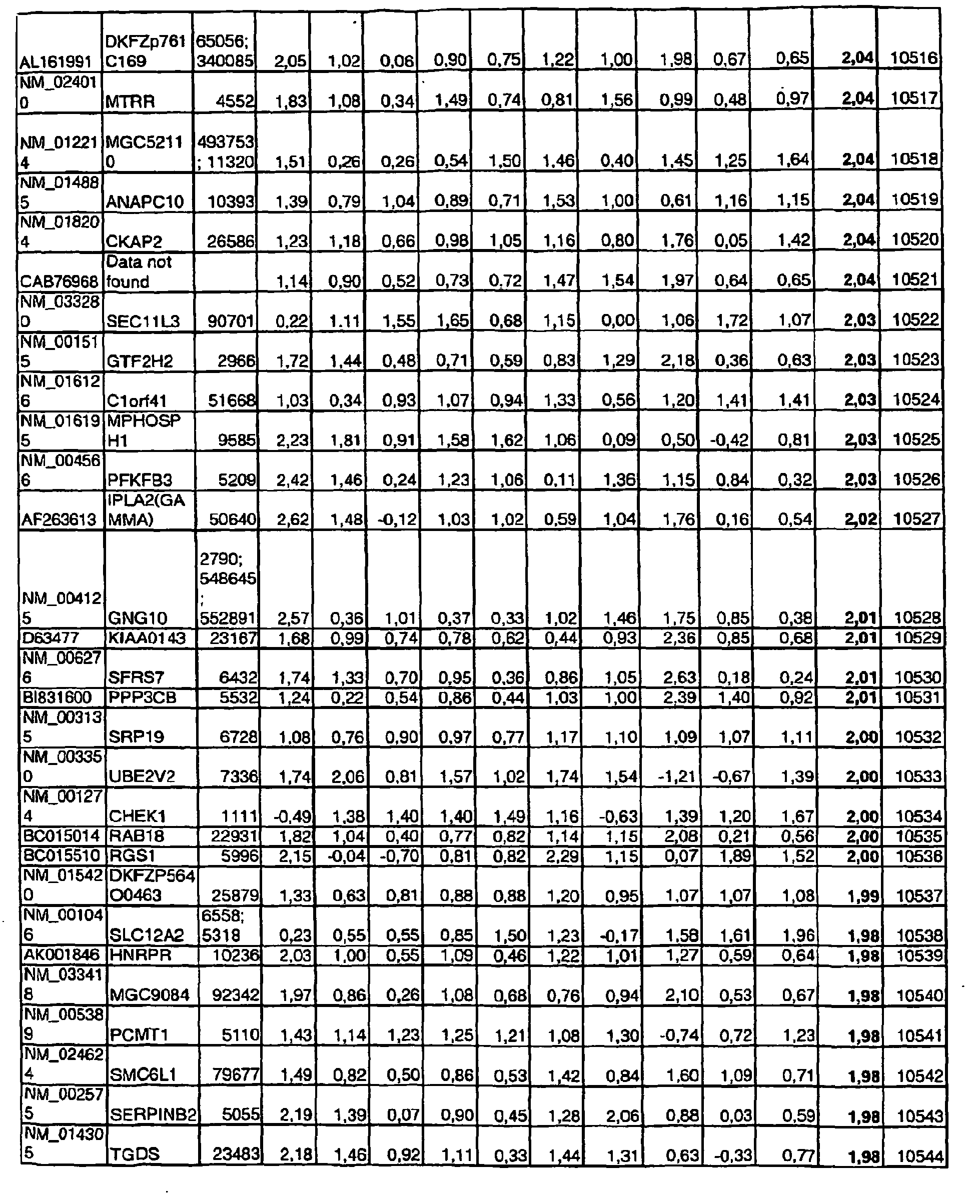

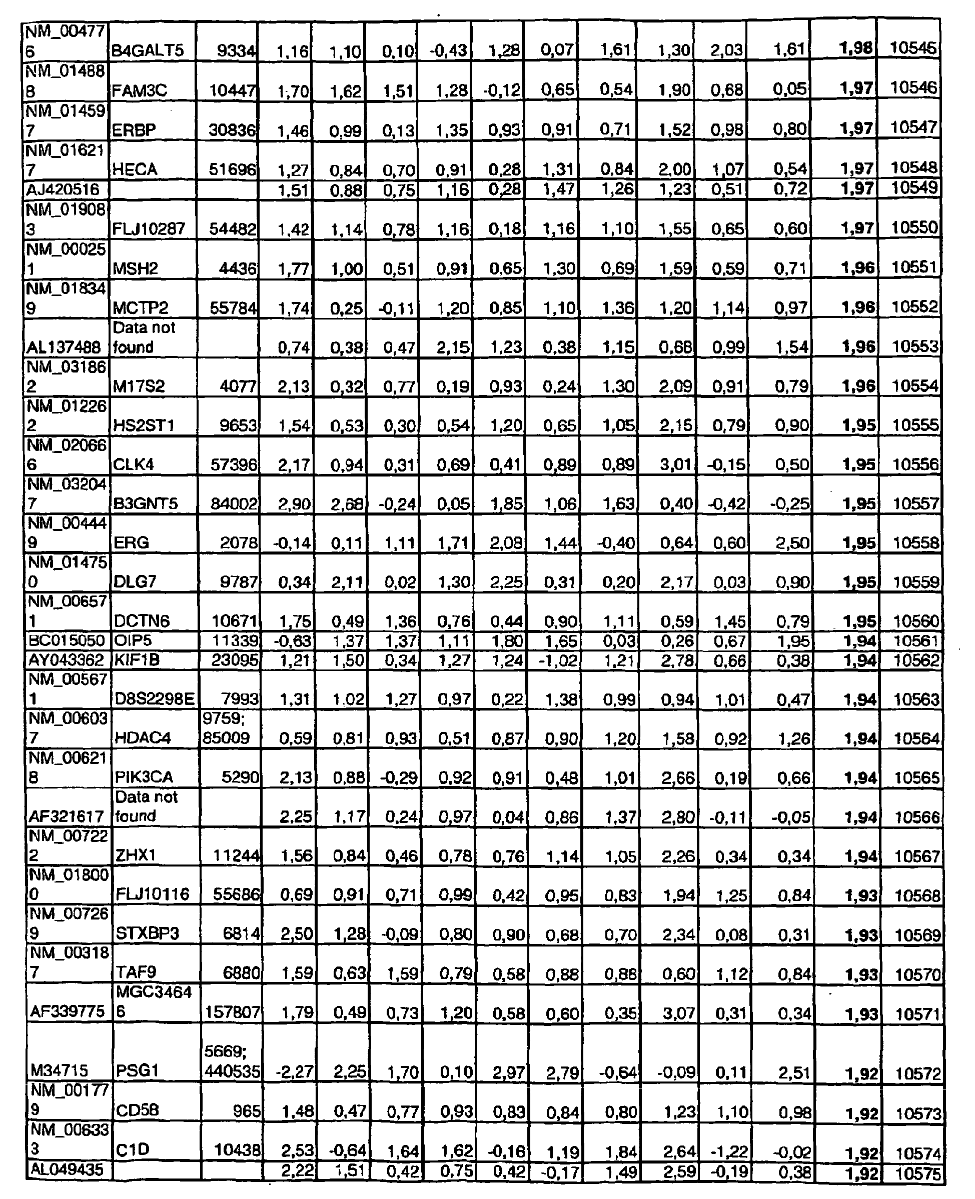

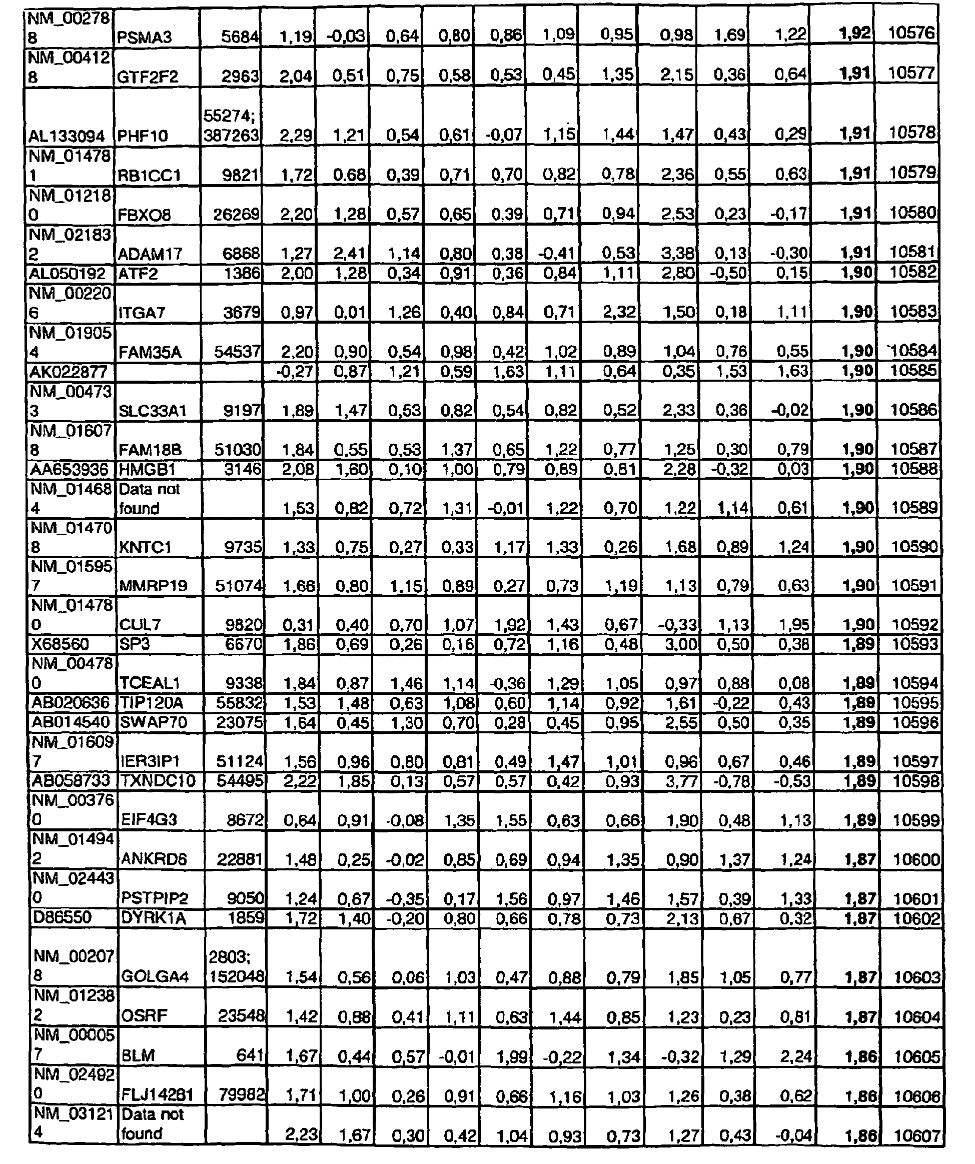

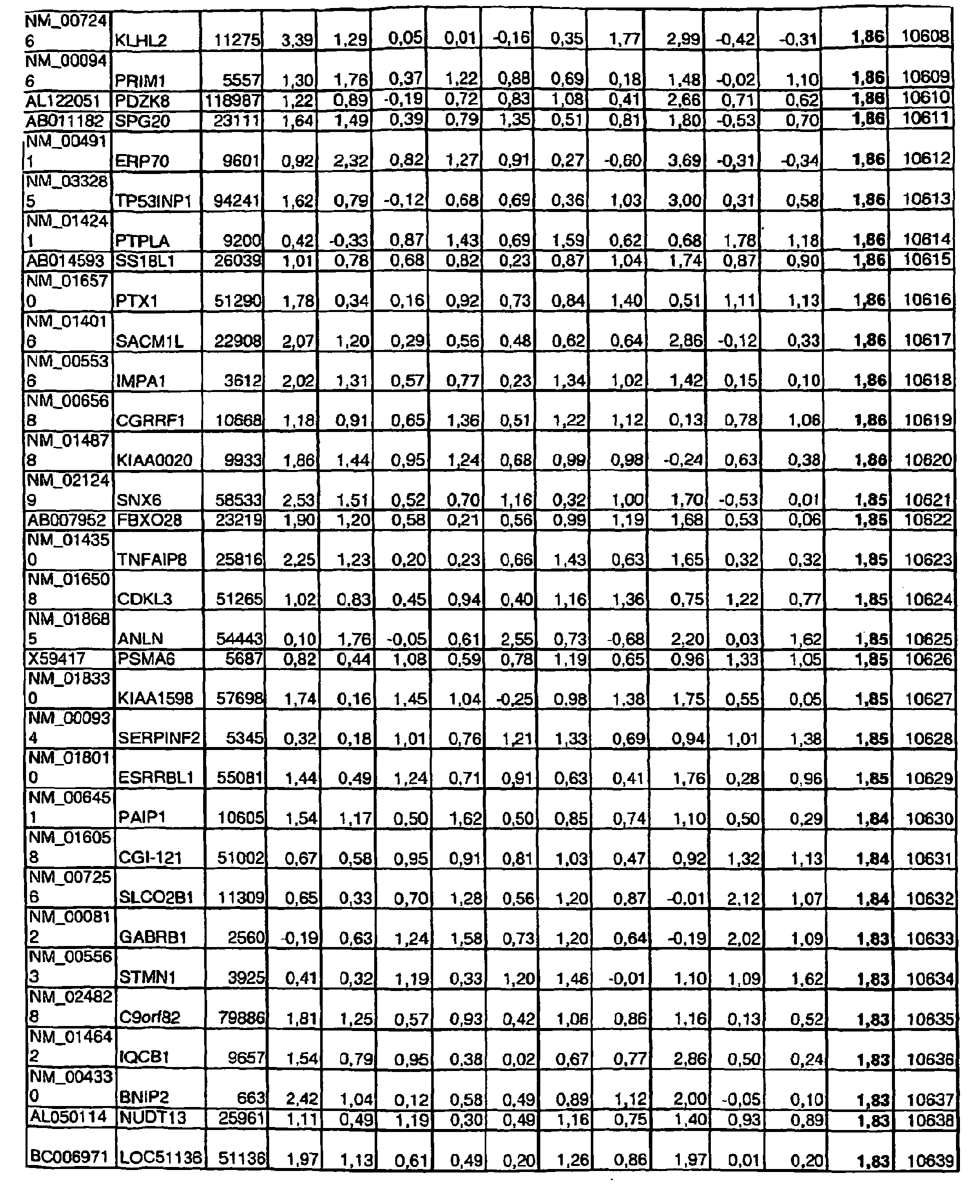

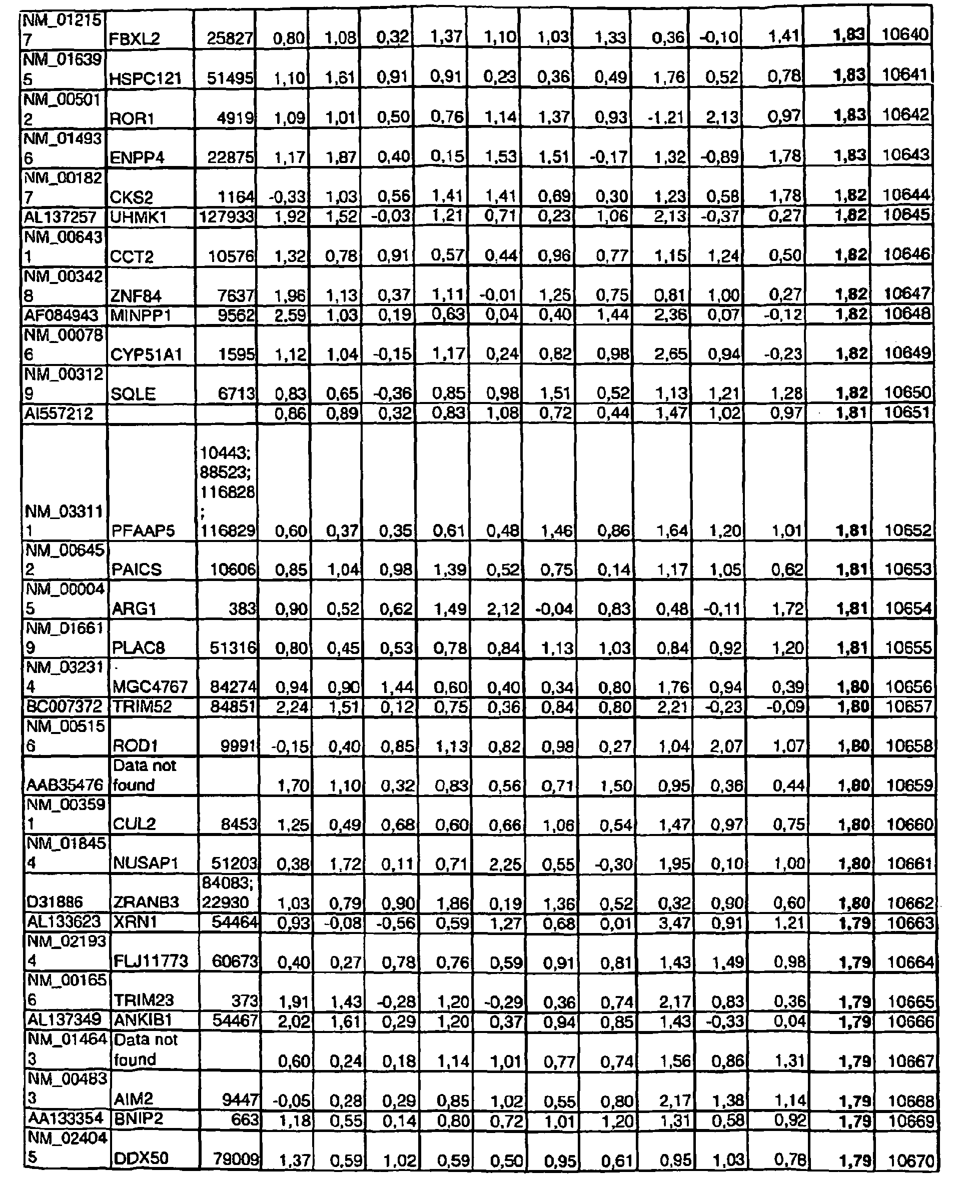

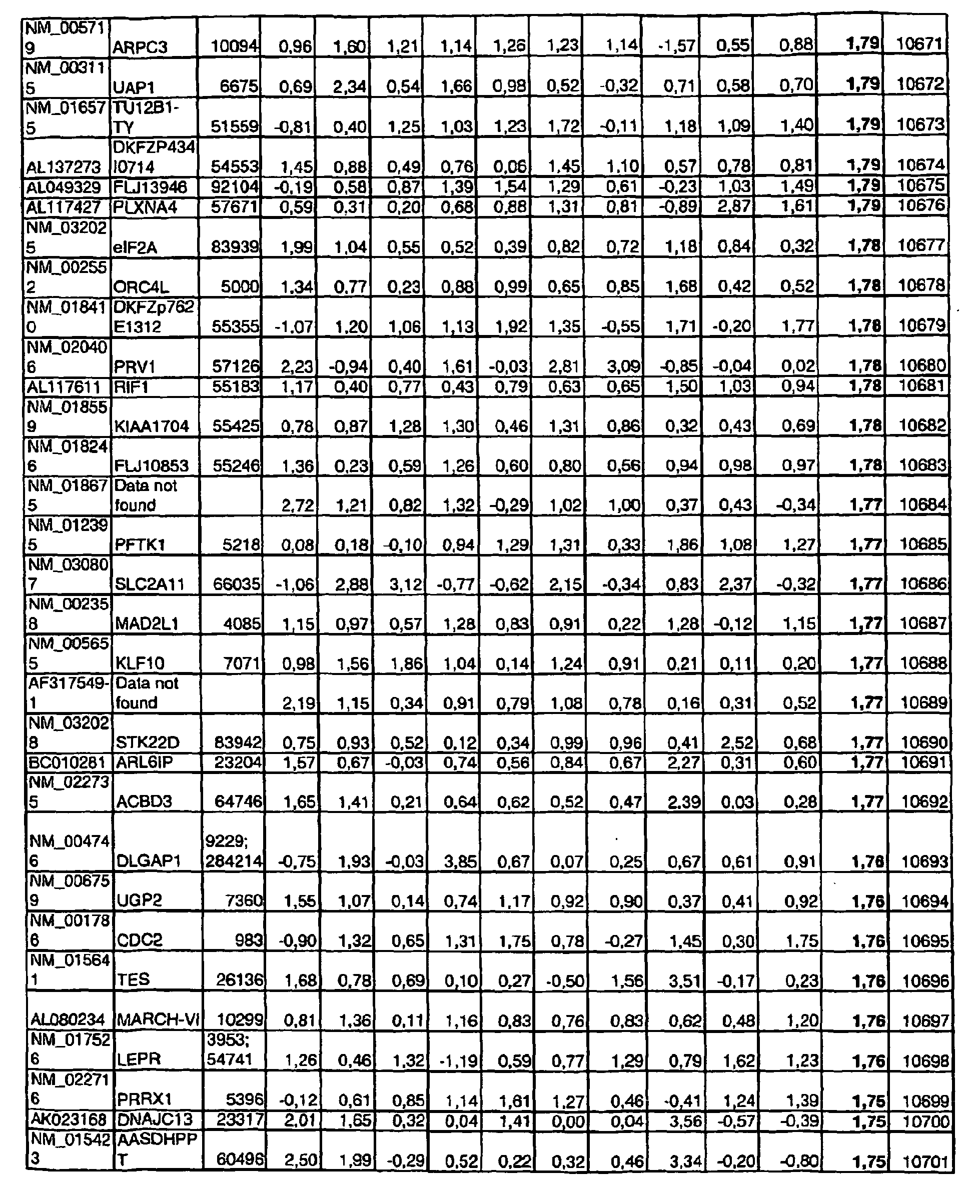

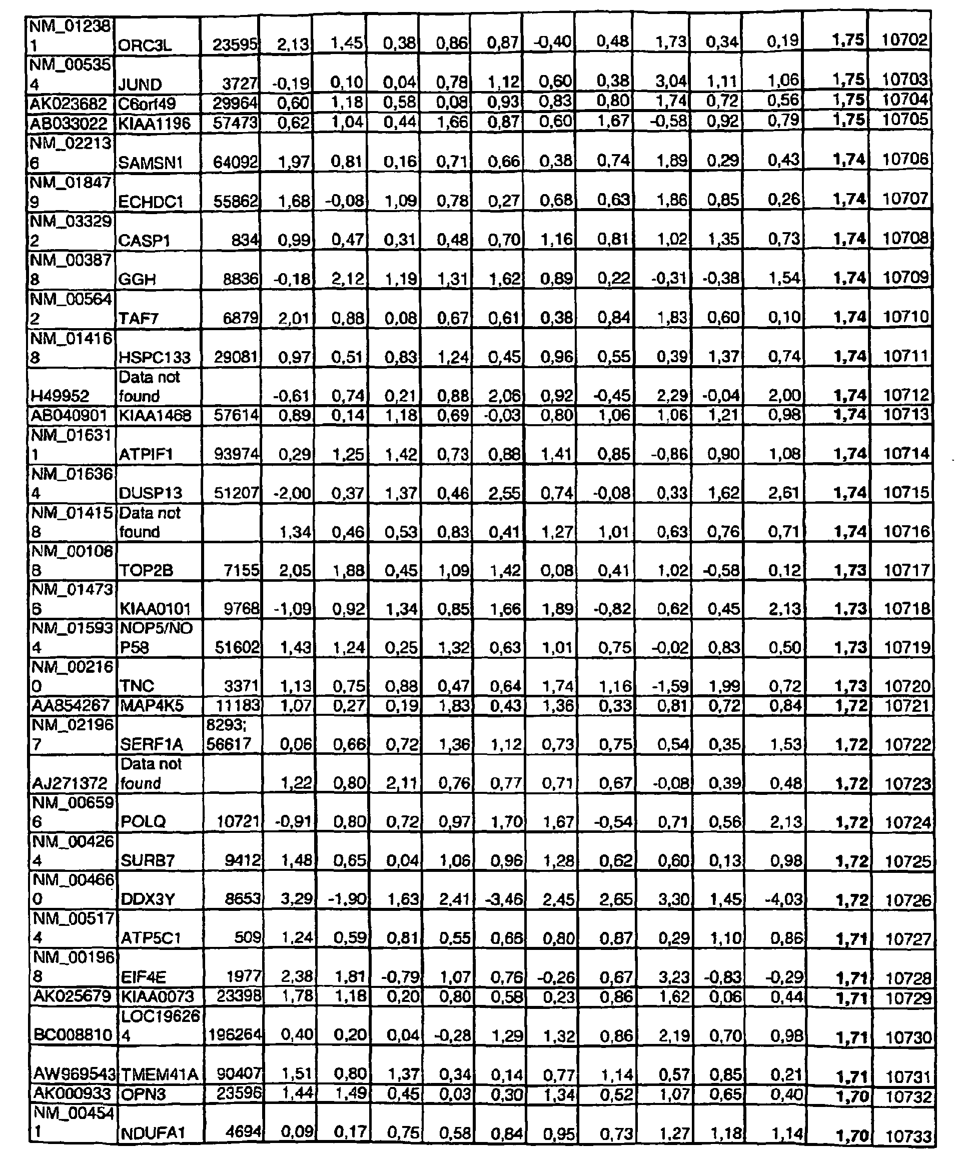

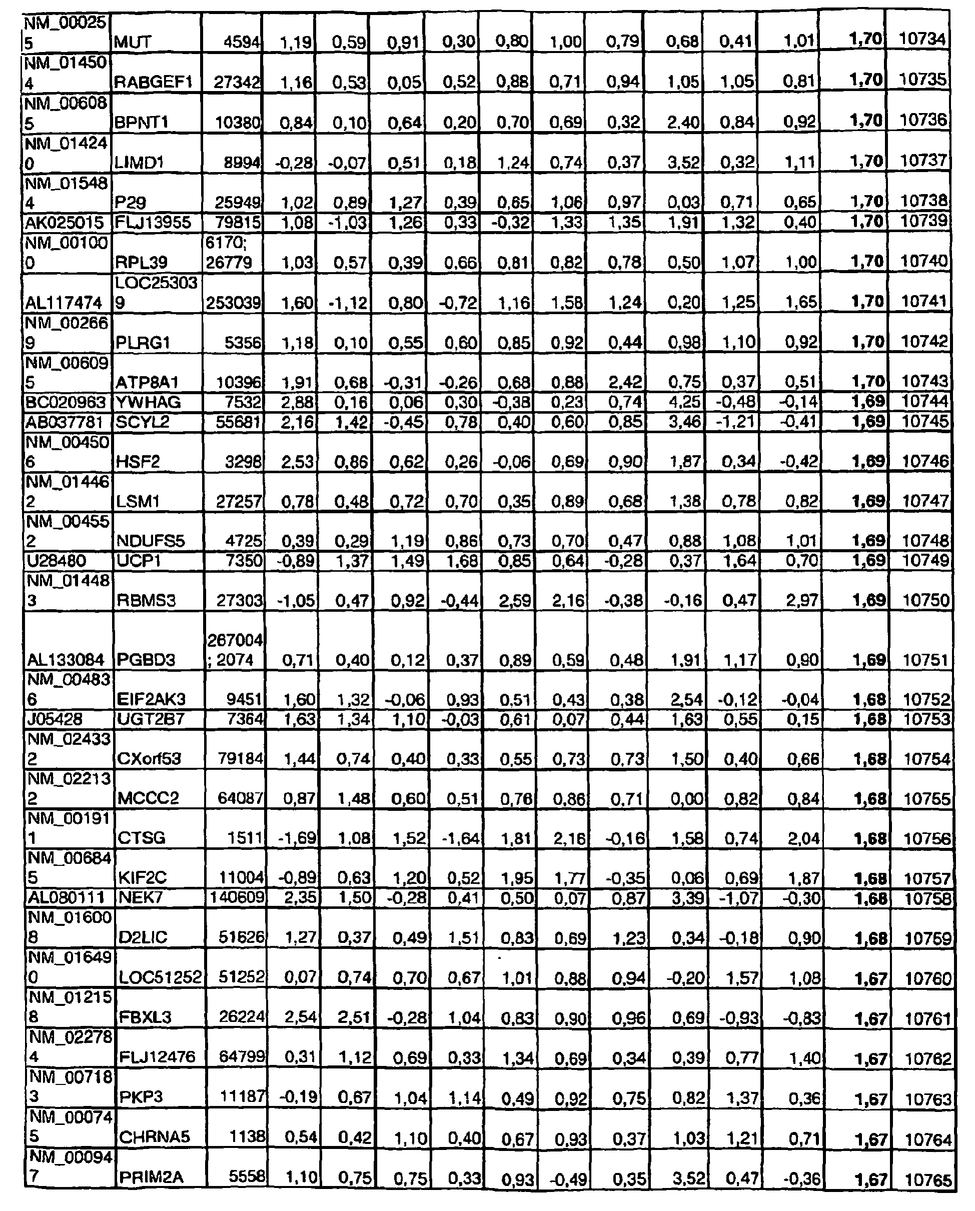

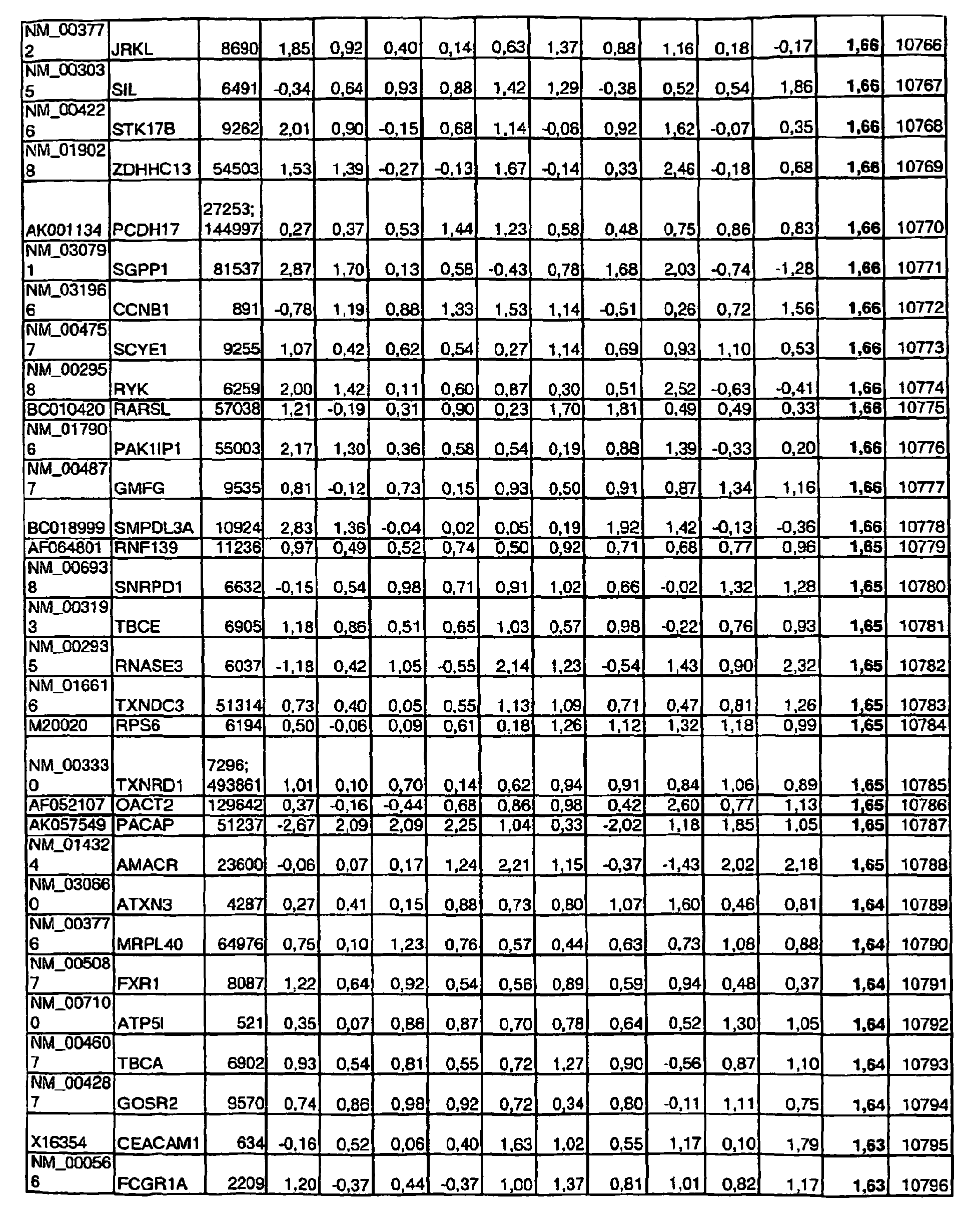

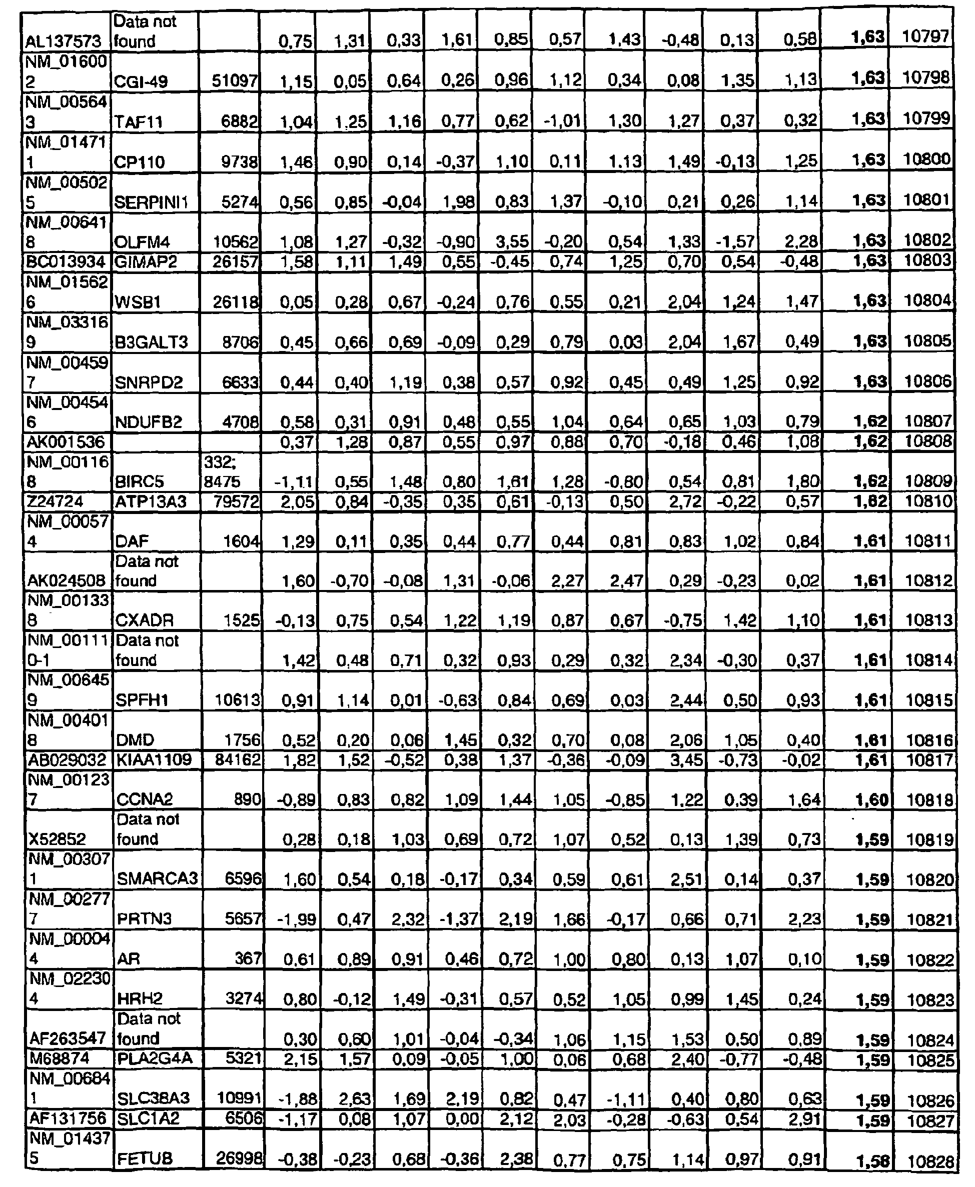

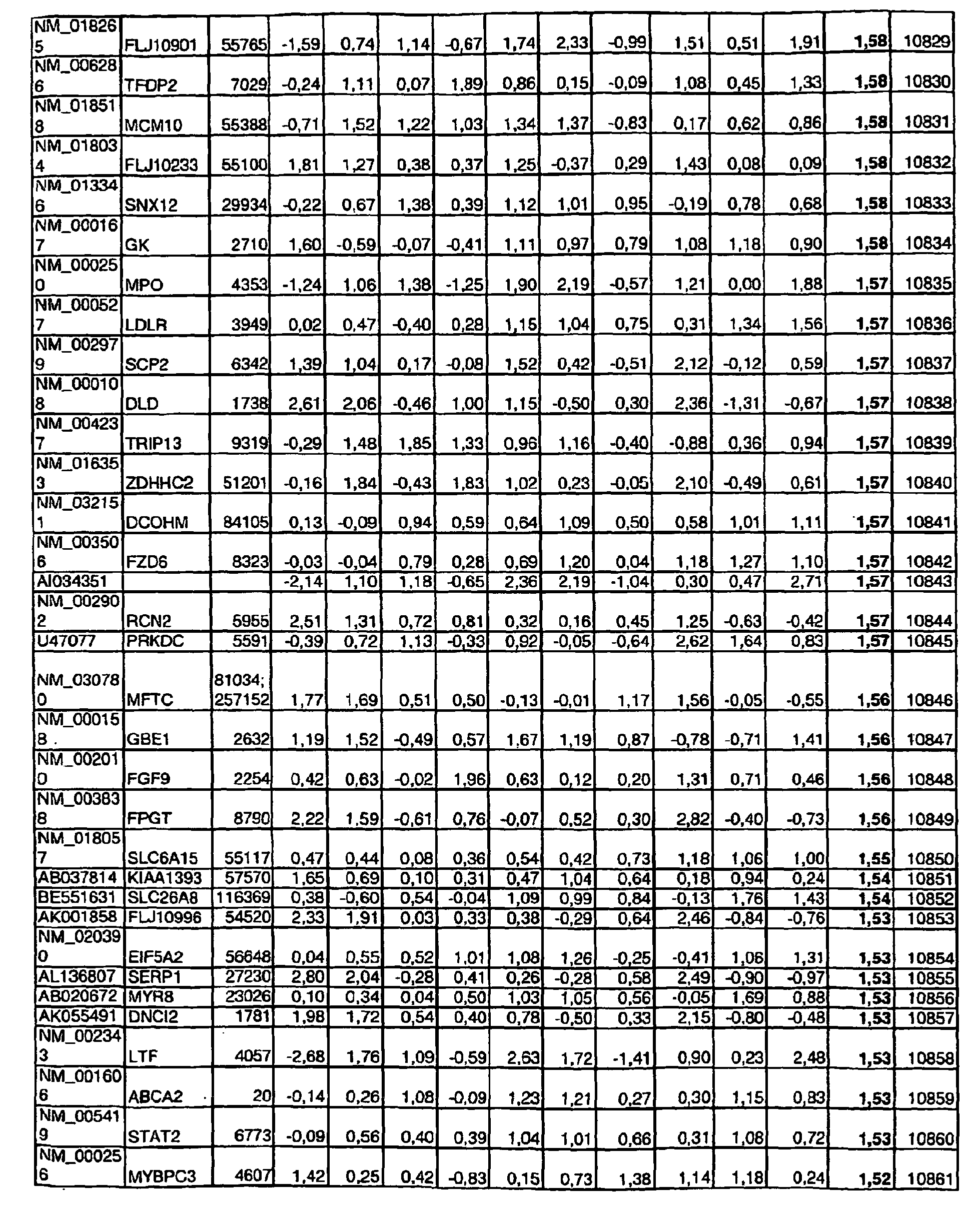

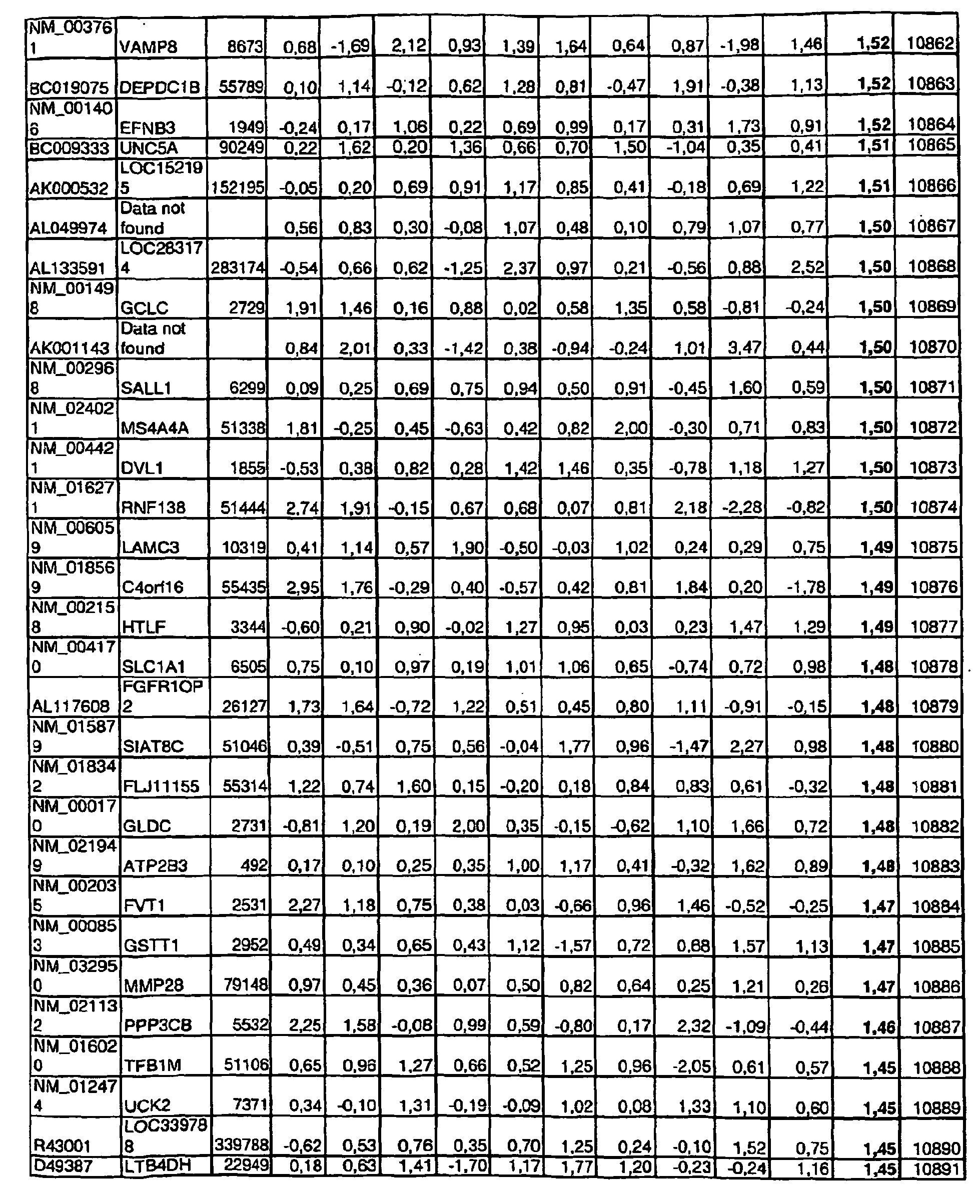

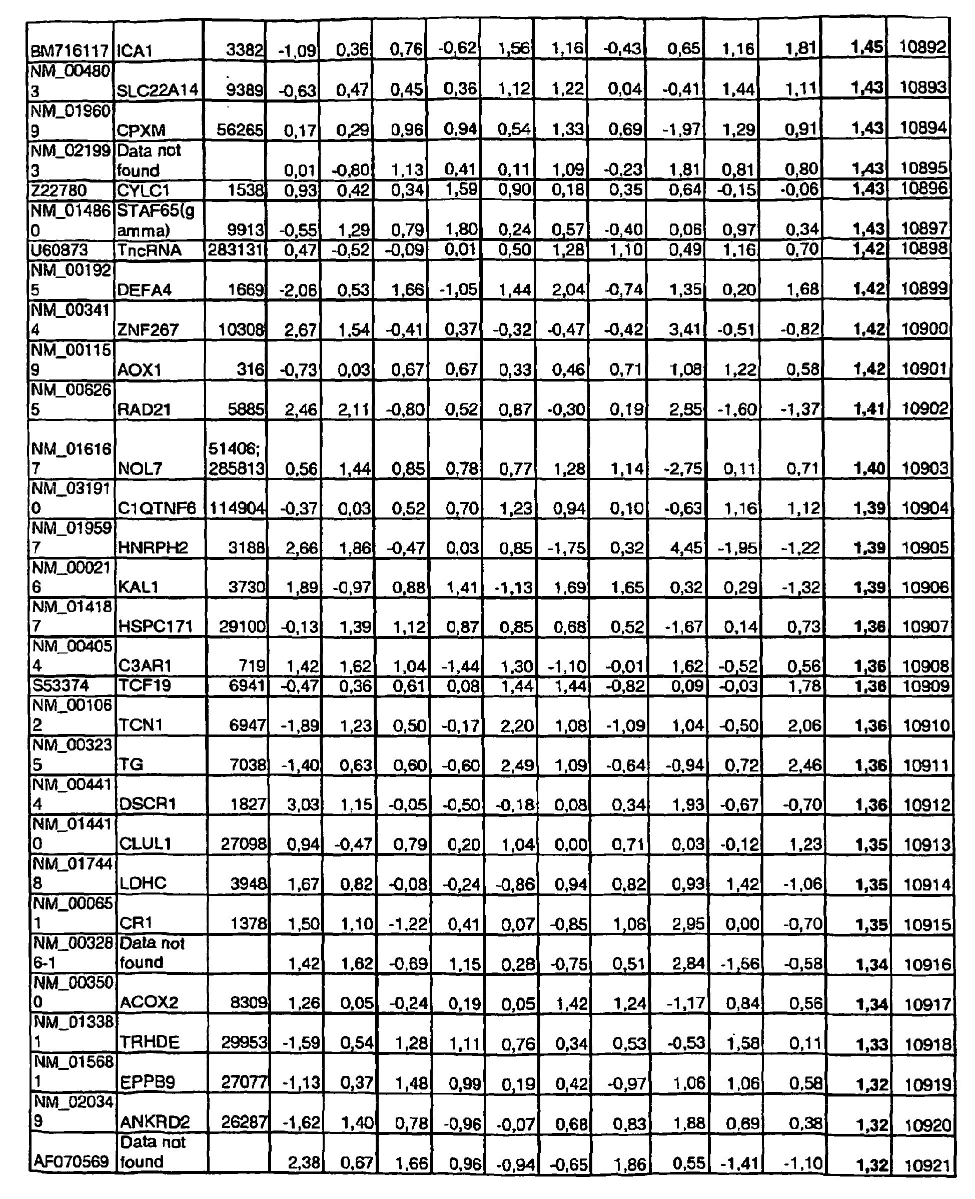

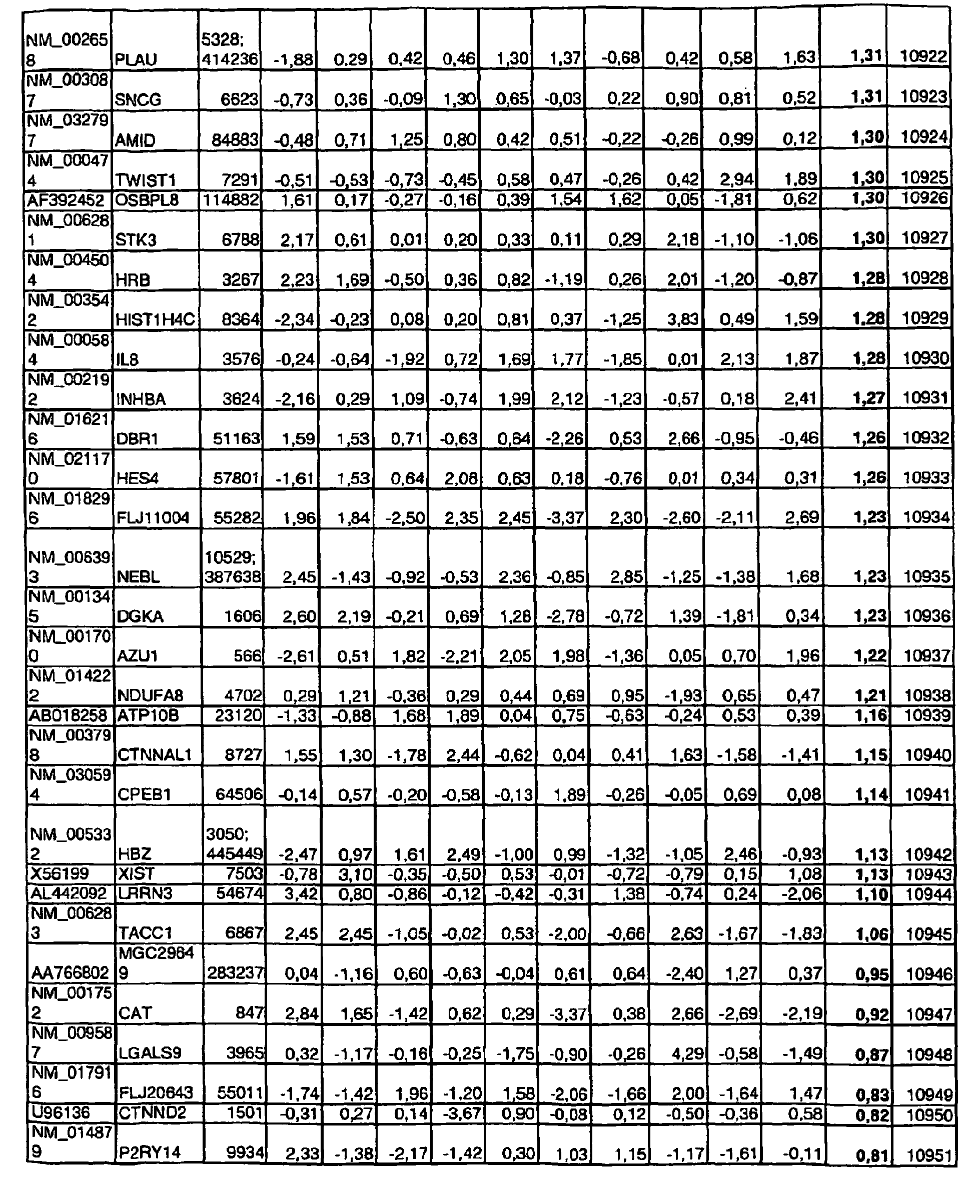

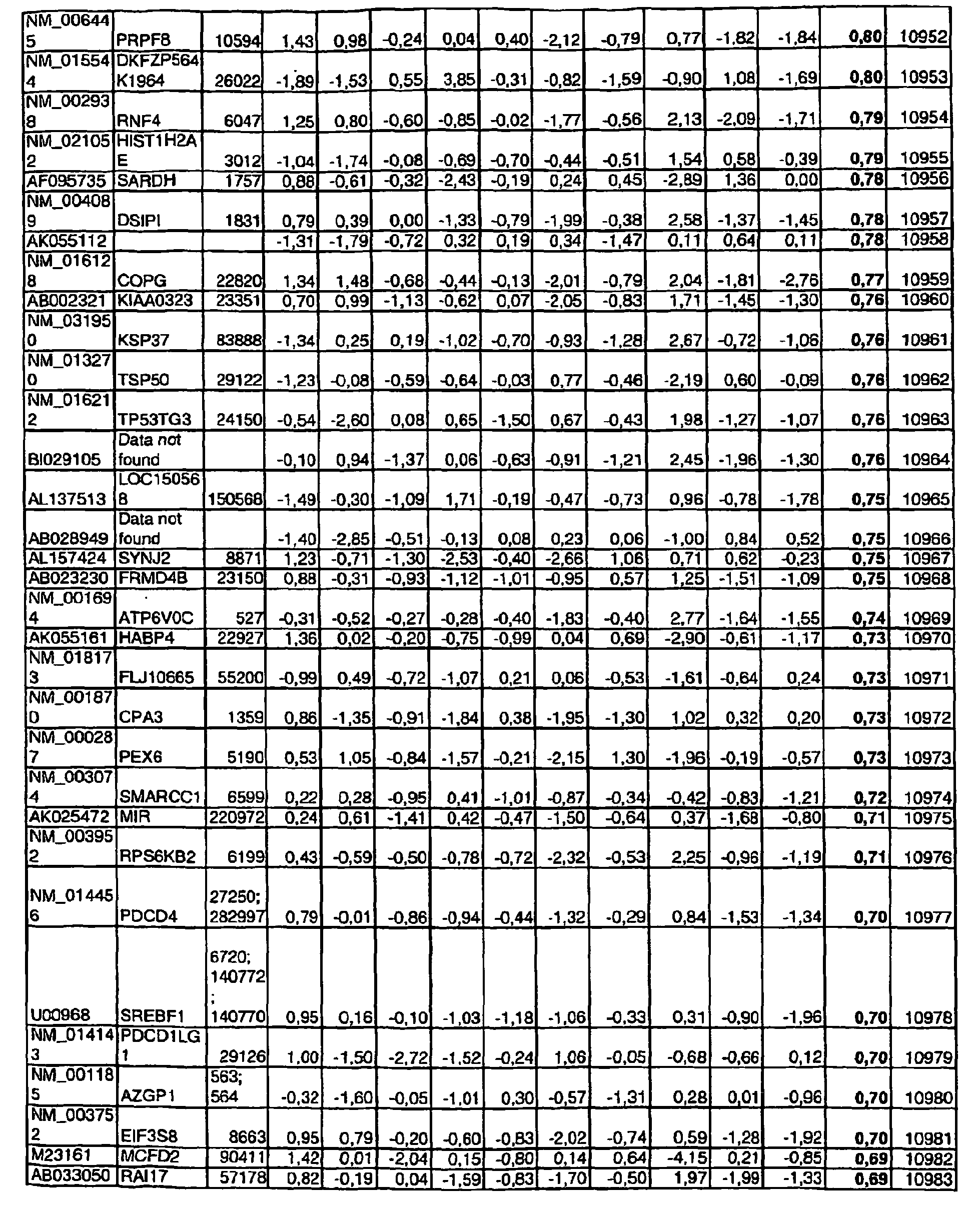

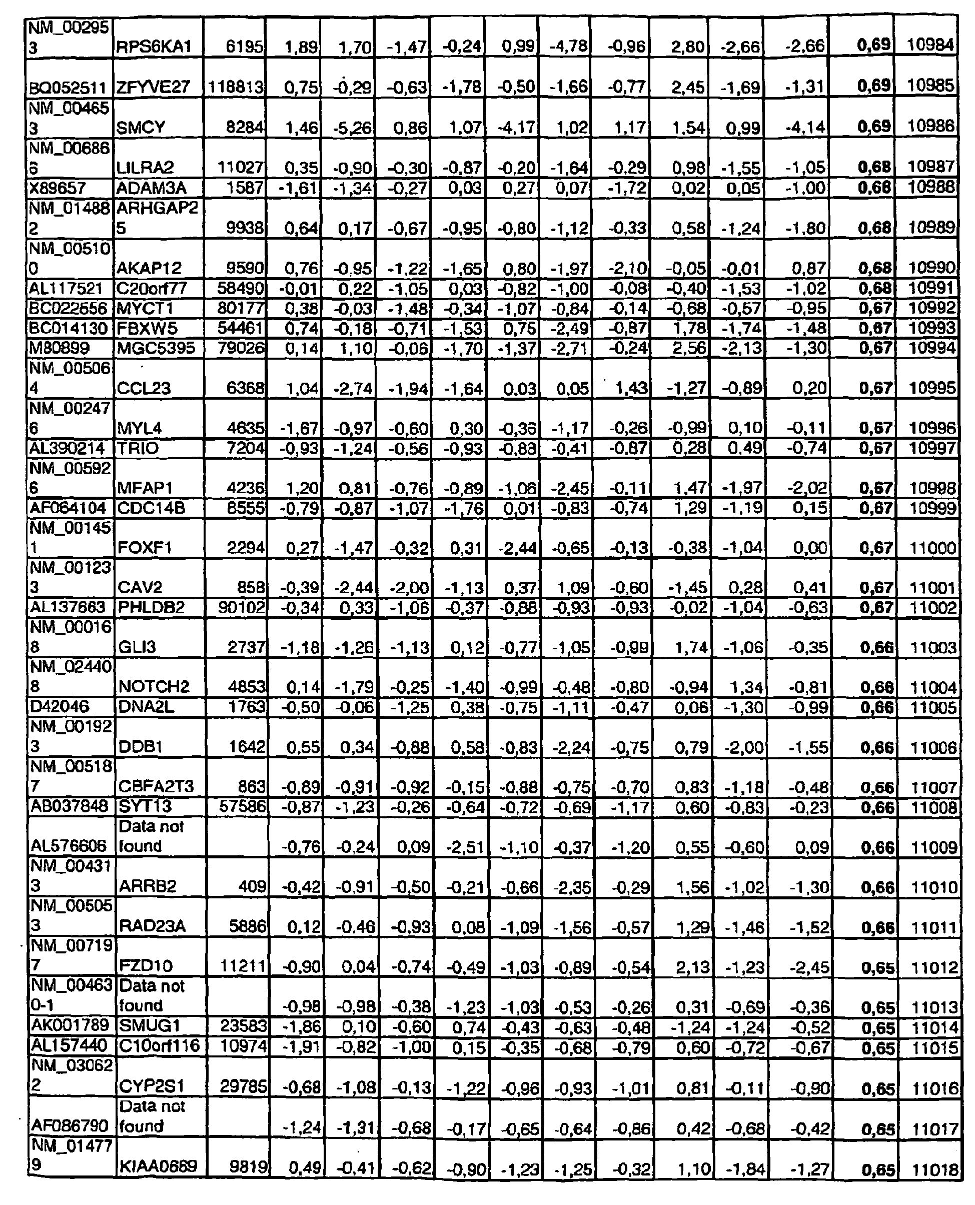

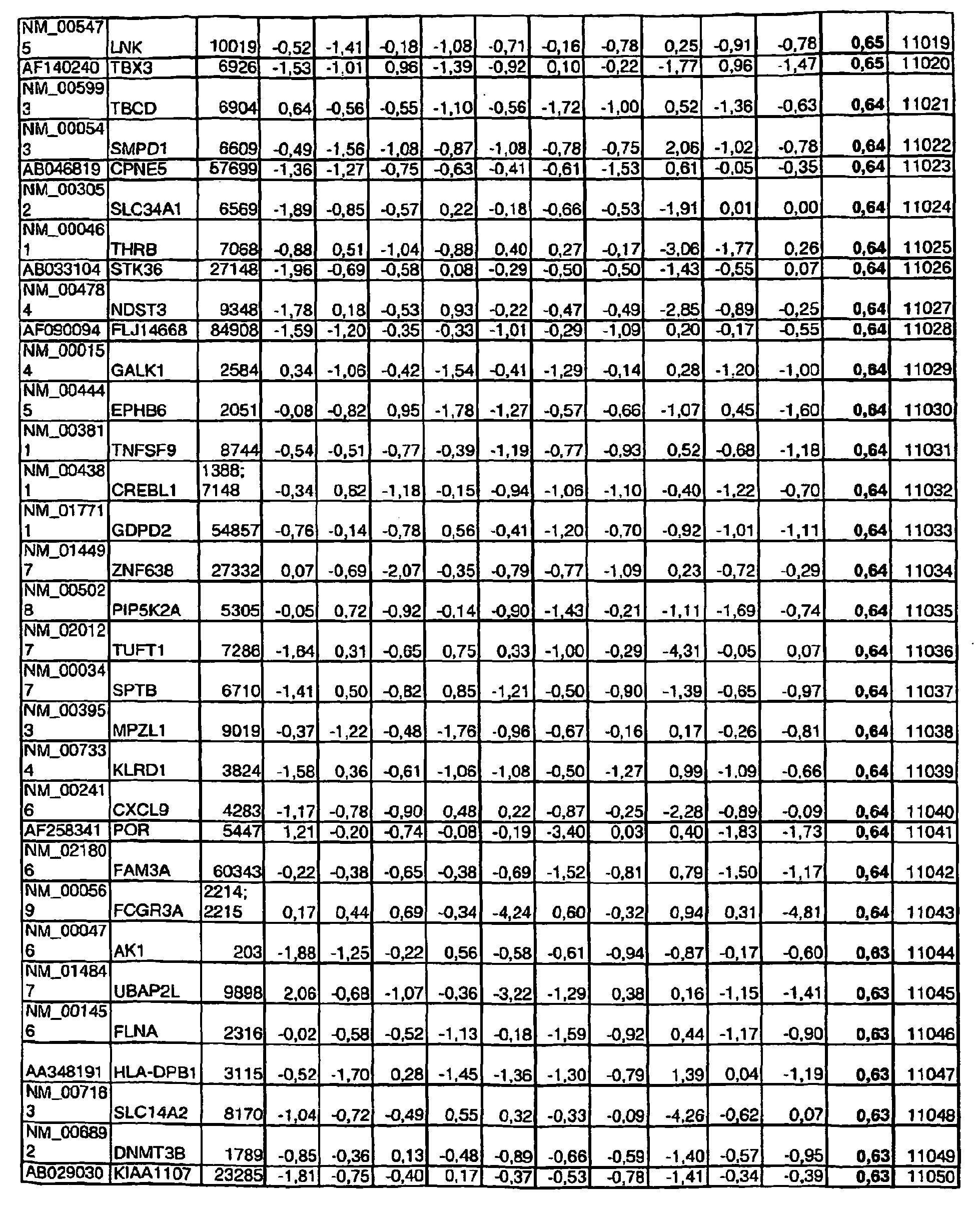

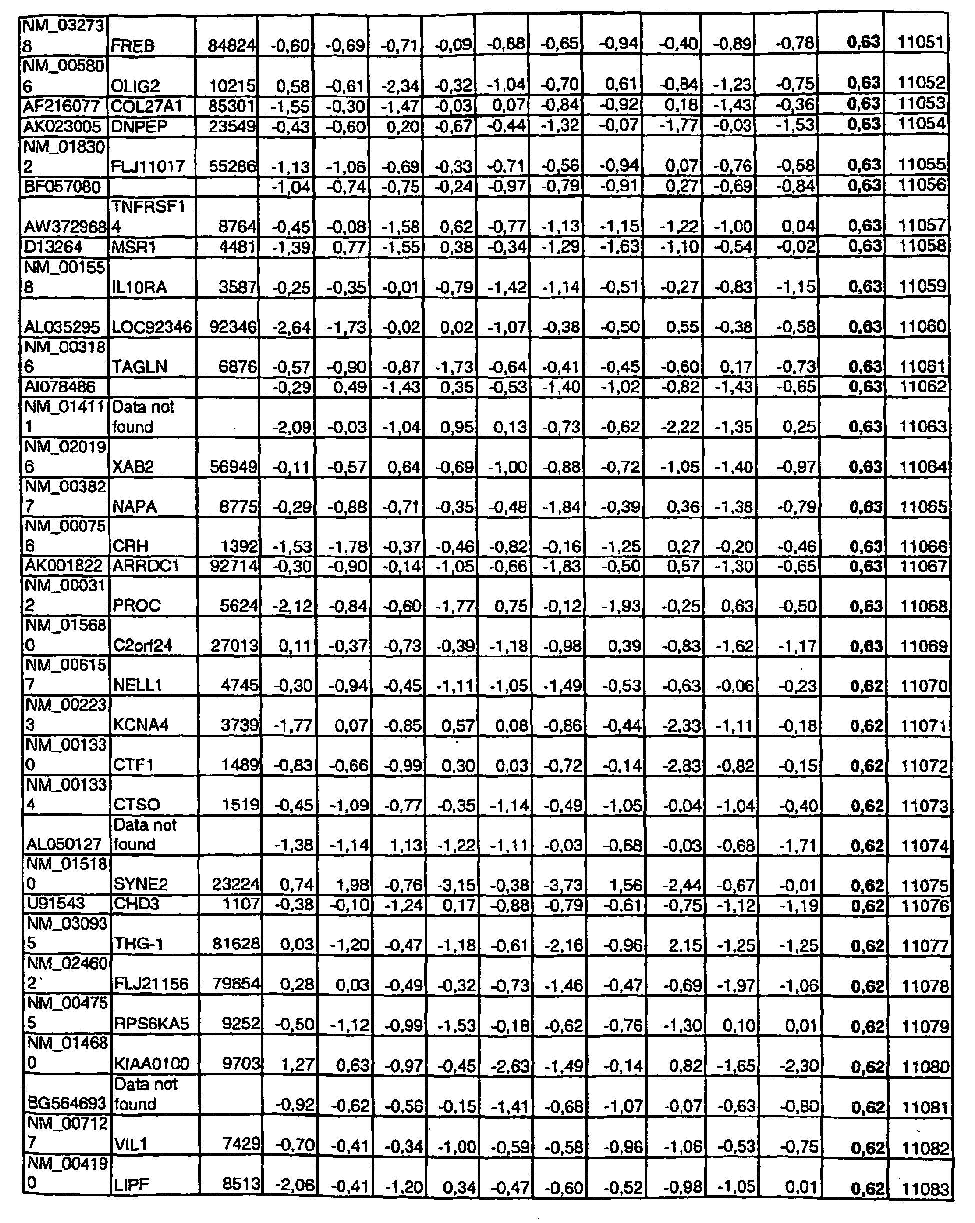

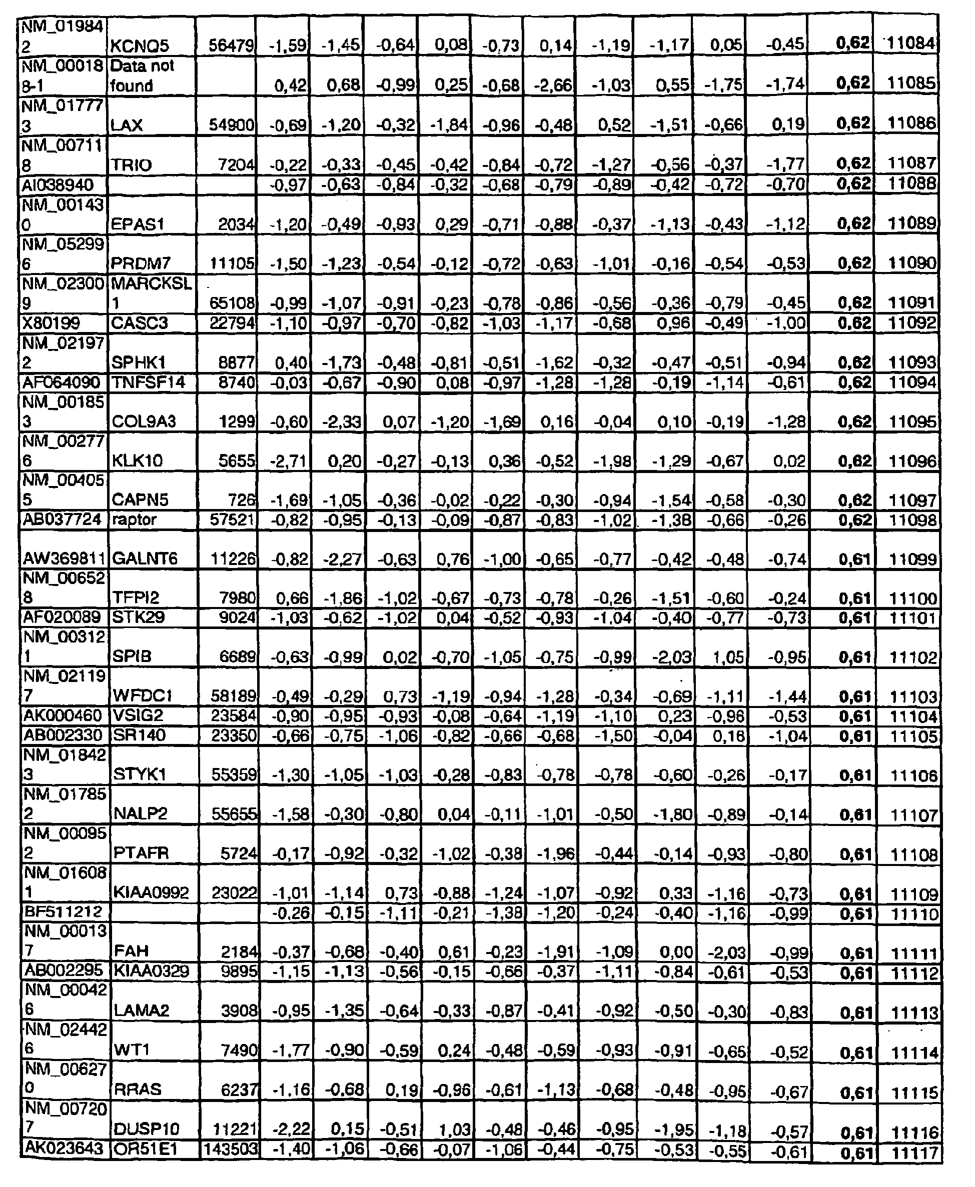

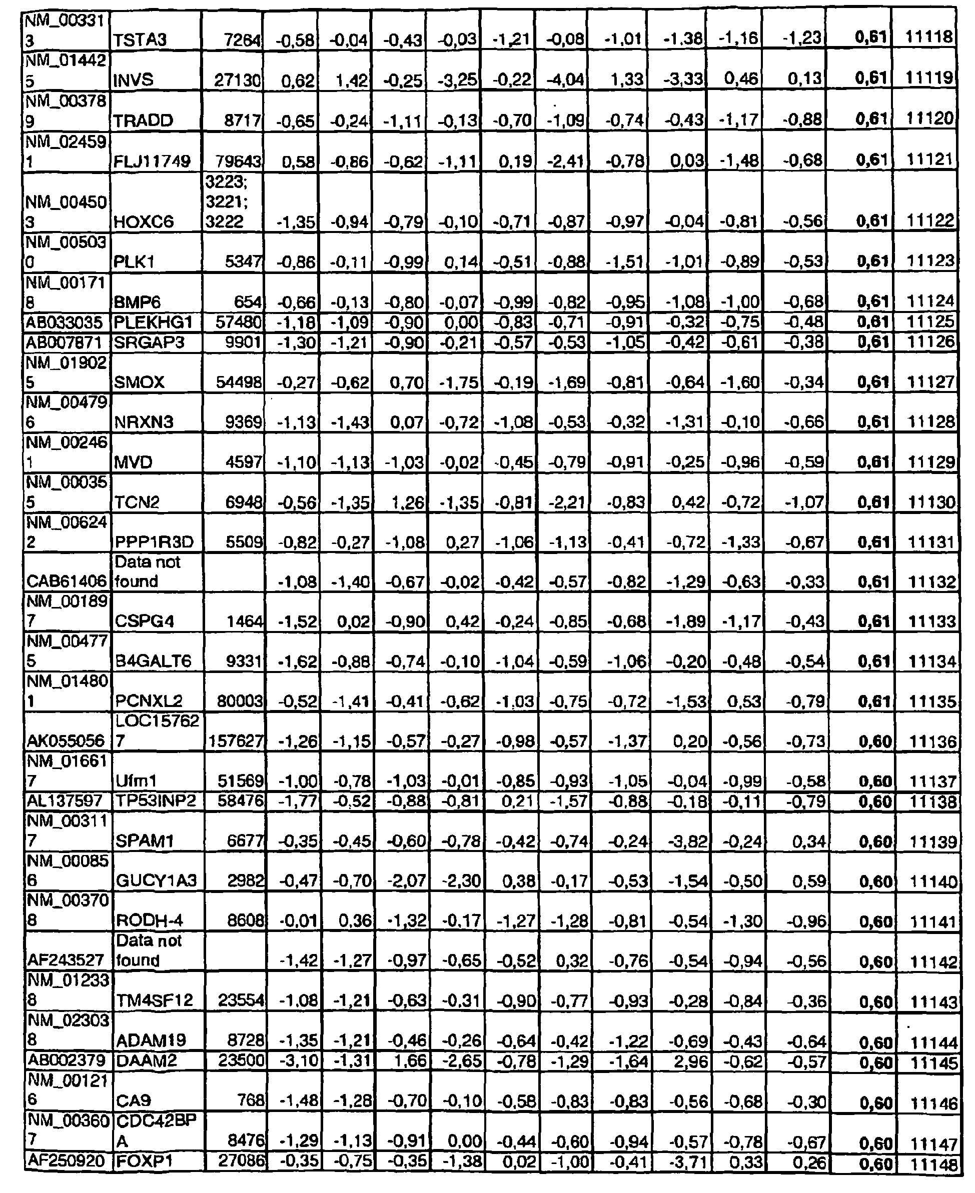

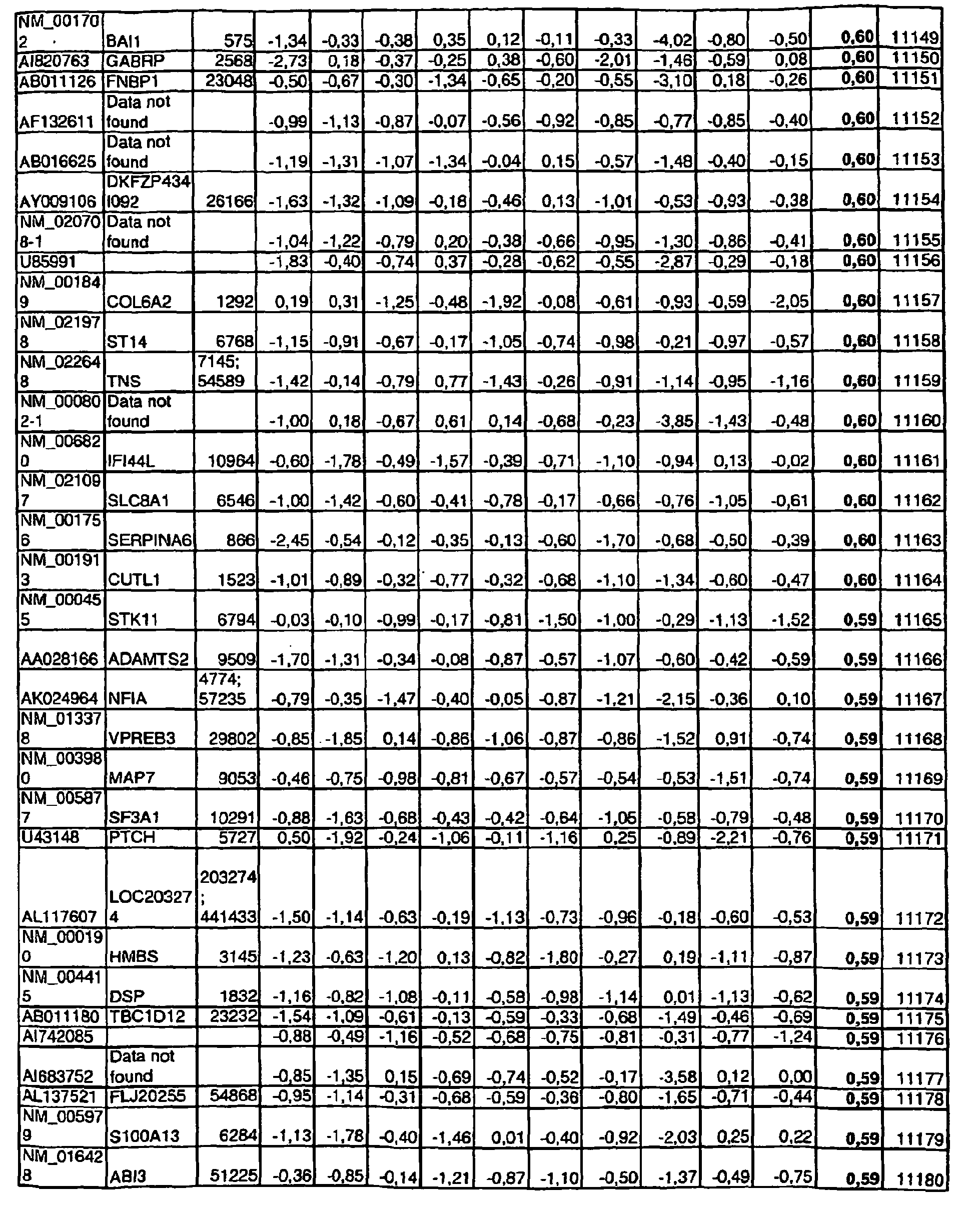

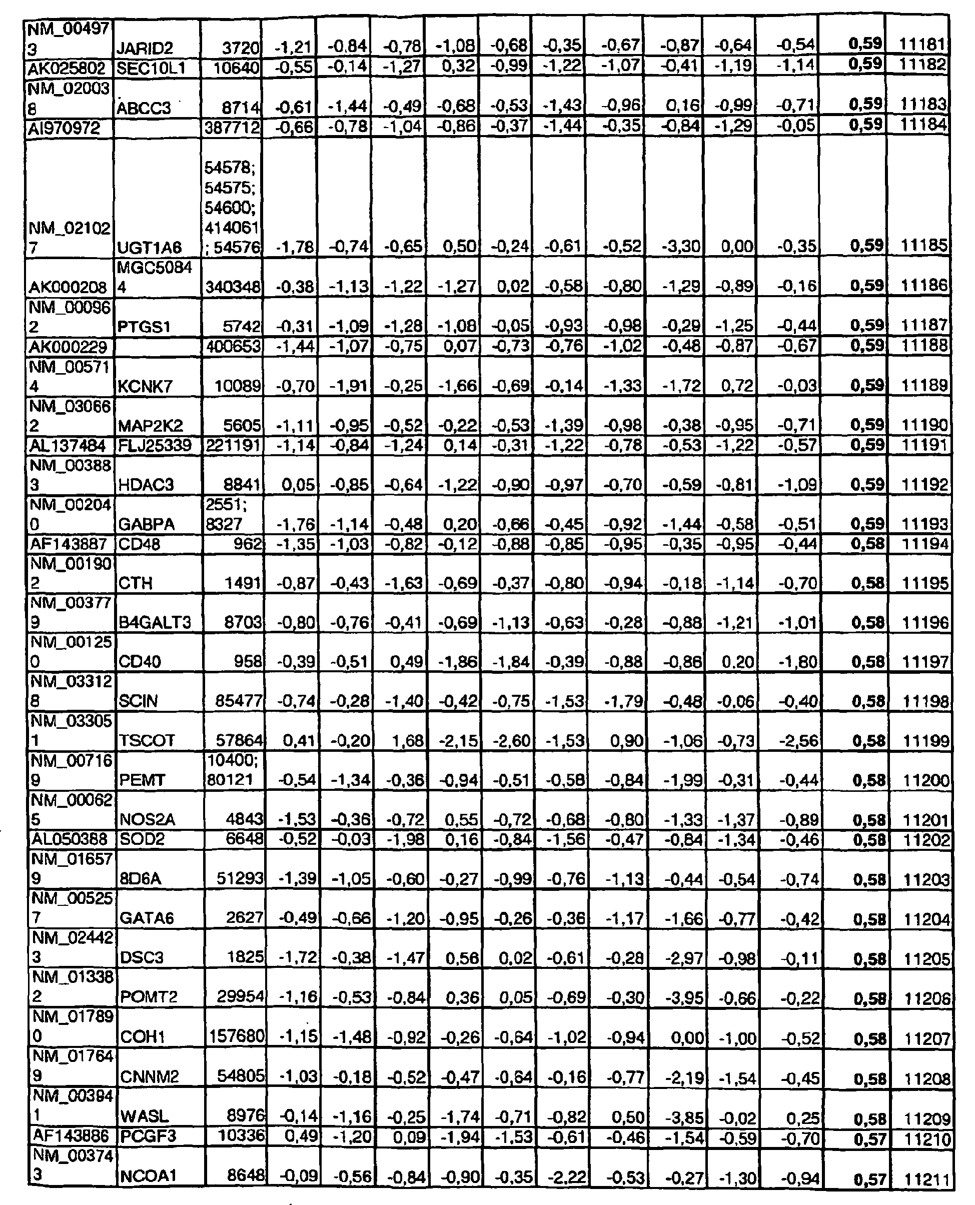

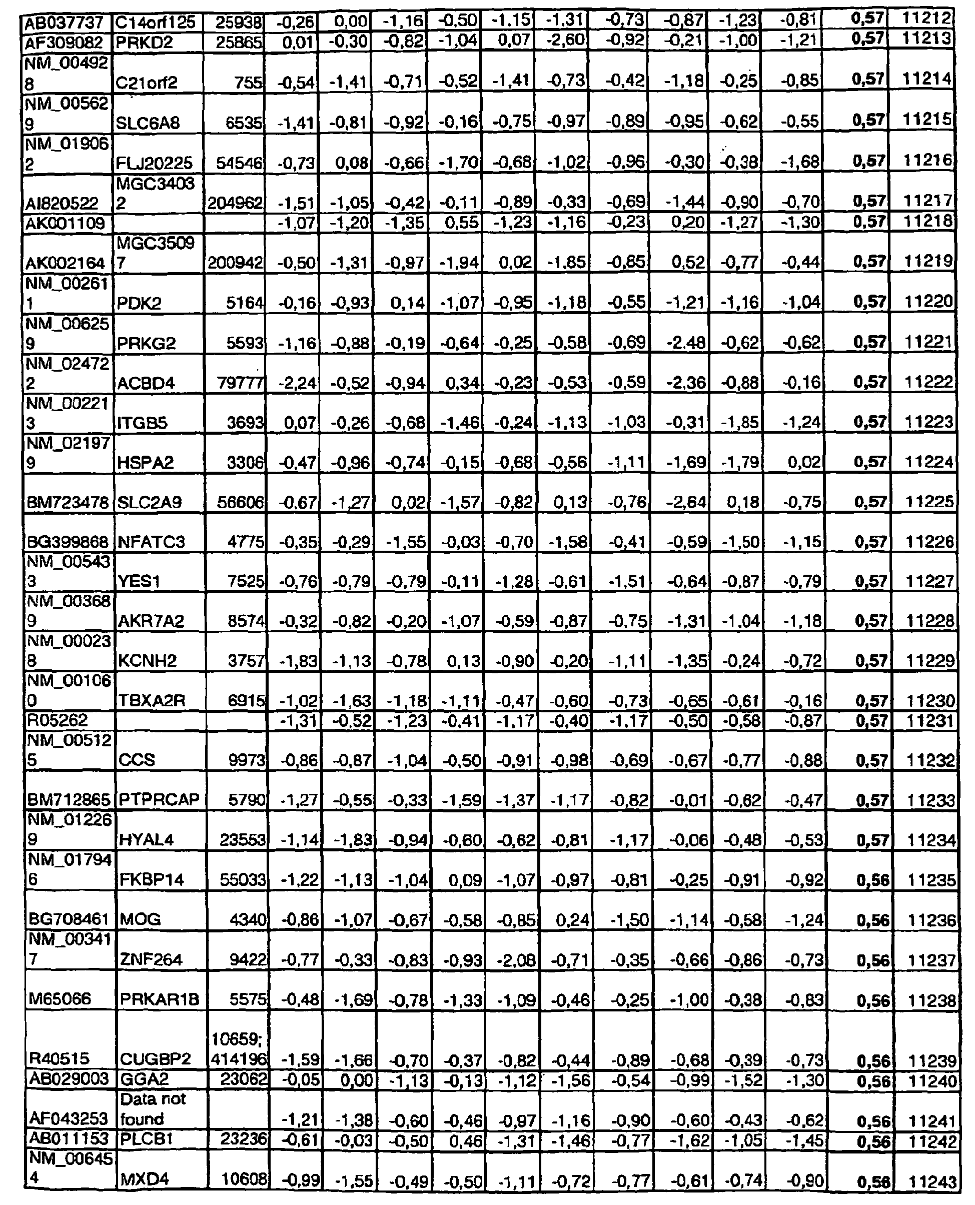

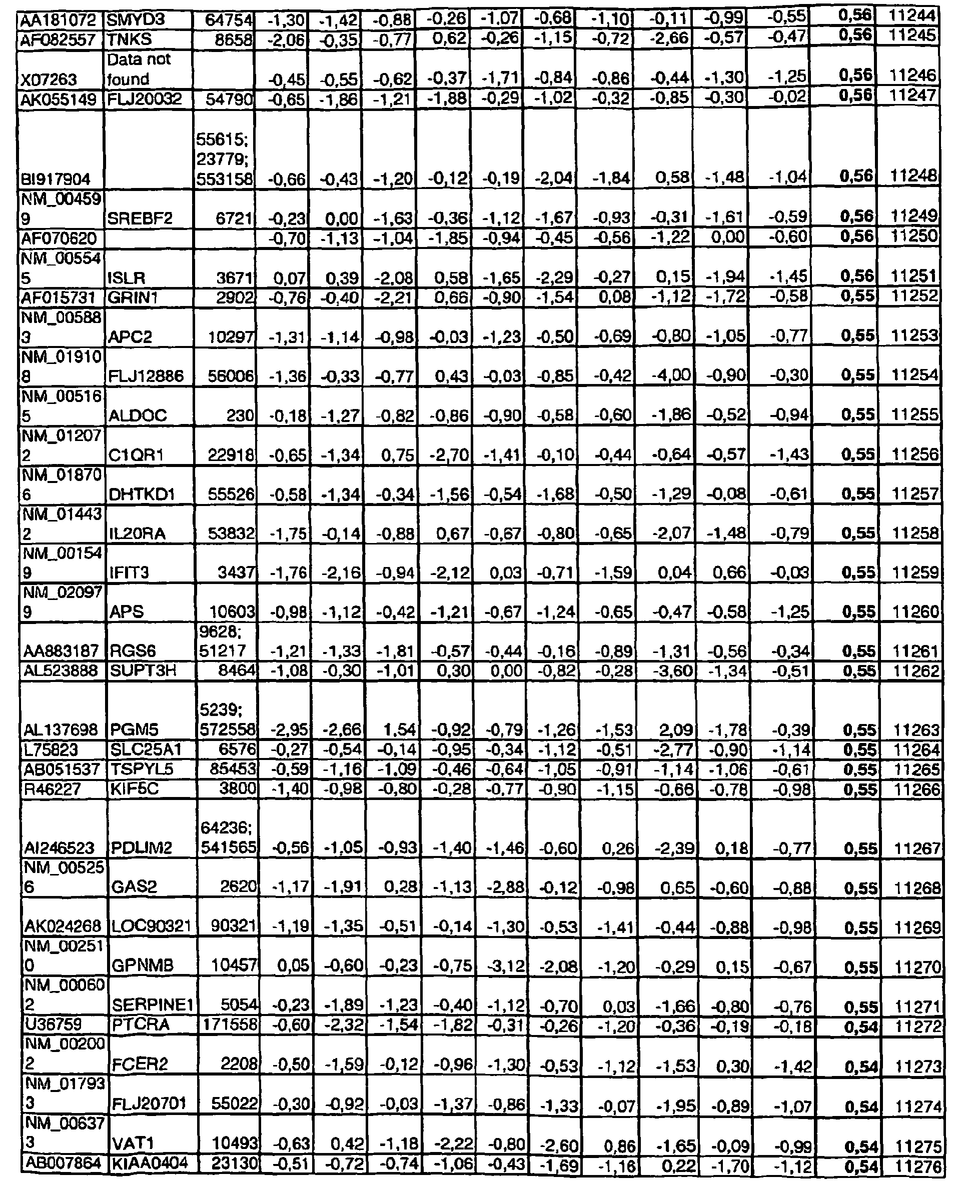

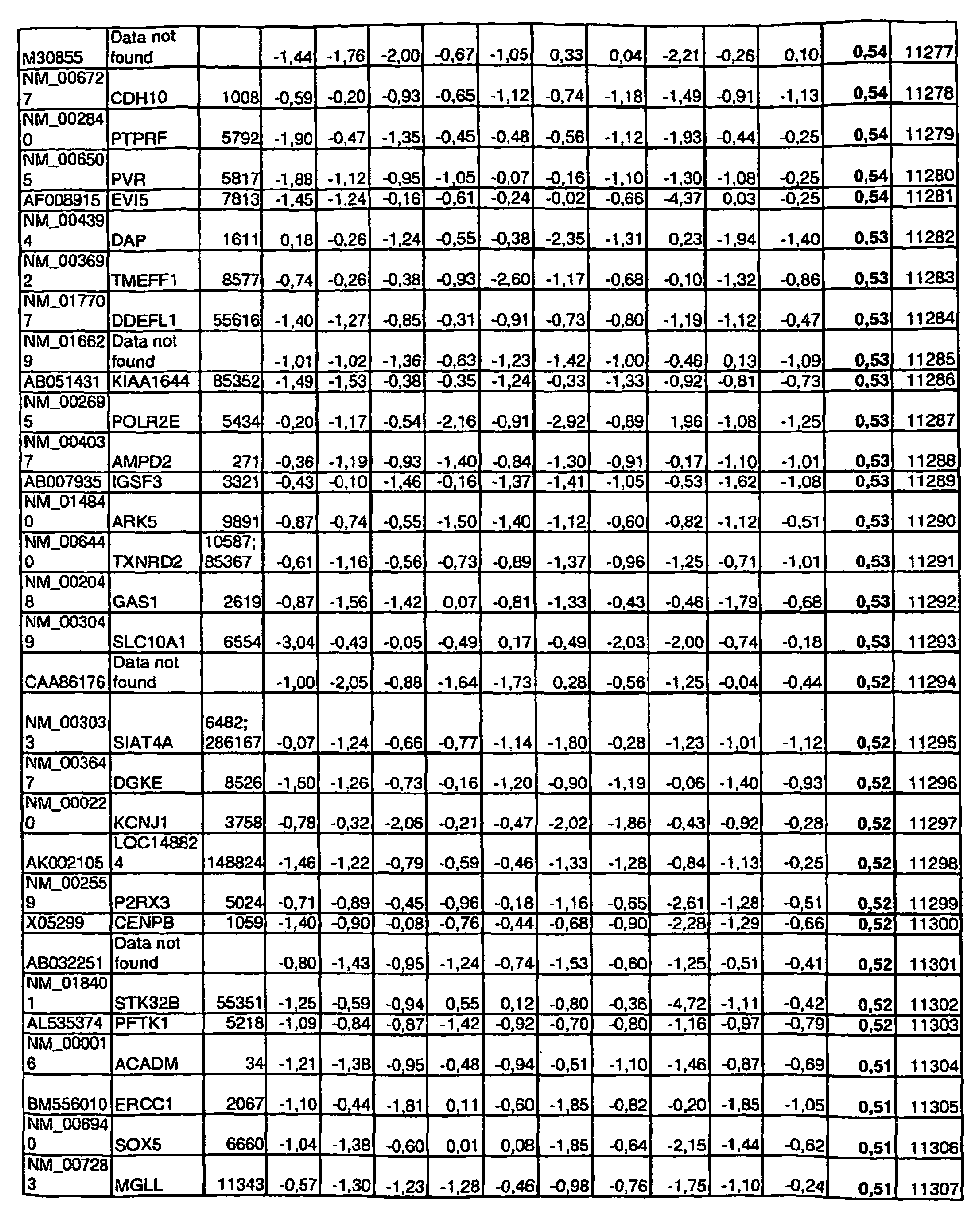

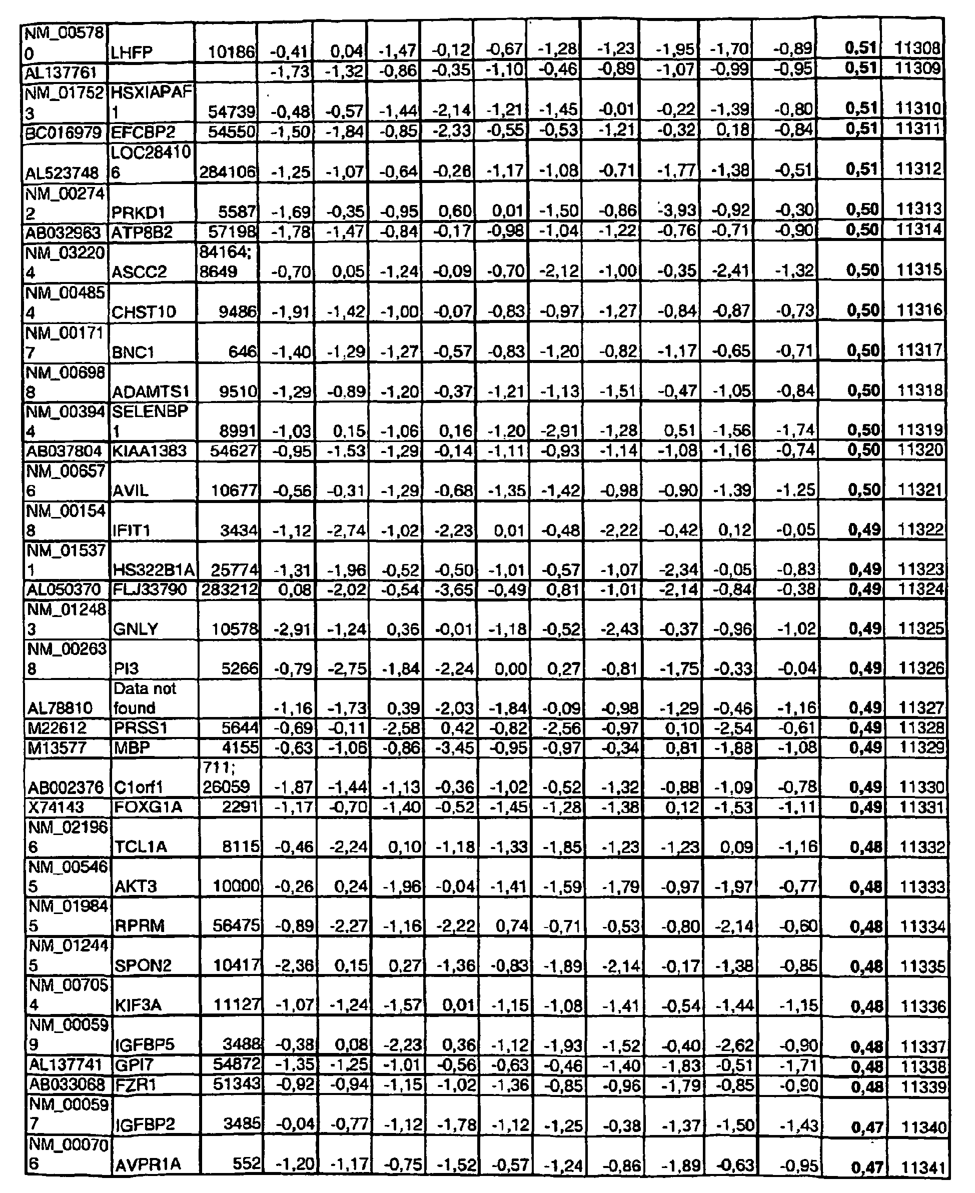

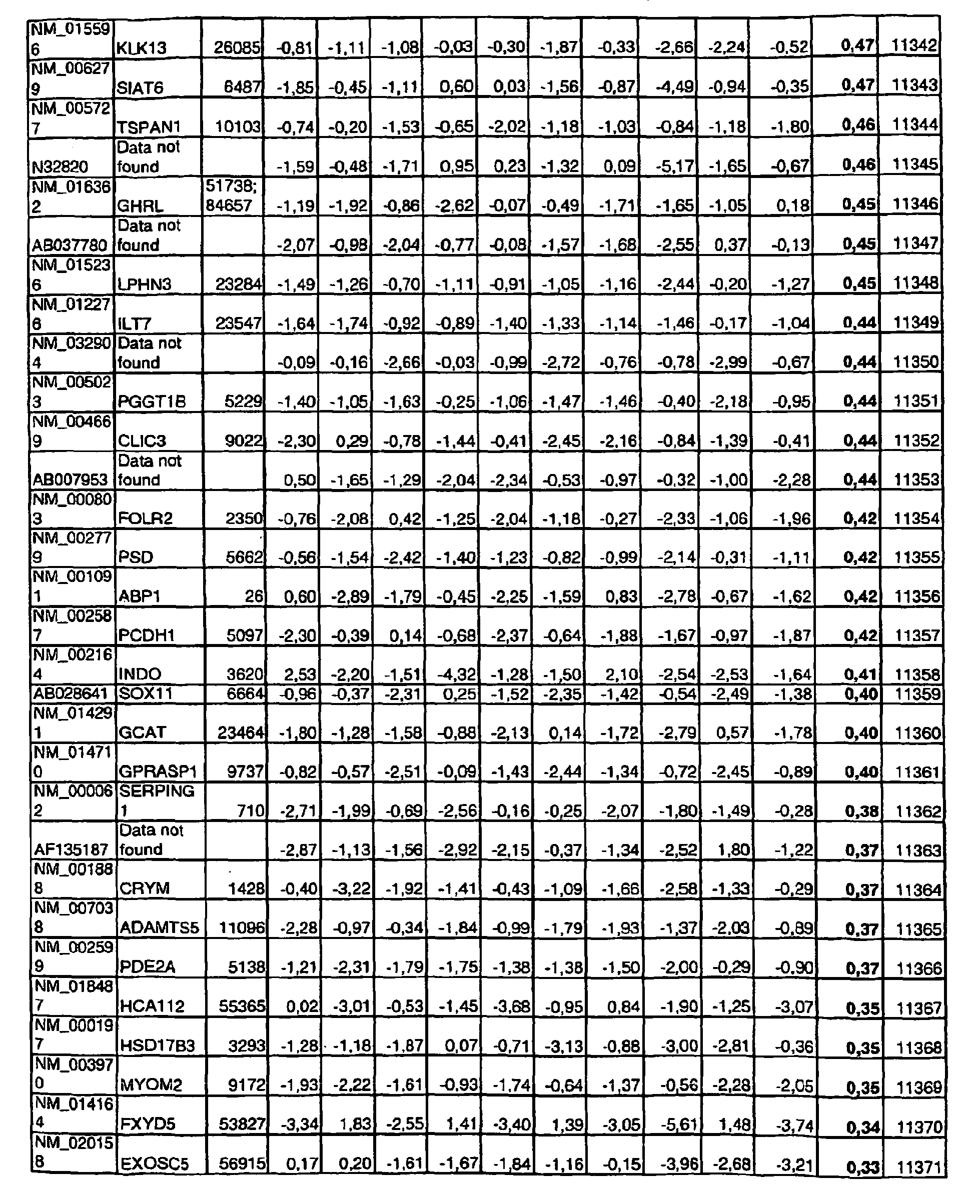

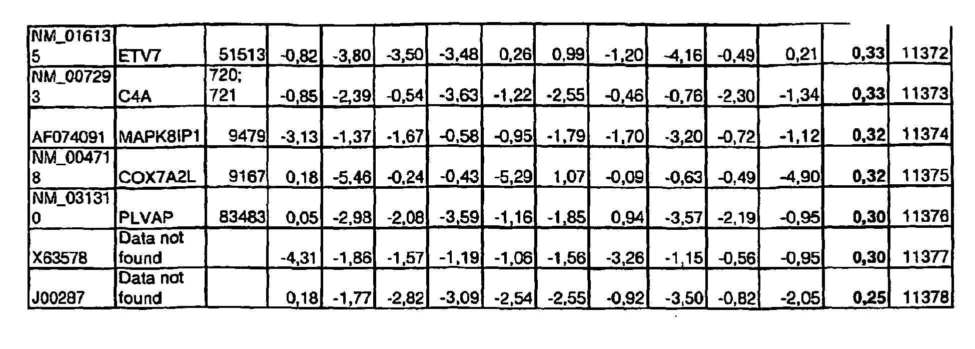

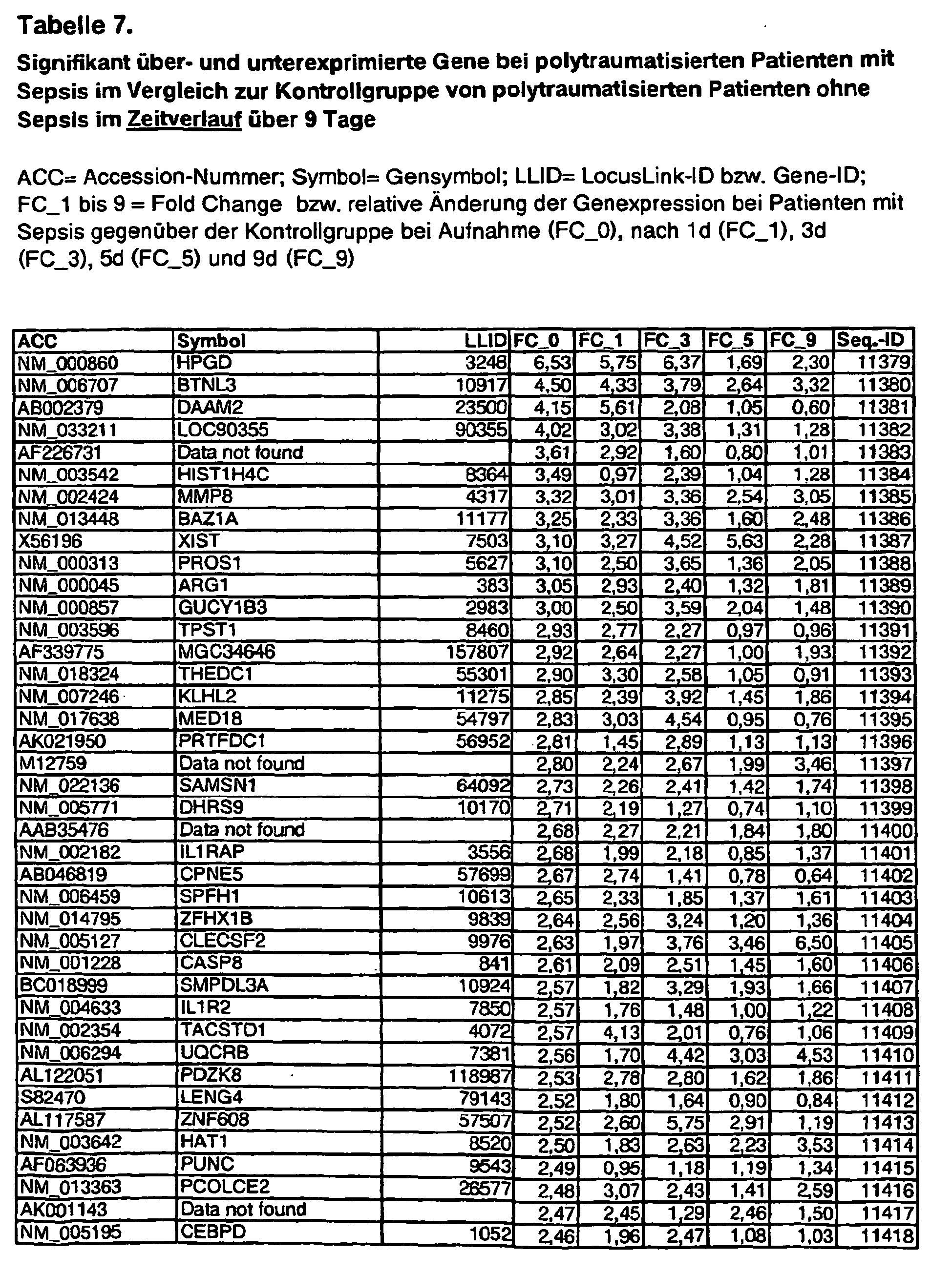

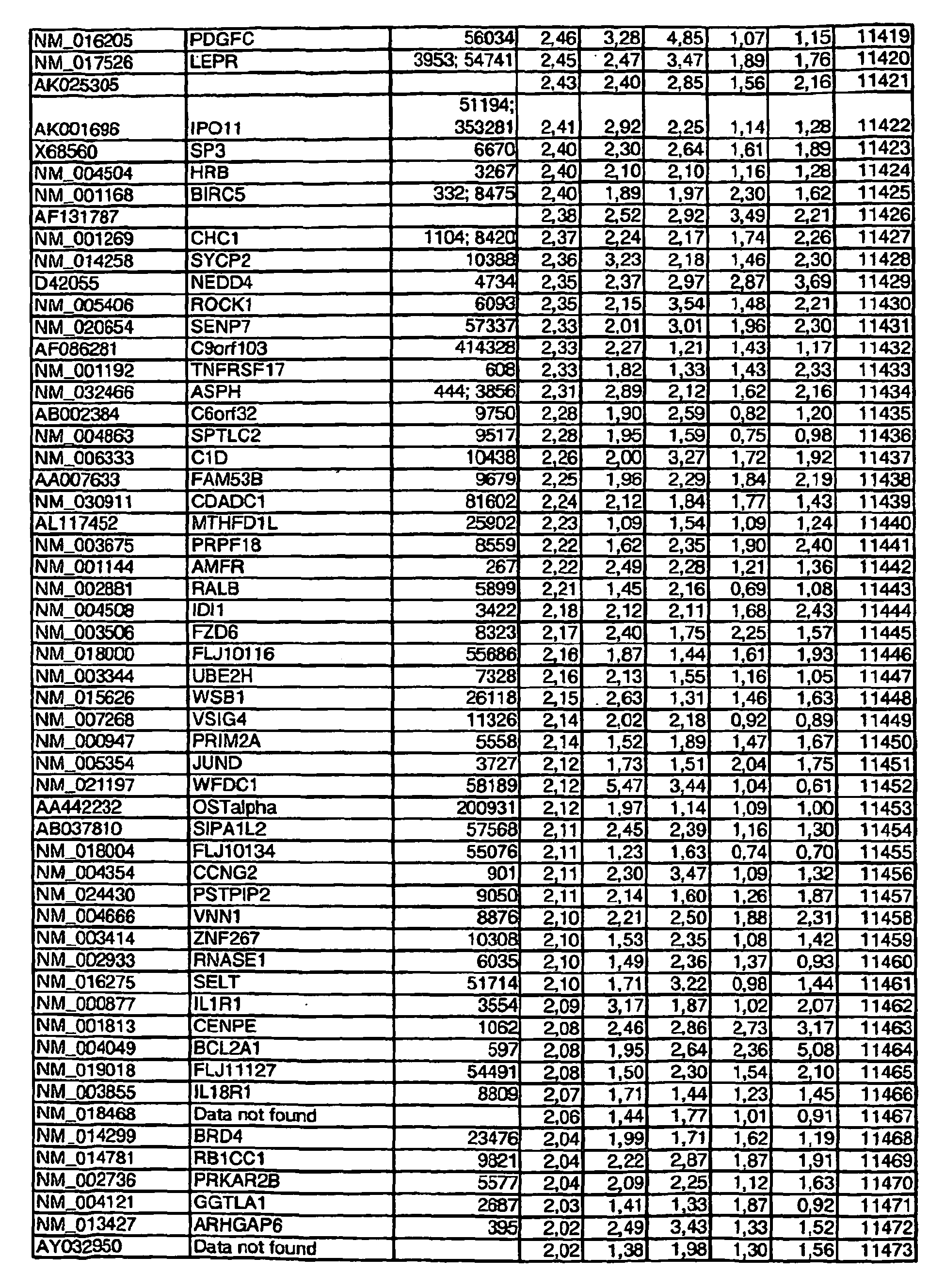

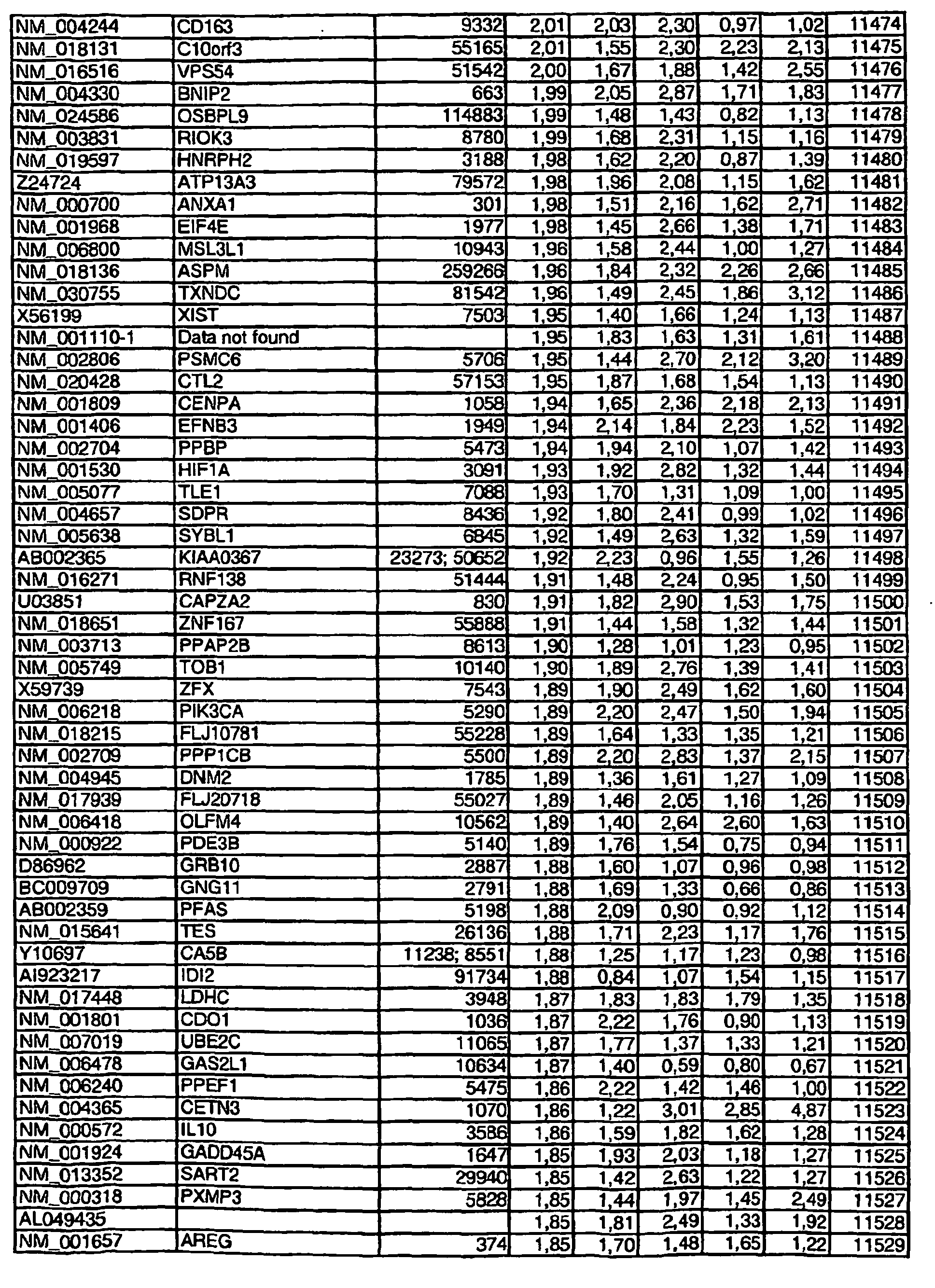

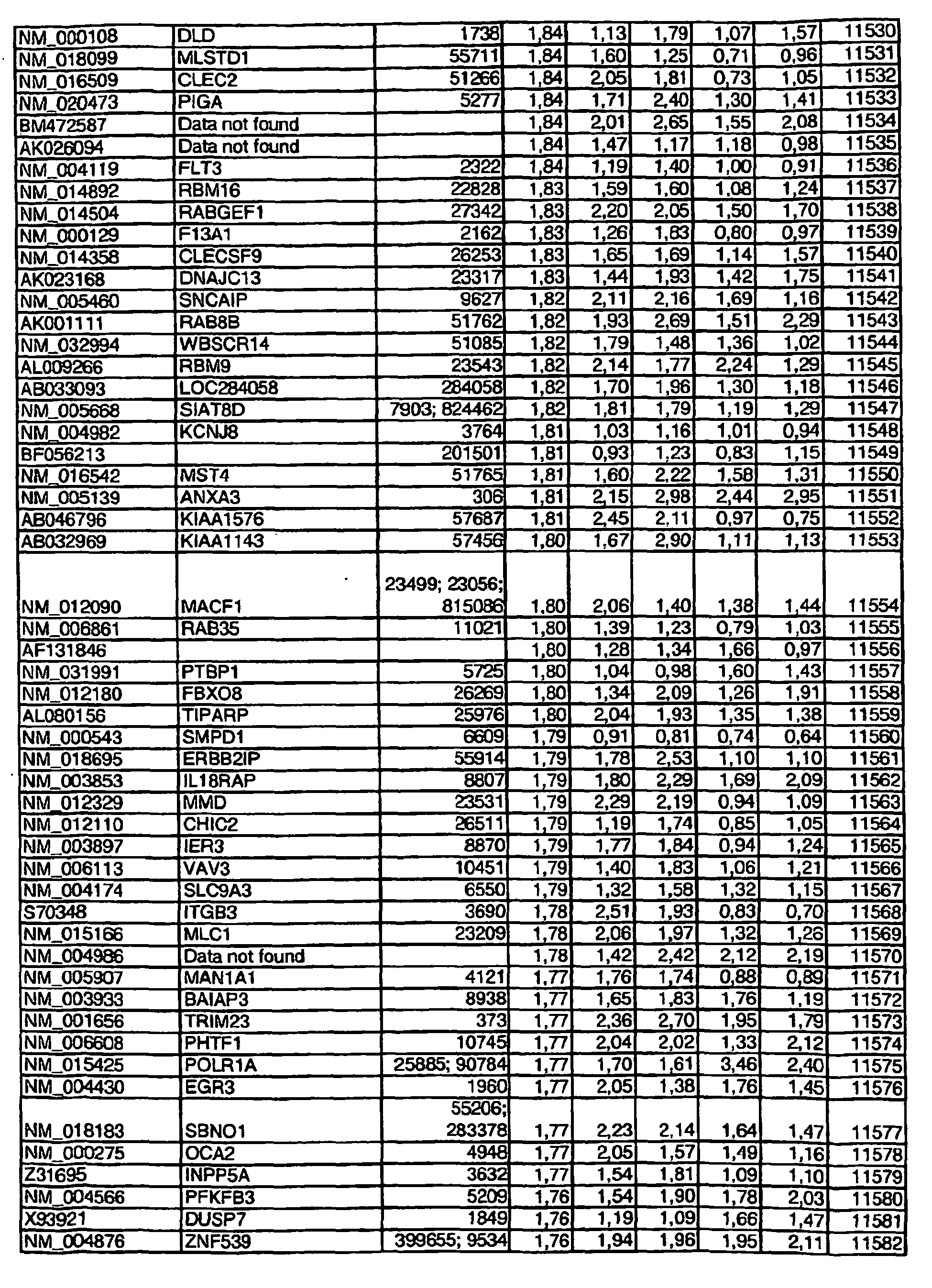

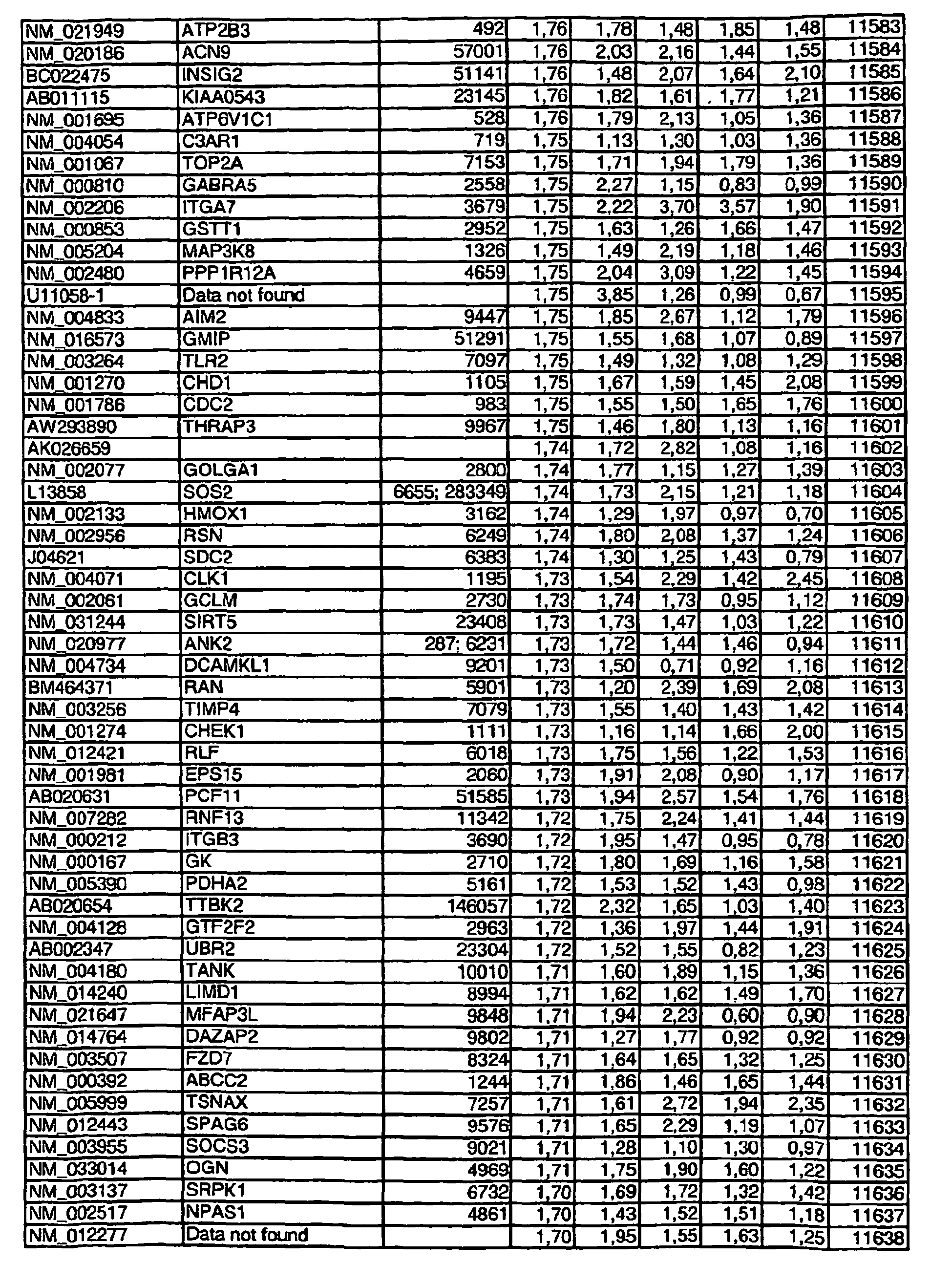

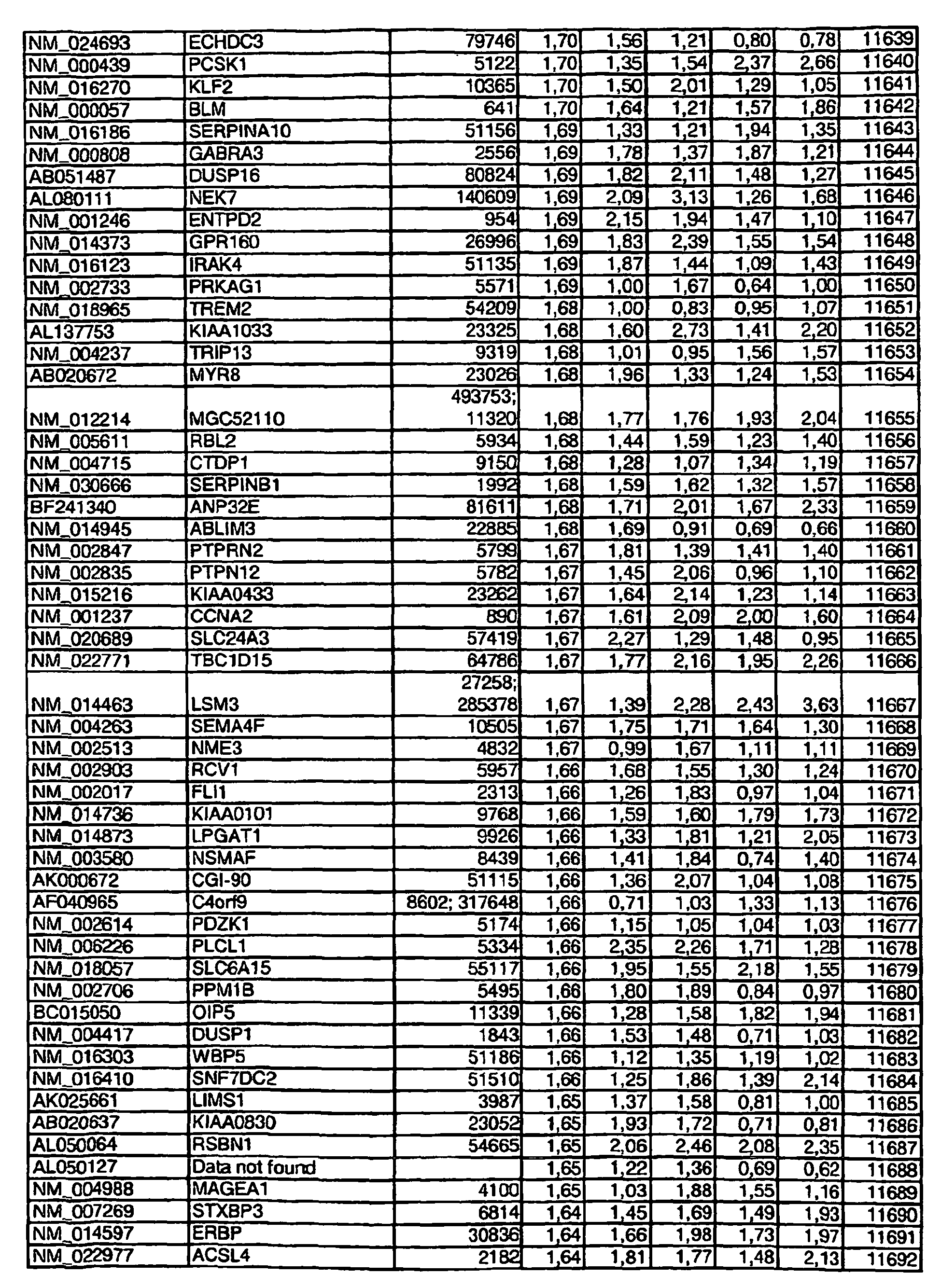

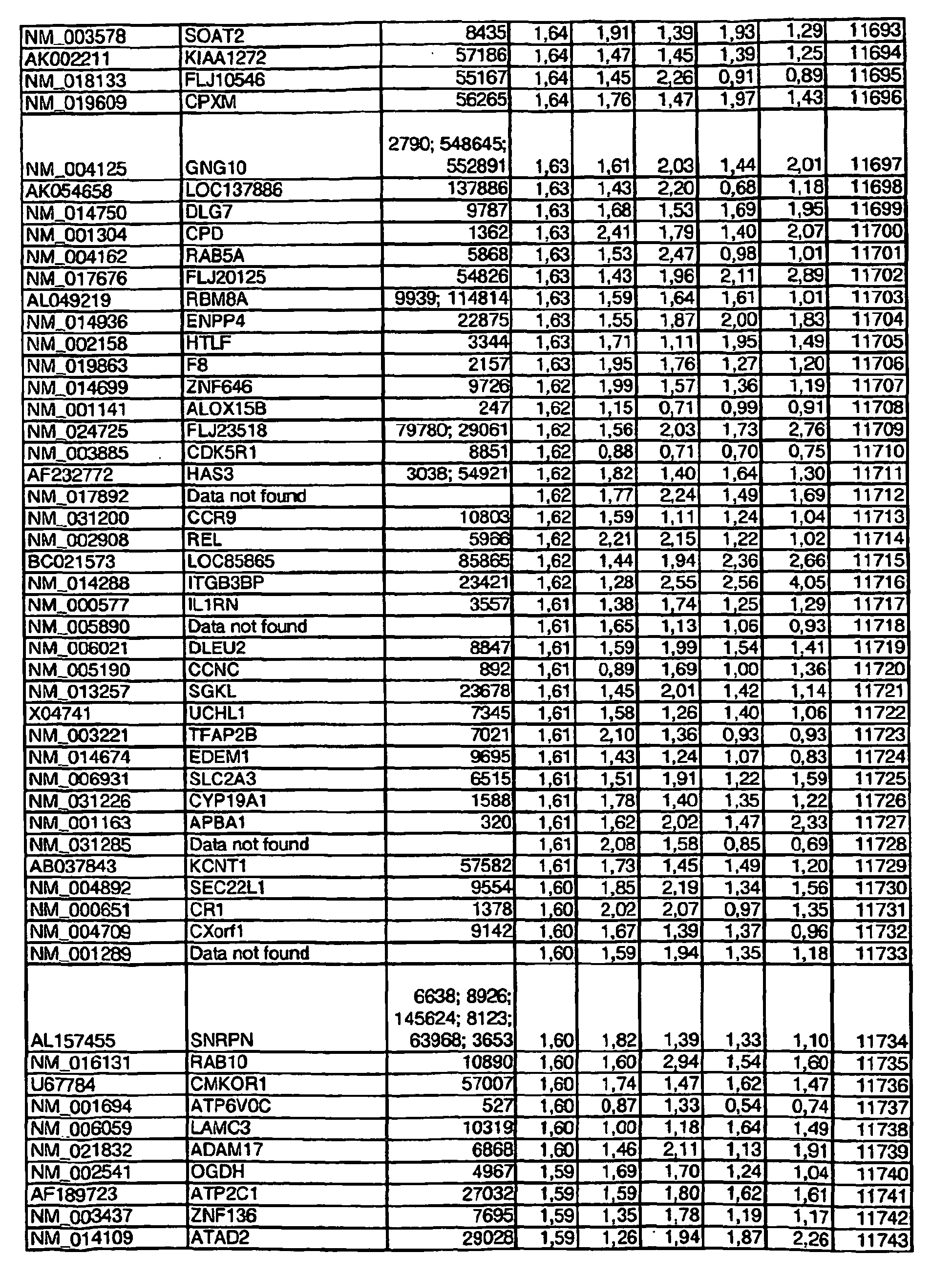

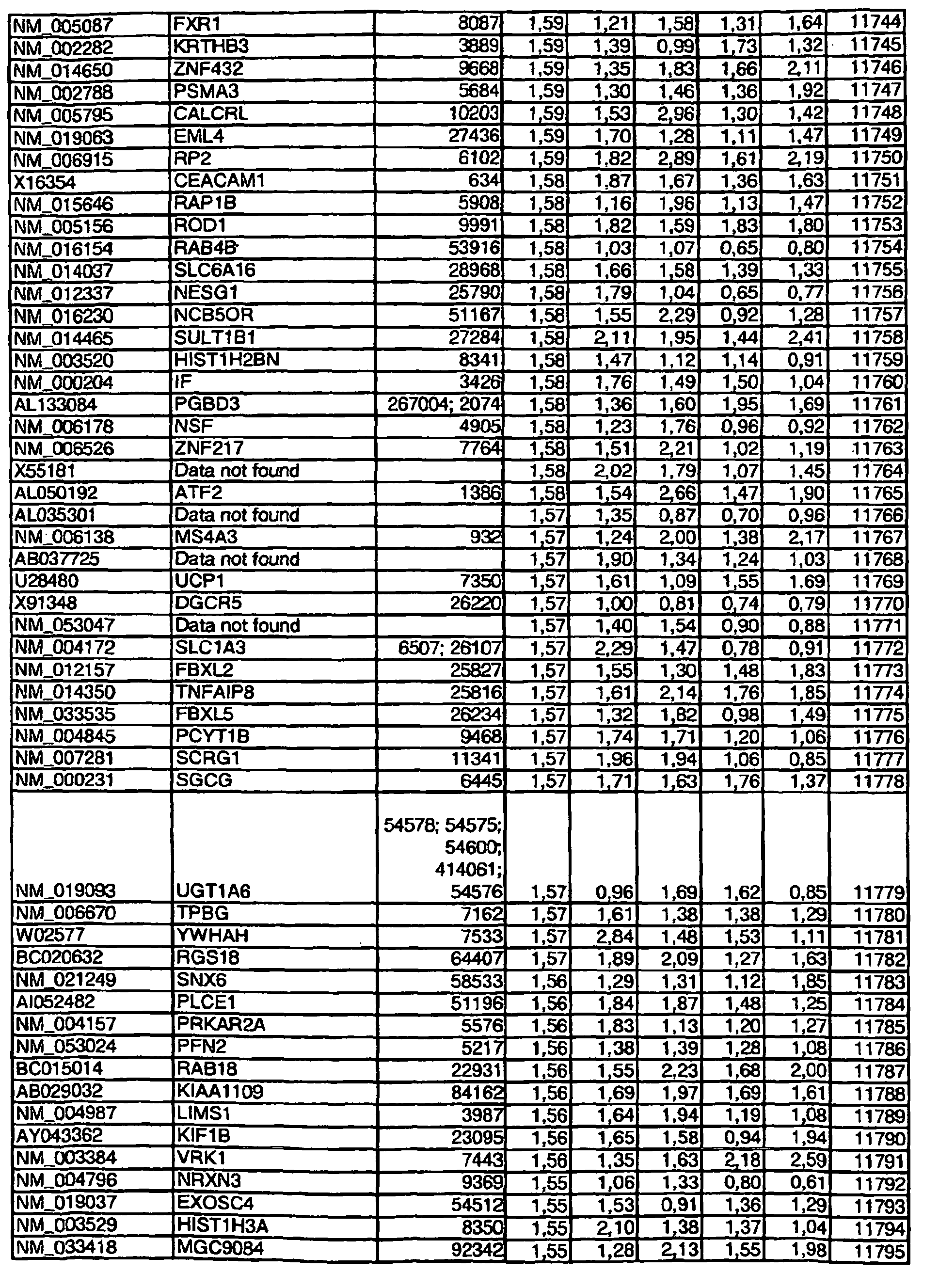

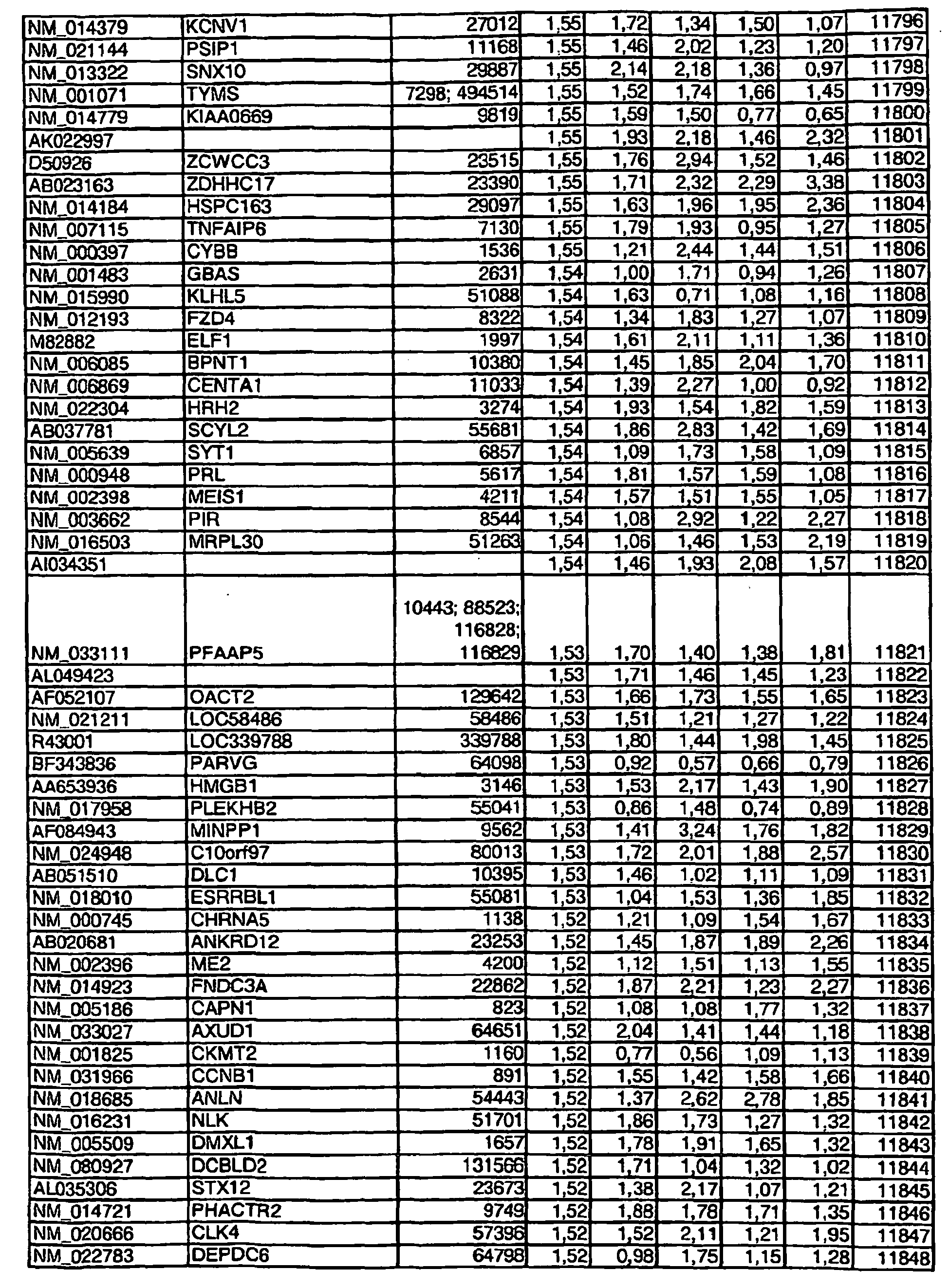

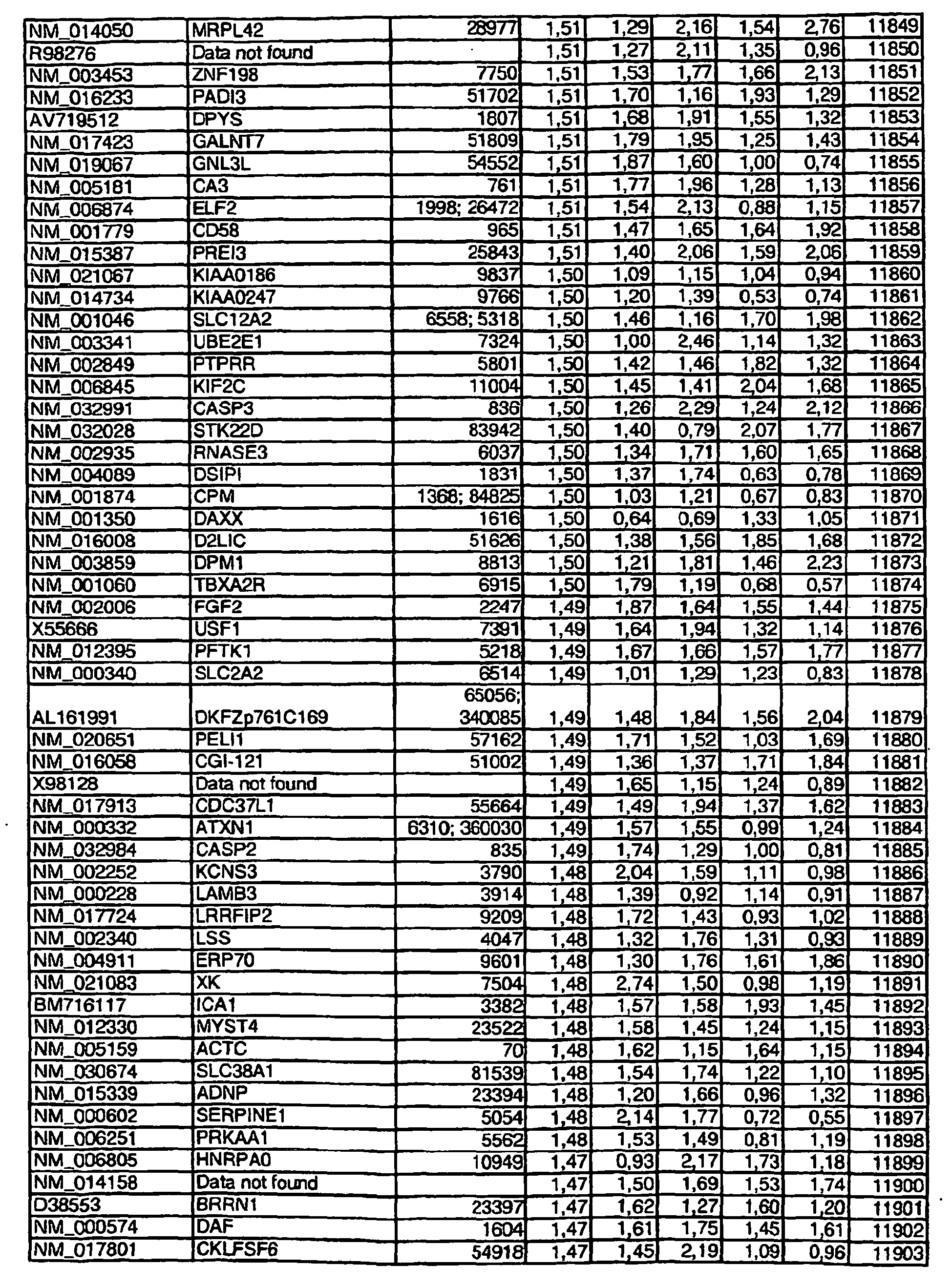

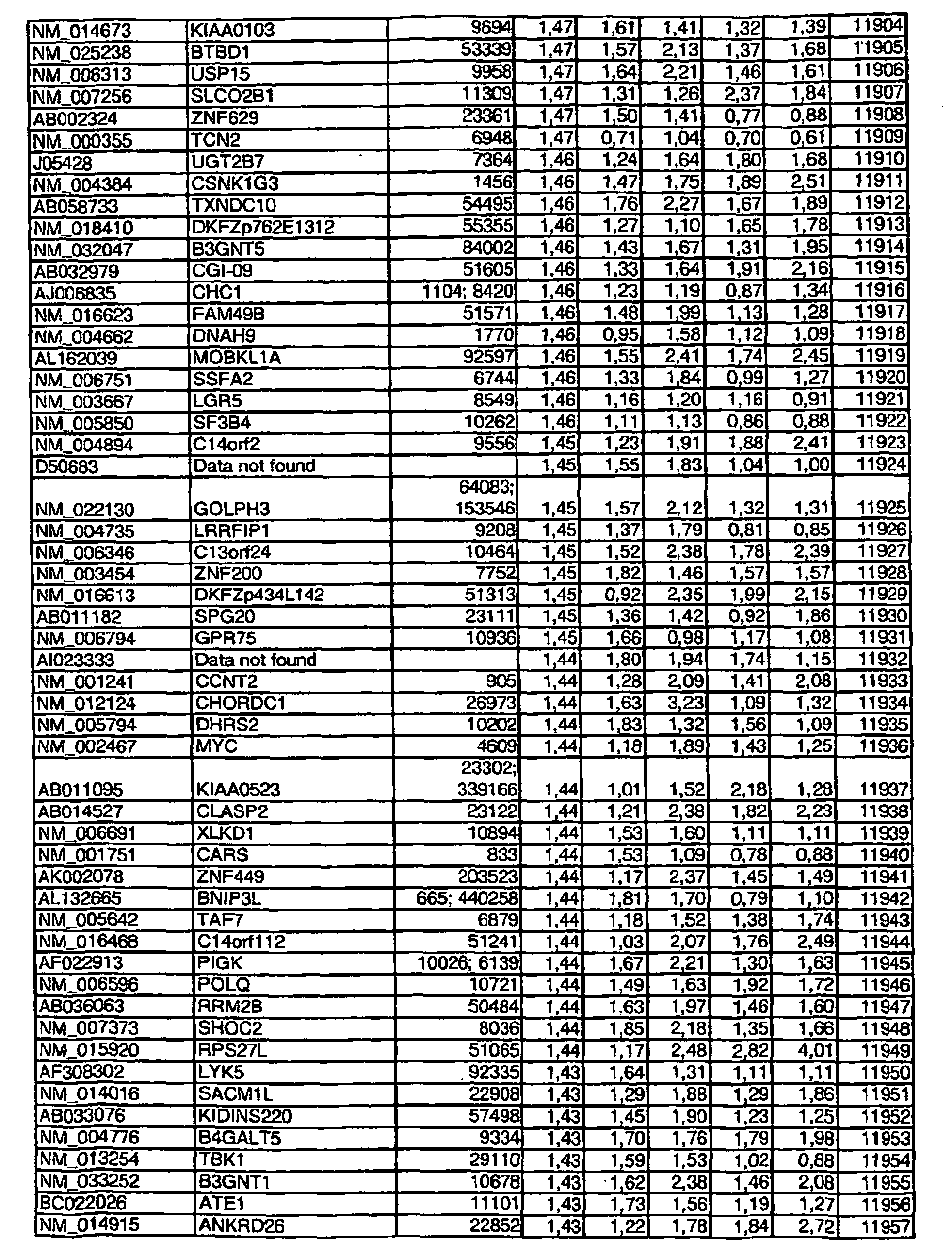

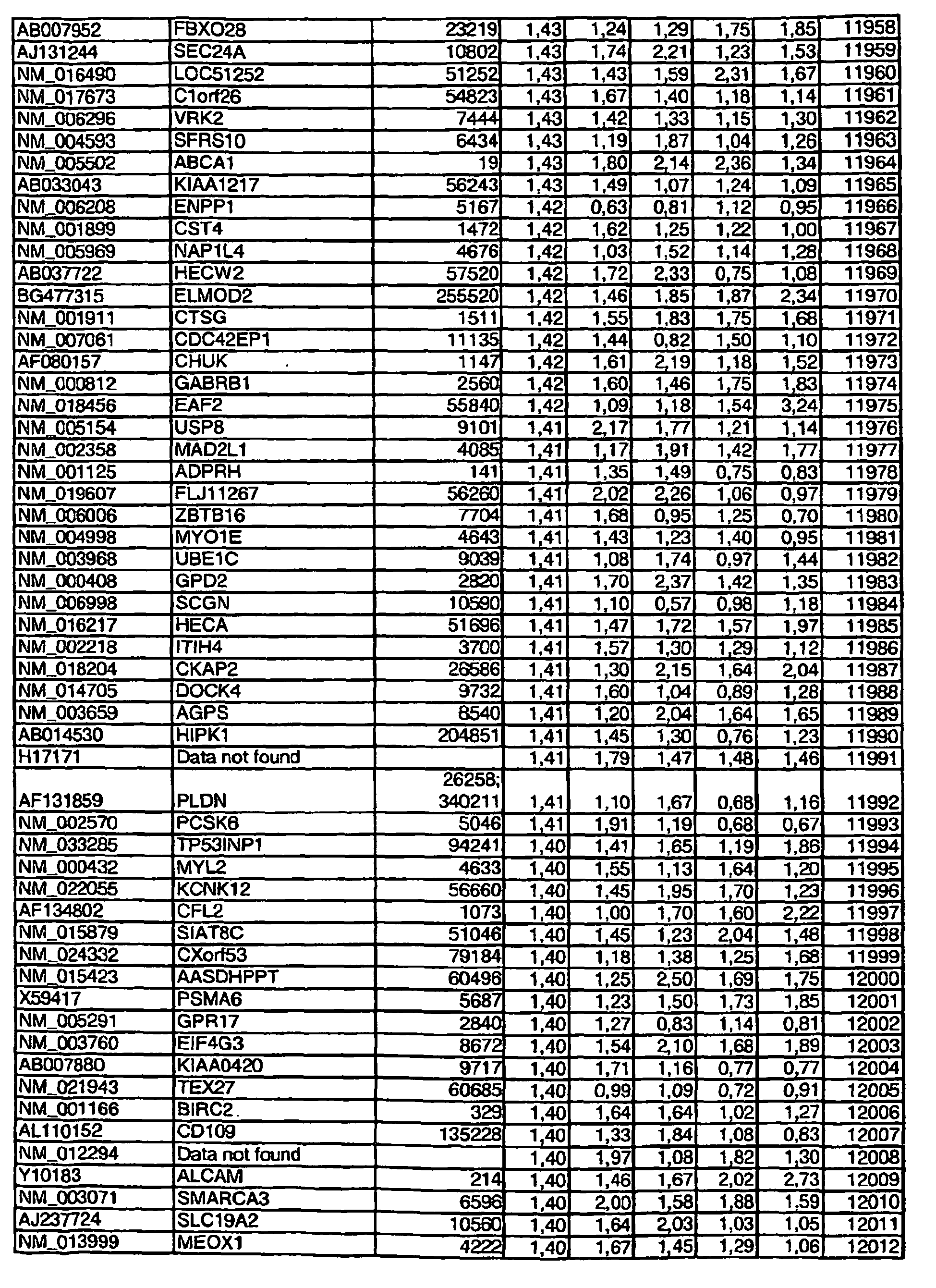

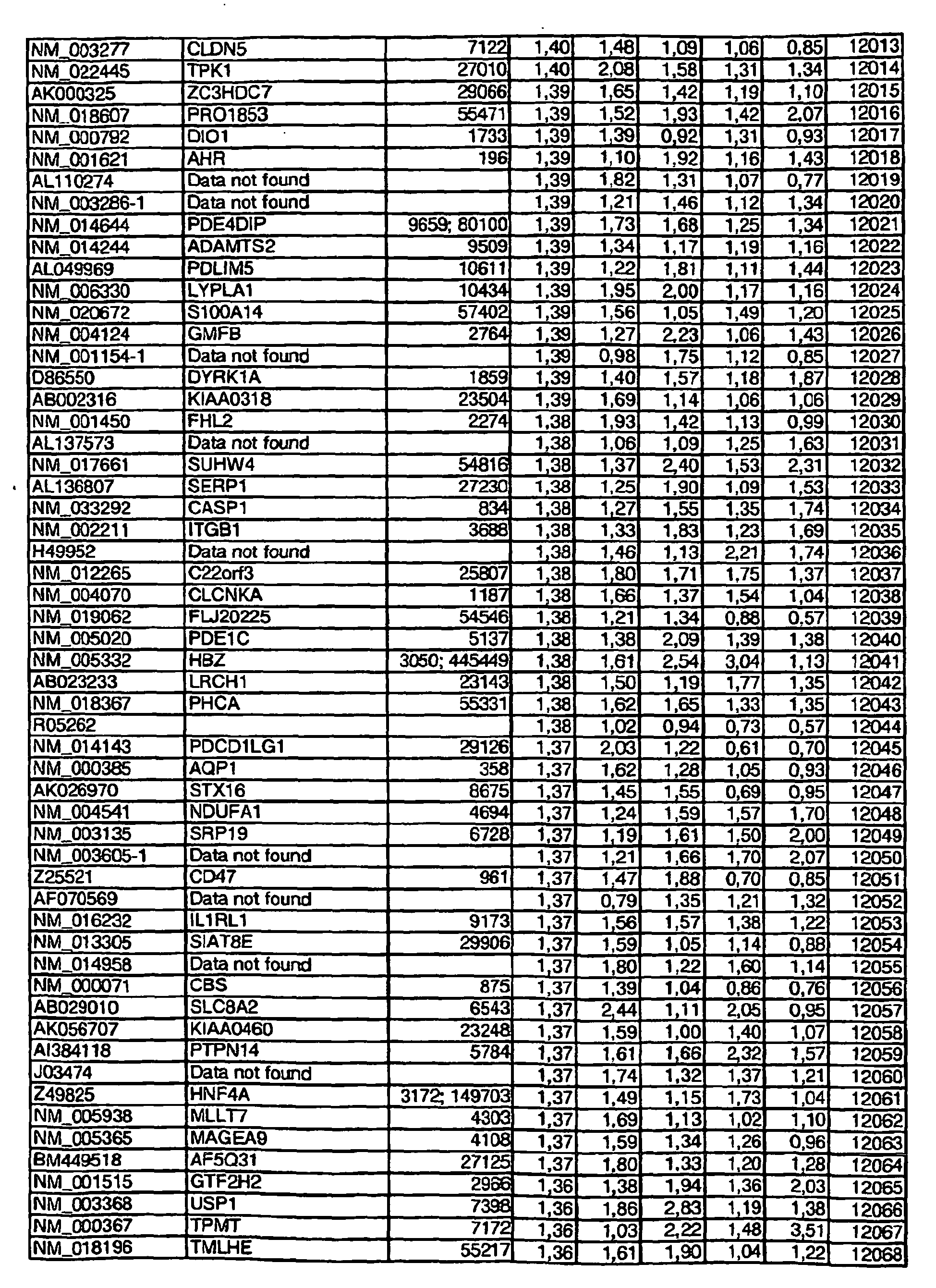

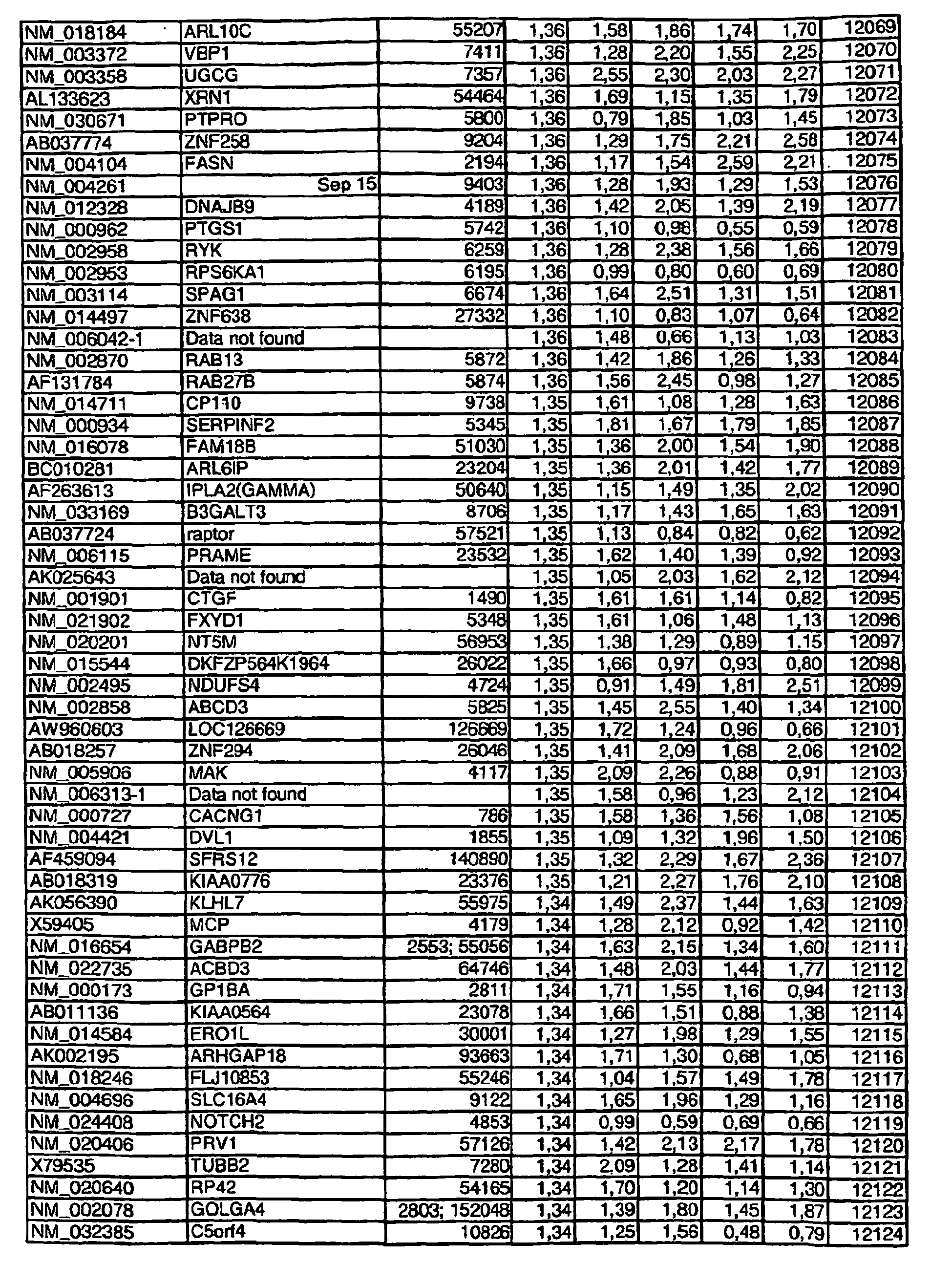

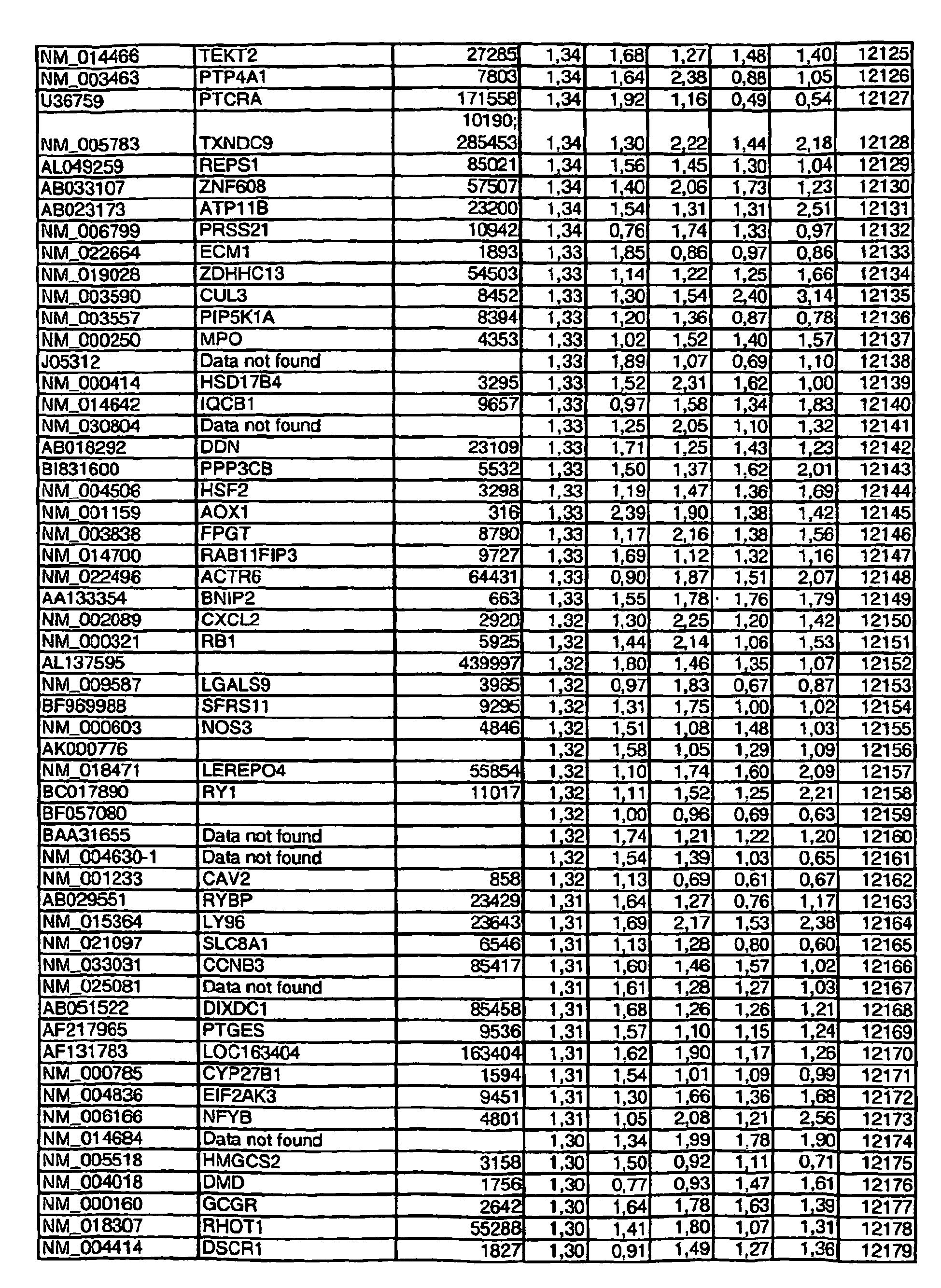

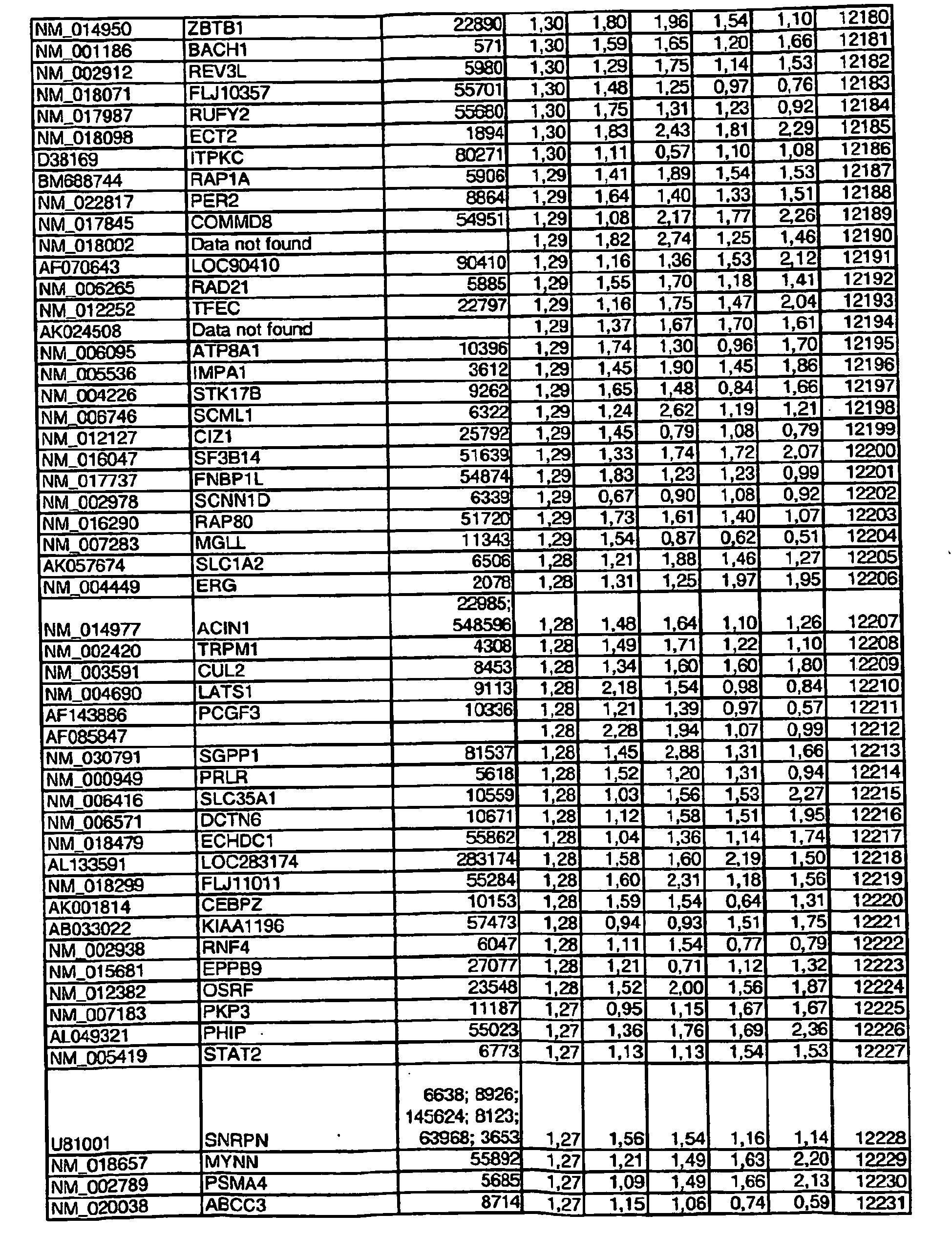

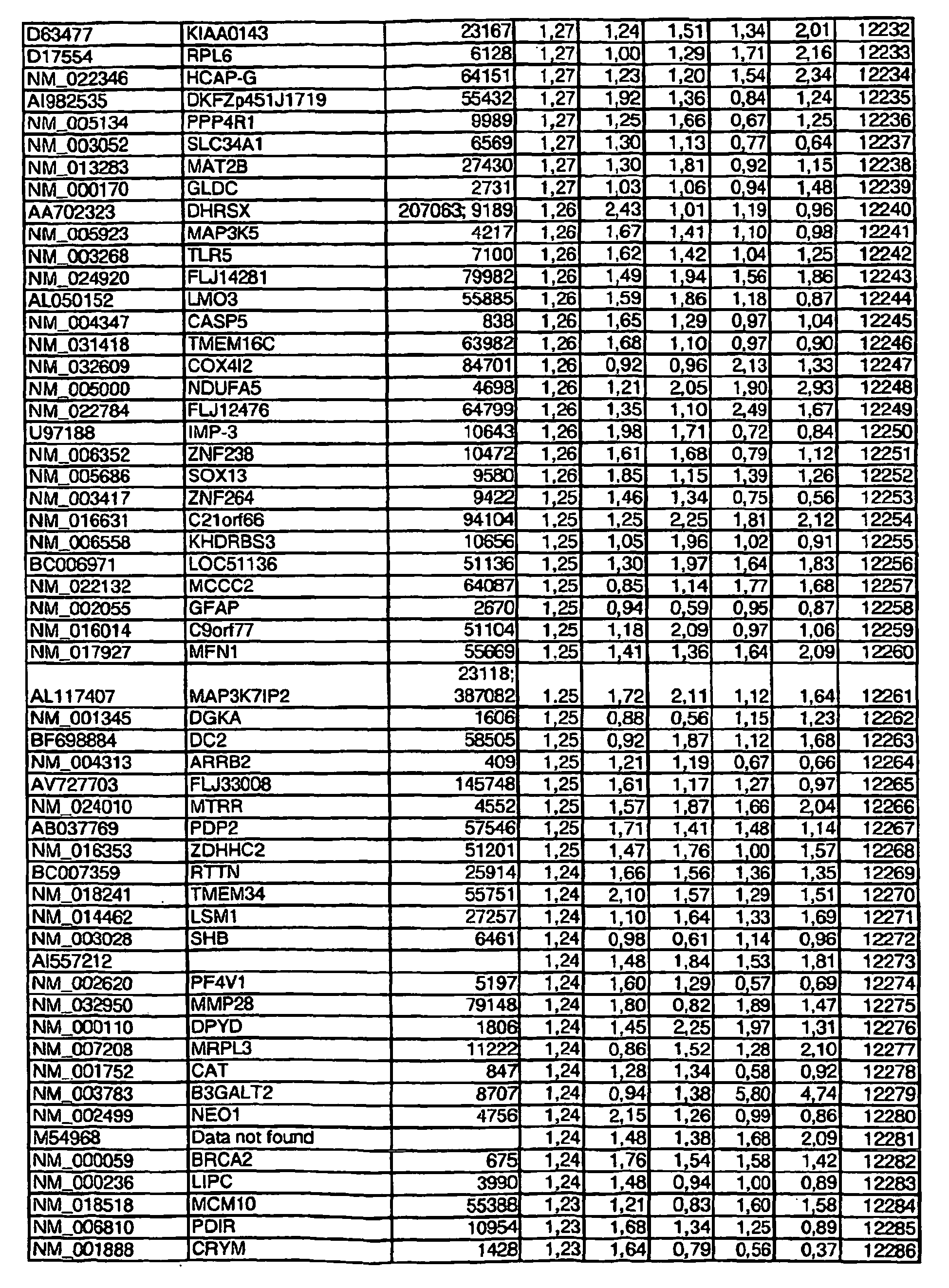

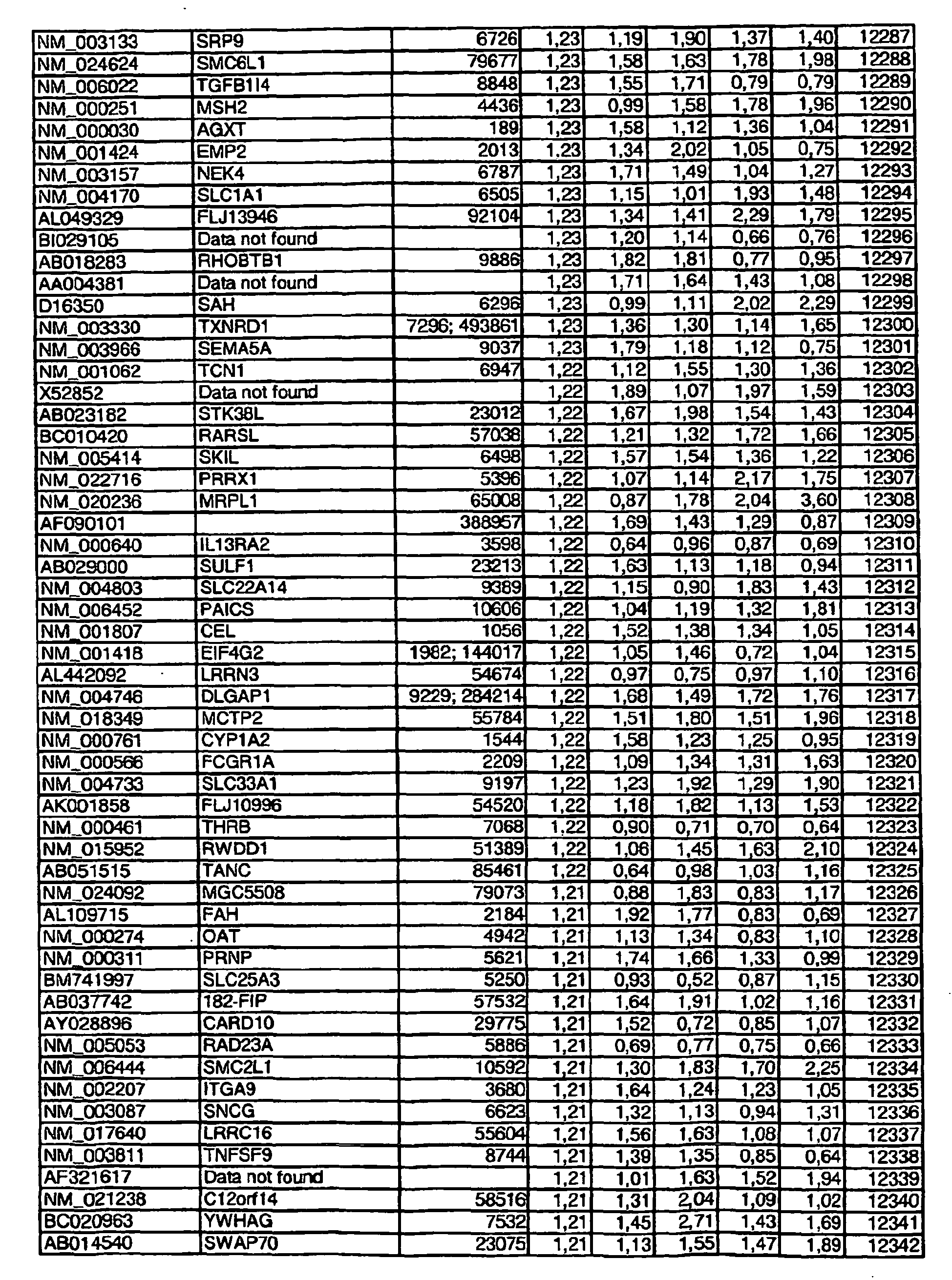

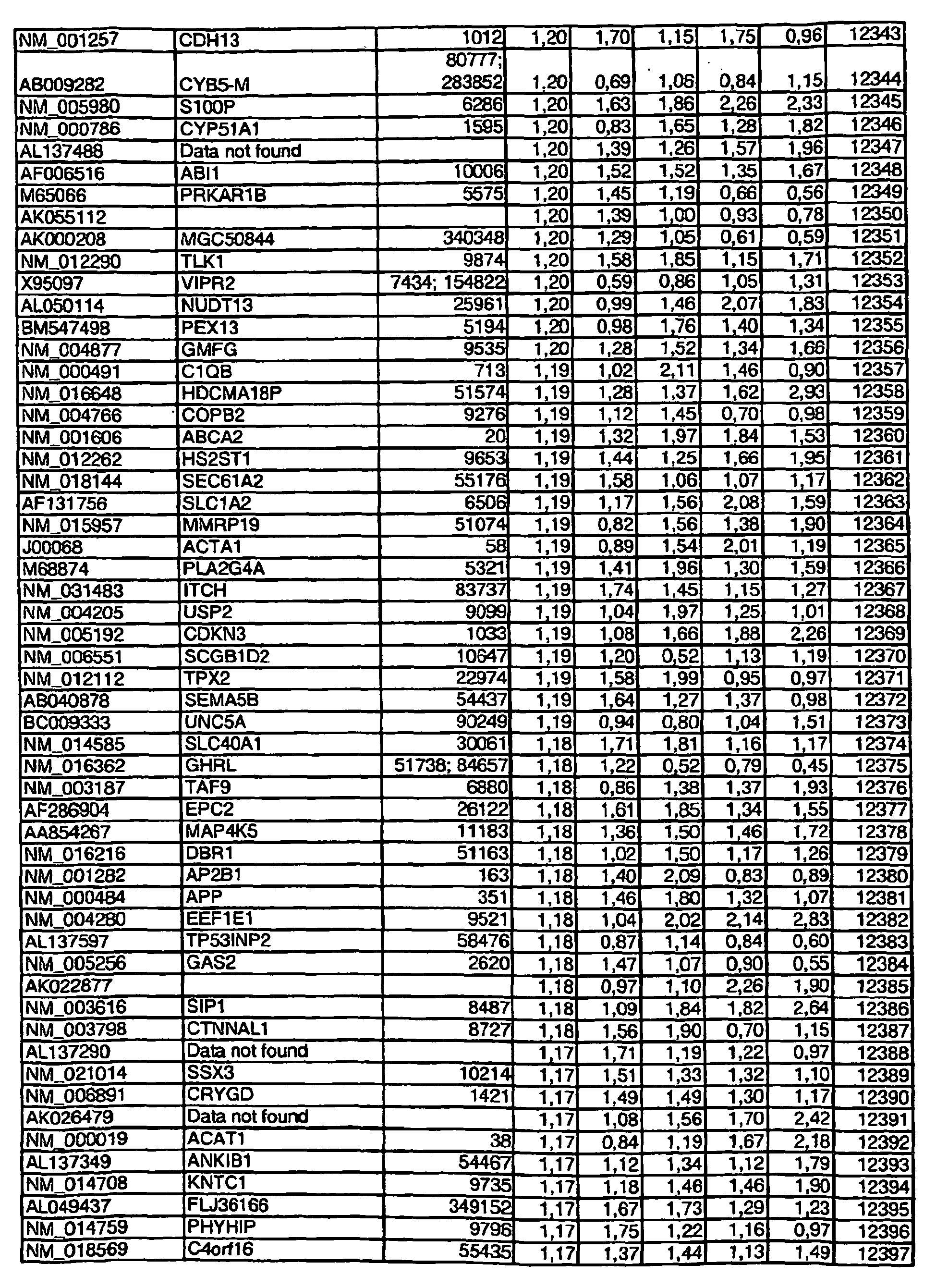

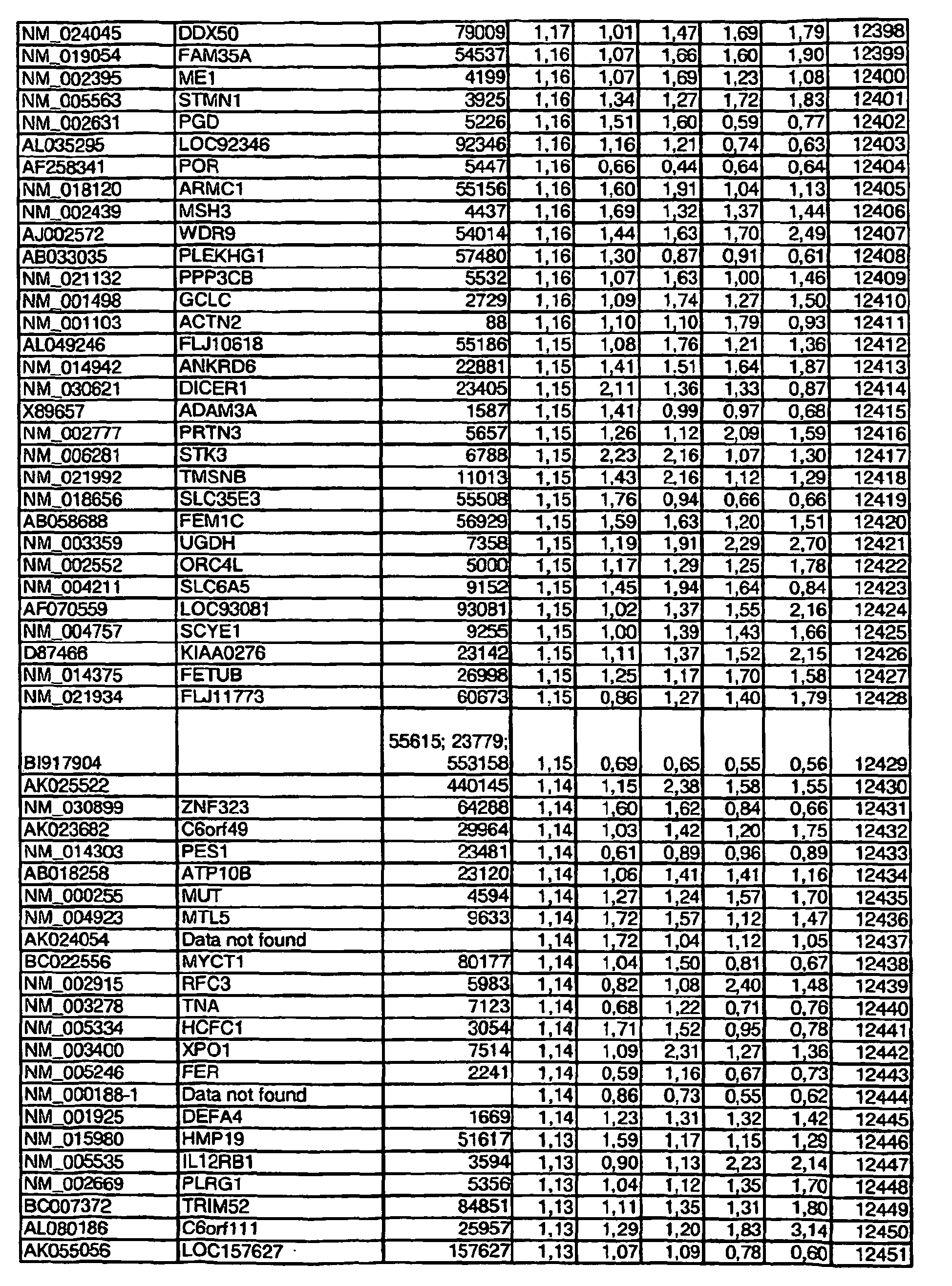

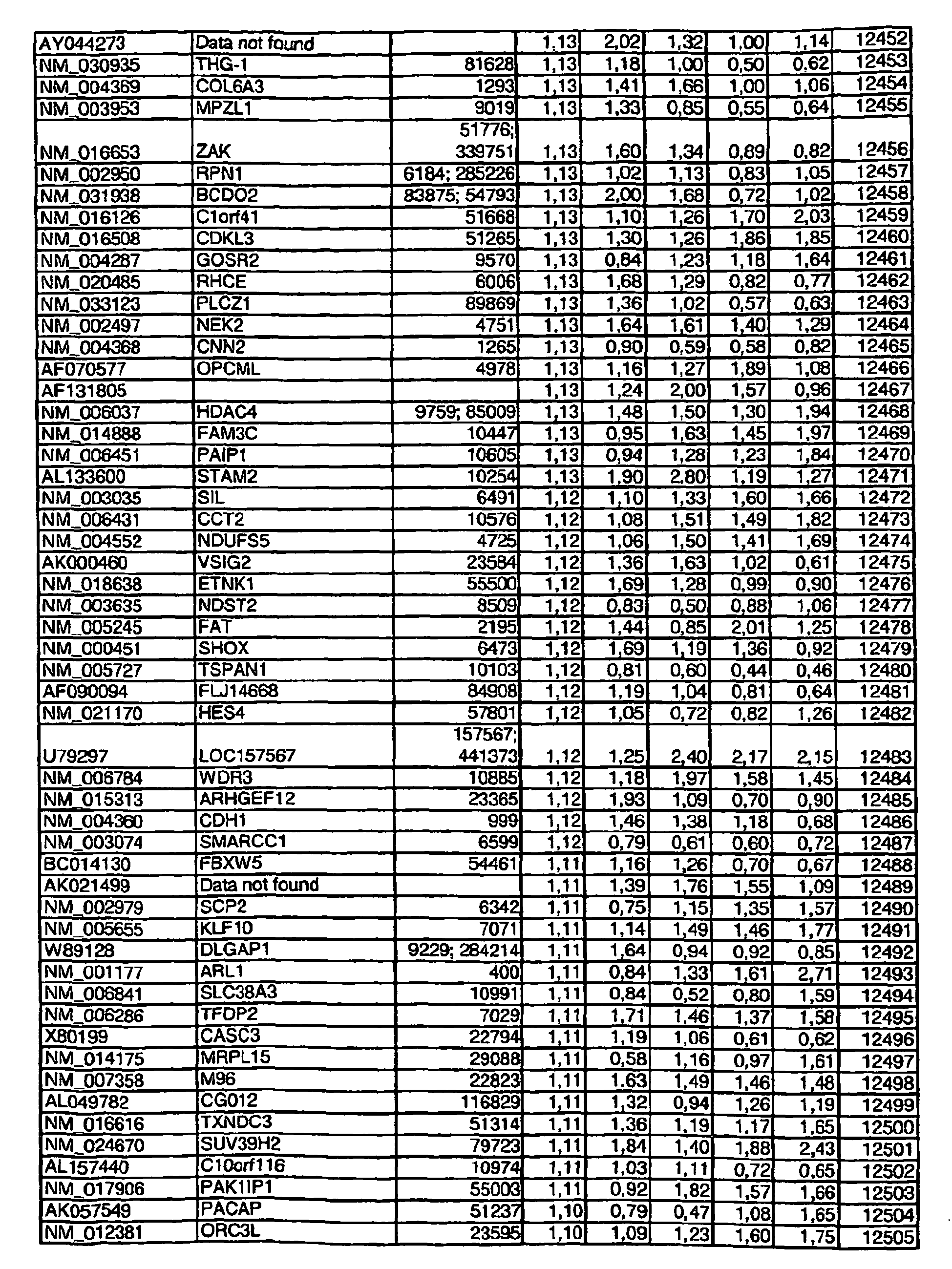

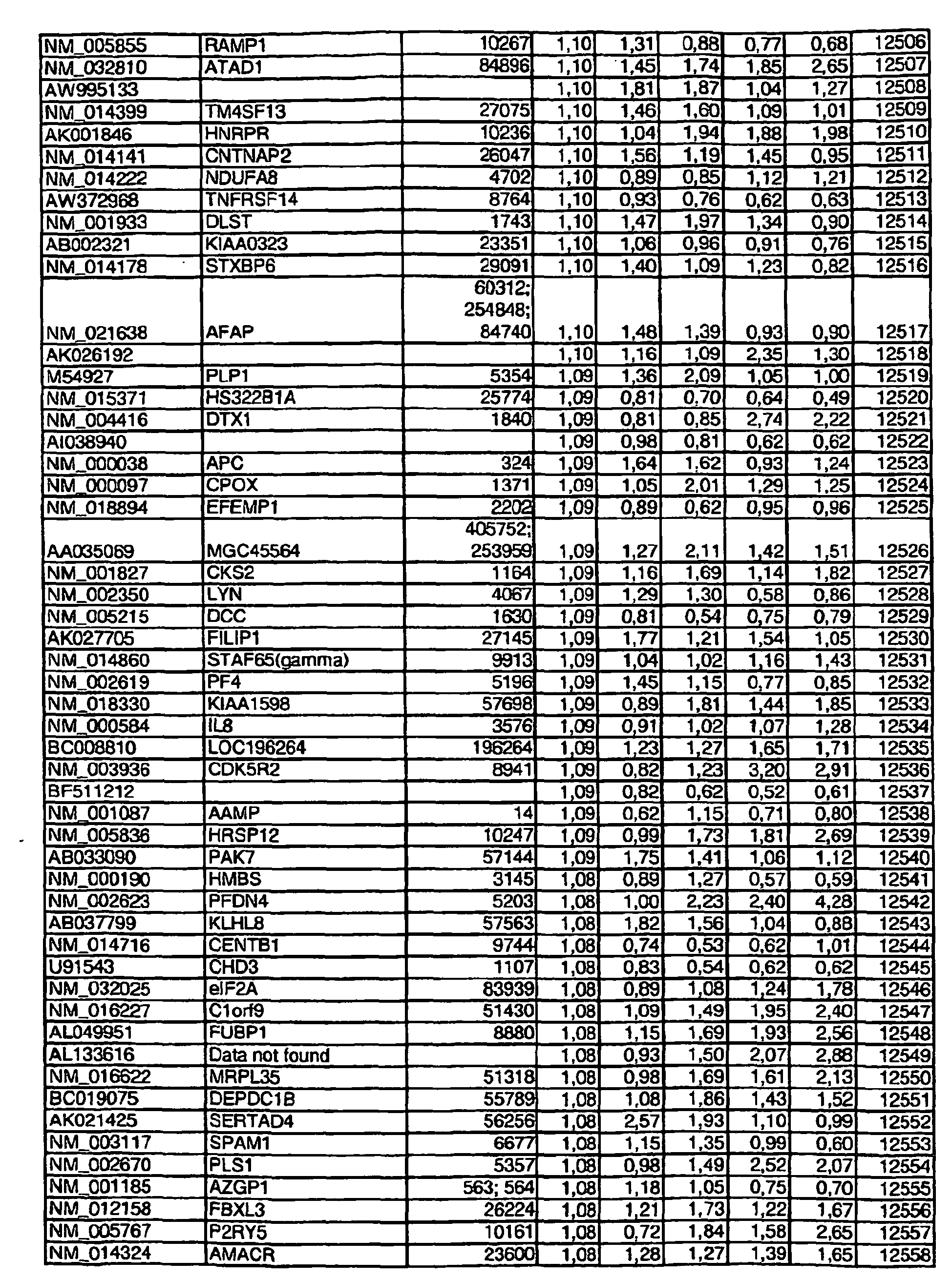

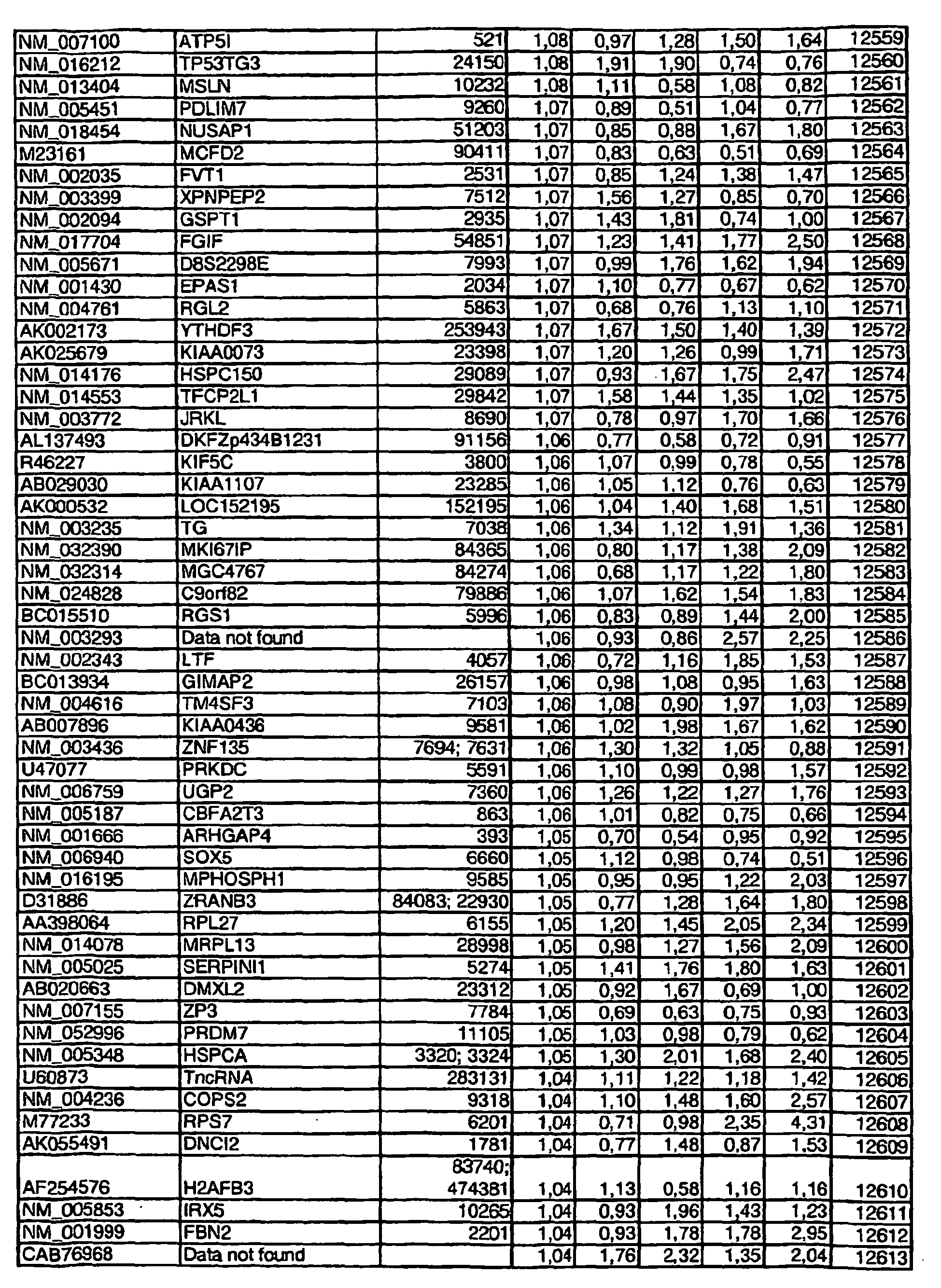

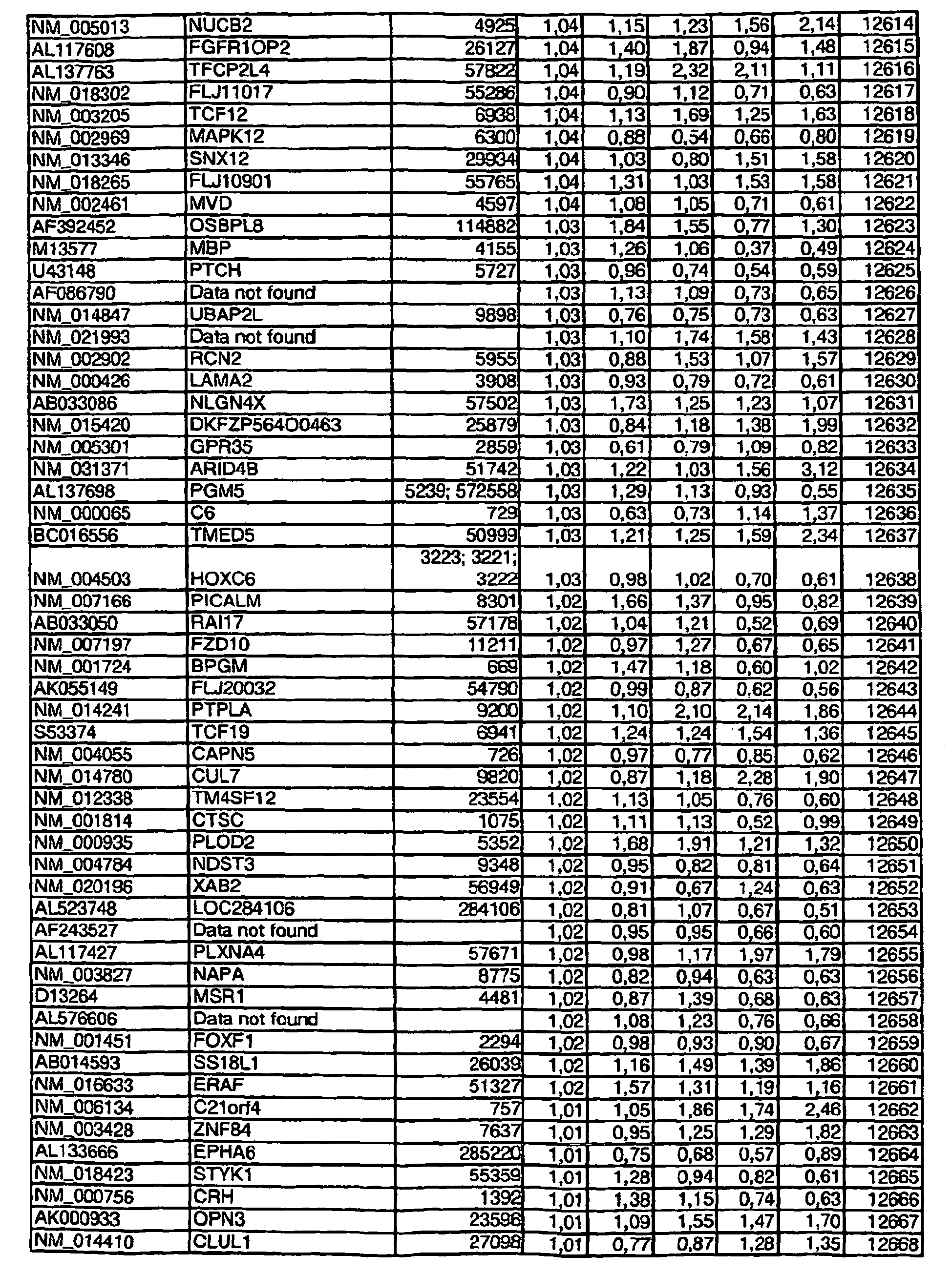

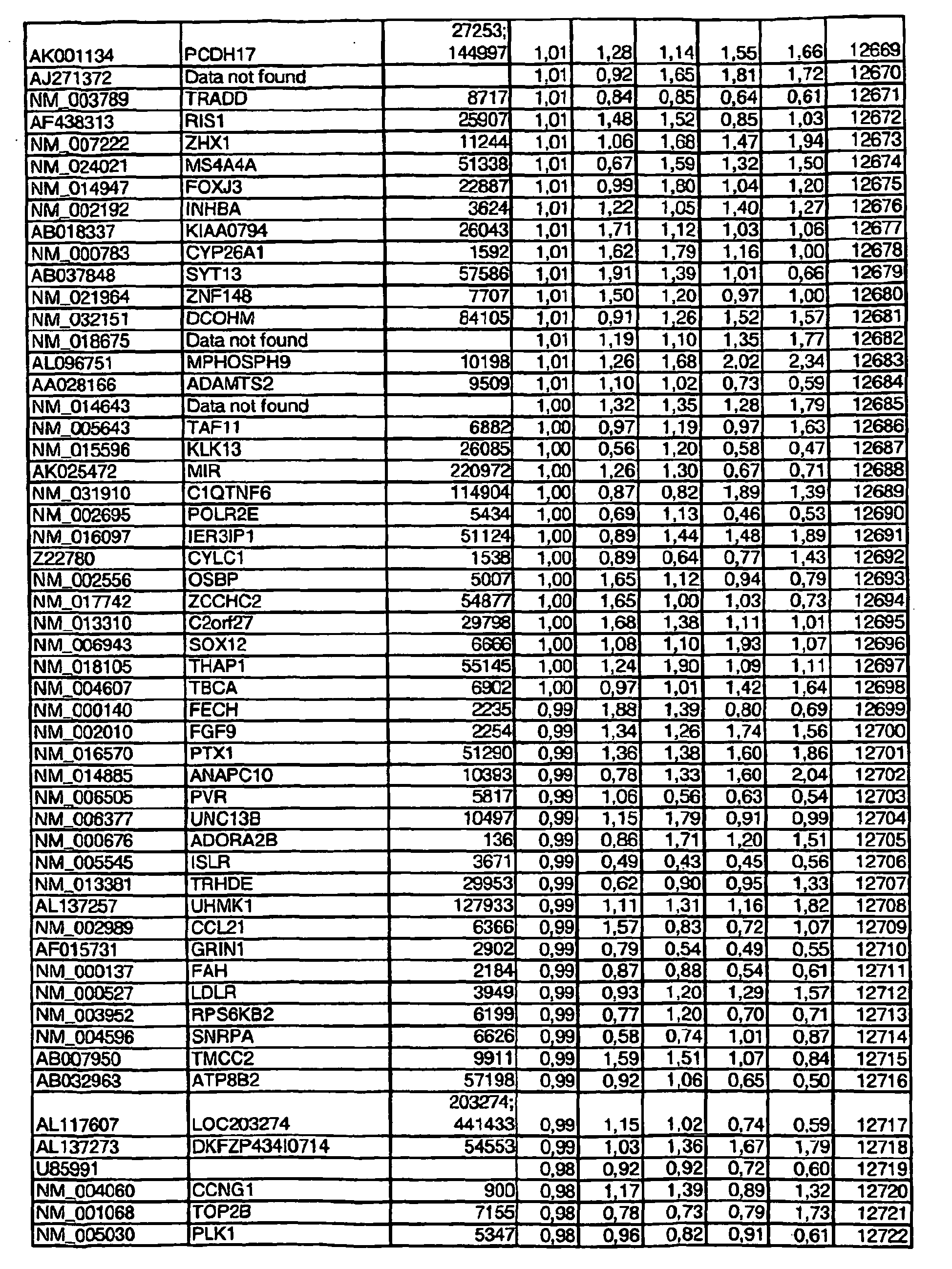

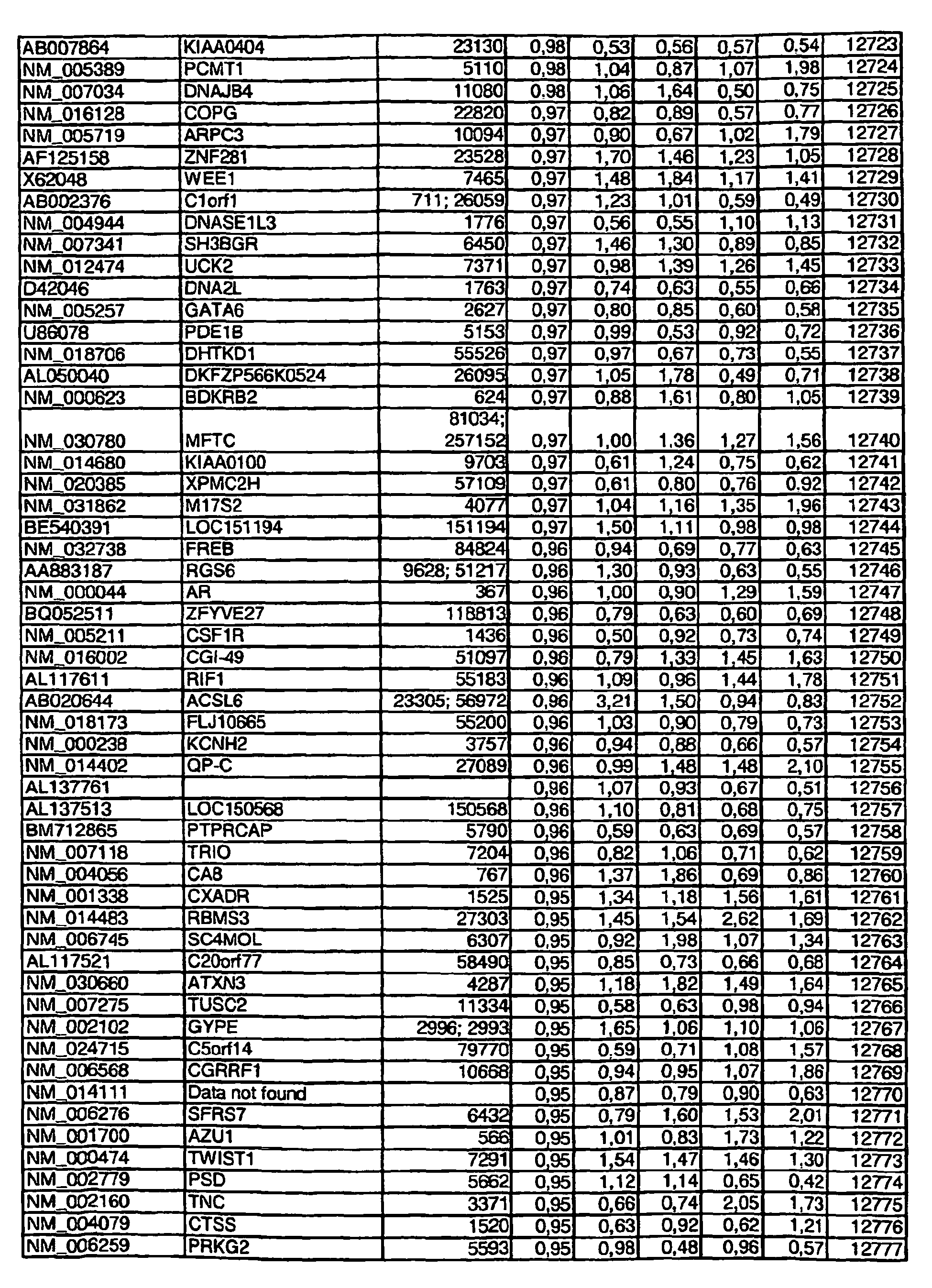

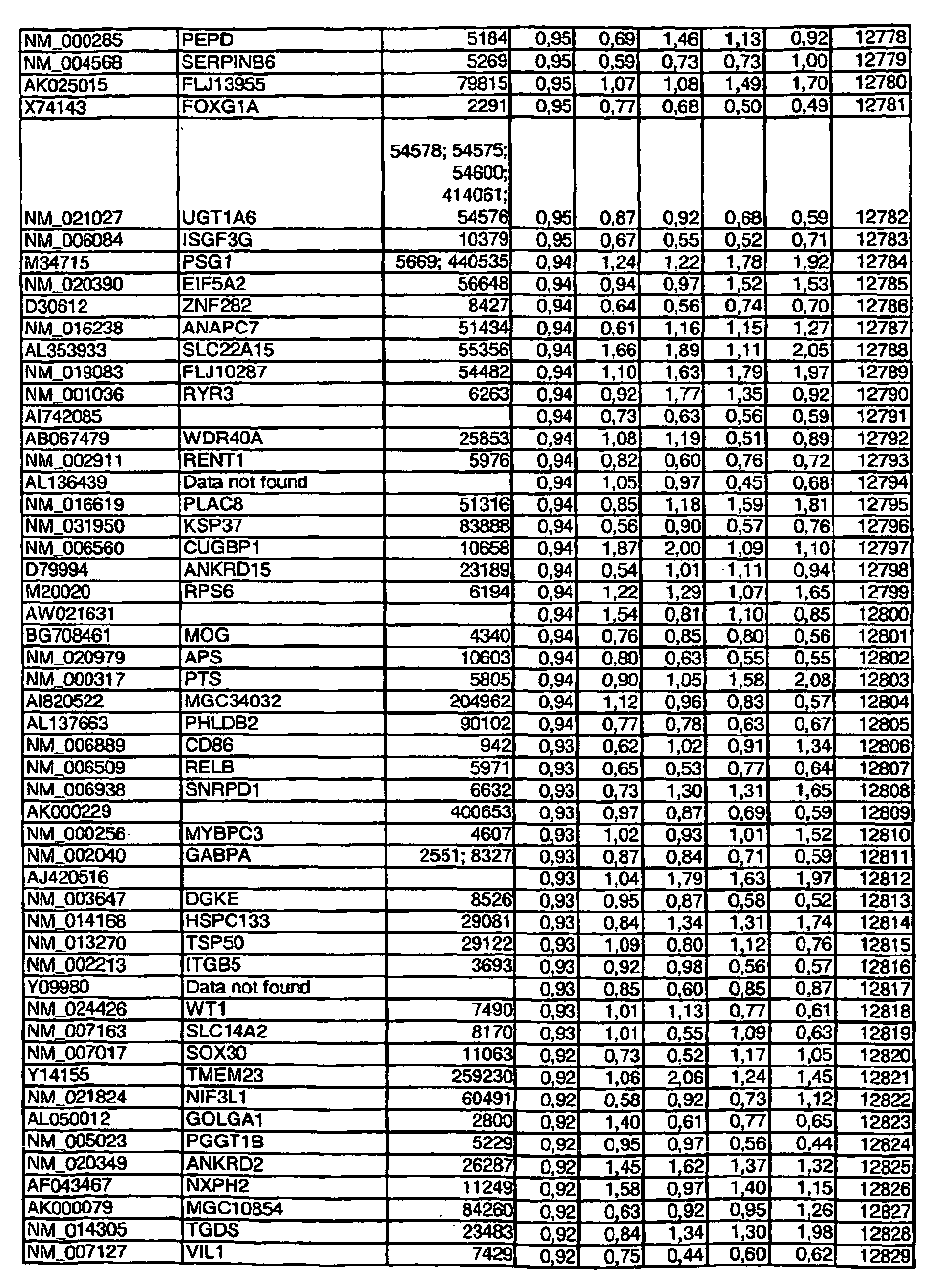

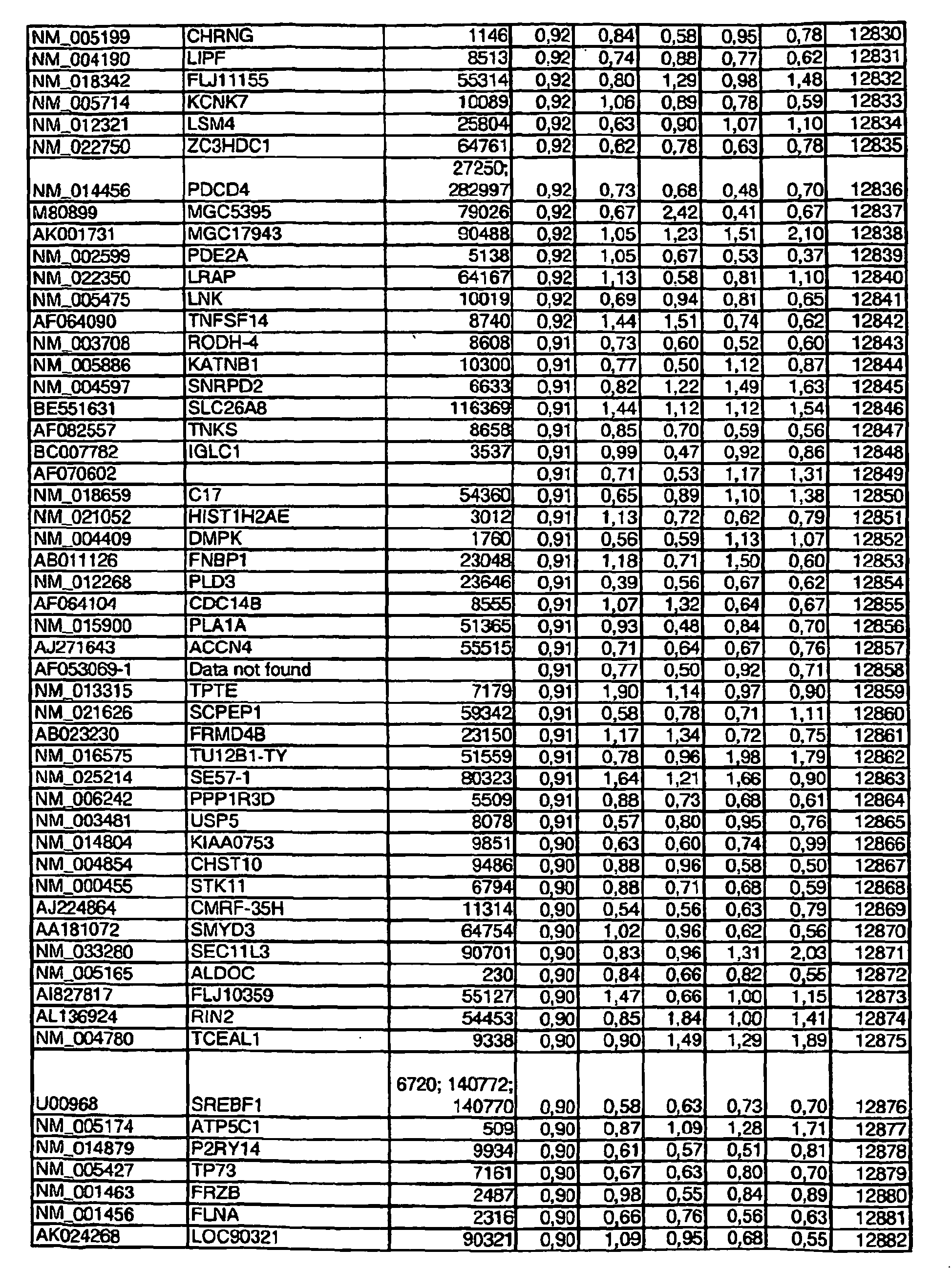

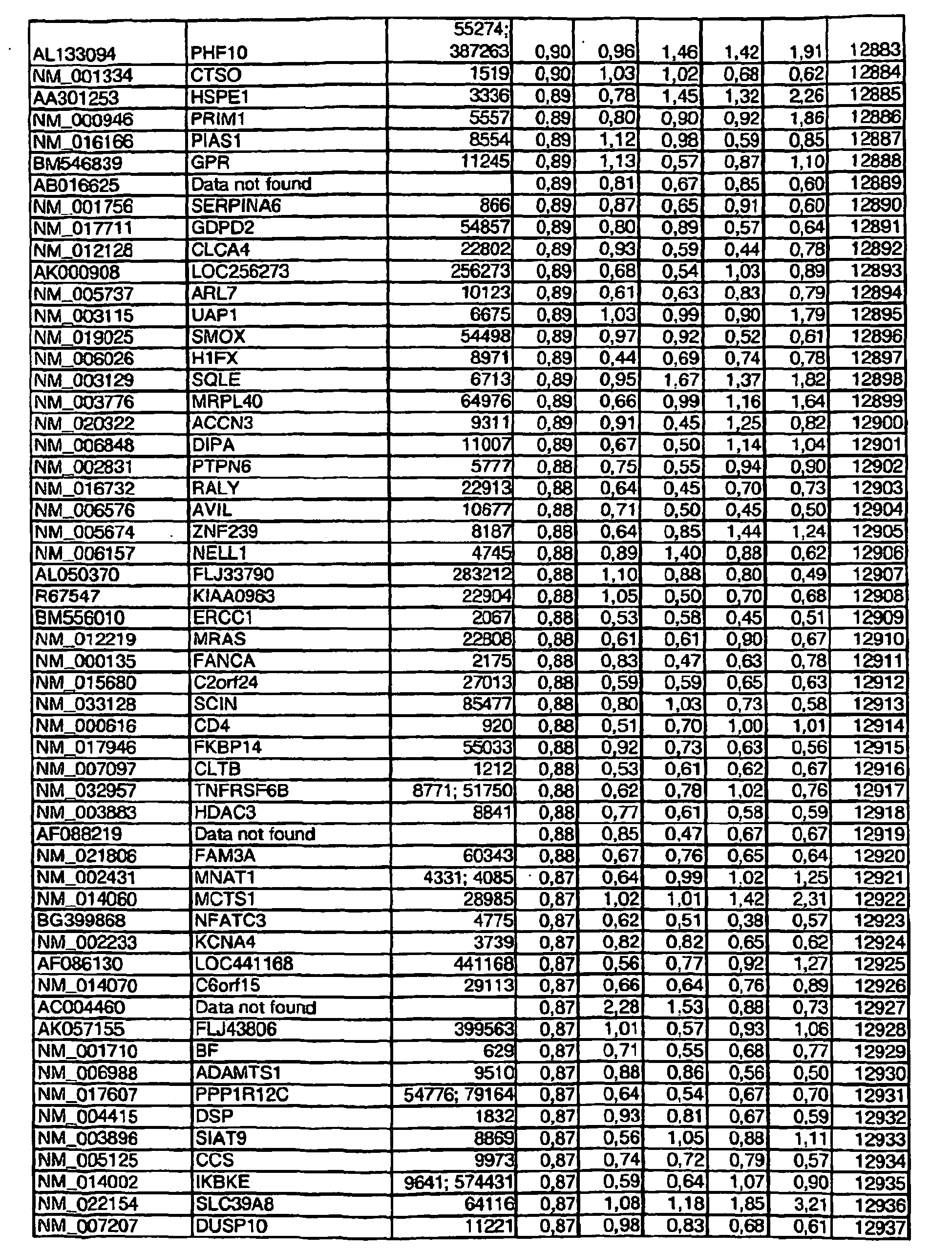

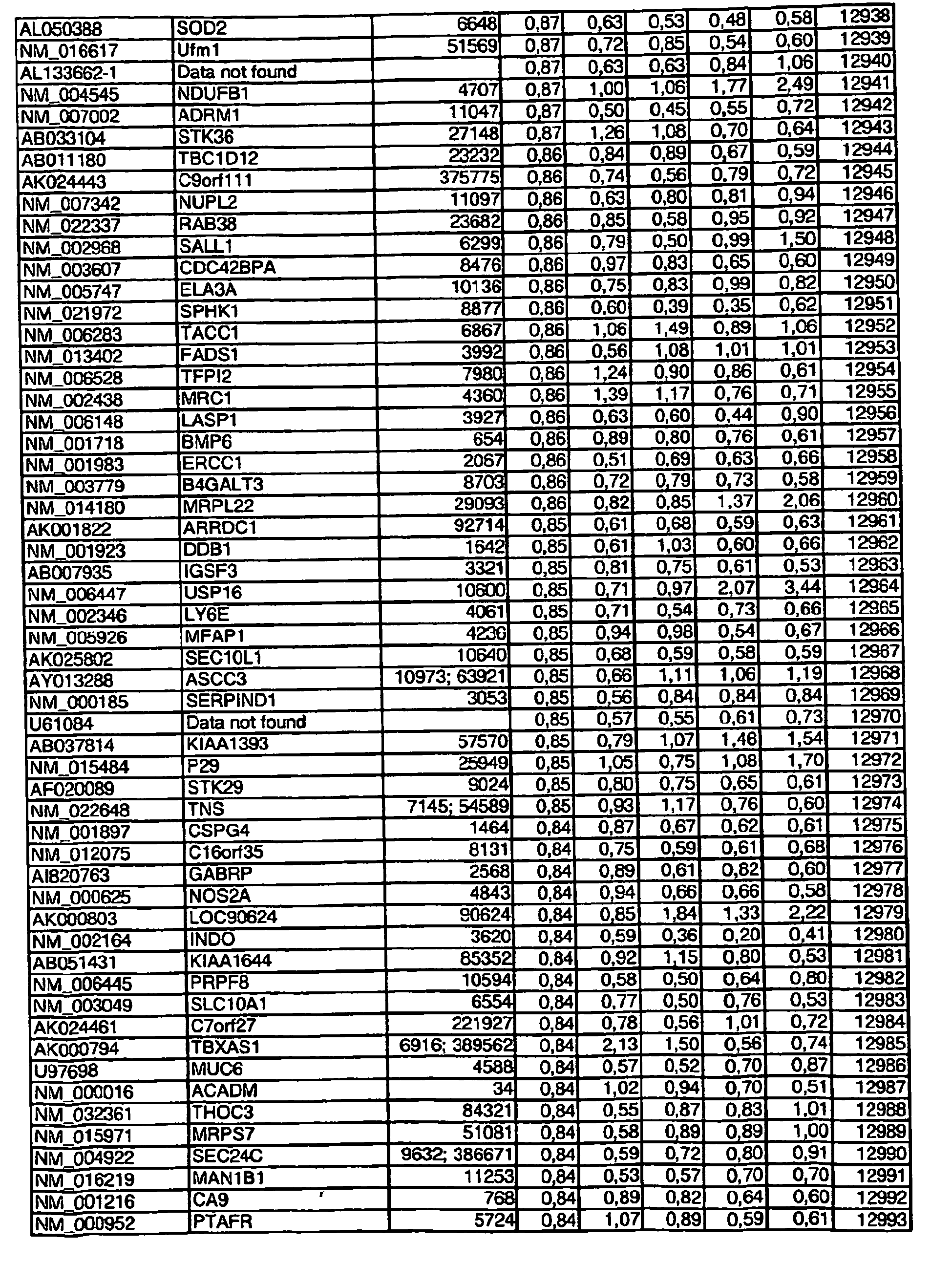

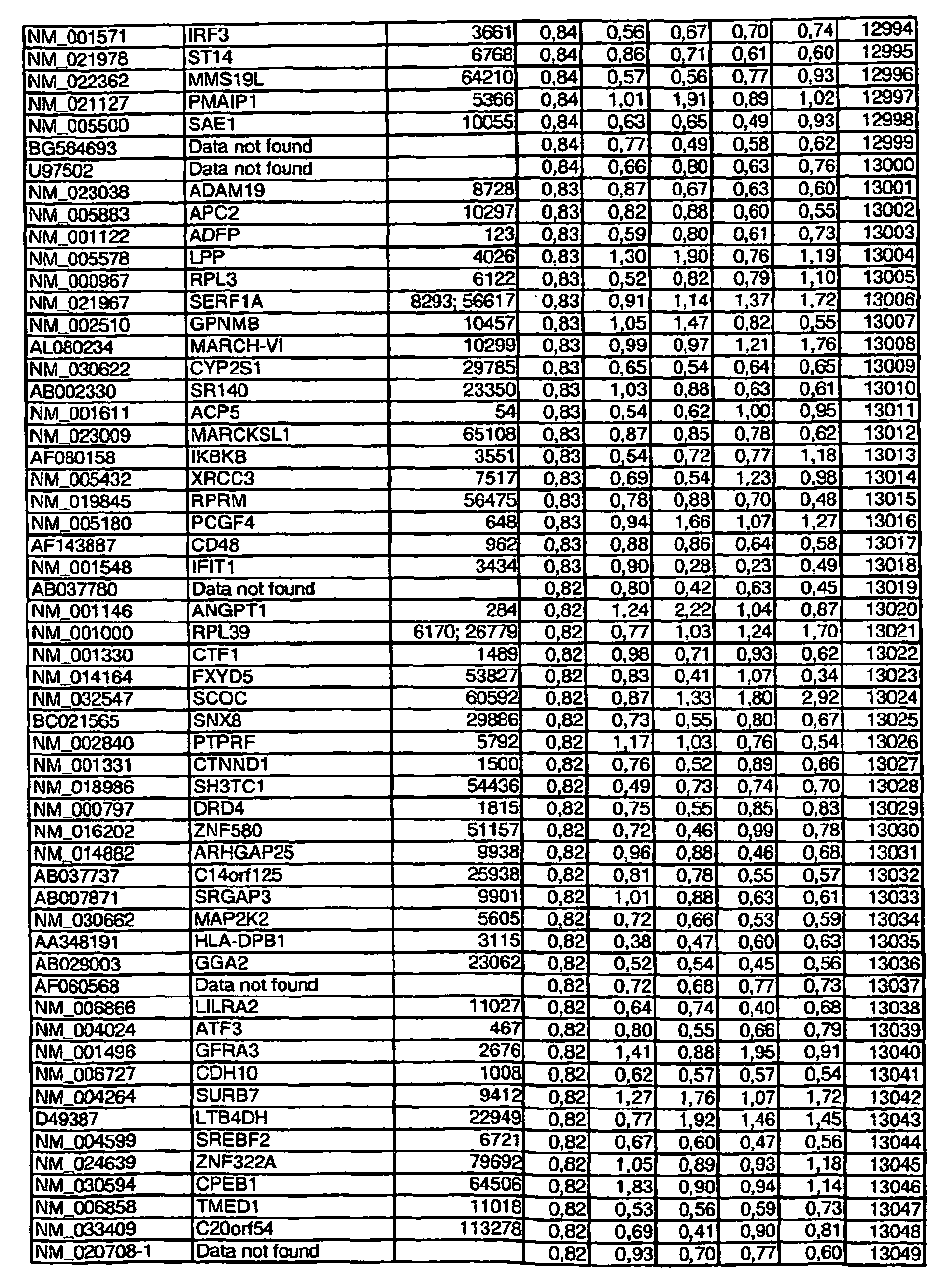

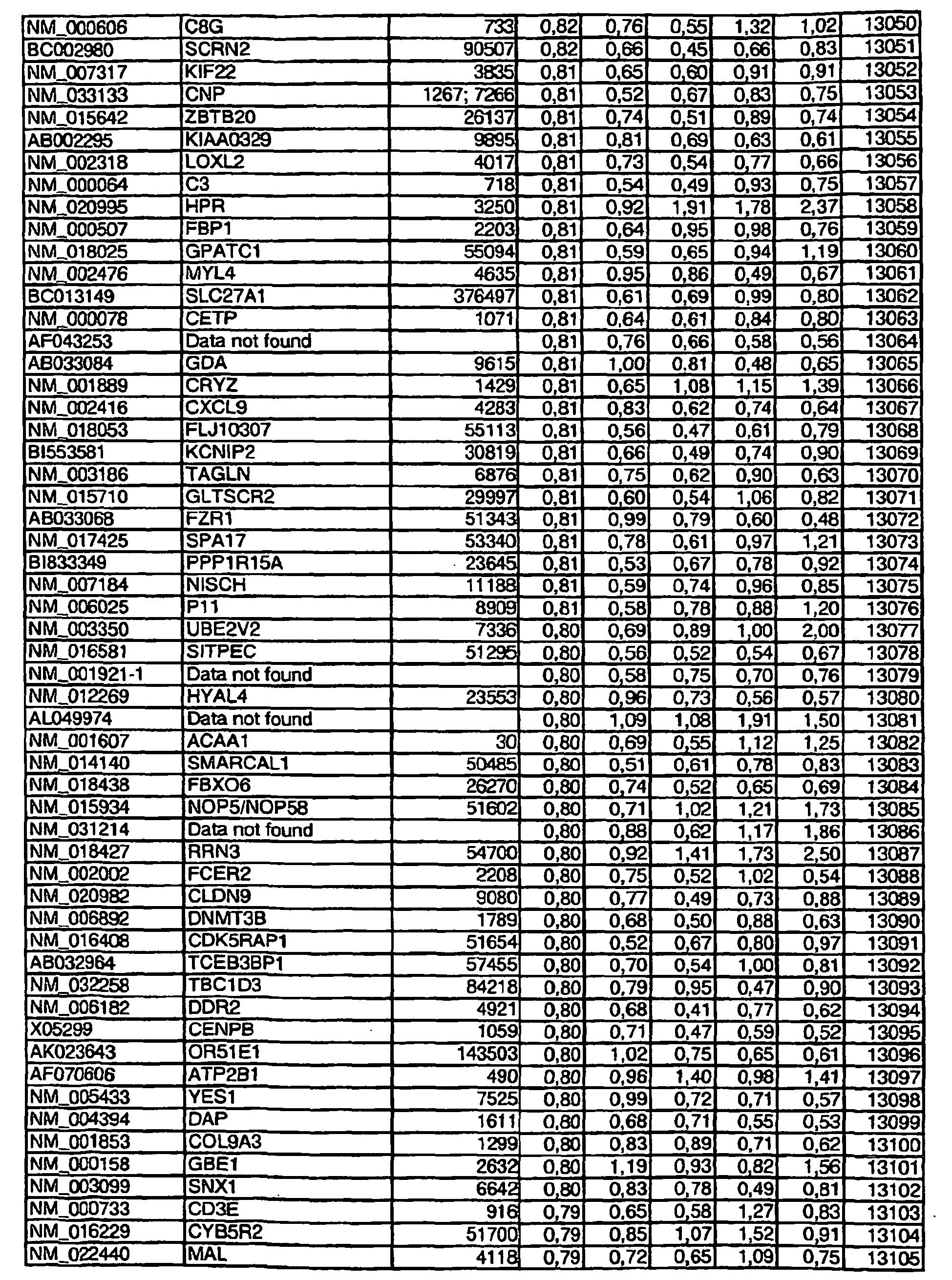

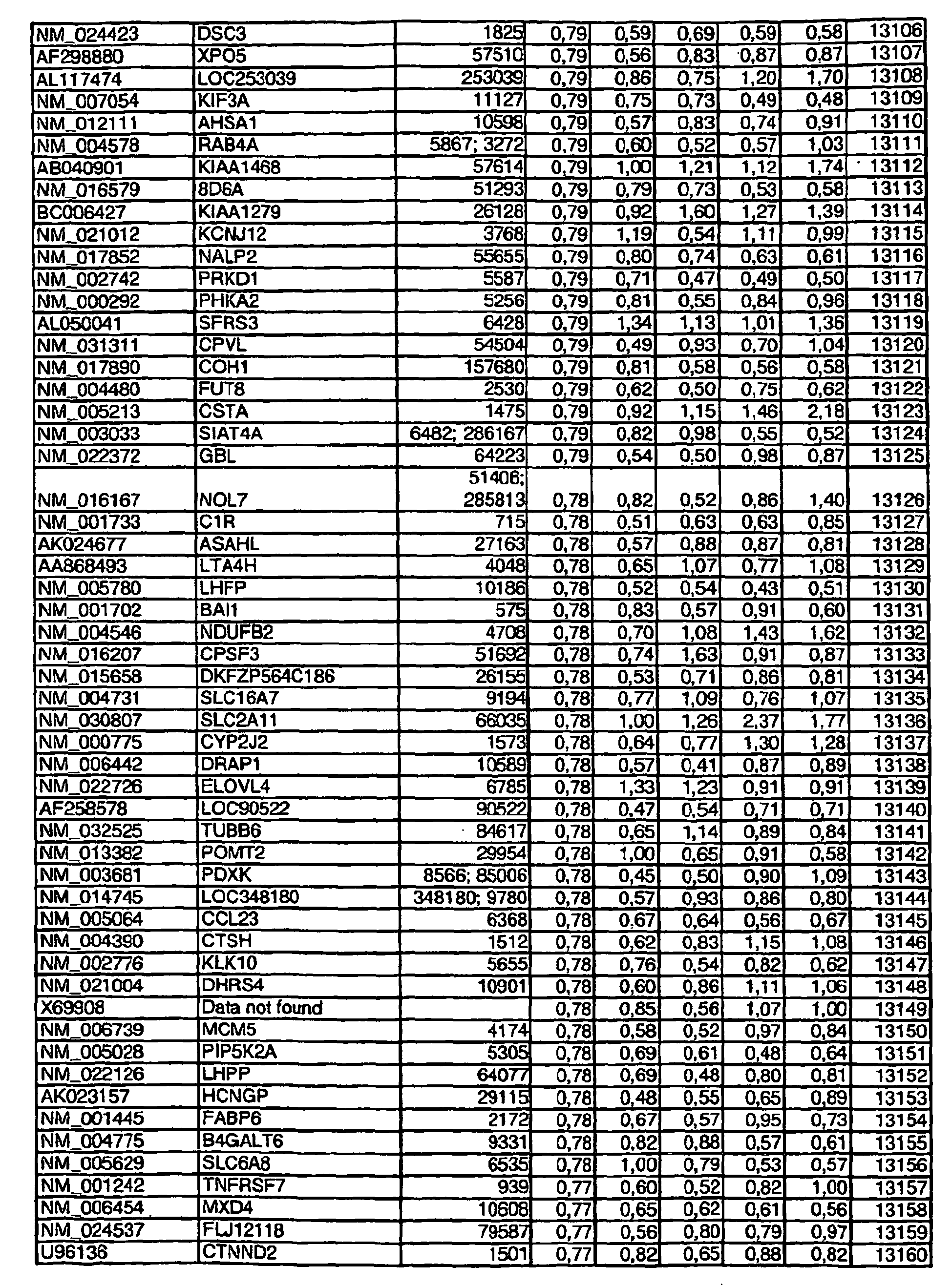

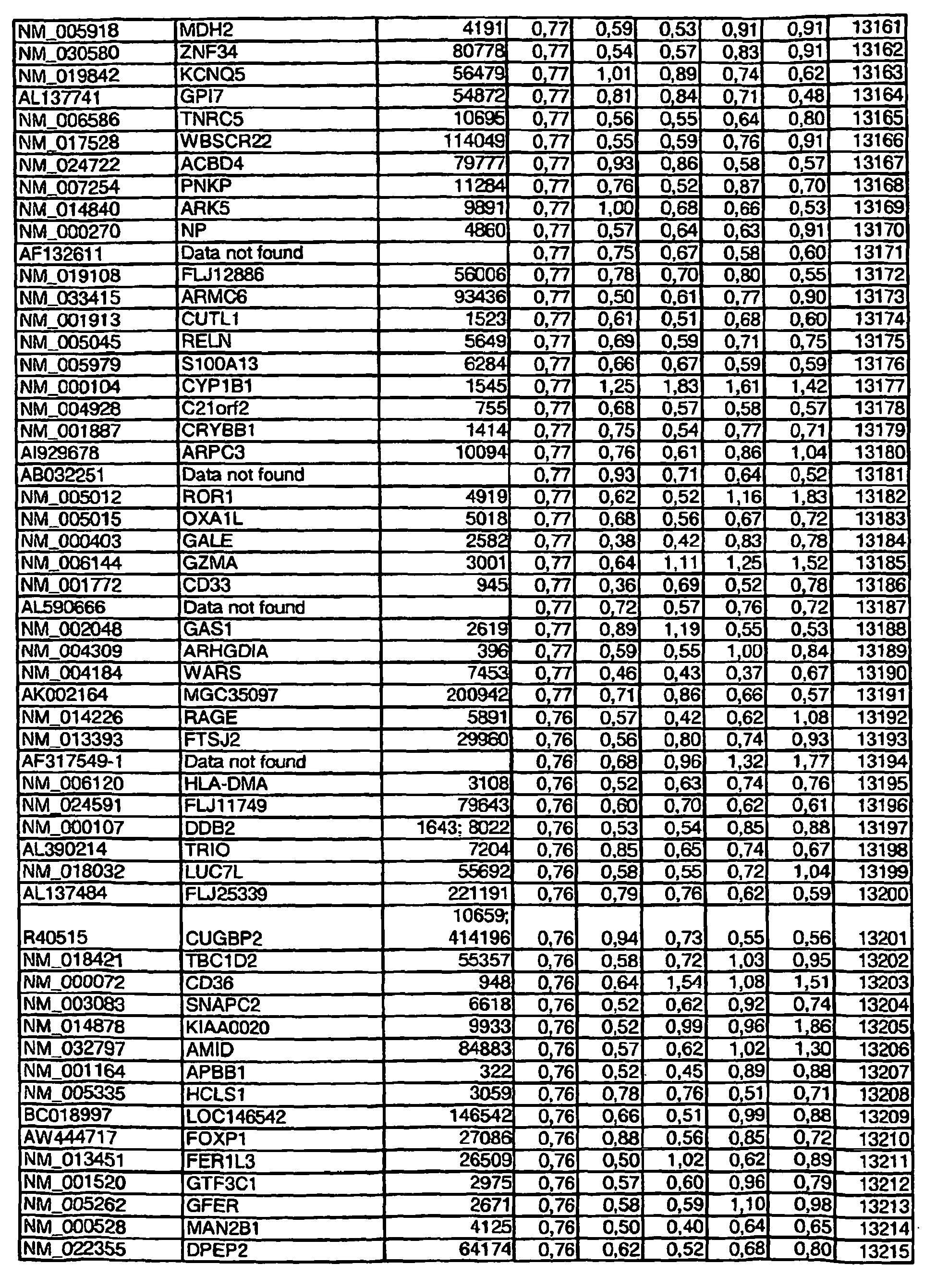

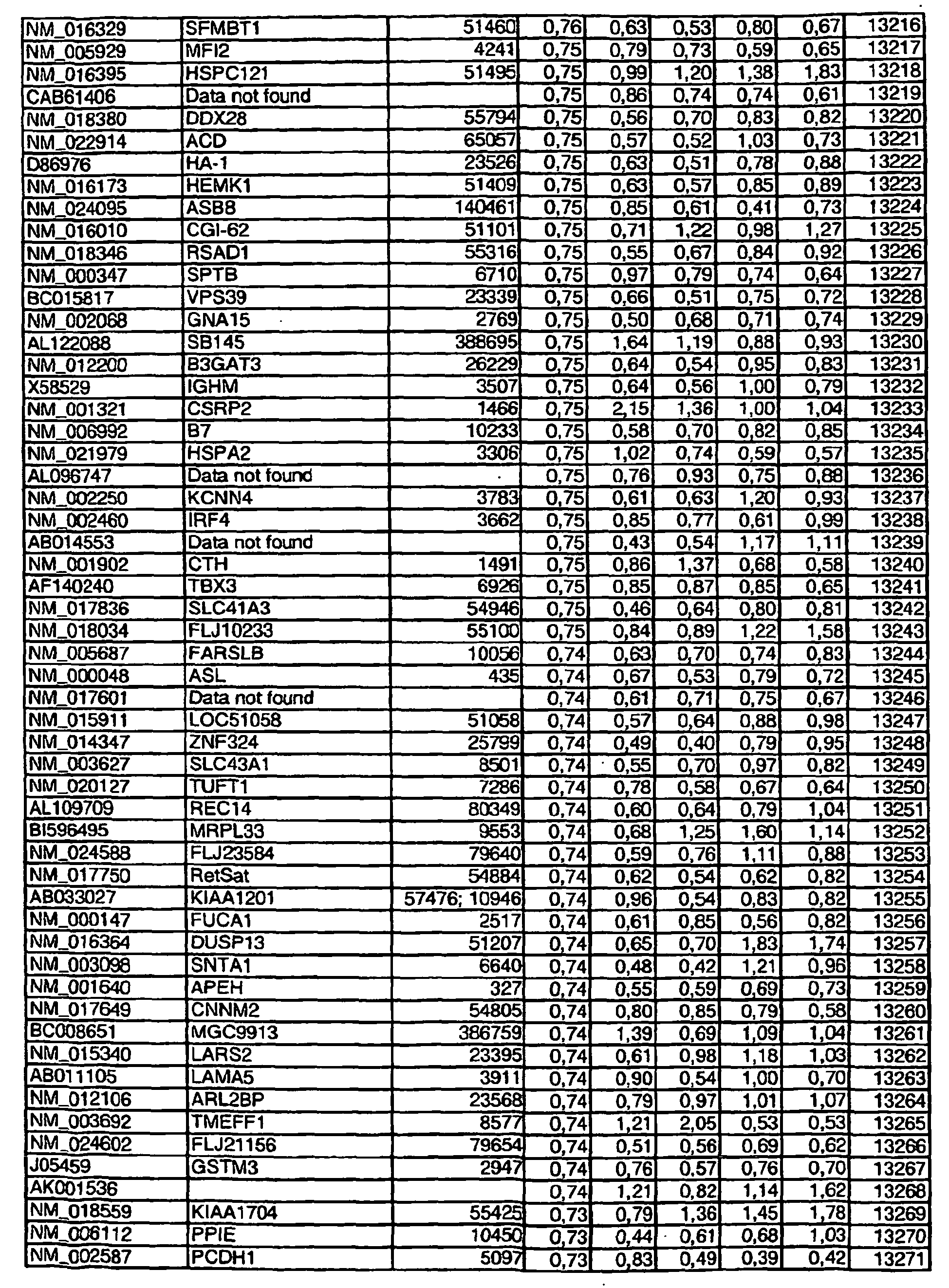

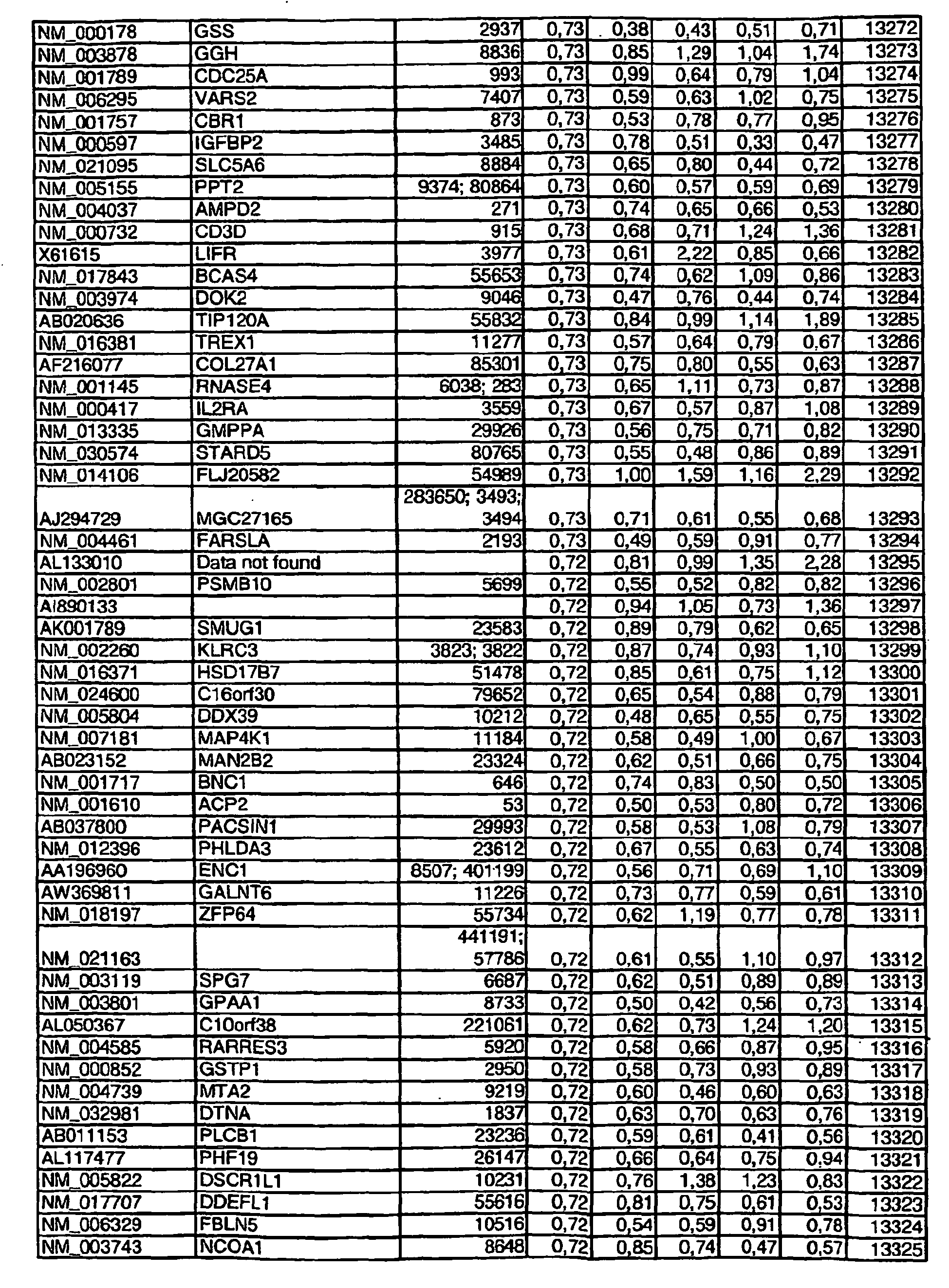

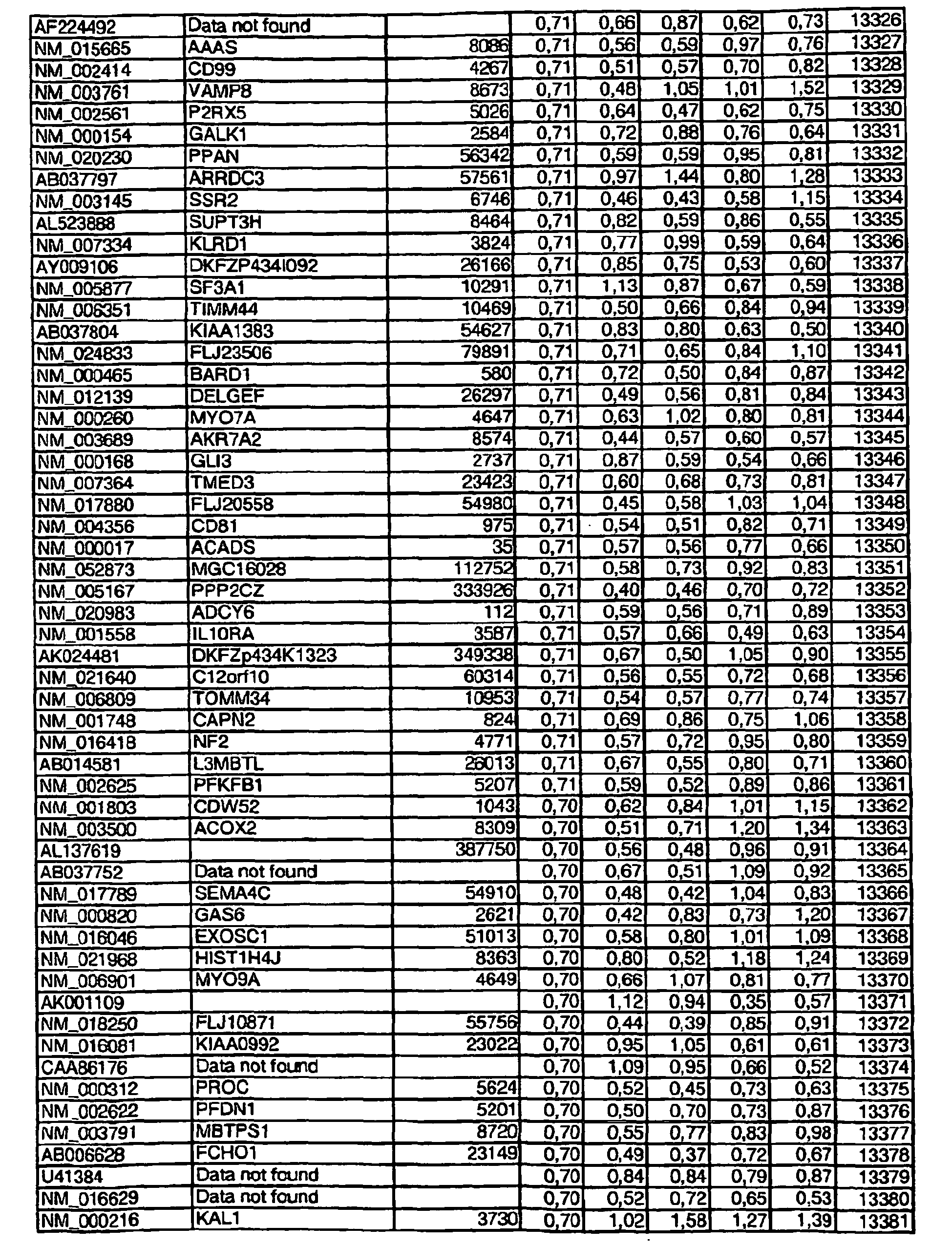

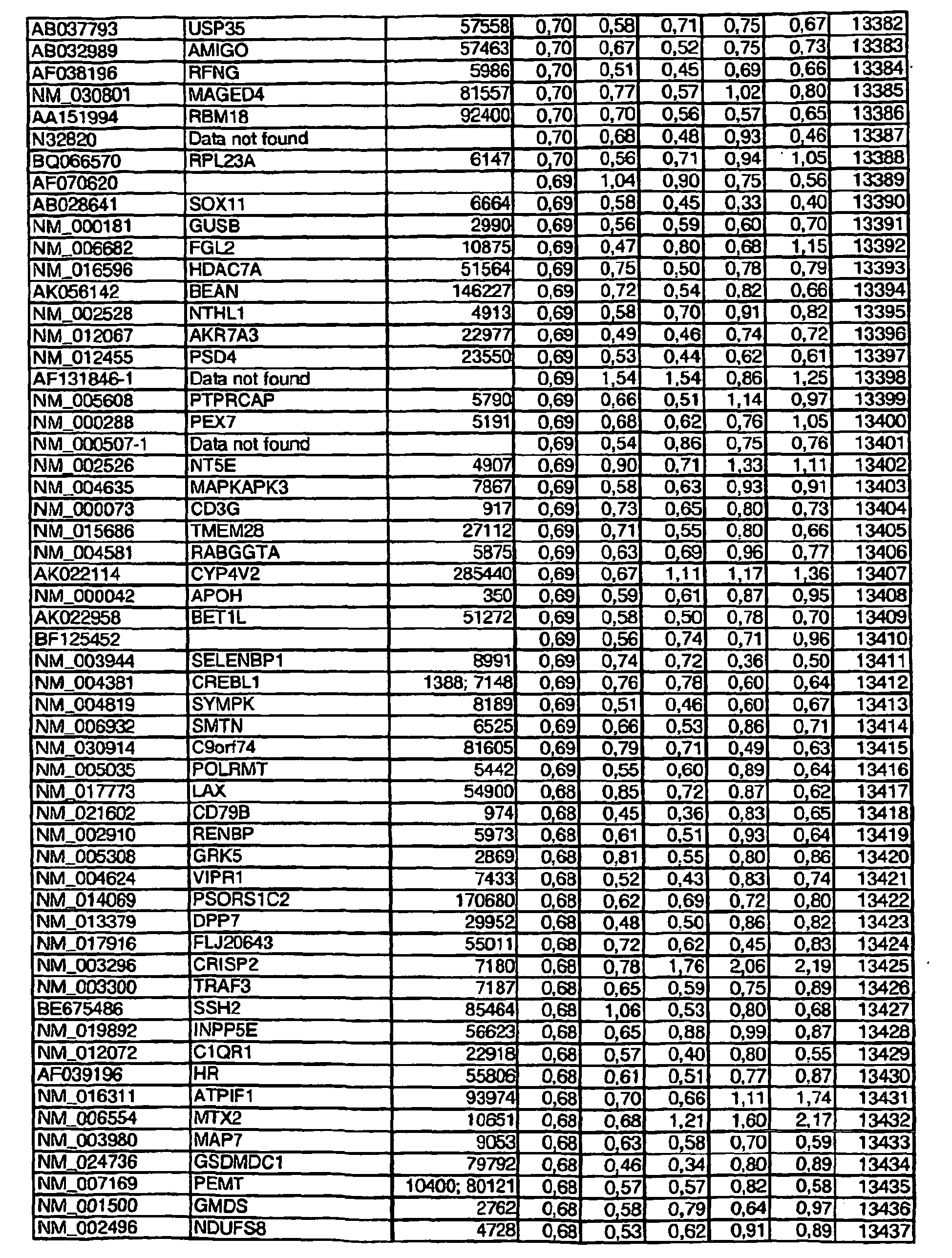

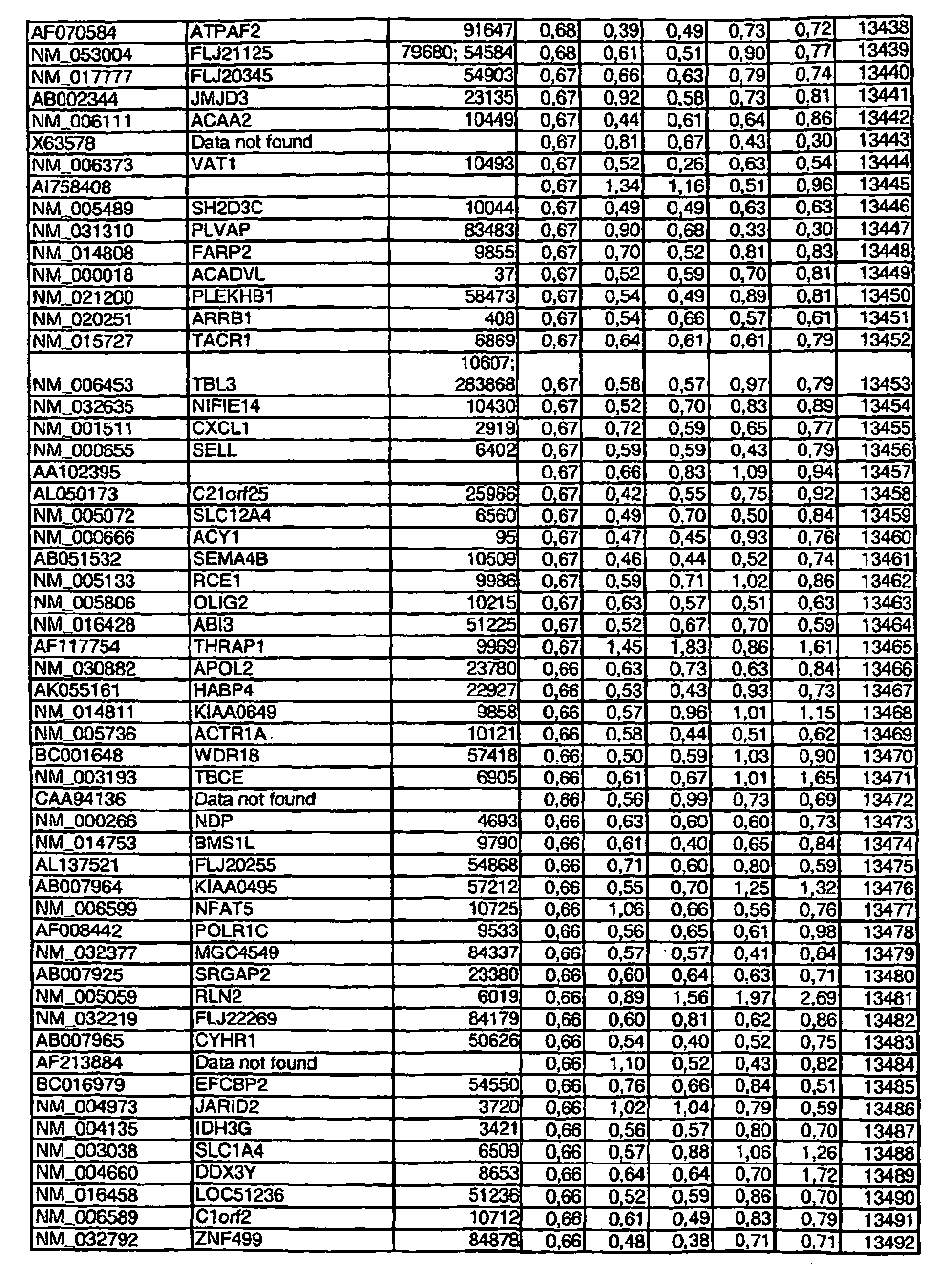

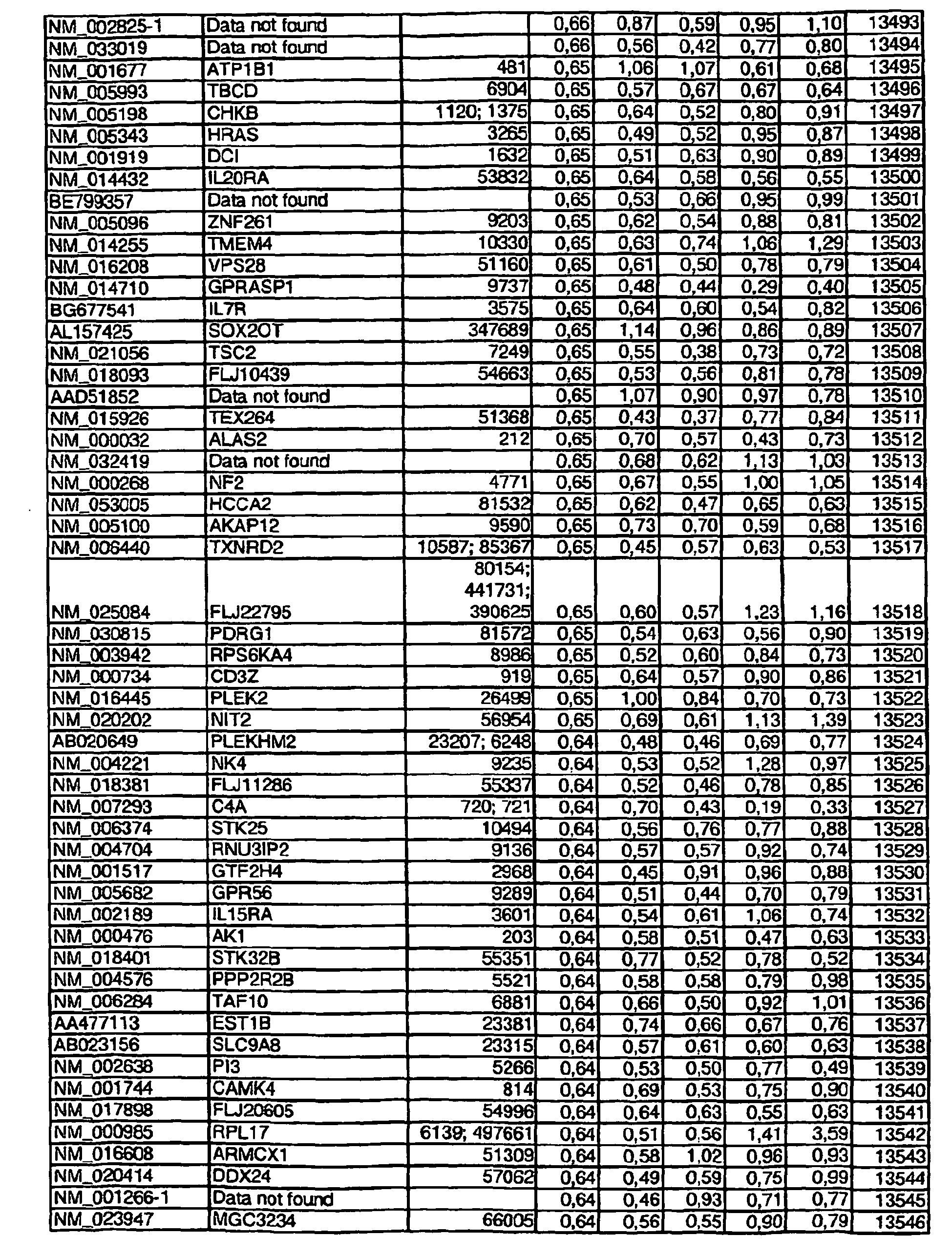

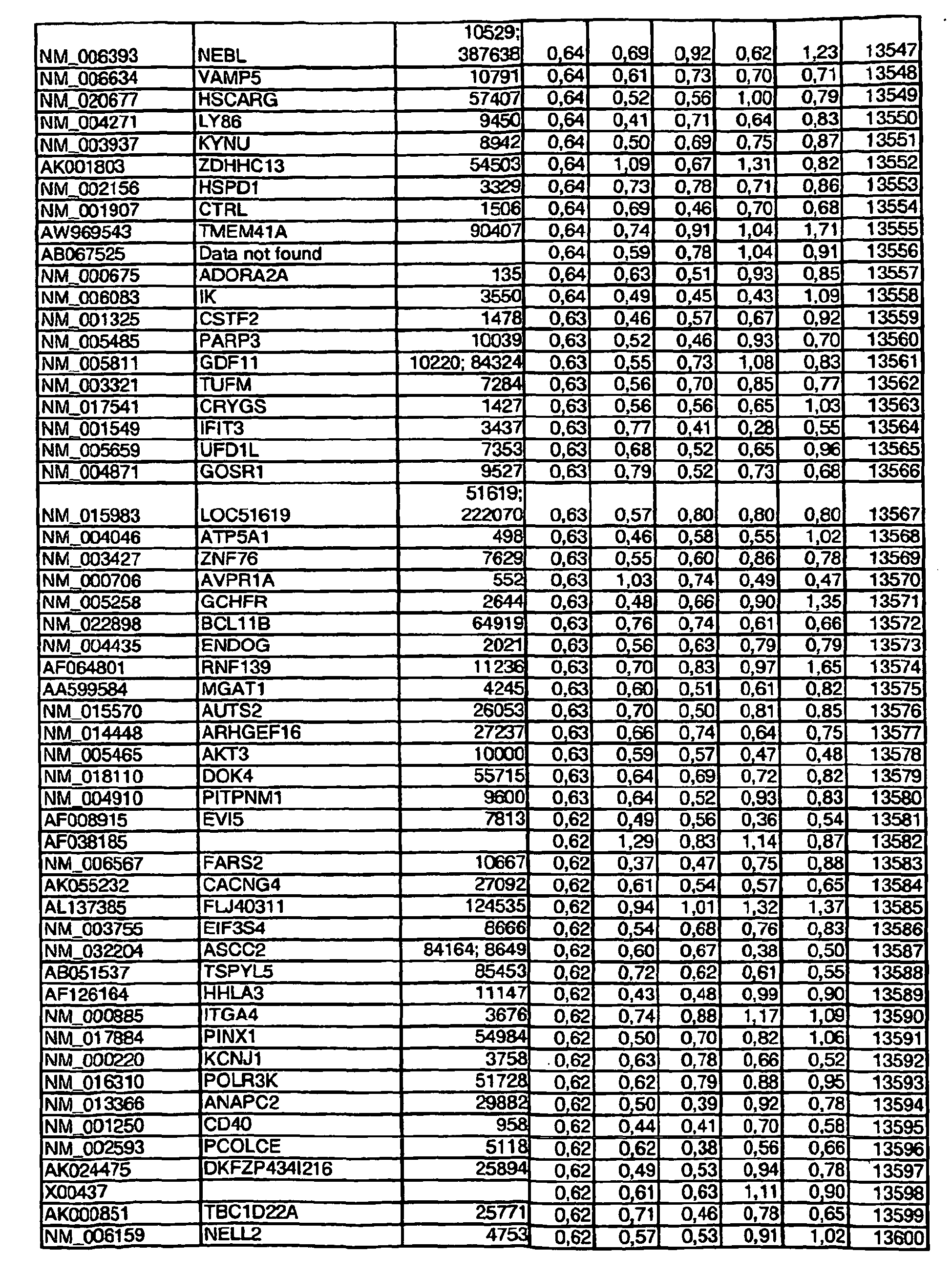

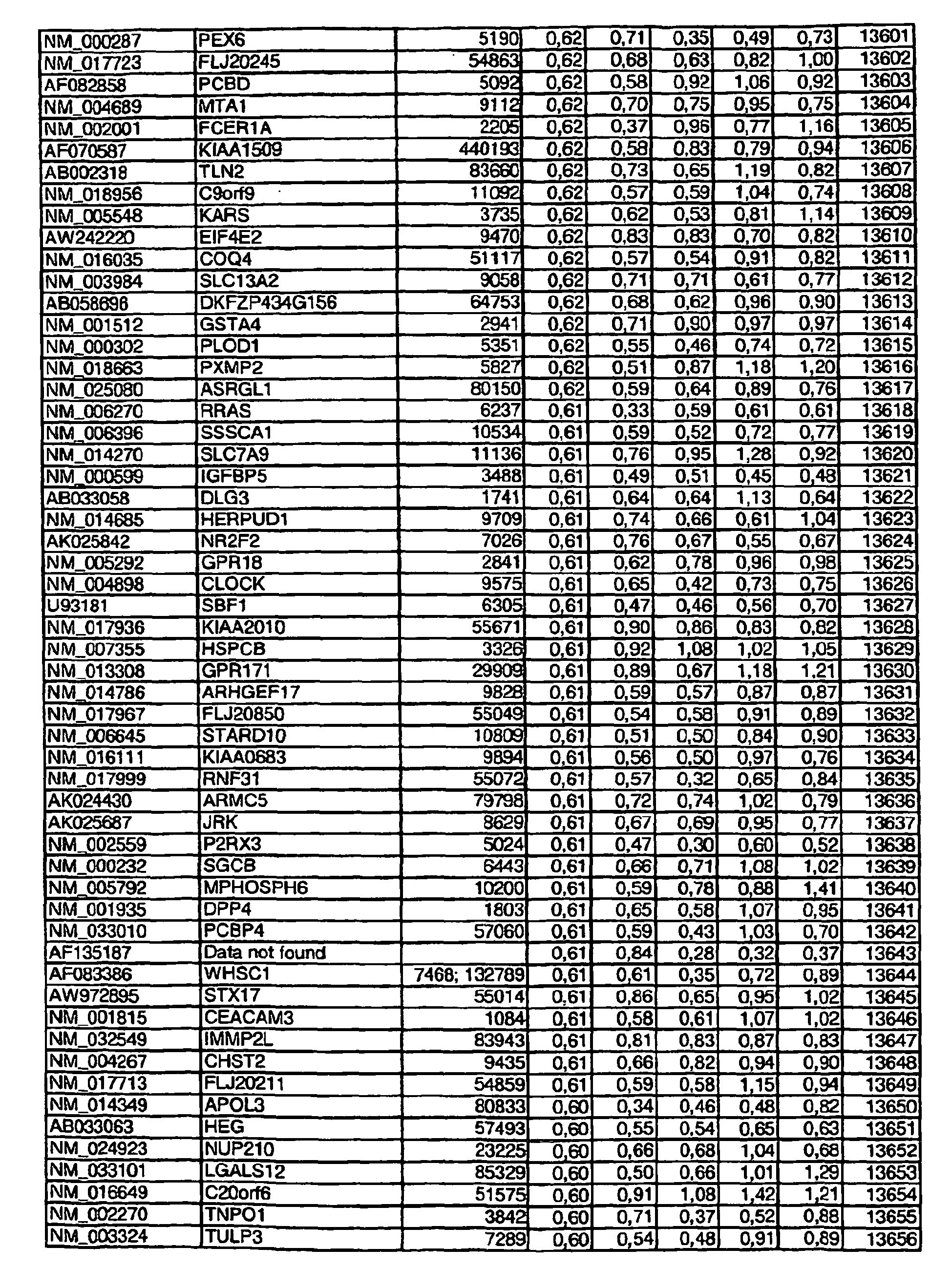

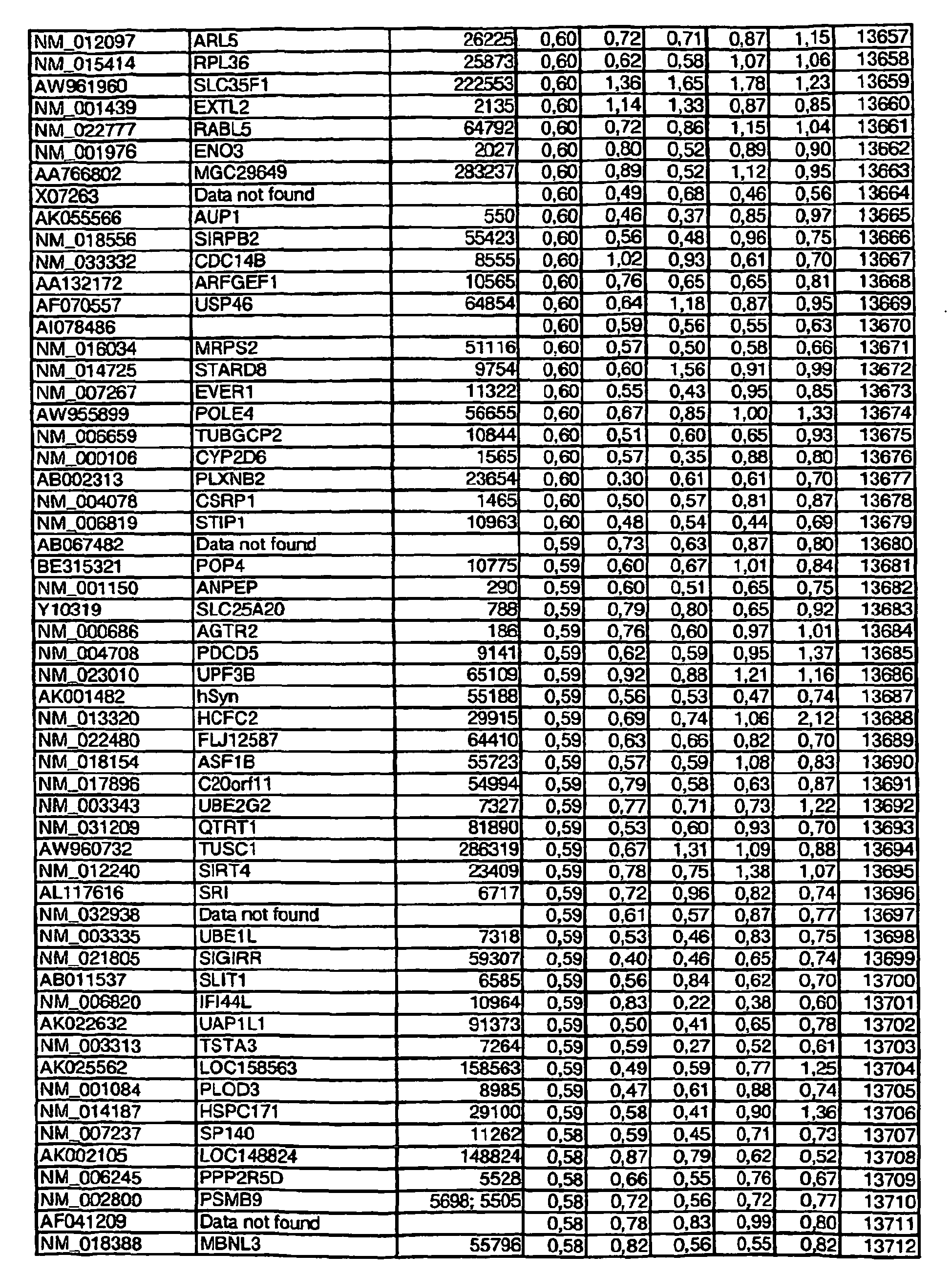

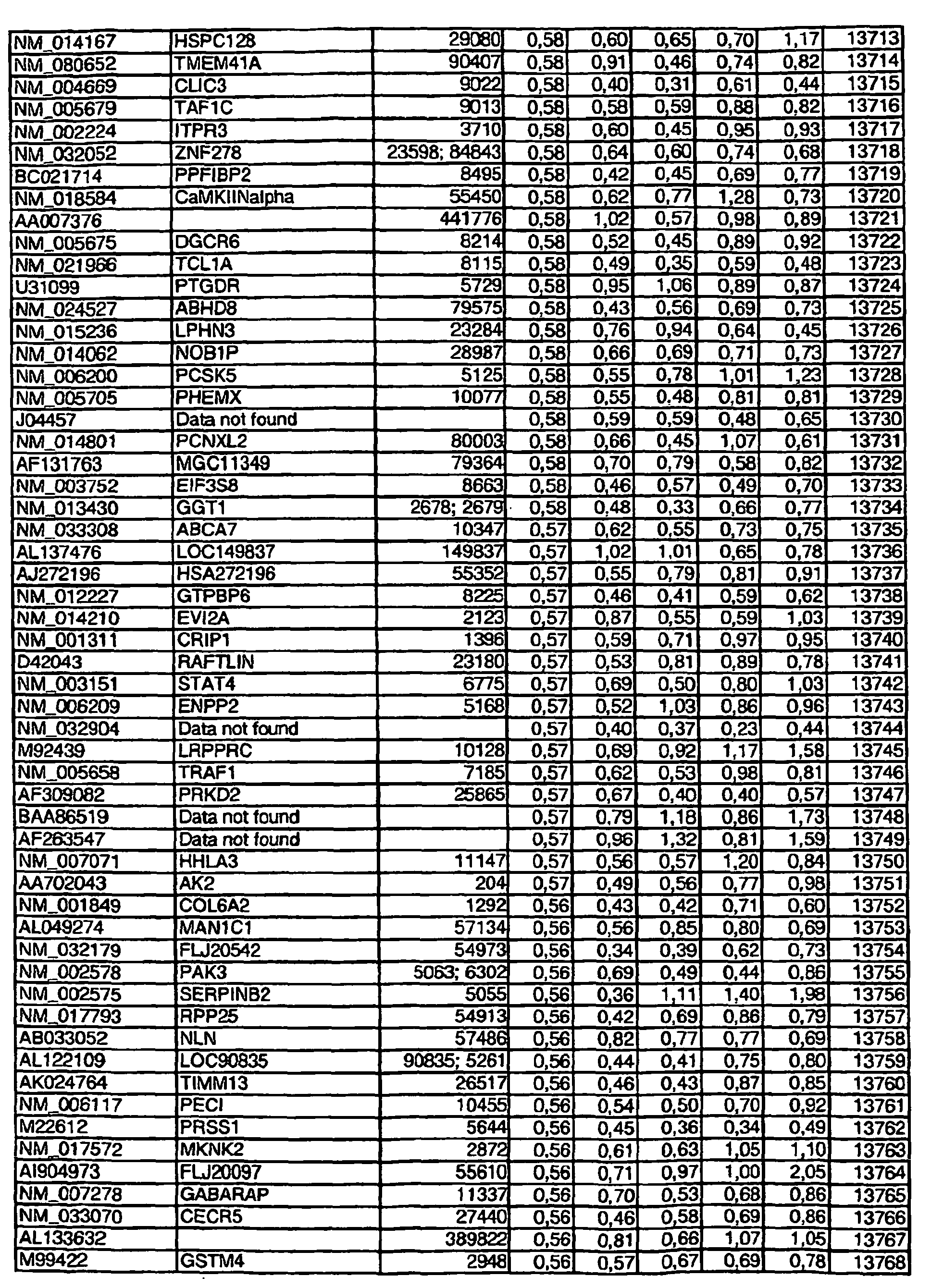

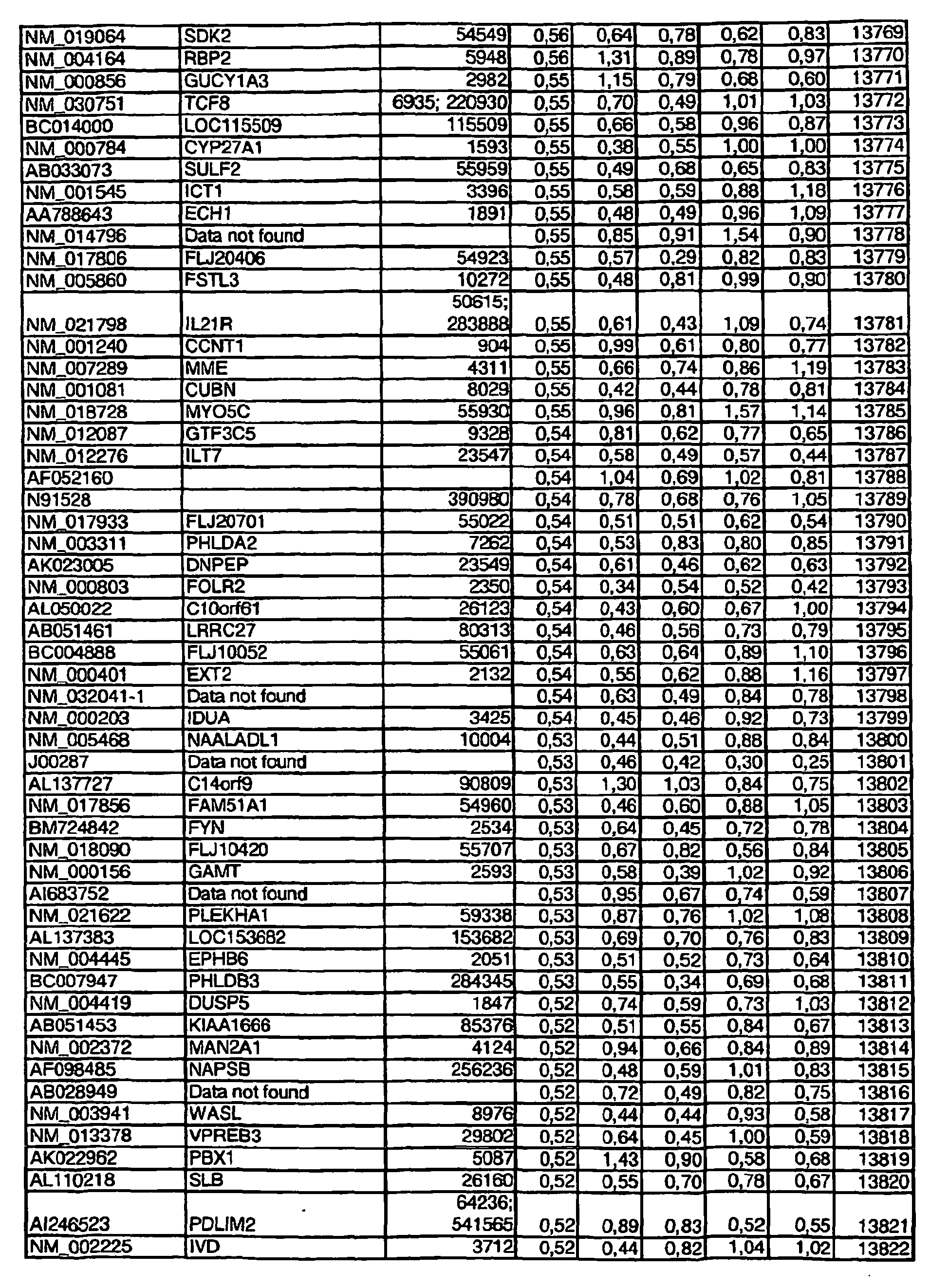

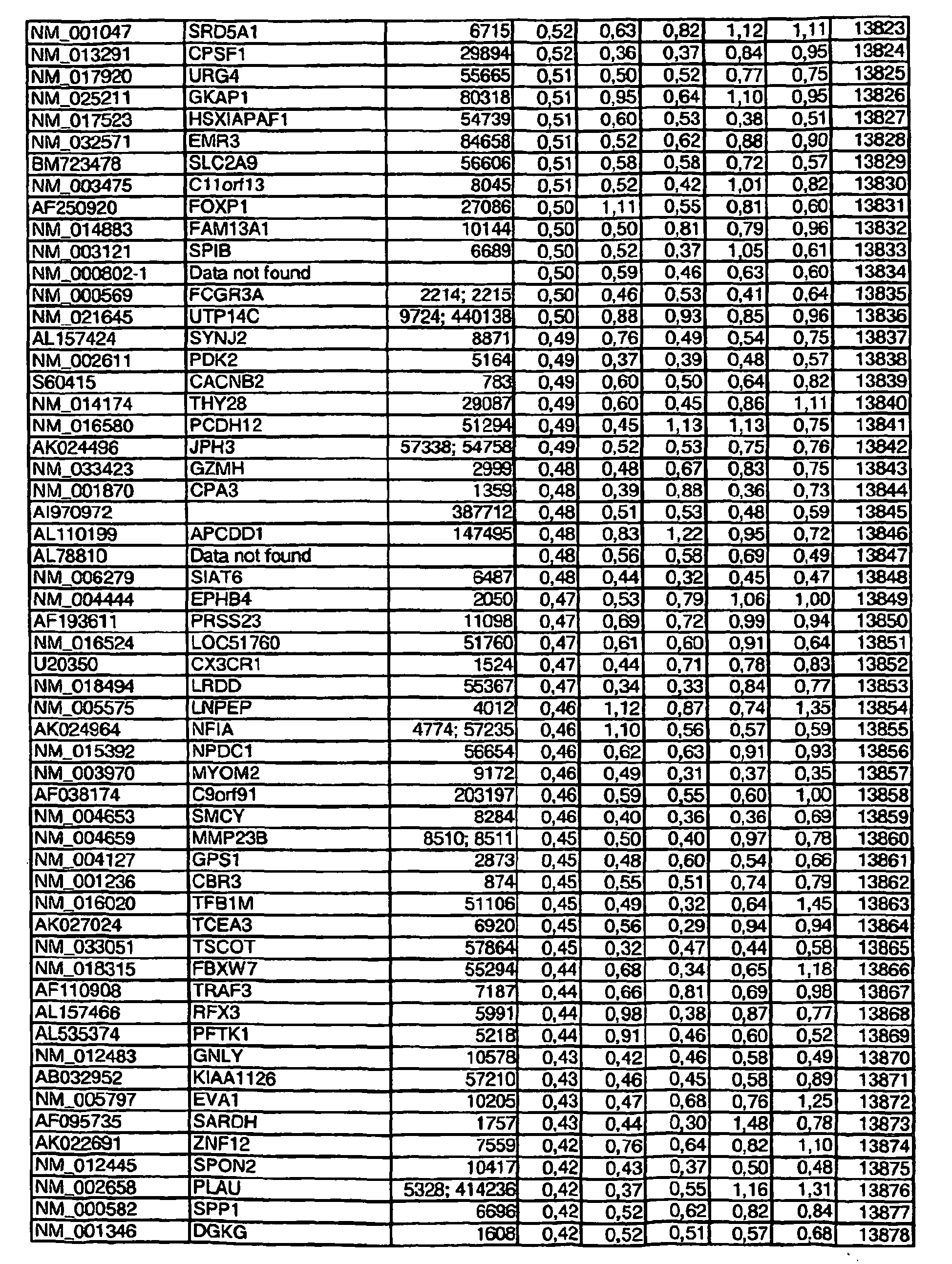

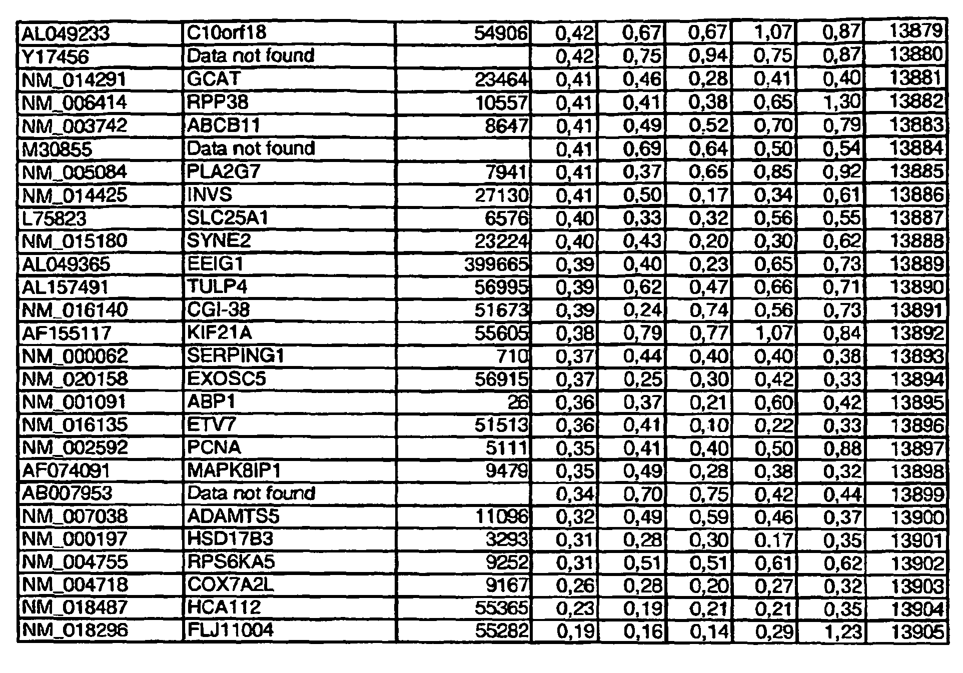

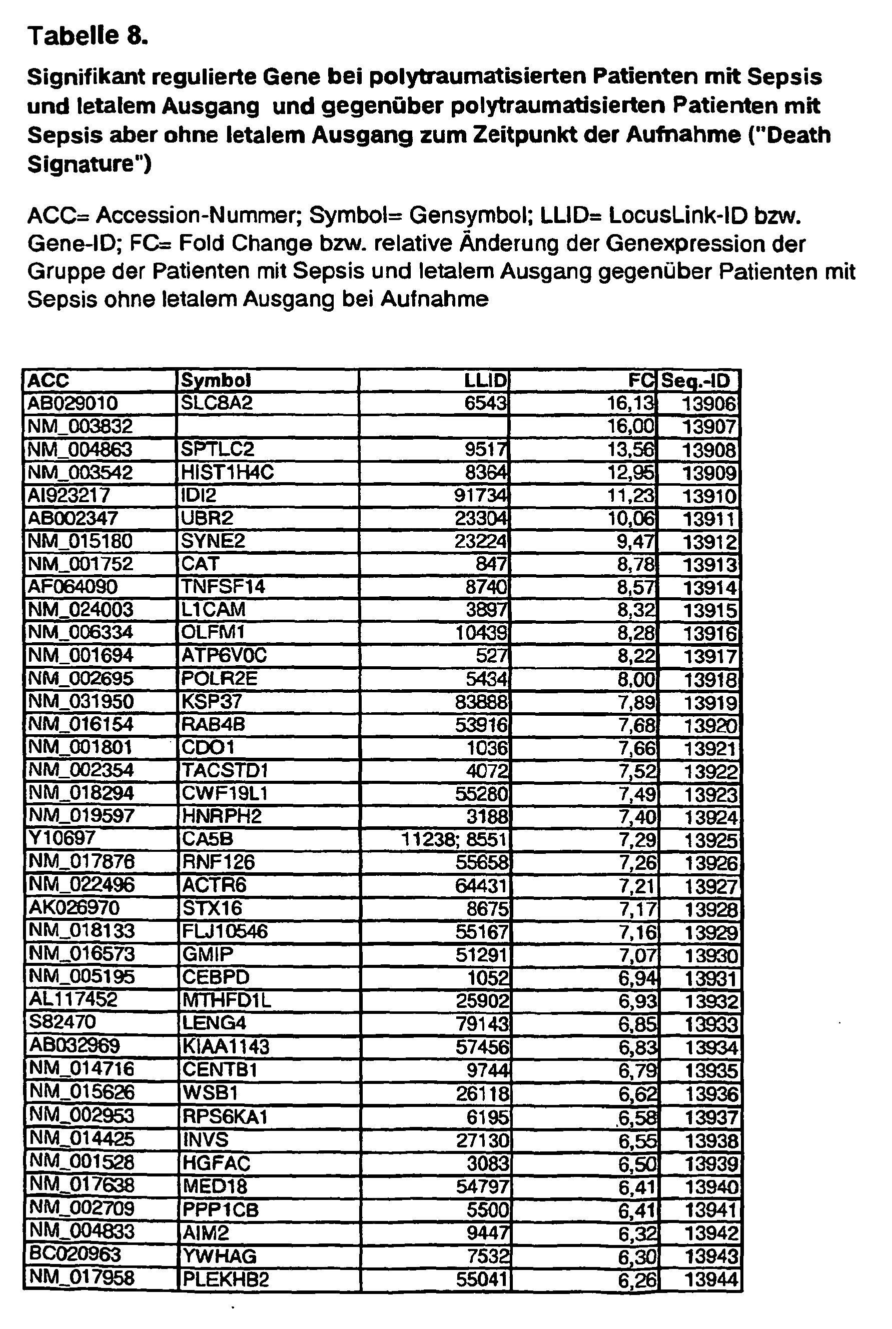

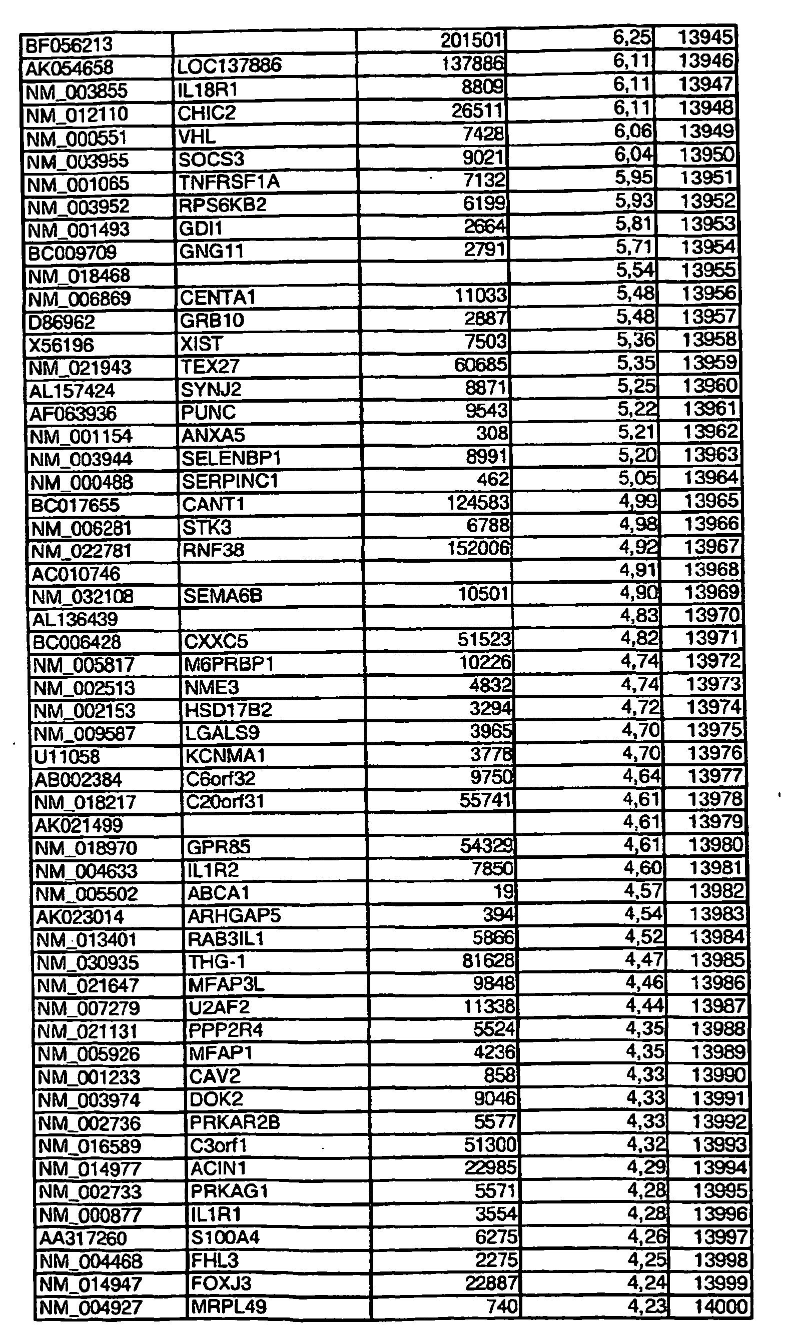

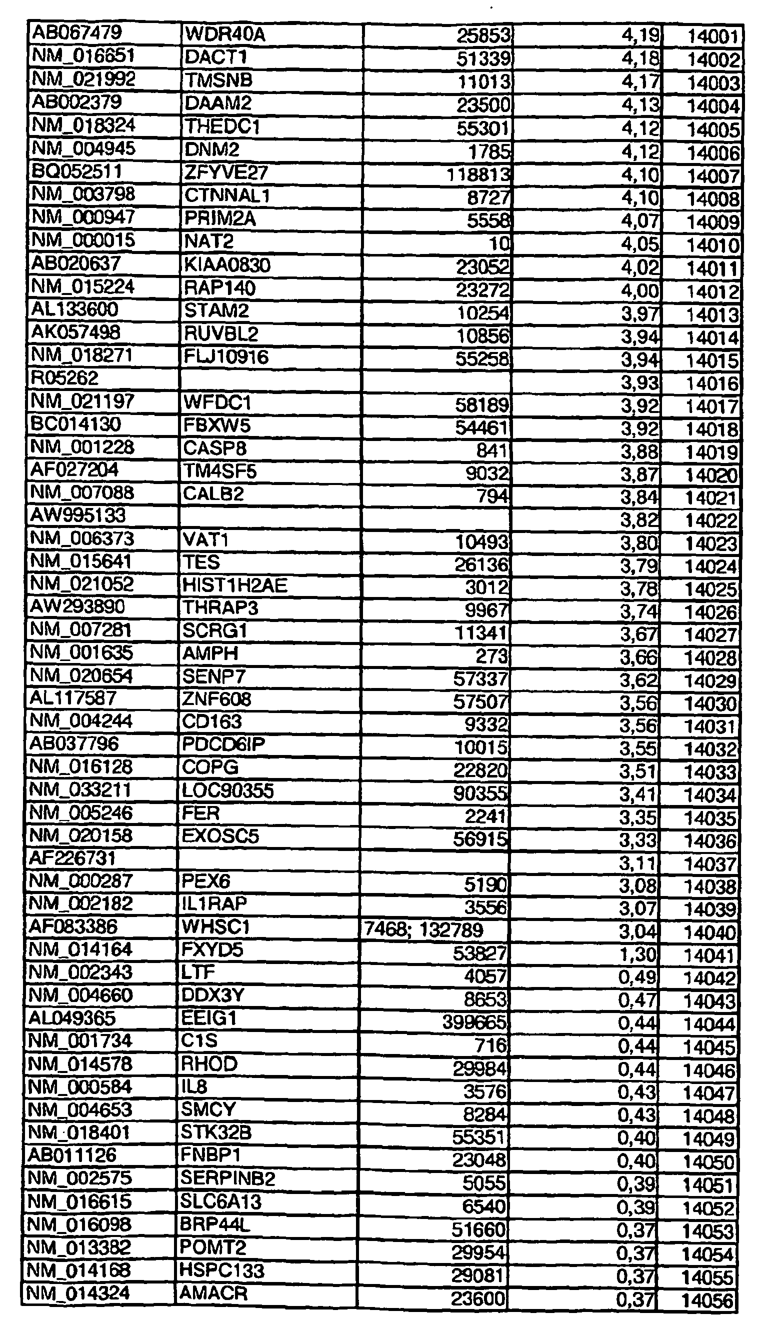

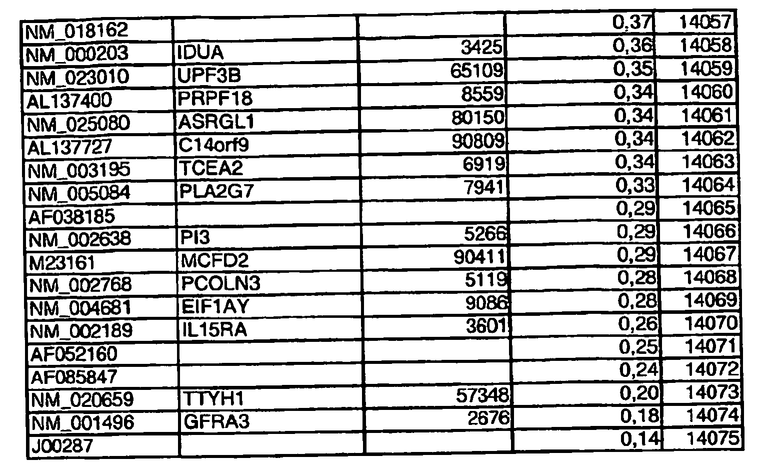

## Patentansprüche

1. Verfahren zum frühen Vorhersagen der Ausbildung einer Sepsis eines Patienten durch Erstellung eines Expressionsprofils 6 bis 24 Stunden nach Trauma, umfassend folgende Schritte:
i) Isolieren von RNA aus humanem Blut,
ii) Markieren der RNA aus Schritt i) mit einem detektierbaren Marker,
iii) Hybridisieren der RNA aus Schritt ii) mit Oligonukleotid-DNA der Gene Seq.-ID 6706 bis Seq.-ID 7562 (Tabelle 2), die auf einem Microarray angeordnet sind, unter Hybridisierungsbedingungen,
iv) Quantitatives Erfassen der Markierungssignale der hybridisierten RNA aus Schritt iii) in einem Expressionsprofil,
v) Vergleich des Expressionsprofiles aus Schritt iv) mit einer Kontrollprobe eines Patienten, der keine Sepsis entwickelt, hinsichtlich der Expression der Gene Seq.-ID 6706 bis Seq.-ID 7562 (Tabelle 2),
vi) Bestimmung von Proteinen und Metaboliten in der humanen Blutprobe.

2. Verfahren zum frühen Vorhersagen der Ausbildung einer Sepsis eines Patienten durch Erstellung eines Expressionsprofils 30 bis 48 Stunden nach Trauma, umfassend folgende Schritte:
i) Isolieren von RNA aus humanem Blut,
ii) Markieren der RNA aus Schritt i) mit einem detektierbaren Marker,
iii) Hybridisieren der RNA aus Schritt ii) mit Oligonukleotid-DNA der Gene Seq.-ID 7563 bis Seq.-ID 8947 (Tabelle 3), die auf einem Microarray angeordnet sind, unter Hybridisierungsbedingungen,
iv) Quantitatives Erfassen der Markierungssignale der hybridisierten RNA aus Schritt iii) in einem Expressionsprofil,
v) Vergleich des Expressionsprofiles aus Schritt iv) mit einer Kontrollprobe eines Patienten, der keine Sepsis entwickelt, hinsichtlich der Expression der Gene Seq.-ID 7563 bis Seq.-ID 8947 (Tabelle 3),
vi) Bestimmung von Proteinen und Metaboliten in der humanen Blutprobe.

3. Verfahren zum Identifizieren von Risikopatienten, nämlich Patienten mit Sepsis und letalem Ausgang, durch Erstellung eines Expressionsprofils 6 bis 24 Stunden nach Trauma umfassend folgende Schritte:
i) Isolieren von RNA aus humanem Blut,
ii) Markieren der RNA aus Schritt i) mit einem detektierbaren Marker,
iii) Hybridisieren der RNA aus Schritt ii) mit Oligonukleotid-DNA der Gene Seq.-ID 13906 bis Seq.-ID 14075 (Tabelle 8), die auf einem Microarray angeordnet sind, unter Hybridisierungsbedingungen,
iv) Quantitatives Erfassen der Markierungssignale der hybridisierten RNA aus Schritt iii) in einem Expressionsprofil,
v) Vergleich des Expressionsprofiles aus Schritt iv) mit einer Kontrollprobe eines Patienten mit Sepsis aber ohne letalen Ausgang hinsichtlich der Expression der Gene Seq.-ID 13906 bis Seq.-ID 14075 (Tabelle 8),
vi) Bestimmung des TNF-α Blutspiegels sowie Genotypisierung der Allele des TNF-α Gens TNF-308A und des Lymphotoxin-a Gens LTA-252G sowie LTA-80A.

4. Verfahren zum Vorhersagen der Ausbildung einer Sepsis und deren Verlaufskontrolle in humanem Blut eines Patienten, umfassend folgende Schritte:
i) Isolieren von RNA aus einer biologischen Probe eines Patienten,
ii) Markieren der RNA aus Schritt i) mit einem detektierbaren Marker,
iii) Hybridisieren der RNA aus Schritt ii) mit DNA der Gene Seq.-ID 11379 bis Seq.-ID 13905 (Tabelle 7), die auf einem Microarray angeordnet sind, unter Hybridisierungsbedingungen,
iv) Quantitatives Erfassen der Markierungssignale der hybridisierten RNA aus Schritt iii) in einem Expressionsprofil,
v) Vergleich des Expressionsprofiles aus Schritt iv) mit einer Kontrollprobe eines Patienten, der keine Sepsis entwickelt, hinsichtlich der Expression der Gene Seq.-ID 11379 bis Seq.-ID 13905 gemäß (Tabelle 7) am Tag 0, Tag 1, Tag 3, Tag 5 und Tag 9 nach Trauma,
vi) Bestimmung von Proteinen und Metaboliten in der humanen Blutprobe, wobei Patienten, welche eine Sepsis entwickeln, diagnostiziert werden, wenn im Verlauf des stationären Aufenthalts im Vergleich zu Patienten, welche keine Sepsis entwickeln,
- eine zunehmende Ausschaltung der transkriptionellen Aktivität einer ersten Gruppe von Genen der angeborenen und erworbenen Immunität, Apoptose, Transkriptionsfaktoren und des Metabolismus, und
- eine zunehmende Einschaltung der transkriptionellen Aktivität einer zweiten Gruppe von Genen der angeborenen Immunität, insbesondere von Defensinen,
festgestellt wird.

5. Verfahren gemäß einem der Ansprüche 1, 2 oder 4, **dadurch gekennzeichnet, dass** die Verknüpfung des Expressionsprofiles mit dem TNF-α Blutspiegel erfolgt.

6. Verfahren gemäß Anspruch 5, **dadurch gekennzeichnet, dass** die Verknüpfung des Expressionsprofiles mit Mutationsscreening erfolgt.

7. Verfahren gemäß Anspruch 6, **dadurch gekennzeichnet, dass** die Verknüpfung des Expressionsprofiles mit einem Mutationsscreening des TNF-α Allels TNF-308A und der Lymphotoxin-a Allele LTA-252G sowie LTA-80A erfolgt.

8. Verfahren gemäß einem der Ansprüche 1 bis 4, **dadurch gekennzeichnet dass** die Verknüpfung des Expressionsprofiles mit den Laborparametern Blutbild, CRP, PCT, Leber- und Nierenwerten und Elektrolyten erfolgt.

9. Verfahren gemäß einem der Ansprüche 1 bis 5, **dadurch gekennzeichnet dass** die Verknüpfung des Expressionsprofiles mit Expressionsraten von Proteinen aus Genen ausgewählt aus der Gruppe Seq.-ID 1 bis Seq.-ID 6705 (Tabelle 1) erfolgt.

10. DNA-Microarray zur frühzeitigen Diagnose von Sepsis in Blut eines Patienten, **dadurch gekennzeichnet, dass** der Microarray aus Oligonukleotiden der Gene
- Seq.-ID 6706 bis Seq.-ID 7562 (Tabelle 2),
- Seq.-ID 7563 bis Seq.-ID 8947 (Tabelle 3),
- Seq.-ID 11379 bis Seq.-ID 13905 (Tabelle 7) oder
- Seq.-ID 13906 bis Seq.-ID 14075 (Tabelle 8)
besteht.

11. Verwendung eines DNA-Microarrays gemäß Anspruch 10 zur Ermittlung eines Genexpressionsprofiles als Biomarker für die Prädisposition, Ermittlung von Frühsymptomen oder dem Schweregrad und Ausgang einer Sepsis.

## Claims

1. Procedure for the early prediction of sepsis formation in a patient by establishing an expression prolife 6 to 24 hours after trauma, comprising the following steps:
i) Isolation of RNA from human blood,
ii) labeling of RNA of step i) with a detectable marker,
iii) hybridization of RNA of step ii) with oligonucleotide DNA of genes Seq.-ID 6706 to Seq.-ID 7562 (Table 2) which are arranged on a microarray under hybridization conditions,
iv) quantitative recording of labeling signals of hybridized RNA of step iii) in an expression profile,
v) comparison of the expression profile of step iv) with a control sample of a patient who does not develop sepsis with respect to the expression of genes Seq.-ID 6706 to Seq.-ID 7562 (Table 2),
vi) determination of proteins and metabolites in the human blood sample.

2. Procedure for the early prediction of sepsis formation in a patient by establishing an expression prolife 30 to 48 hours after trauma, comprising the following steps:
i) Isolation of RNA from human blood,
ii) labeling of RNA of step i) with a detectable marker,
iii) hybridization of RNA of step ii) with oligonucleotide DNA of genes Seq.-ID 7563 to Seq.-ID 8947 (Table 3) which are arranged on a microarray under hybridization conditions,
iv) quantitative recording of labeling signals of hybridized RNA of step iii) in an expression profile,
v) comparison of the expression profile of step iv) with a control sample of a patient who does not develop sepsis with respect to the expression of genes Seq.-ID 7563 to Seq.-ID 8947 (Table 3),
vi) determination of proteins and metabolites in the human blood sample.

3. Procedure for the identification of risk patients, namely patients with sepsis with lethal outcome, by establishing an expression prolife 6 to 24 hours after trauma, comprising the following steps:
i) Isolation of RNA from human blood,
ii) labeling of RNA of step i) with a detectable marker,
iii) hybridization of RNA of step ii) with oligonucleotide DNA of genes Seq.-ID 13906 to Seq.-ID 14075 (Table 8) which are arranged on a microarray under hybridization conditions,
iv) quantitative recording of labeling signals of hybridized RNA of step iii) in an expression profile,
v) comparison of the expression profile of step iv) with a control sample of a patient who does not develop sepsis with respect to the expression of genes Seq.-ID 13906 to Seq.-ID 14075 (Table 8),
vi) determination of the TNF-α blood level as well as genotyping of allels of the TNF-α gene TNF-308A and the lymphotoxin genes LTA-252G and LTA-80A.

4. Procedure for the prediction of sepsis formation and monitoring the progress thereof in human blood of a patient, comprising the following steps:
i) Isolation of RNA from a biological sample of a patient,
ii) labeling of RNA of step i) with a detectable marker,
iii) hybridization of RNA of step ii) with DNA of genes Seq.-ID 11379 to Seq.-ID 13905 (Table 7) which are arranged on a microarray under hybridization conditions,
iv) quantitative recording of labeling signals of hybridized RNA of step iii) in an expression profile,
v) comparison of the expression profile of step iv) with a control sample of a patient who does not develop sepsis with respect to the expression of genes Seq.-ID 11379 to Seq.-ID 13905 according to (Table 7) on day 0, day 1, day 3, day 5 and day 9 after trauma,
vi) determination of proteins and metabolites in the human blood sample, whereby patients who develop sepsis are diagnosed on the basis of the fact that during the course of the inpatient stay and in comparison to patients who do not develop sepsis,
• increased turn-off of transcriptional activity of a first group of genes of the innate and acquired immunity, apoptosis, transcriptional factors and the metabolism, and
• increased turn-on of transcriptional activity of a second group of genes
of the innate immunity, in particular of defensins
is observed.

5. Procedure according to one of the claims 1, 2 or 4, **characterized in that** a combination of the expression profiles with the TFN-α blood level is performed.

6. Procedure according to claim 5, **characterized in that** said combination of the expression profile is performed using mutation screening.

7. Procedure according to claim 6, **characterized in that** said combination of the expression profile is performed using a mutation screening of the TFN-α allele TFN-308A and lymphotoxin-α alleles LTA-252G as well as LTA-80A.

8. Procedure according to one of the claims 1 to 4, **characterized in that** said combination of the expression profile is performed with the laboratory parameters blood picture, CRP, PCT, liver and kidney values and electrolytes.

9. Procedure according to one of the claims 1 to 5, **characterized in that** said combination of the expression profile is performed with protein expression rates of genes chosen from the group of Seq.-ID 1 to Seq.-ID 6705 (Table 1).

10. DNA-microarray for an early diagnosis of sepsis in the blood of a patient, **characterized in that** said microarray is equipped with oligonucleotides of the genes
• Seq.-ID 6706 to Seq.-ID 7562 (Table 2)
• Seq.-ID 7563 to Seq.-ID 8947 (Table 3)
• Seq.-ID 11379 to Seq.-ID 13905 (Table 7) or
• Seq.-ID 13906 to Seq.-ID 14075 (Table 8)

11. Utilization of a DNA-microarray according to claim 10 for the determination of a gene expression profile as biomarker for the predisposition, determination of early symptoms or the severity and outcome of sepsis.

## Revendications

1. Procédé de prévision précoce de l'apparition d'une septicémie chez un patient par établissement d'un profil d'expression 6 à 24 heures après un traumatisme, comprenant les étapes suivantes :
i) isolation d'ARN du sang humain,
ii) marquage de l'ARN de l'étape i) avec un marqueur détectable,
iii) hybridation de l'ARN de l'étape ii) avec de l'ADN oligonucléotide des gènes à numéros d'identification SEQ ID 6706 à SEQ ID 7562 (tableau 2) qui sont disposés sur une puce à ADN dans des conditions d'hybridation,
iv) détection quantitative des signaux de marquage de l'ARN hybridé de l'étape iii) dans un profil d'expression,
v) comparaison du profil d'expression de l'étape iv) avec un prélèvement de contrôle d'un patient développant une septicémie, en vue de l'expression des gènes allant de la SEQ ID 6706 à la SEQ ID 7562 (tableau 2),
vi) détermination des protéines et métabolites dans le prélèvement de sang humain.

2. Procédé de prévision précoce d'une septicémie d'un patient par établissement d'un profil d'expression 30 à 48 heures après un traumatisme, comprenant les étapes suivantes :
i) isolation d'ARN du sang humain,
ii) marquage de l'ARN de l'étape i) avec un marqueur détectable,
iii) hybridation de l'ARN de l'étape ii) avec de l'ADN oligonucléotide des gènes à numéros d'identification SEQ ID 7563 à SEQ ID 8947 (tableau 3) qui sont disposés sur une puce à ADN dans des conditions d'hybridation,
iv) détection quantitative des signaux de marquage de l'ARN hybridé de l'étape iii) dans un profil d'expression,
v) comparaison du profil d'expression de l'étape iv) avec un prélèvement de contrôle d'un patient développant une septicémie, en vue de l'expression des gènes allant de la SEQ ID 7563 à la SEQ ID 8947 (tableau 3),
vi) détermination des protéines et métabolites dans le prélèvement de sang humain.

3. Procédé d'identification de patients à risque, à savoir de patients ayant une septicémie avec issue létale, par établissement d'un profil d'expression 6 à 24 heures après un traumatisme, comprenant les étapes suivantes :
i) isolation d'ARN du sang humain,
ii) marquage de l'ARN de l'étape i) avec un marqueur détectable,
iii) hybridation de l'ARN de l'étape ii) avec de l'ADN oligonucléotide des gènes à numéros d'identification SEQ ID 13906 à SEQ ID 14075 (tableau 8) qui sont disposés sur une puce à ADN dans des conditions d'hybridation,
iv) détection quantitative des signaux de marquage de l'ARN hybridé de l'étape iii) dans un profil d'expression,
v) comparaison du profil d'expression de l'étape iv) avec un prélèvement de contrôle d'un patient développant une septicémie mais sans issue létale, en vue de l'expression des gènes allant de la SEQ ID 13906 à la SEQ ID 14075 (tableau 8),
vi) détermination du taux de TNF-α dans le sang et génotypage des allèles du gène de TNF-α TNF-308A et du gène de lymphotoxine-α LTA-252G et LTA-80A.

4. Procédé de prévision précoce d'une septicémie et du contrôle de son évolution chez un patient, comprenant les étapes suivantes :
i) isolation d'ARN d'un prélèvement biologique d'un patient,
ii) marquage de l'ARN de l'étape i) avec un marqueur détectable,
iii) hybridation de l'ARN de l'étape ii) avec de l'ADN oligonucléotide des gènes à numéros d'identification SEQ ID 11379 à SEQ ID 13905 (tableau 7) qui sont disposés sur une puce à ADN dans des conditions d'hybridation,
iv) détection quantitative des signaux de marquage de l'ARN hybridé de l'étape iii) dans un profil d'expression,
v) comparaison du profil d'expression de l'étape iv) avec un prélèvement de contrôle d'un patient développant une septicémie, en vue de l'expression des gènes allant de la SEQ ID 11379 à la SEQ ID 13905 (tableau 7) le jour 0, le jour 1, le jour 3, le jour 5 et le jour 9 après un traumatisme,
vi) détermination des protéines et métabolites dans le prélèvement de sang humain,
les patients développant une septicémie étant diagnostiqués lorsque, au cours de leur séjour stationnaire, comparativement aux patients qui ne développent pas de septicémie, il est constaté
- une désactivation croissante de l'activité de transcription d'un premier groupe de gènes de l'immunité innée et acquise, de l'apoptose, des facteurs de transcription et du métabolisme et
- une activation croissante de l'activité de transcription d'un second groupe de gènes de l'immunité innée, en particulier des défensines.

5. Procédé selon une des revendications 1, 2 ou 4, **caractérisé en ce qu'**il y a une interconnexion du profil d'expression avec le taux de TNF-α dans le sang.

6. Procédé selon la revendication 5, **caractérisé en ce qu'**il y a une interconnexion du profil d'expression avec le balayage de mutation.

7. Procédé selon la revendication 6, **caractérisé en ce qu'**il y a une interconnexion du profil d'expression avec un balayage de mutation de l'allèle de TNF-α TNF-308A et de l'allèle de lymphotoxine- a LTA-252G et LTA-80A.

8. Procédé selon une des revendications 1 à 4, **caractérisé en ce qu'**il y a une interconnexion du profil d'expression avec les paramètres de laboratoire que sont la formule sanguine, la protéine créative, la PCT, les taux hépatiques et rénaux et les électrolytes.

9. Procédé selon une des revendications 1 à 5, **caractérisé en ce qu'**il y a une interconnexion du profil d'expression avec des taux d'expression de protéines de gènes sélectionnés dans le groupe allant de SEQ ID 1 à SEQ ID 6705 (tableau 1).

10. Puce à ADN permettant le diagnostic précoce d'une septicémie dans le sang d'un patient, **caractérisée en ce que** la puce à ADN est composée d'oligonucléotides des gènes
- SEQ ID 6706 à SEQ ID 7562 (tableau 2),
- SEQ ID 7563 à SEQ ID 8947 (tableau 3),
- SEQ ID 11379 à SEQ ID 13905 (tableau 7) ou
- SEQ ID 13906 à SEQ ID 14075 (tableau 8).

11. Utilisation d'une puce à ADN selon la revendication 10 pour déterminer un profil d'expression génique sous forme de biomarqueur pour la prédisposition, la détermination des symptômes précoces ou du degré de gravité et de l'issue d'une septicémie.
